# EUROPEAN PATENT APPLICATION

(11) **EP 2 692 871 A1**
(43) Date of publication of application: **05.02.2014**
(21) Application number: 13190520.0
(22) Date of filing: 01.12.2010
(51) Int. Cl.: C12Q 1/68

(54) **Classification of cancers**

(30) Priority: 01.12.2009 US 265495 P
(62) Divisional of application: 10835030.7
(71) Applicant: Compendia Bioscience, Inc., Ann Arbor, Michigan 48104 (US)
(72) Inventor: Rhodes, Daniel R, Ann Arbor, MI 48103 (US)
(74) Representative: Simpson, Verena

(57) **Abstract**

A system for classifying a patient's cancer as belonging to one or more Cancer Modules of 1 of 15 different cancer types is provided. The Cancer Modules are useful to identify patient populations and individual patients demonstrating specific prognosis, risk of metastasis and/or recurrence, response or lack of response to drugs, and the like.

## Description

Gene expression profiling can stratify cancers into molecular subtypes. The general approach has been to perform two-dimensional hierarchical clustering, identify sets of samples that cluster together (*i.e.*, molecular subtypes), and then describe the sets of genes that best correlate with the sets of samples. There are two main disadvantages to this approach. First, molecular subtypes are subject to study-specific biases such as sampling bias, tissue collection bias (*e.g*., stromal contamination), technology bias, tissue processing bias, and a host of others. As a result, a molecular subtype defined in one study may not be representative of molecular subtypes in general. Second, most analyses to date have assumed that every cancer sample must fall into one molecular subtype, limiting practical associations of prognosis and treatment to a single molecular subtype that might not fully define an individual's cancer. Provided herein is a new multi-dimensional approach of classifying cancers.

### SUMMARY OF THE INVENTION

Disclosed herein are Cancer Modules that provide a robust, molecularly based system for classifying cancers. Each Cancer Module, shown in Tables 1-161, contains coexpressed gene members demonstrating patterns unique patterns of gene for classifying cancer. A cancer patient's biological sample is interrogated to identify a pattern of gene expression that is consistent with an expression pattern of gene members of one or more Cancer Module to identify the patient's cancer as belonging to that one or more specific Cancer Module. Tools, systems, and methods for identifying a patient population or individual subject's cancer as belonging to one or more identified Cancer Module, for identifying a patient population or individual patient that can respond to a class of therapeutic drugs and/or to a particular drug, for selecting a drug for treating an individual cancer patient, for predicting risk of cancer metastasis, recurrence, drug toxicity, and other therapeutic and diagnostic characteristics in a patient population and/or for a specific patient arc provided using the Cancer Modules described herein.

Based upon the Cancer Modules disclosed herein, methods for treating a subject include one or more steps of interrogating a biological sample obtained from a subject for expression of one or more gene members of one or more of the Cancer Modules listed in Tables 1-161, identifying the subject's cancer as belonging to one or more Cancer Module, administering to the subject a drug with demonstrated activity against cancers belonging to an identified Cancer Module, identifying if a drug has been demonstrated to lack activity, cause toxicity, or otherwise be detrimental for treating cancers belonging to an identified Cancer Module.

### DETAILED DESCRIPTION

The present invention provides coexpressed genes having an expression pattern in cancer patients that identifies the cancer as belonging to a Cancer Module, and provides methods for classifying cancers as belonging to a disclosed Cancer Module and for identifying a patient population as belonging to a disclosed Cancer Module. Methods using the Cancer Modules as a cancer classification system are provided for example, methods for predicting drug responsiveness in a patient population and methods for treating a subject with a drug demonstrating activity against cancers belonging to the Cancer Module identified for that patient's cancer. Further provided are methods for predicting risk of metastasis and/or cancer recurrence, drug toxicity, drug interactions, and other such diagnostic, prognostic, and therapeutic predictions in a patient population.

Disclosed herein are specific genes that are members of defined Cancer Modules. These defined cancer modules can be used to identify specific patient populations, for example, within a cancer type according to a desired diagnostic, prognostic, or therapeutic outcome. The disclosed Cancer Modules can be used to interrogate and classify a training group of cancer patients and thereby identify a diagnostic, prognostic, or therapeutic outcome for a test patient or population.

A biological sample having a pattern of gene expression that is consistent with a pattern of gene expression of one or more of the disclosed Cancer Modules identifies the patient's cancer as belonging to that one or more Cancer Module, and provides a basis for diagnostic, prognostic, and therapeutic predictions. Tools, methods, systems, kits, platforms, and similar devices comprising specific genes, amplification primers, probes, and/or other tools adapted for determining the expression of a gene member of the provided Cancer Modules and identification of a subject's cancer as belonging to one or more of the Cancer Modules arc also provided herein.

### Definitions

Unless defined otherwise, all technical and scientific terms used herein have the same meaning as commonly understood by those of ordinary skill in the art to which the invention belongs. For the purposes of the present invention, the following terms are defined below.

The articles "a" and "an" arc used herein to refer to one or to more than one (*i.e.* at least one) of the grammatical object of the article. By way of example, "an element" means one or more element.

The term "biological sample" as used herein refers to a sample that may be obtained and assayed for gene expression. The biological sample can include a biological fluid (*e.g.*, blood, cerebrospinal fluid, urine, plasma), tissue biopsy, and the like. In some embodiments, the sample is a tissue sample, for example, tumor tissue, and may be fresh, frozen, or archival paraffin embedded tissue.

Throughout this specification, unless the context requires otherwise, the words "comprise," "comprises," and "comprising" will be understood to imply the inclusion of a stated step or element or group of steps or elements but not the exclusion of any other step or element or group of steps or elements.

The term "gene expression" as used herein refers to the production of a gene product from a gene. A gene product can include, for example, RNA or protein. Gene expression can be measured directly or indirectly using known methods and those disclosed herein. Gene expression, as measured in a biological sample from a subject having cancer, can be modulated as compared to a control.

The term "coexpression," as used herein refers to the relation of a gene's expression with the expression of one or more other genes. Genes that are coexpressed have a pattern of expression that is constant relative to one another, and each can be overexpressed, underexpressed, or remain the same relative to a control.

The term "Cancer Module" as used herein refers to a specific list of coexpressed genes (gene members) useful for classifying a specific cancer. The gene members of particular Cancer Modules are disclosed in Tables 1-161 along with gene identification numbers (GenInfo Identifiers) for each gene member obtained from Genbank ® (http://www.ncbi.nlm.nih.gov/Genbank/).

The term "gene expression profile" as used herein refers to an expression pattern of two or more gene members of a particular Cancer Module, such as any of those listed in Tables 1-161. A gene expression profile can include the expression pattern of two or more (e.g., 2, 3, 4, 5, 10, 15, or more) gene members from the table corresponding to the Cancer Module of interest.

The term "primer" is defined as an oligonucleotide that, when paired with a strand of DNA, is capable of initiating synthesis of a primer extension product in the presence of a suitable polymerizing agent.

"Probe" refers to a molecule that binds to a specific sequence or sub-sequence or other moiety of another molecule. Unless otherwise indicated, the term "probe" typically refers to a polynucleotide probe that binds to another polynucleotide, often called the "target polynucleotide", through complementary base pairing.

A drug that that shows "activity" against a cancer identified as belonging to a particular Cancer Module means that when the drug is administered to a patient having a cancer belonging to the Cancer Module, the cancer exhibits a statistically significant reduction in proliferation or size, or an alteration in any other measurement of a type generally accepted as indicative of cancer responsiveness.

### Gene Expression Profiles

A gene expression profile obtained from a cancer patient's biological sample that is consistent with a pattern of gene expression of two or more members of a Cancer Module identifies the patient's cancer as belonging to that particular Cancer Module. In an embodiment, a gene expression profile includes the expression pattern of, for example, 2-3, 2-5, 3-5, 5-8, 6-8, 8-10, 1-10, 1-15, 1-20, 5-10, 5-15, 5-20, 10-15, 10-20, or 15-20 gene members of a selected Cancer Module.

To identify a patient's cancer as belonging to a Cancer Module, a patient's sample can be interrogated for expression of one or more gene selected from gene members belonging to one or more of the presently identified Cancer Modules (Tables 1-161). In some embodiments, the interrogated genes include gene members from some or all of the Cancer Modules of a given cancer type, for example, breast, colon, brain, and the like. In some embodiments, the interrogated genes will be selected from more than one related cancer type, for example, breast and ovary.

The genes to be interrogated can be chosen from the members of a particular Cancer Module by any appropriate means. Considerations in determining which genes, which modules, and how many genes or modules to interrogate can include the availability of probes or primers for a particular gene member, the number of genes easily tested using the method of choice (e.g., microarray or PCR), the type of cancer, the purpose of the analysis, and the like. For example, gene members ranked highest in each module can be selected; gene members associated with a particular metabolic pathway can be selected; or gene members can be randomly selected for interrogation. Cancer Modules can be selected for association with a known diagnosis, prognosis, drug response, and the like.

### Examples of Gene Expression Profiles

Examples of gene members of Cancer Modules to be interrogated include two or more of any of the gene members listed in the Cancer Module Tables 1-161. For example, for Brain Cancer Module 1, two or more of any of the gene members listed in Table 7 can be interrogated. For Brain Cancer Module 2, two or more of any of the gene members listed in Table 8 can be interrogated. Table 162 provides non-limiting examples of gene members that can be interrogated for identifying a patient's cancer as belonging to a specific Cancer Module.

| **Table 162. Examples of Genes to be Interrogated** | | | | |
|---|---|---|---|---|
| | **Method of gene selection** | | | |
| **Cancer Module** | **Highest 2 ranks** | **Rank >3** | **Random** | **Random** |
| Bladder Cancer Module 3 | DSC2 | DSC2 | ABCA12 | SPRR1B |
| | SPRR1B | SPRR1B | S100A8 | SLPI |
| | | | CRCT1 | PKP1 |
| | | | VSNL1 | SERPINB4 |
| | | | RHCG | SERPINB3 |
| | | | PI3 | S100A8 |
| | | | | CRCT1 |
| | | | | LGALS7 |
| Brain Cancer Module 1 | DLL3 | DLL3 | SOX4 | CHD7 |
| | SCN3A | SCN3A | FAM110B | HEY1 |
| | KLRC2 | KLRC2 | HEY1 | ZEB1 |
| | TOX3 | TOX3 | | HN1 |
| | KLRC3 | KLRC3 | | RAP2A |
| | EPHB1 | EPHB1 | | RAP2B |
| | C20orf42 | C20orf42 | | BID |
| | DLL1 | DLL1 | | ENAH |
| | SOX4 | SOX4 | | |
| | | MYCN | | |
| | | LOC254559 | | |
| | | LRRN1 | | |
| | | HES6 | | |
| | | C1AL1 | | |
| | | ASCL1 | | |
| | | SHD | | |
| | | GLCCI1 | | |
| Brain Cancer Module 4 | IGKC | IGKC | IGHM | IGKC |
| | IGL@ | IGL@ | IGHG1 | IGHG3 |
| | IGHA1 | IGHA1 | LCK | LOC91316 |
| | | IGLJ3 | EAF2 | IGHG1 |
| | | LOC91316 | IGHV1-69 | LCK |
| | | IGHG3 | CD37 | EAF2 |
| | | IGHM | | |
| | | NTN2L | | |
| Breast Cancer Module 14 | HIST2H2BE | HIST2H2BE | HIST2H2BE | HIST1H1C |
| | HIST1H1C | HIST1H1C | HIST1H1C | HIST1H3D |
| | HIST1H2BC | HIST1H2BC | HIST1H2BK | HIST1H4H |
| | HIST1H2BF | HIST1H2BF | HIST2H2AA3 | HIST1H3H |
| | | HIST1H2BK | H2BFS | HIST2H2AA3 |
| | | HIST1H3D | HIST1H2BE | H2BFS |
| | | HIST1H4H | HIST1H2BH | HIST1H2BH |
| | | HIST1H3H | HIST1H2AE | HIST1H2AE |
| | | HIST2H2AA3 | HIST1H2BI | HIST1H2BI |
| | | H2BFS | HIST1H2AD | HIST1H2AJ |
| | | HIST1H2BE | HIST1H2AJ | HIST1H2BO |
| | | HIST1H2BH | | HIST1H3G |
| | | HIST1H2AE | | CPS1 |
| | | HIST1H2BI | | |
| | | HIST1H2AD | | |
| | | HIST1H2AJ | | |

The gene expression profile can indicate that a patient's cancer belongs to more than one Cancer Module, as described more fully in the Examples below. In some embodiments, interrogation of one gene member may be sufficient to identify the patient's cancer as belonging to that module.

### Analysis of Gene Expression Profiles

The classification of a patient's cancer as belonging to a Cancer Module is determined by interrogating a biological sample for the expression of one or more gene member of the Cancer Module and identifying the patient's cancer as belonging to the Cancer Module if the module has a pattern of gene expression consistent with the pattern of expression of the one or more gene member interrogated in the biological sample.

Gene expression can be analyzed in a variety of platforms, assays, and methods, and is generally analyzed by amplification and/or detection of mRNA extracted from the subject's biological sample, or by detection of gene expression products such as, for example, cDNA and protein, or by analysis of genomic DNA. A subject's sample can be interrogated for the expression of a gene member of a Cancer Module using various known techniques as described below.

In some embodiments, a tissue other than the subject's cancer can be interrogated for expression of one or more gene member of a Cancer Module. For example, a lymph node, blood, serum, or urine can be interrogated for expression of a gene member in a cancer. In such embodiments, presence of a nucleic acid, protein, or cell originating from the cancer can be interrogated in the selected tissue or fluid.

Prior to interrogation, the biological sample can be processed. Such processing can affect the way the analysis is performed on the sample. For example, formalin-fixed paraffin embedded samples arc generally analyzed using different techniques than arc used with fresh or frozen samples. Such differences will be apparent to those skilled in the art.

The sample is interrogated for the expression of at least one gene member of a Cancer Module using at least one analytic technique. An analytic technique can directly measure gene expression, for example, by RNA analysis or protein analysis, or can measure gene expression indirectly, for example by genomic analysis. A review of methods for quantitative detection of gene expression can be found, for example, in Nygaard, et al., 2009, Front Biosci, 14:552-69.

A gene expression profile can be identified from analysis of a sample, and can be compared to one or more Cancer Module to identify a patient's cancer as belonging to one or more Cancer Module.

### RNA analysis

In some embodiments, a biological sample containing RNA originating from a patient's cancer can be interrogated using known methods. Biological samples can be purified as appropriate for the analytic technique to be used. Purification techniques can include, for example, laser-capture microdissection to isolate cancer cells from non-cancer tissue. Tumor cells in blood or other biological fluid can be isolated using purification techniques such as antibody-mediated purification, for example, fluorescence activated cell sorting, centrifugation- or gravity-based cell sorting, magnetic activated cell sorting, and the like.

RNA can be used directly as extracted and purified for analytic techniques such as Northern blotting, which can be used to measure relative RNA expression. Methods for isolating mRNA from a tissue sample for further analysis are described, for example, in Ausubel et al., 2002, Short Protocols in Molecular Biology, 4:4-1 - 4-29. Methods for isolating mRNA from paraffin embedded tissue in particular are discussed, for example, in Penland, et al., 2007, Laboratory Investigation, 87:383-391. RNA isolation kits are commercially available, including, for example, Paraffin Block RNA Isolations Kits available from Ambion, Inc. (Austin, TX).

In some embodiments, RNA is subjected to gel electrophoresis and detected using probes labeled with a tag that may be radioactive. RNA levels in the sample can be compared with RNA levels from a reference sample, such as a normal control tissue, and the like, to determine relative expression levels of the selected gene member(s).

For some analytic techniques, RNA is processed to produce complementary DNA (cDNA) or complementary RNA (cRNA). A reverse transcriptase (RT) enzyme, in conjunction with appropriate primers (e.g., oligo(dT), T7-oligo(dT) primers, or random primers), can be used to reverse transcribe RNA into cDNA. Single stranded or double stranded cDNA can be used directly in PCR-based assays, such as non-quantitative PCR, quantitative PCR, and/or quantitative real time PCR. Quantitative PCR using cDNA can be used to analyze expression levels of the RNA, and PCR products from cDNA can be sequenced for mutation analysis. Additionally, cDNA can be used in microarray analysis to measure expression levels of the RNA.

Complementary DNA can be further processed into cRNA. Typically, cRNA is produced from cDNA that incorporates a primer containing an RNA polymerase promoter, such as T7-oligo(dT) primer. An RNA polymerase that recognizes the promoter can be used to *in vitro* transcribe cRNA, resulting in linearly amplified cRNA from the cDNA template. Complementary RNA can be used in assays such as microarrays (e.g., Affymctrix GeneChips©) and the like, to analyze gene expression levels.

In some embodiments, cancer cells are left intact and RNA is analyzed using *in situ* analytic techniques. For example, RNA can be reverse transcribed *in vitro*, and subsequently analyzed using immunohistochemistry PCR, (Fassunke, et al., Labaratoy Investigation, 84:1520-5 (2004)) and the like.

Methods for analyzing expression RNA include, for example, Northern blotting (Brown, 2001, Curr Protoc Immunol, Chapter 10:10.12; Parker & Barnes, 1999, Methods in Molecular Biology 106:247-283), reverse transcriptase polymerase chain reaction (RT-PCR) (Nygaard, et al., 2009, Front Biosci, 14:552-69; Weis et al., 1992, Trends in Genetics, 8:263-64, massively parallel signature sequencing (MPSS) (Kutlu, 2009, BMC Med Genomics., 2:3; Brenner, 2000, Nature Biotechnol, 18:1021), Serial Analysis of Gene Expression (SAGE) (Boon, 2009, PLoS ONE, 4:e5134; Velculescu, 1995, Science, 270:368-9, 371), RNA-mediated annealing, selection, and ligation (RASL) assay (Ycakley, 2002, Nat Biotechnol, 20:353-8), a cDNA mediated annealing, selection, extension, and ligation (DASL) assay (Abramovitz, 2008, Biotechniques, 44:417-423; Fan, 2004, Genome Research, 14:878-85), microarray techniques (Ravo, et al., 2008, Lab Invest, 88:430-40; Schena, 1996, Proc Nat. Acad Sci USA, 93:106-149), including Incyte's microarray technology, Affymetrix's GenChip technology; high throughput sequencing techniques developed by 454 Life Sciences, Inc. (Branford, CT) (Marguilies, 2005, Nature, 437:376-80), and the like.

### RT-PCR

RT-PCR methods useful for determining gene expression in a sample arc described, for example, in Sambrook, 2001, Molecular Cloning: A Laboratory Manual*.* Clinical samples such as tumor biopsy tissue or archived paraffin embedded and/or frozen samples provide RNA templates for genetic analysis. General methods of performing PCR are described, for example, in Ausubel, et al., 2002, Short Protocol in Molecular Biology*;* Mullis and Faloona, 1987, Methods Enzymol, 155:335). Primers for performing RT-PCR can be obtained commercially, or can be designed using commercially available software (e.g., Scientific Software, Primer Designer 1).

### DASL

In a DASL assay, total RNA is converted to cDNA using biotinylated primers, and the biotinylated DNA is attached to a streptavidin solid support, followed by the annealing of assay oligonucleotides to their target sequences in the cDNA. A pair of oligonucleotides is annealed to a given target site, with three to ten target sites per gene. The upstream annealed oligonucleotides are extended and ligated to corresponding nucleotides downstream to create a PCR template that is then amplified with universal PCR primers. The PCR products, having been labeled by incorporation of a labeled primer, are hybridized to capture sequences on the solid support array, and the fluorescence intensity is then measured for each bead.

Complete custom designed DASL assay panels for up to 1536 genes comprising 1-3 probe groups per gene arc available commercially from Illumina, Inc. (San Diego, CA), as well as a standard DASL human cancer panel comprising a set of probe groups targeting 502 genes that have been associated with cancer in the literature.

### MassARRAY

The MassARRAY system is used to isolate and reverse transcribe RNA to cDNA. The cDNA is amplified, dephosphorylated, extended with primers, and then placed onto a chip array for analysis via MALDI-TOF mass spectrometry. Hardware and software for carrying out MassARRAY analysis is commercially available from Sequenom, Inc. (San Diego, CA).

### SAGE

In SAGE, multiple sequence tags of about 10-14 base pairs, each corresponding to a unique position within an RNA transcript are linked together to form extended molecules for sequencing and identifying the sequence of multiple tags simultaneously. A transcript's expression pattern can be quantitated by determining the abundance of a given tag and identifying the gene corresponding to that tag. Kits for performing SAGE as well as software for analyzing SAGE data arc commercially available, including, for example, the I-SAGE Kit (Invitrogen, Carlsbad, CA). SAGE data can be used to search, for example, the SAGEmap database at www.ncbi.nlm.nih.gov/SAGE.

### Protein and polypeptide analysis

In some embodiments, a biological sample containing a protein or polypeptide originating from a patient's cancer can be interrogated. Such a sample can comprise cancer cells, or can contain a protein or polypeptide substantially free of cancer cells, such as protein or polypeptide that is secreted from a cancer cell or is released during cancer cell necrosis. Protein and/or polypeptide expression levels can be used to infer gene expression levels since mRNA levels arc generally well correlated with protein expression levels (Guo, et al., 2008, Acta Biochim Biophys Sin, 40:426-36).

Tumor cells can remain unpurified or can be purified using known methods. Depending on analysis method(s) used, more or less purification may be desired.

In some embodiments, protein/polypeptide levels can be determined using Western blotting with antibodies specific to a protein/polypeptide gene product of a gene member of a Cancer Module listed in Tables 1-161. Similarly, other antibody-based assays, such as enzyme-linked immunosorbcnt assays (ELISAs) or protein arrays (see, for example, Joos and Bachman, 2009, Front Biosci, 14:4376-85), can utilize antibodies to measure protein/polypeptide levels.

In some embodiments, a protein or polypeptide can be detected using a molecule other than an antibody. For example, a protein receptor can be used to detect the presence of its cognate ligand or *vice versa.* Other methods for detecting polypeptides include mass spectrometry.

A chosen method of protein/polypeptide analysis used can depend on the source of thc protein/polypeptide. For example, more sensitive methods would be desirable for measuring the level of proteins or polypeptides that are dilute in the biological sample, for example proteins secreted by a cancer into blood. Conversely, the analysis method does not need to be as sensitive if the source of the protein is concentrated, such as protein extracted from a cancer cell sample.

The method of protein/polypeptide analysis used can also depend on the number of proteins and/or polypeptides that are to be measured. For example, Western blotting can be used if only a few proteins/polypeptides arc to be measured, while a protein array would be useful for detecting many proteins and/or polypeptides.

The results of protein expression level analysis can be compared to protein expression levels in a control to infer relative gene expression levels and identify a gene expression pattern. In some embodiments, it is possible to infer a gene expression pattern based on absolute protein expression levels. The gene expression pattern can then be matched to a pattern of expression of gene members of a disclosed Cancer Module in Tables 1-161.

### Genomic analysis

In some embodiments, a biological sample containing DNA originating from a patient's cancer can be interrogated. DNA analysis results can be used to infer gene expression levels as described below.

Biological samples can be purified as appropriate for the analytic technique being used. For example, purified cancer cells can be appropriate for analyzing acetylation or methylation status of cancer DNA, whereas cancer cell purification can be less important when analyzing for the presence of a DNA sequence mutation.

DNA is extracted from the biological sample or purified cancer cells using known techniques. A DNA sample can be subjected to one or more types of analysis, including DNA sequence analysis, SNP (single nucleotide polymorphism) analysis, gene copy number analysis, nucleic acid insertions and/or deletions, viral insertions, and acetylation/methylation status. Other appropriate types of analyses, and techniques for performing such analyses, are known. For example, DNA can be amplified using polymerase chain reaction (PCR) and used in various protocols such as SNP analysis (sec, for example, Kwok, 2002, "Single Nucleotide Polymorphisms: Methods and Protocols." In: Methods in Molecular Biology, Vol. 212. Walker (ed.). Humana Press).

Gene copy number variance can be analyzed using a variety of known methods, assays, and platforms. A review of methods for detecting and analyzing copy number variations (CNV) can be found, for example, in Lee, et al., 2008, Cytogenet Genome Res, 123:333-42. Specific methods for performing CNV analysis include, for example, qt-PCR (Wu, et al., 2007, J Immunol, 179:3012-25), DNA microarrays based upon fluorescent in situ hybridization (FISH) or SNP arrays (Redon, et al., 2006, Nature, 444:444-54), sequencing methodologies such as those reviewed in Hall, 2007, J Exp Biol, 209:1518-25), RFLP/Southern blot analysis (Yang, et al., 2007, Am J Hum Genet, 80:1037-54), ligation detection reaction (LDR) (Seo, et al., 2007, BMC Genet, 8:81), invader assays (Pielberg, et al., 2003, Genome Res, 13:2171-7), and pyrosequencing (Sodcrback, et al., 2005, Clin Chem, 51:522-31).

In some embodiments, cancer cells arc left intact and DNA is analyzed using *in situ* techniques, such as fluorescent *in situ* hybridization in conjunction with fluorescence microscopy, fluorescence activated cell sorting, or image scanning flow cytometry (Basiji et al., 2007, Clin Lab Med, 27(3):653).

The results of genomic analysis can be compared to a control to identify differences between the control DNA sequence, gene copy number, and nucleic acid acetylation/mcthylation status of the sample DNA, as appropriate for the analysis mcthod(s) used. Any differences can be identified and used to infer gene expression. For example, increases in DNA acetylation of a gene would be expected to be associated with an increase in expression of that gene. Conversely, increases in DNA methylation of a gene would be expected to be associated with a decrease in expression of that gene.

### Kits and Tools

Representative tools applying the newly identified associations between genetic expression profiles and Cancer Modules include assay systems for microarray, protein array, ELISA, hybridization, amplification, PCR, DASL, SAGE, and the like systems, as well as kits, chips, cards, multi-well assay plates, probes and primers, and the like, adapted and designed to measure the expression of one or more gene members of one or more Cancer Module selected from Tables 1-161.

In some embodiments, panels of nucleic acid probes and/or primers can be designed to amplify and/or detect the presence of the gene members of one or more Cancer Module selected from Tables 1-161. Such probes include isolated genes, including reference and mutated genes, or portions of such genes, isolated mRNA, cDNA derived from these, amplified nucleic acids, and the like useful, for example in microarray and hybridization platforms. Such primers include nucleic acids flanking a desired amplicon and useful in amplification reactions such as PCR to amplify a desired gene or portion of a gene for detection and quantifying gene expression.

In some embodiments, panels of binding molecules can be produced to bind, detect, and/or quantify gene expression products of one or more gene member of one or more Cancer Module, such as proteins or peptides. Such binding molecules can include antibodies, ligands, ligand receptors, small molecules, and the like.

An assay substrate such as a hybridization plate, chip, card, and the like, is adapted and designed to include primer pairs and/or probes that amplify and/or identify and/or sequence and thereby measure gene expression in a sample obtained from a subject.

Kits include reagents and tools useful to measure gene expression and include, for example, nucleic acid probes and/or primers designed to amplify, detect, and/or quantify in a sample one or more gene member from one or more Cancer Module selected from Tables 1-161.

### Methods for Cancer Classification

The newly identified Cancer Modules described herein allow for the molecular classification of cancers. Generally, methods for classifying cancers involve obtaining gene expression data from a cancer subject's biological sample, identifying a pattern of gene expression obtained from the biological sample that matches a pattern of gene expression in a Cancer Module identified in Tables 1-161, and thereby identifying the subject's cancer as belonging to the matched Cancer Module.

A subject's cancer can be classified as belonging to a Cancer Module by interrogating a biological sample from the subject for the expression of one or more gene member of a Cancer Module and identifying the patient's cancer as belonging to a Cancer Module having a pattern of gene expression consistent with the expression of the one or more interrogated gene member. In some embodiments, a cancer patient's biological sample can be interrogated for the expression of a single gene member of a Cancer Module to identify whether or not the patient's cancer belongs to the Cancer Module. In other embodiments, a cancer patient's biological sample can be interrogated for the expression of more than one gene member of one or more Cancer Module to identify whether or not the patient's cancer belongs to the one or more Cancer Module. In some embodiments, a patient's cancer gene expression profile can indicate that the cancer can be classified in more than one Cancer Module.

### Methods for predicting efficacy of drug treatment

The newly identified Cancer Modules allow for identifying a population of cancer patients responsive to a selected drug. Generally, methods for predicting a population of patients responsive to a drug involve identifying the patient's cancer as belonging to one or more Cancer Module of Tables 1-161 and determining in a training group of patients, each patient's response to the administered drug as well as one or more Cancer Module to which the patient's cancer belongs (Tables 1-161). Responsiveness of test patients having cancers identified as belonging to one or more Cancer Module can then be predicted according to the demonstrated response of the training group patient's having cancer belonging to that one or more Cancer Module.

For example, methods for predicting patient responsiveness can involve interrogating biological samples obtained from cancer patients in a training group for expression of one or more gene member of one or more Cancer Module, identifying each training patient's cancer as belonging to a Cancer Module having a pattern of gene expression consistent with the expression of the one or more interrogated gene, identifying response of each training patient's cancer to a test drug as responsive or non-responsive to the drug, identifying one or more Cancer Module as consistent with training patient responsiveness or non-responsiveness to the drug, and predicting responsiveness or non-responsiveness of test patients with a cancer identified as belonging to the one or more identified Cancer Module. Thus, a patient's likelihood of responsiveness or non-responsiveness can be correlated with the level of responsiveness or non-responsiveness associated with a Cancer Module assigned to the patient's cancer.

### Methods for selecting a therapeutic drug

Gene expression modules are also useful in selecting a therapeutic drug for individual cancer patients. Generally, methods for selecting a therapeutic drug involve interrogating a Biological sample obtained from a cancer patient for expression of one or more gene member of one or more Cancer Module, identifying the patient's cancer as belonging to a Cancer Module having a pattern of gene expression consistent with the expression of the one or more interrogated gene, and selecting a drug having demonstrated activity for cancers belonging to the identified one or more Cancer Module and/or not selecting a drug that does not demonstrate activity for cancers belonging to the identified one or more Cancer Module.

### Methods for predicting metastasis or recurrence

Cancer Modules are also useful in identifying a patient population at risk for cancer metastasis and/or recurrence. Generally, methods for identifying a patient population at risk for metastasis and/or recurrence include determining in a training group of cancer patients, demonstration of metastasis/no metastasis and/or recurrence/nonrecurring and also identifying each training patient's cancer as belonging to one or more Cancer Module of Tables 1-161. Risk of metastasis and/or recurrence of test patients having cancers identified as belonging to the one or more Cancer Module can then be predicted according to the demonstrated metastasis/no metastasis and/or recurrence/no recurrence of the training group patients having cancer belonging to the identified, Cancer Module.

For example, methods for predicting cancer metastasis and/or recurrence can involve interrogating biological samples obtained from cancer patients in a training group for expression of one or more gene member of one or more Cancer Module, identifying each training patient's cancer as belonging to a Cancer Module having a pattern of gene expression consistent with the expression of the one or more interrogated gene, identifying the incidence of metastasis and/or recurrence of each training patient's cancer, identifying one or more Cancer Module as consistent with training patient incidence of metastasis and/or recurrence, and predicting risk of metastasis and/or recurrence in test patients with a cancer identified as belonging to the one or more identified Cancer Module. Thus, a patient's risk of metastasis and/or recurrence can be correlated with the risk of metastasis and/or recurrence associated with a Cancer Module assigned to the patient's cancer.

### Methods of Treatment

Gene expression modules are also useful in selecting a method of treatment for individual cancer patients. Generally, methods for selecting a method of treatment involve interrogating a biological sample obtained from a cancer patient for expression of one or more gene member of one or more Cancer Module, identifying the patient's cancer as belonging to a Cancer Module having a pattern of gene expression consistent with the expression of the one or more interrogated gene, administering a drug having demonstrated activity for cancers belonging to the identified one or more Cancer Module, and not administering a drug demonstrating a lack of activity for cancers belonging to the identified one or more Cancer Modules.

Methods for determining an appropriate means and dosage of administration of a drug for a particular patient's cancer can be determined generally or can be identified as consistent with effective treatment modalities for the identified Cancer Module.

### Other Methods

As described herein, the disclosed Cancer Modules arc useful for identifying patient populations having a unique pattern of gene expression that can be correlated with disease prognosis, therapy, resistance, and the like.

### EXAMPLES

The invention may be more clearly understood and practiced with reference to the specific embodiments described in the following non-limiting examples.

### Example 1

### Meta-analysis of Gene expression in Cancer

Analytical results of gene expression in cancer patients was obtained from the Oncomine database at http://www.oncomine.org and was processed and normalized as described in Rhodes et al., Neoplasia, 2007 Feb;9(2):166-80. Datascts from the 15 most represented cancer types were analyzed. Average linkage hierarchical clustering, using the Pearson correlation as the distance metric, was performed on each dataset. Up to 10,000 features (but not more than 50% of all features) with the largest standard deviations were included in the analysis. To reduce the hierarchical clustering results to discrete gene expression clusters, the clusters with the most features having a minimum Pearson correlation of 0.5 and a minimum of 10 features were identified (Rhodes, Neoplasia, 2007). Pair-wise association analysis was performed on each pair of clusters, counting the number of overlapping genes, computing an odds ratio and calculating a p-value based on Fisher's exact test. Significant associations were defined as those with at least 3 genes overlapping, an odds ratio > 10, and a p-value < 1E-6.

### Example 2

### Identification of Cancer Modules

A network representation (Cytoscape) was used to visualize the pairwise cluster associations and identify modules of highly interconnected clusters. To reduce the cluster association network to a discrete set of modules, edges without at least two supporting indirect associations were removed and nodes and edges that linked two otherwise mostly unlinked sets of interlinked clusters were removed. Each Cancer Module was defined as a list of interlinked clusters.

Representative genes were ranked for each module based on the number of clusters in which they were a member. Identified Cancer Modules for 15 cancer types are shown in Tables 1-161.

### Example 3

### Using Quantitative RT-PCR to Identify Cancers Belonging to a Cancer Module

A tumor biopsy is obtained from a patient. Messenger RNA is purified from the biopsy using a Dynabeads® Oligo(dT)₂₅ mRNA purification kit (Invitrogen, Carlsbad, CA), according to the manufacturer's protocol. Briefly, tumor cells are lysed by grinding the sample in liquid nitrogen to form crude lysate. The lysate is added to washed Dynabeads® Oligo(dT)₂₅ beads and allowed to incubate at room temperature to allow the annealing of poly-A mRNA to the beads. The beads arc recovered with the bound mRNA using a magnet, and other cell components arc washed away. The mRNA is eluted from the beads for use in RT-PCR.

The purified mRNA is reverse transcribed using a RETROscript® cDNA kit (Ambion®, Austin, TX), according to the manufacturer's protocol for two-step RT-PCR. Briefly, 20-200 ng mRNA is mixed with random decamer primers and denatured at 85° C. The primers are then allowed to anneal to the mRNA template on ice. A dNTP mixture, MMTV-RT, an RNasc inhibitor and RT buffer arc added, and the mixture is incubated at 42-44° C to allow reverse transcription. The reverse transcriptasc is deactivated by a brief incubation at 92° C. The cDNA is used in PCR immediately, or can be stored at -20° C for later PCR analysis.

The cDNA is analyzed using the LightCycler® thermocycler (Roche Diagnostics Corporation, Indianapolis, IN). PCR is performed using the LightCycler® Multiplex DNA Master HybProbe kit (Roche), according to the manufacturer's protocol, to assay for up to four gene targets at once. A segregation panel containing Cancer Modules is chosen based on the cancer type (e.g., breast cancer, bladder cancer, colon cancer, etc.). HybProbe probes designed for any or each of the selected target sequences in each of the Cancer Modules for the chosen segregation panel are used. For example, if the cancer is breast cancer, probes representing at least one gene from each of Breast Cancer Modules 1-25 of Tables 22-46 would be used. Three to five genes from each of the modules for a specific cancer type can be used. Genes from fewer than all of the Cancer Modules of a given cancer type can be used. Genes from Cancer Modules of multiple cancer types can be used, for example, breast and ovary Cancer Modules. At least one set of HybProbe probes can be used to detect a reference gene, such as actin, that can be used to normalize cDNA content. The cDNA is mixed with master mix (containing Taq DNA polymerase, reaction buffer, MgCl₂, and dNTP mix with dUTP instead of dTTP), additional MgCl₂, if necessary, up to 4 sets of HybProbes (including at least one set targeted to a normalizing gene), and nuclease free water. The mixture is put into LightCycler® capillaries and placed in the LightCycler® thermocycler. PCR is run using a cycle appropriate for the probes used. Typically, the samples are subjected to a denaturing step at 95° C for 5-10 minutes. The samples arc then denatured (95° C, for 10 seconds), annealed (temperature depending on the probes used, for 5-15 seconds), and the primers extended (72° C, for a length of time dependent on the expected length of the PCR products), for several cycles as needed for the signal strength of each of the probes to plateau. The samples are then slowly heated to denature the probes from the PCR product to determine melting temperature, which can then be used to determine purity of the PCR product.

The number of cycles required for the signal of each probe set in the tumor samples to reach a set threshold can be compared to the number of cycles required in a control sample to reach the same threshold in order to determine the relative expression level of the initial mRNA. The threshold point of the normalizing gene can be used to normalize general mRNA quantity between the tumor sample and the control sample and allow accurate comparison of gene expression levels between the two samples. The relative expression for each measured gene can be used to identify the patient's cancer as belonging to one or more of the Cancer Modules set forth in Tables 1-161.

The patient's prognosis, including response to therapy, recurrence, and/or metastasis, can be predicted based on the Cancer Module identified, for example, using RT-PCR and the expression profiles of the Cancer Modules set forth in Tables 1-161. Prognosis is based on the prognosis, recurrence, metastasis, or drug response demonstrated by cancers belonging to the same Cancer Module(s) of the patient's cancer as determined by comparing the patient's cancer gene expression pattern with that of the disclosed Cancer Modules.

### Example 4

### Using Microarray to Identify Cancers Belonging to a Cancer Module

Purified total RNA is obtained from a cancer patient's tumor biopsy using standard procedures. The RNA is further prepared for Affymetrix® GeneChip® analysis using the GeneChip® 3' IVT Express Kit (Affymetrix®, Santa Clara, CA), according to the manufacturer's protocol. Briefly, 50-500 ng total RNA is mixed with diluted polyA RNA controls, and RNase-free water to a total volume of 5 µl. The mixture is combined with a first strand cDNA synthesis master mix containing RT enzyme and buffer and incubated at 42° C for two hours to produce first strand cDNA. Second strand cDNA is produced by combining the first-strand cDNA with a second strand master mix containing DNA polymerase and buffer, and incubating at 16° C for an hour, followed by an incubation period of 10 minutes at 65° C. The cDNA is then *in vitro* transcribed by adding an IVT master mix containing an IVT enzyme mix, biotin label, and buffer, and incubating at 40° C for 4 to 16 hours to produce biotin-labeled cRNA (i.e., aRNA). The cRNA is purified, washed, and eluted using the magnetic beads, wash buffer, and elution solution provided in the kit. The labeled cRNA is fragmented into fragments having 35-200 nucleotides using the fragmentation buffer provided in the kit.

Fragmented and labeled cRNA is prepared for hybridization to a GeneChip® Human Genome Focus Array containing all the genes from each Cancer Module for a given cancer type, according the manufacturer's instructions. Briefly, the fragmented cRNA is mixed with a hybridization cocktail containing hybridization controls, control oligonucleotide B2, DMSO and buffer, and incubated at 99° C for 5 minutes, and then at 45° C for 5 minutes, while the array is prewet and incubated with prehybridization mix at 45° C. The prchybridization mix is then replaced with the hybridization cocktail containing the labeled, fragmented cRNA, and incubated for 16 hours at 45° C.

Following hybridization, the array is washed, stained, and scanned using the Affymetrix® Hybridization, Wash, and Stain kit, according to the manufacturer's protocol. Briefly, the array is washed twice using the provided wash buffers, stained with a first stain cocktail, washed, stained with a second stain cocktail, stained again with the first stain cocktail, washed, and then filled with a buffer. The array is then scanned using an Agilent GeneArray® Scanner or a GeneChip® Scanner 3000. The raw scanning data can be normalized according to the controls included at various steps during processing, and relative and absolute gene expression can be determined from the scanned array. The relative expression for each measured gene can be used to identify the patient's cancer as belonging to one or more of the Cancer Modules set forth in Tables 1-161.

The patient's prognosis, including response to therapy, recurrence, and/or metastasis, can be predicted based on the Cancer Module identified, for example, using microarray analysis and the expression profiles of the Cancer Modules set forth in Tables 1-161. Prognosis is based on the prognosis demonstrated by cancers belonging to the same Cancer Module(s) as that of the patient's cancer as determined by comparing the patient's cancer gene expression pattern with that of the disclosed Cancer Modules.

### Example 5

### Use of Cancer Modules to Identify a Tumor Signature

Genes in a Cancer Module (Tables 1-161) can be categorized by gene ontology and relation to cell signaling pathways. Gene ontology information is identified for a gene using data available from the GO Consortium (http://www.geneontology.org/) and other known methods, such as the online search tool for gene ontology, AmiGO (http://amigo.geneontology.org/cgi-bin/amigo/go.cgi), and the like. For example, a search using AmiGO for COL1A2 (found in Bladder Cancer Module 6) indicates that COL1A2 is categorized as relating to the ontologies shown below in Table 163.

| Table 163. COL1A2 Gene Ontology | | |
|---|---|---|
| **Ontology: Biological Process** | | |
| | **Category** | **Accession number** |
| | blood vessel development | go:0001568 |
| | collagen fibril organization | go:0030199 |
| | Odontogenesis | go:0042476 |
| | regulation of blood pressure | go:0008217 |
| | Rho protein signal transduction | go:0007266 |
| | skeletal system development | go:0001501 |
| | skin morphogenesis | go:0043589 |
| | transforming growth factor beta receptor signaling pathway | go:0007179 |

| **Ontology: Cellular Components** | | |
|---|---|---|
| | **Category** | **Accession number** |
| | collagen type I | go:0005584 |
| | extracellular region | go:0005576 |
| | extracellular space | go:0005615 |
| | plasma membrane | go:0005886 |

| **Ontology: Molecular Functions** | | |
|---|---|---|
| | **Category** | **Accession number** |
| | extracellular matrix structural constituent | go:0005201 |
| | identical protein binding | go:0042802 |
| | protein binding, bridging | go:0030674 |

Gene ontology information for the genes in a selected Cancer Module can be analyzed for patterns, either manually, or using computer software designed to identify patterns in gene ontology data. Patterns can include a high frequency of genes with related ontologies or association with a cell signaling pathway. For instance, Bladder Cancer Module 2 contains a high percentage of member genes having ontologies related to protein production.

As shown in Table 164, for example, 9 of 20 member genes of Bladder Cancer Module 2 are classified as having a molecular function in Gene Ontology Accession No. go:000373structural constituent of ribosome, defined as the action of a molecule that contributes to the structural integrity of the ribosome. Sixteen of 20 member genes of Bladder Cancer Module 2 arc classified as having a biological process in Gene Ontology Accession No. go:0006414-translational elongation, defined as the successive addition of amino acid residues to a nascent polypeptide chain during protein biosynthesis. Fifteen of 20 member genes of Bladder Cancer Module 2 arc classified in the cell composition Gene Ontology Accession No. go:0005829-cytosol, defined as the part of the cytoplasm that does not contain organelles but does contain other particulate matter, such as protein complexes.

| **Table 164. Ontology of Genes in Bladder Cancer Module 2** | | | | |
|---|---|---|---|---|
| **Gene Symbol** | **Gene Name** | **Accession No. go:0003735 (structural constituent of ribosome)** | **Accession No. go:0006414 (translational elongation)** | **Accession No. go:0005829 (cytosol)** |
| RPL5 | ribosomal protein L5 | X | X | X |
| RPL10 A | ribosomal protein L10a | X | X | |
| EIF3E | eukaryotic translation initiation factor 3, subunit E | | X | |
| EEF2 | eukaryotic translation elongation factor 2 | X | | |
| RPL23 | ribosomal protein L23 | X | X | |
| RPS7 | ribosomal protein S7 | X | X | |
| RPL6 | ribosomal protein L6 | X | X | X |
| RPL15 | ribosomal protein L15 | X | X | X |
| RPS23 | ribosomal protein S23 | X | X | |
| RPS8 | ribosomal protein S8 | X | X | X |
| BTF3 | basic transcription factor 3 | | | X |
| RPS24 | ribosomal protein S24 | X | X | X |
| RPS4X | ribosomal protein S4, X-linked | X | X | X |
| RPS15A | ribosomal protein S15a | X | | X |
| RPS25 | ribosomal protein S25 | X | X | |
| RPL18 | ribosomal protein L18 | X | X | X |
| GNB2L1 | guanine nucleotide binding protein (G protein), beta polypeptide 2-like 1 | | | |
| RPS6 | ribosomal protein S6 | X | X | |
| EIF3EIP | eukaryotic translation initiation factor 3, subunit L | | | |
| RPS12 | ribosomal protein S12 | X | X | |

Table 164 does not include all ontological classifications for each gene.

Gene Ontology patterns identified within a Cancer Module can be used to define a cancer signature. For example, Bladder Cancer Module 2 can be identified as having a protein biosynthesis signature based on the ontologies of the member genes that define Bladder Cancer Module 2 (Table 164).

Tumor signatures arc useful for predicting cancer sensitivity to certain classes of drugs. For example, cancers classified in Bladder Cancer Module 2 can be predicted to be sensitive to translation inhibitors such as tedanolides and related molecules. Sensitivity of cancers belonging to a Cancer Module with a known cancer signature to a certain drug class can be confirmed using *in vitro* and *in vivo* experiments. In some embodiments, cancer sensitivity can be confirmed using retroactive studies on cancer samples from patients treated with known classes of drugs.

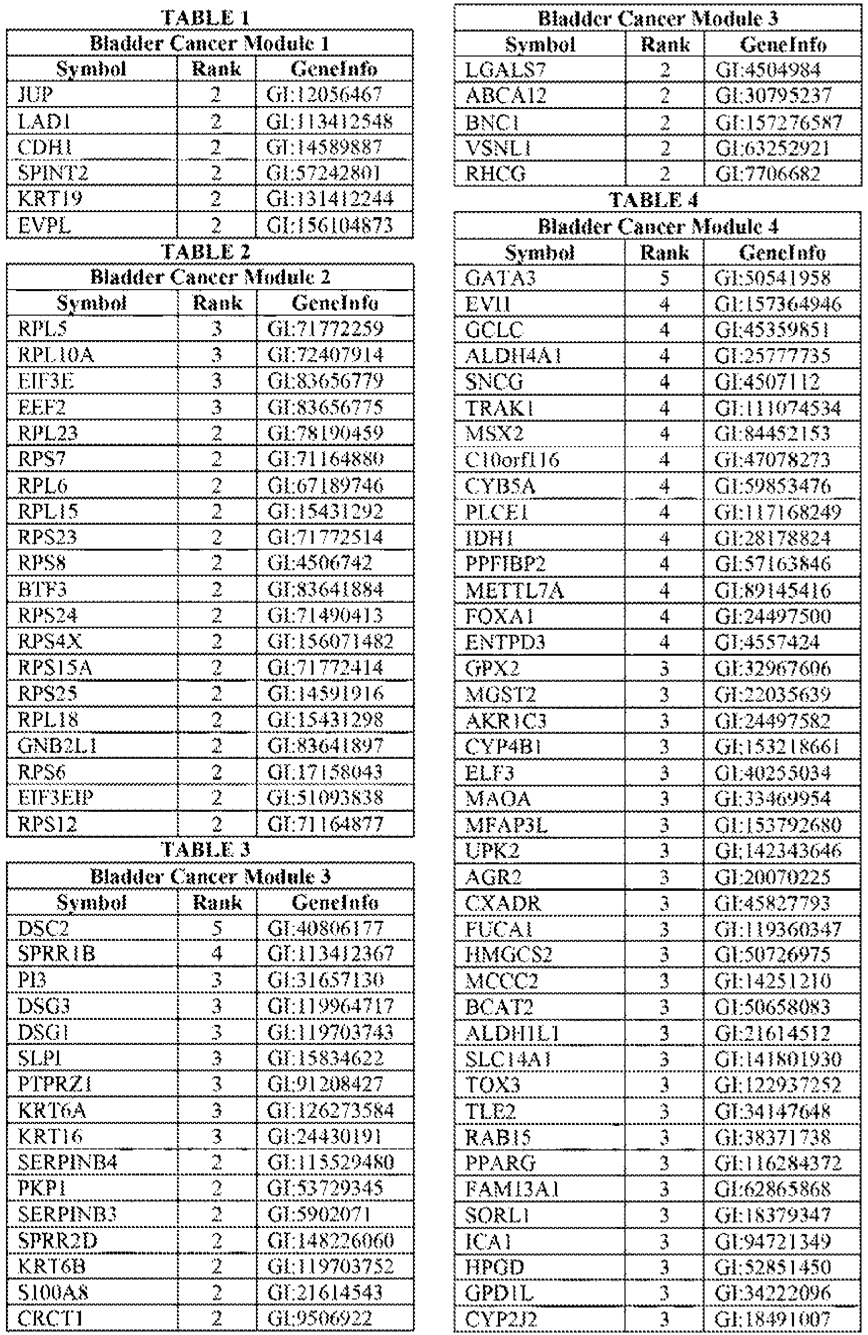

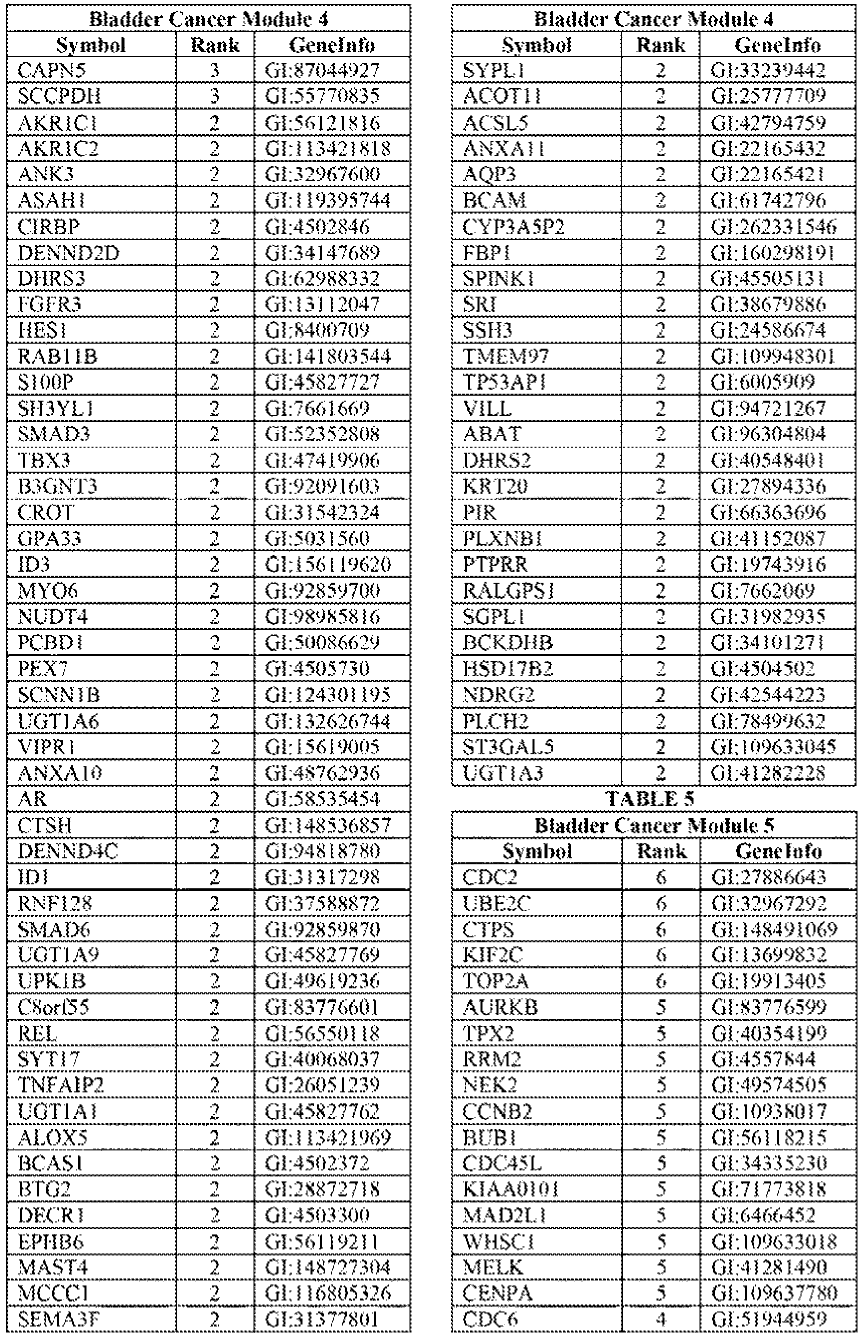

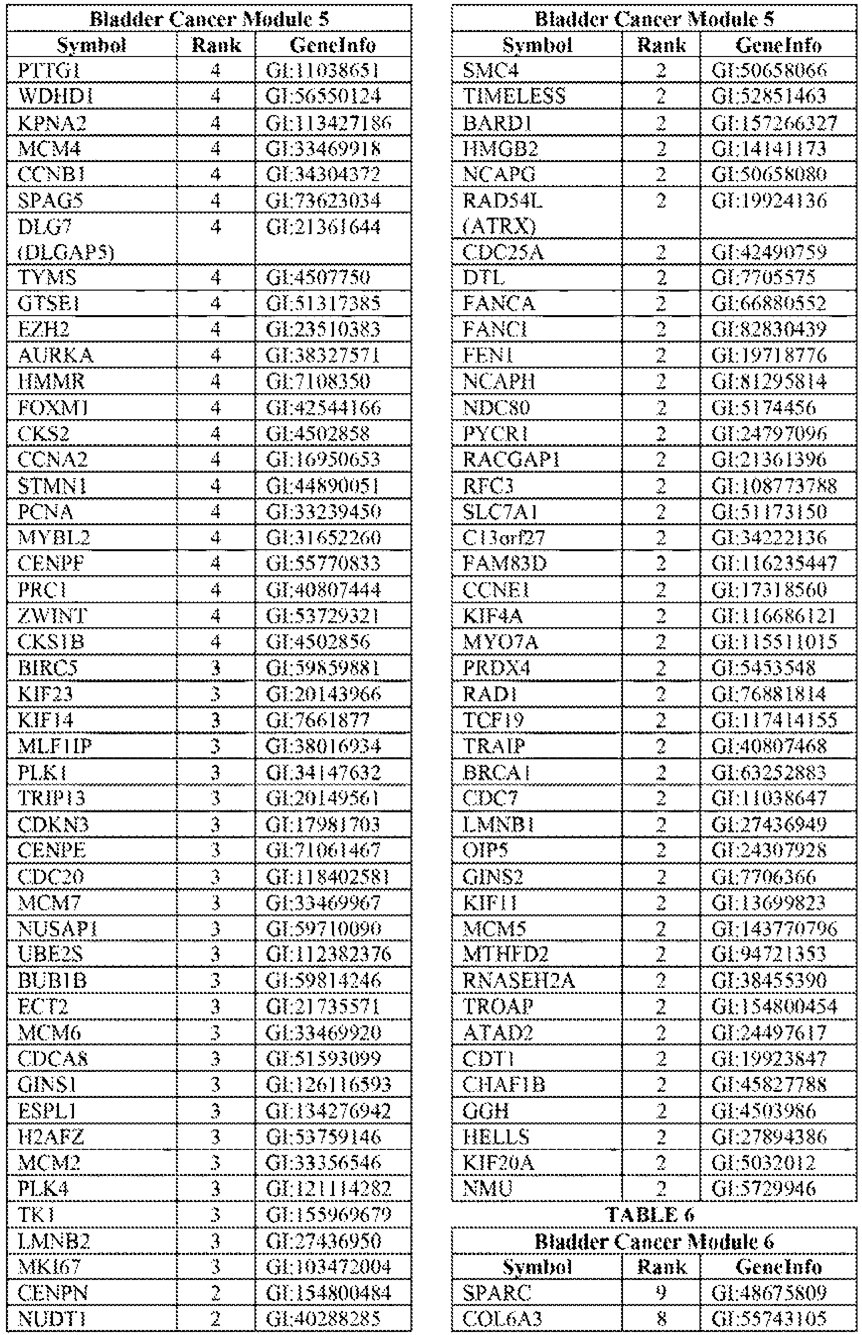

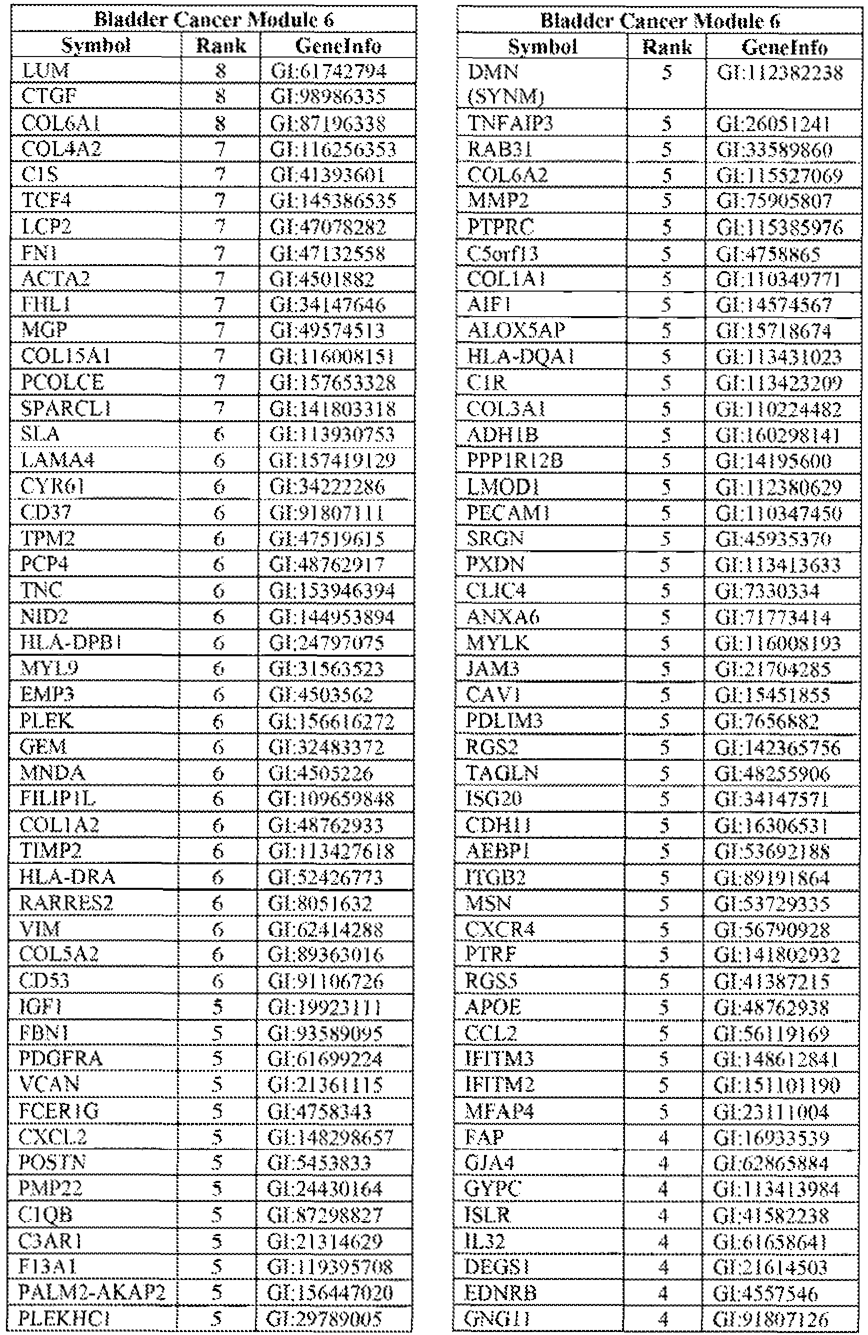

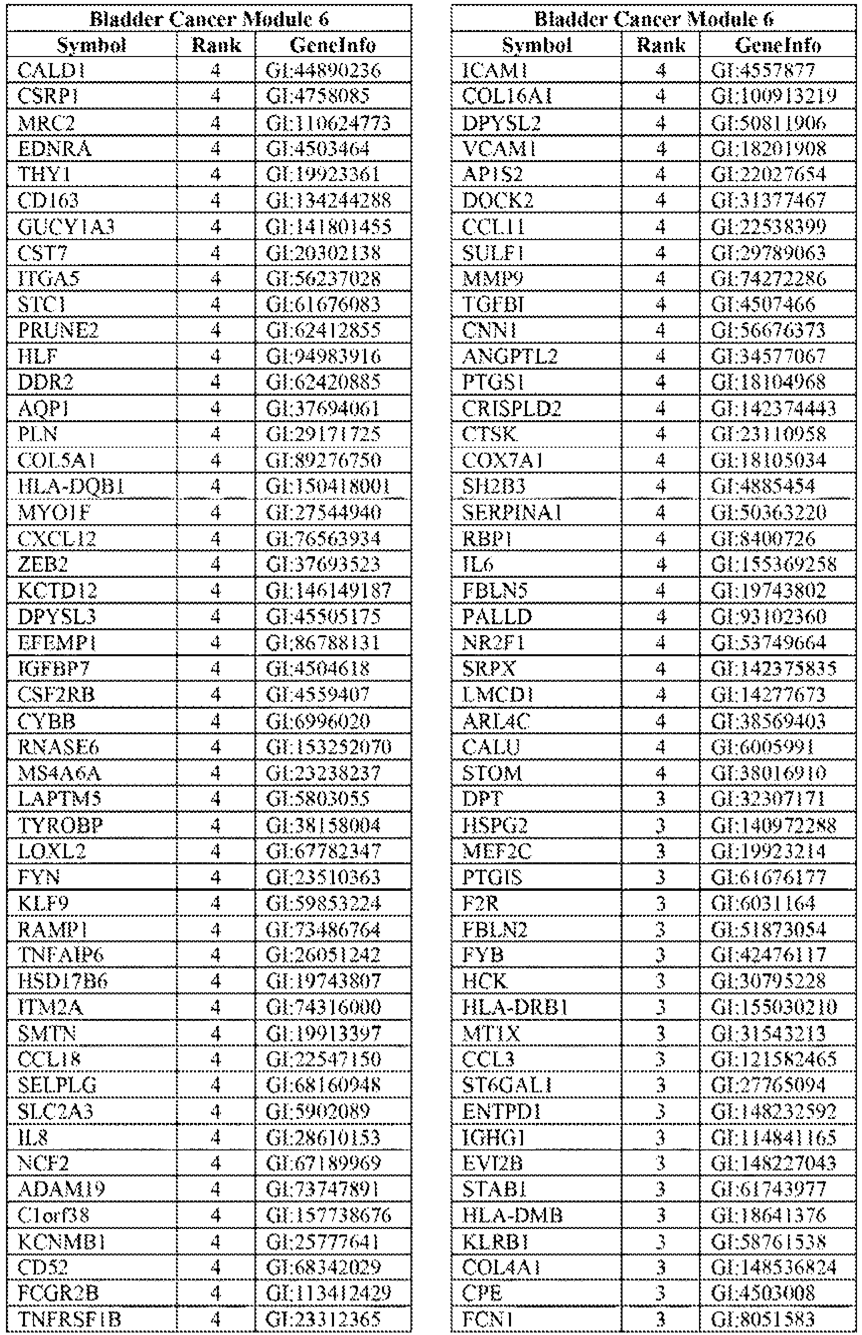

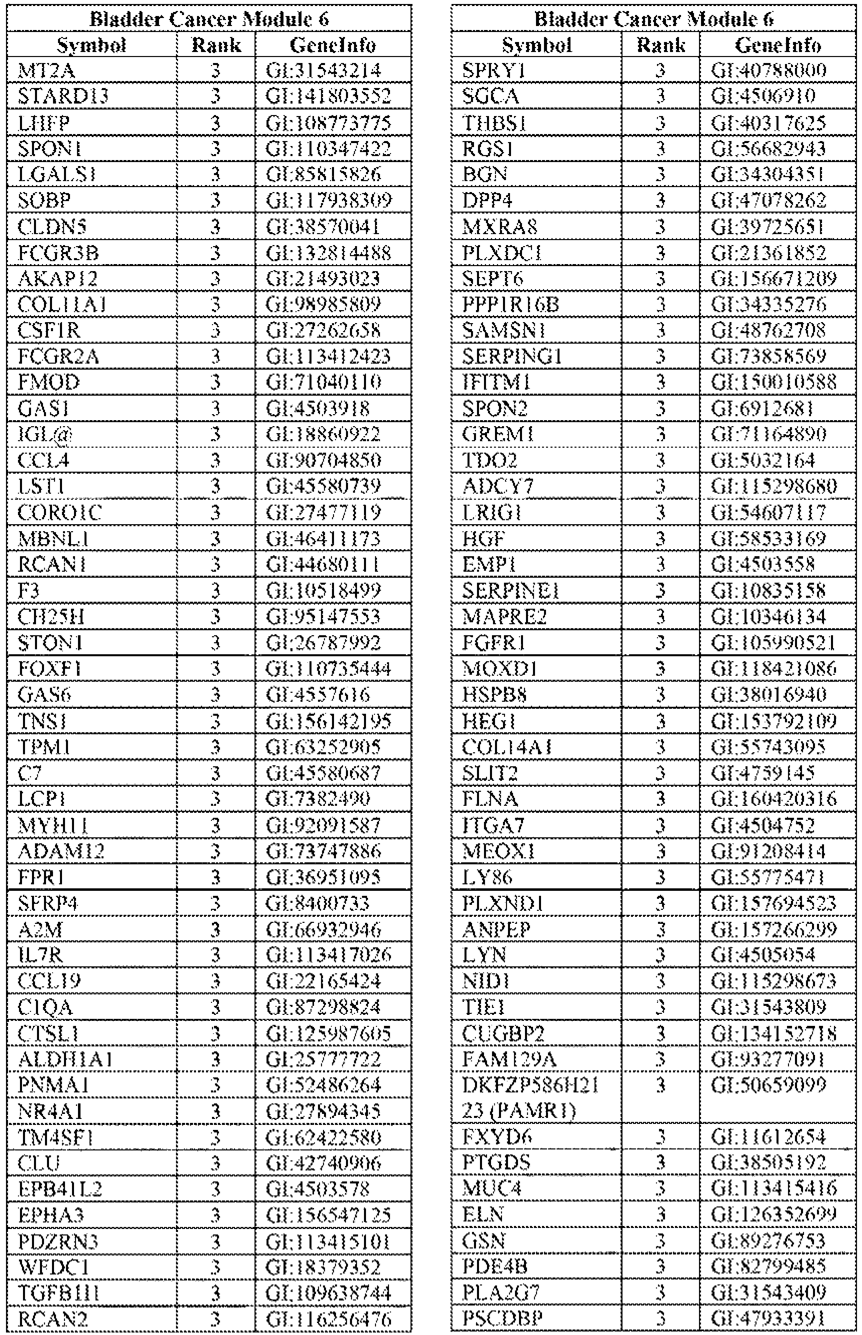

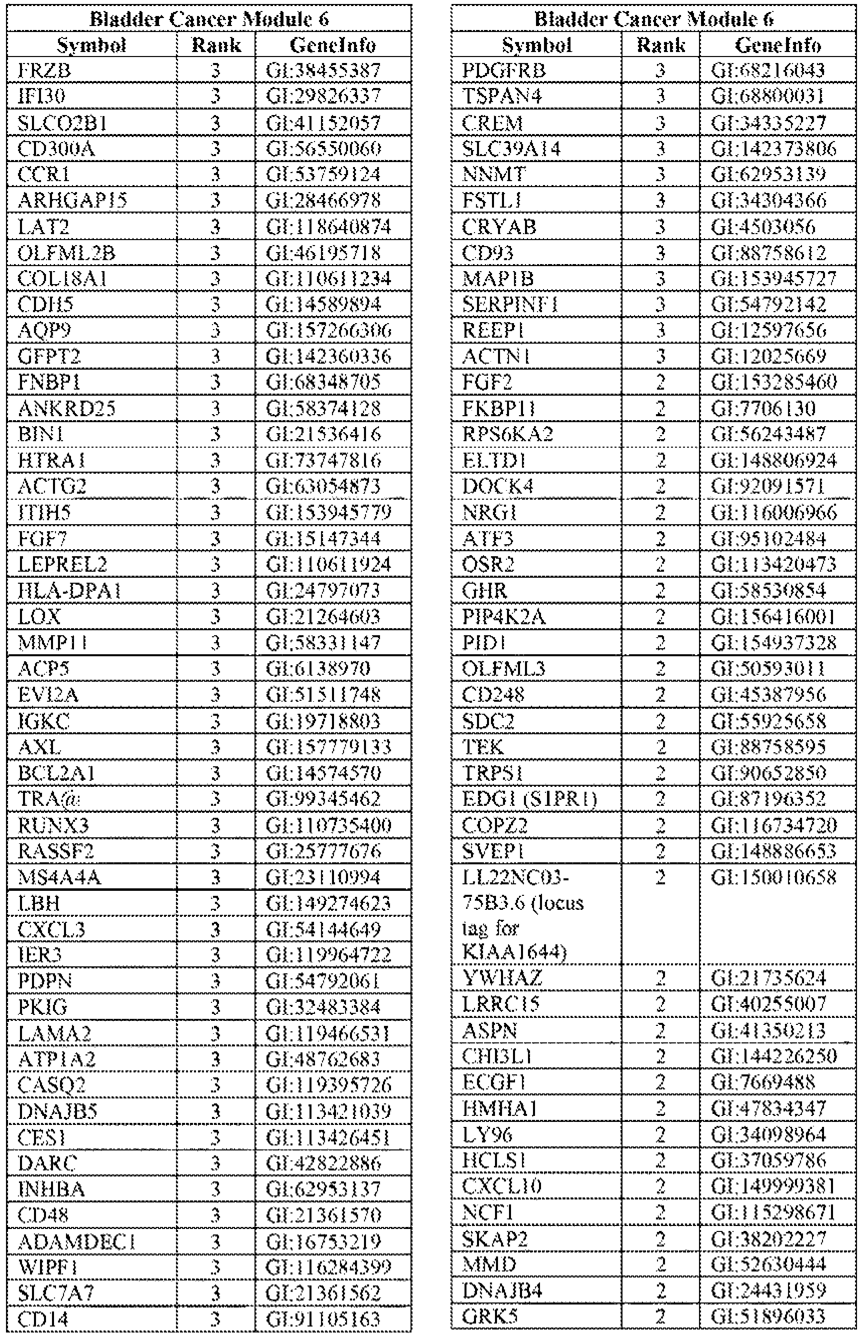

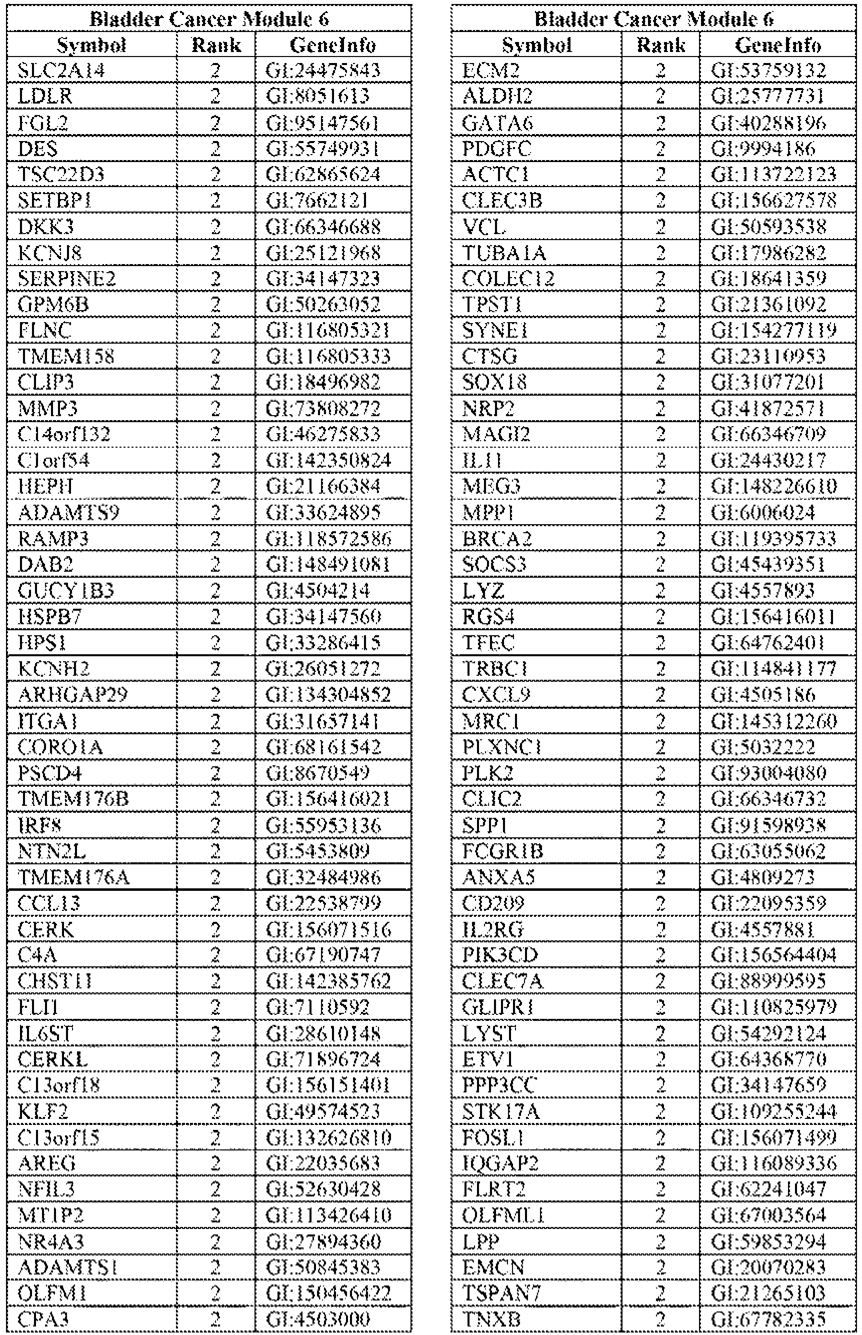

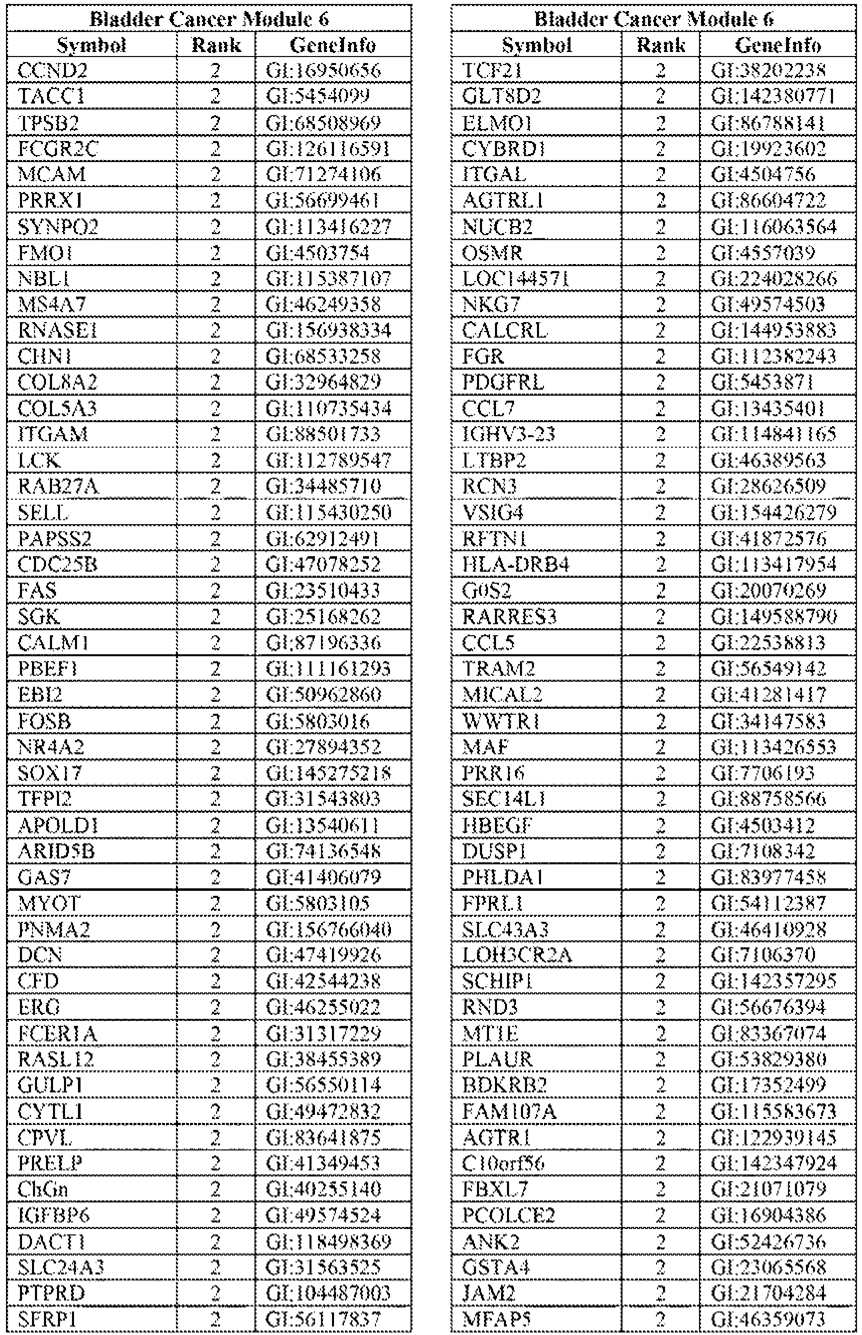

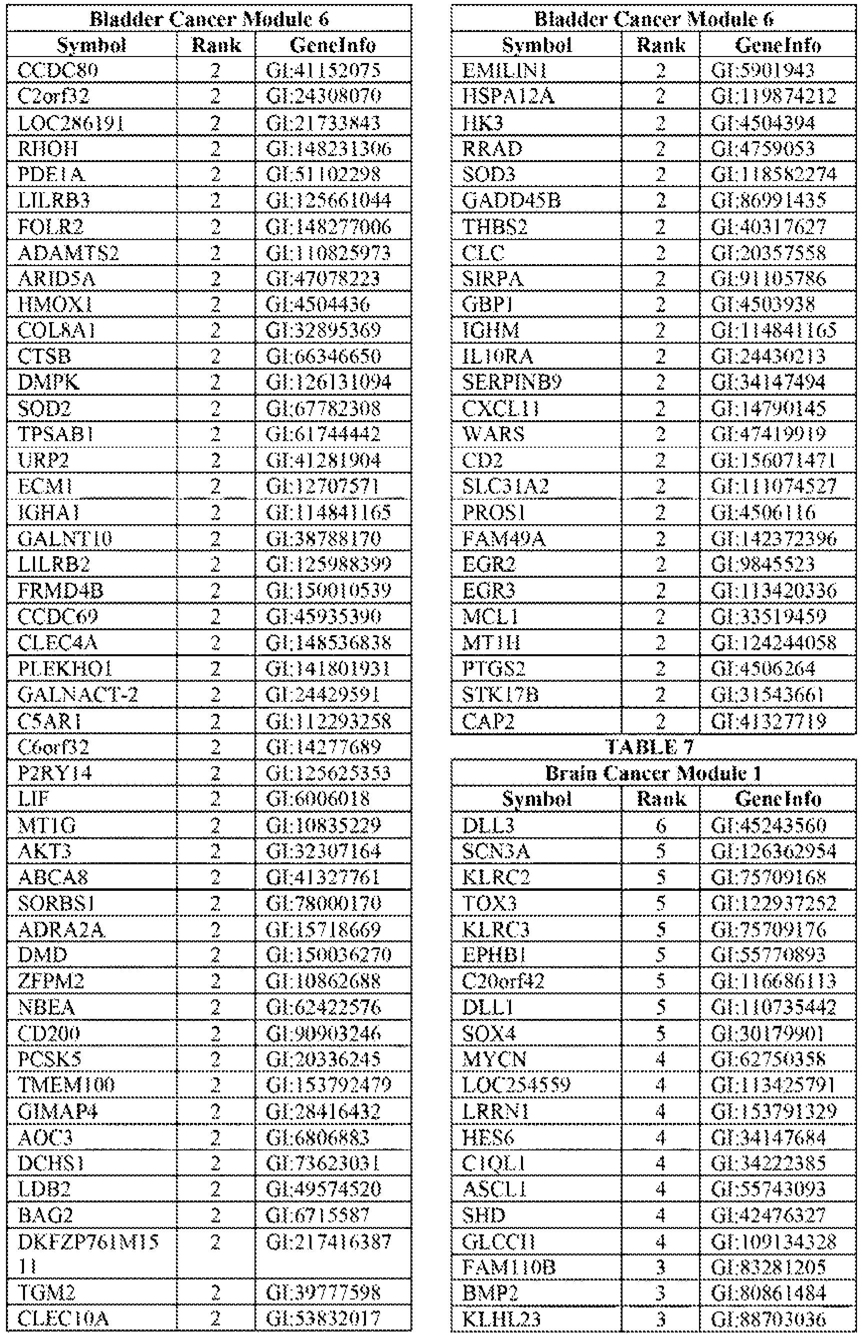

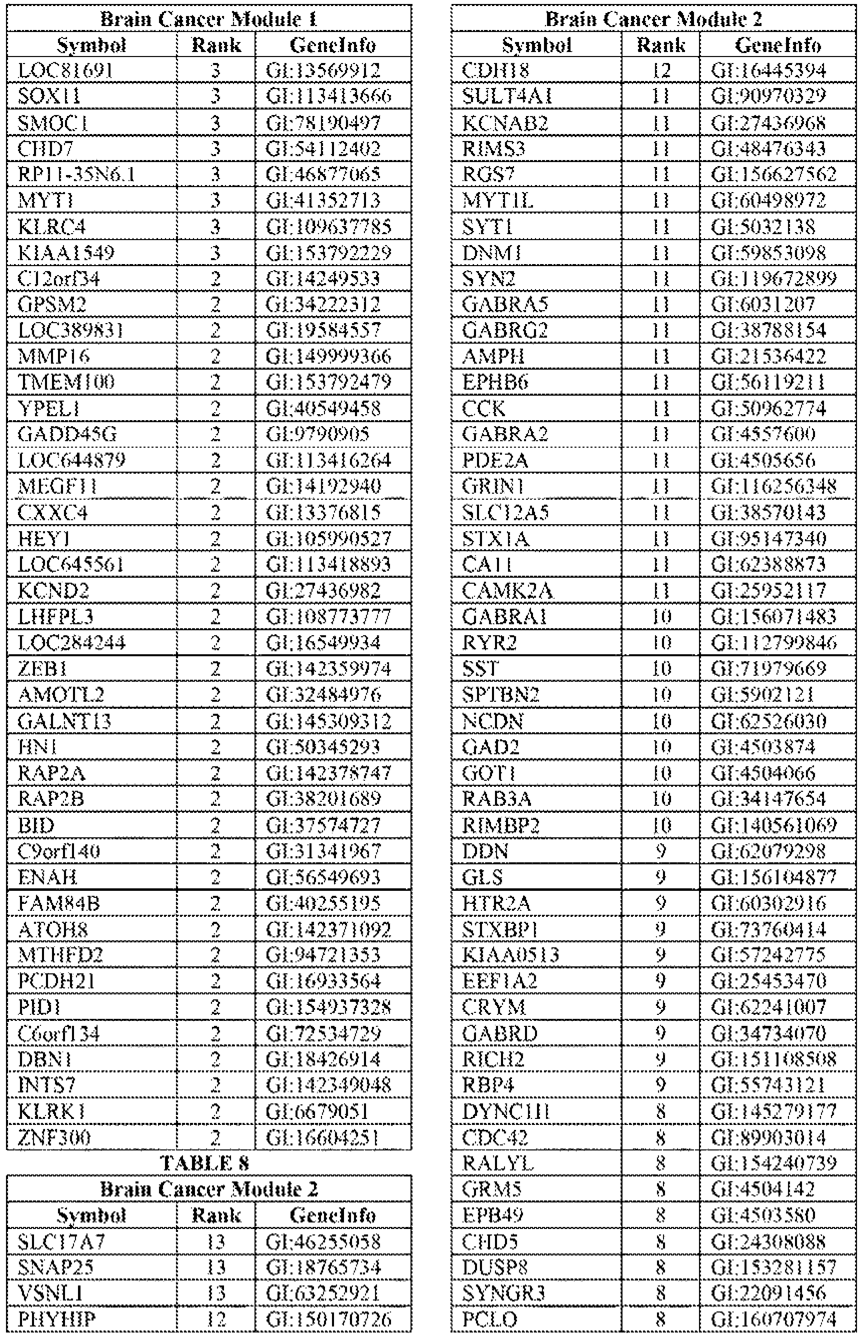

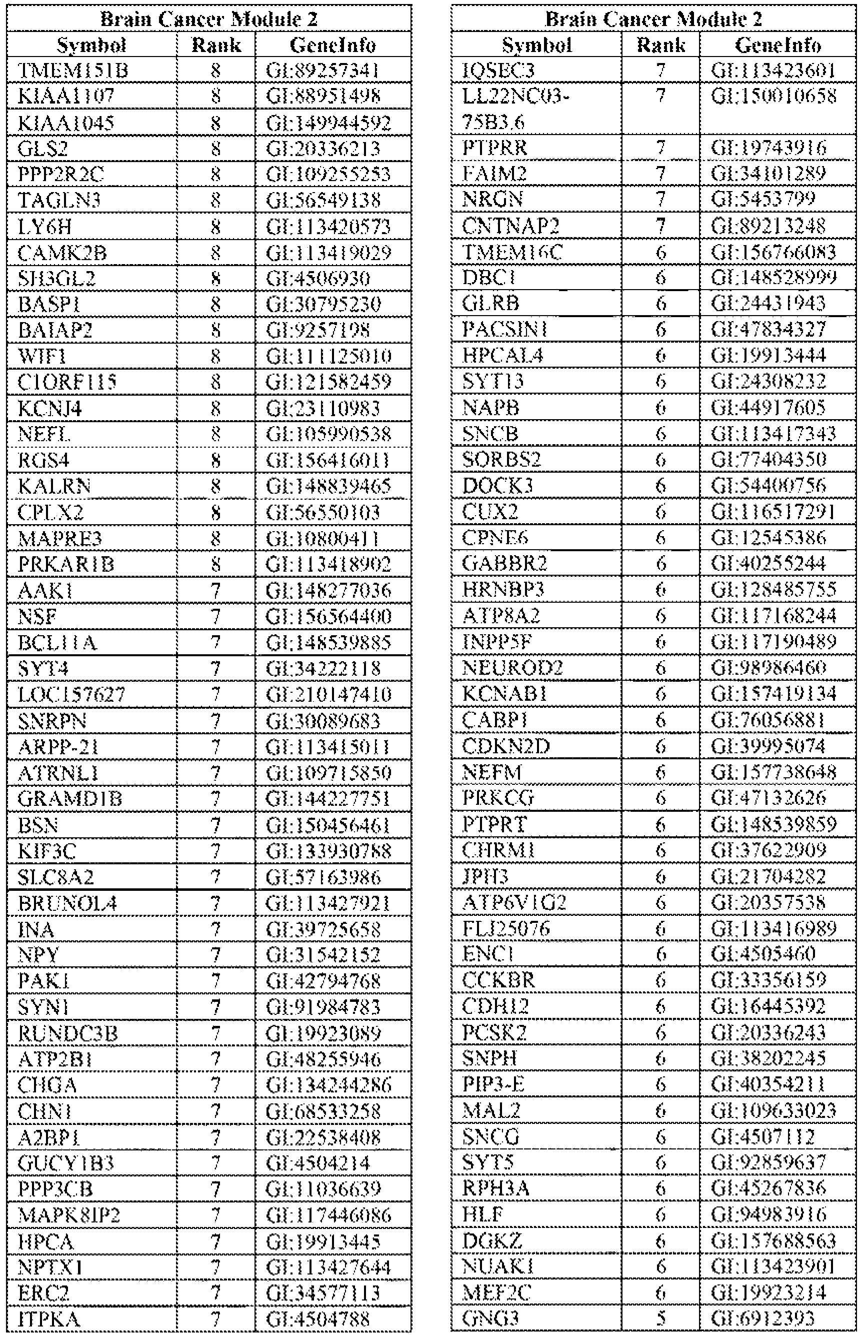

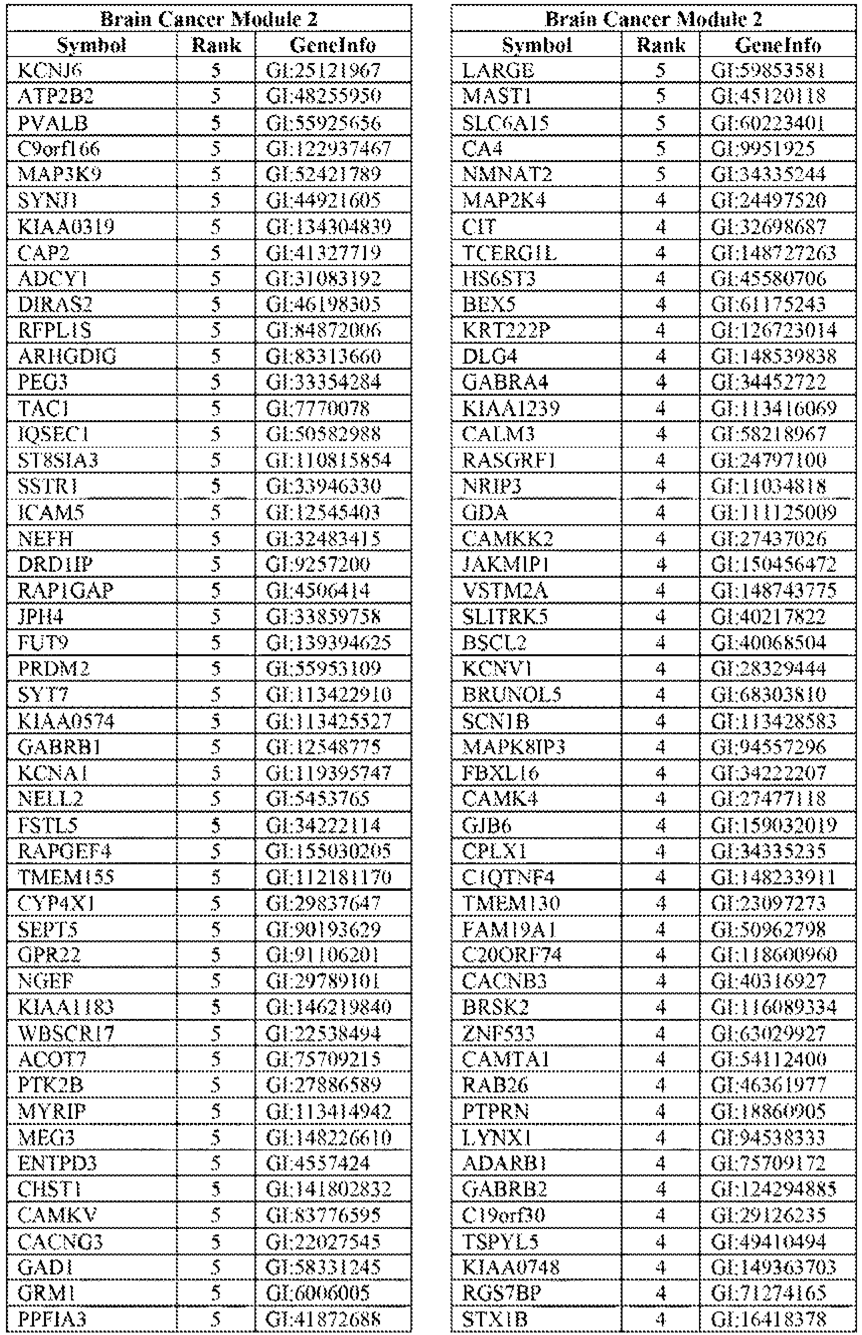

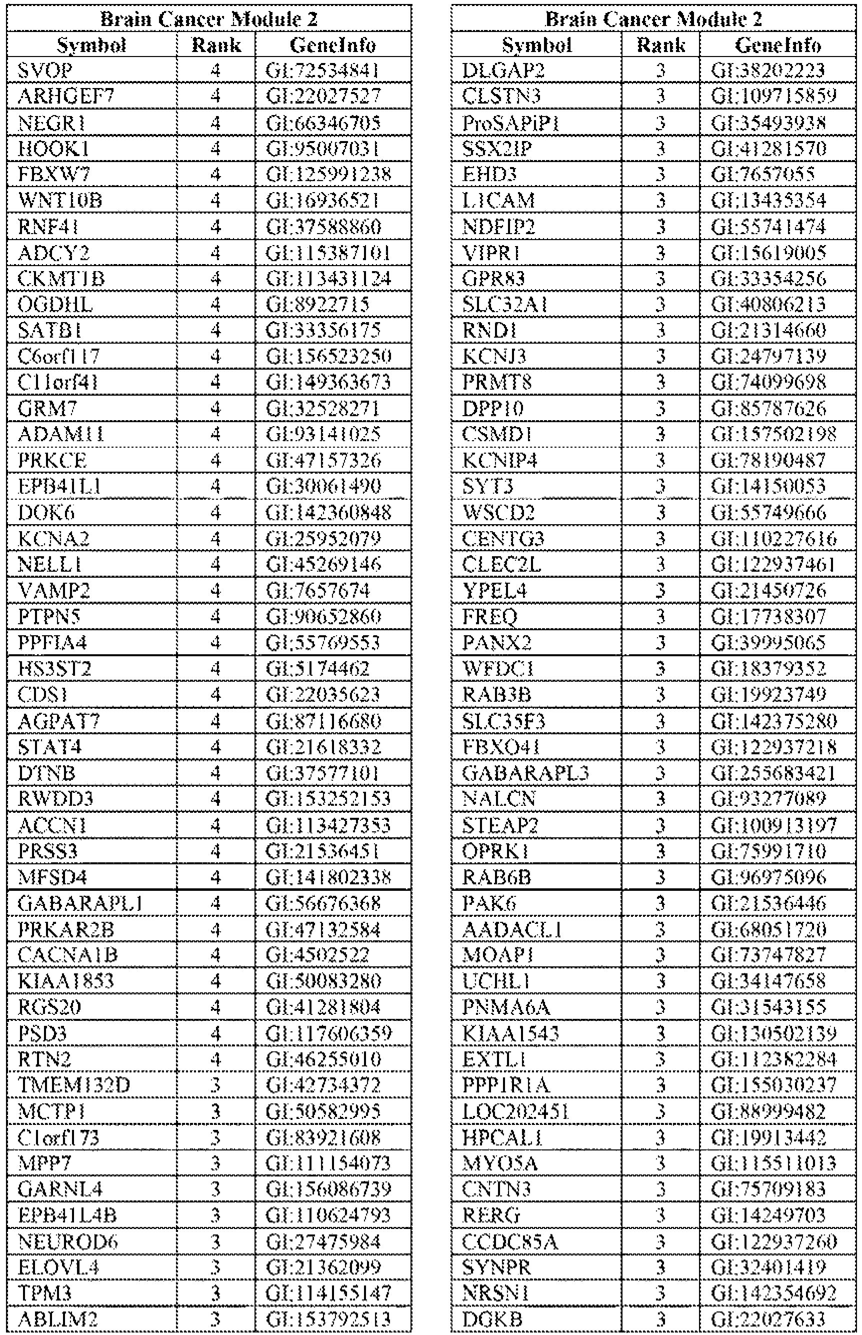

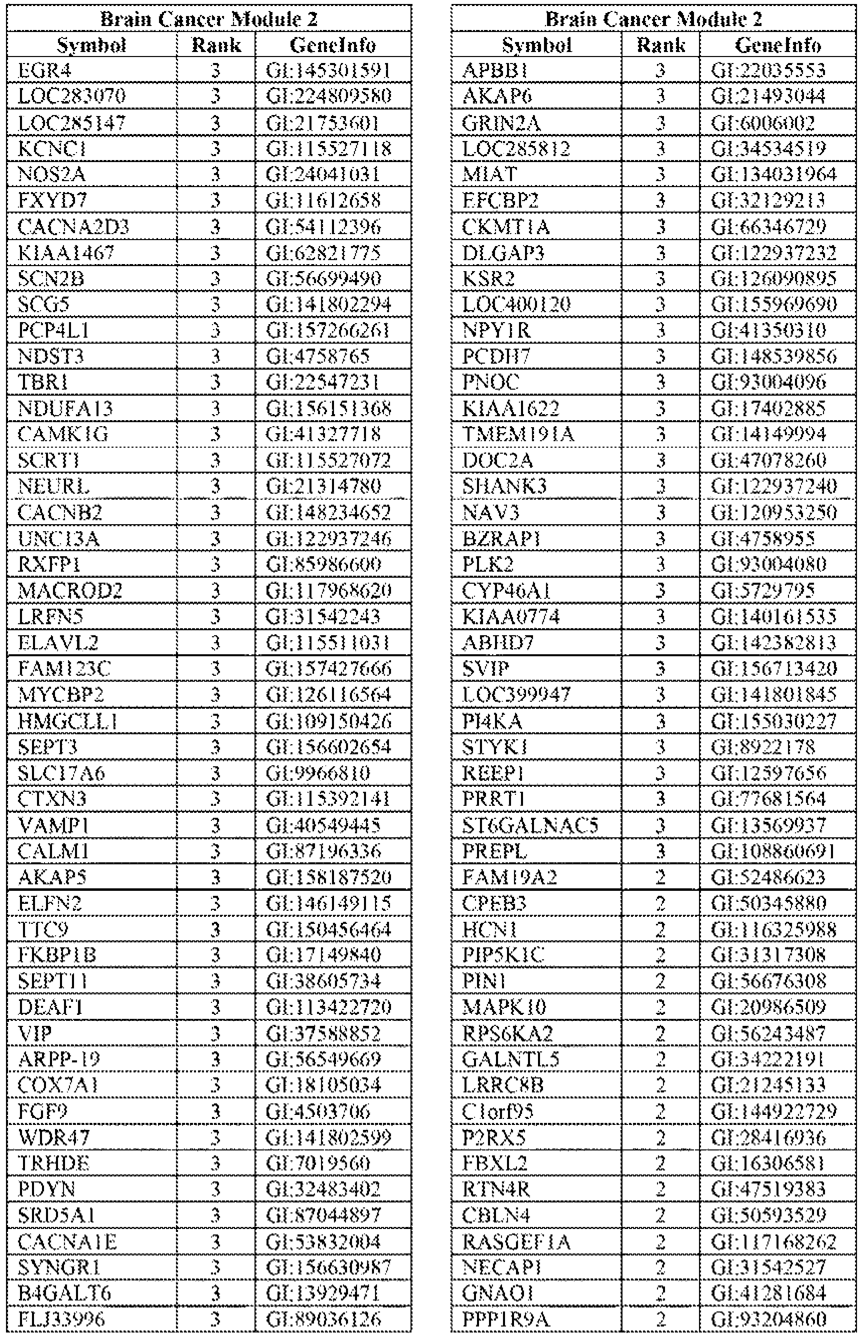

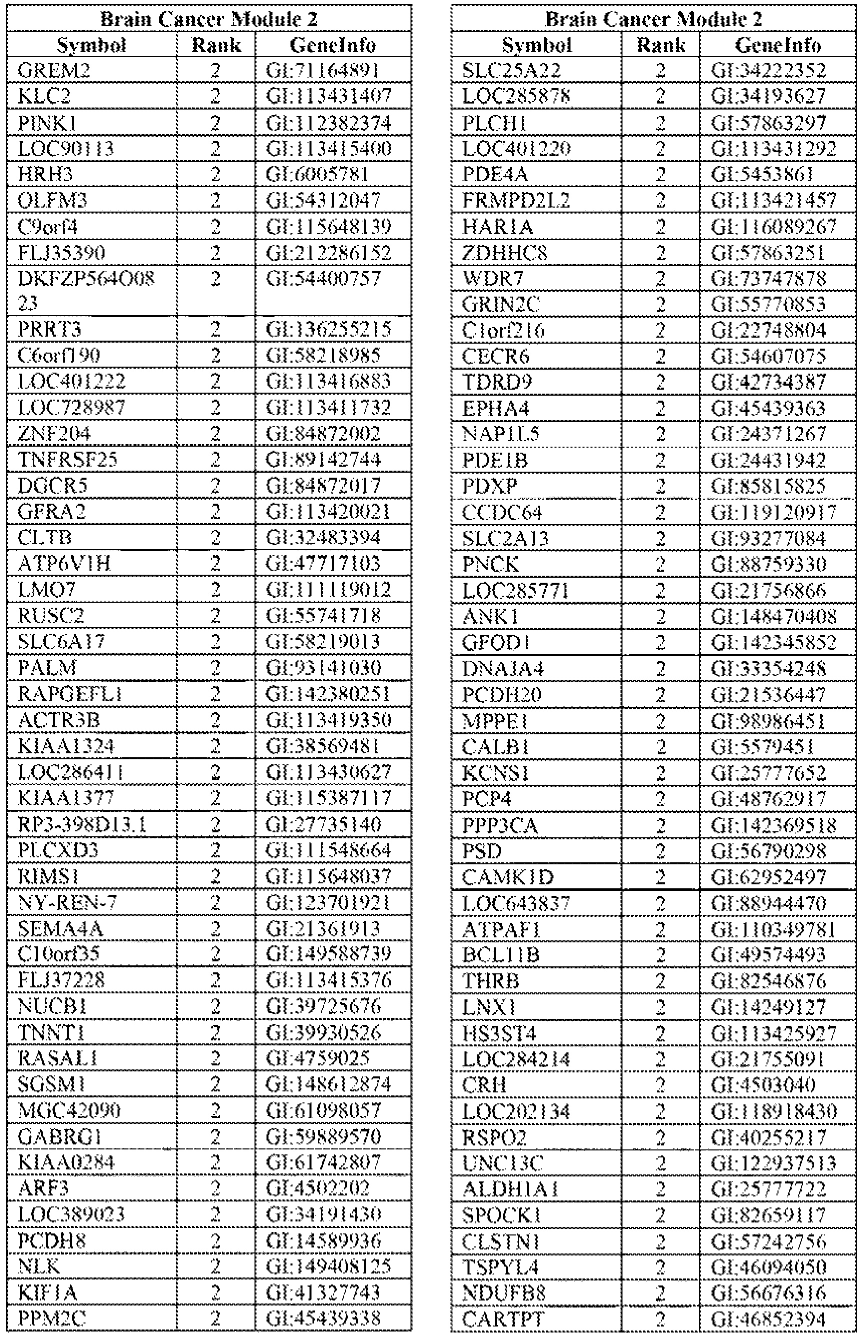

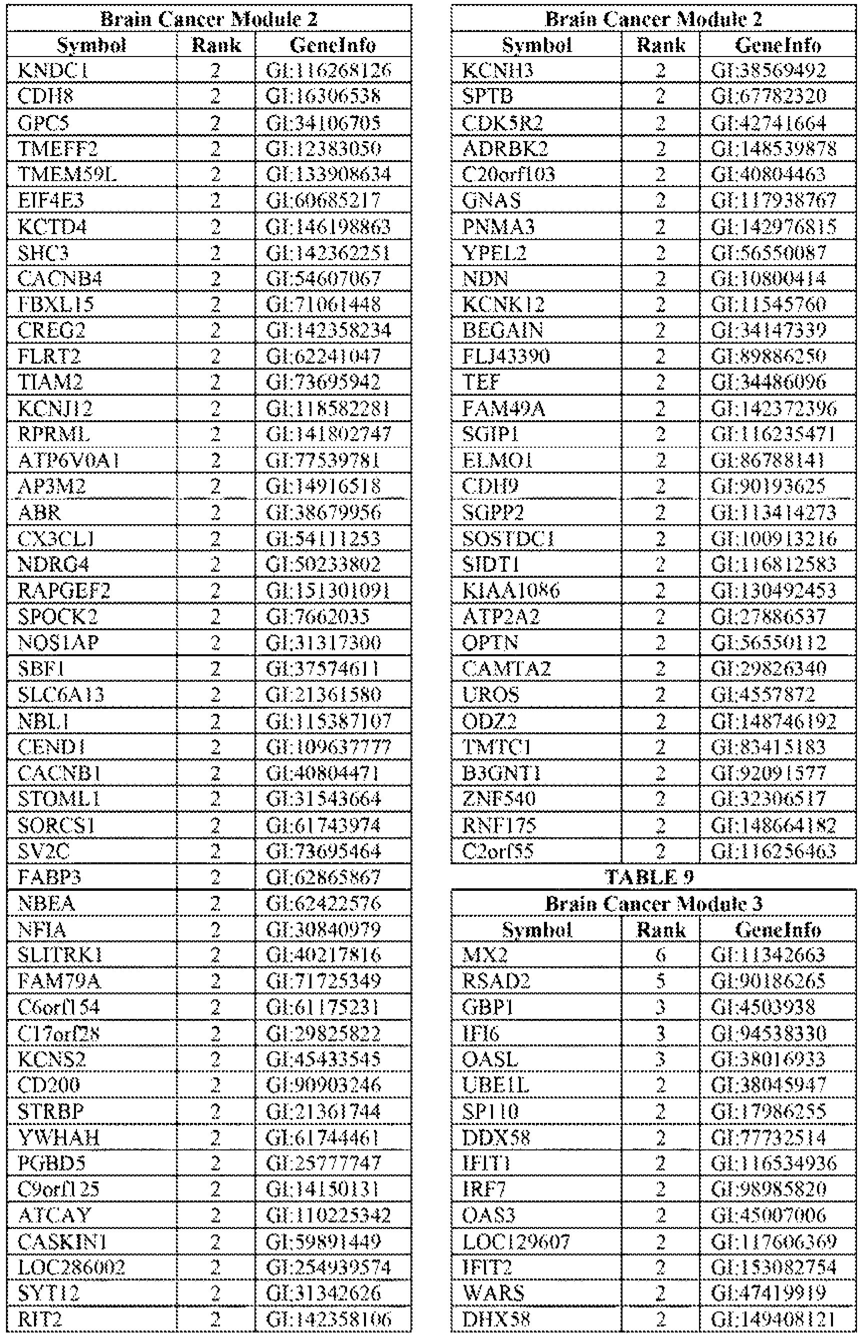

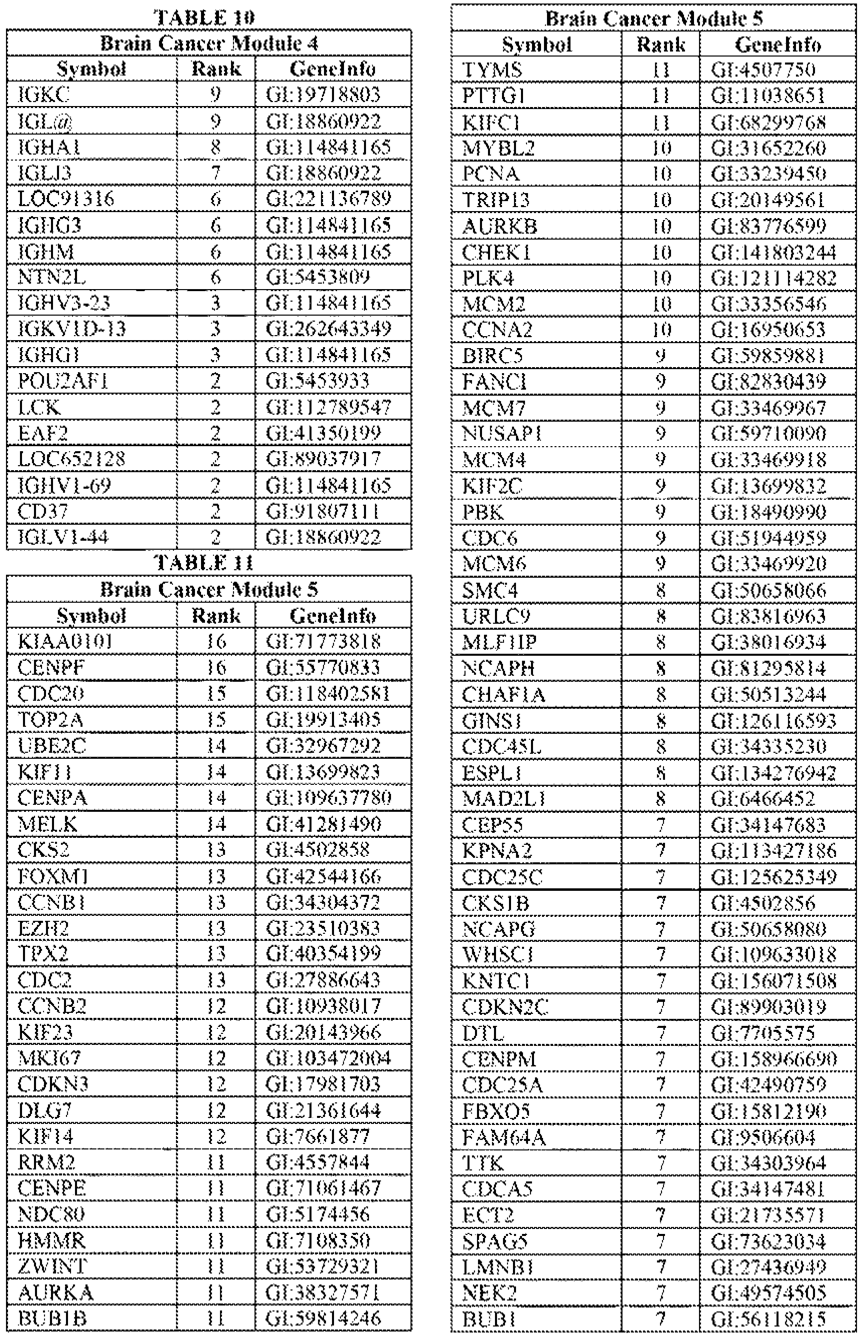

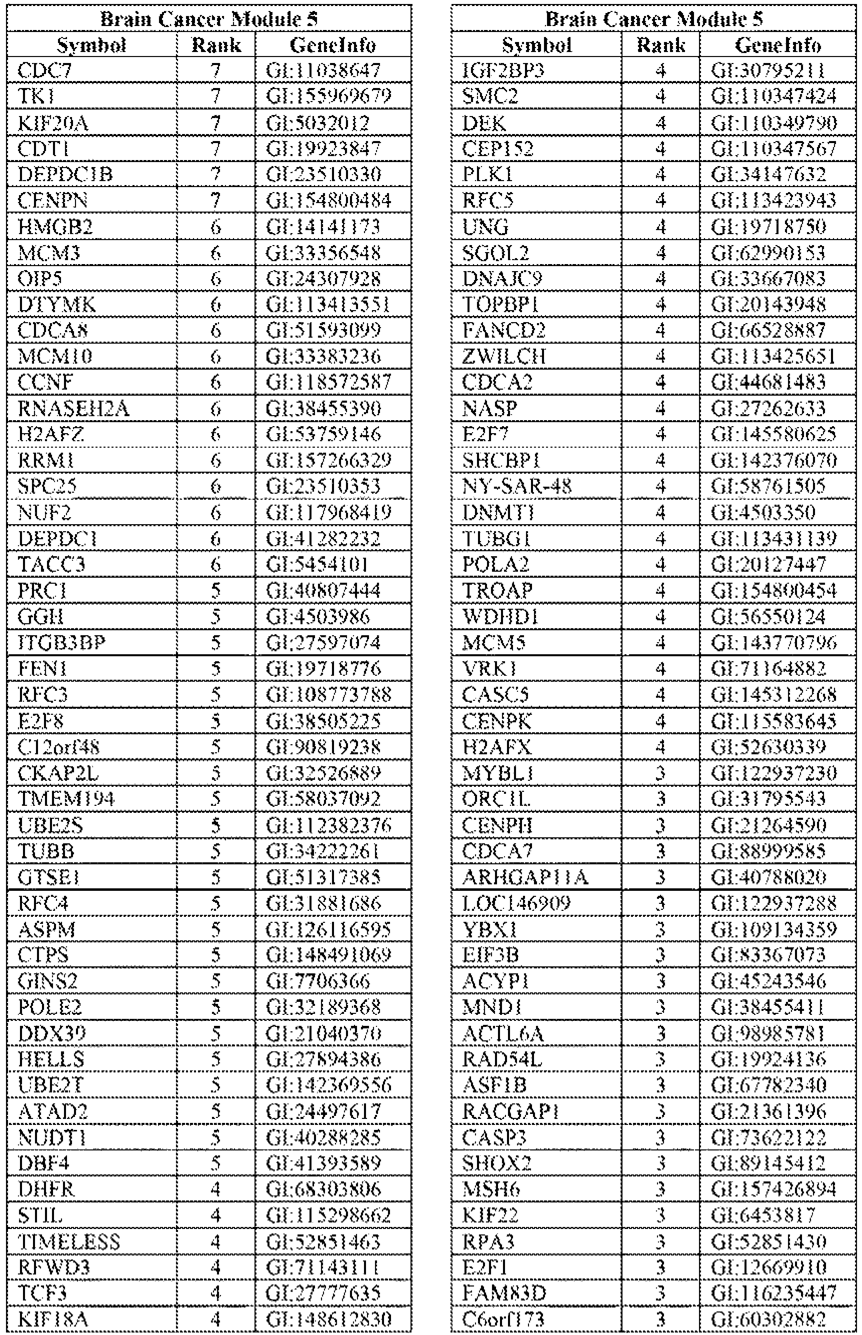

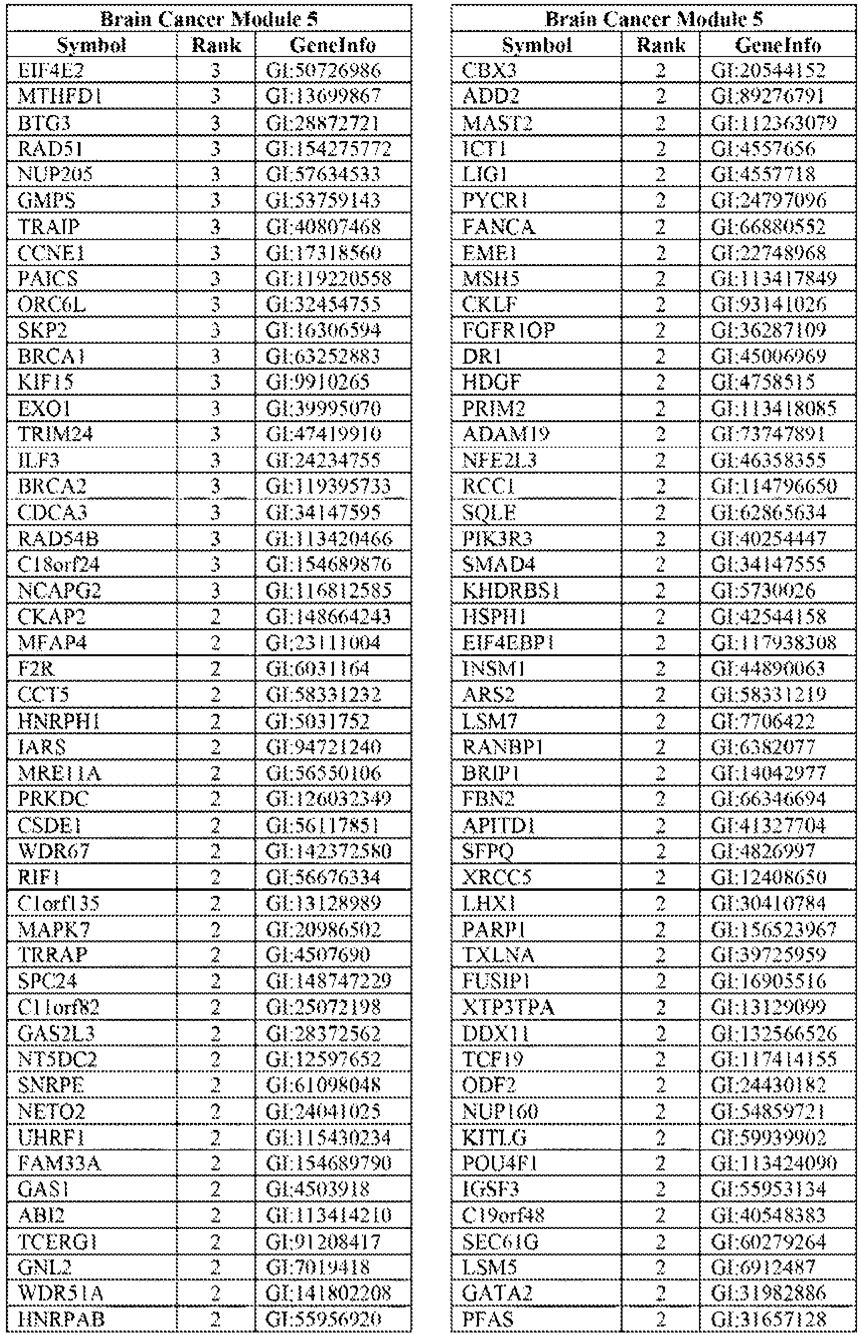

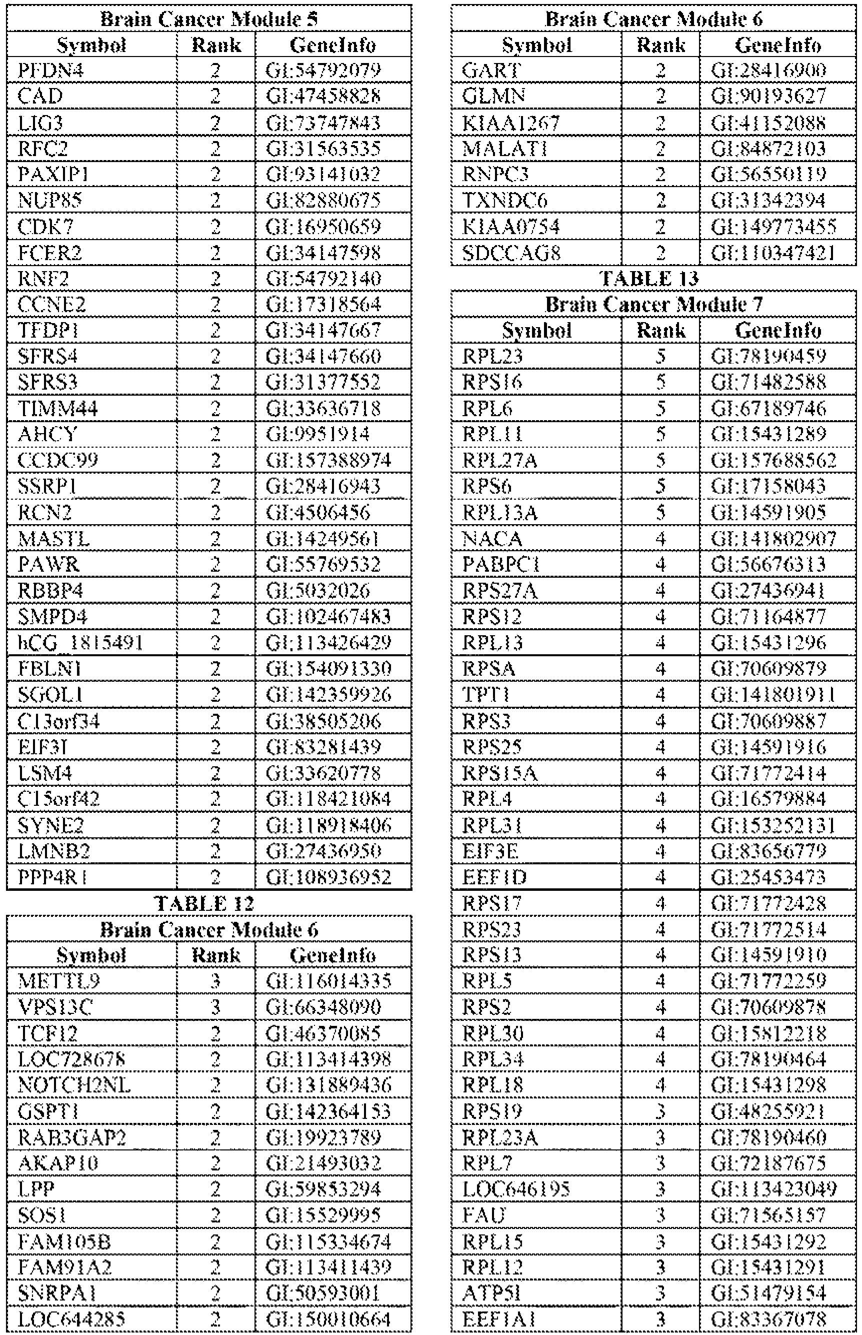

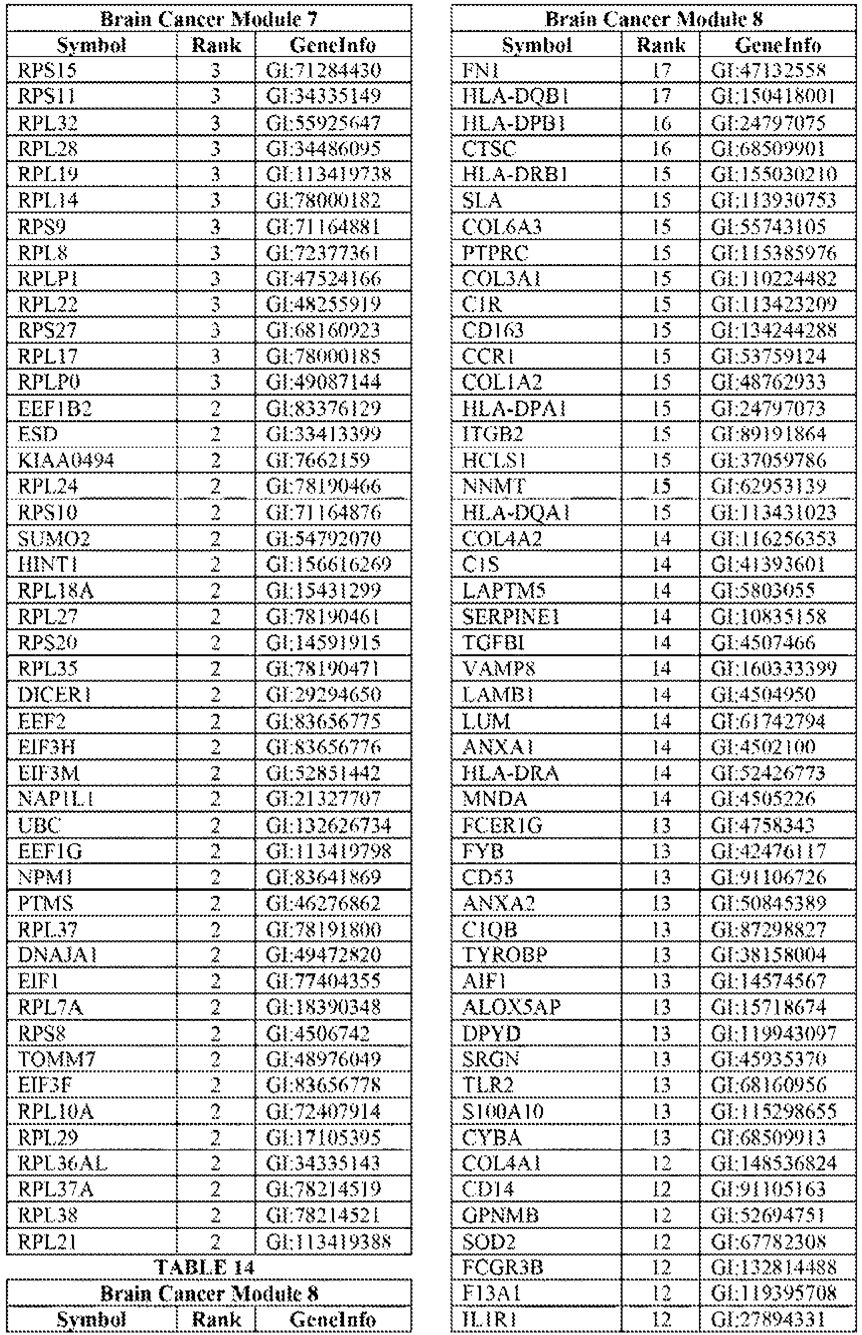

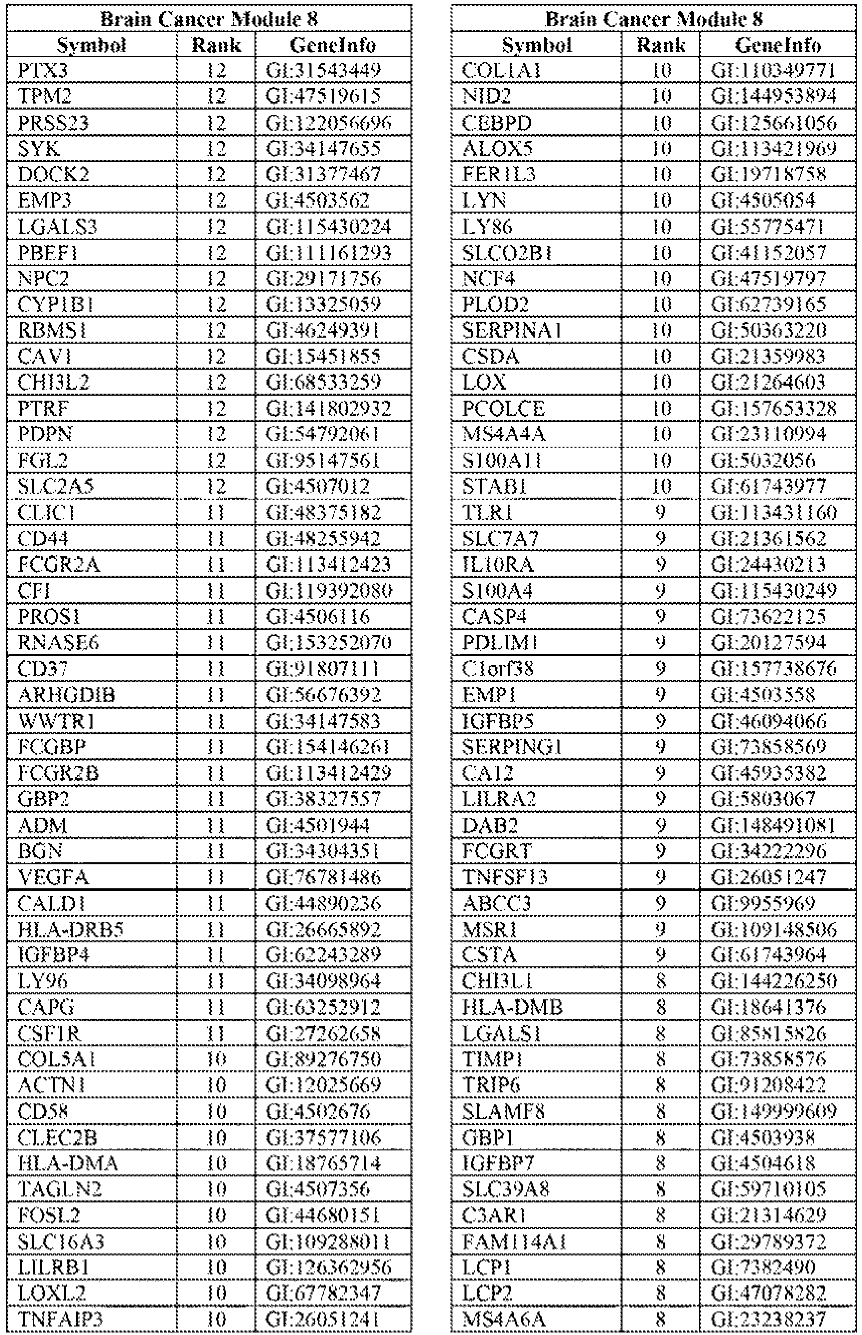

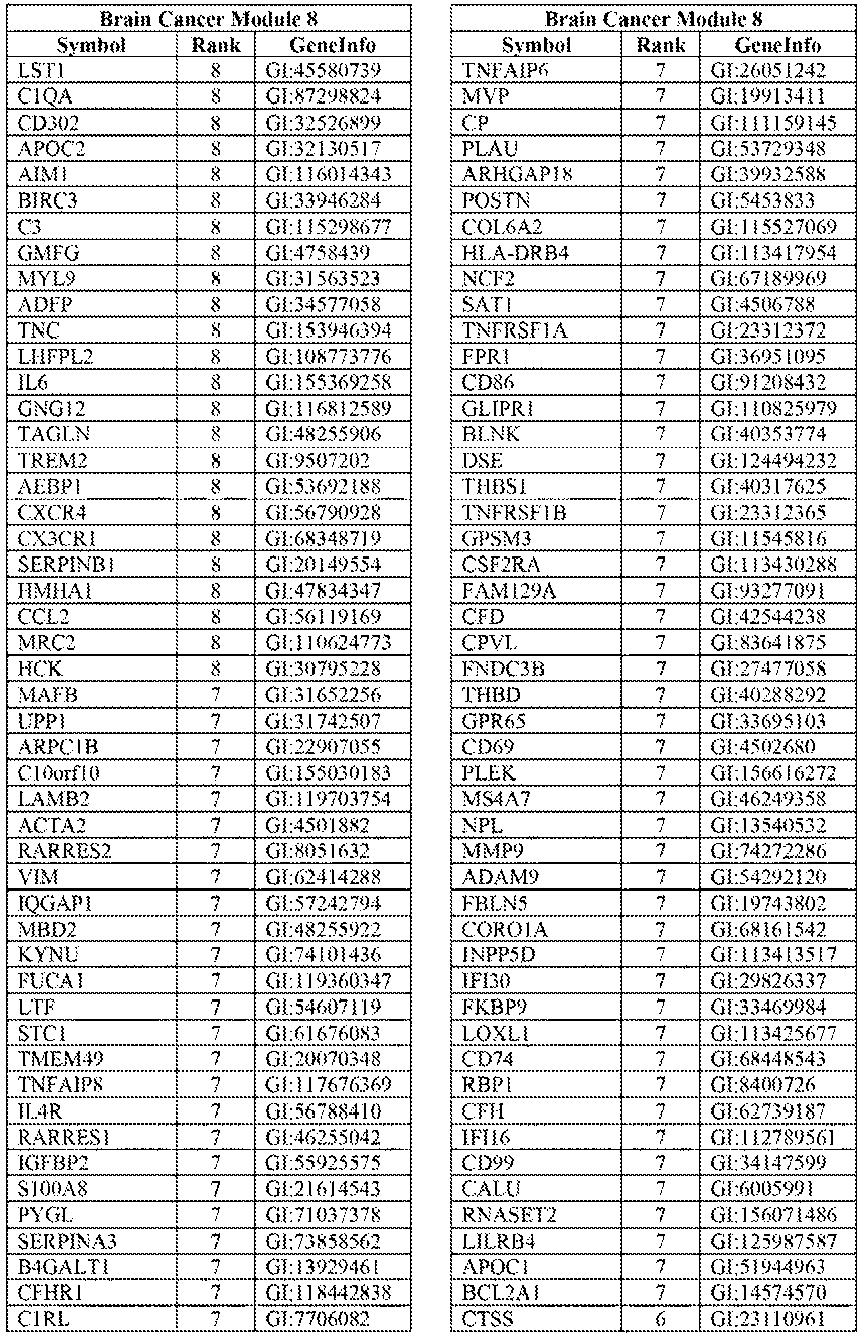

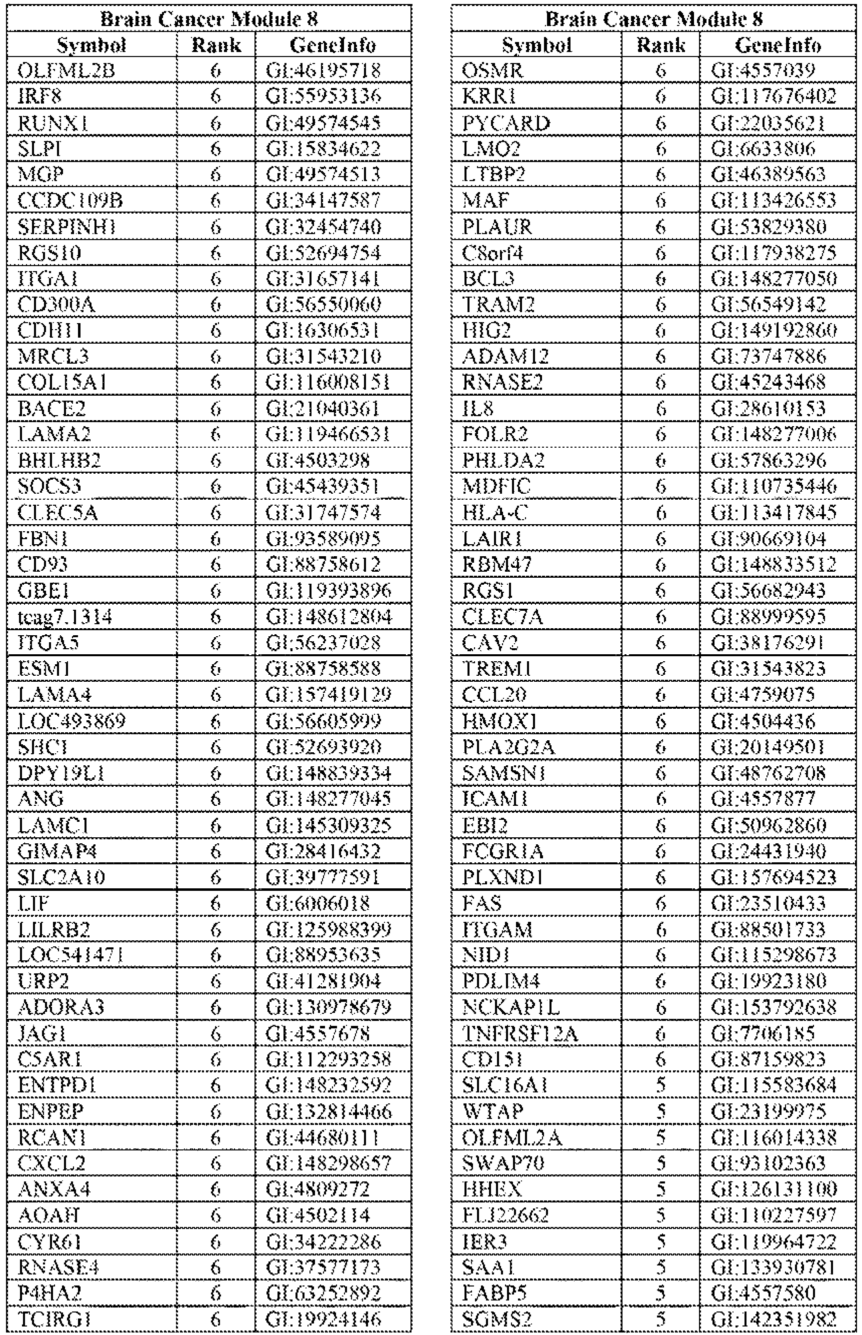

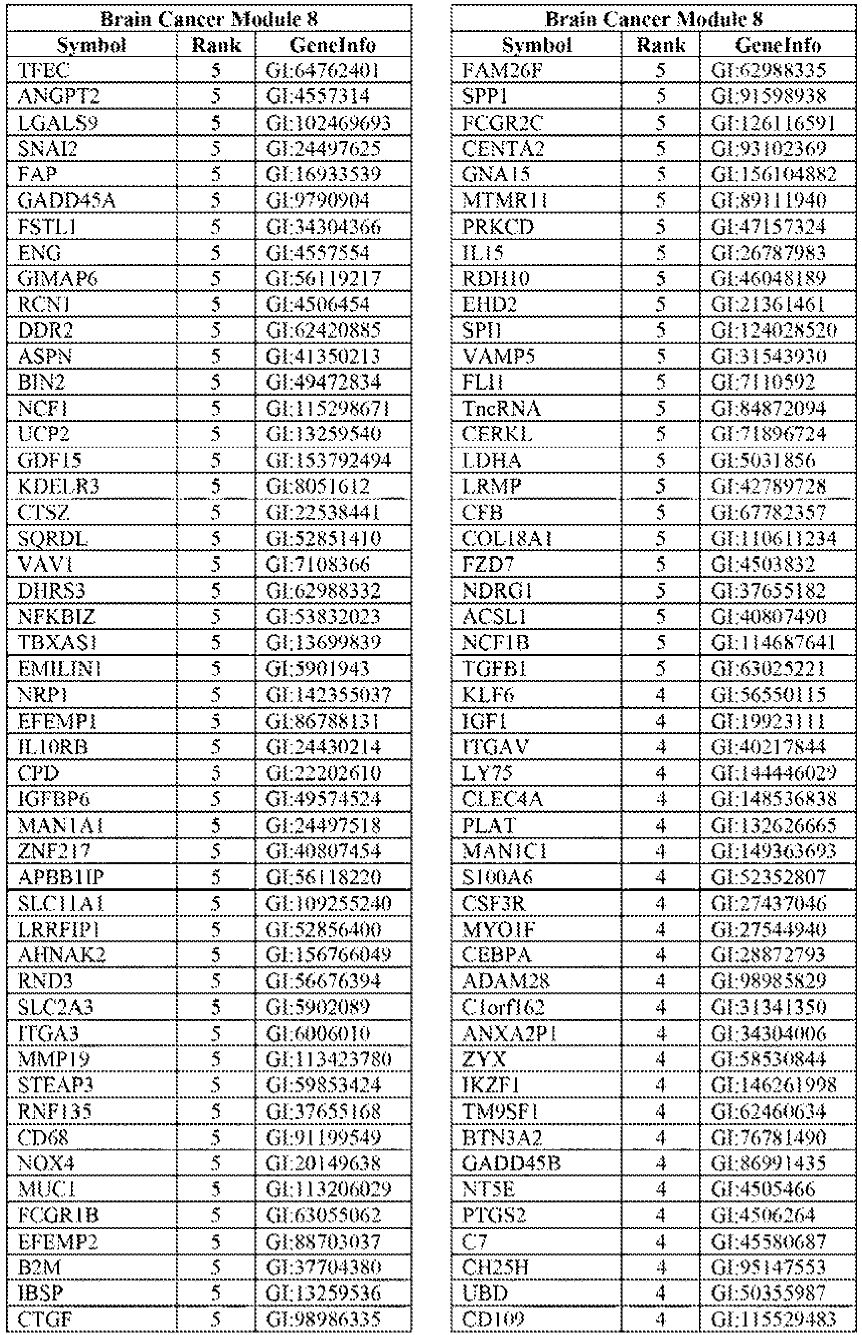

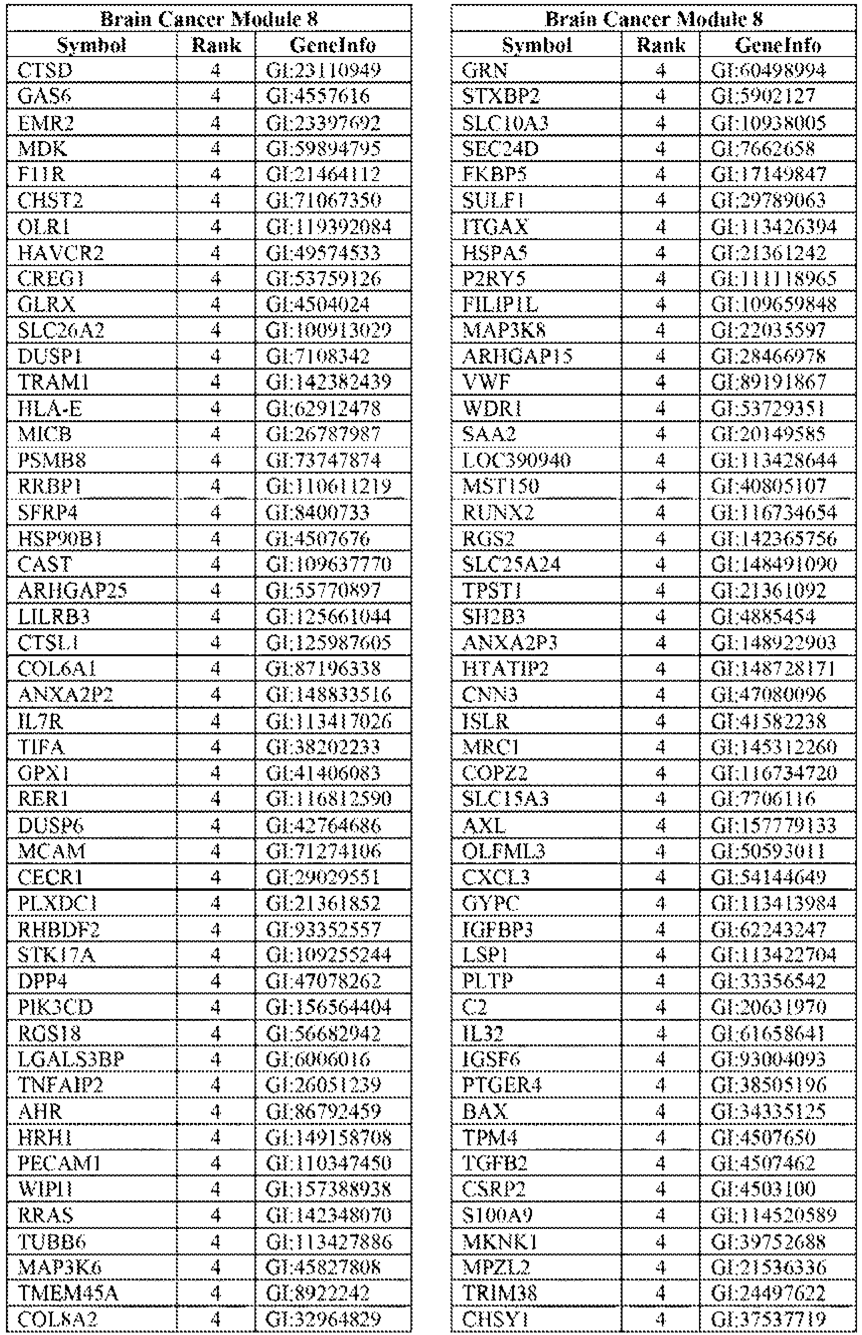

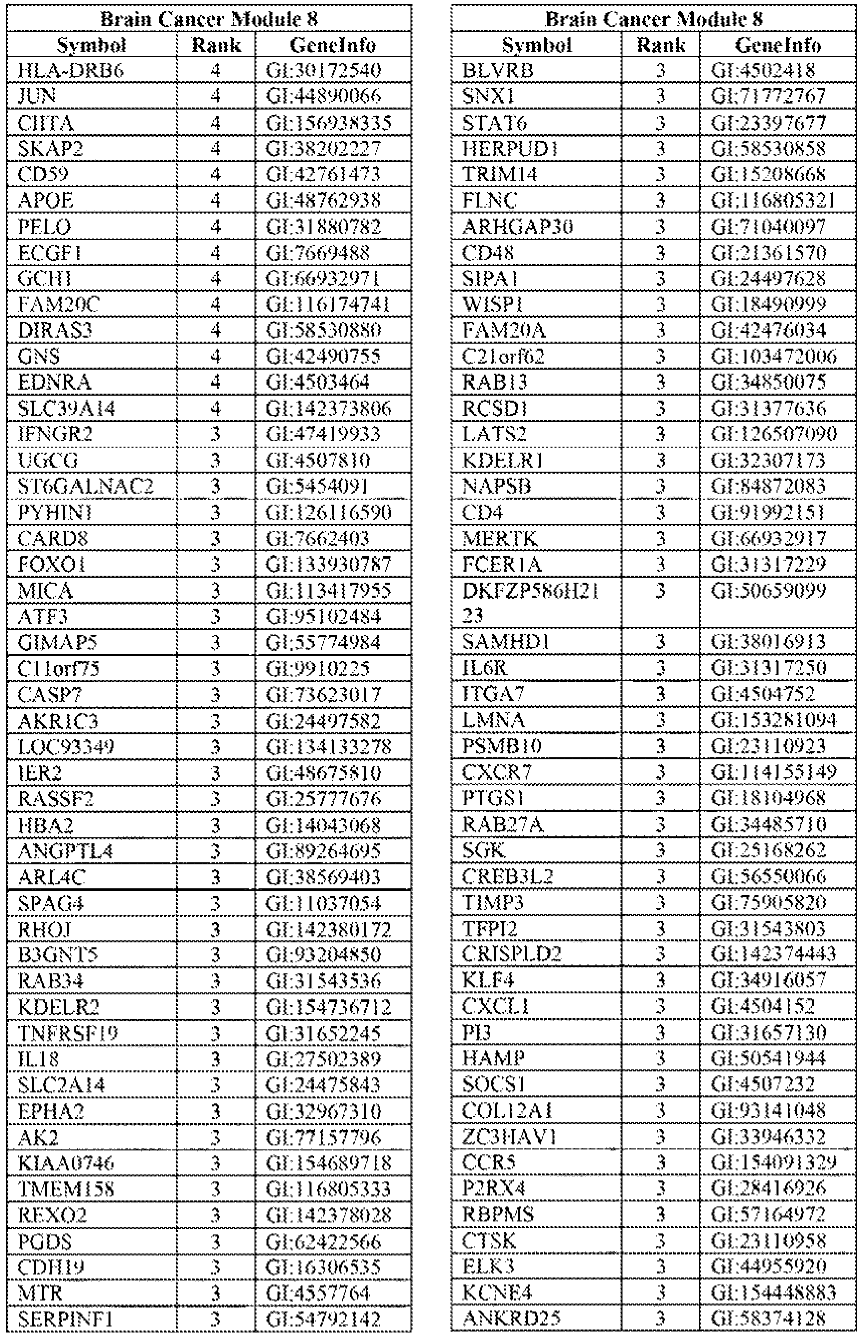

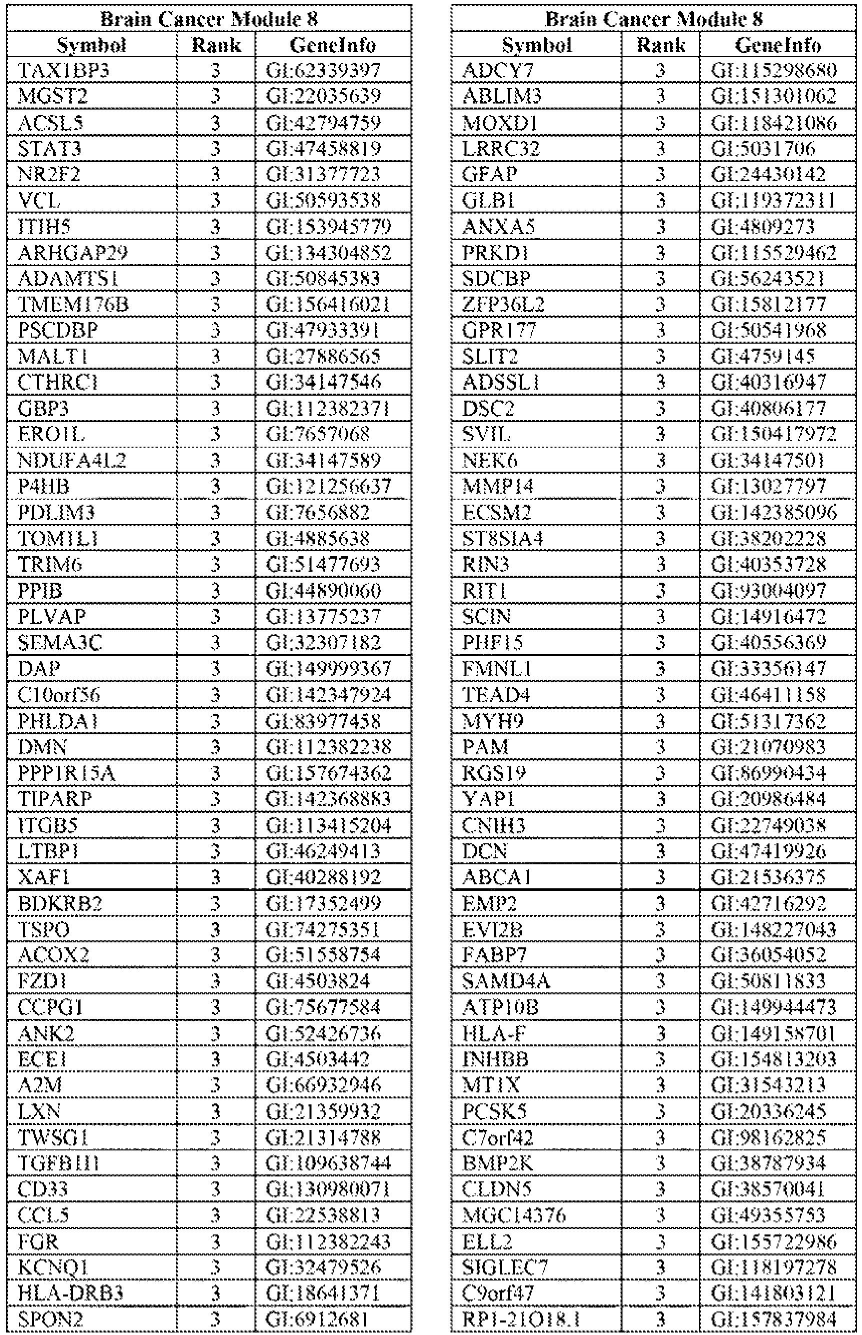

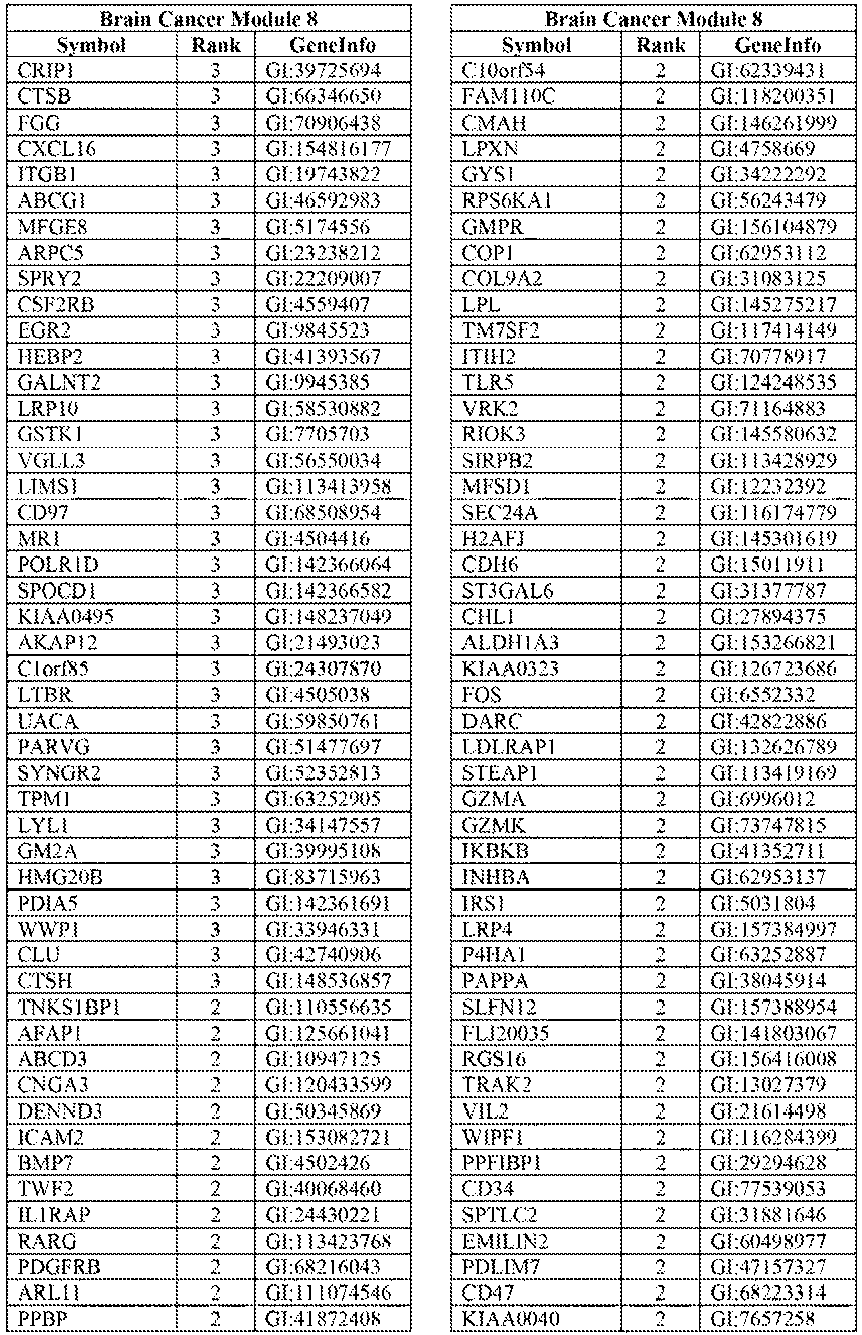

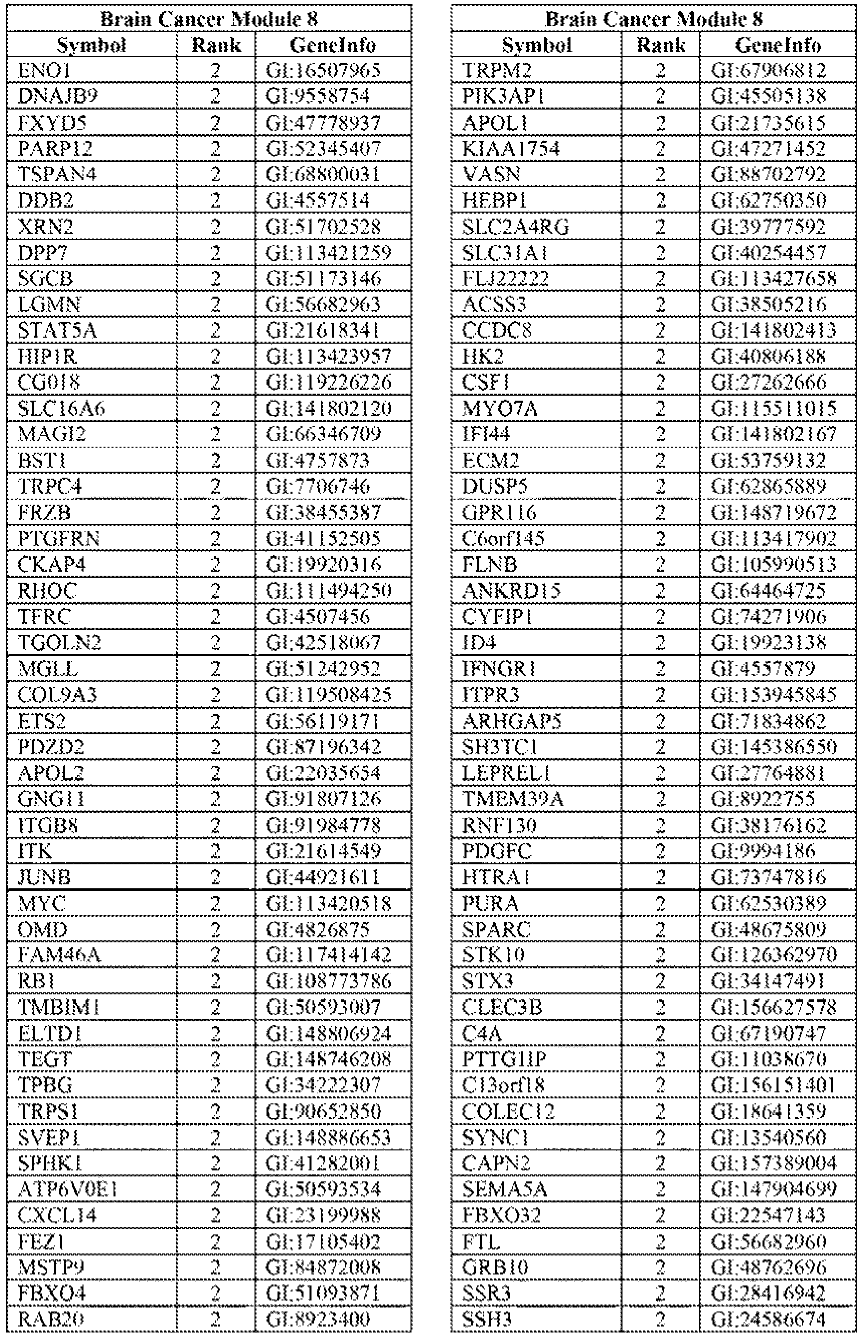

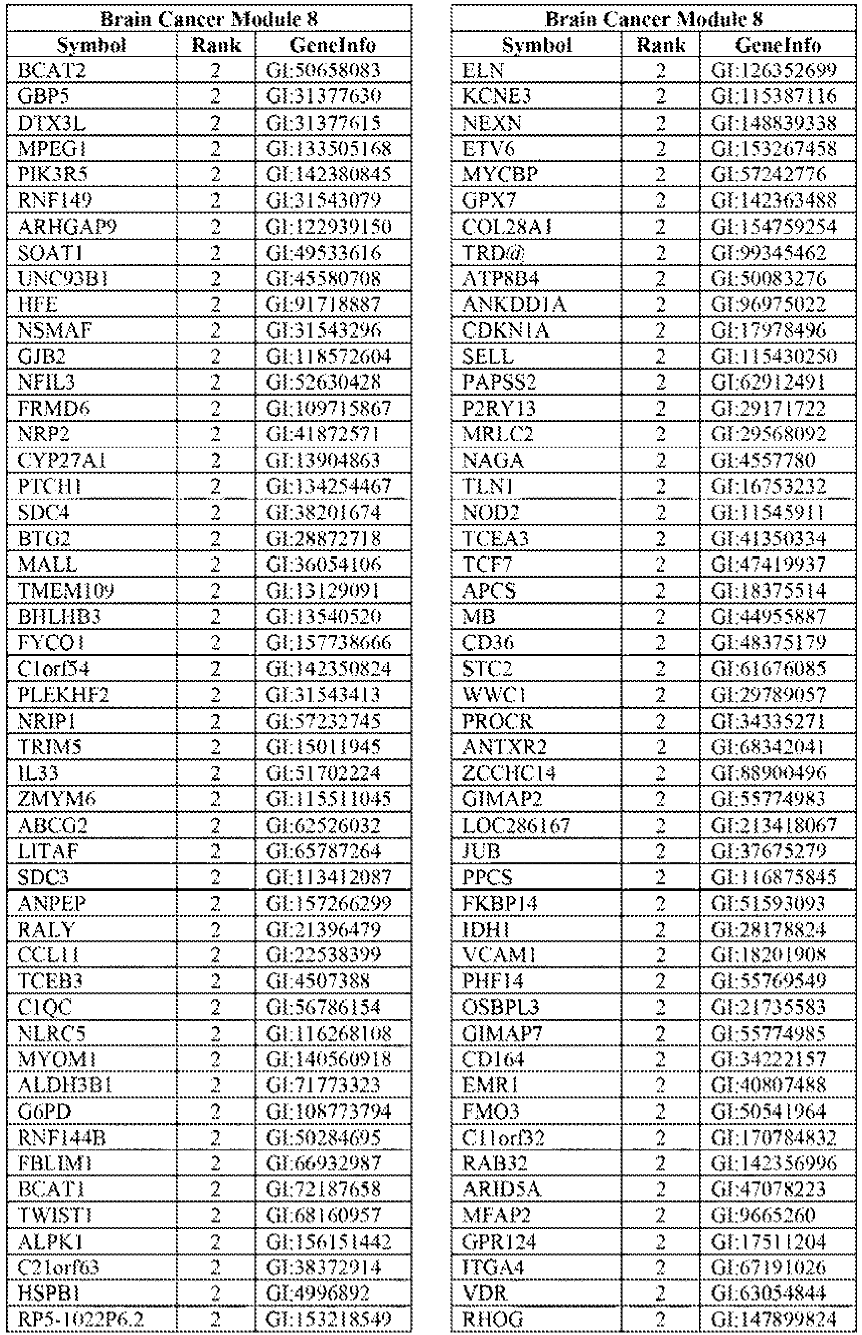

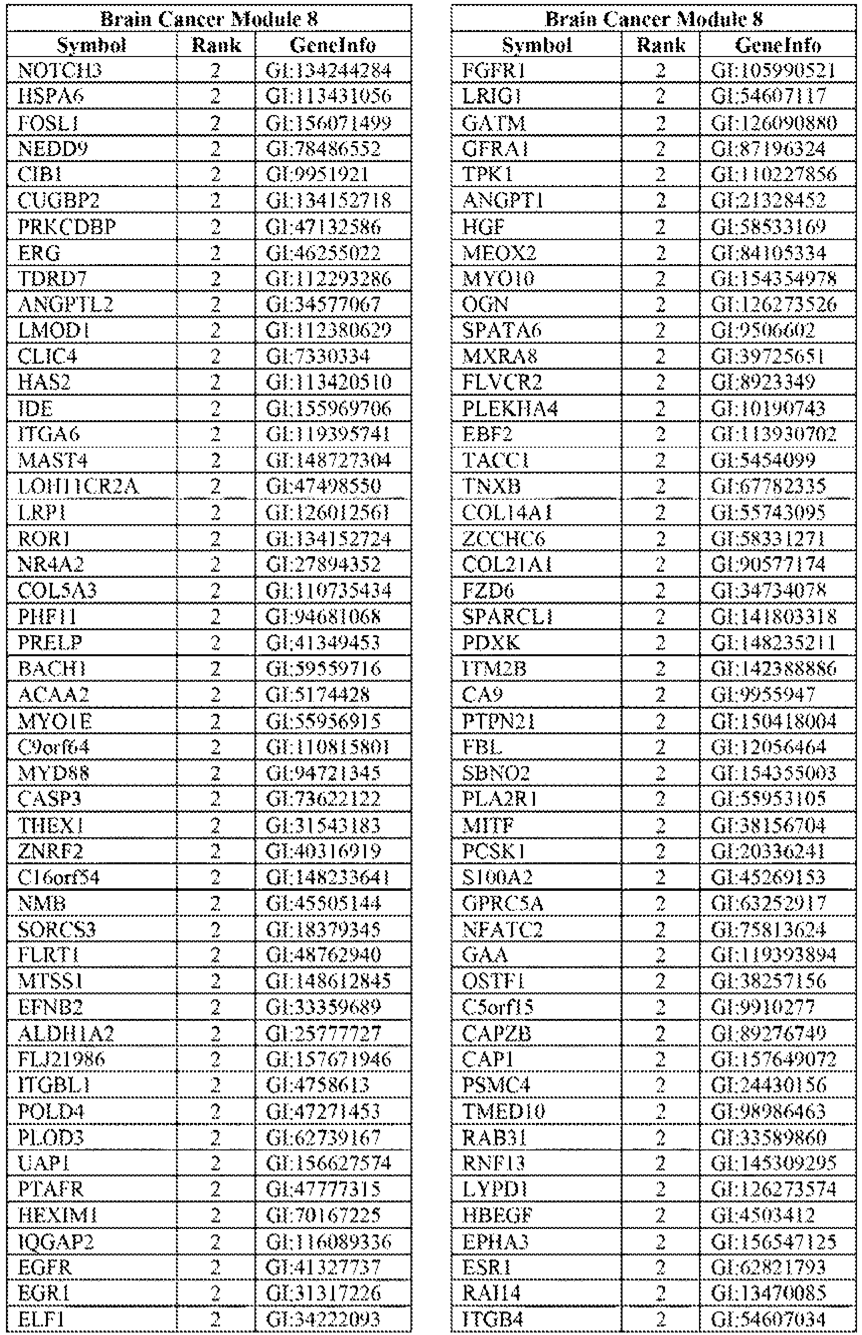

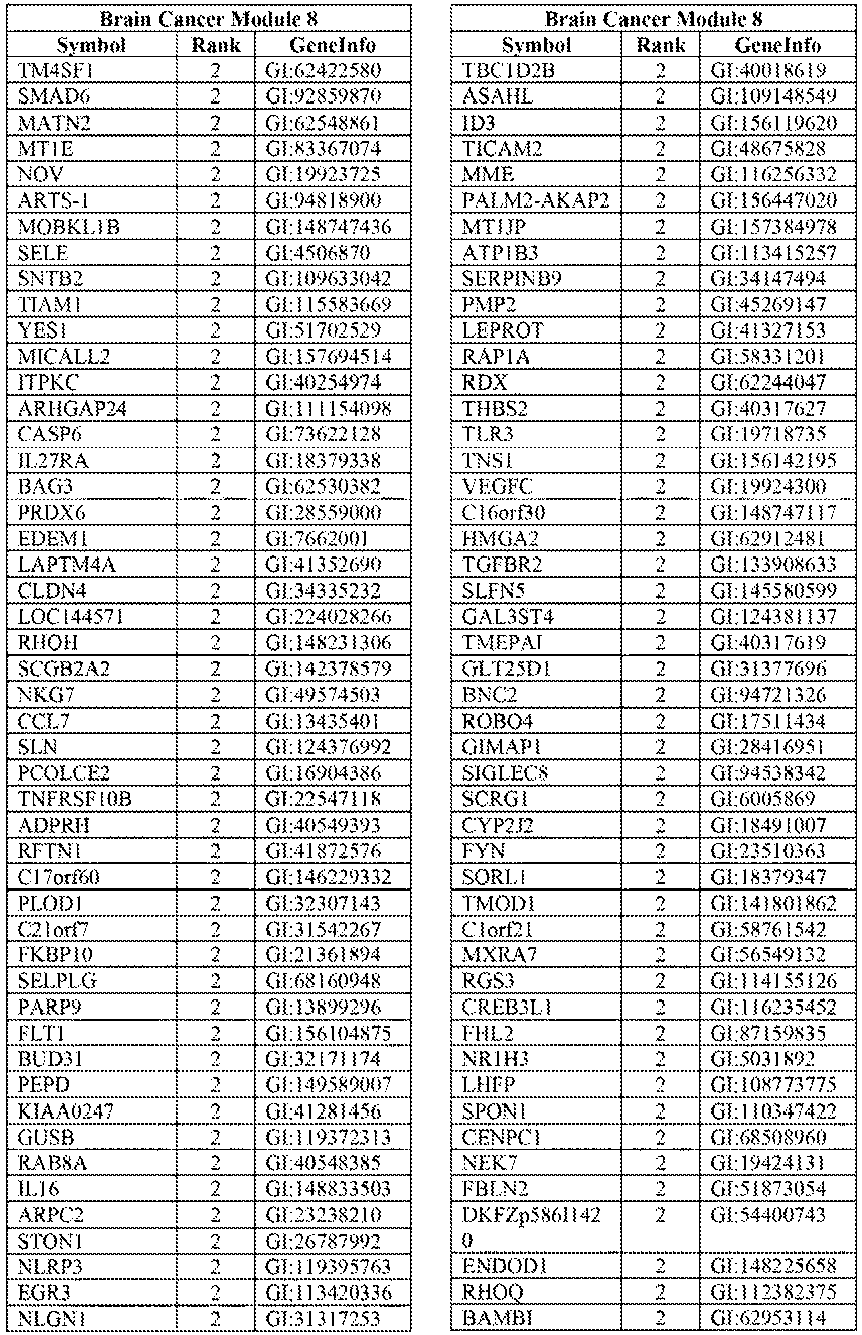

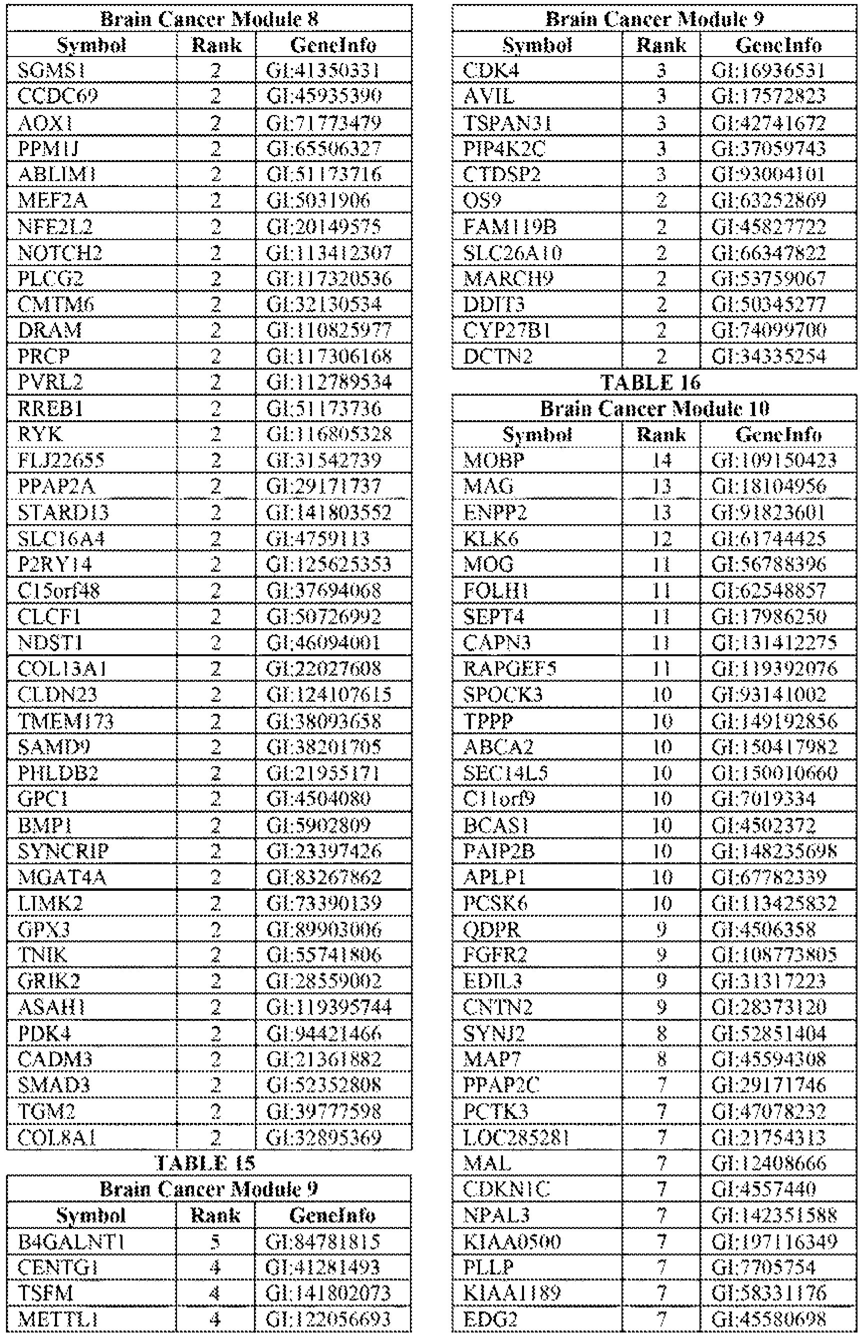

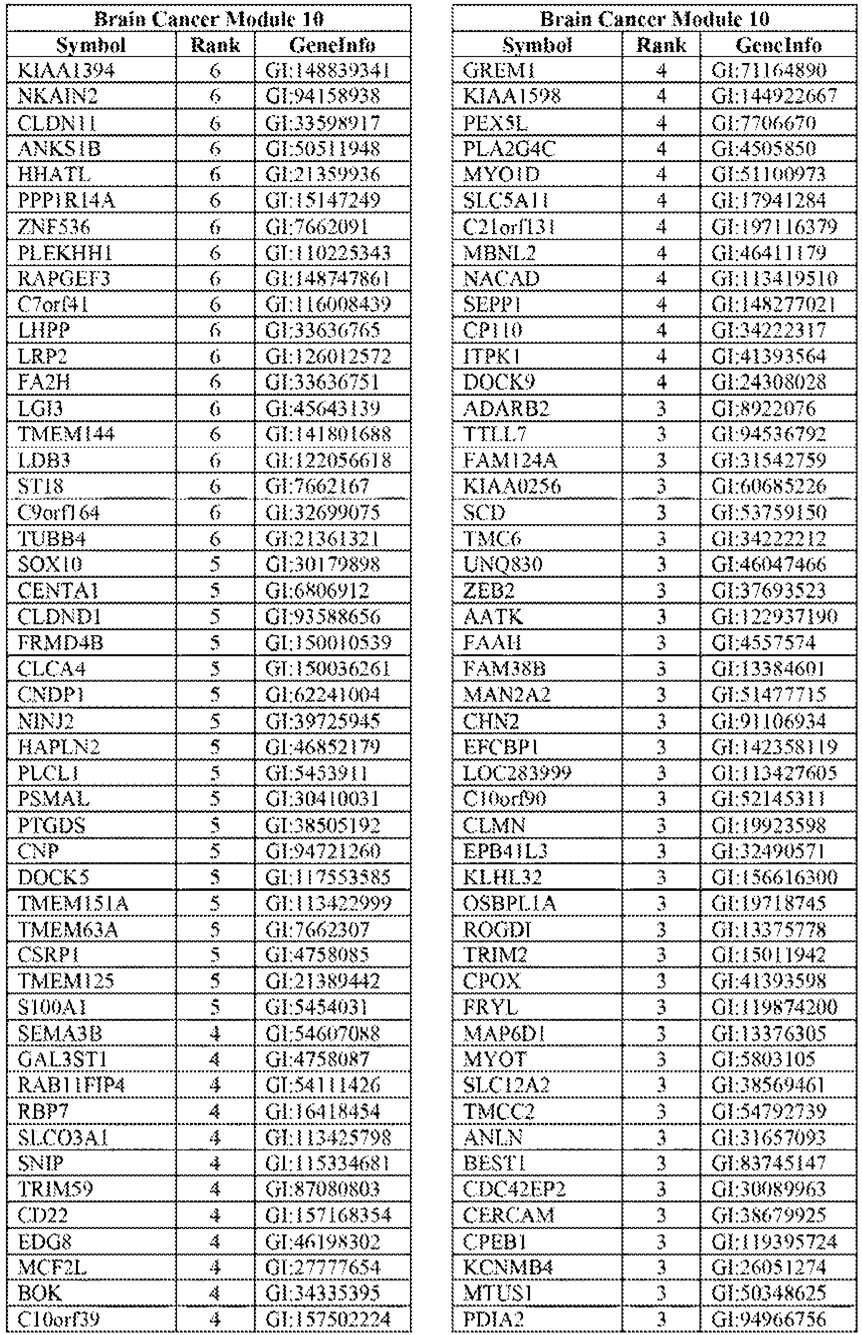

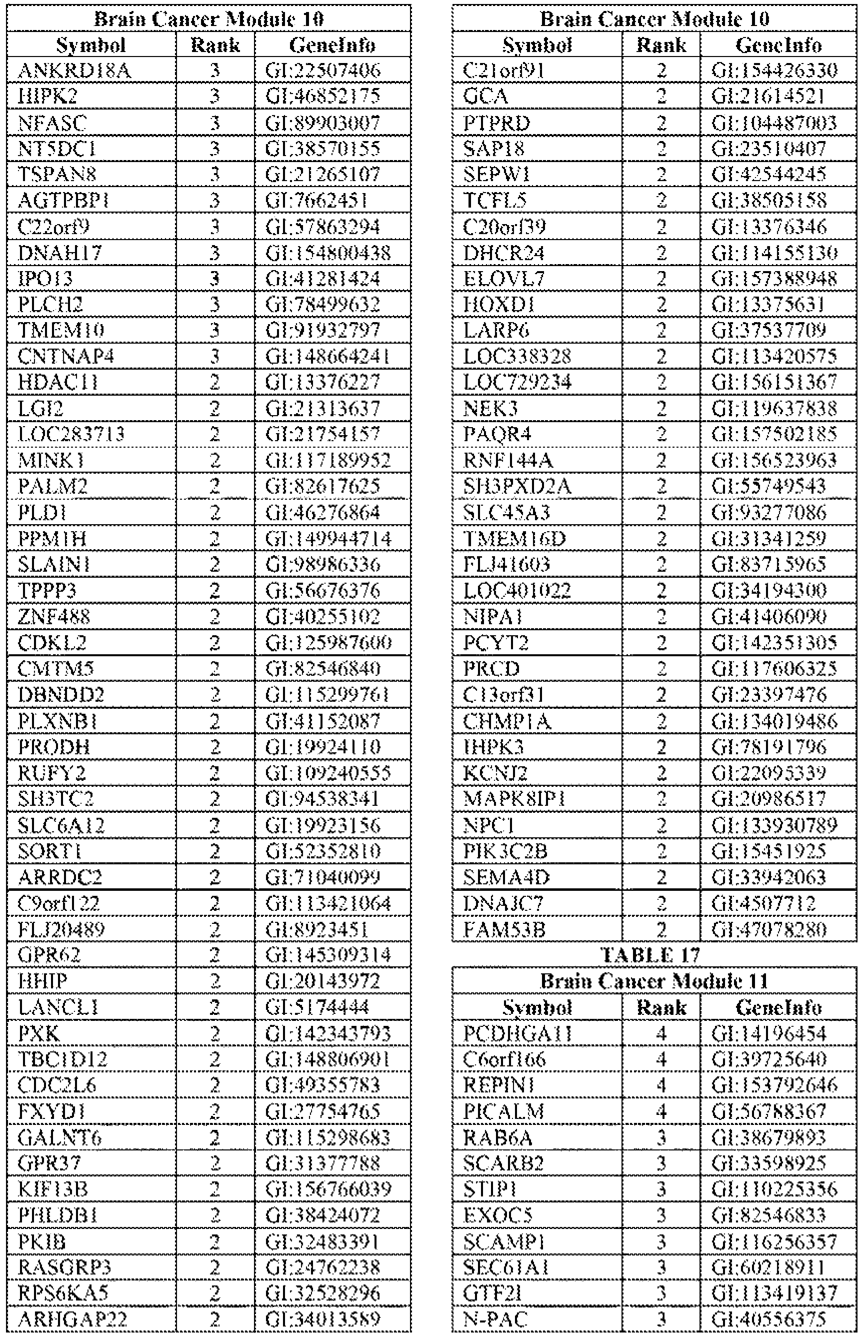

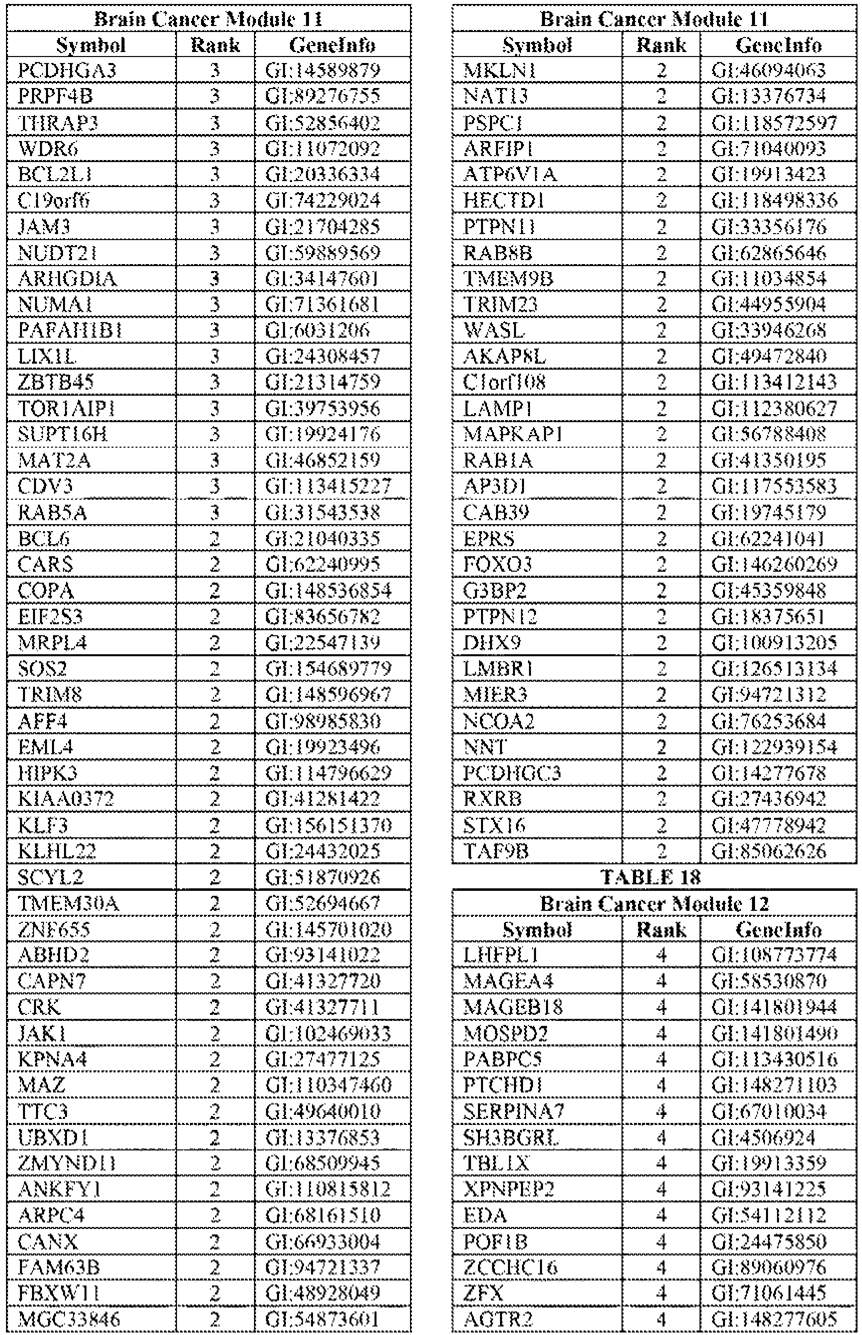

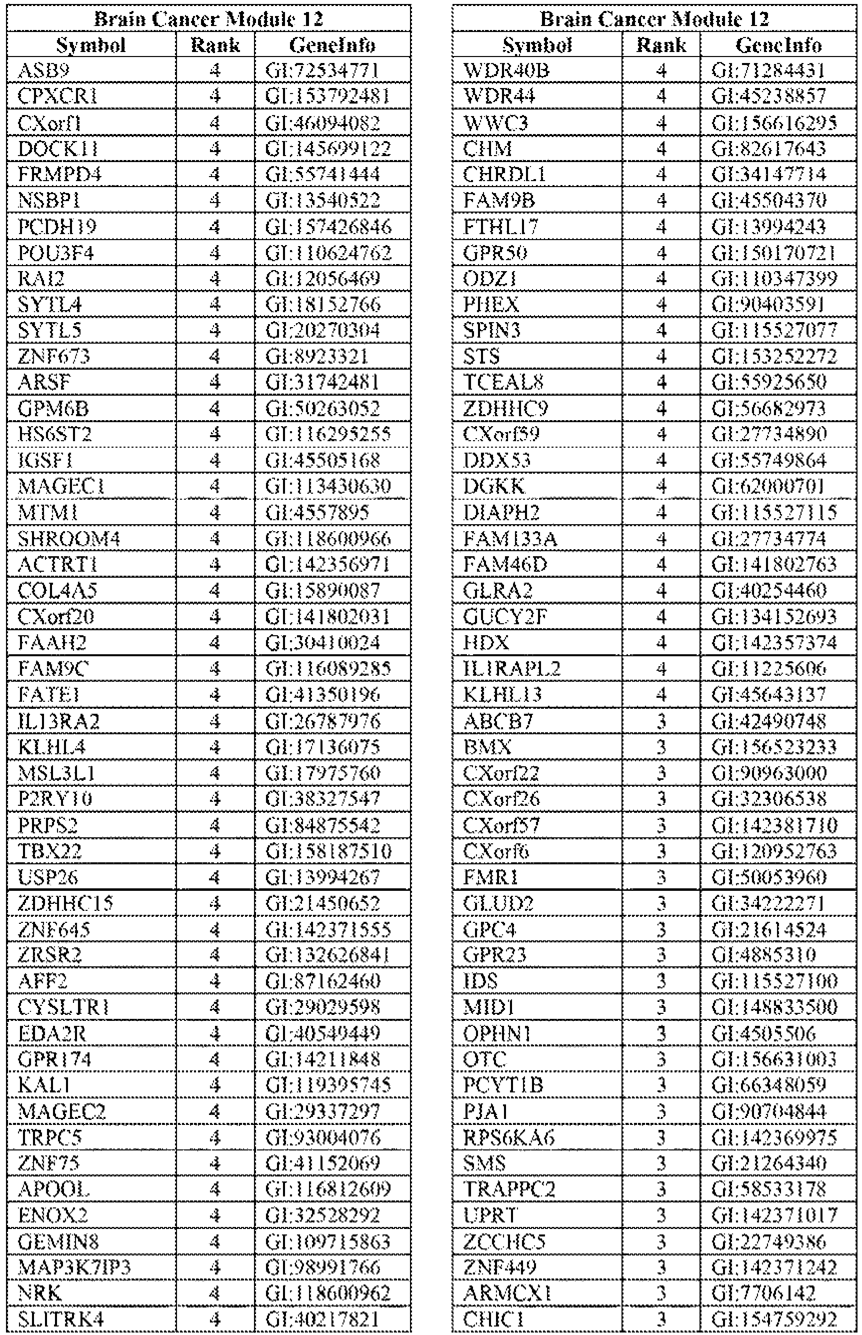

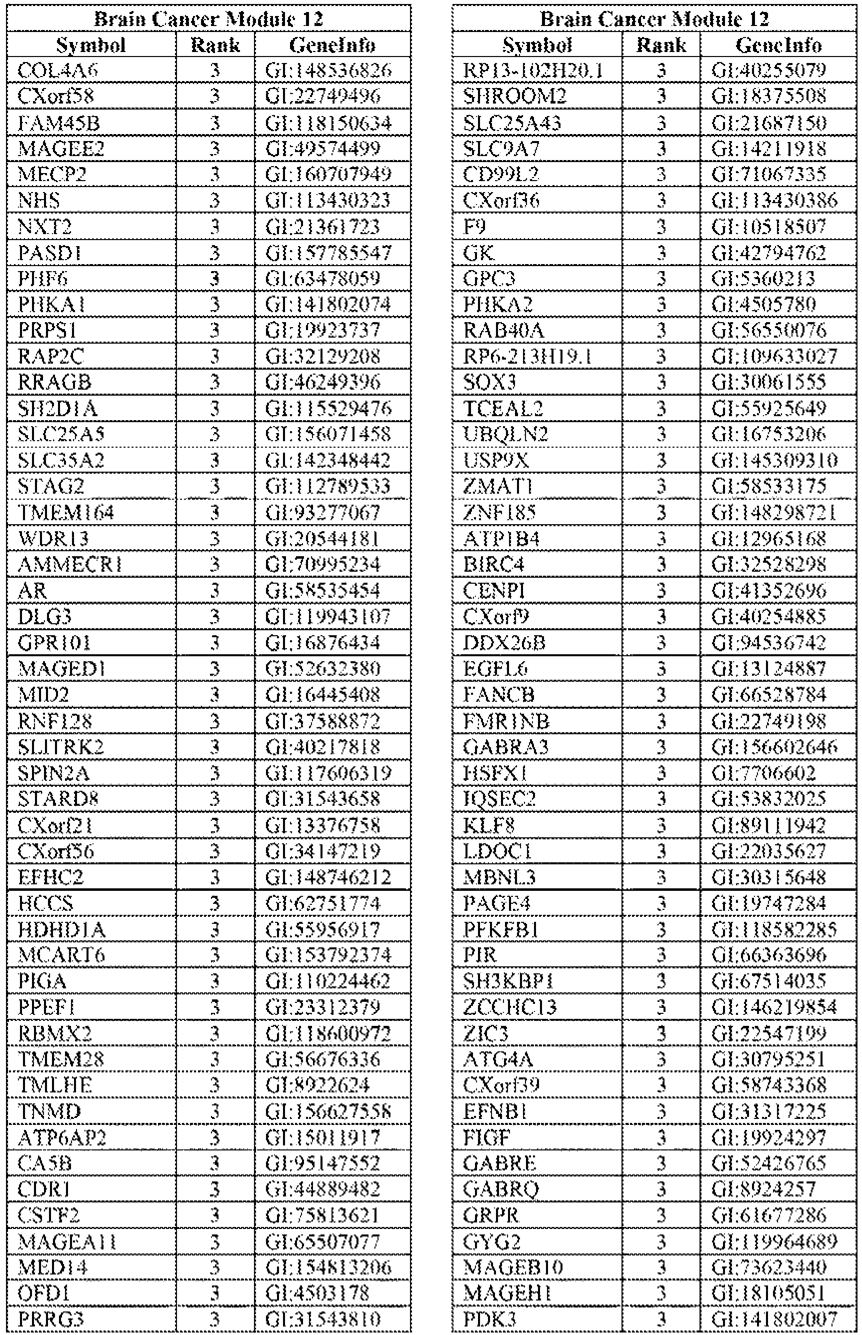

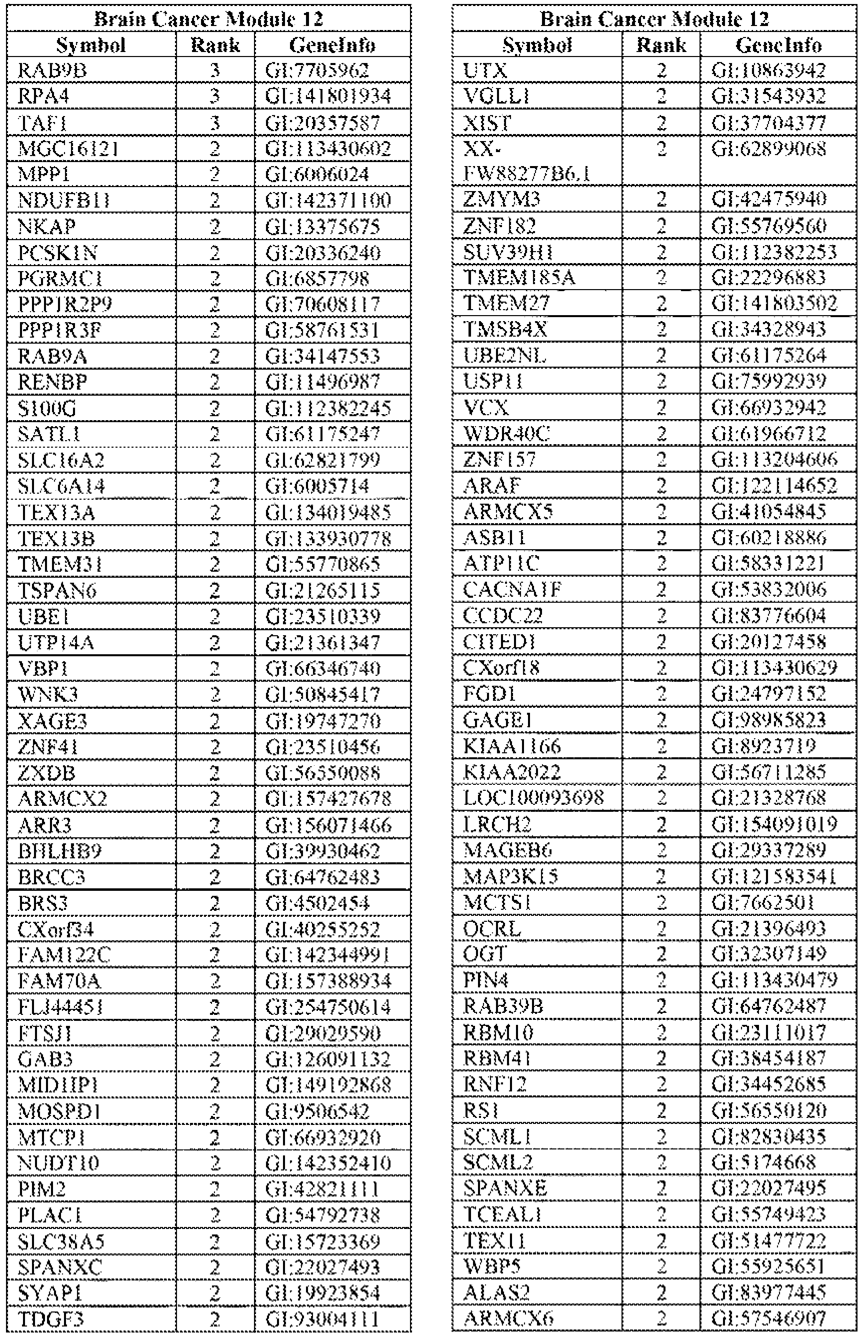

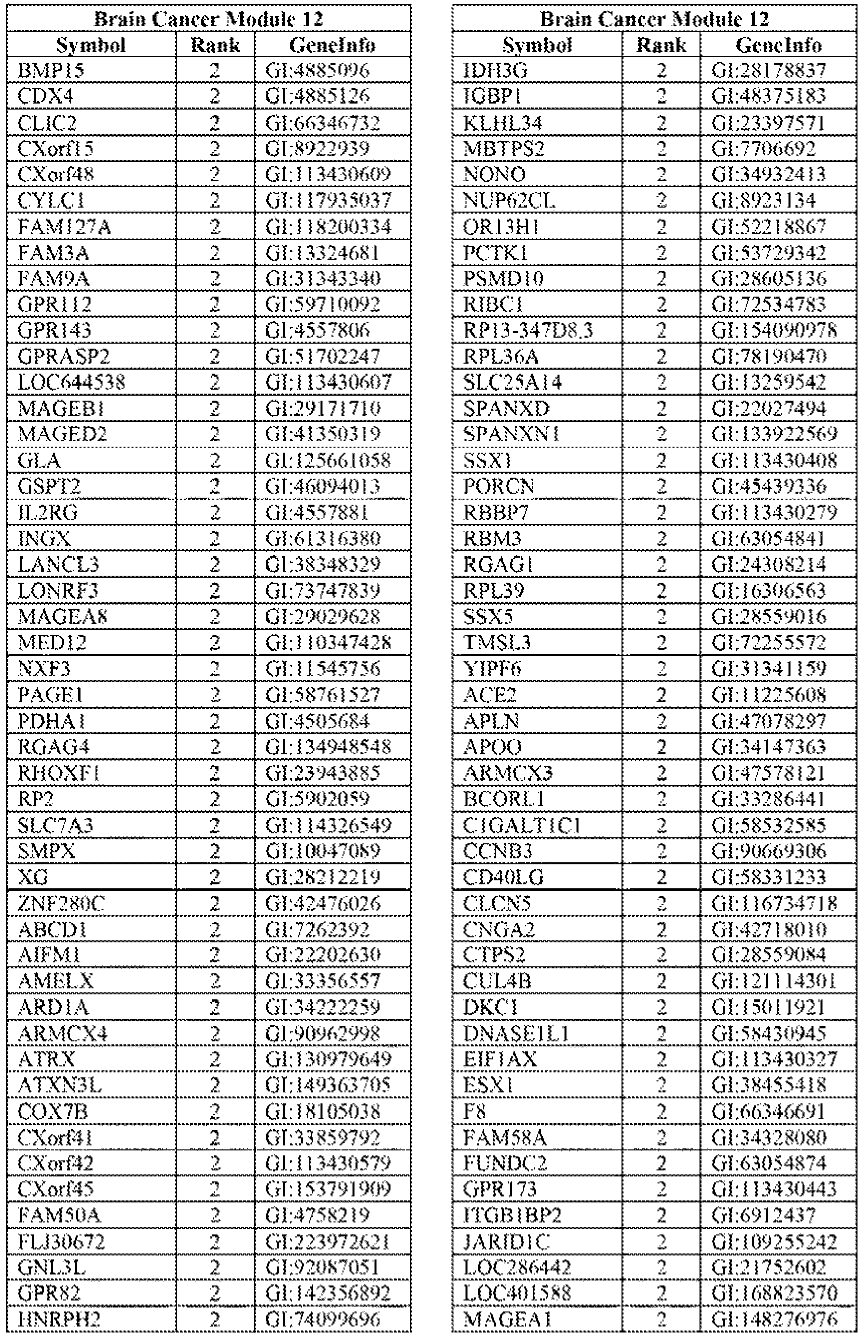

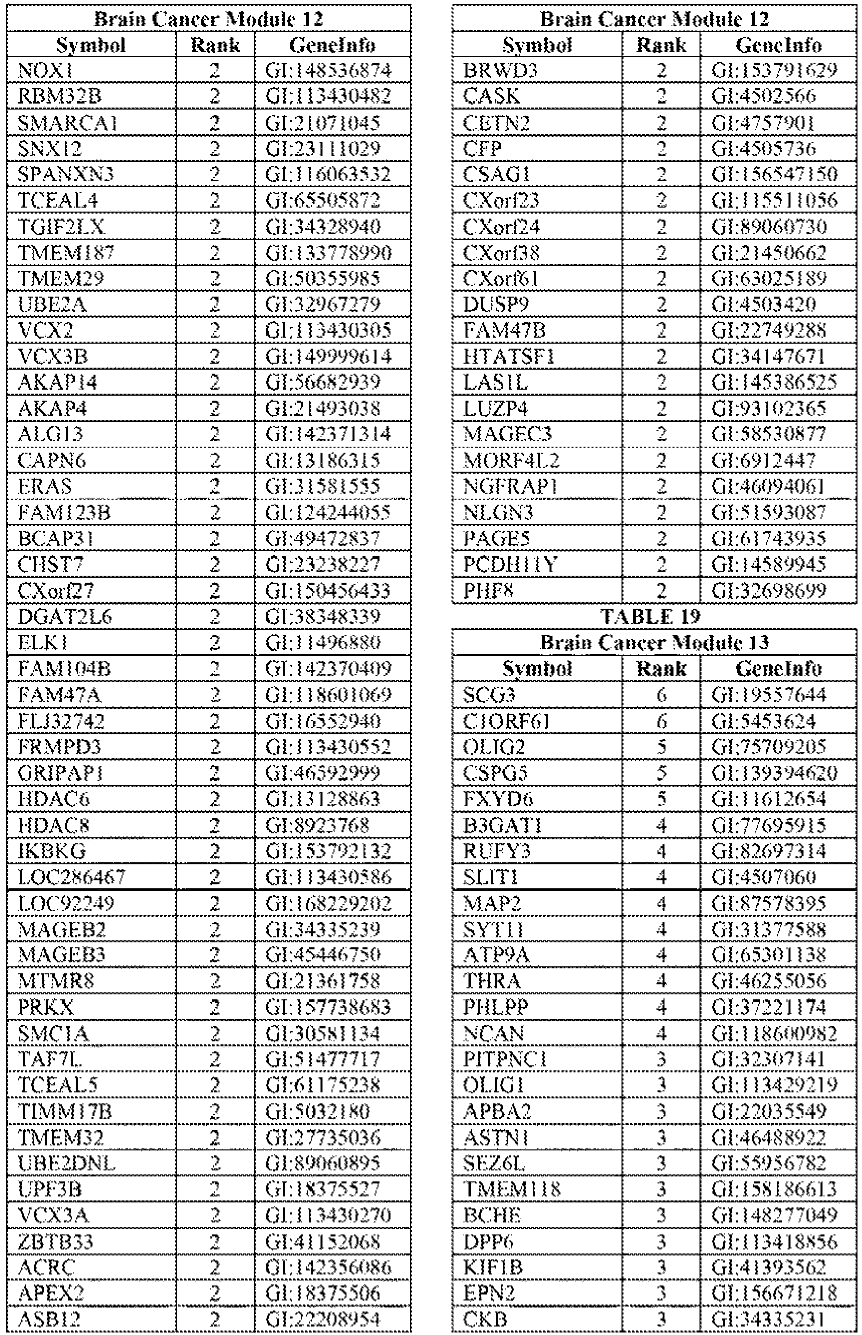

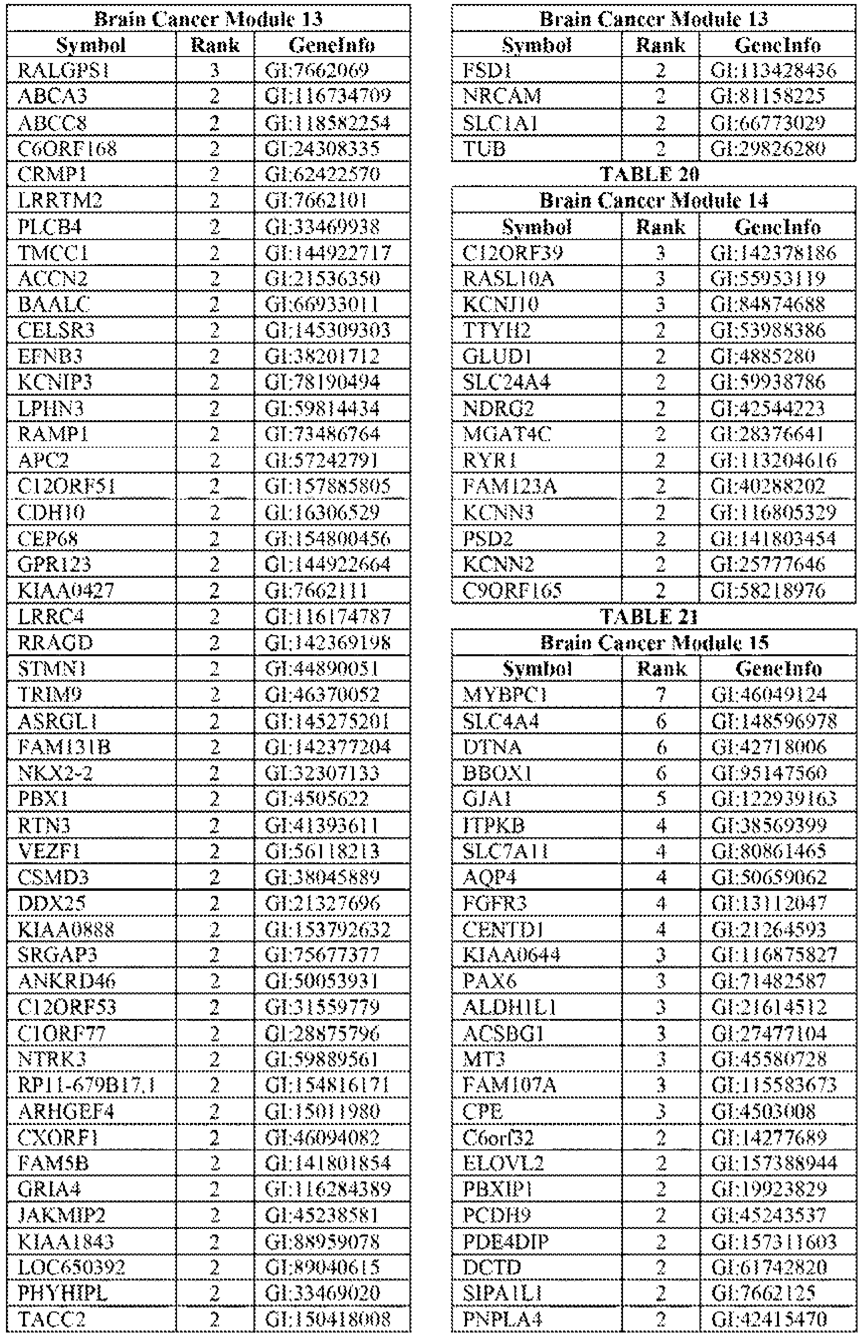

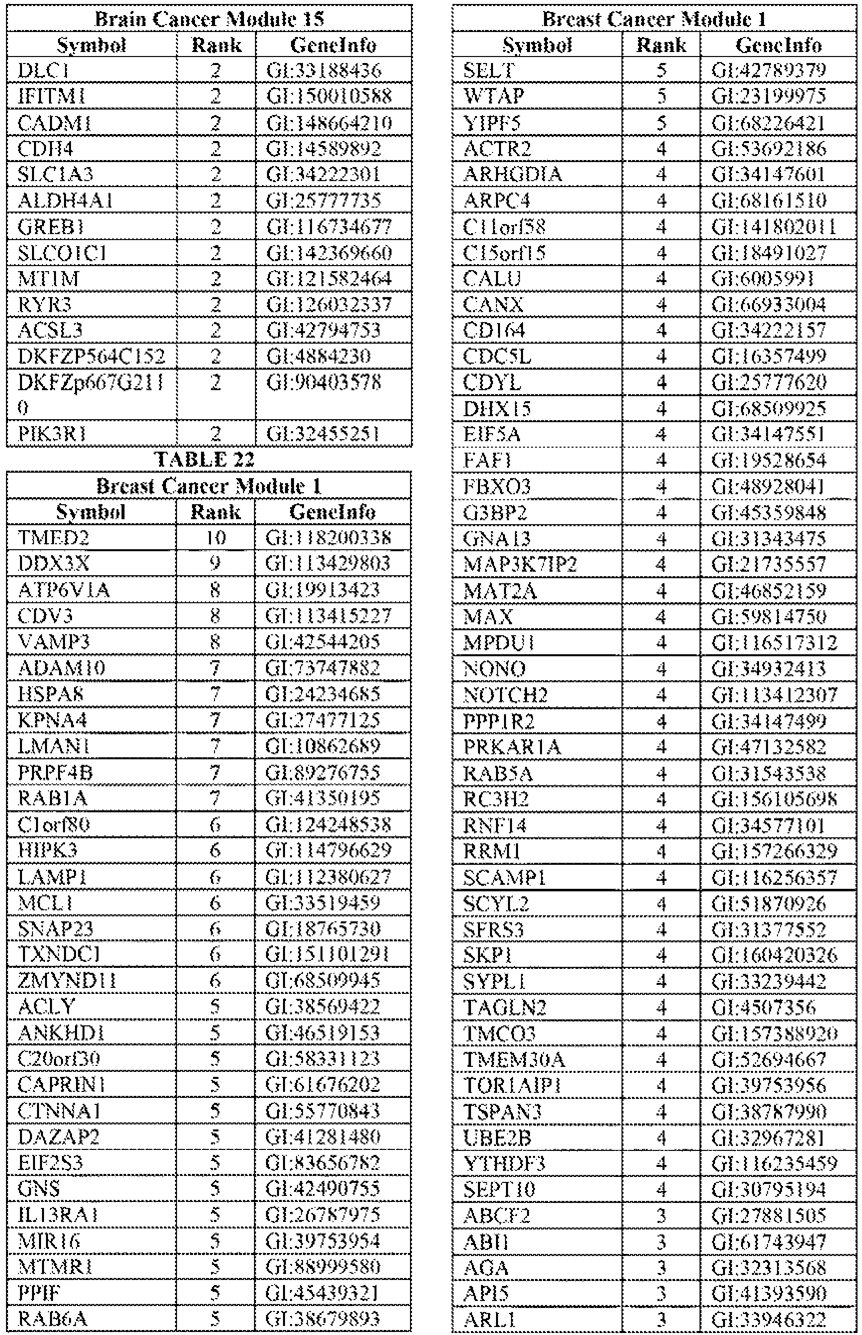

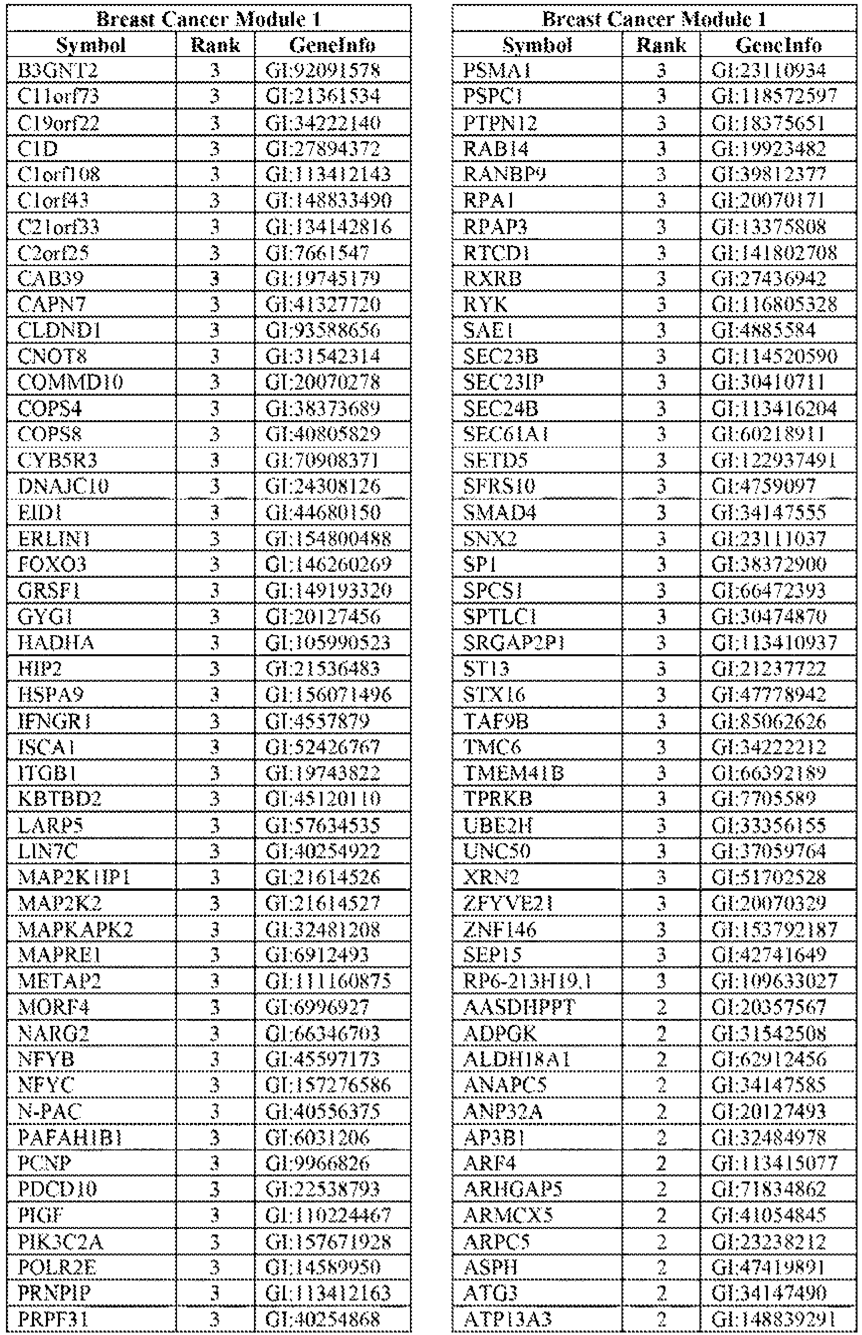

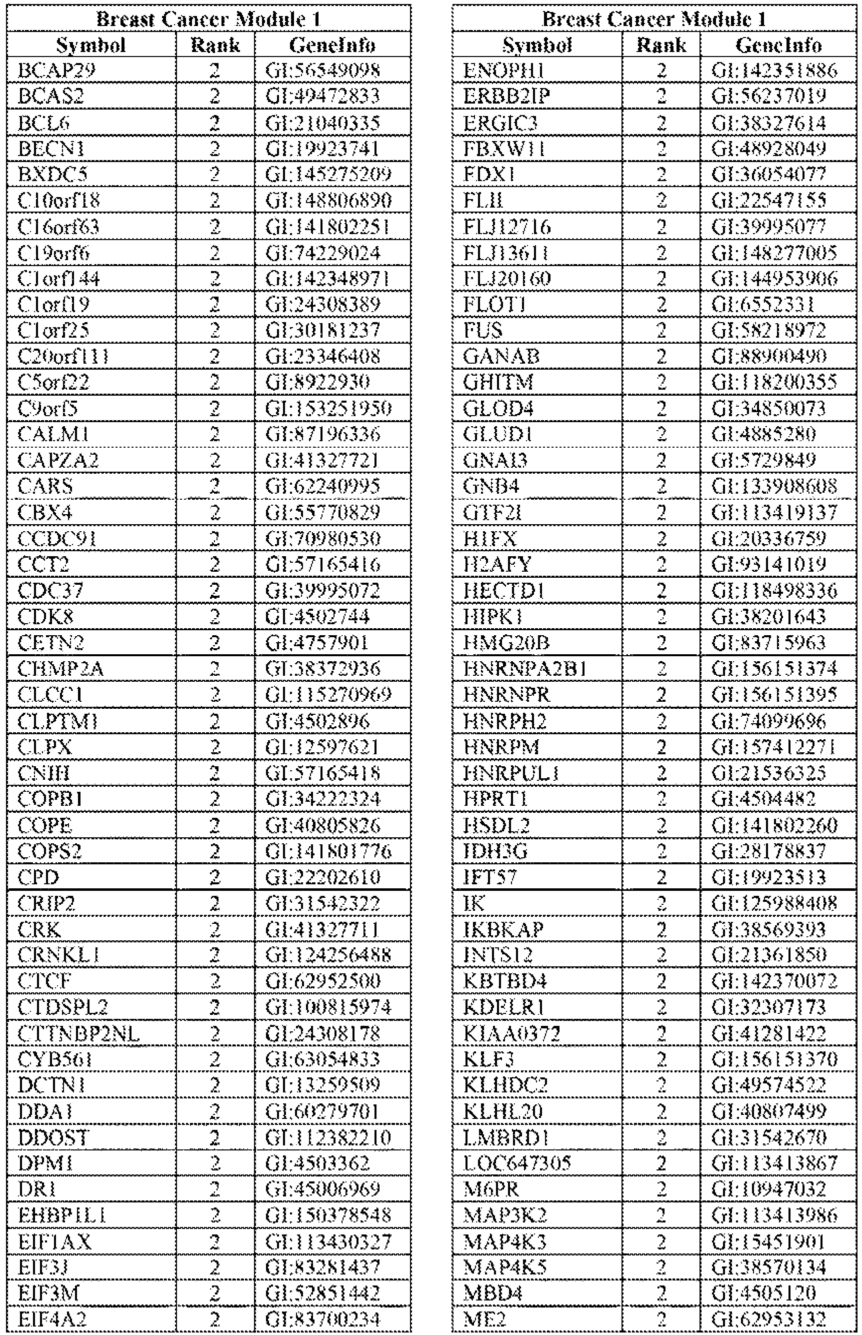

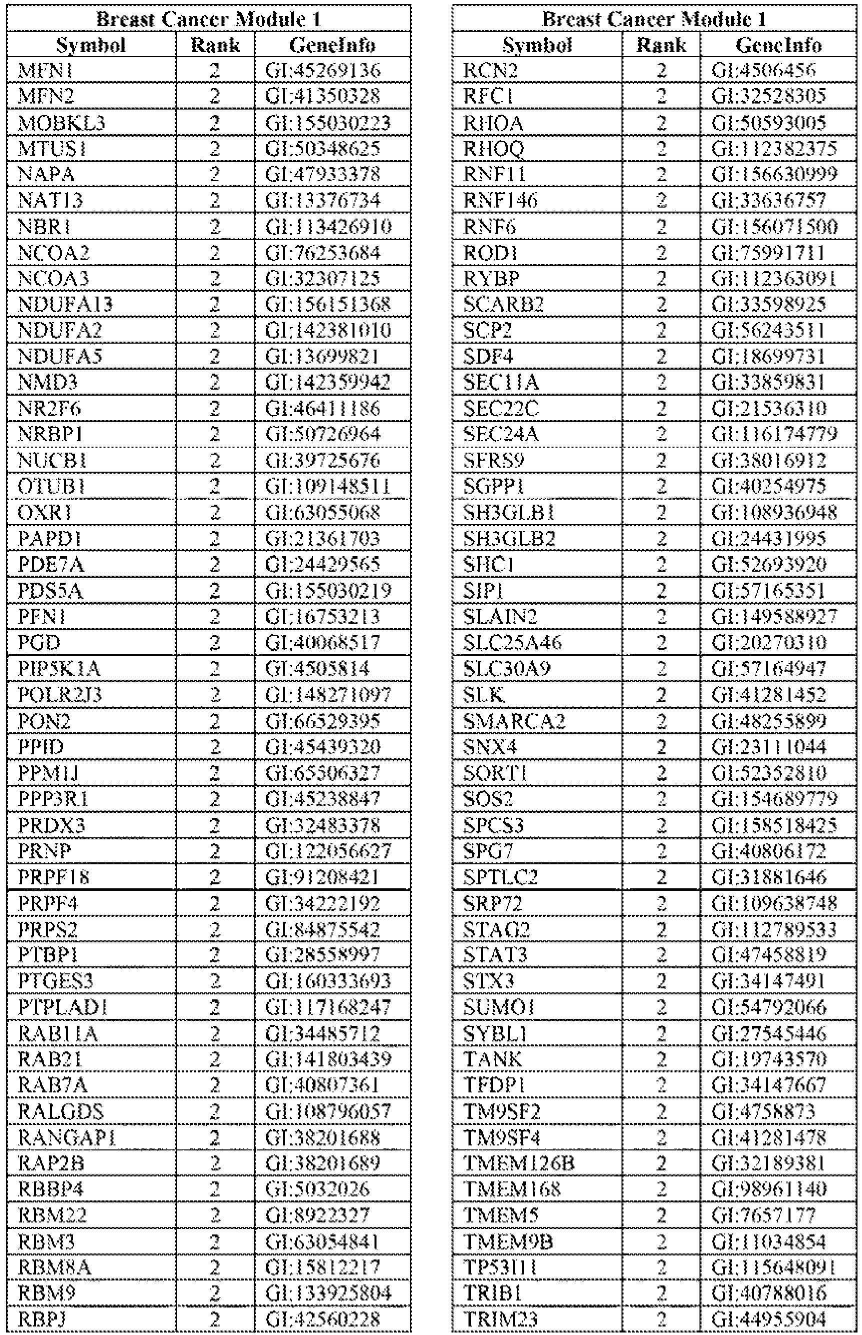

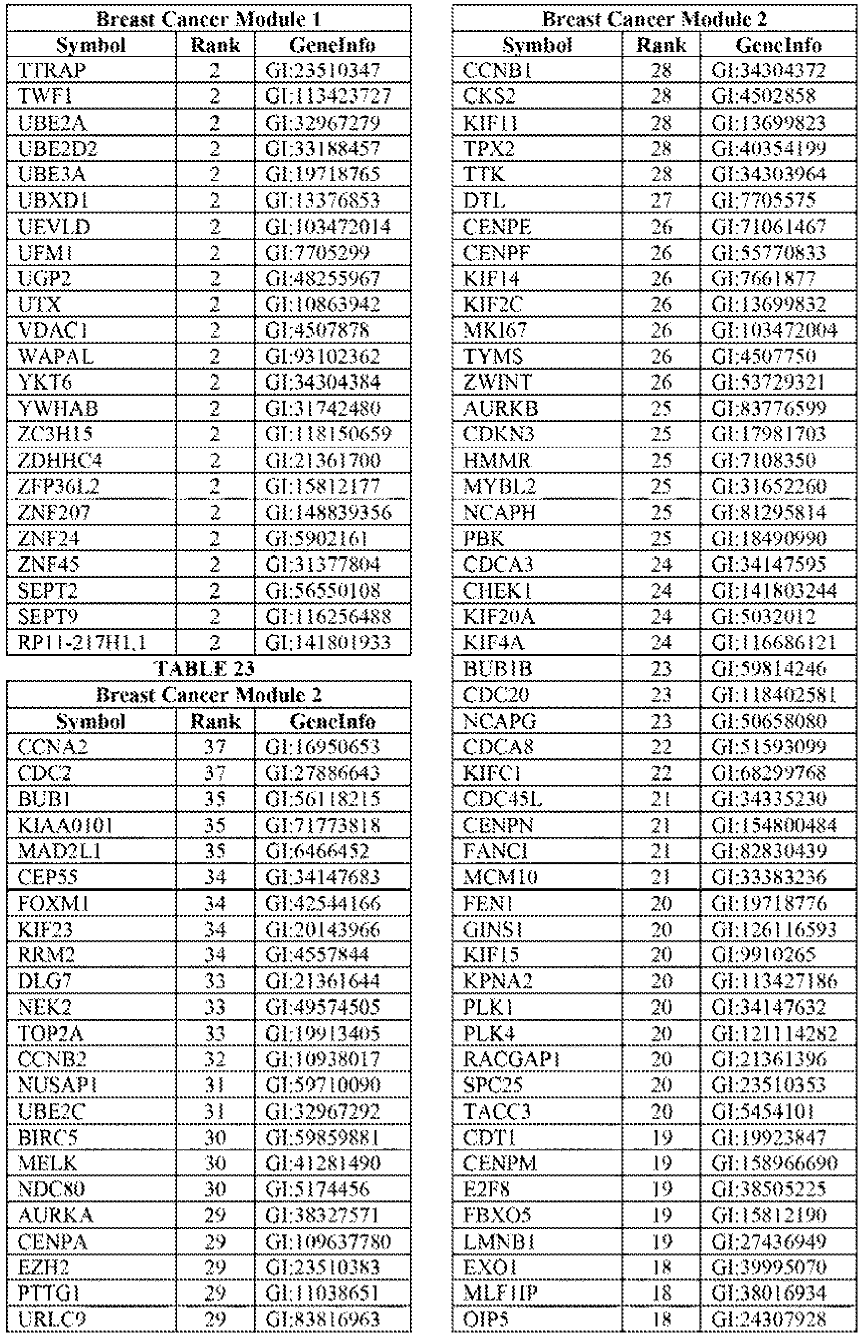

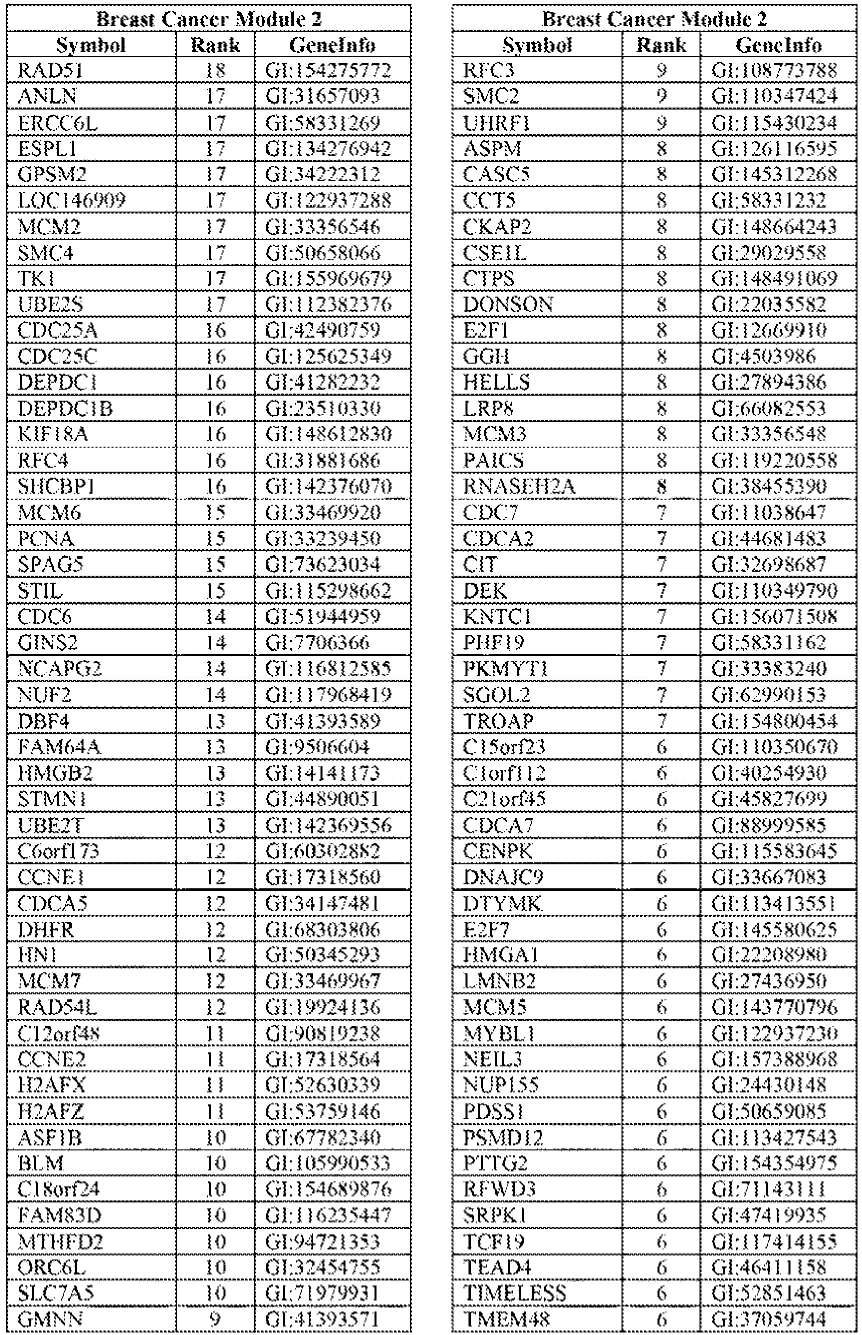

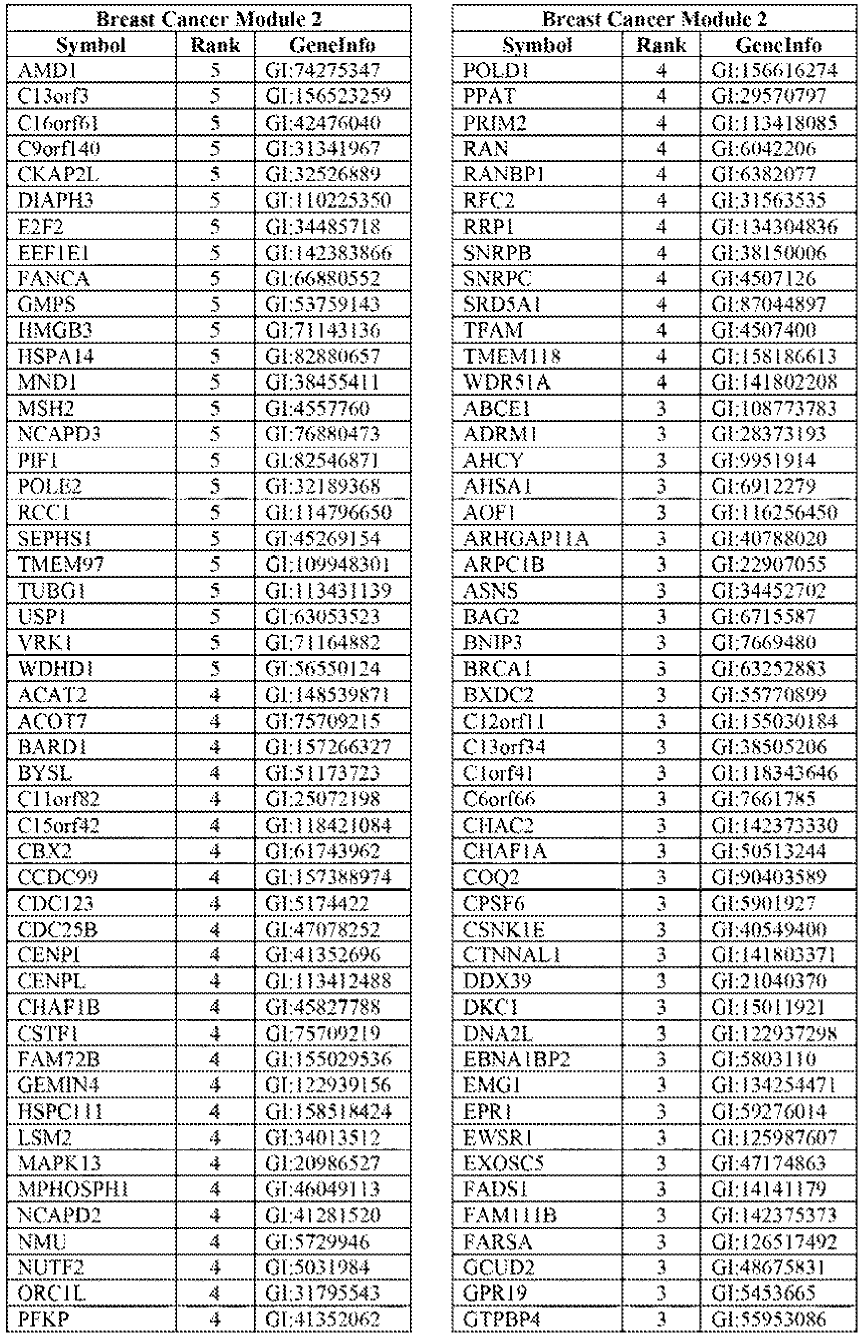

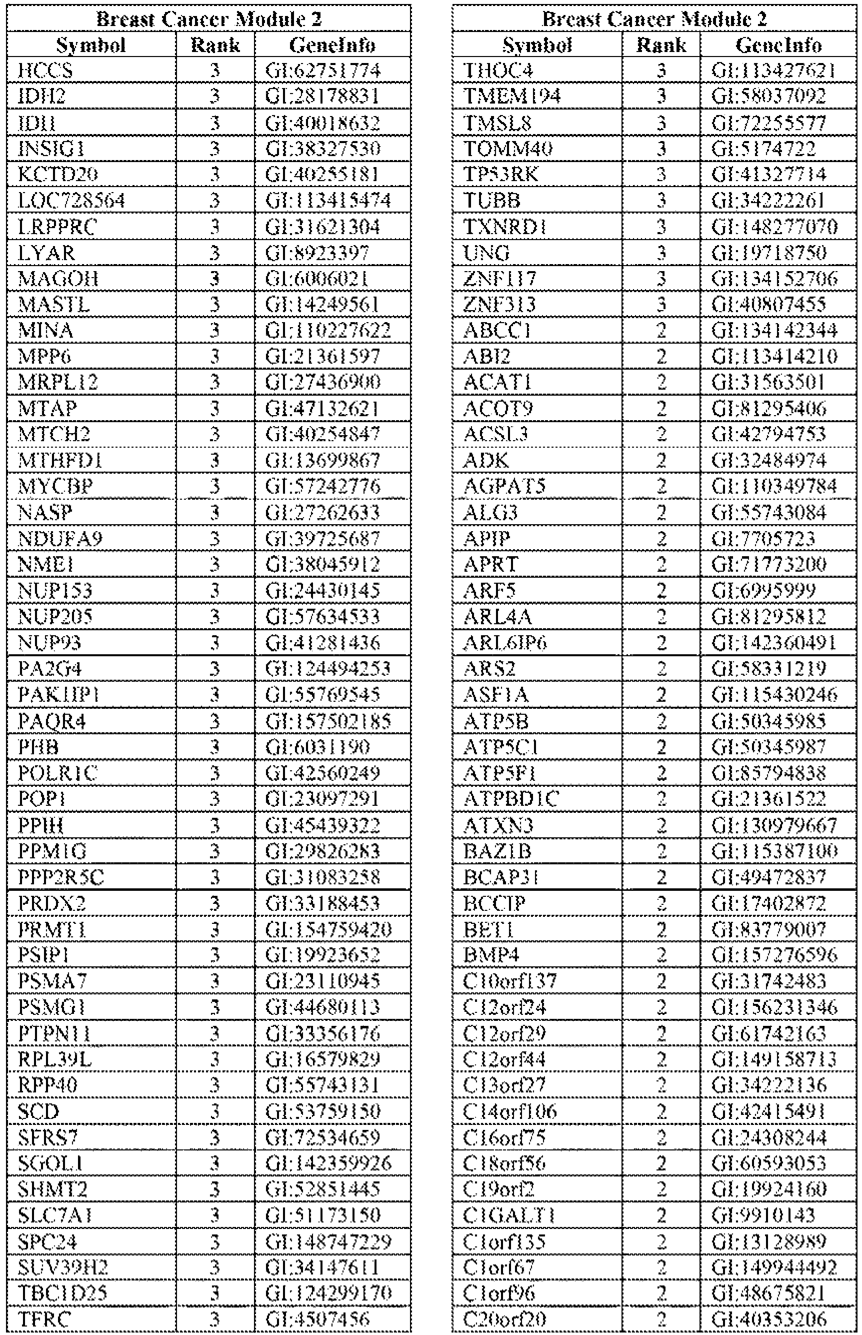

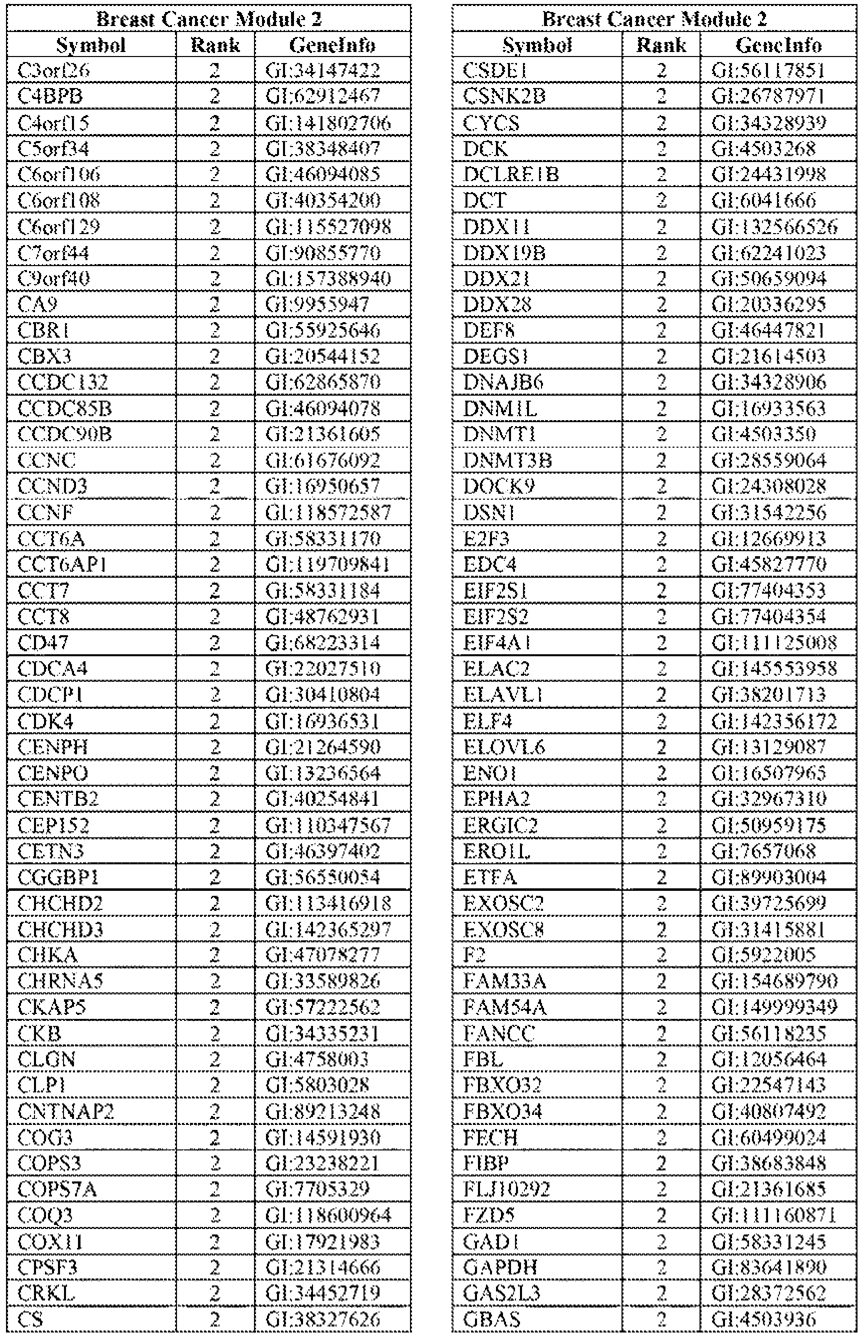

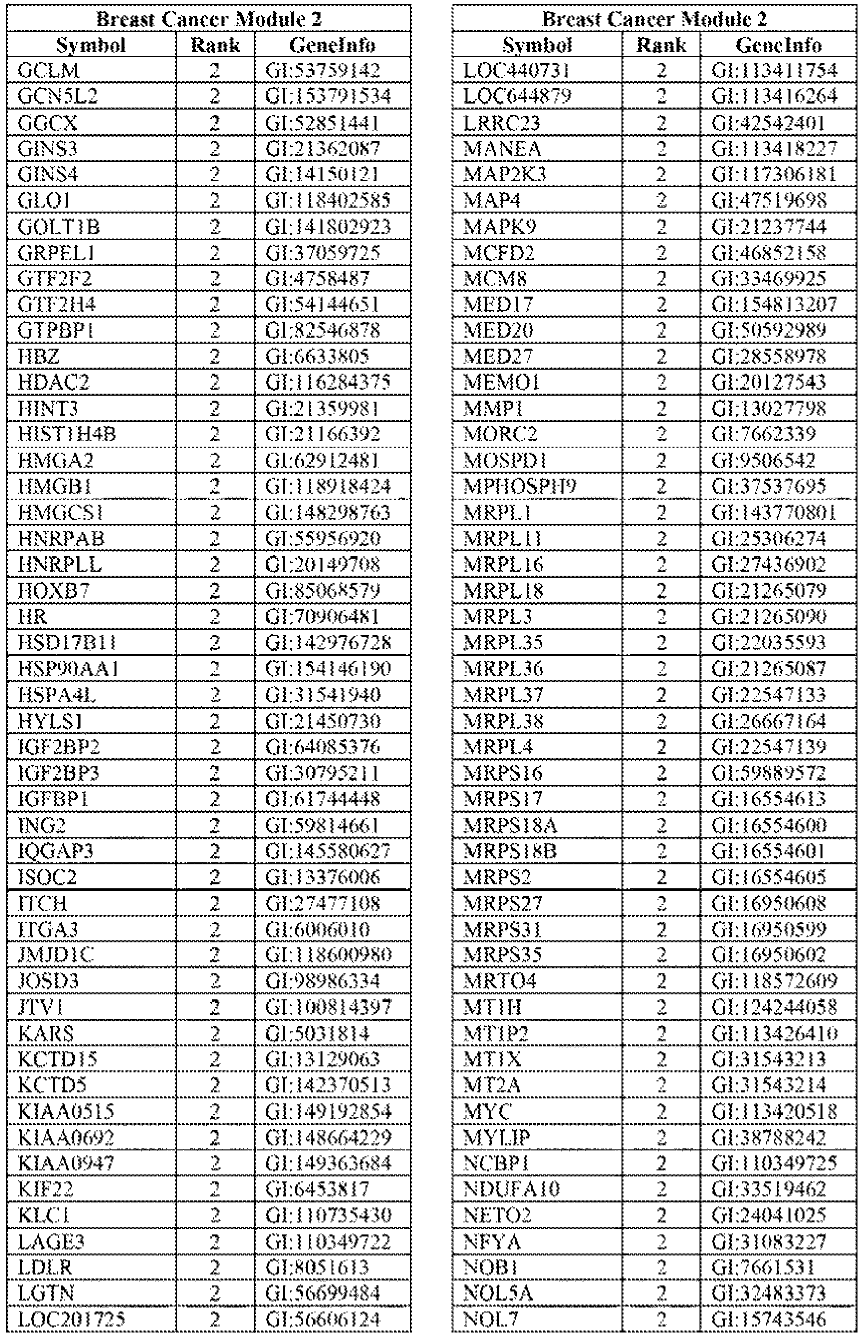

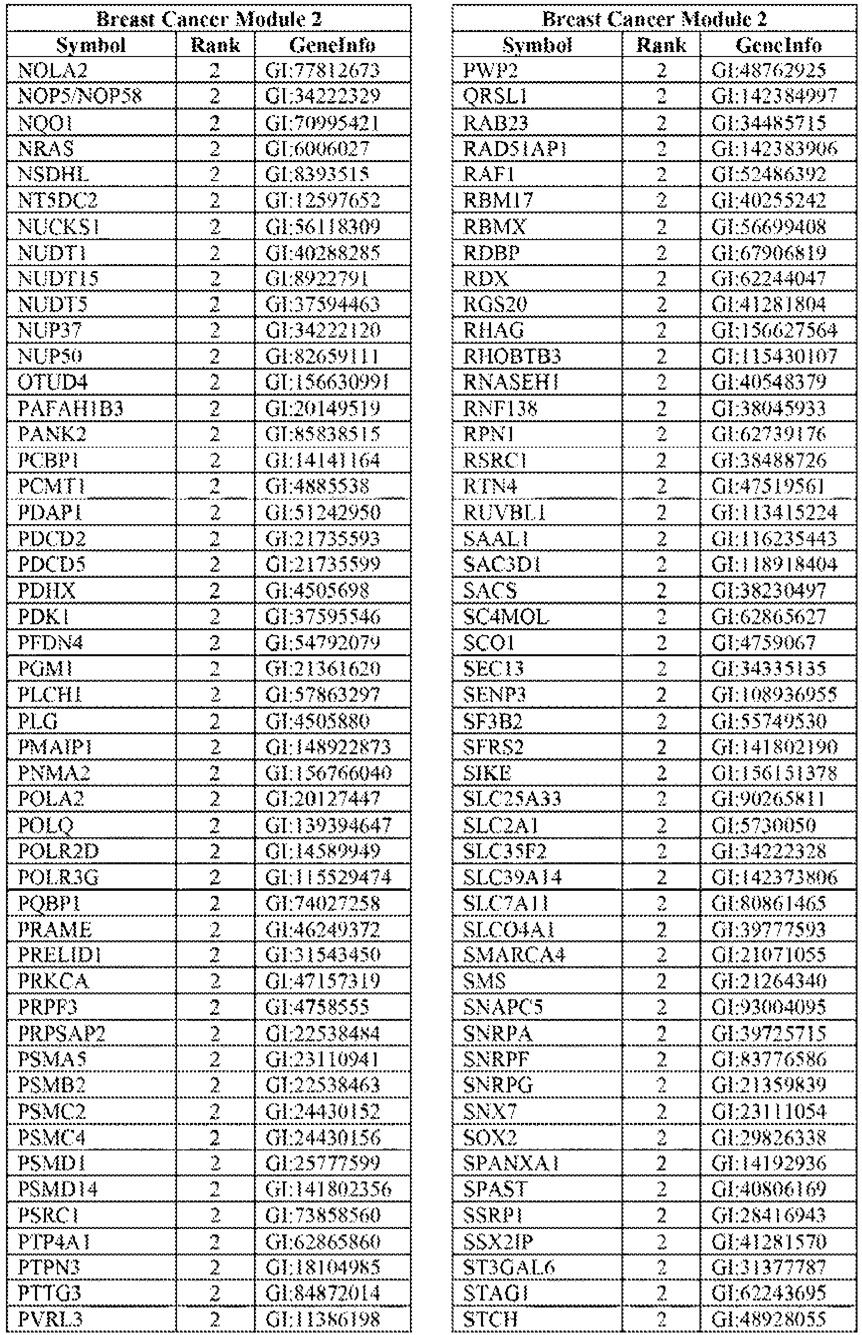

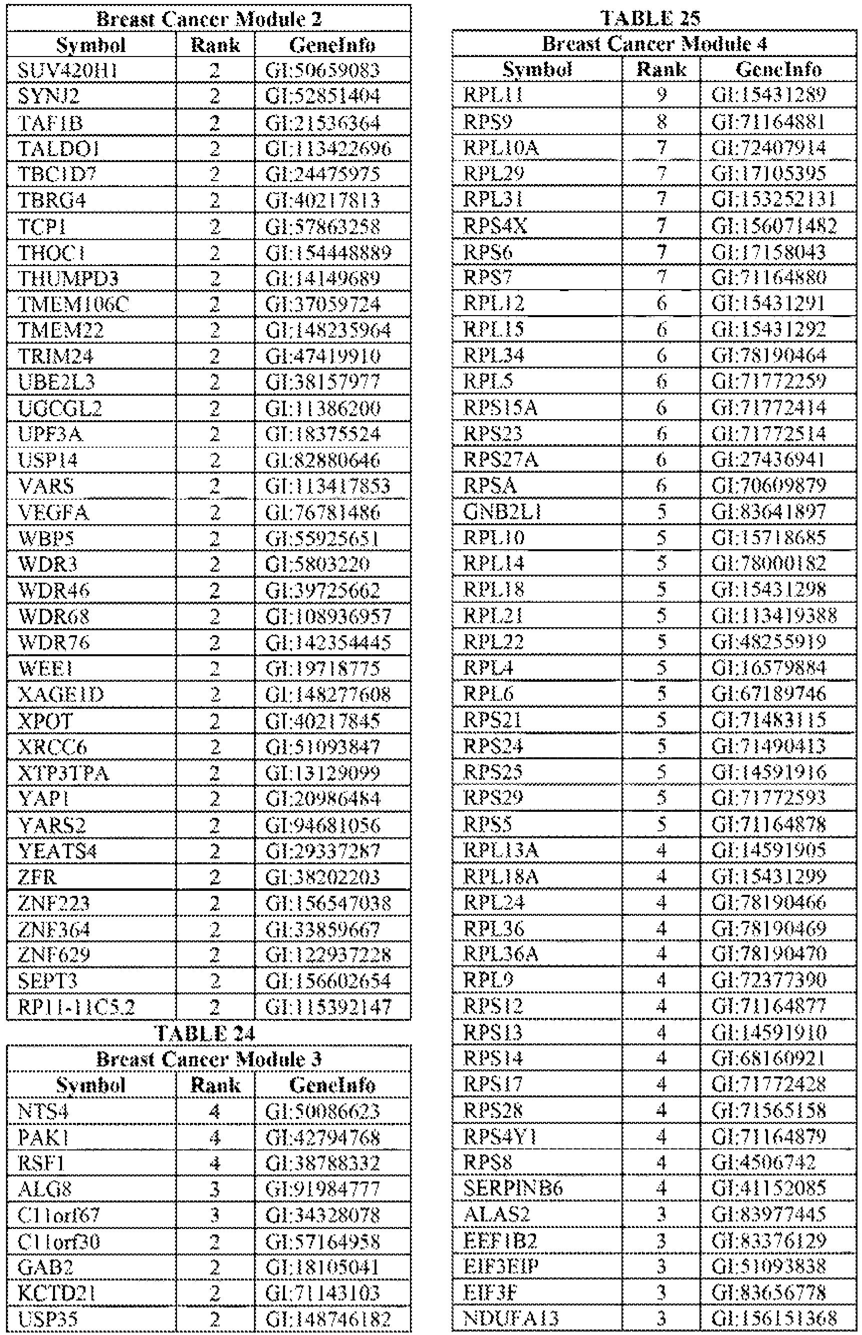

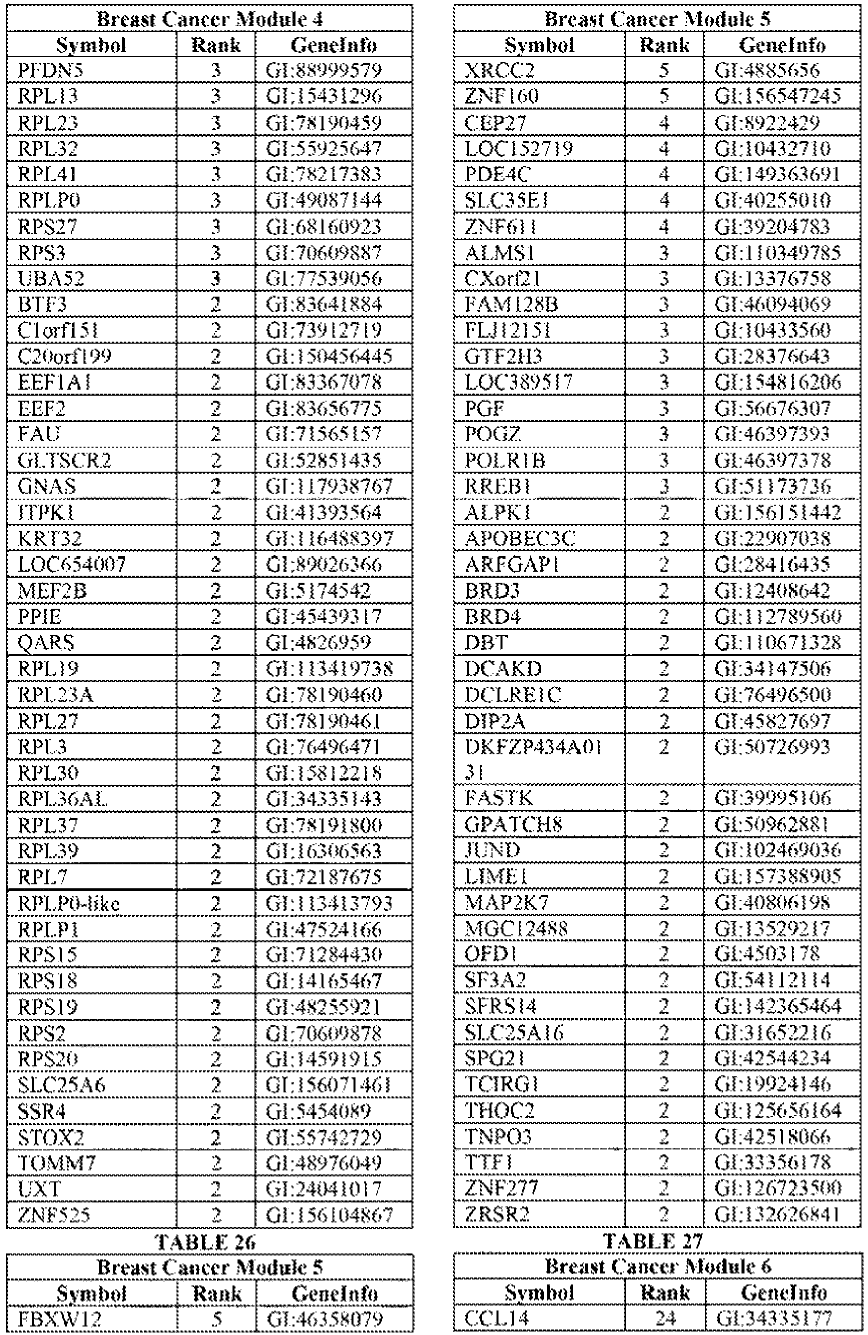

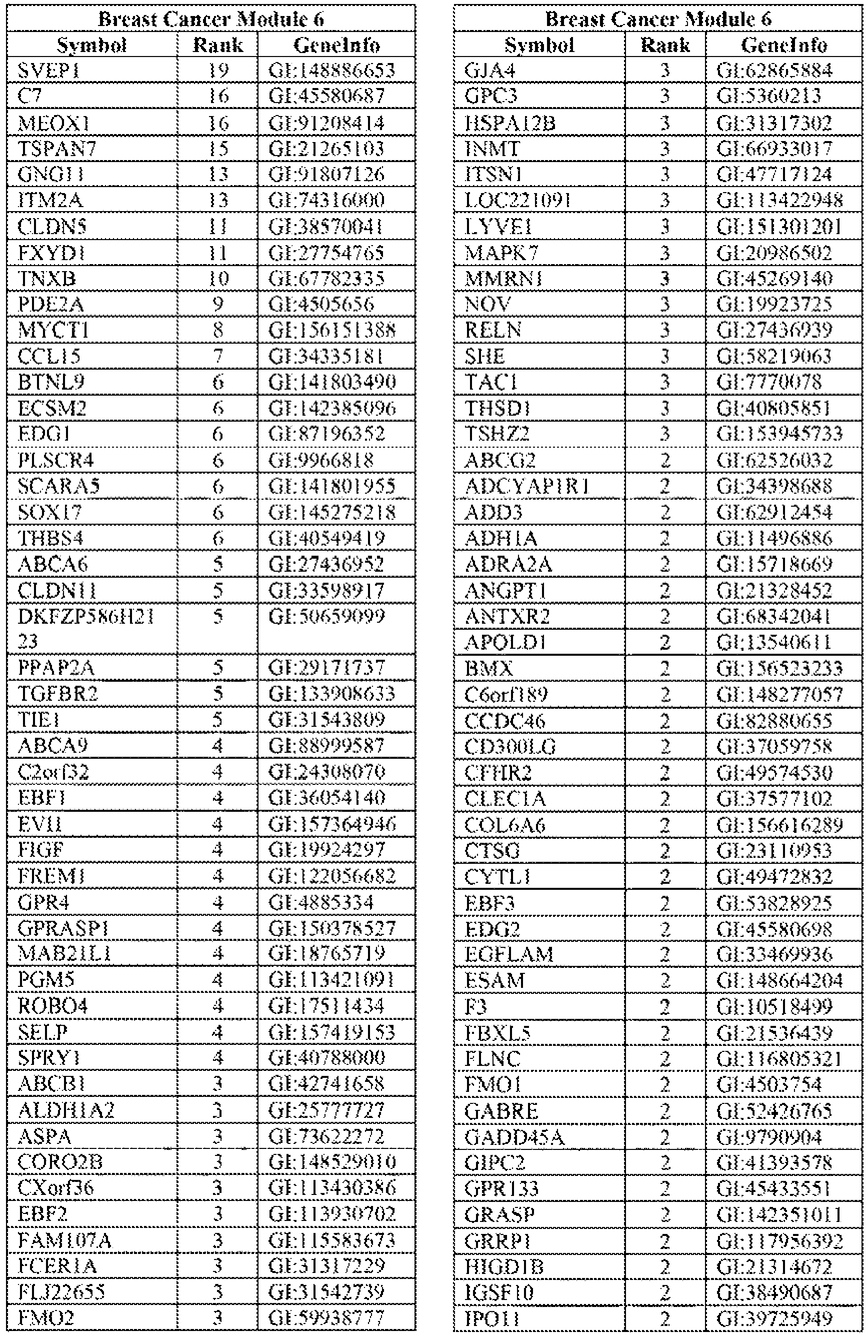

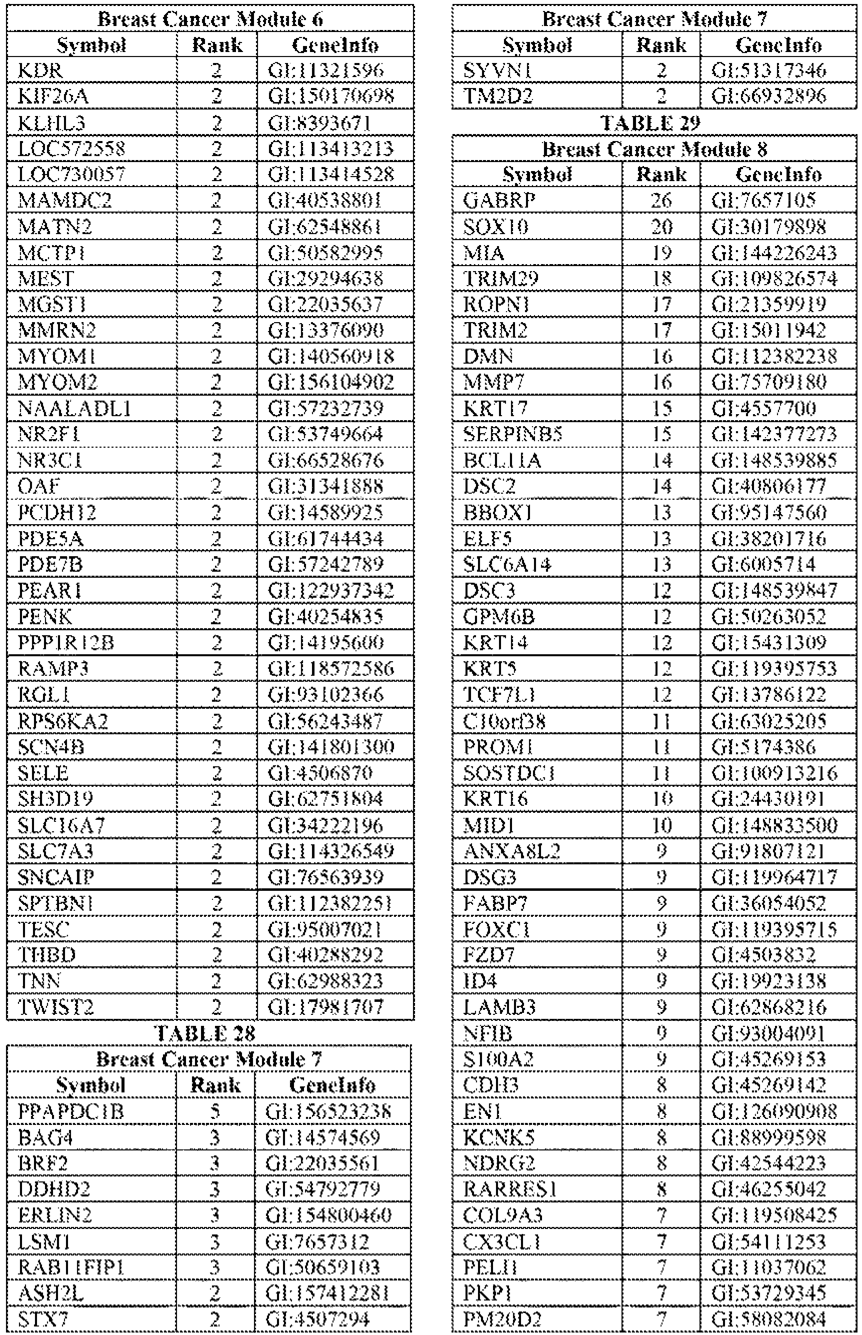

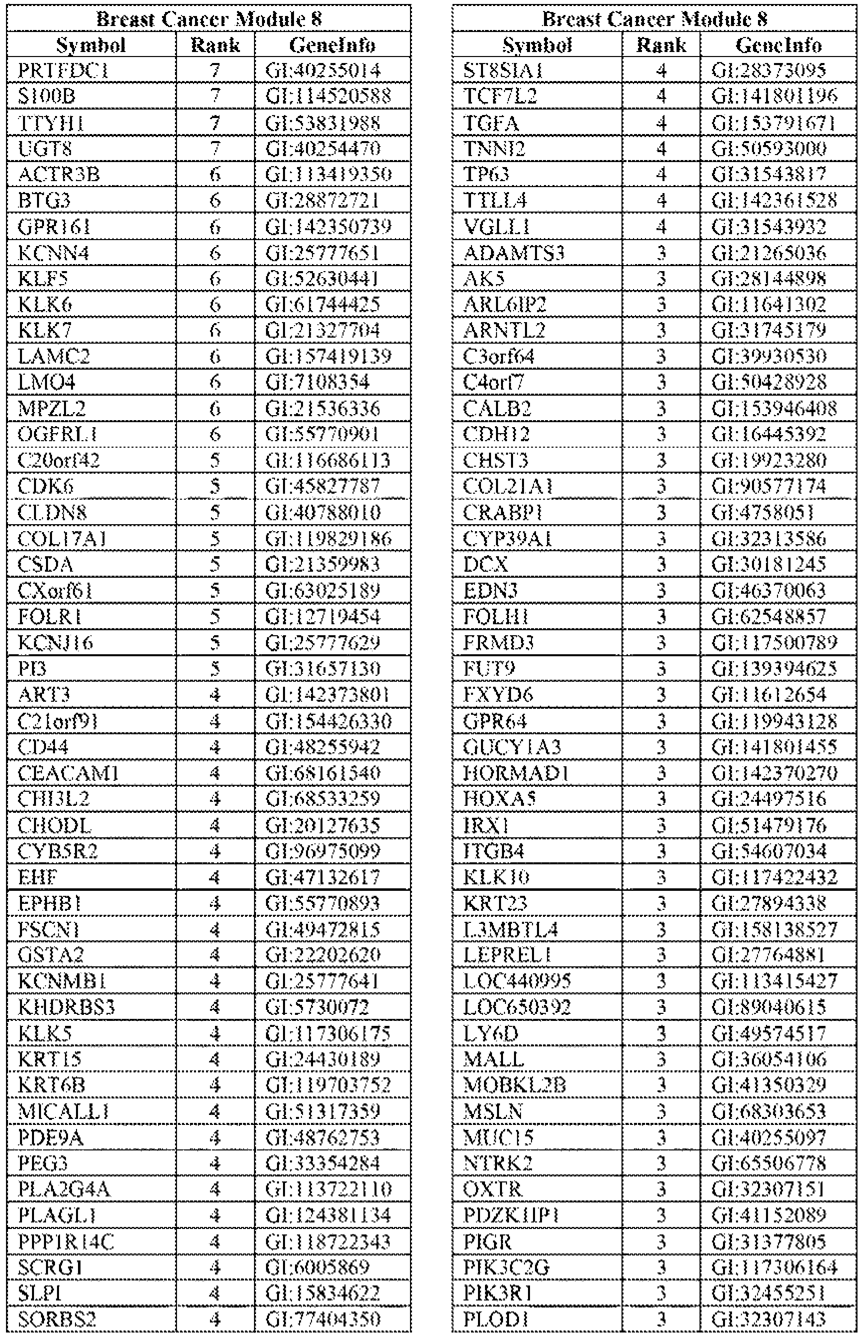

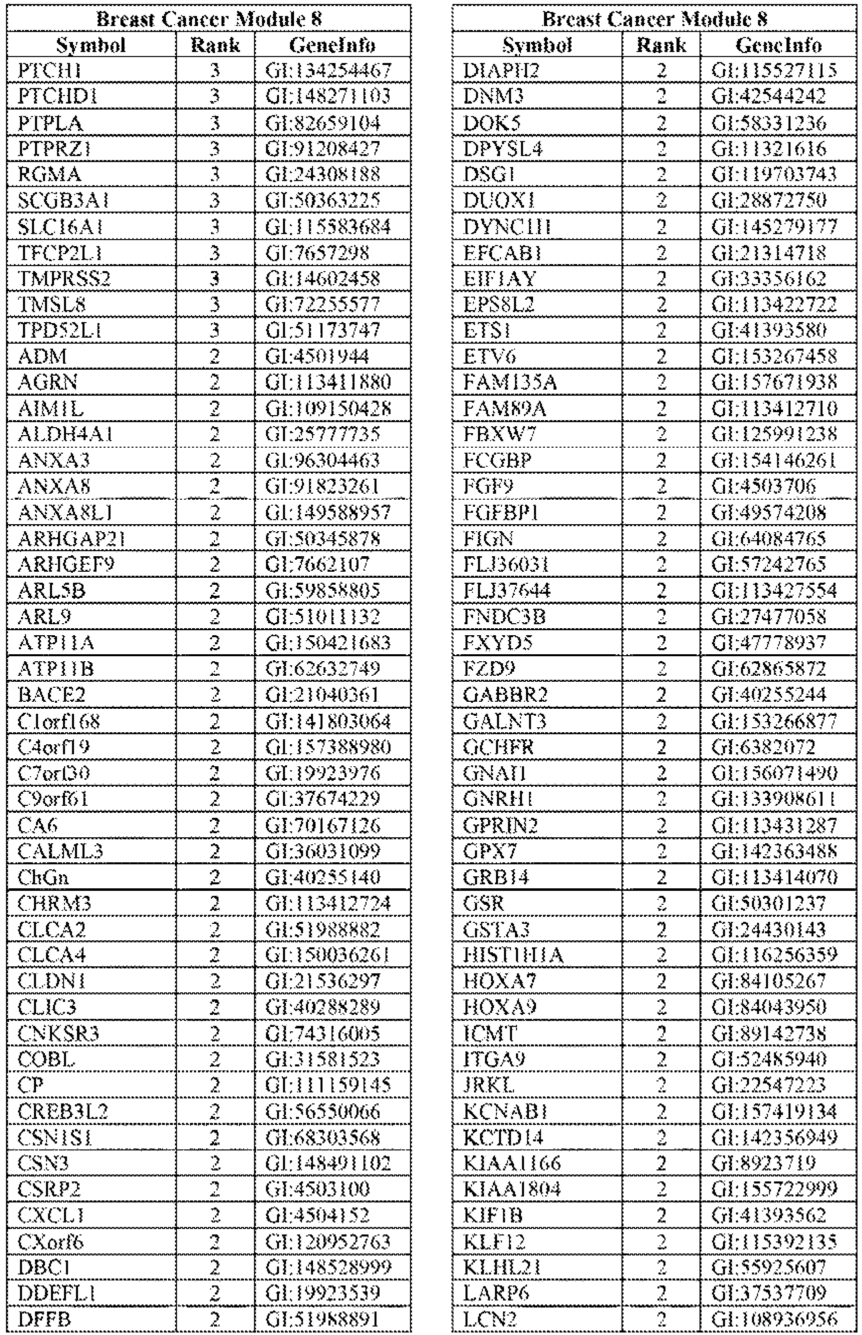

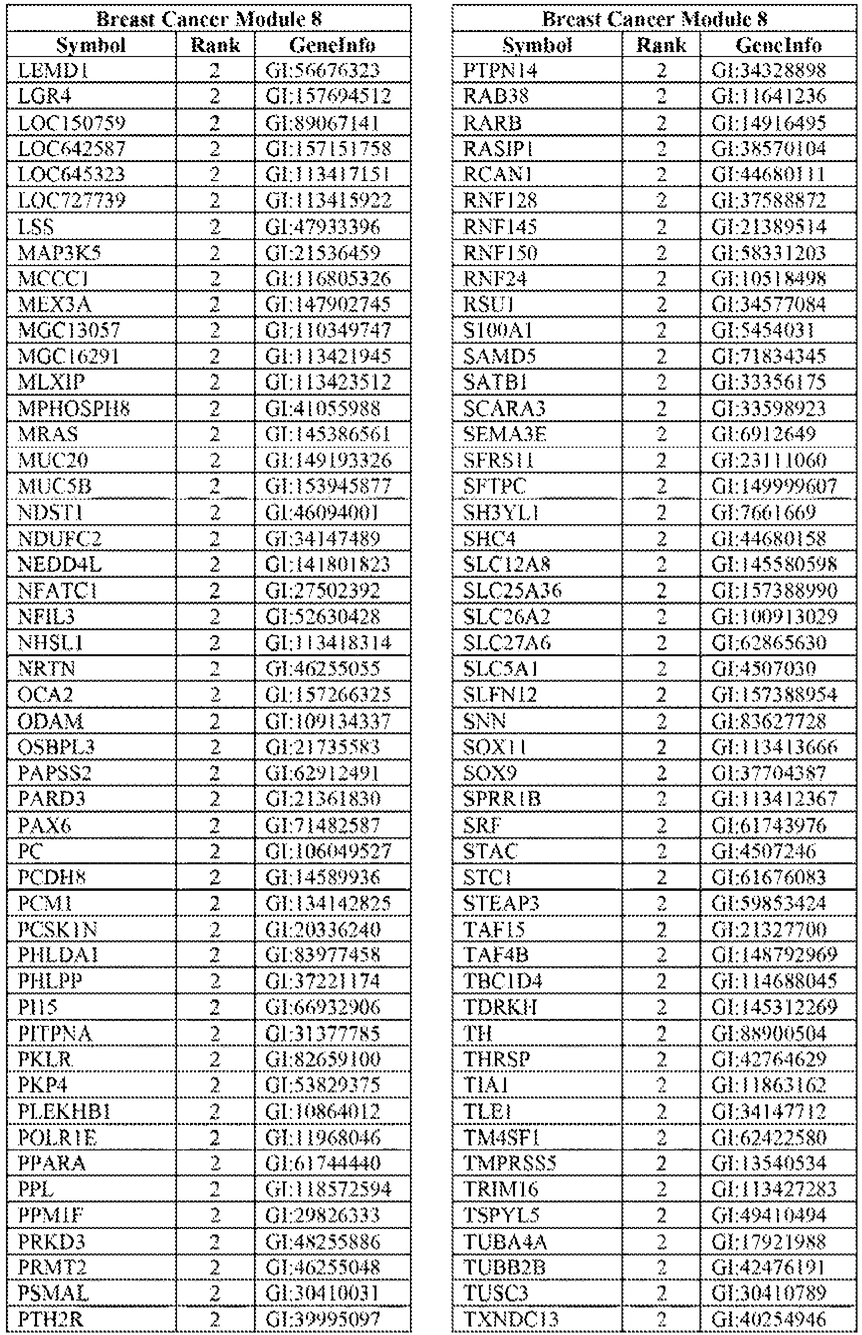

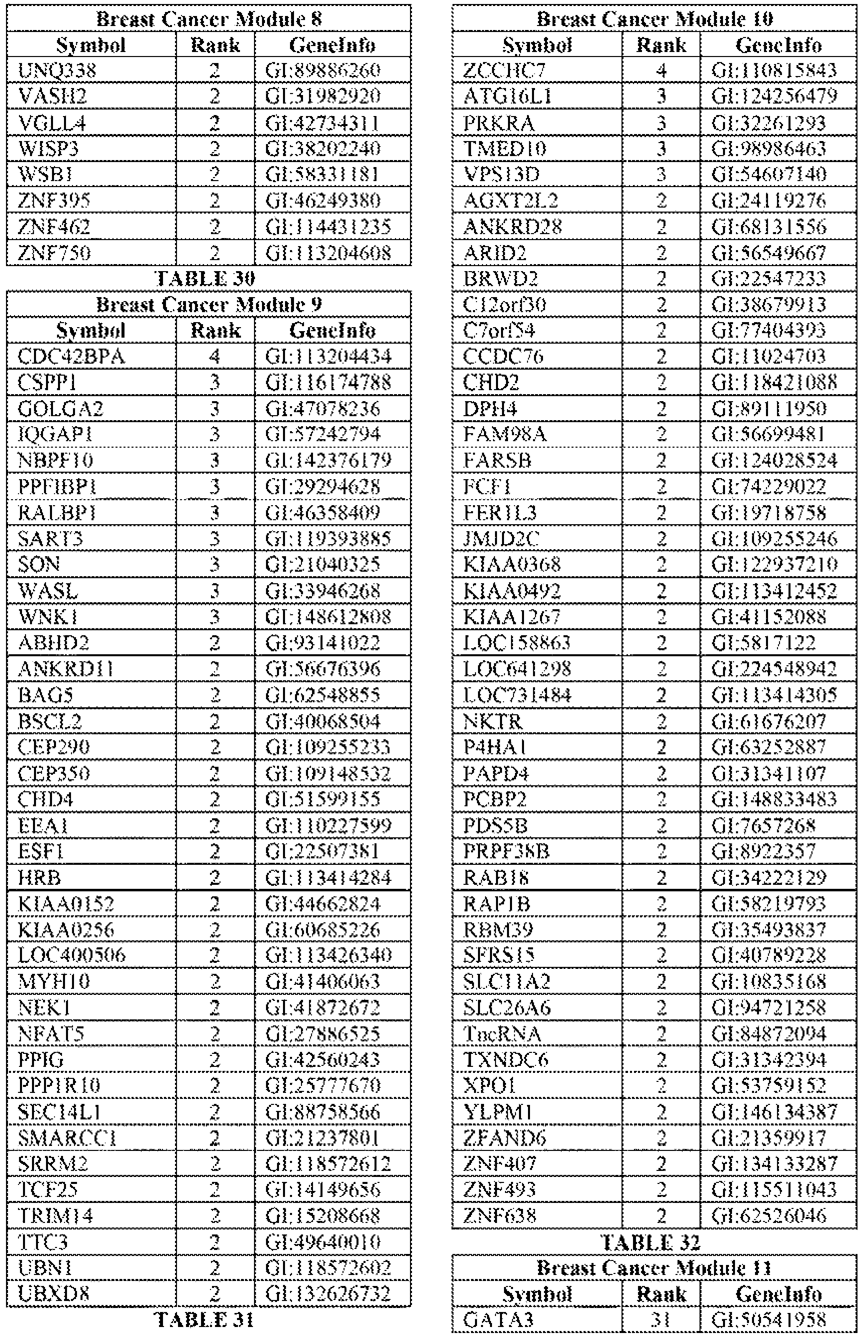

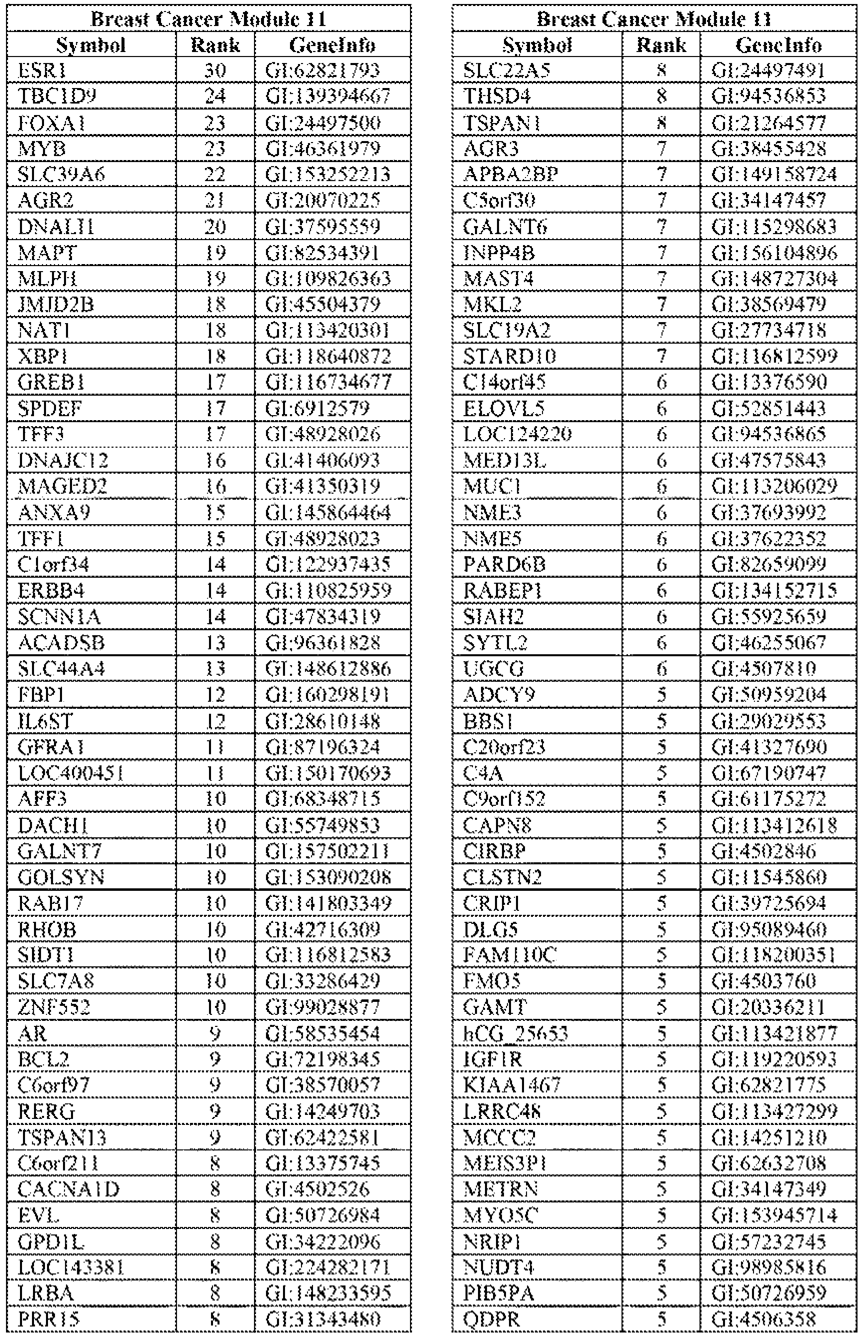

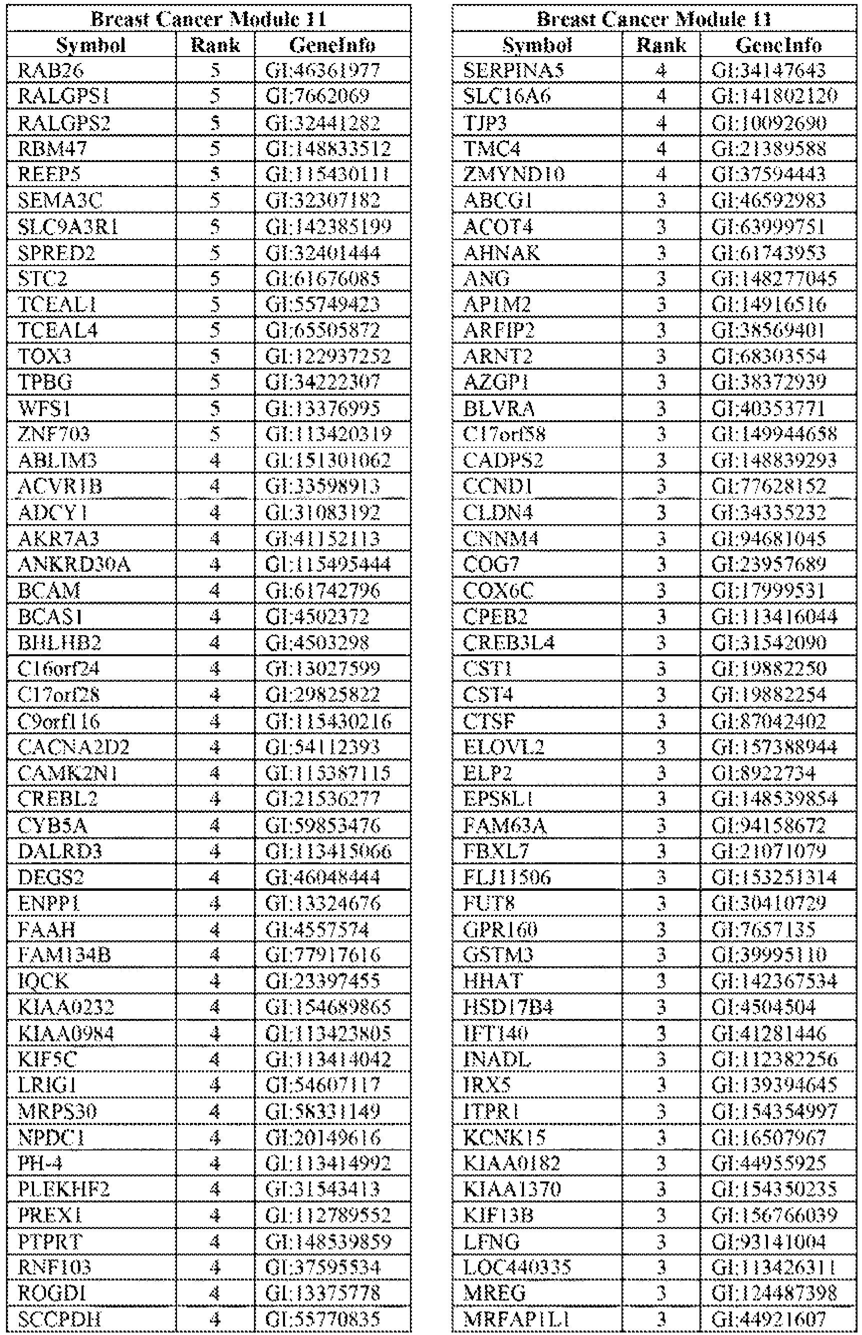

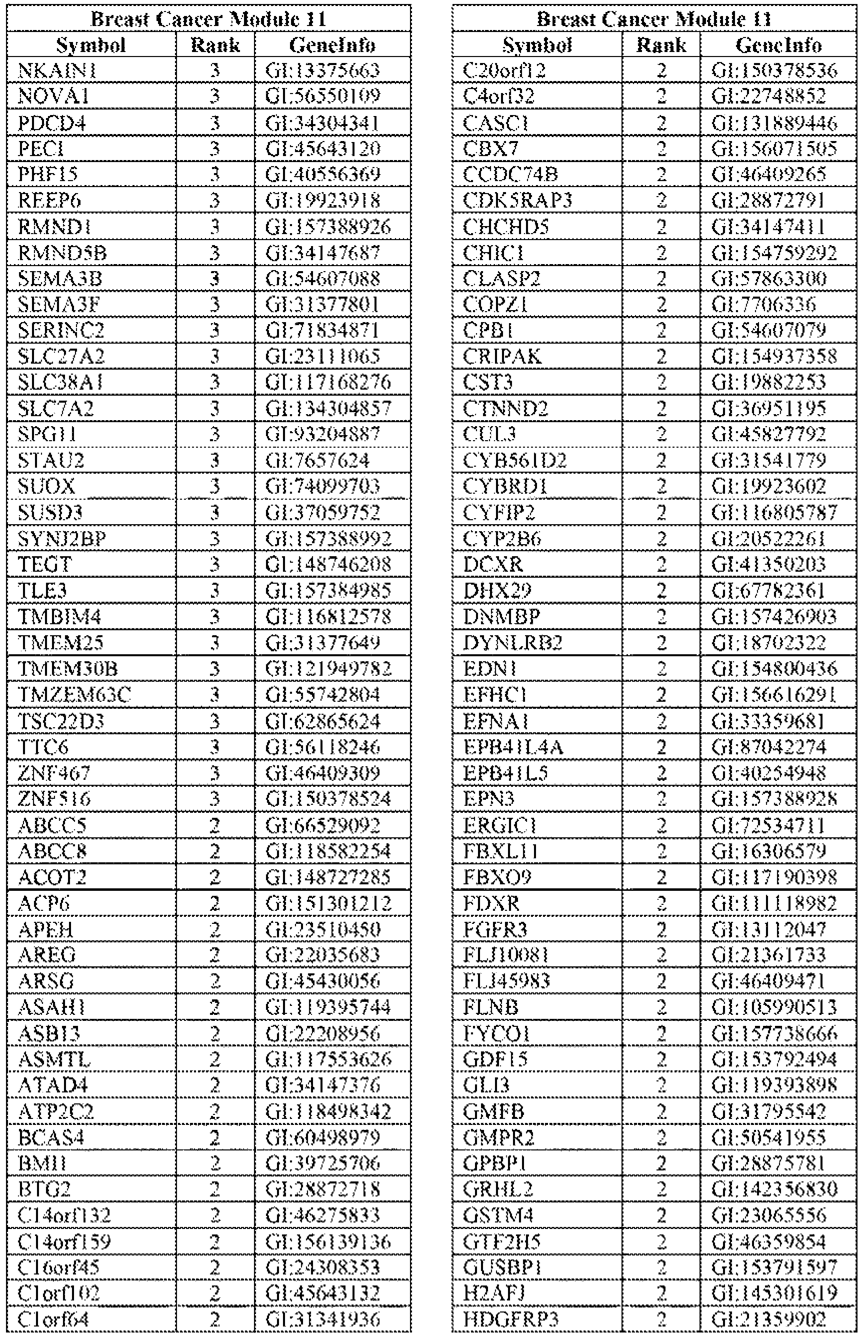

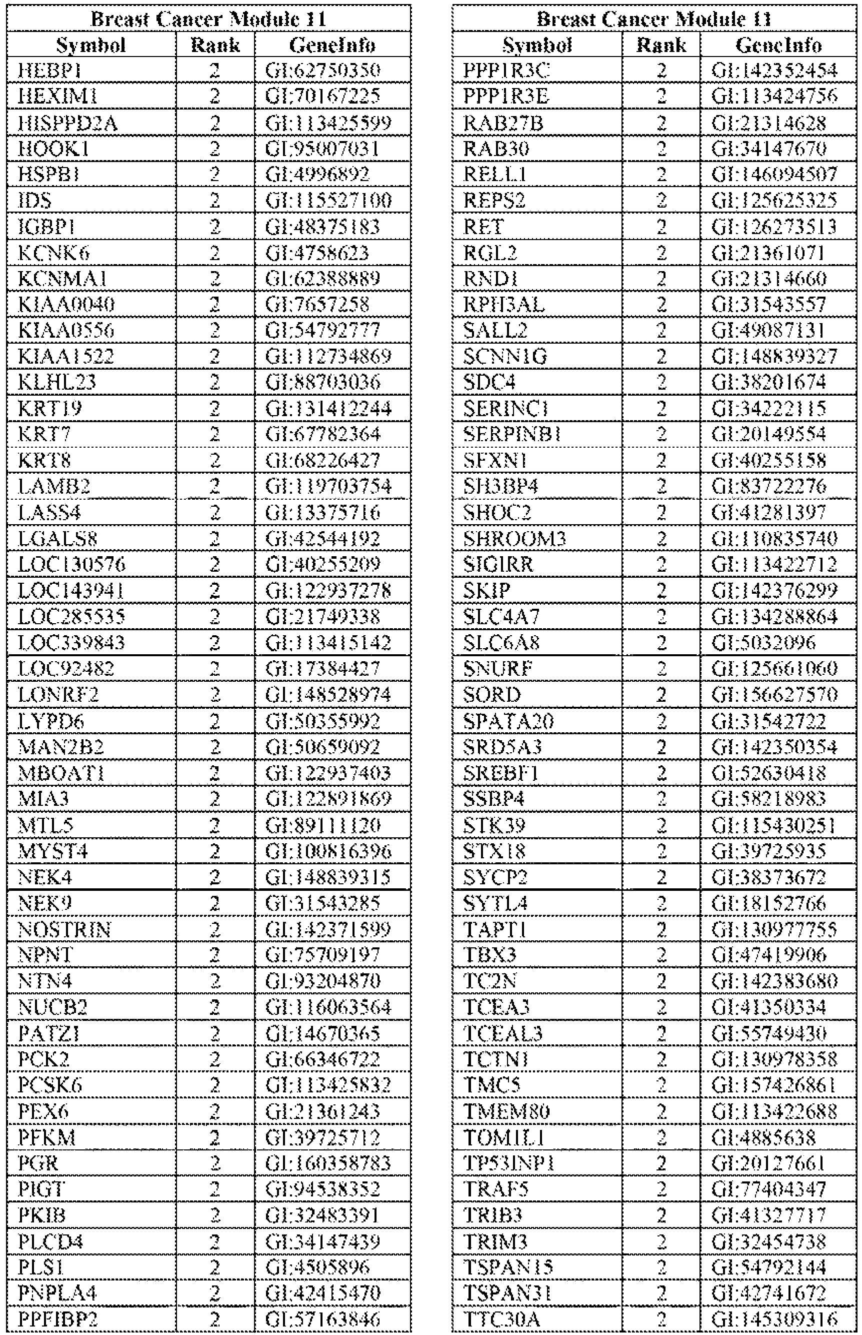

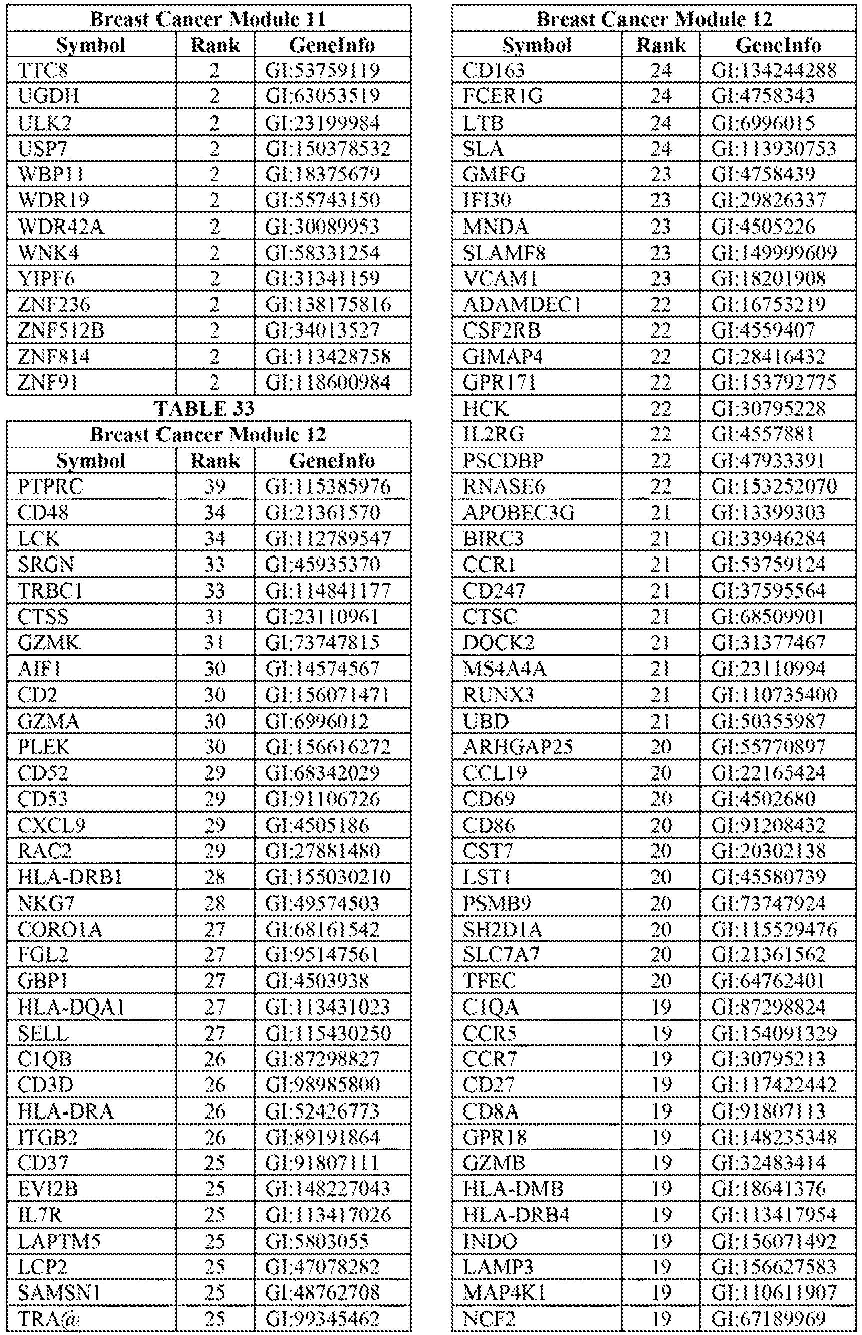

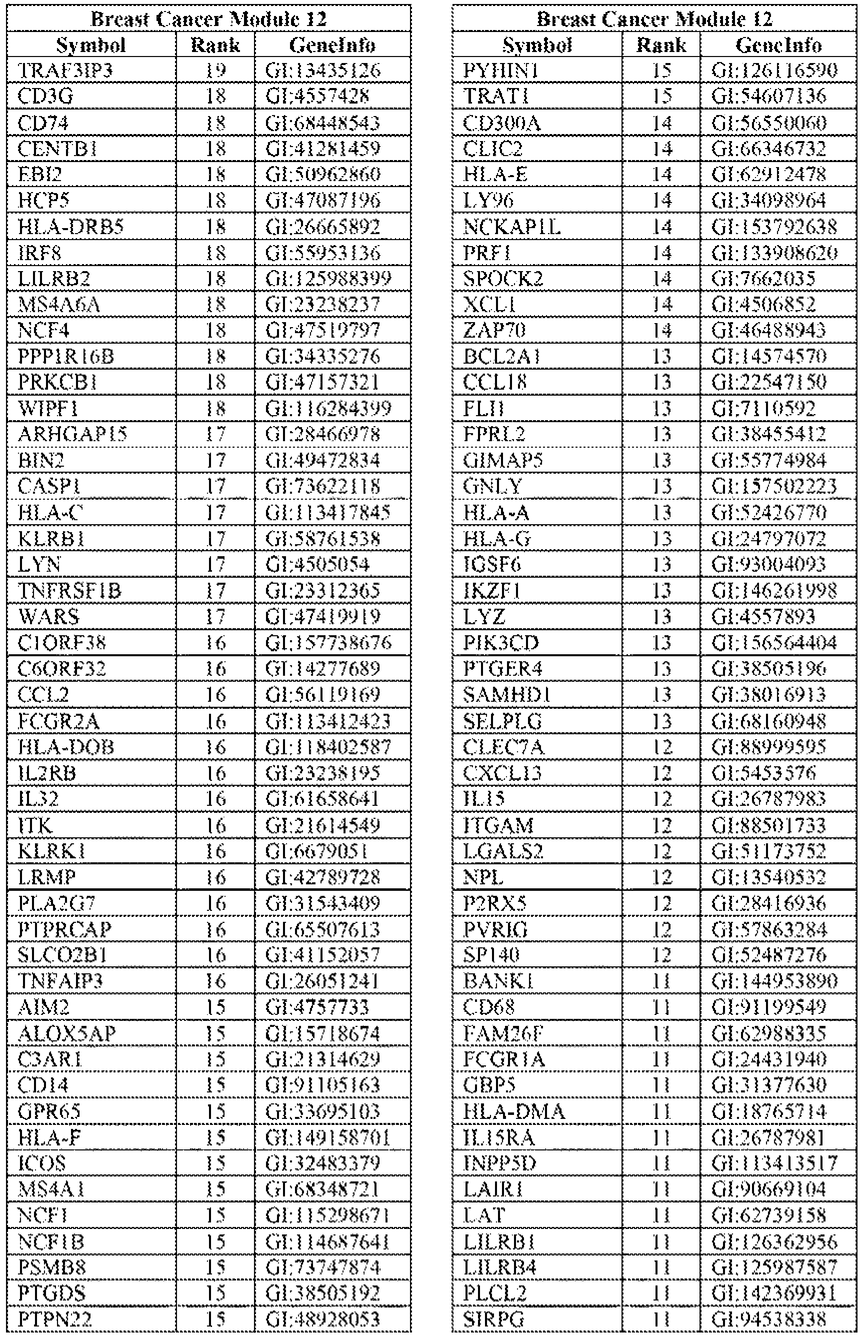

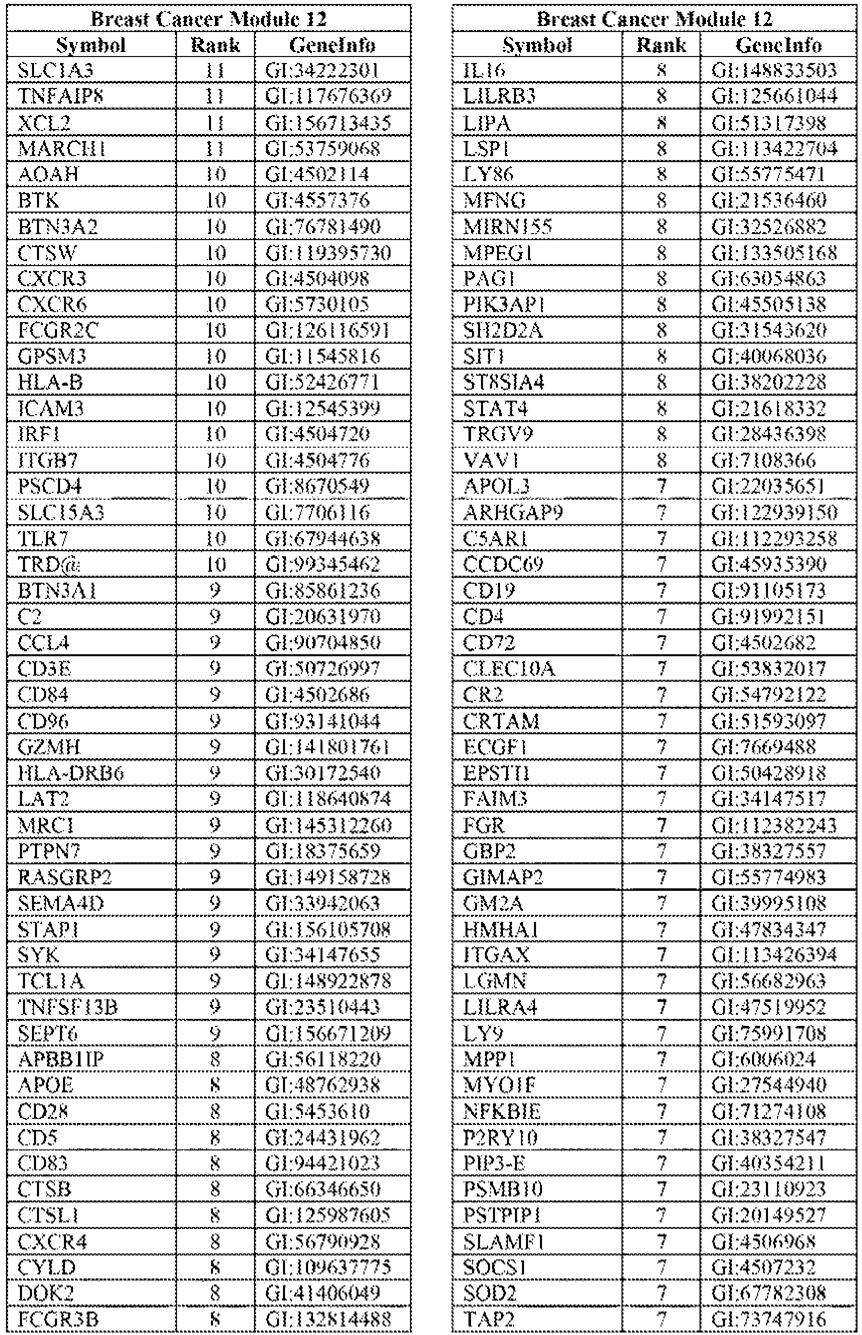

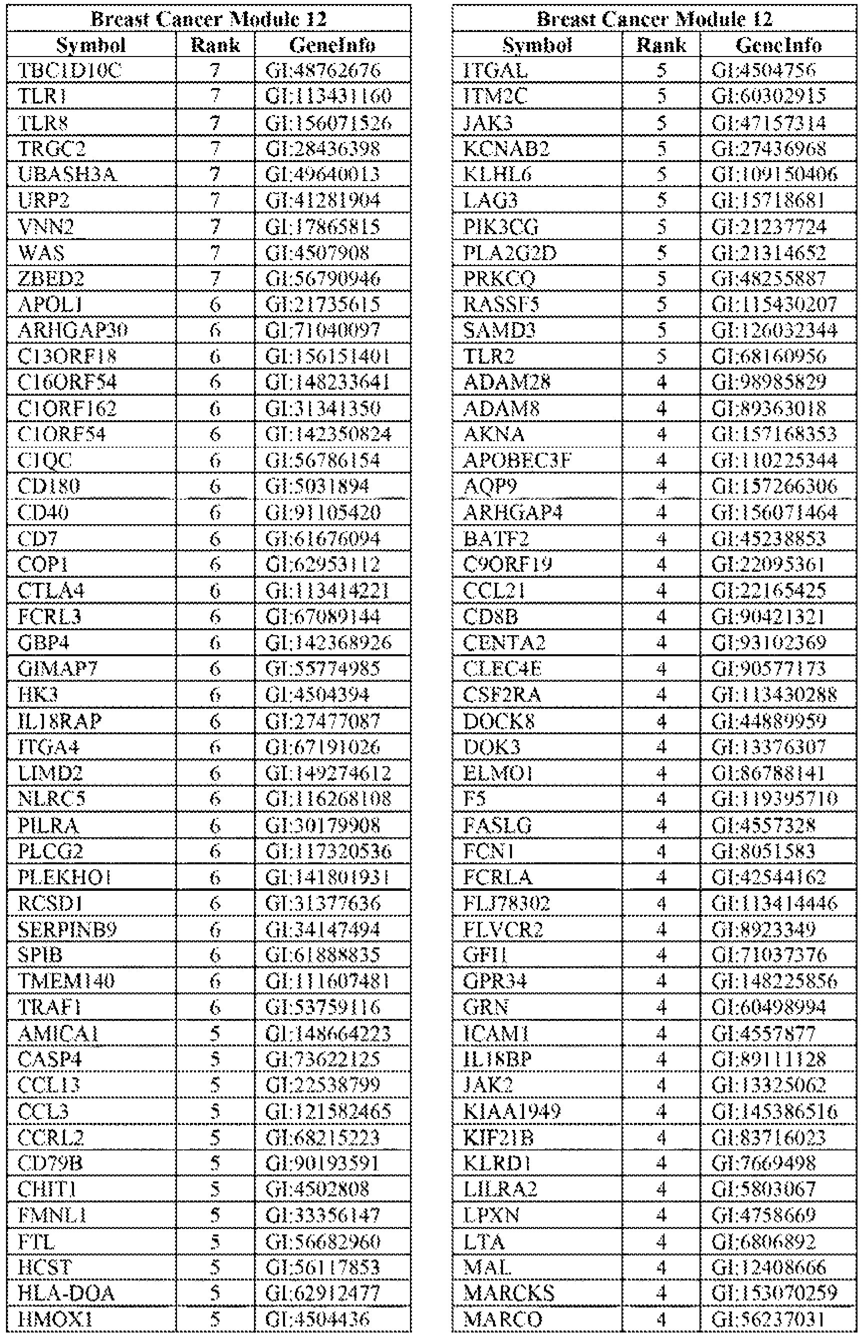

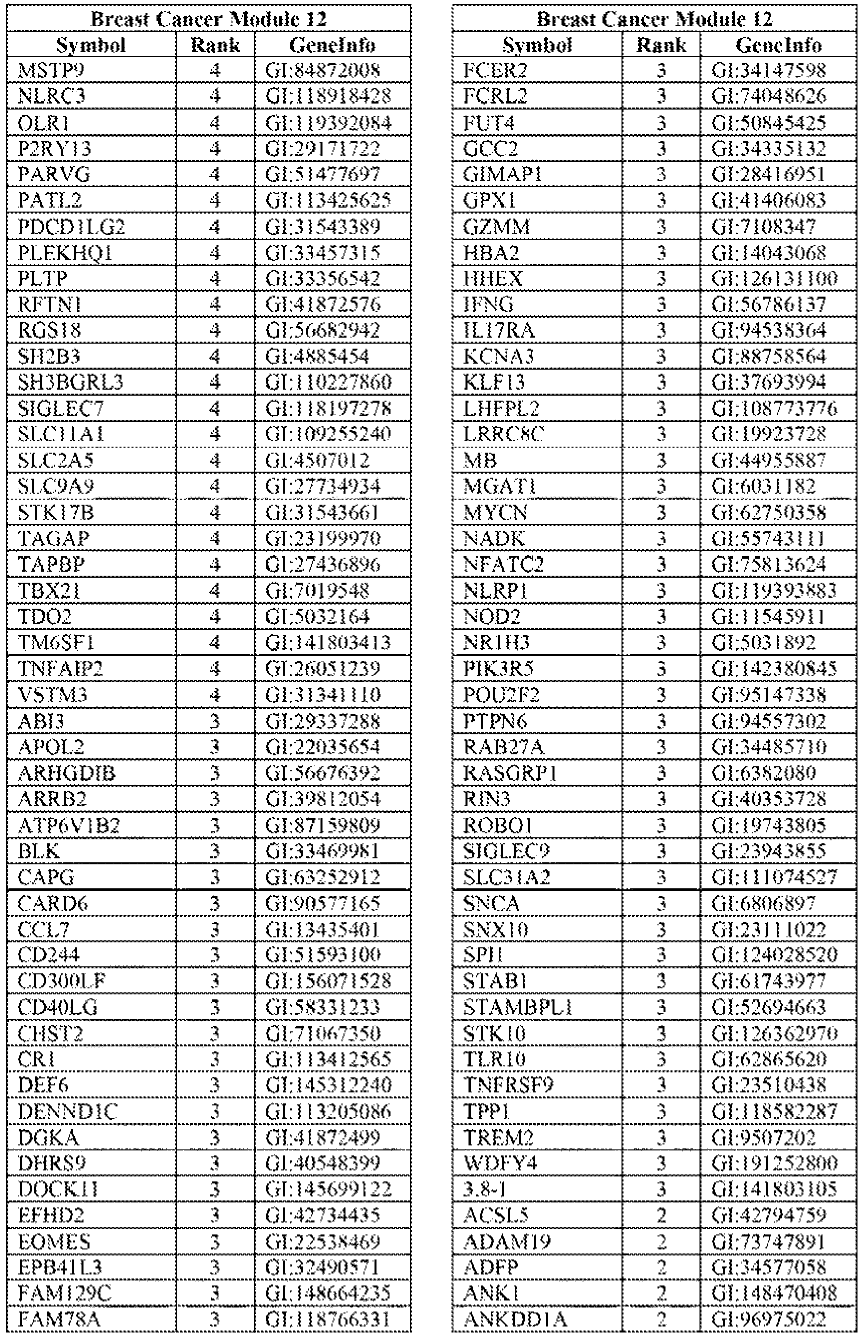

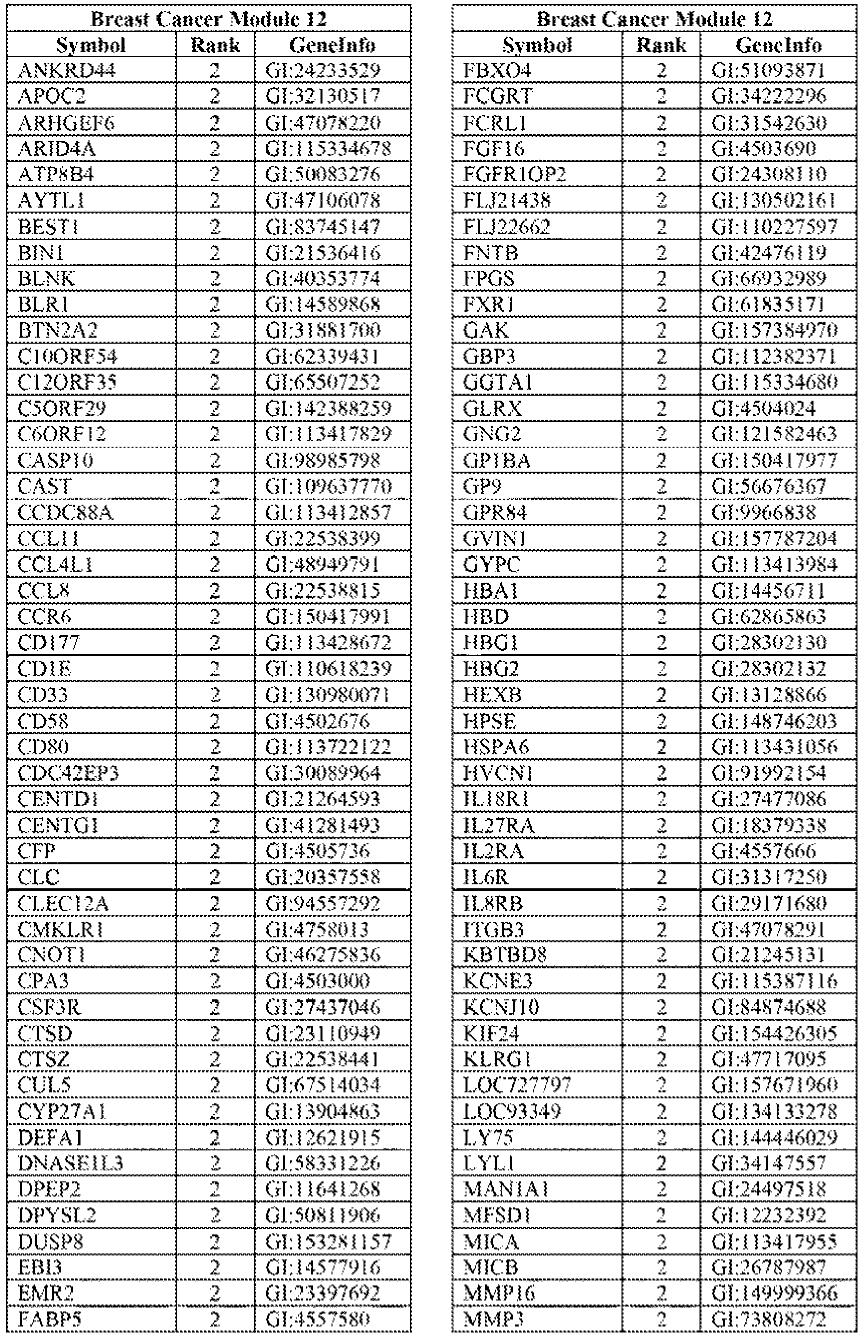

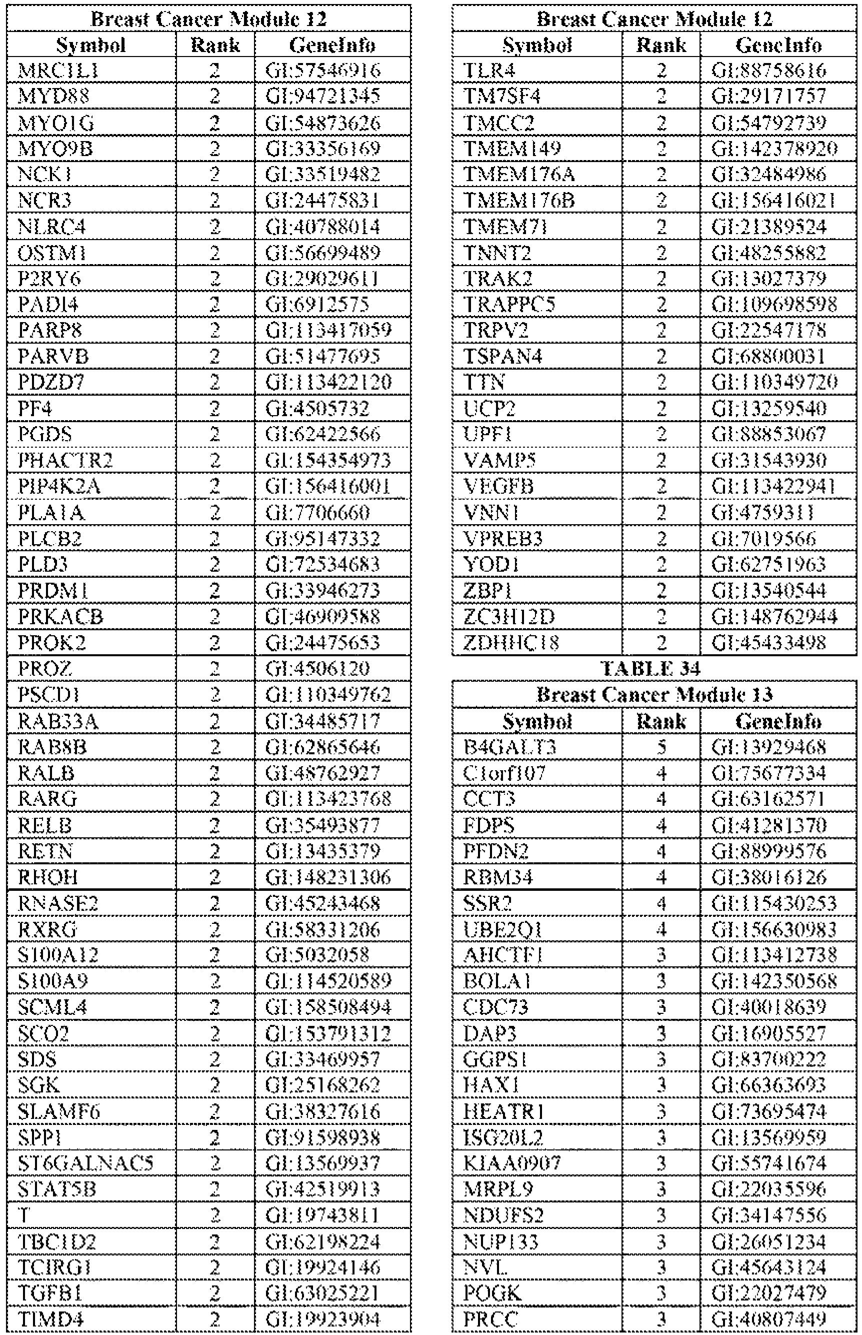

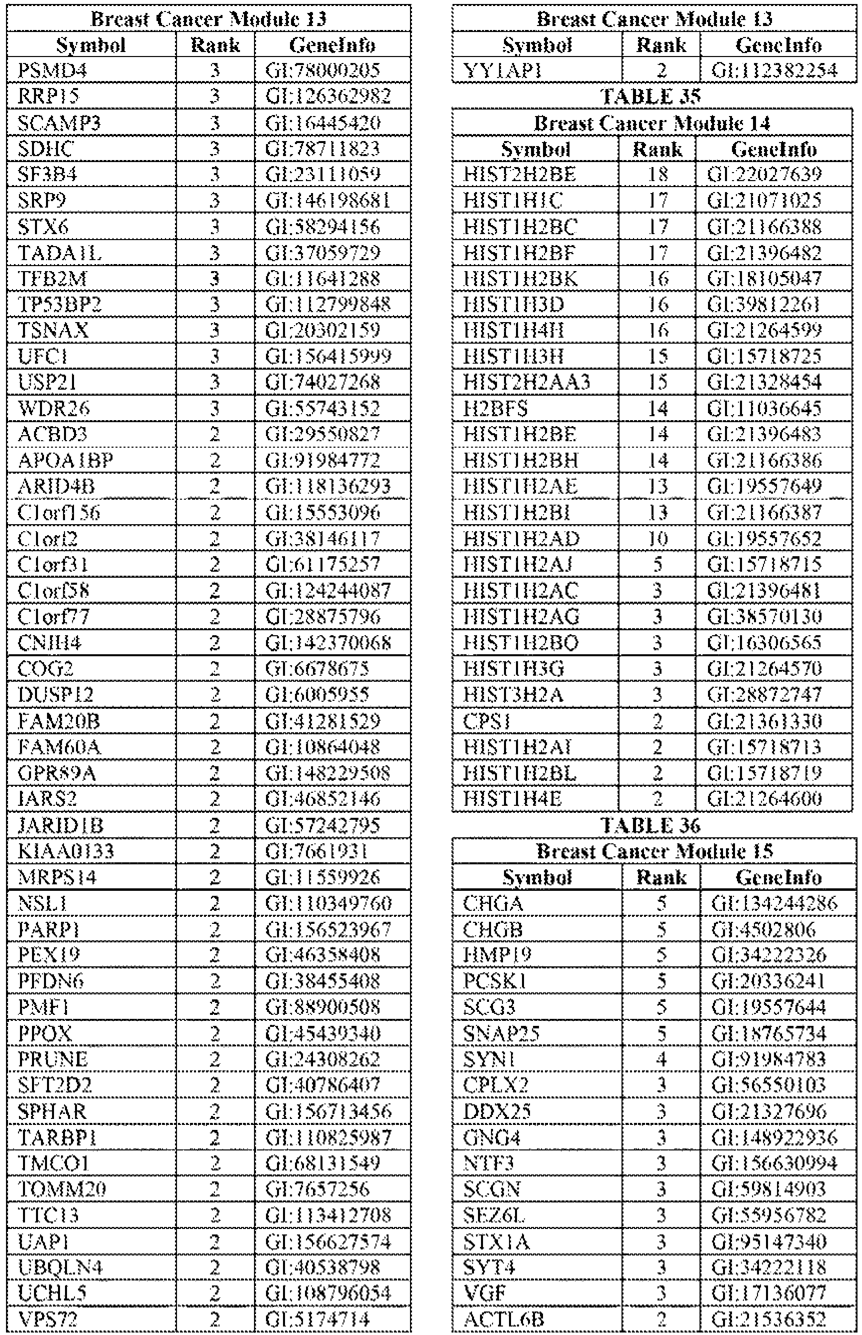

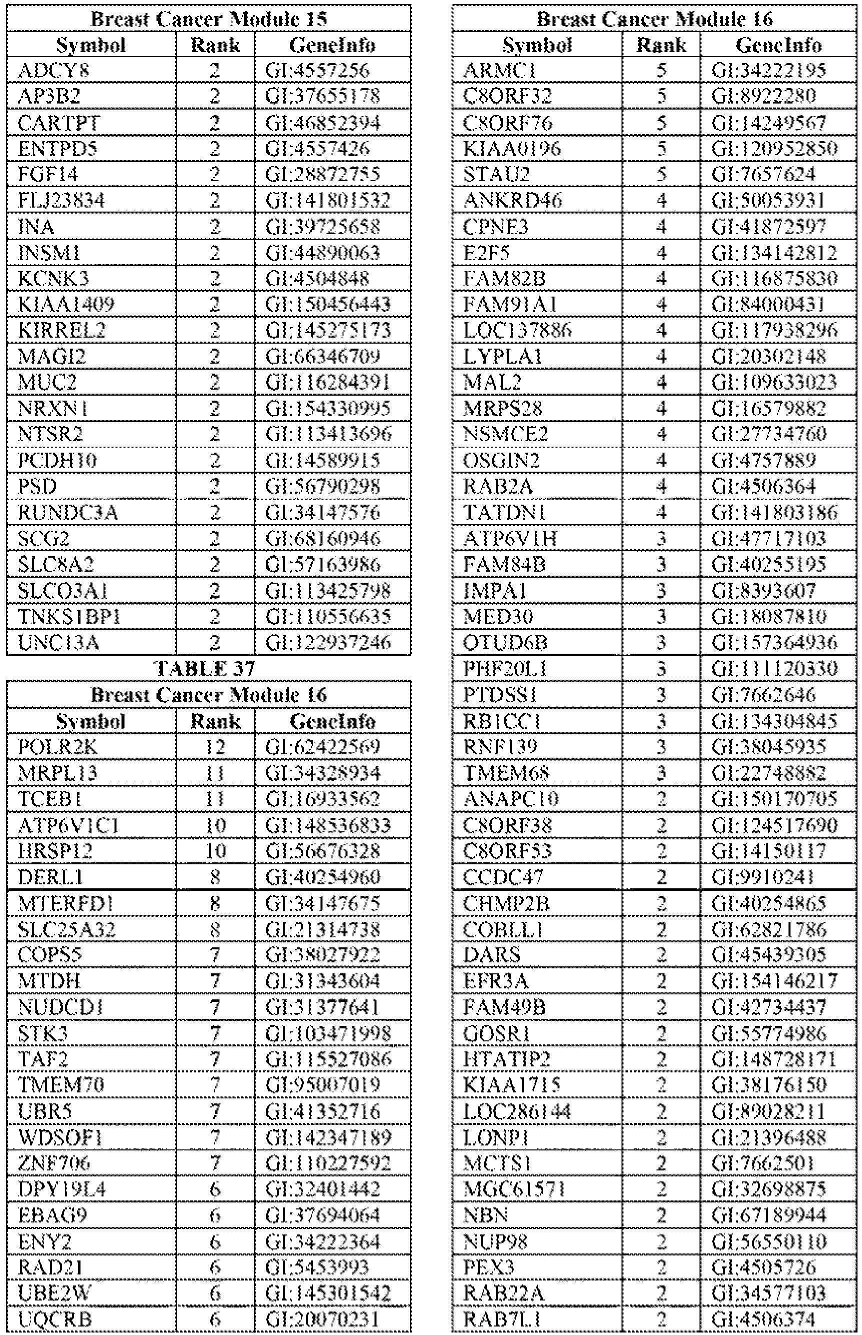

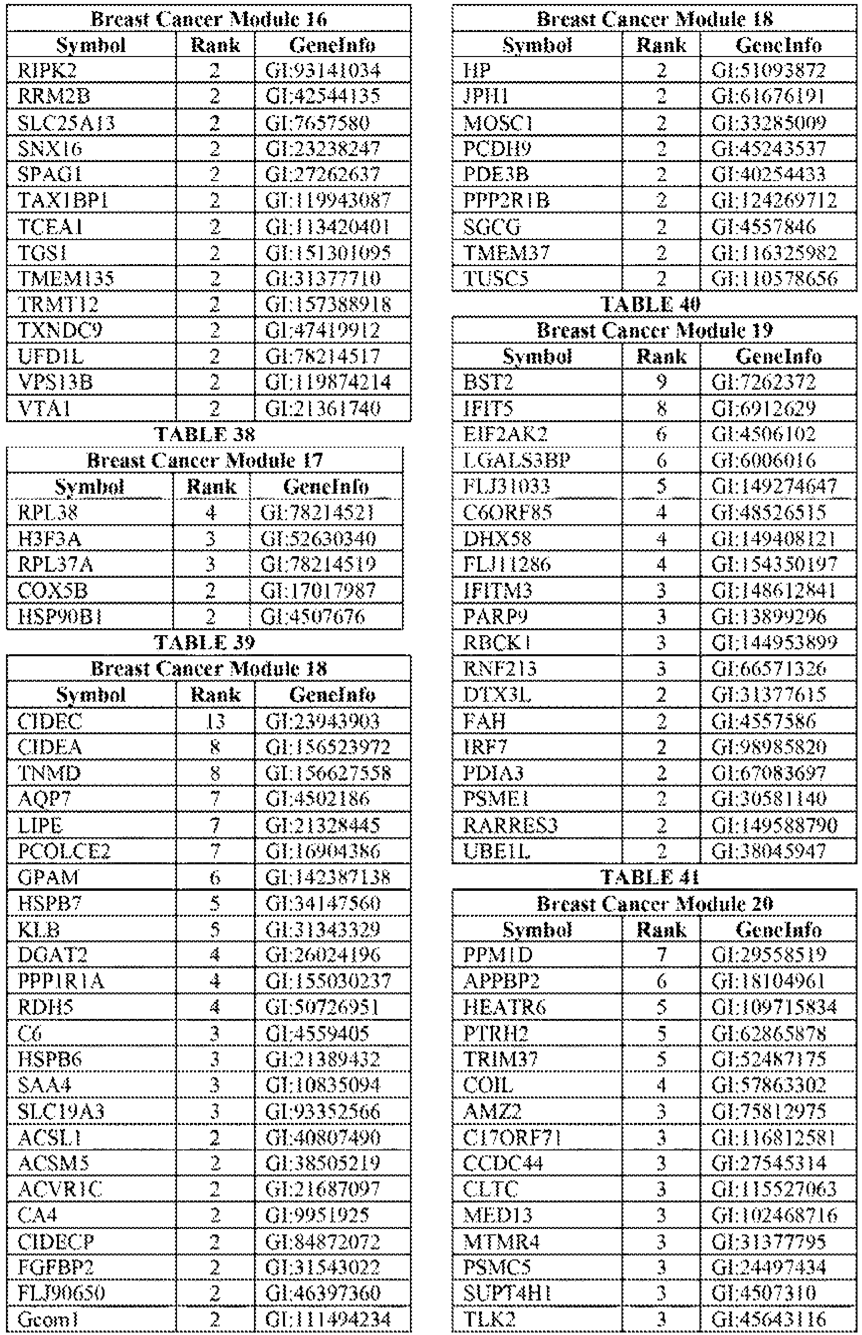

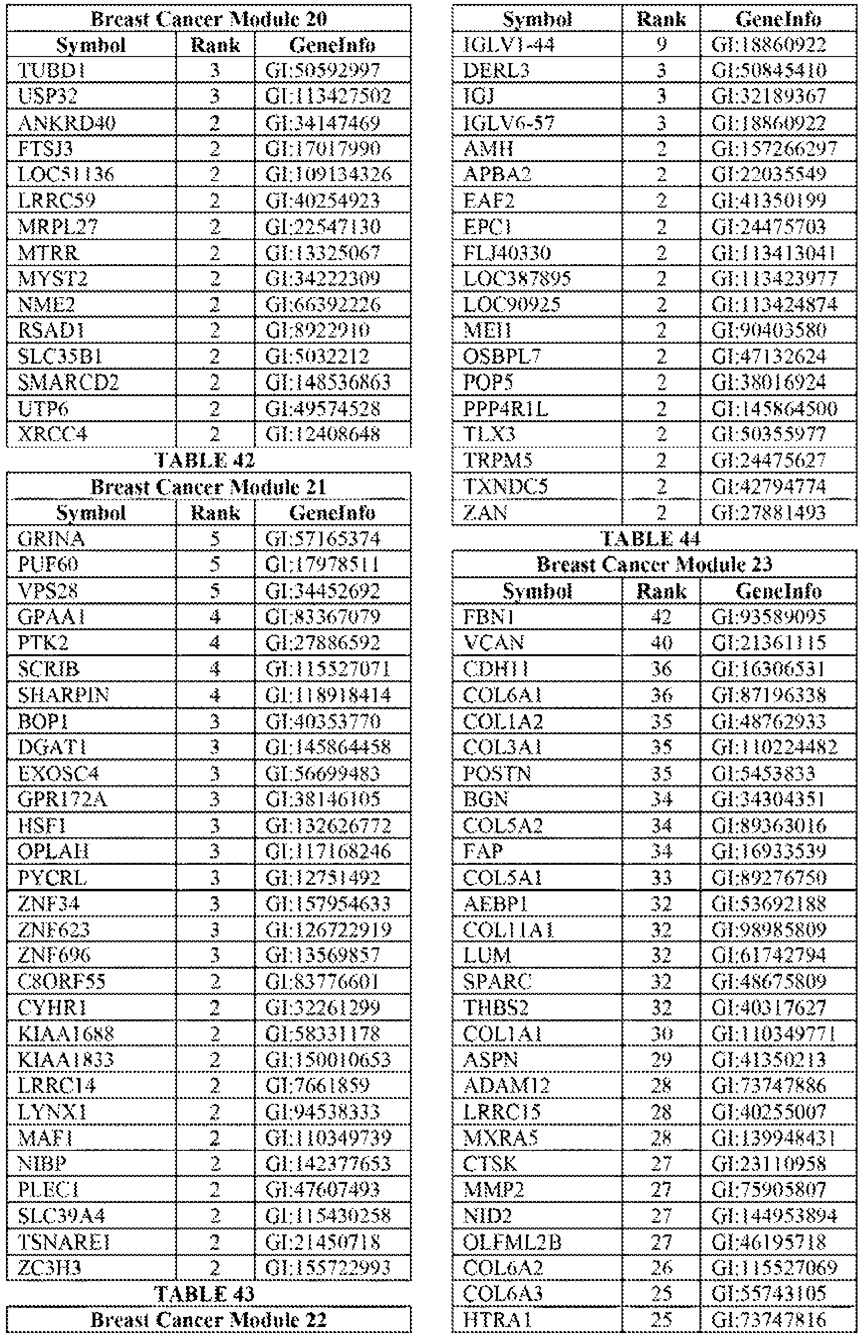

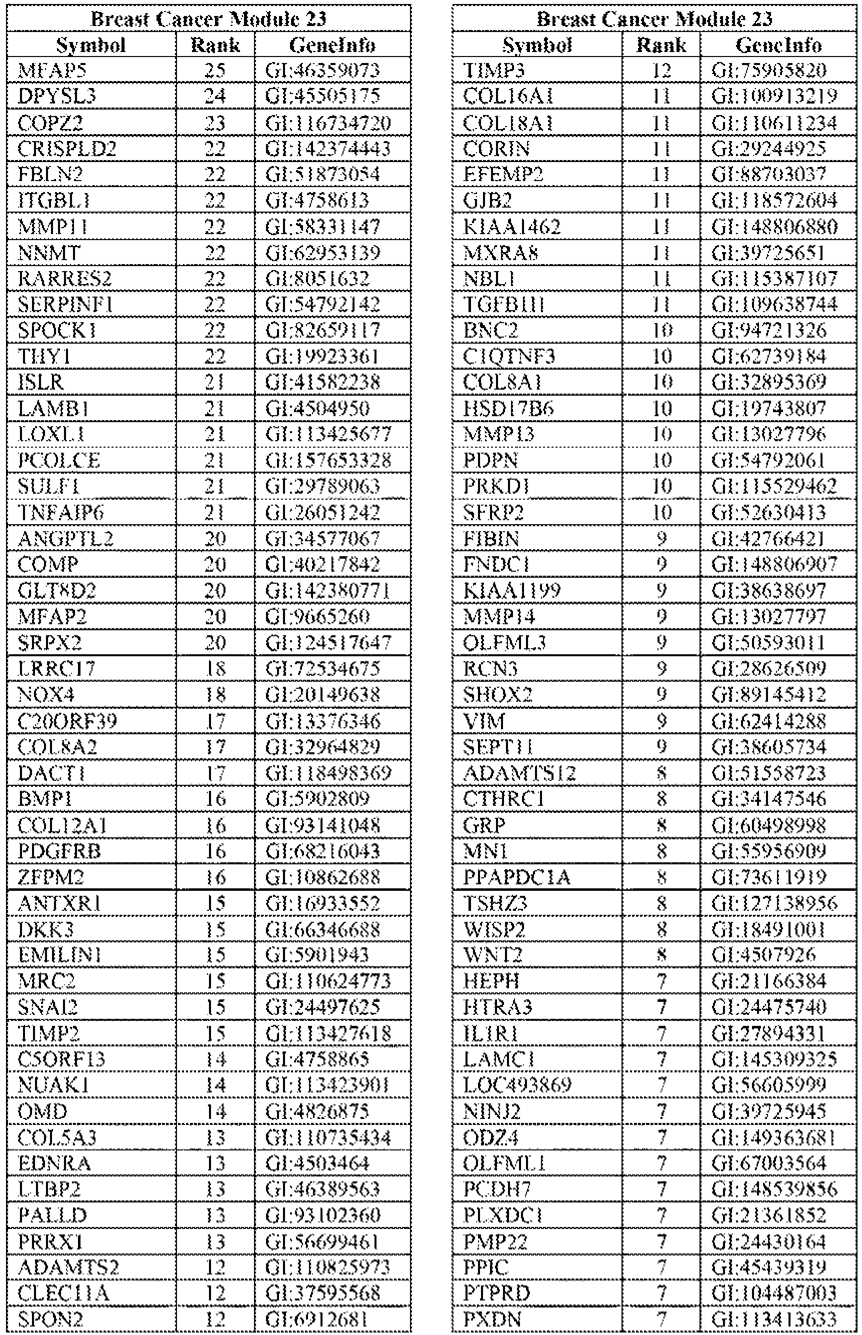

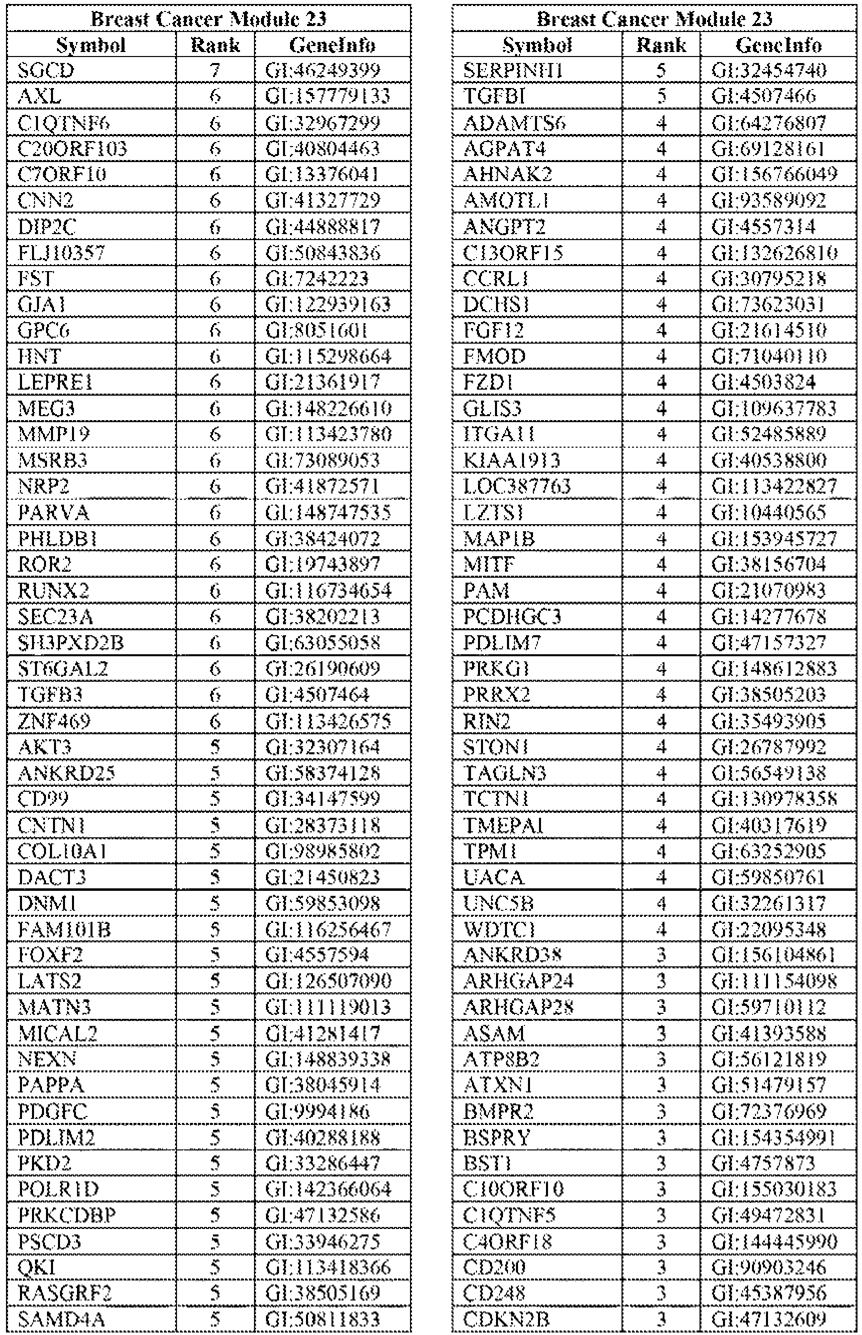

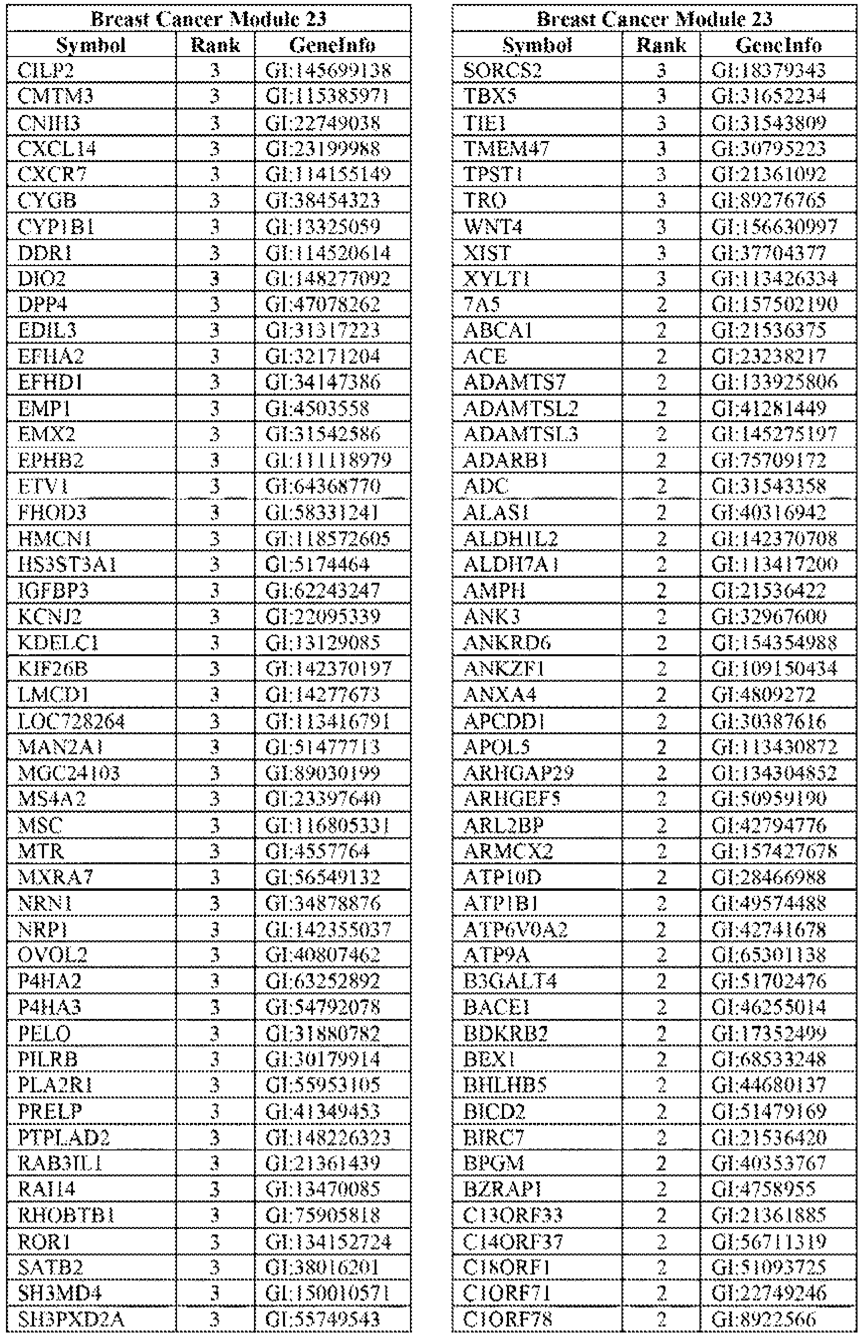

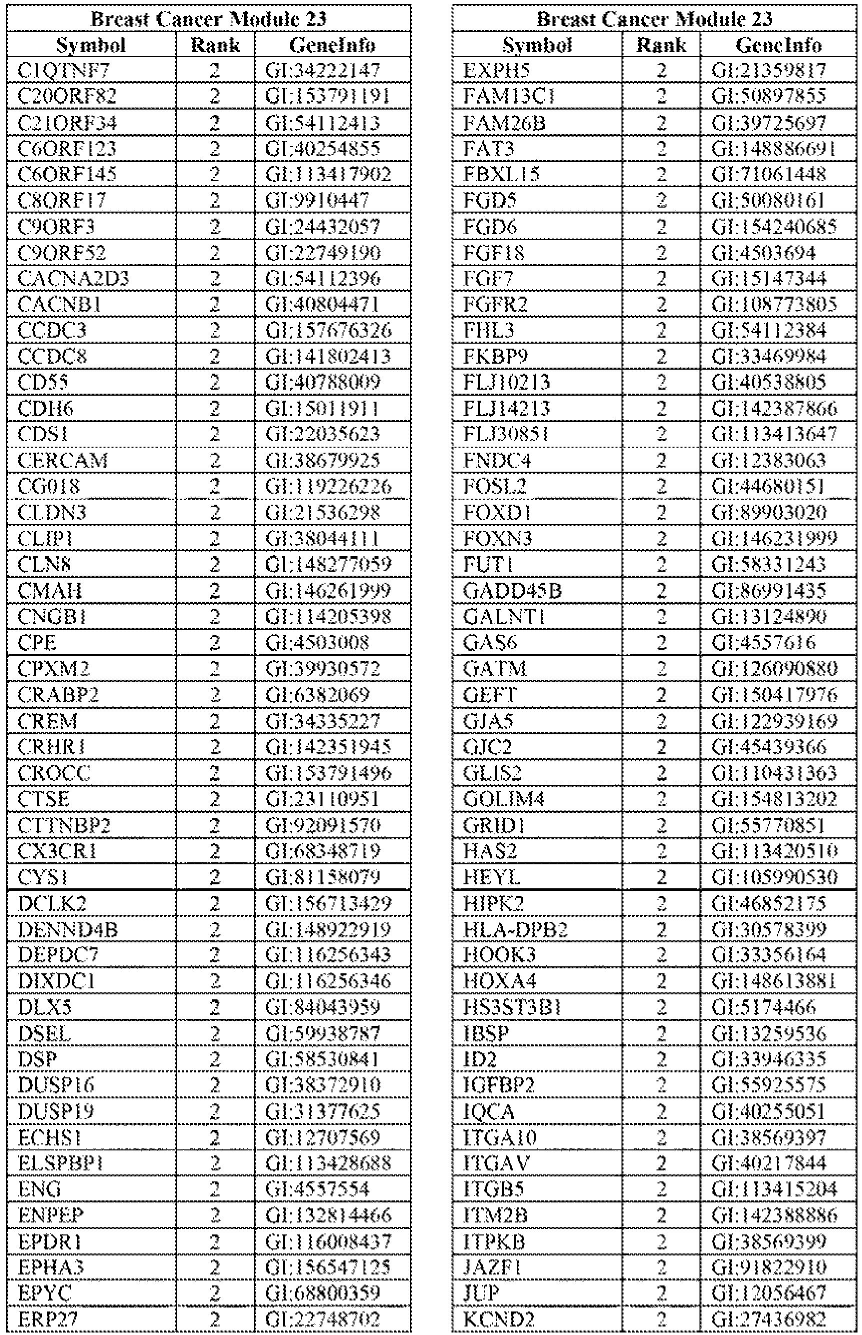

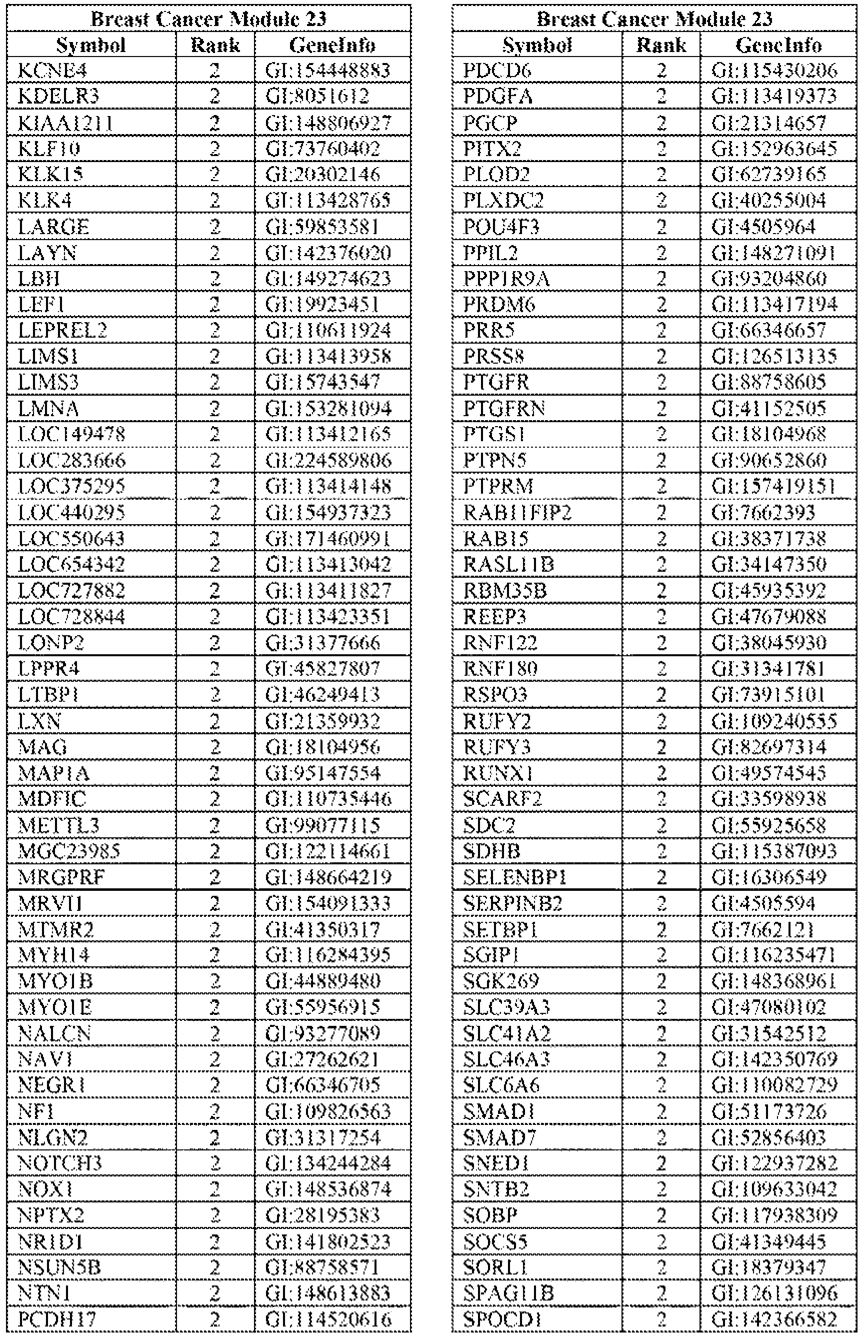

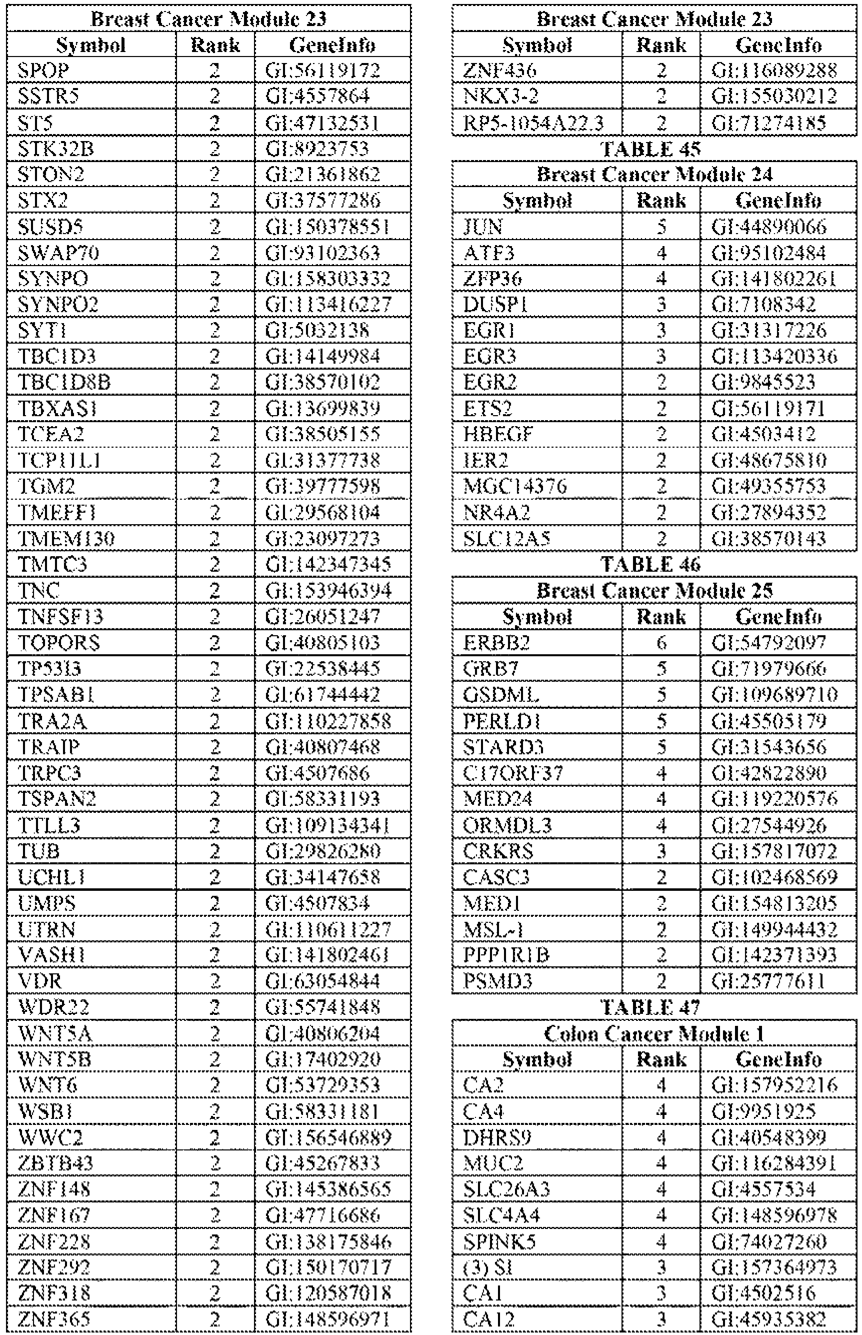

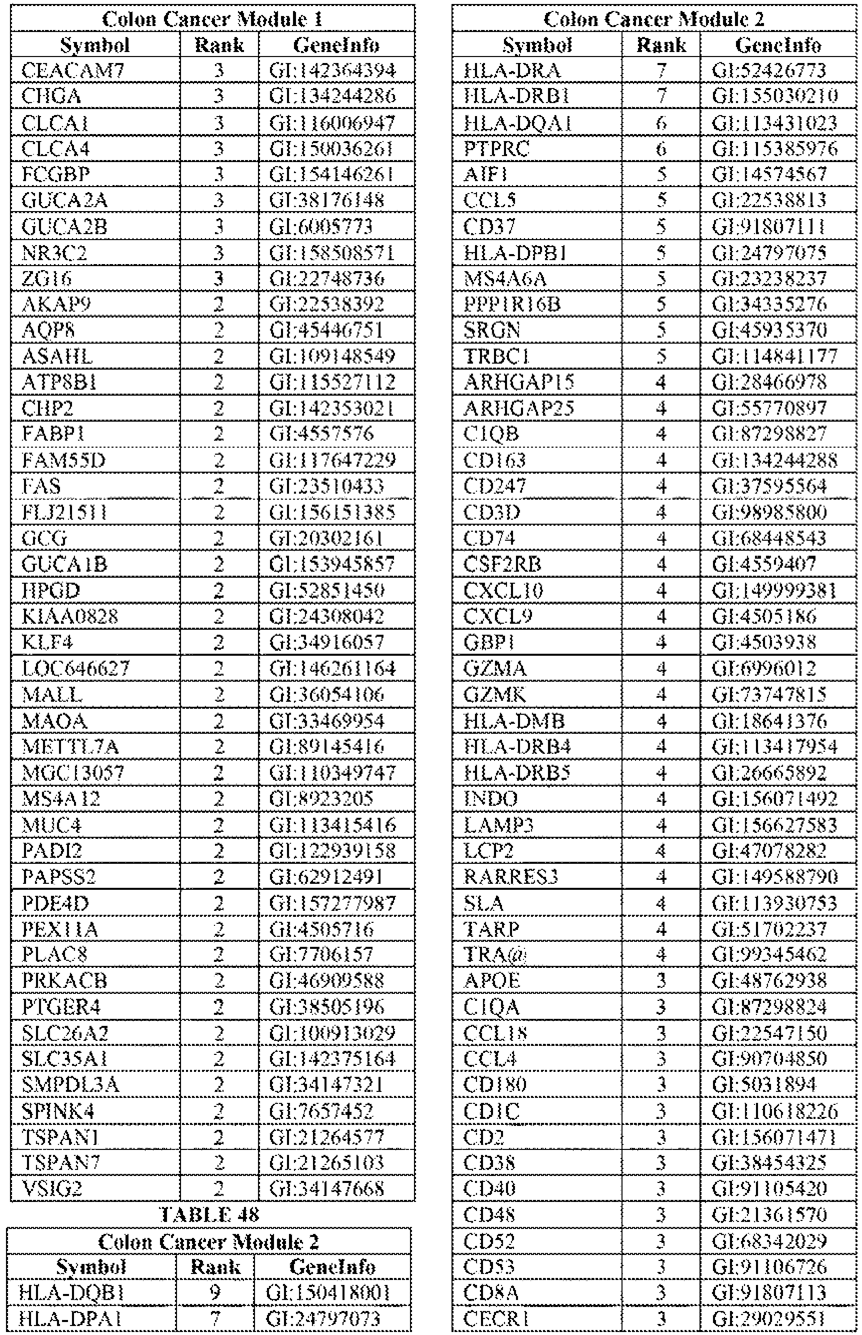

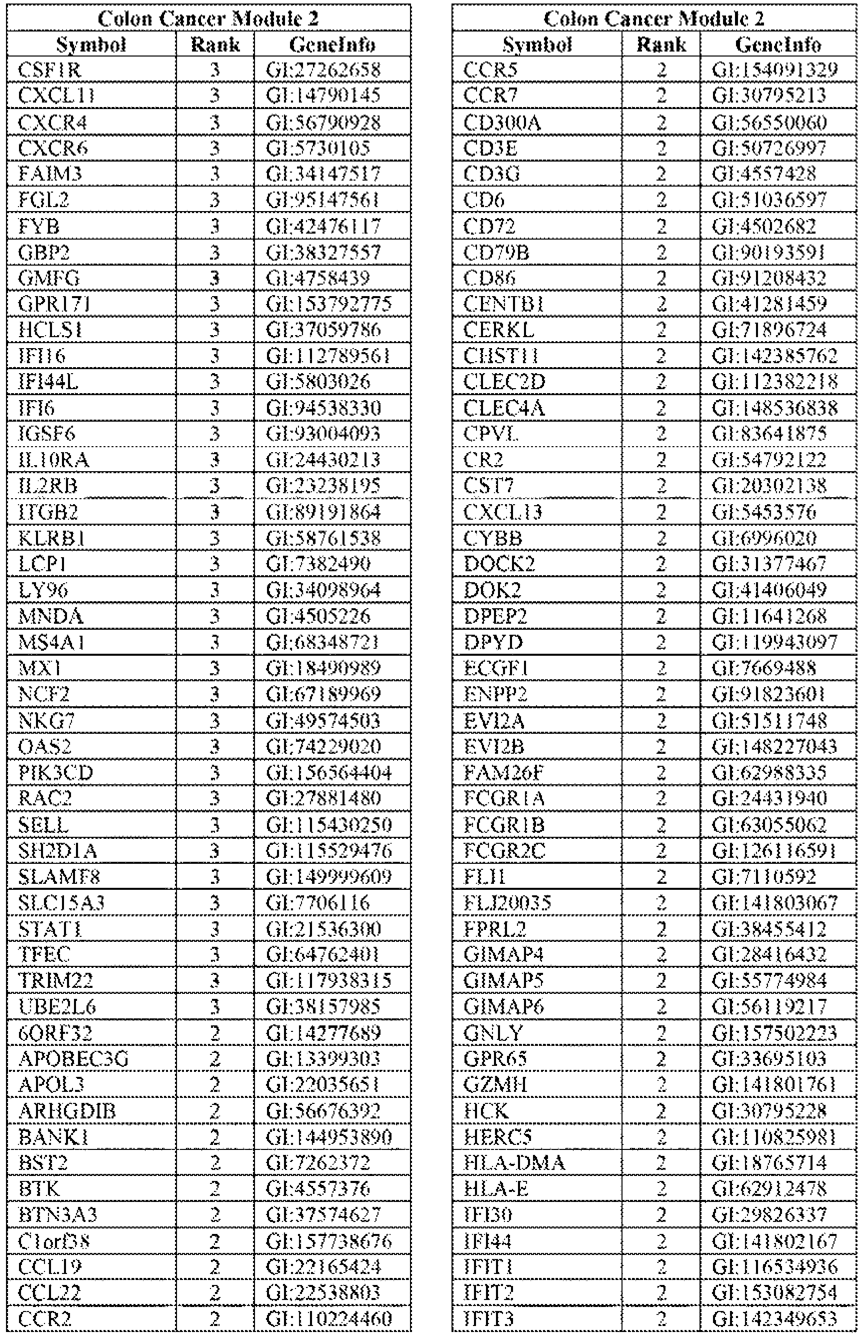

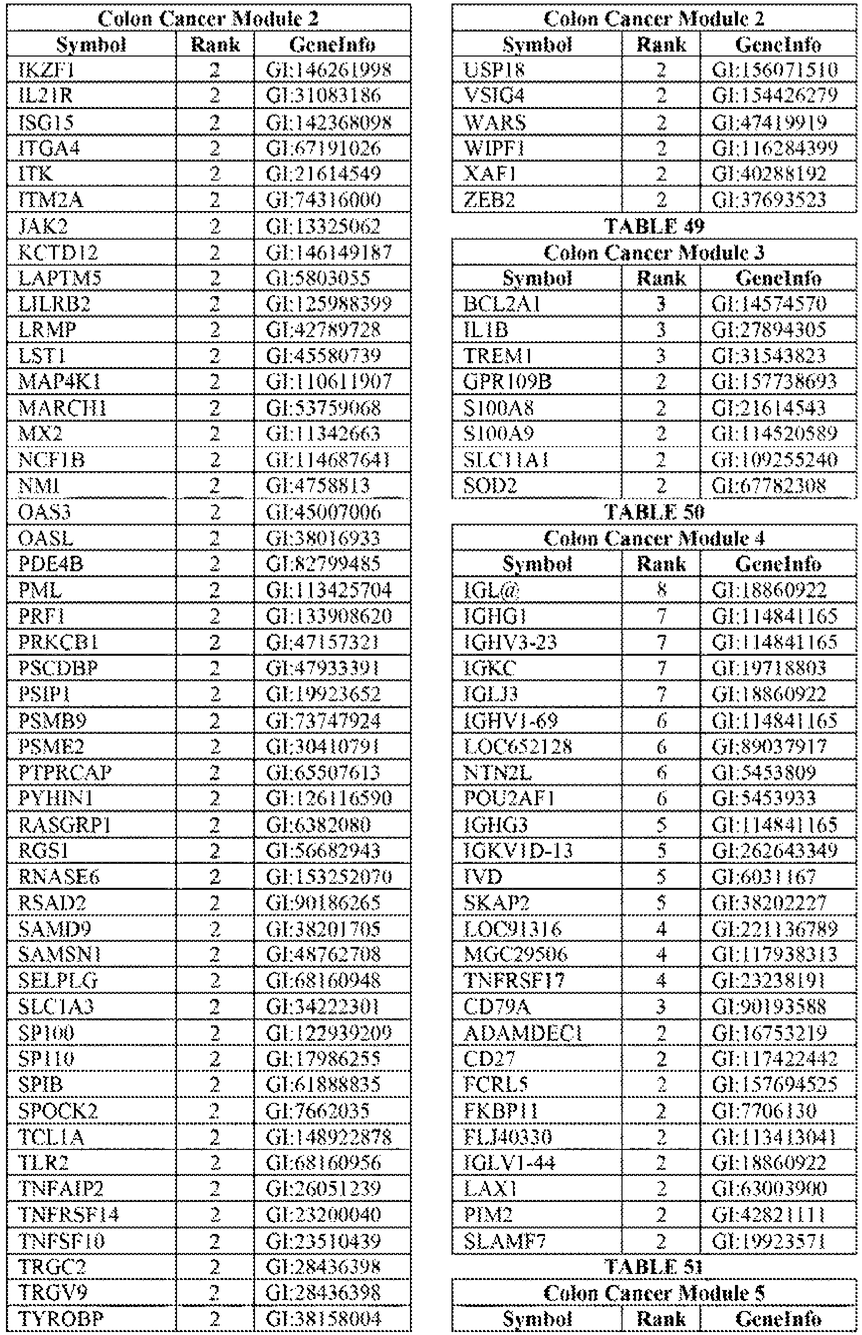

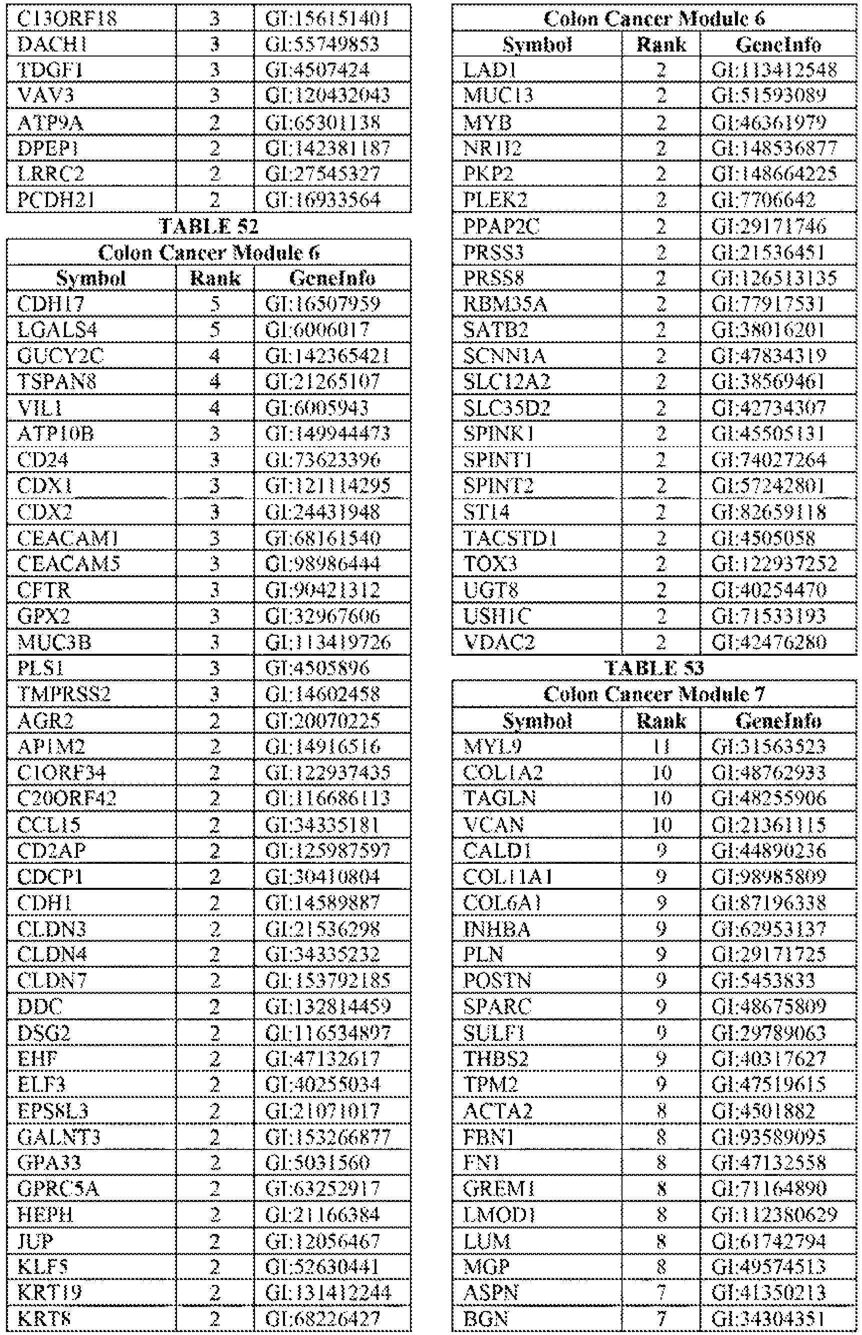

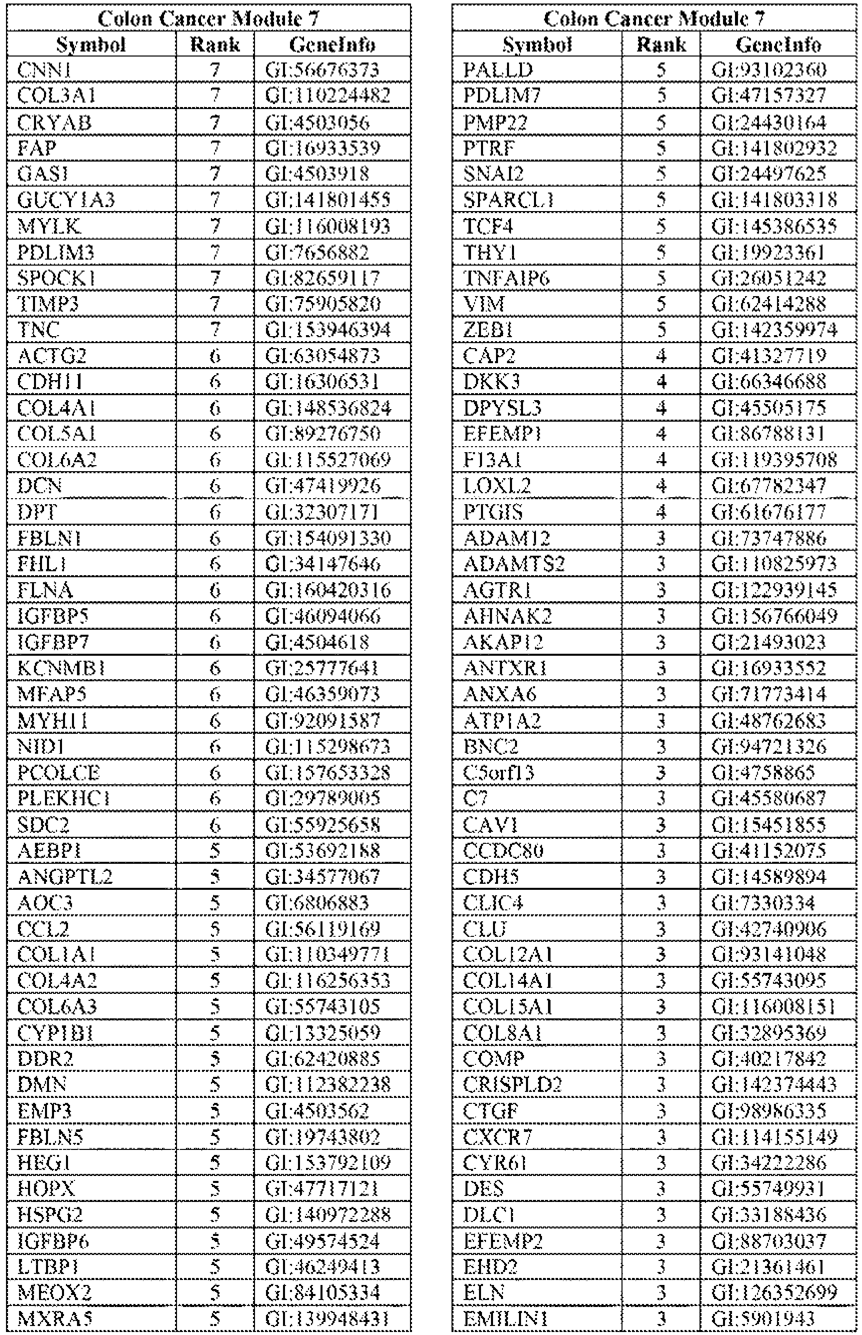

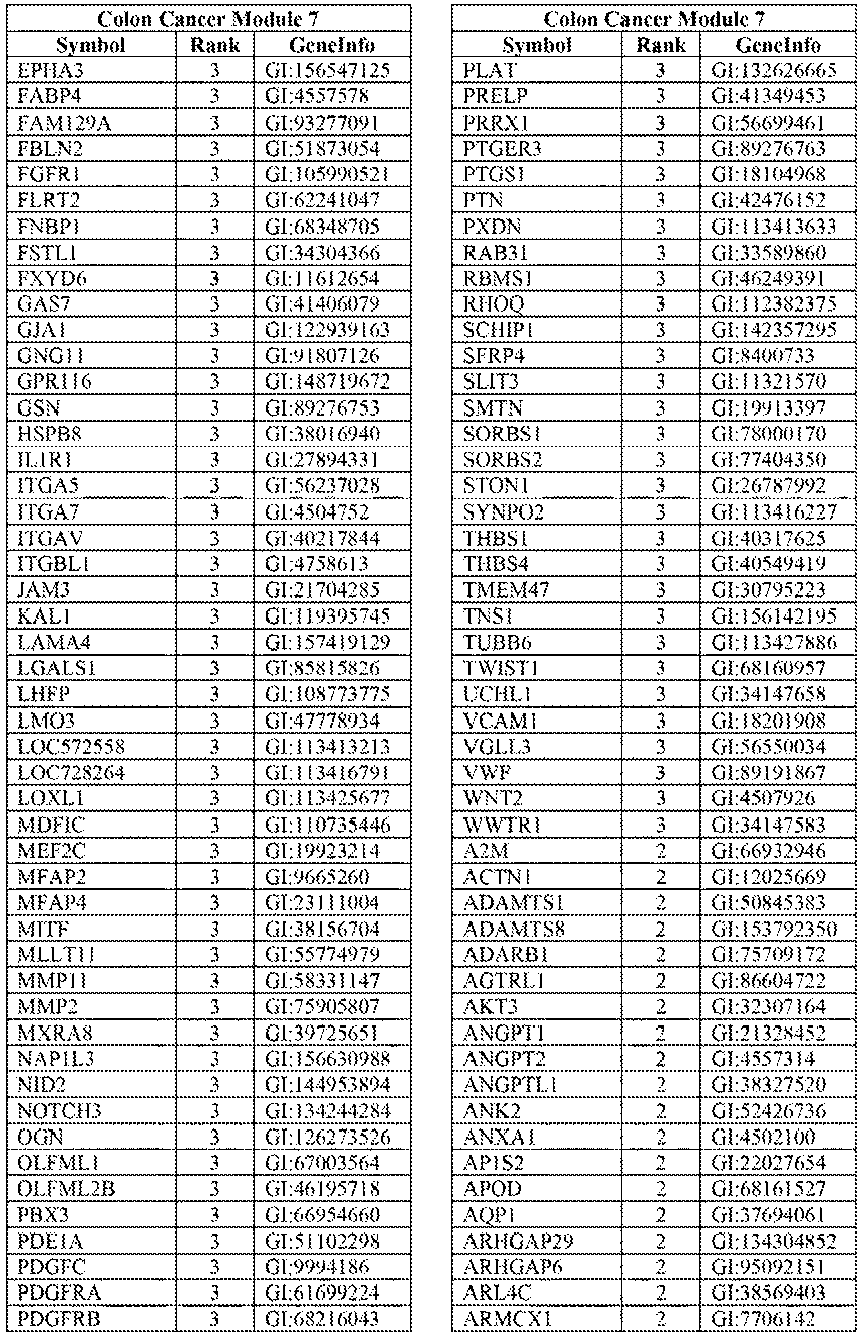

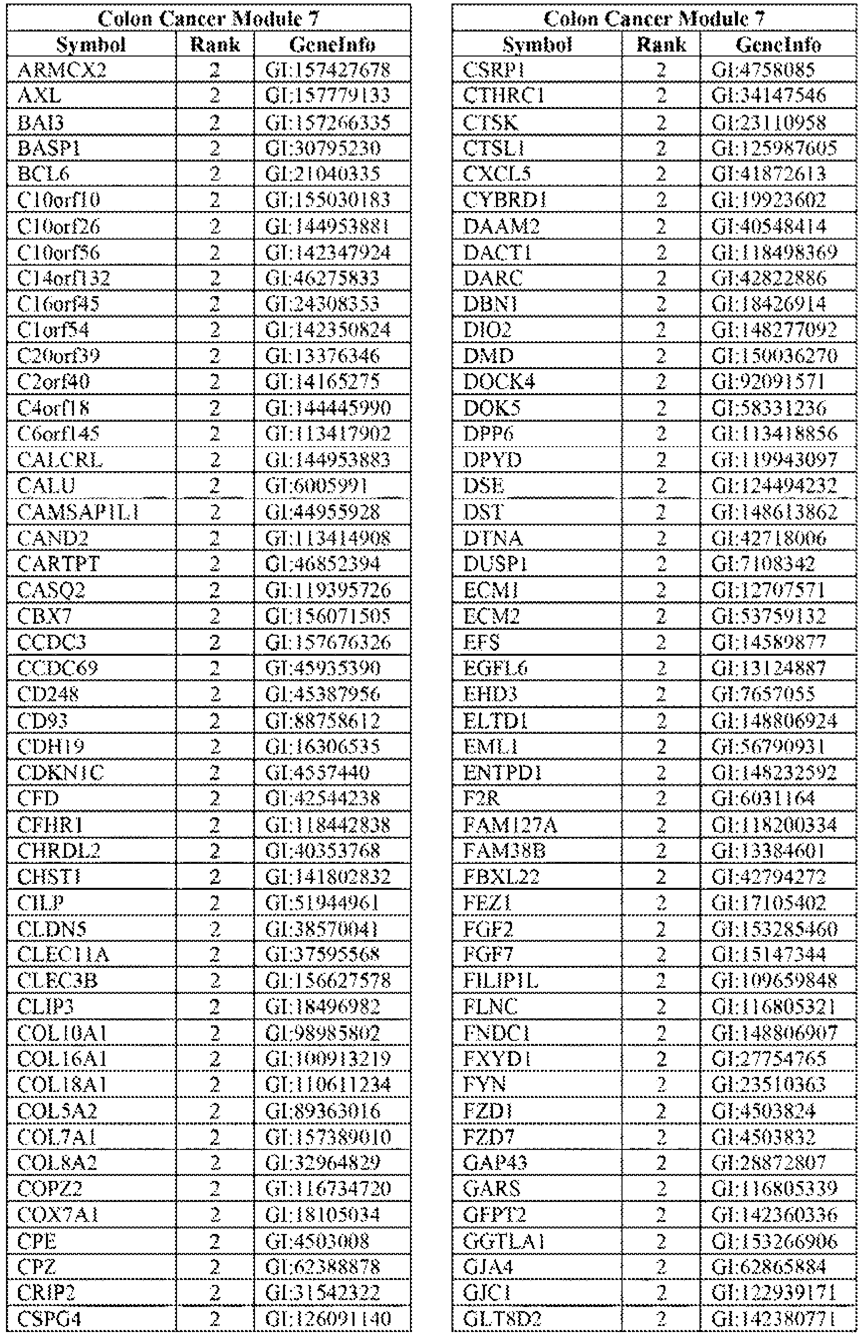

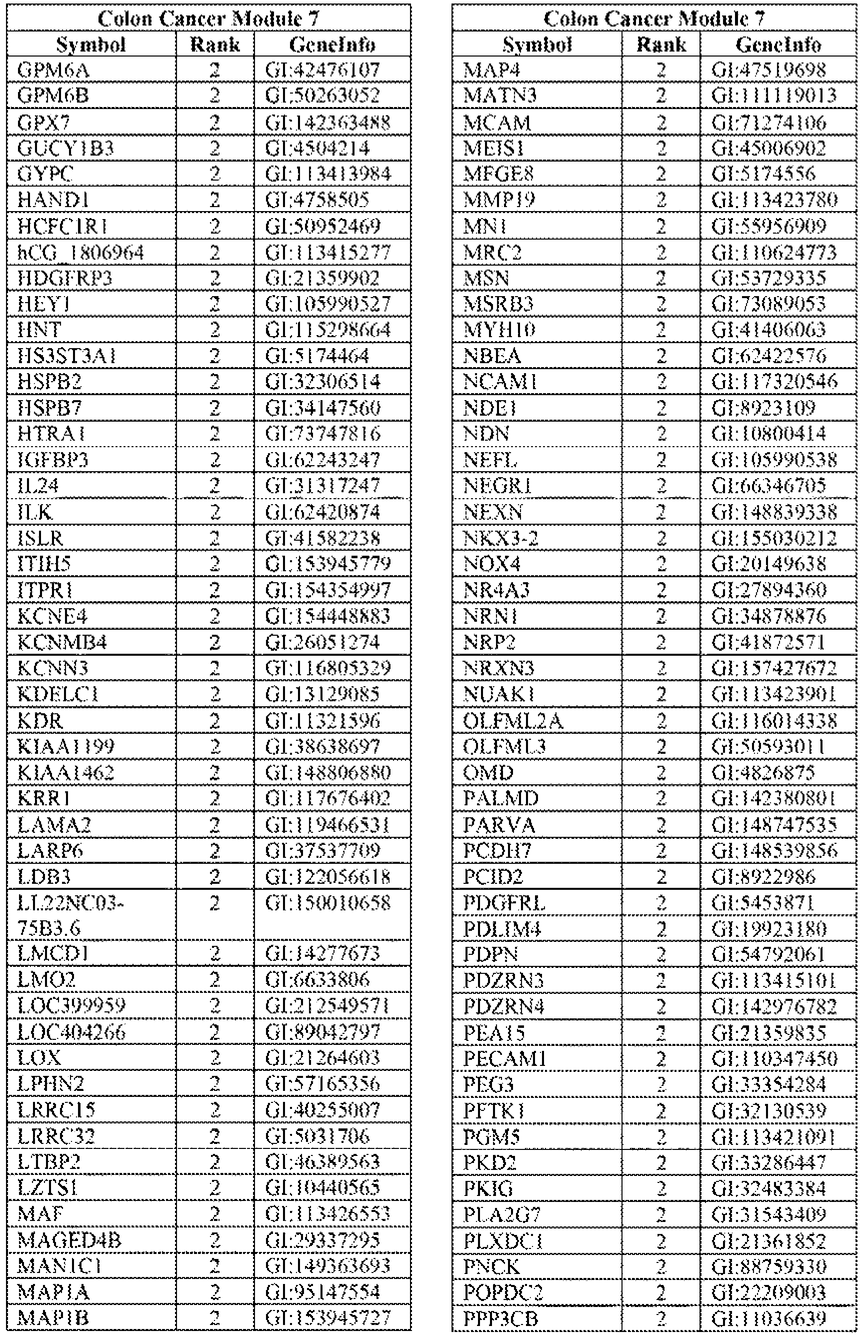

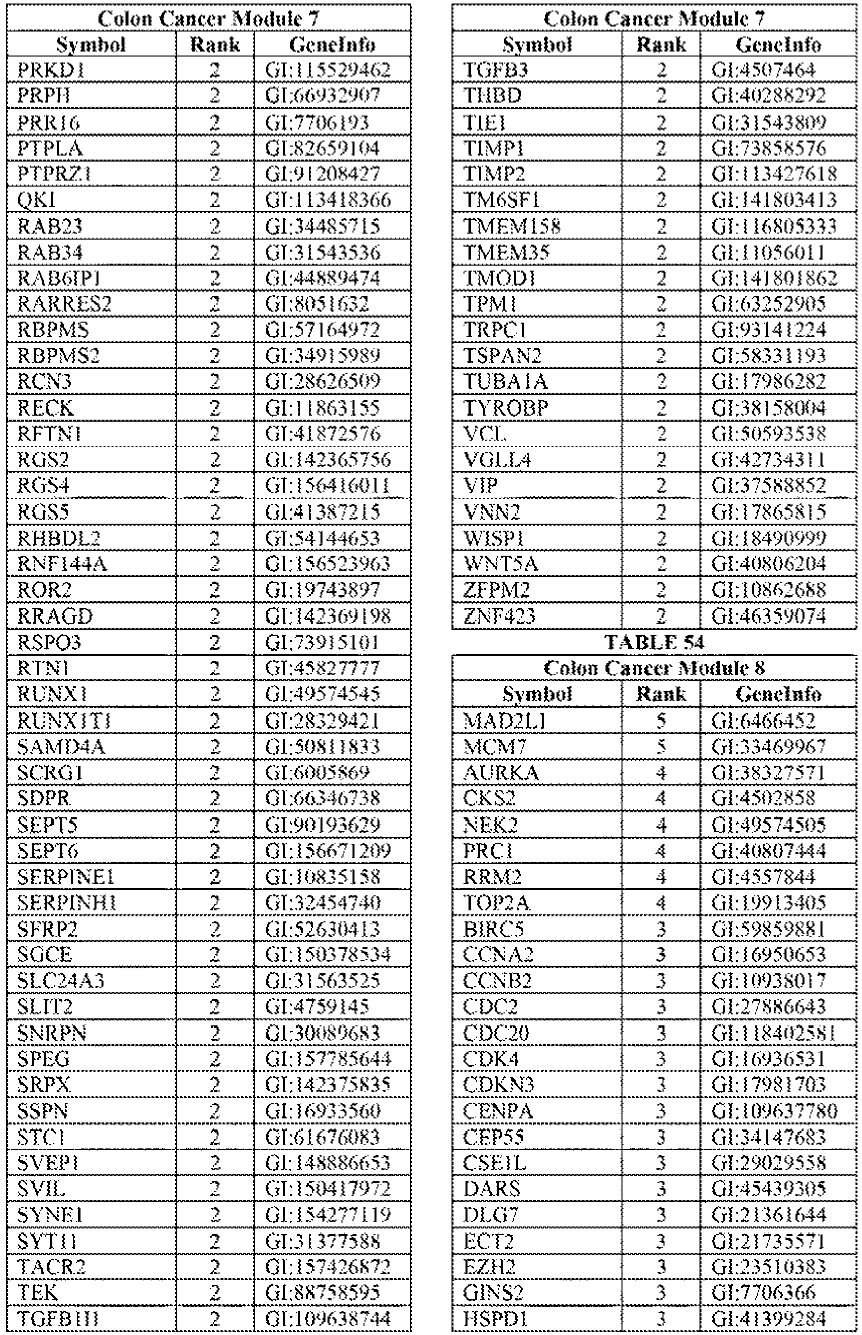

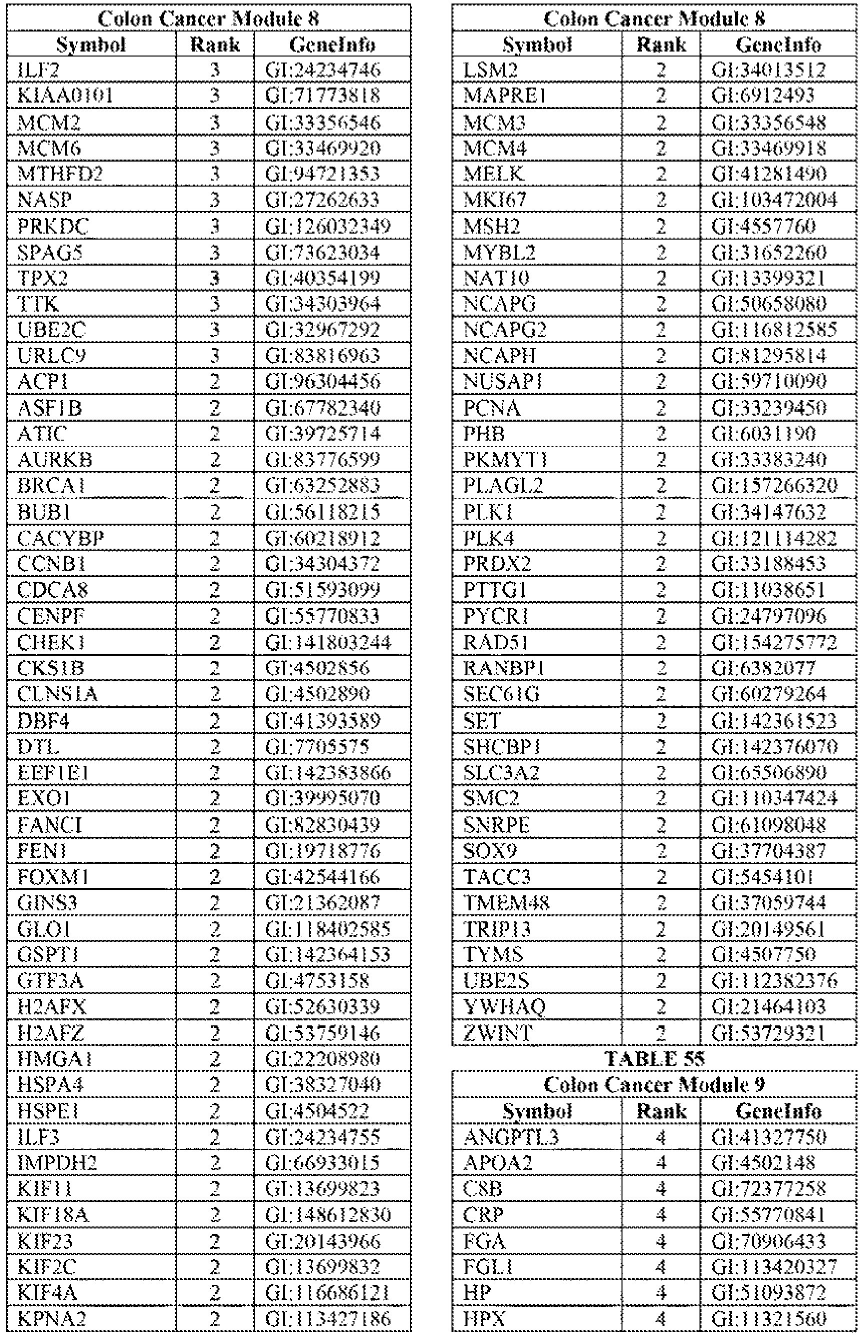

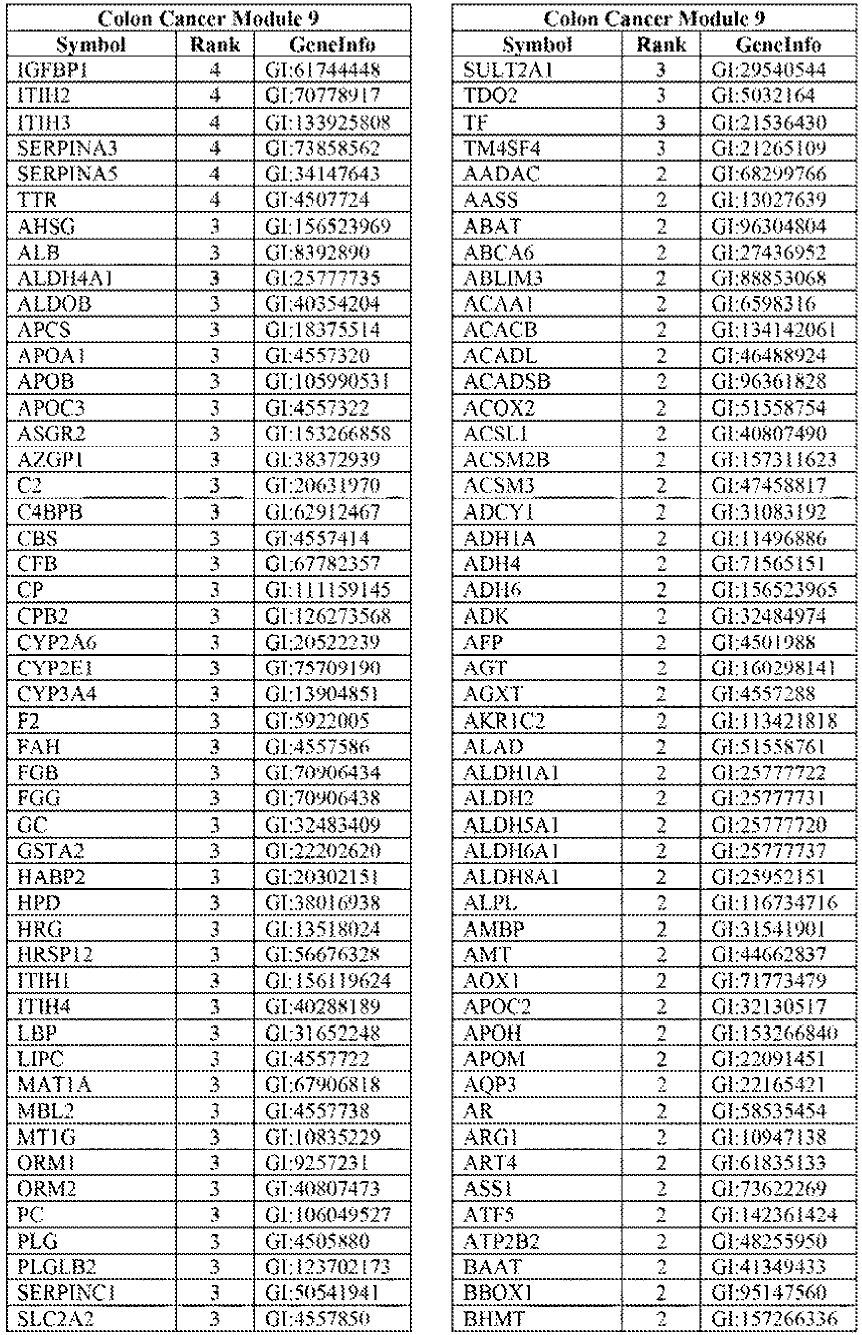

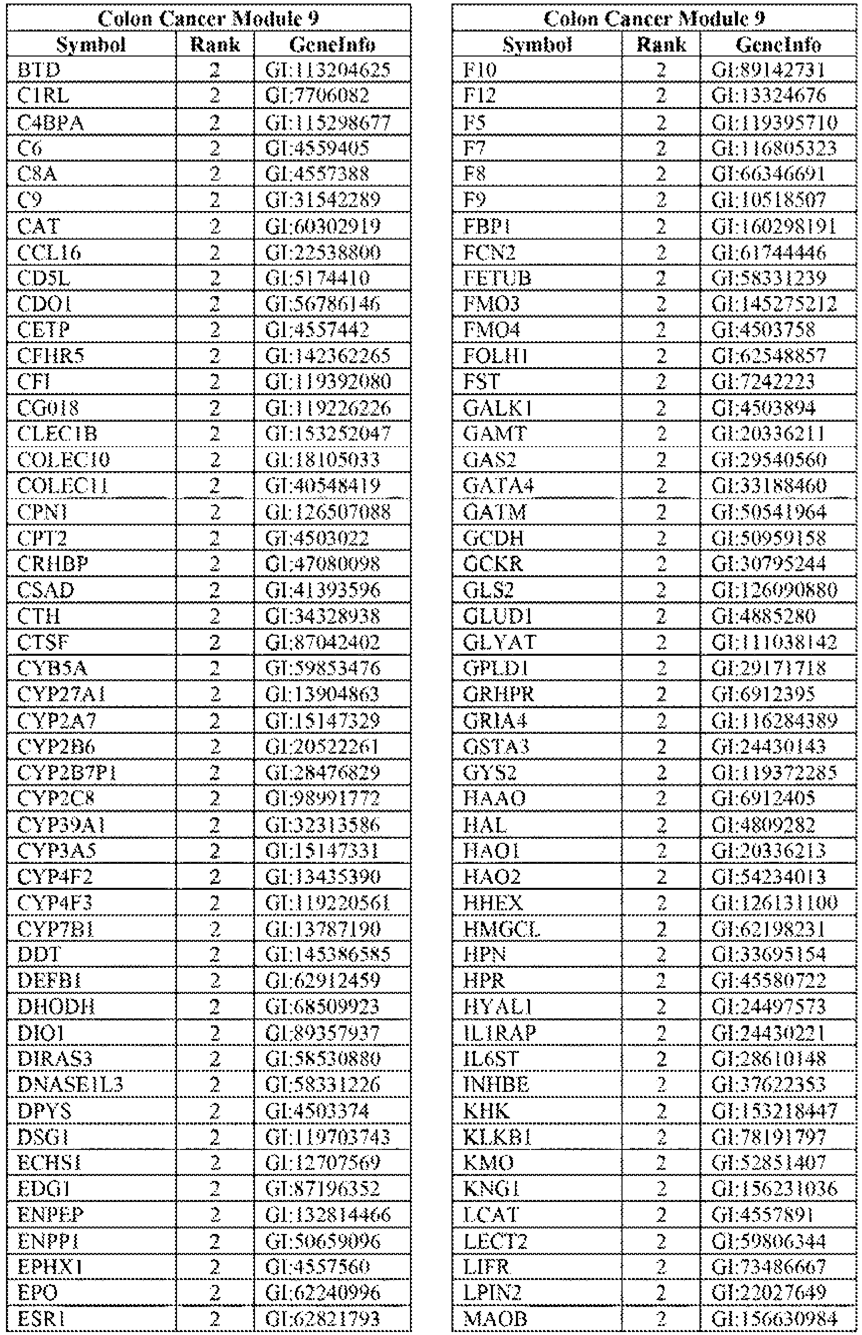

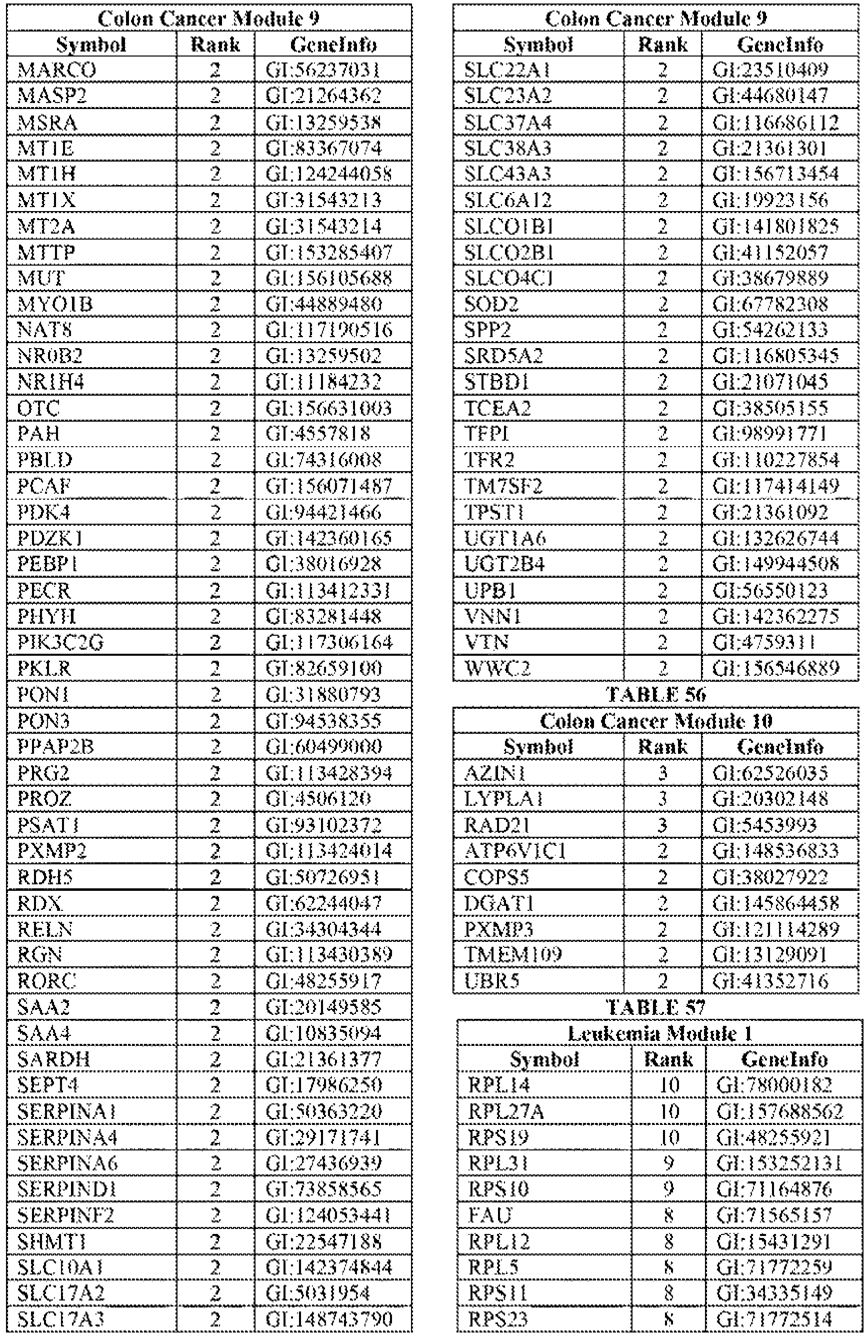

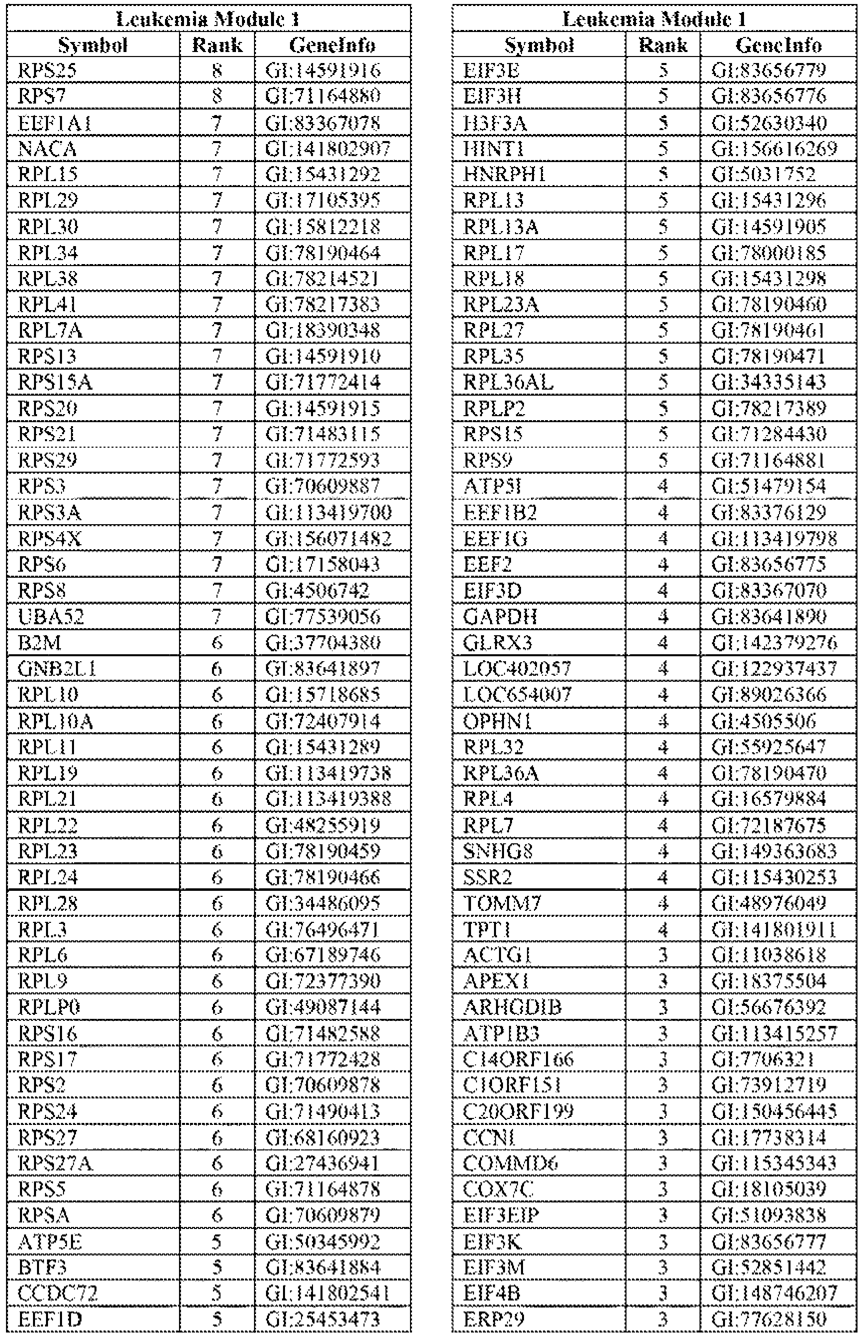

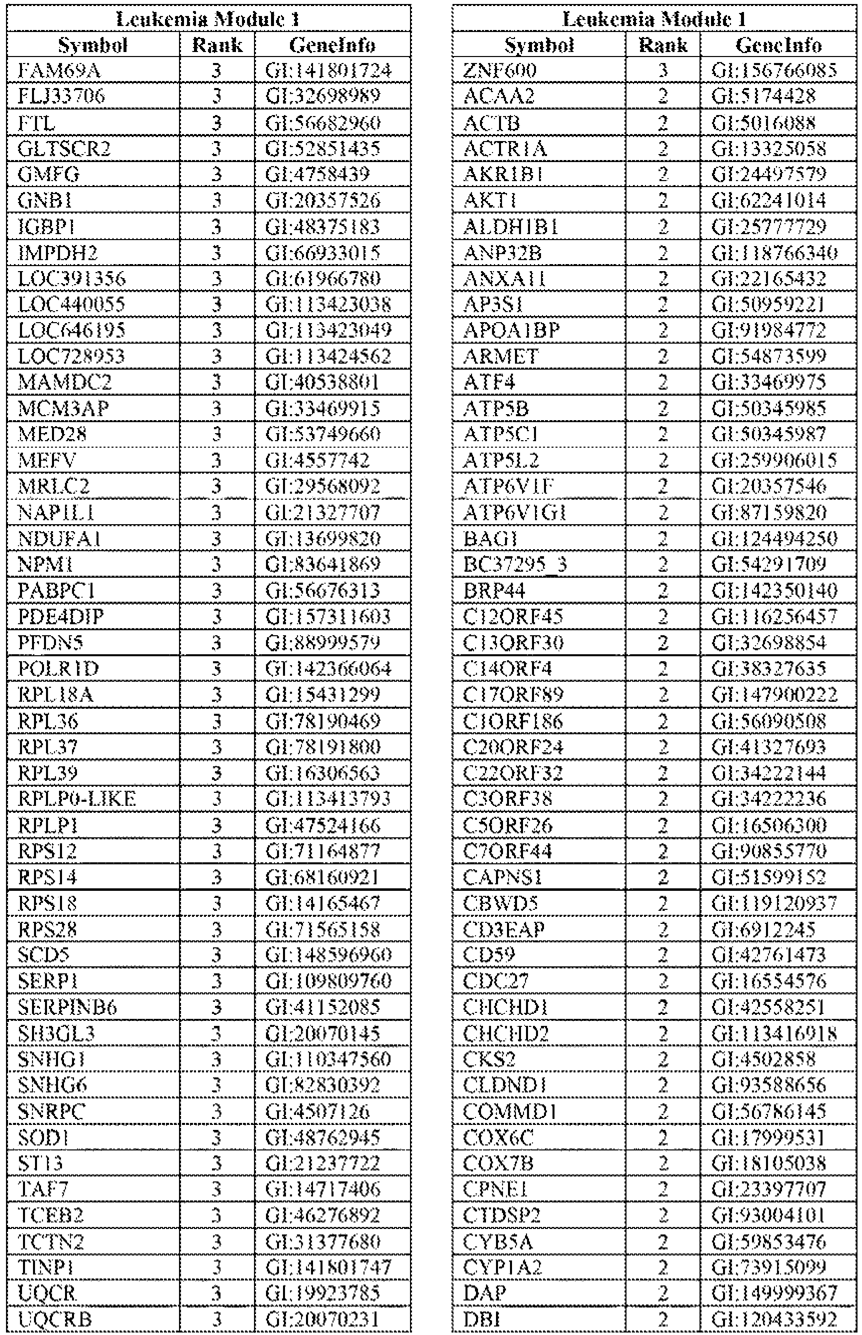

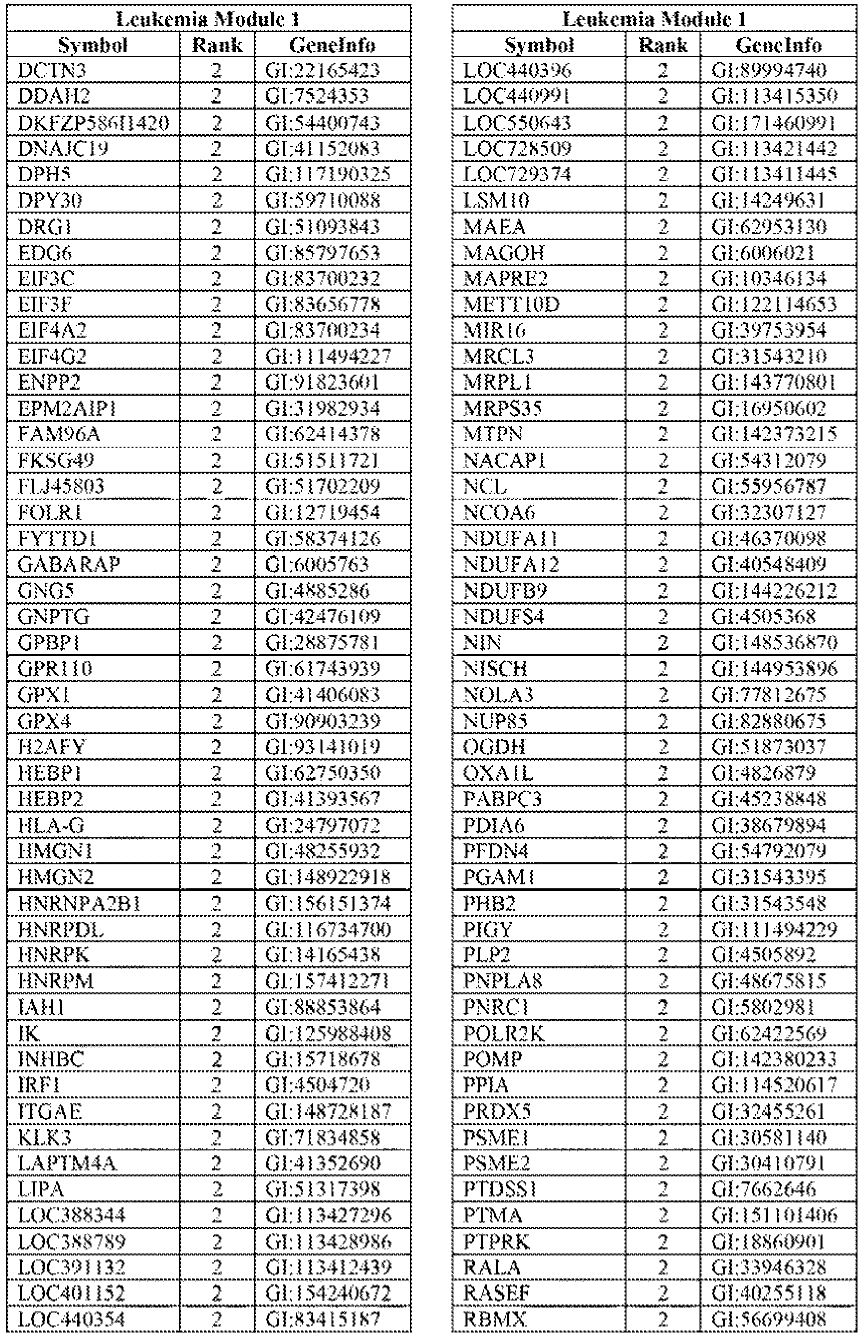

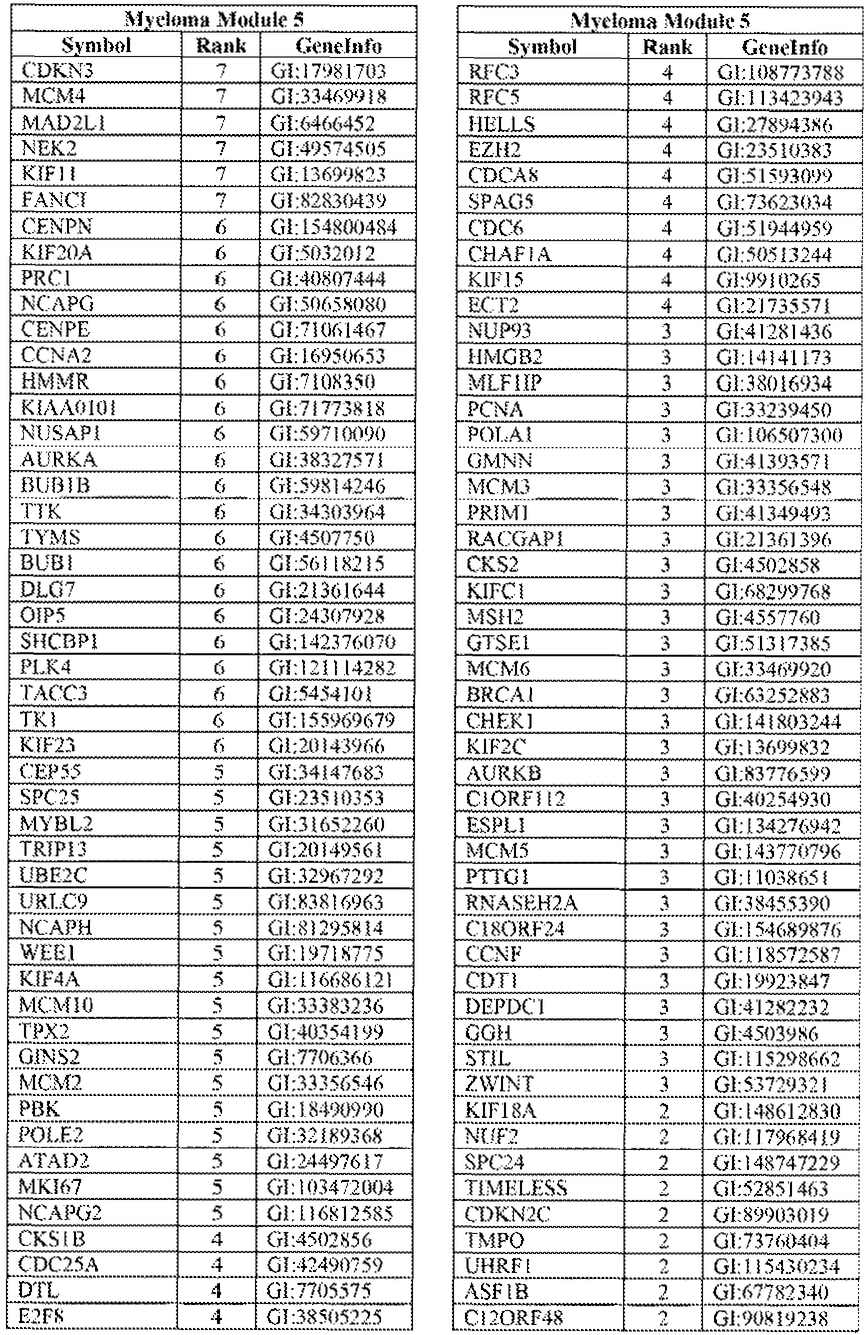

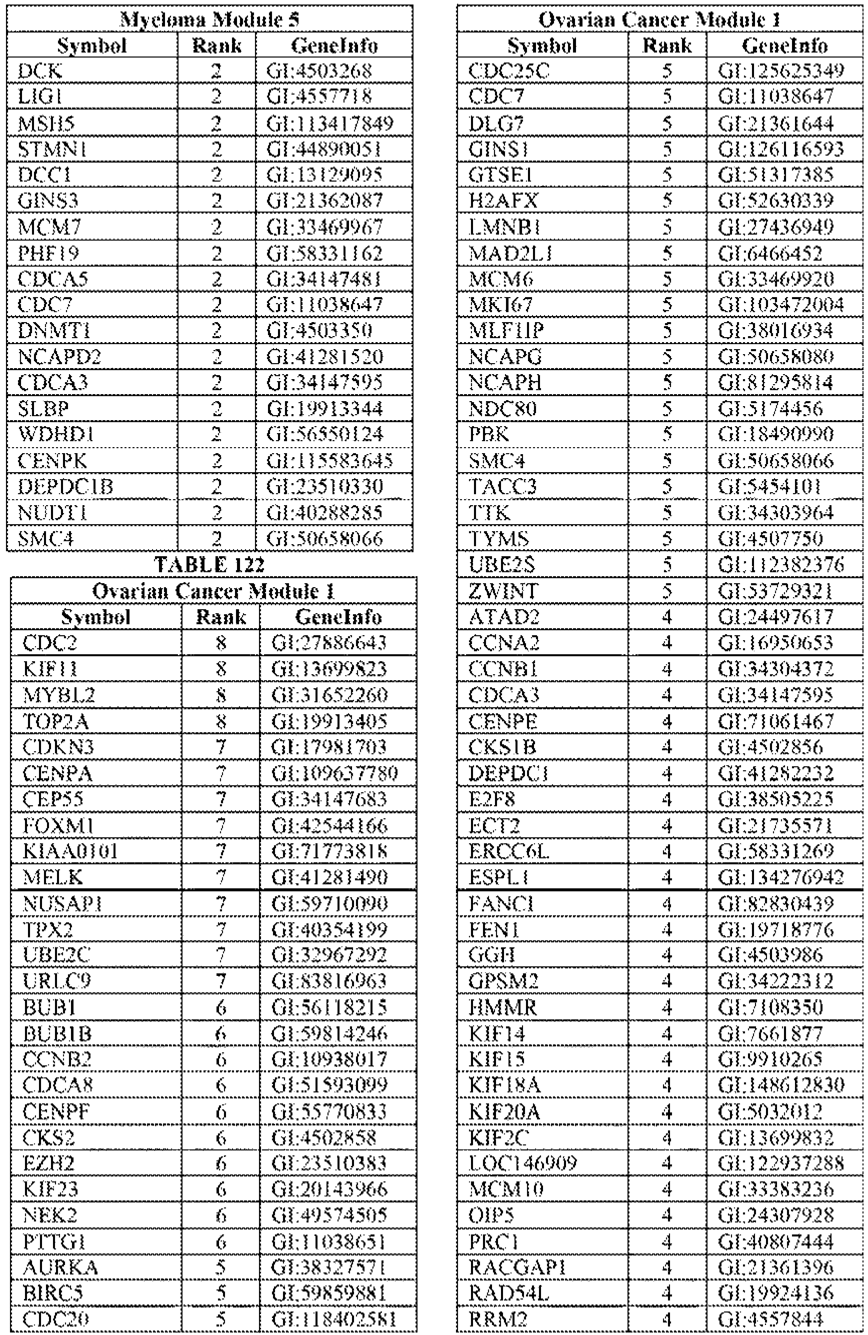

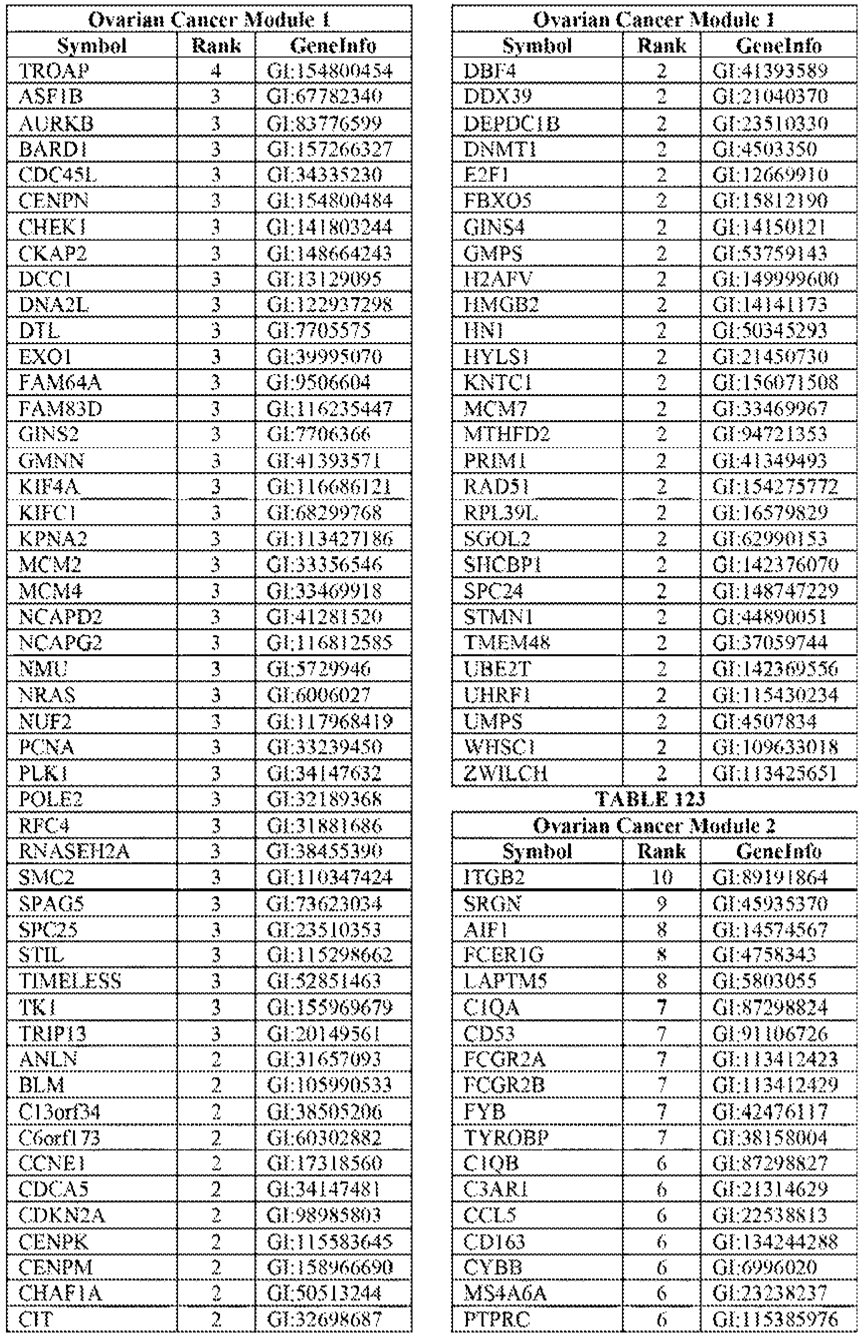

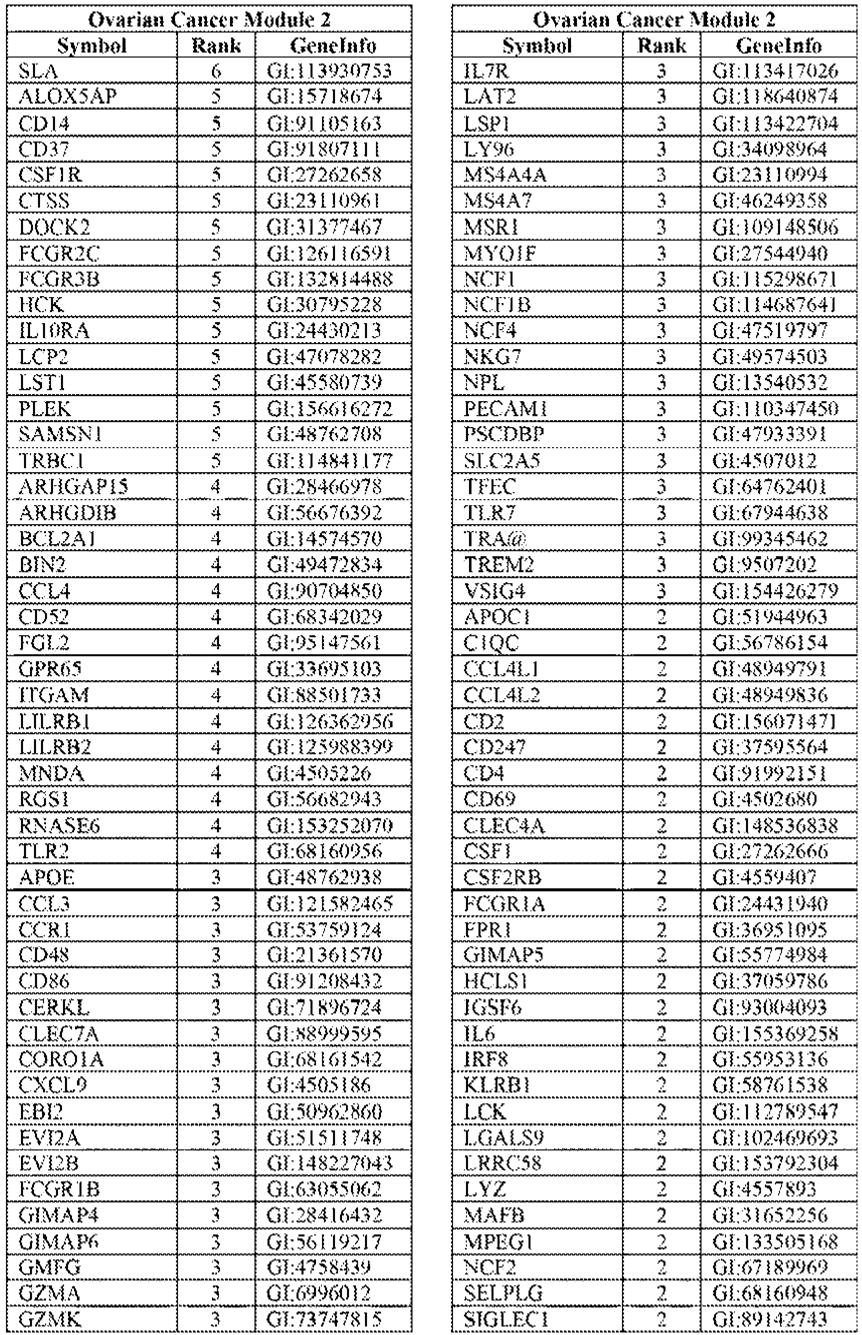

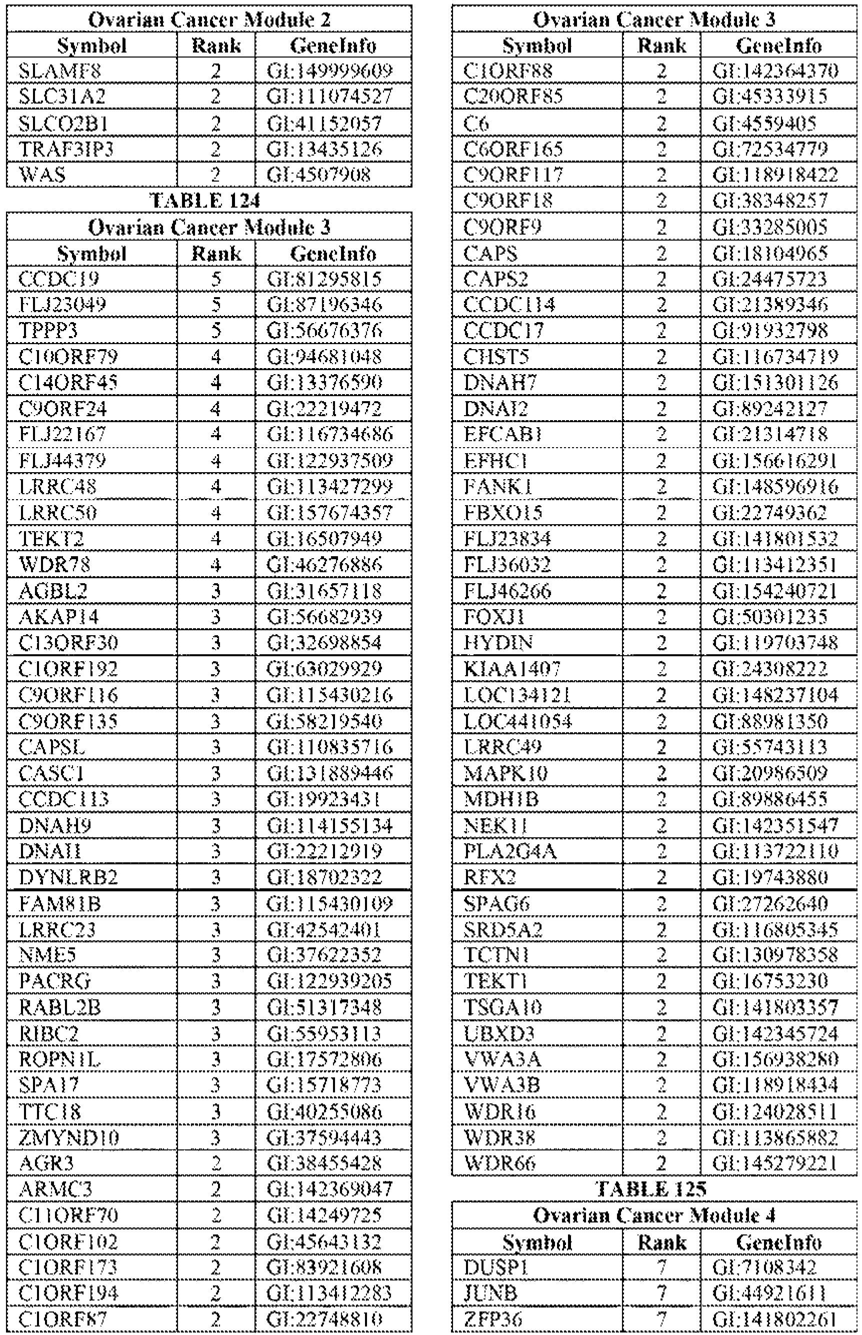

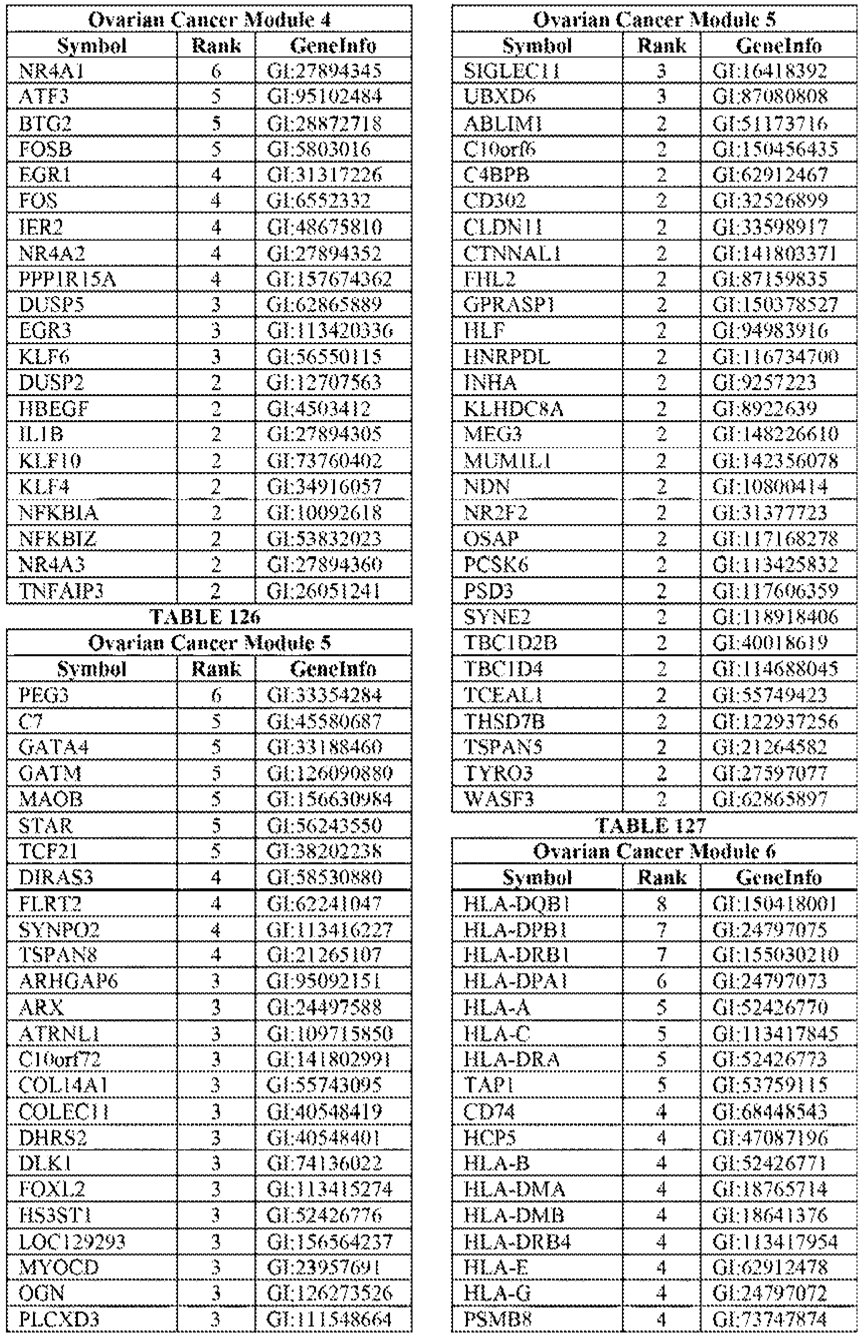

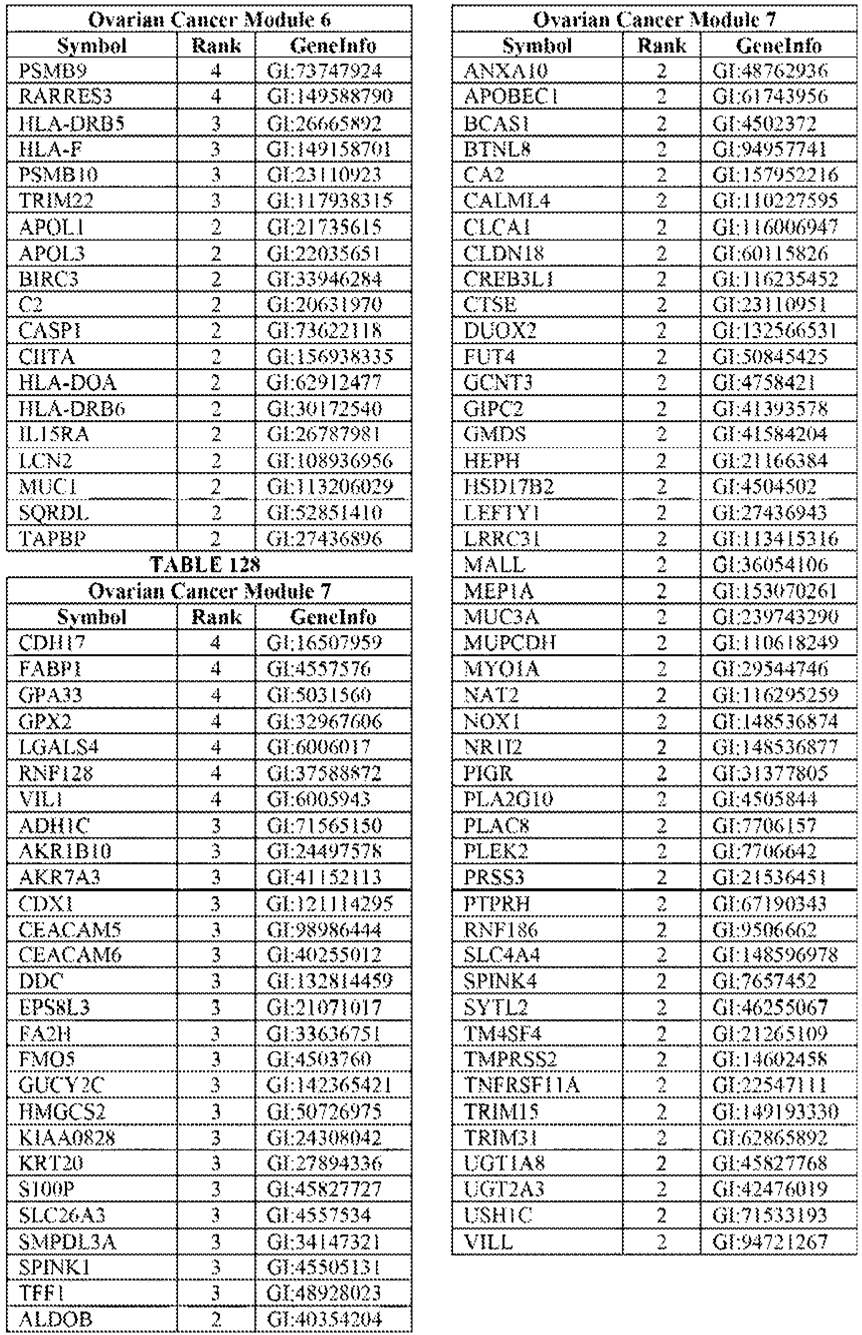

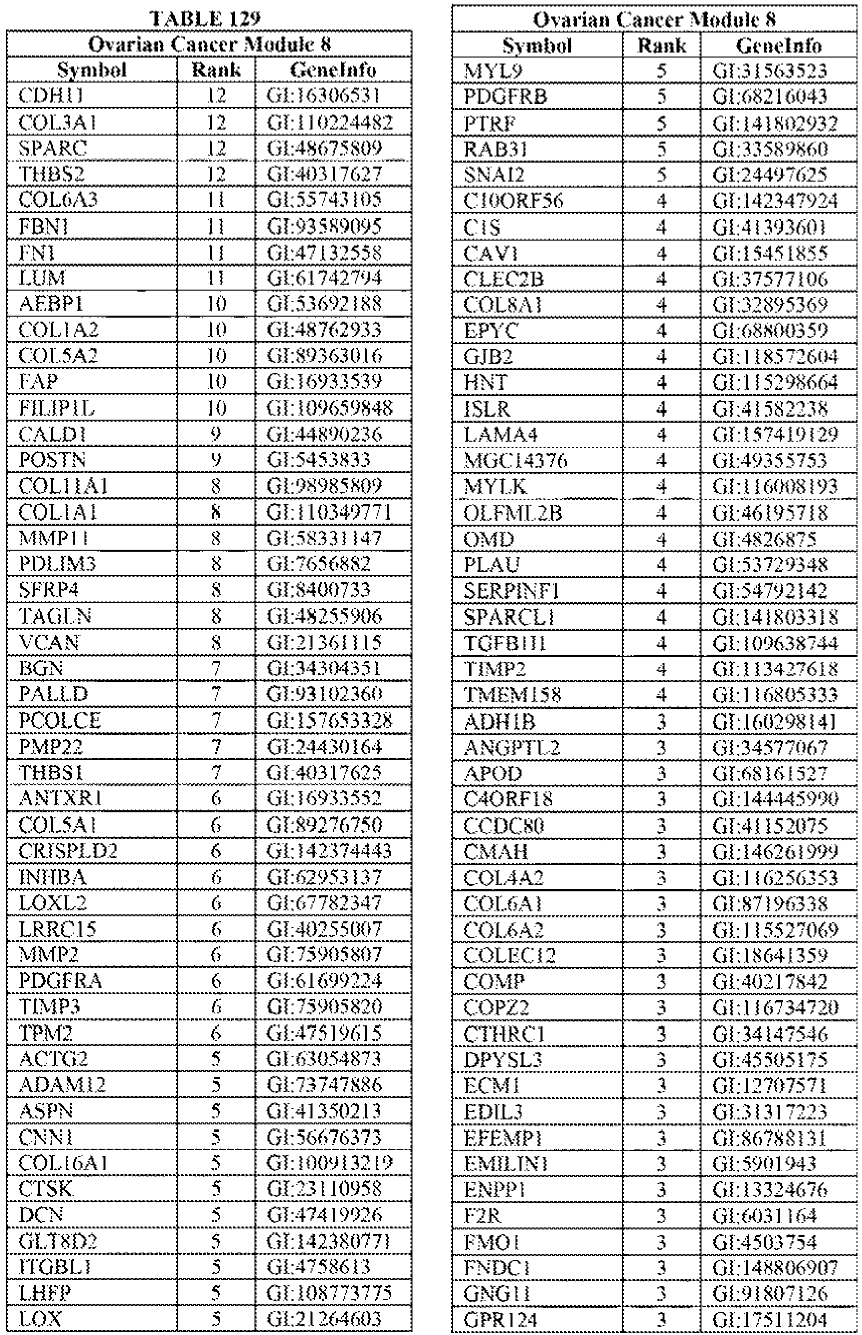

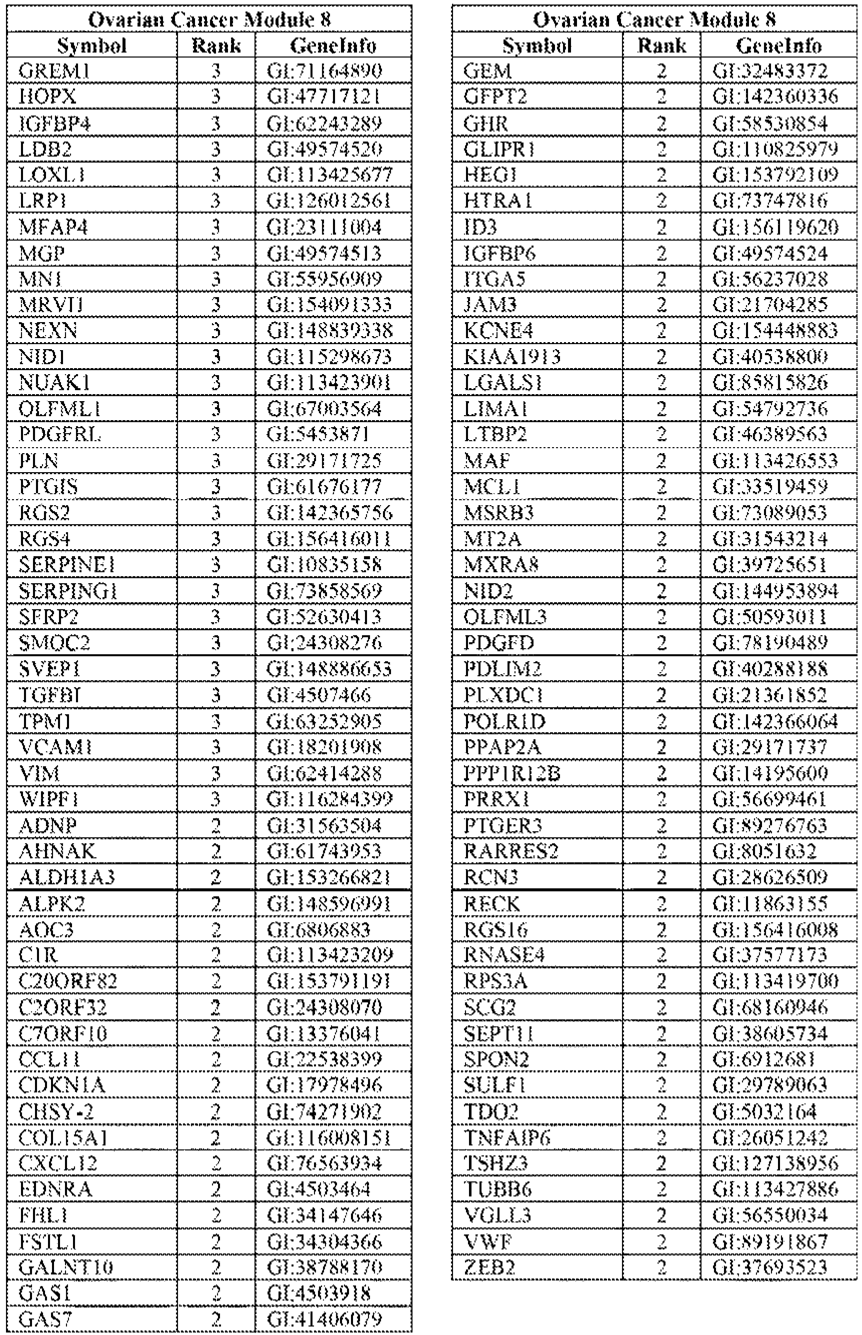

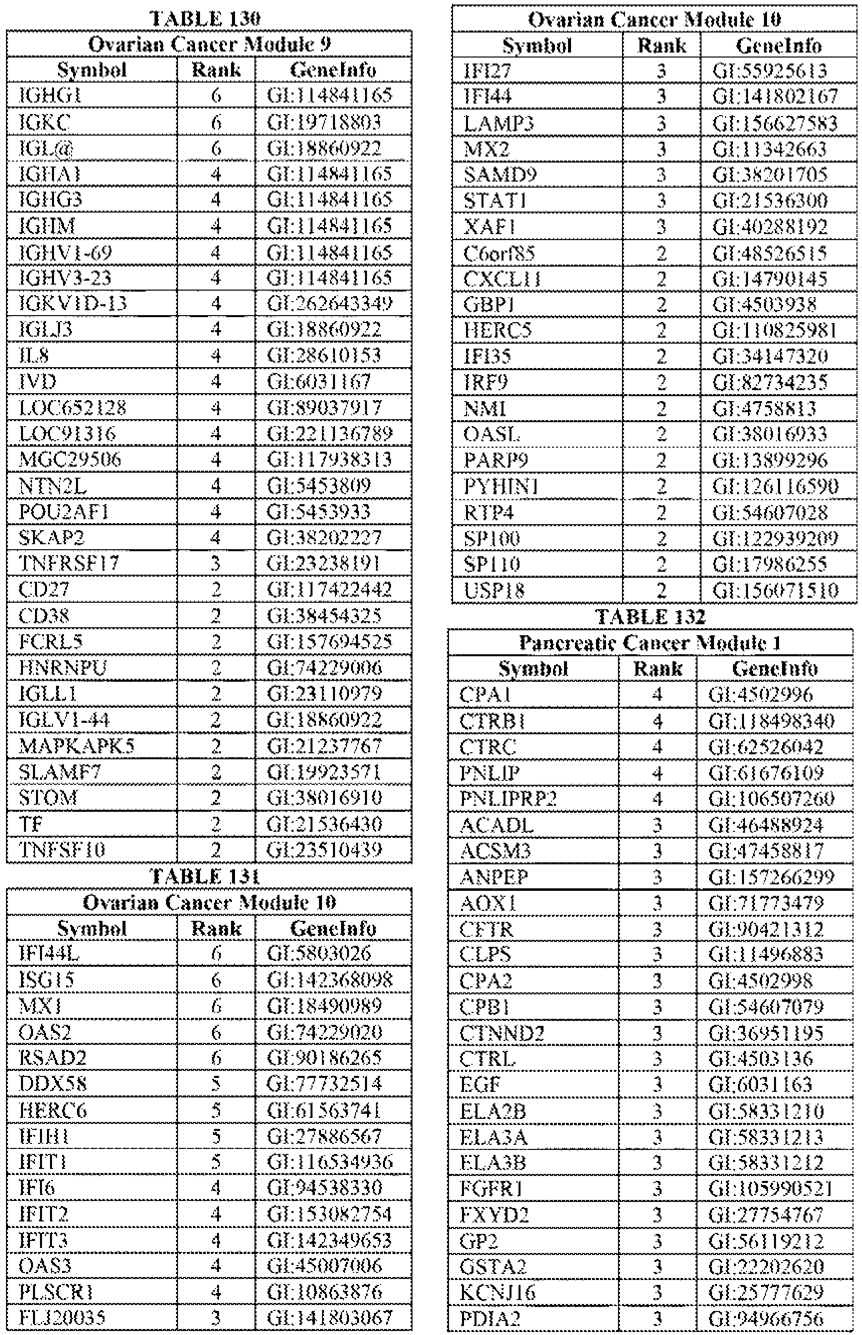

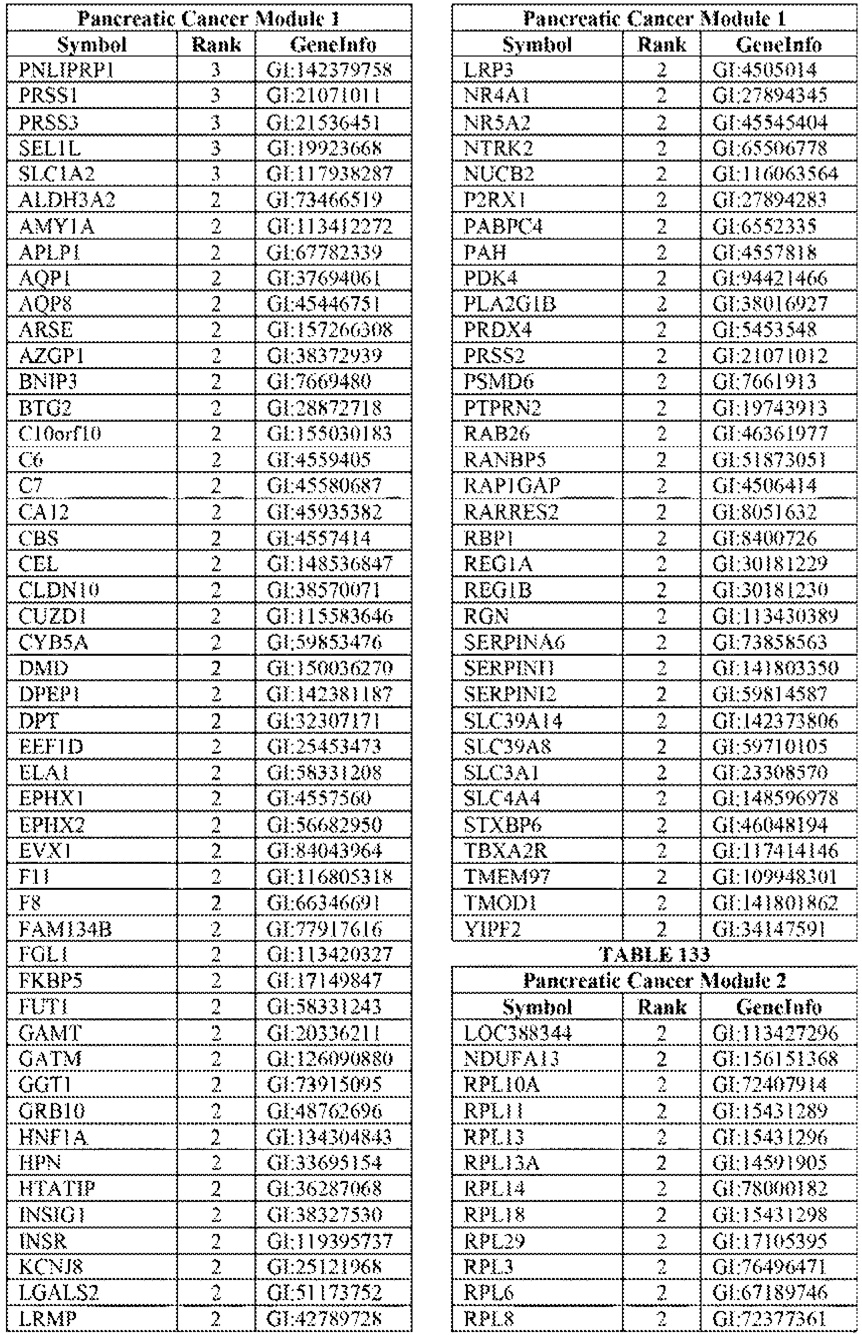

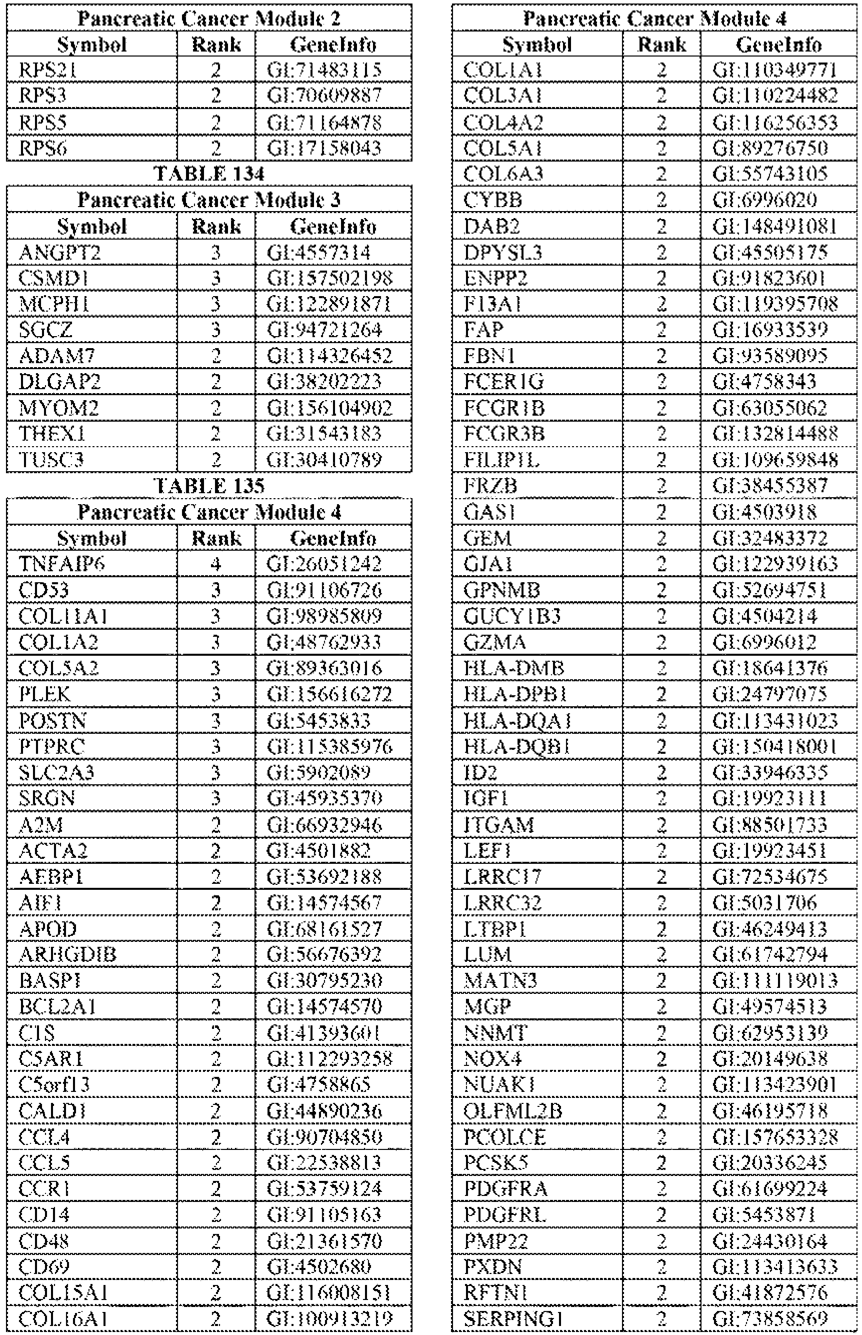

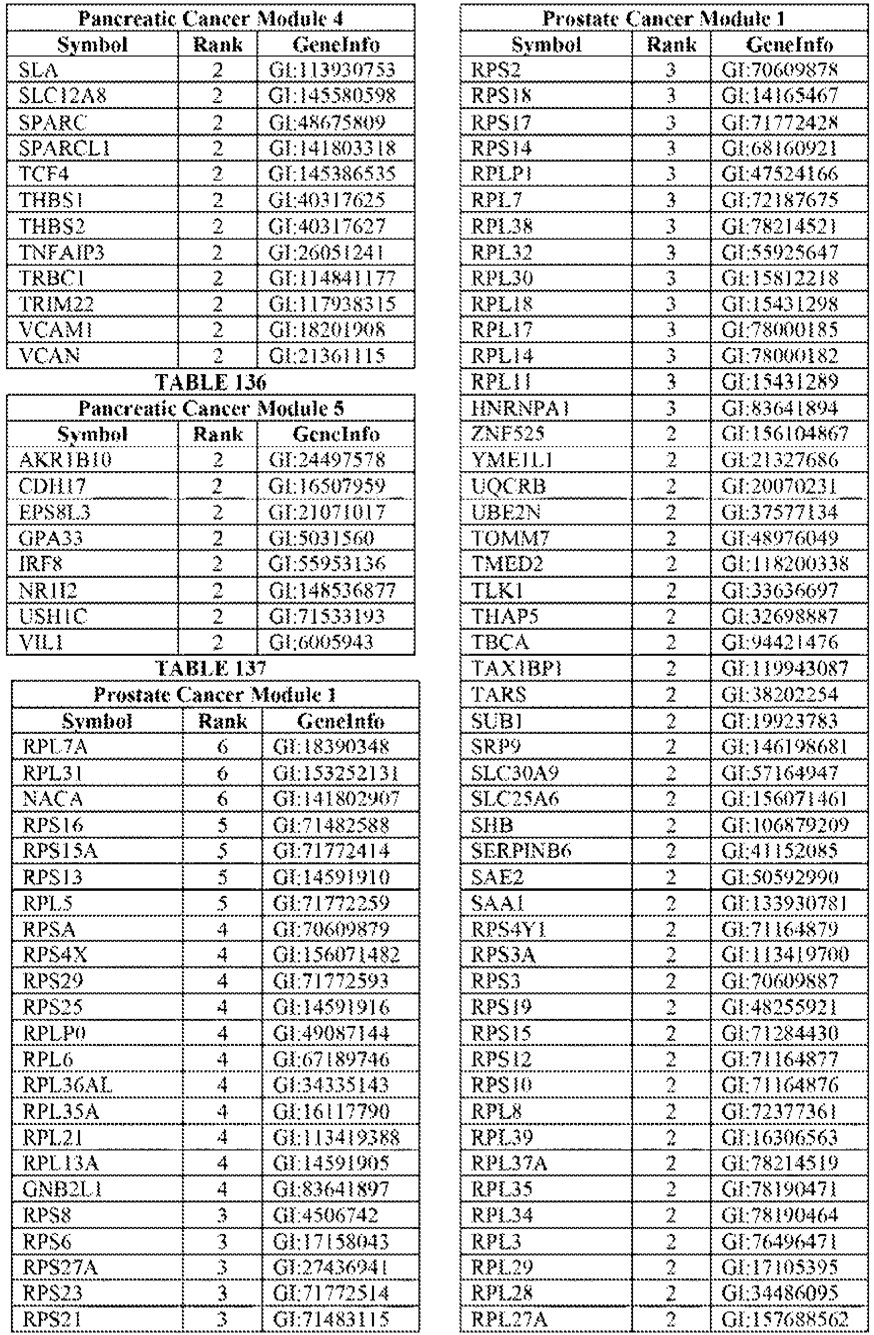

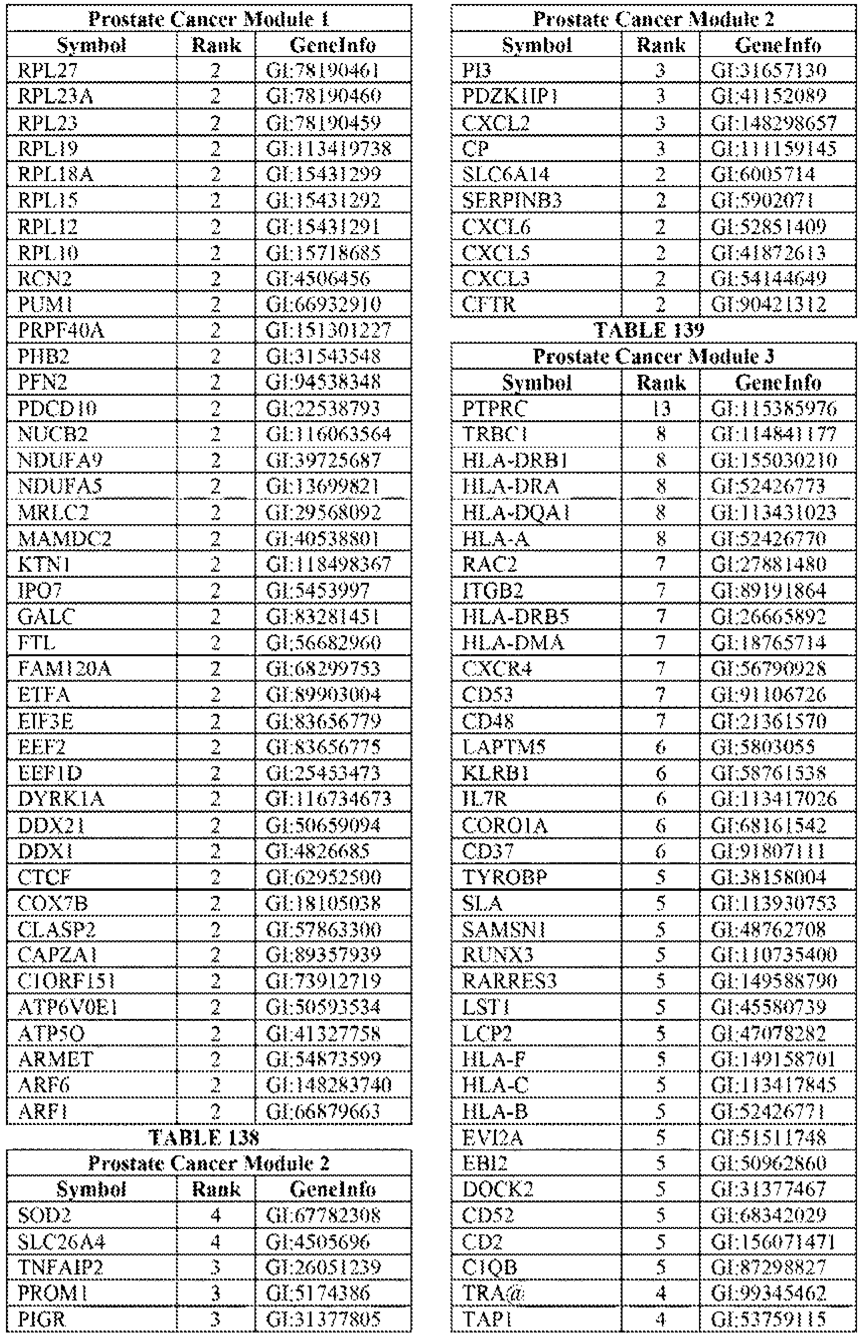

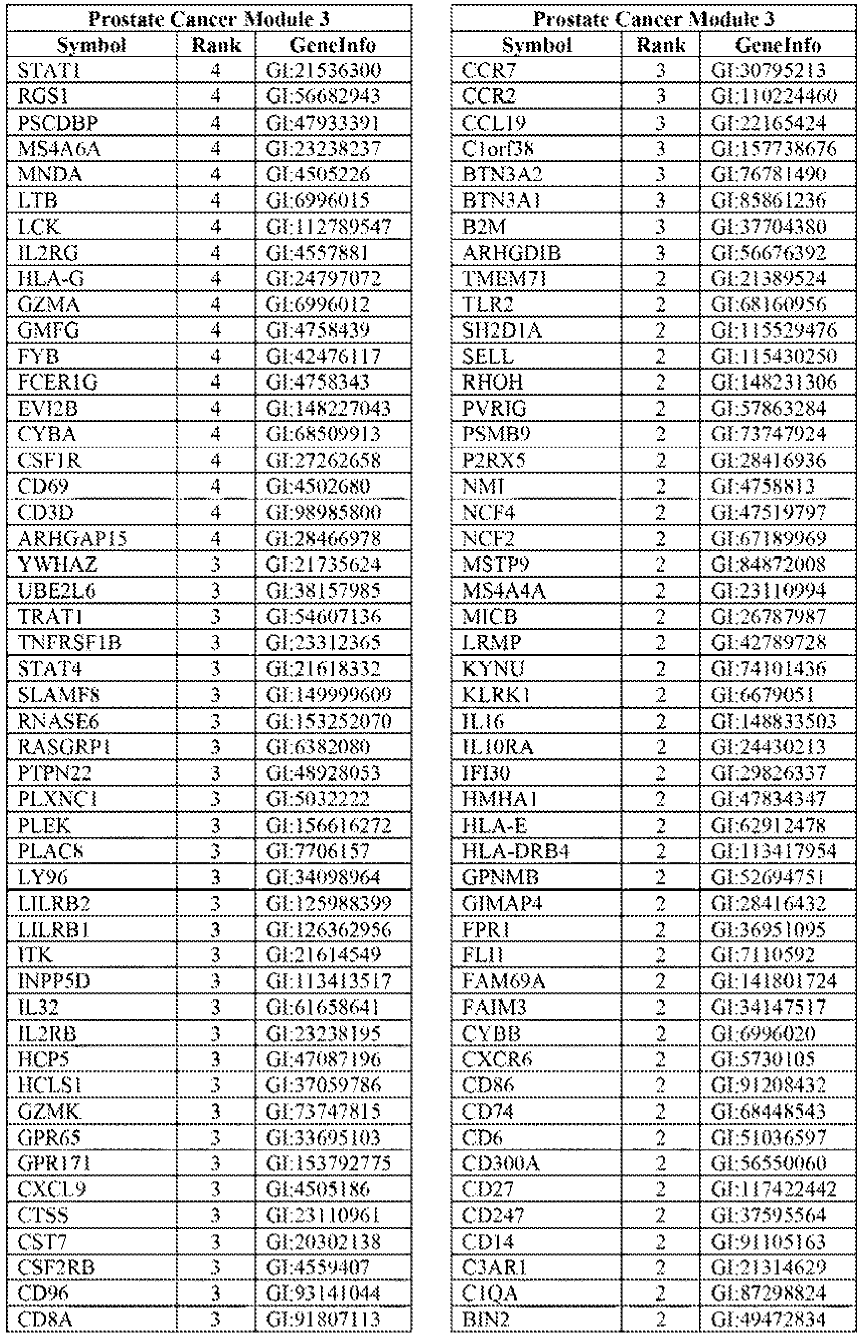

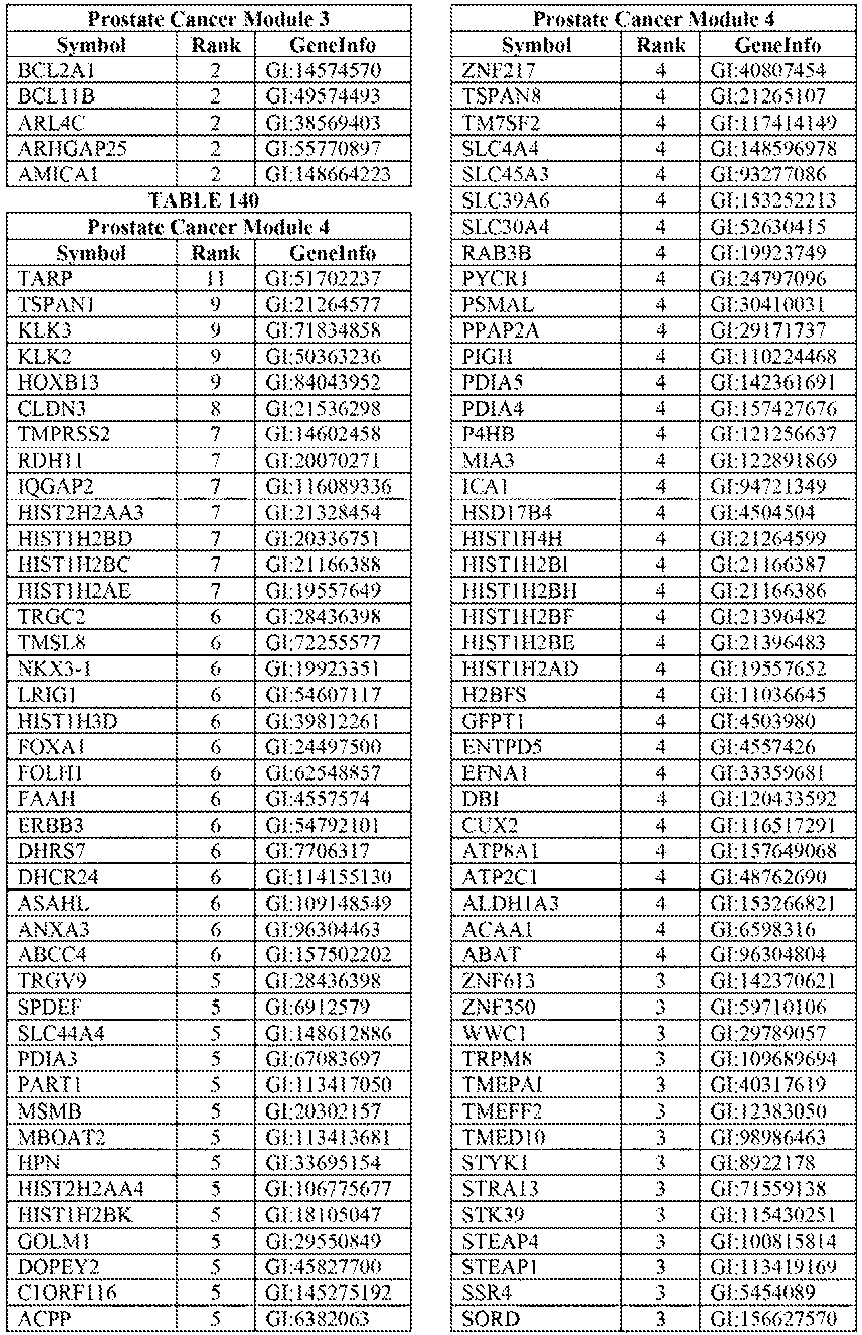

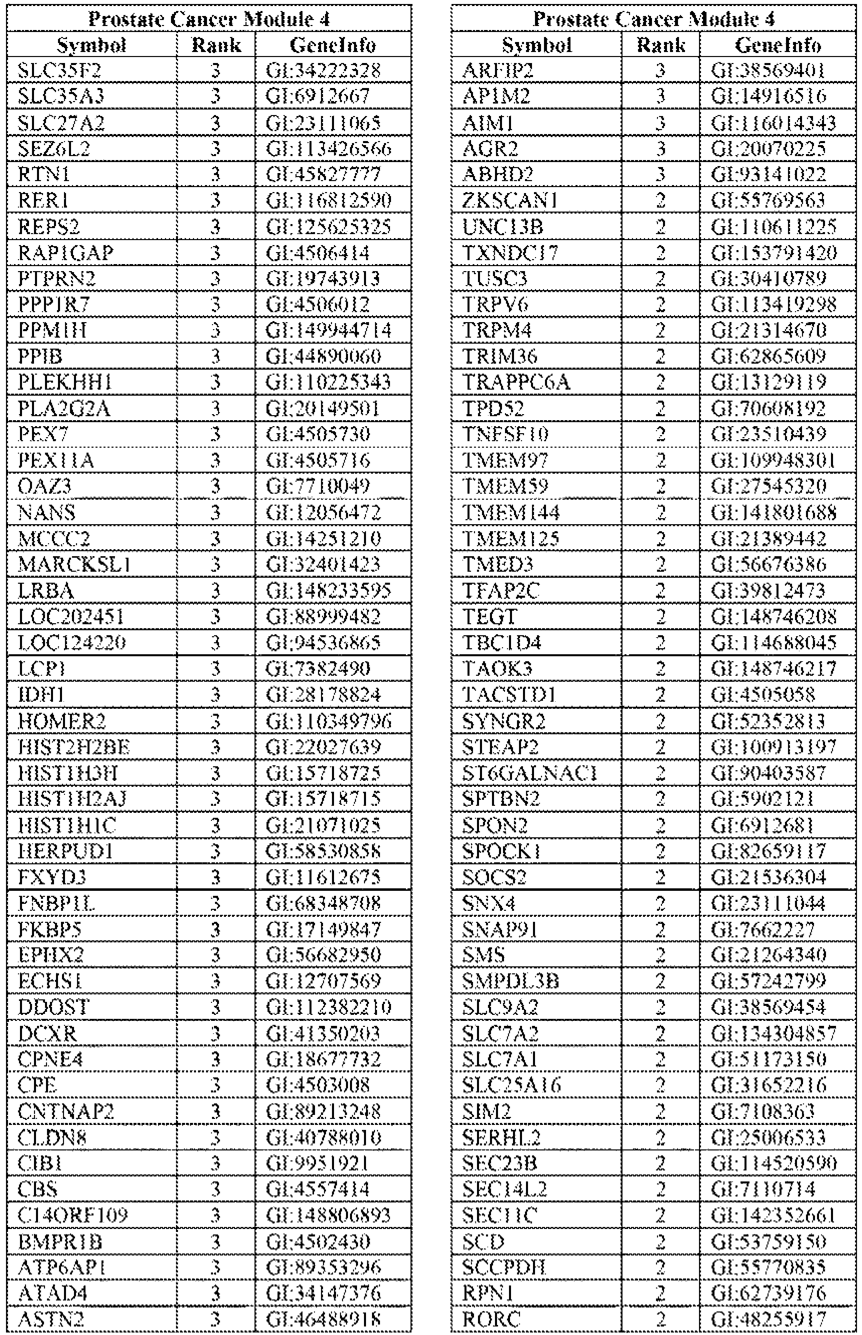

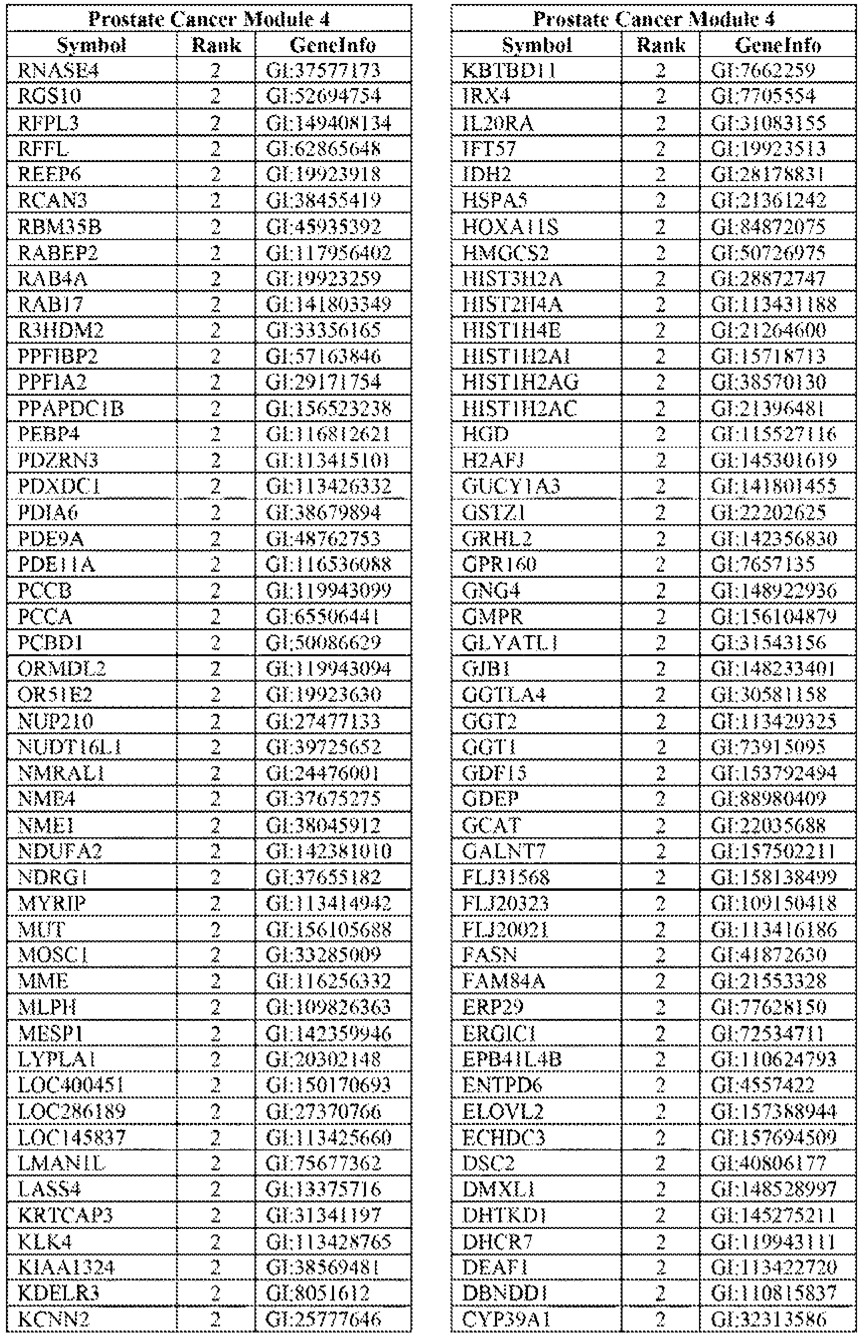

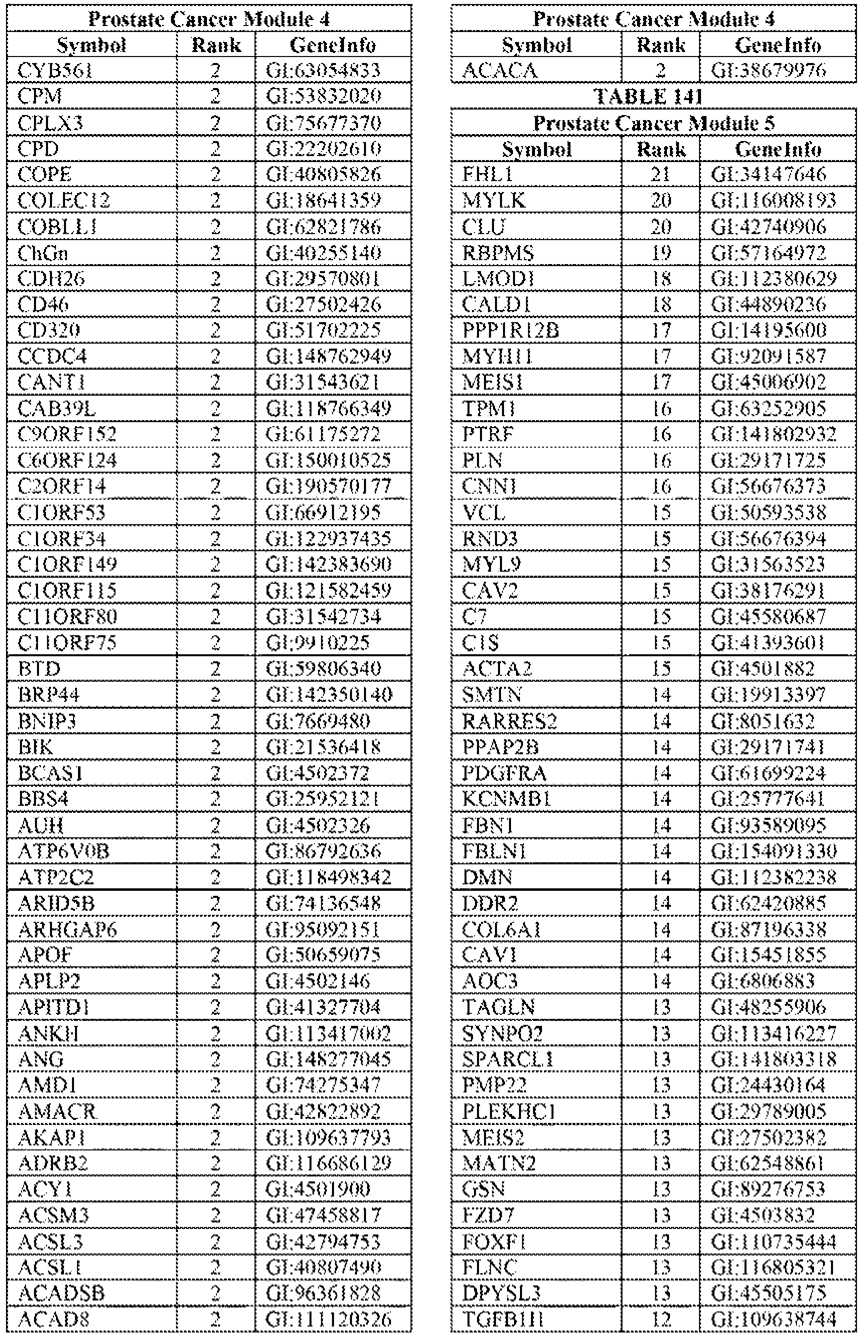

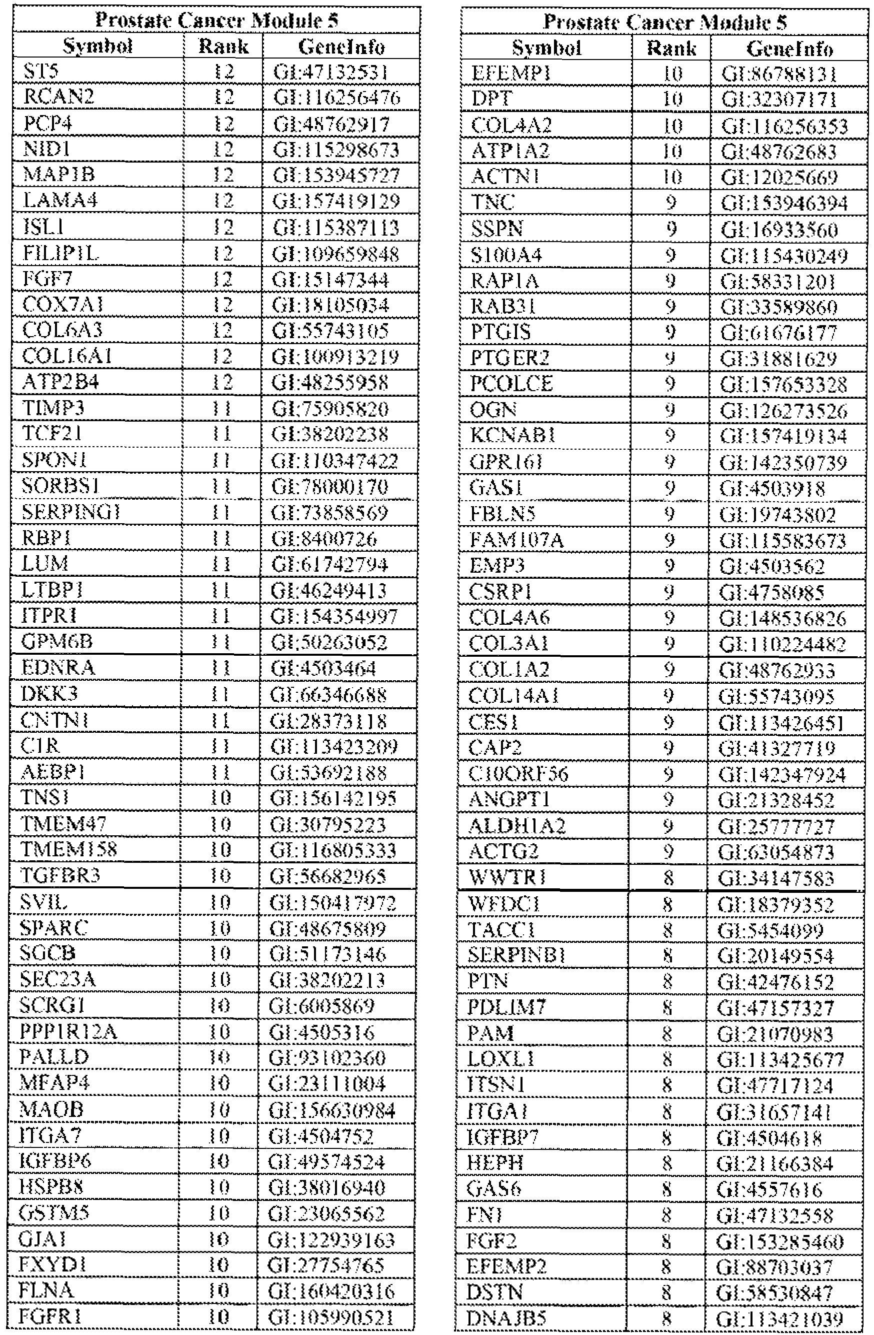

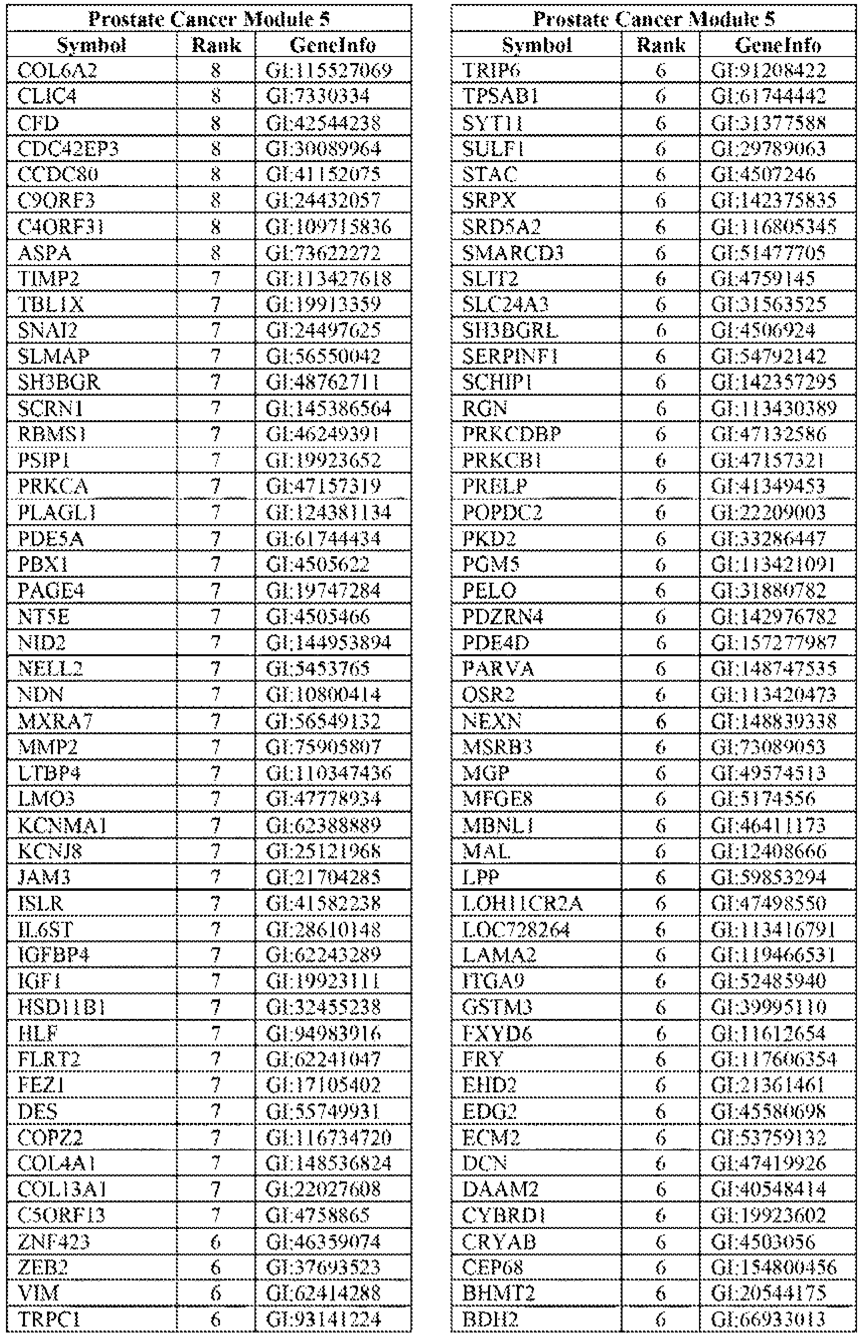

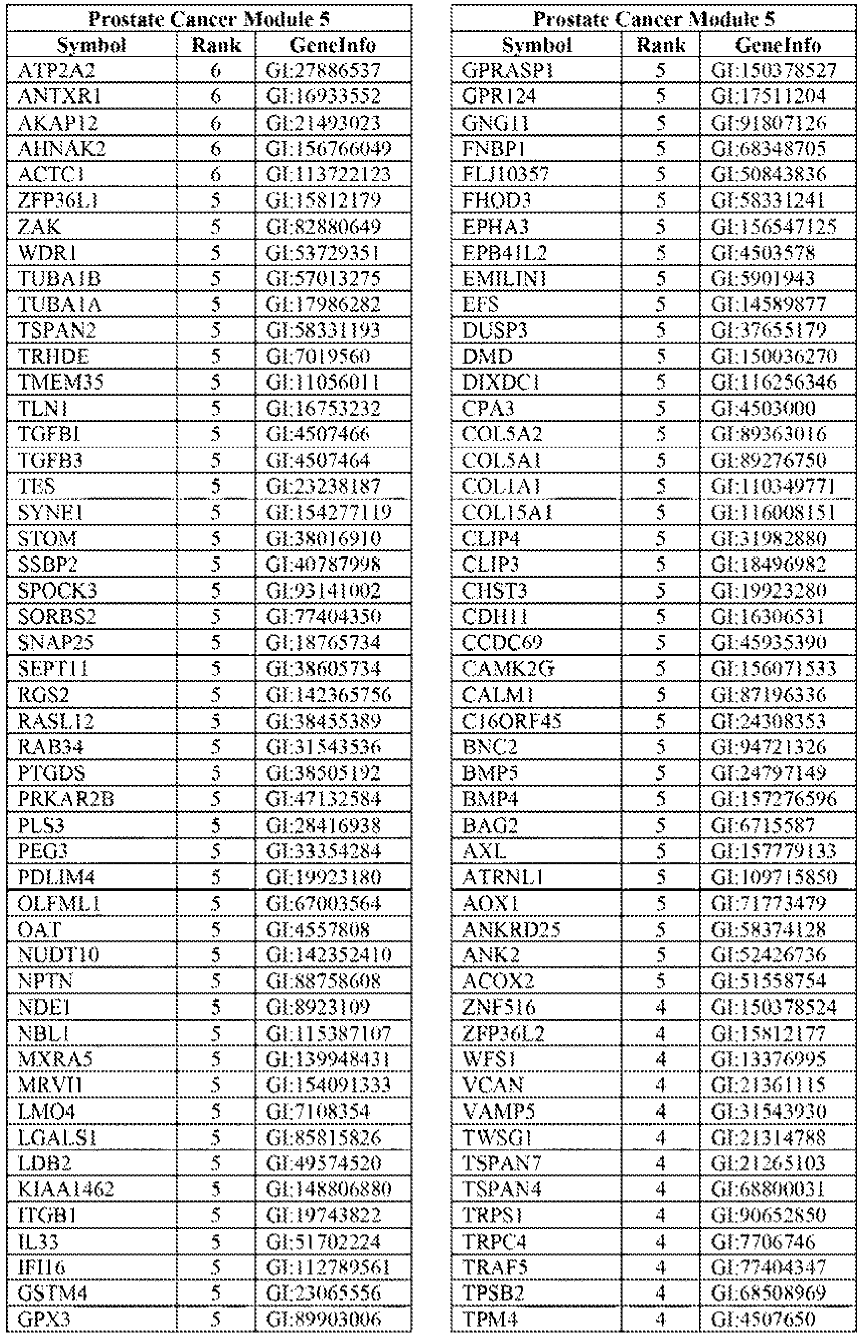

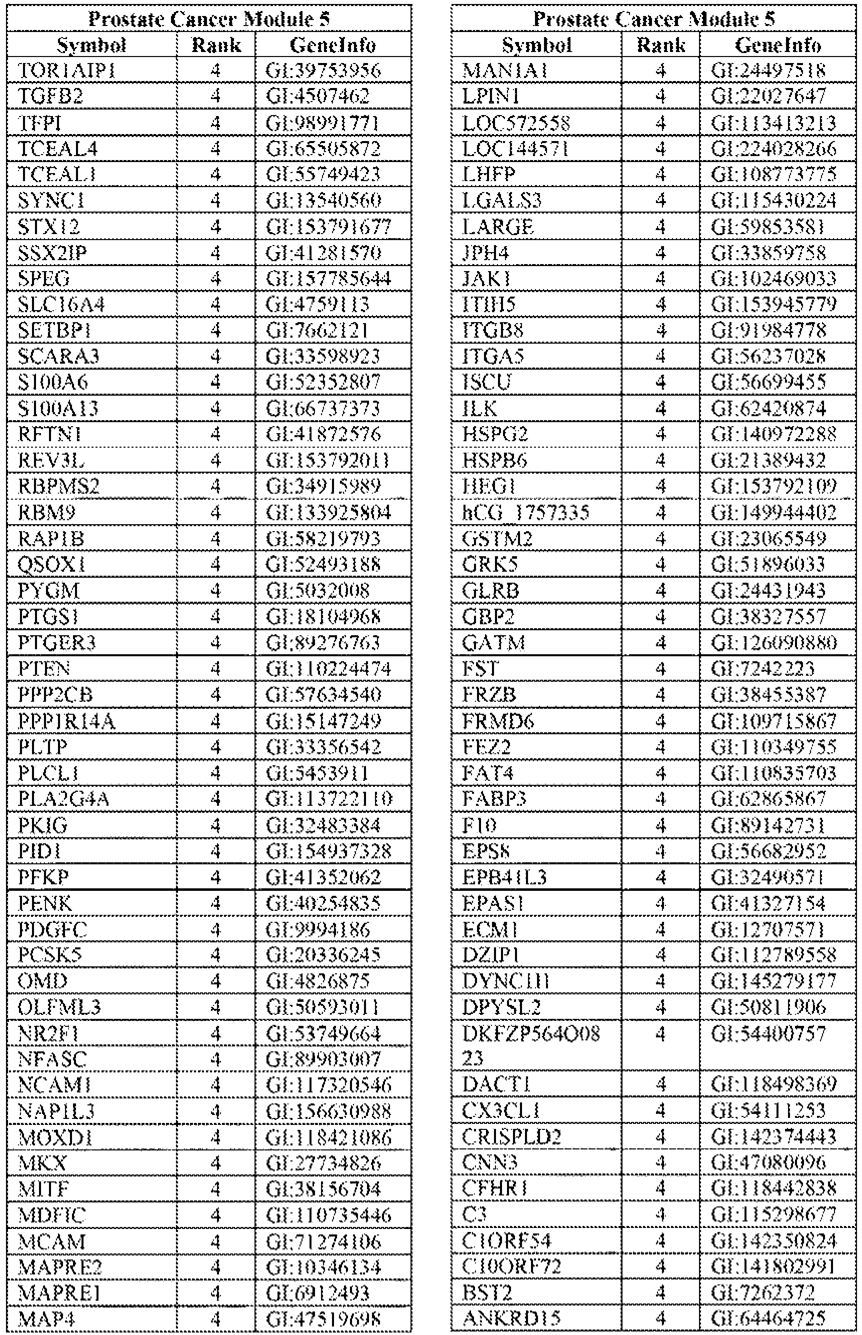

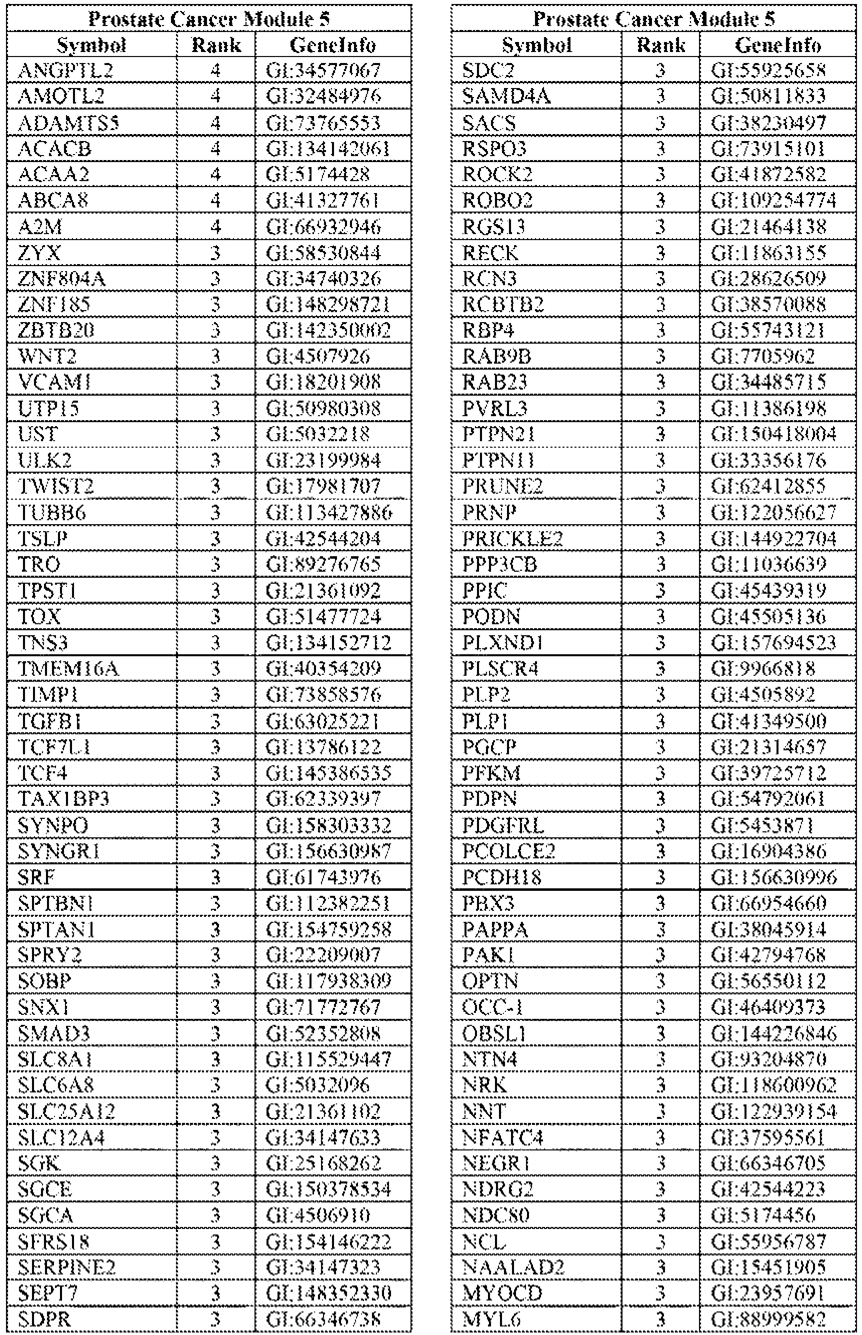

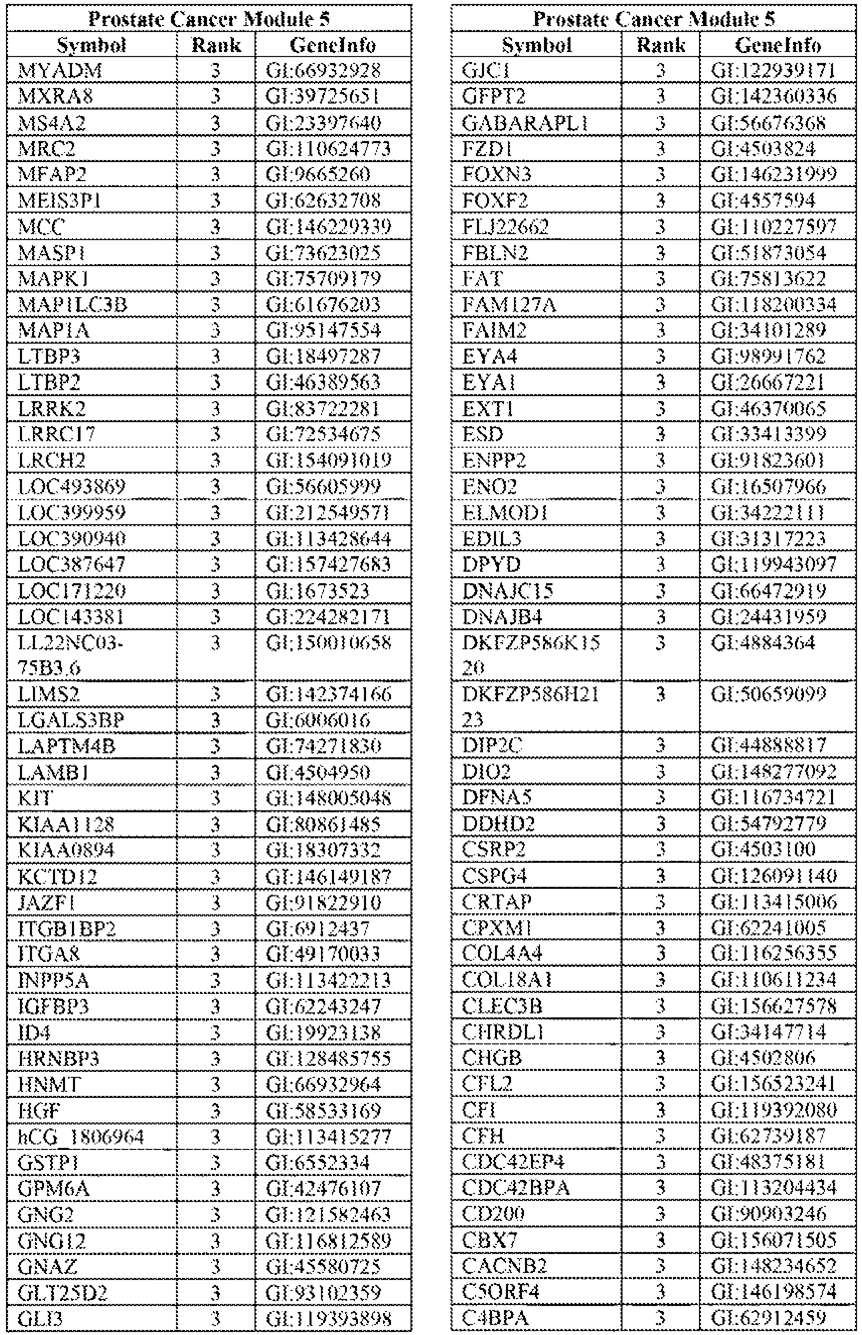

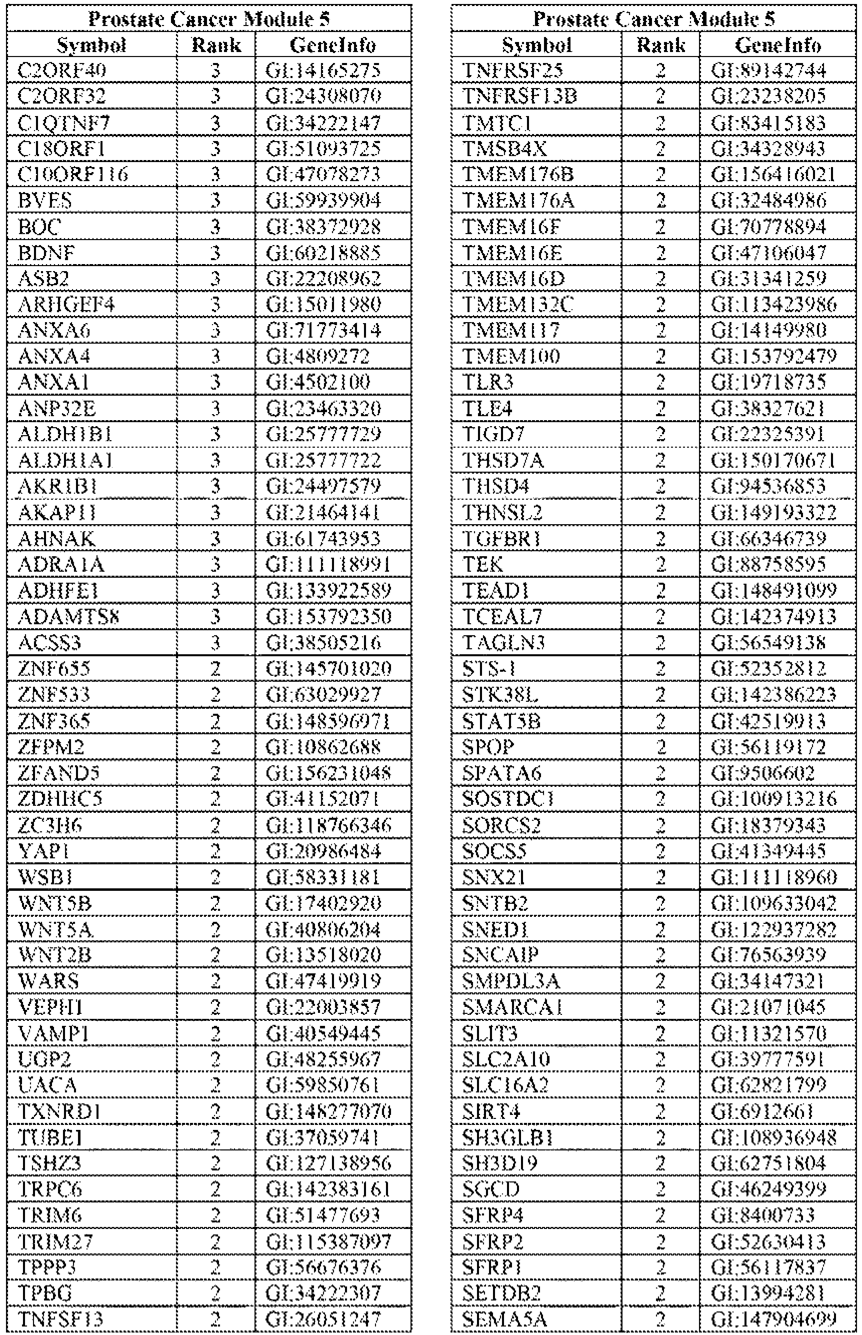

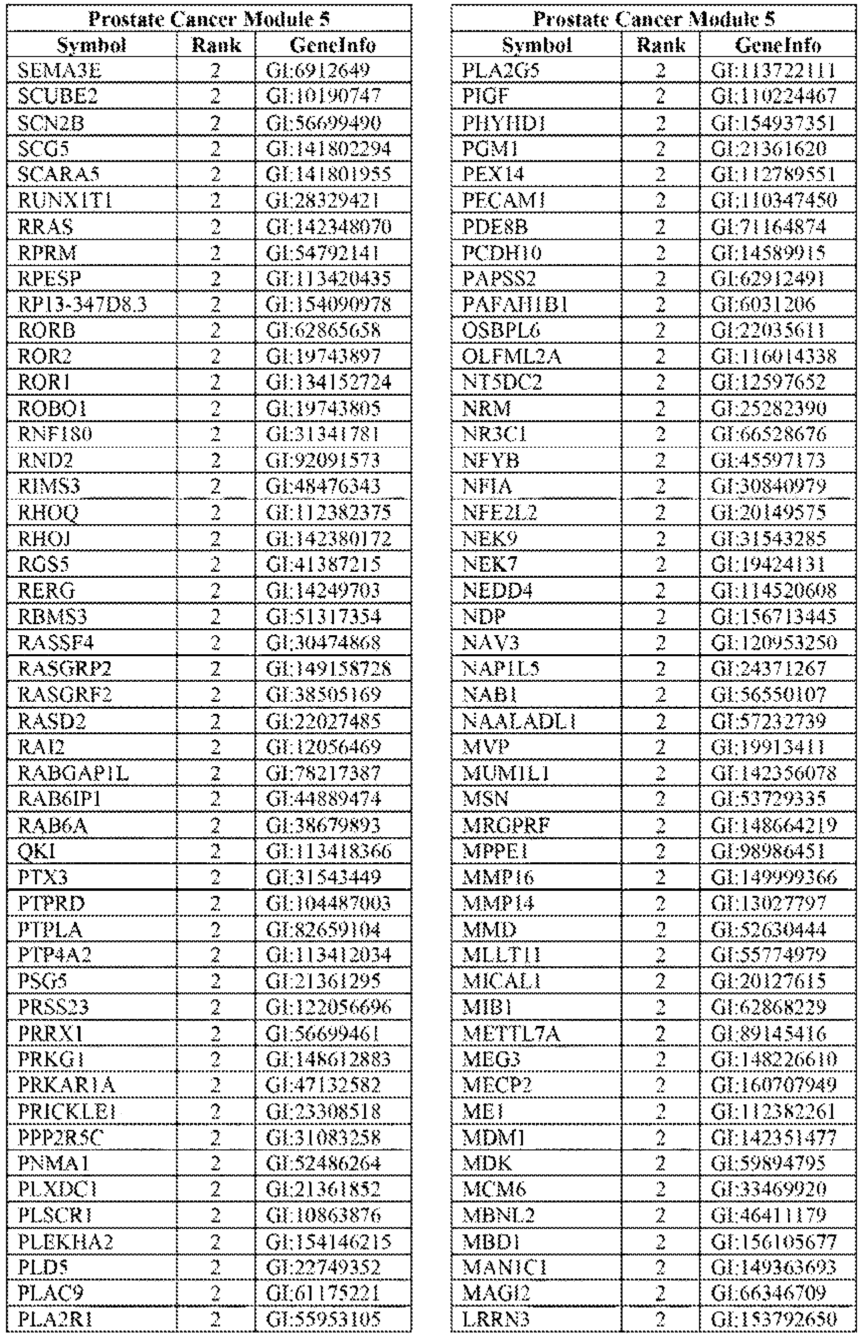

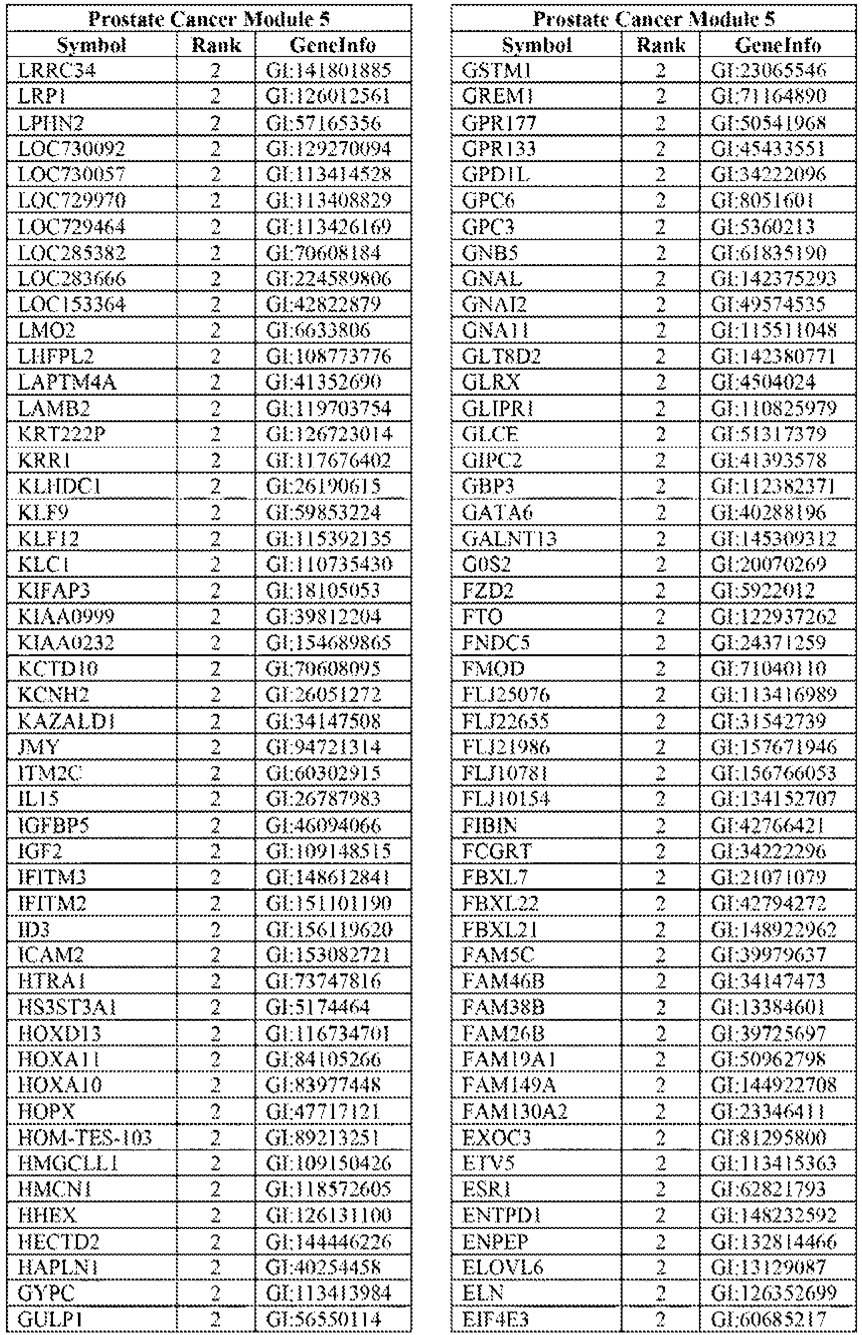

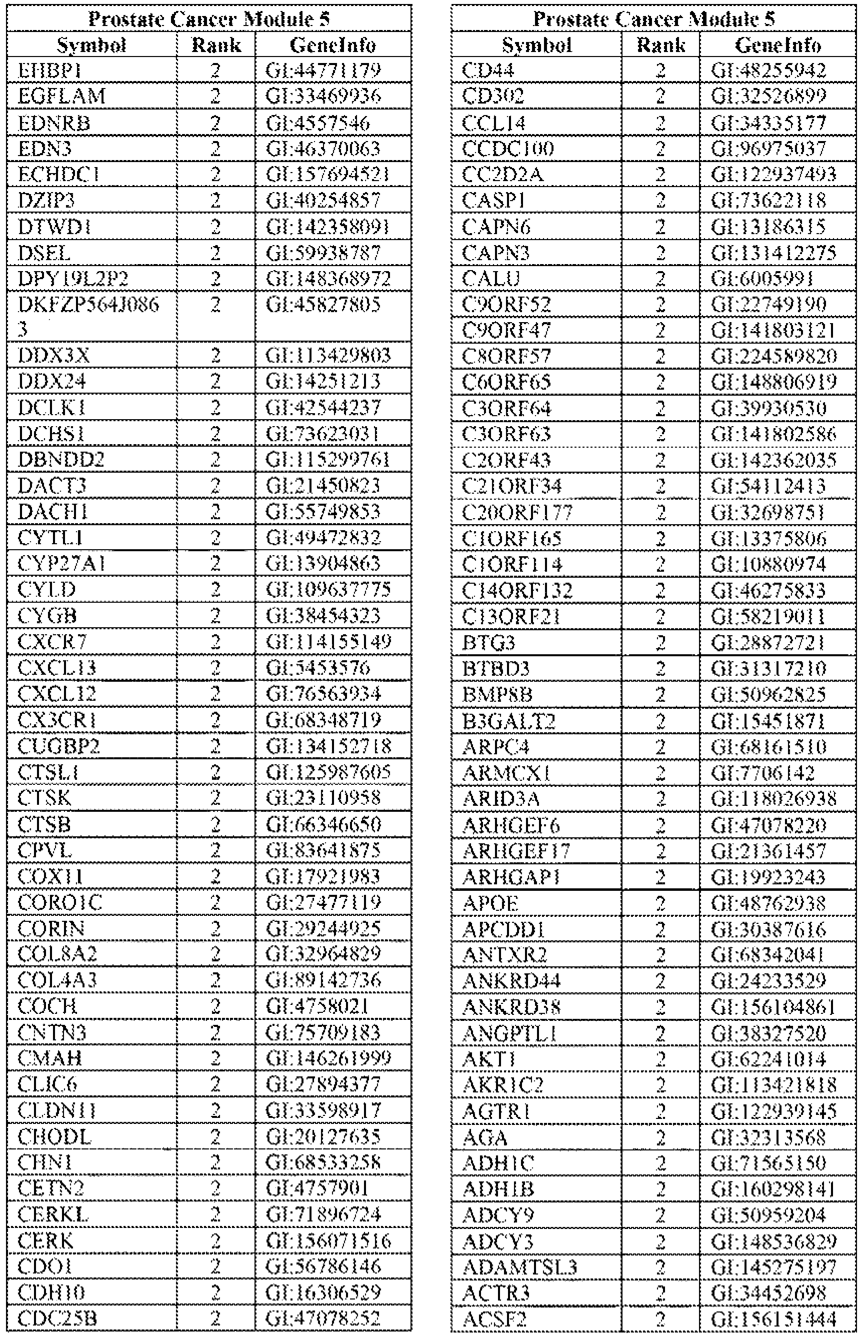

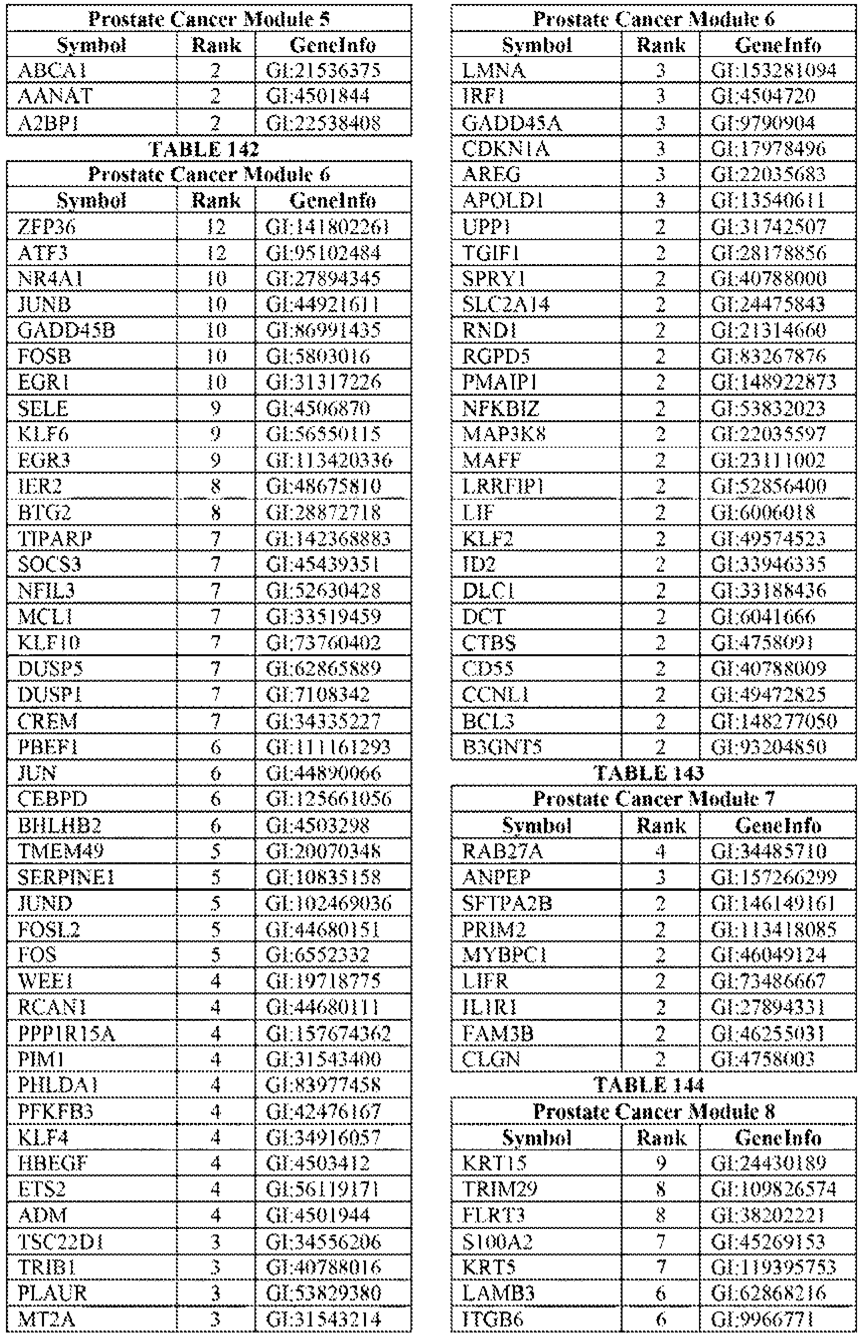

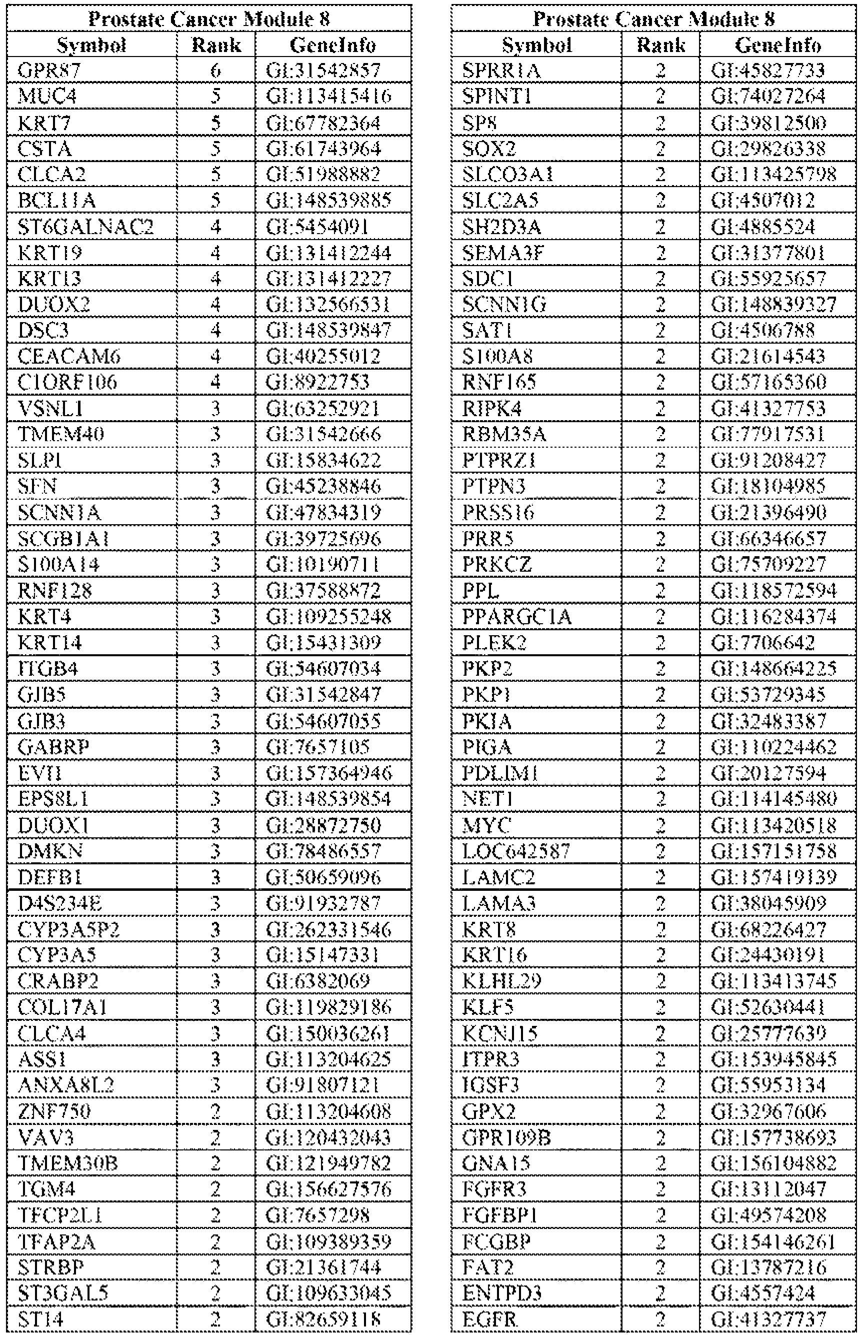

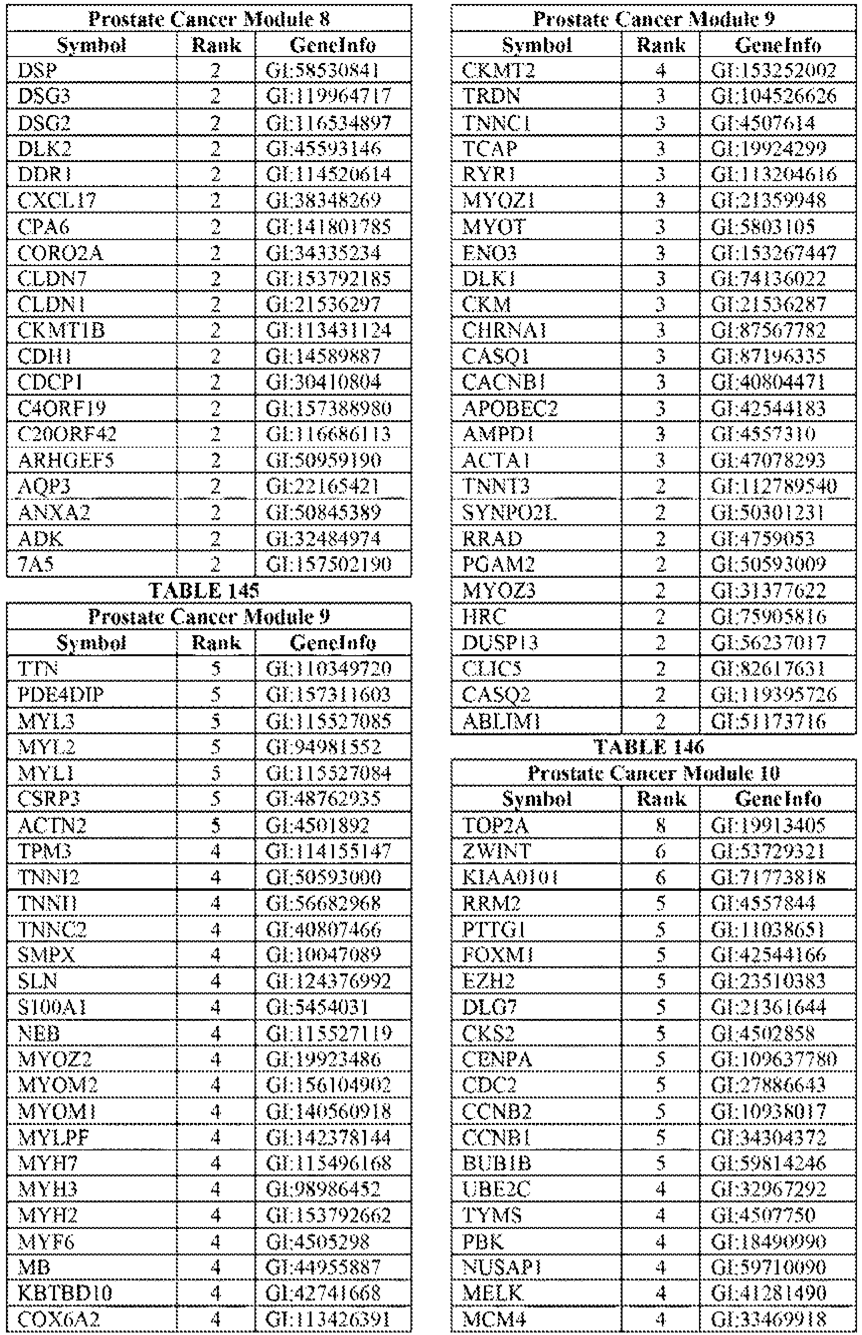

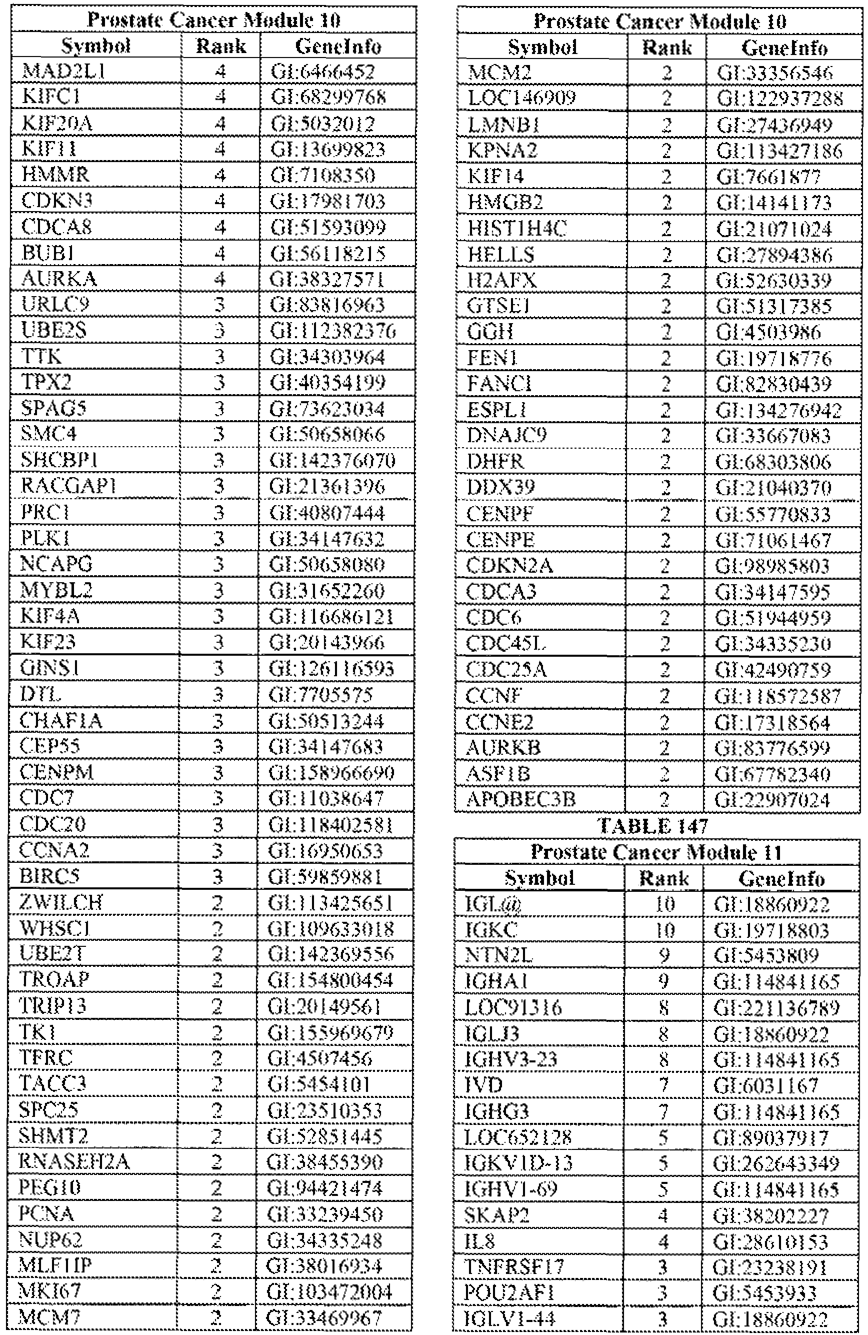

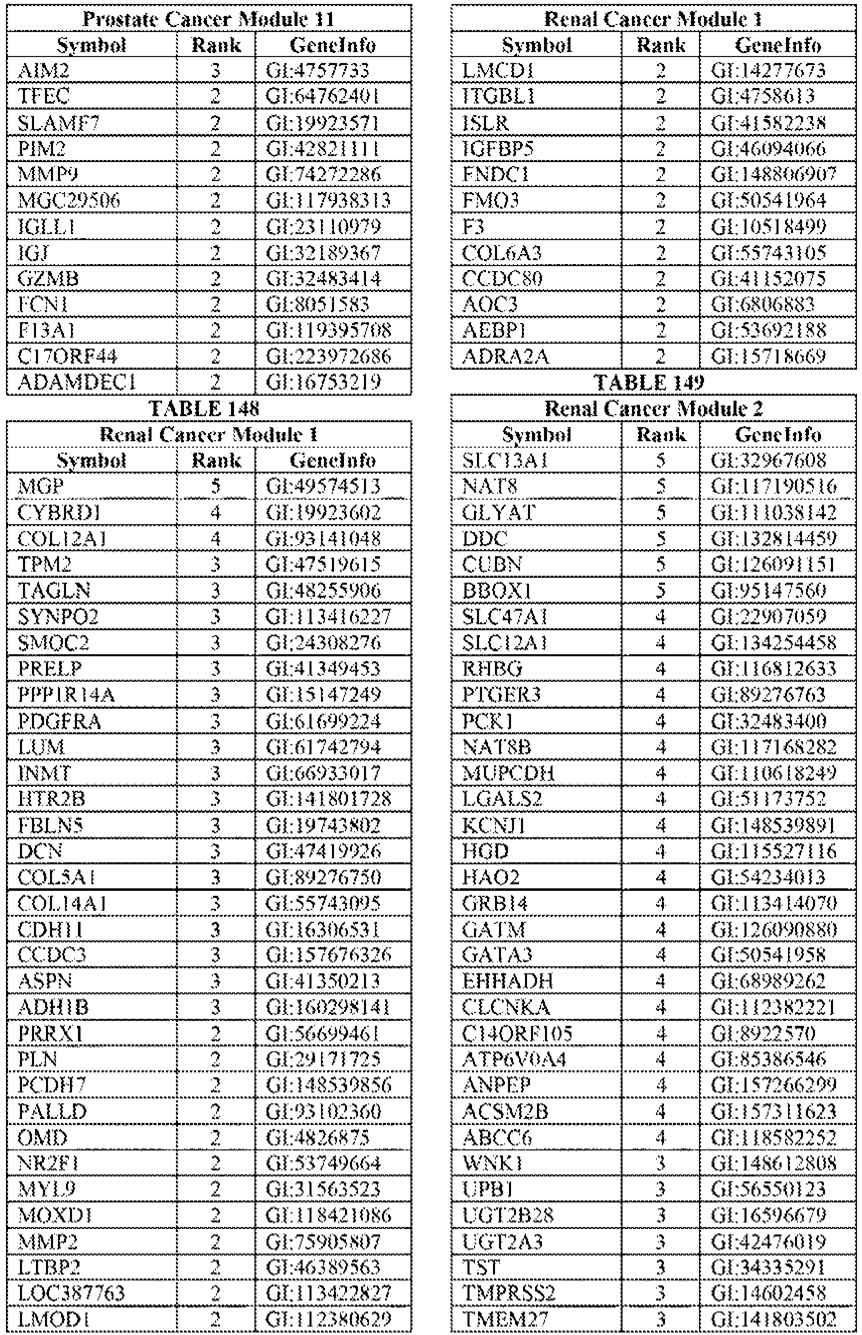

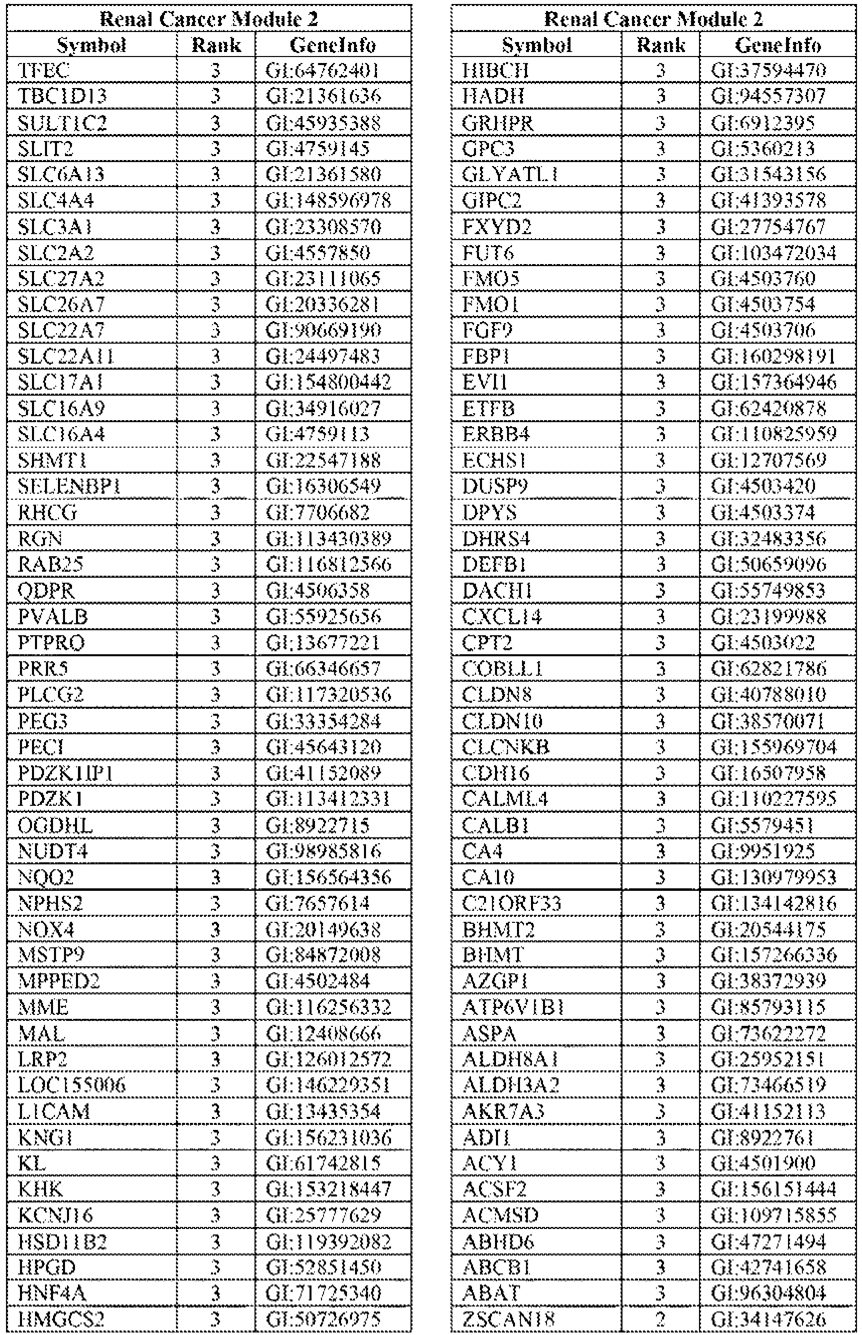

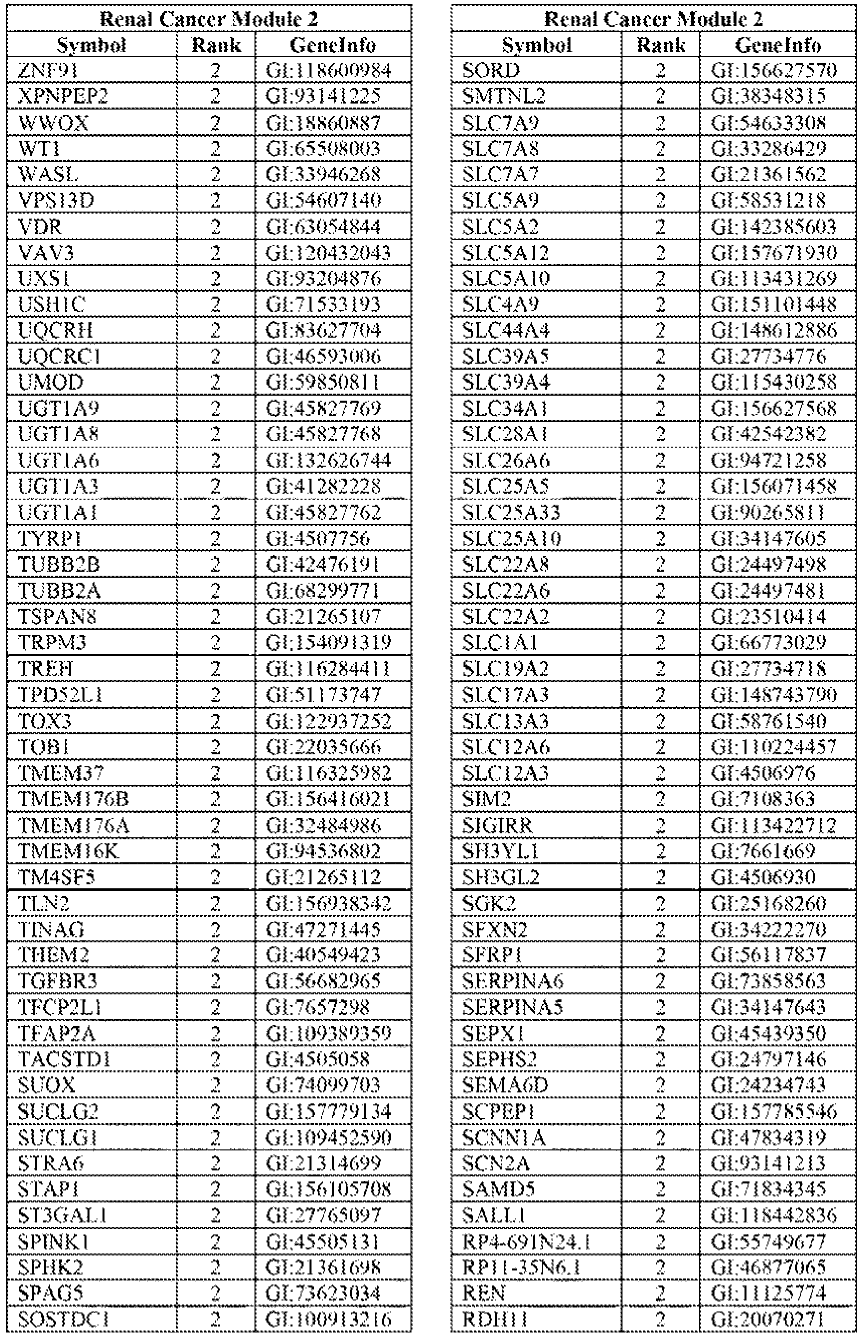

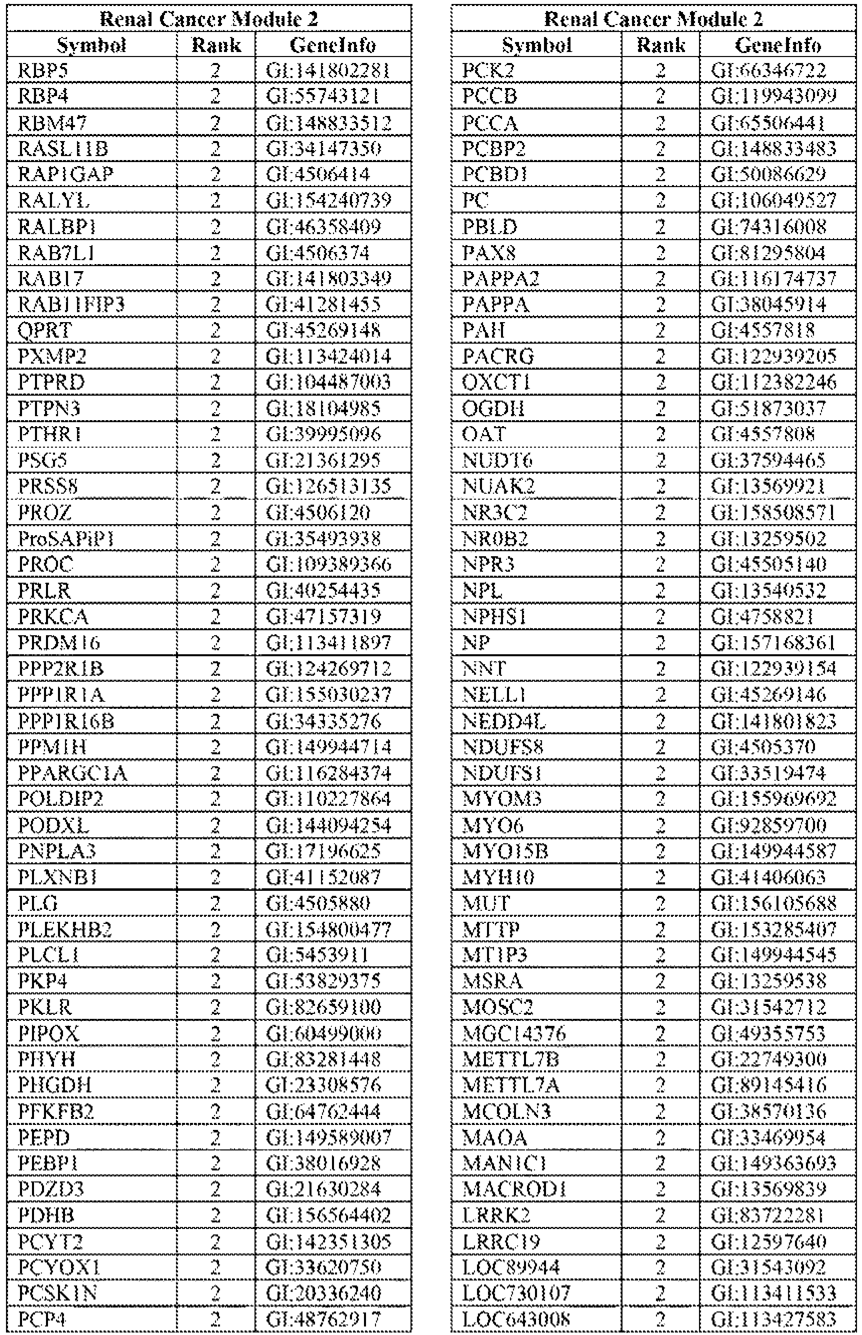

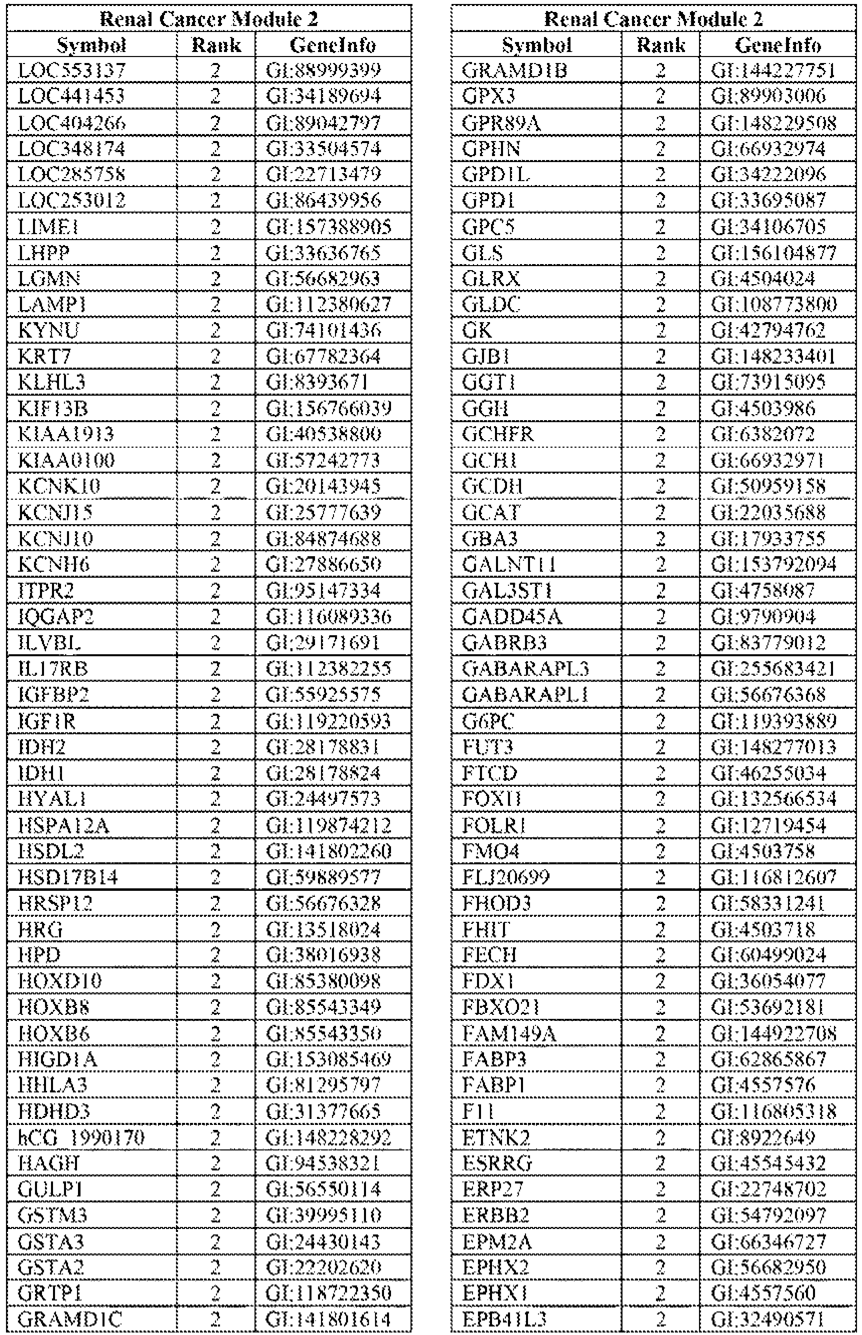

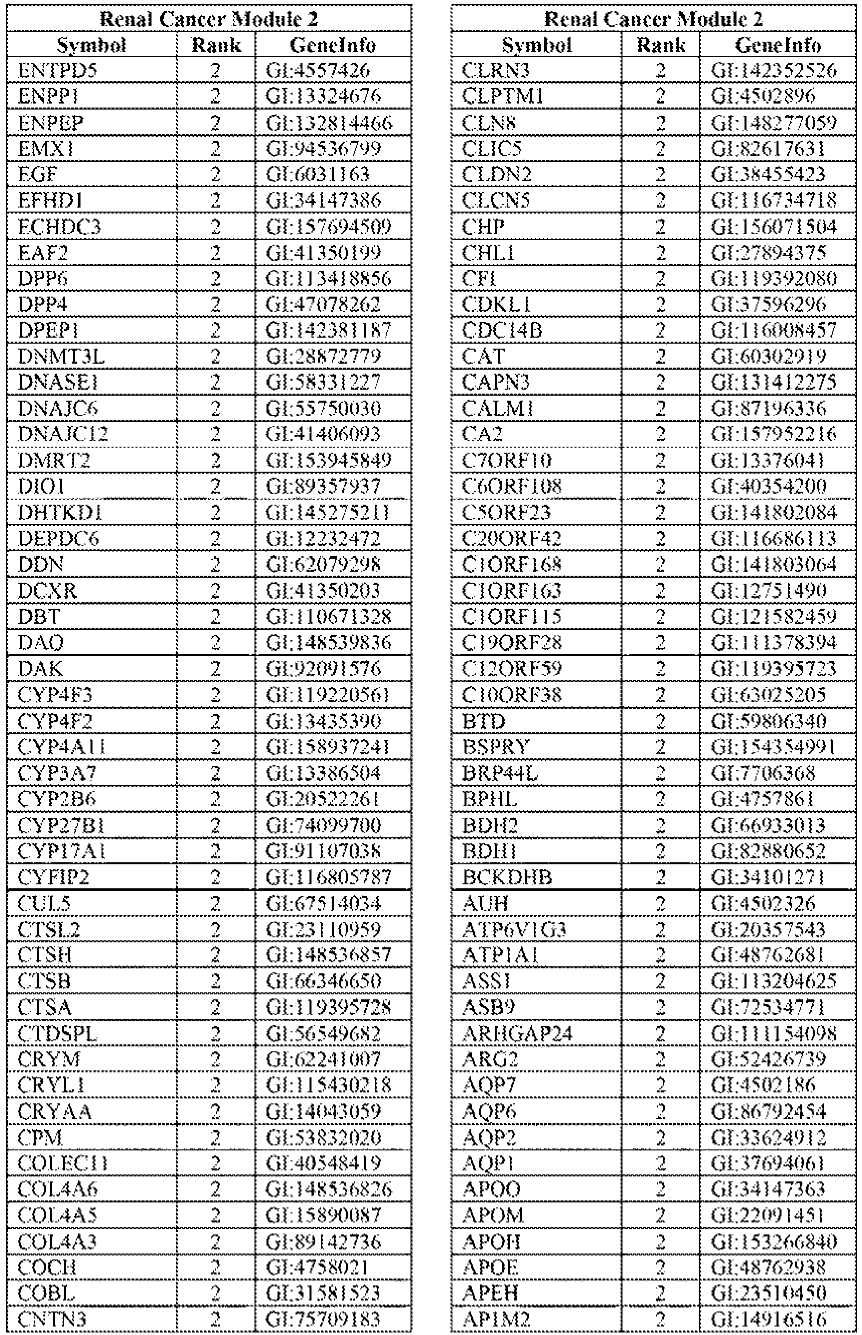

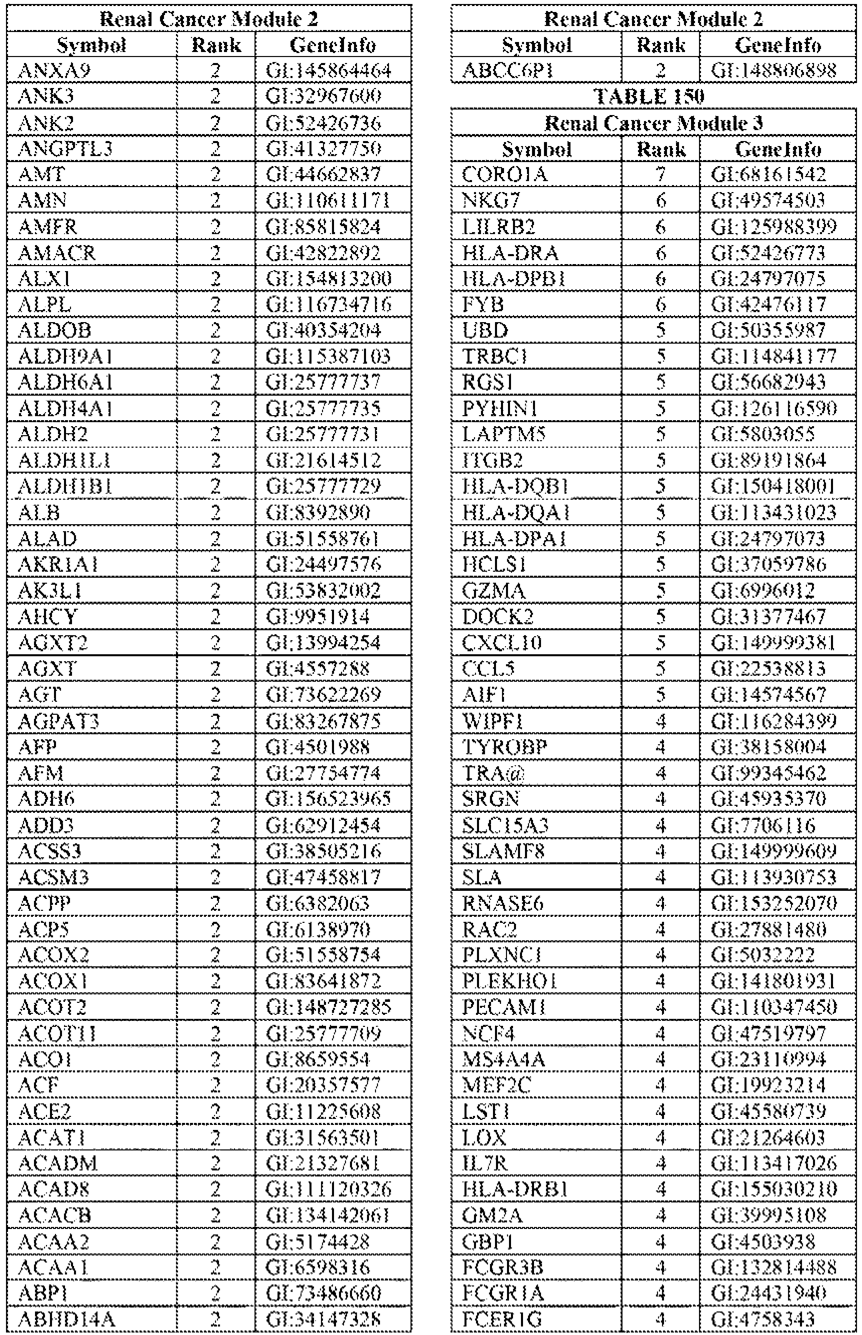

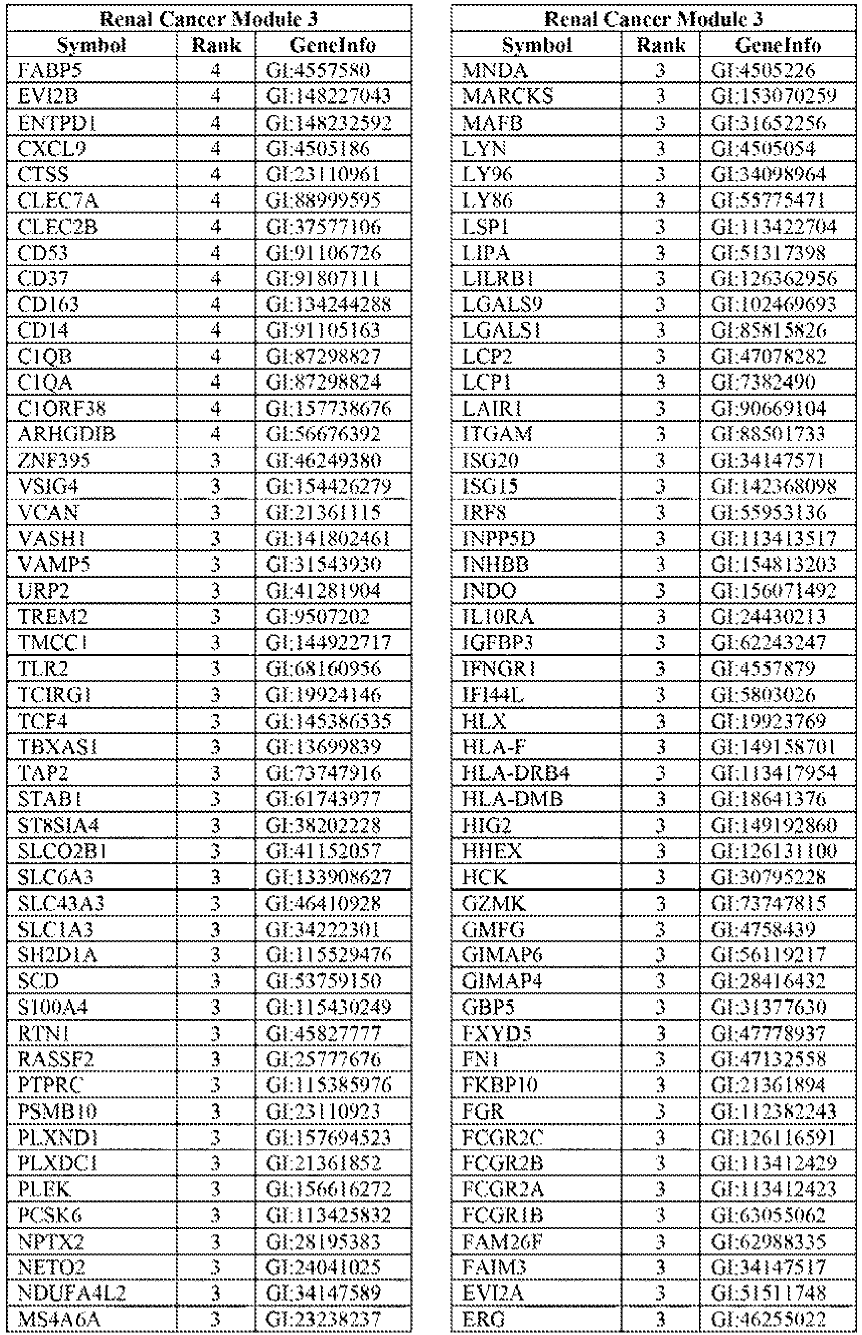

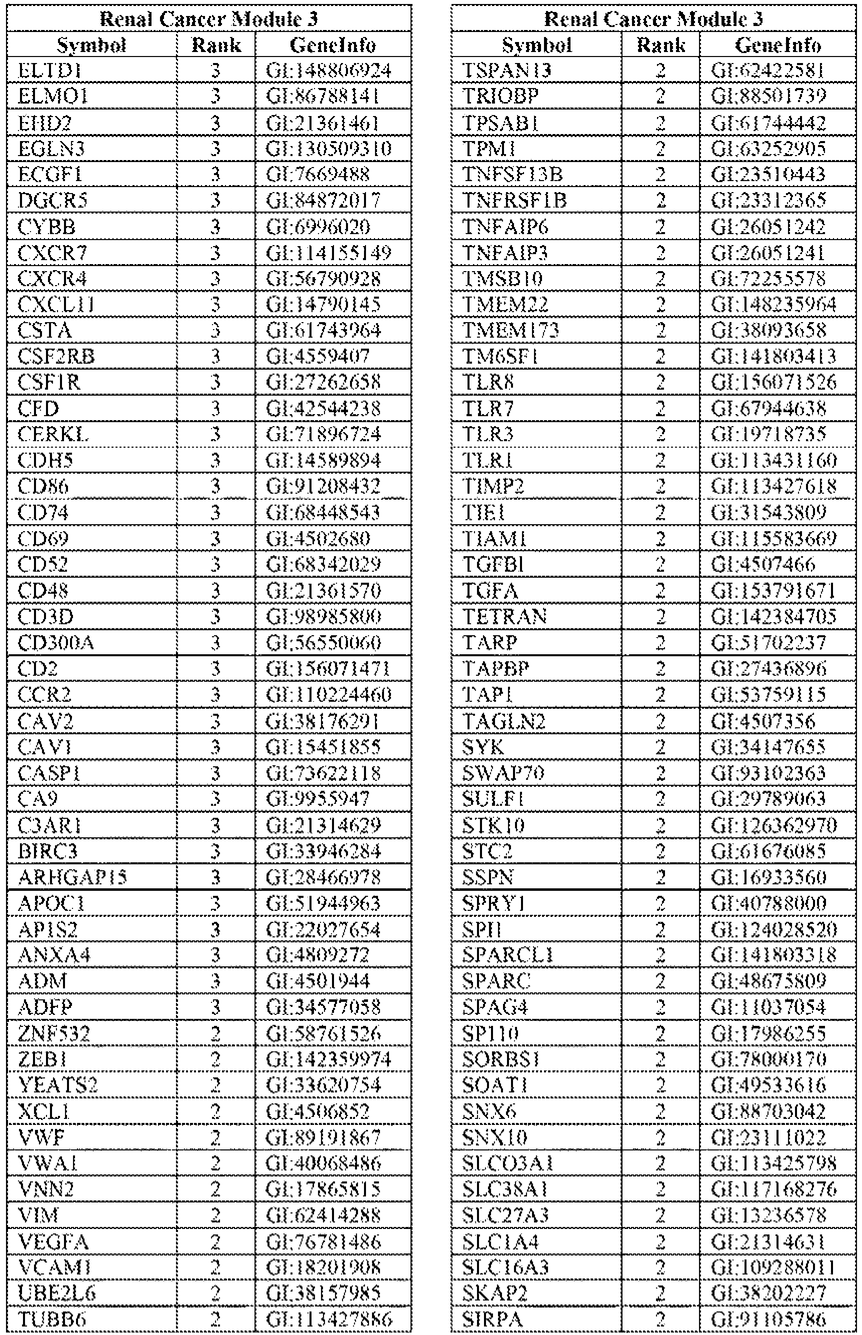

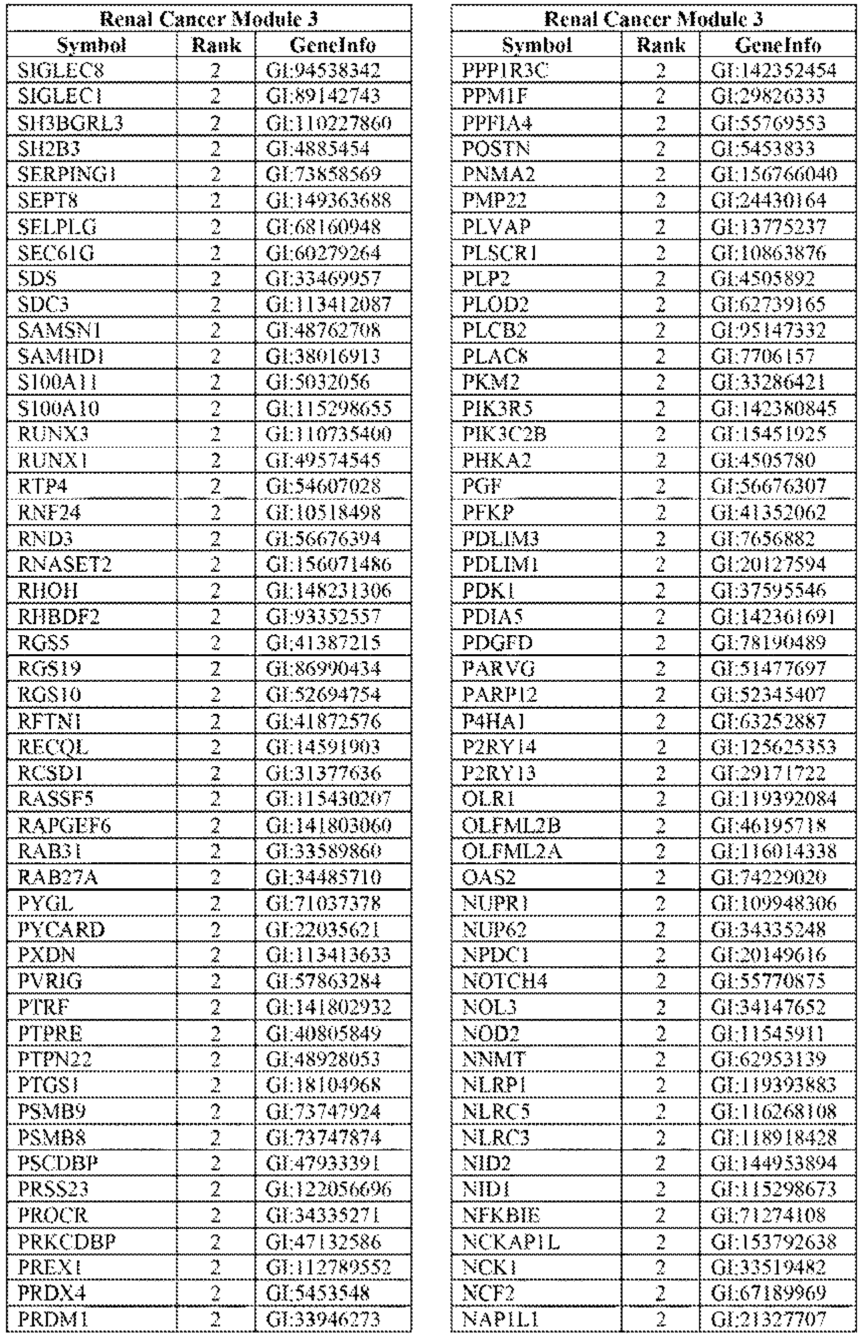

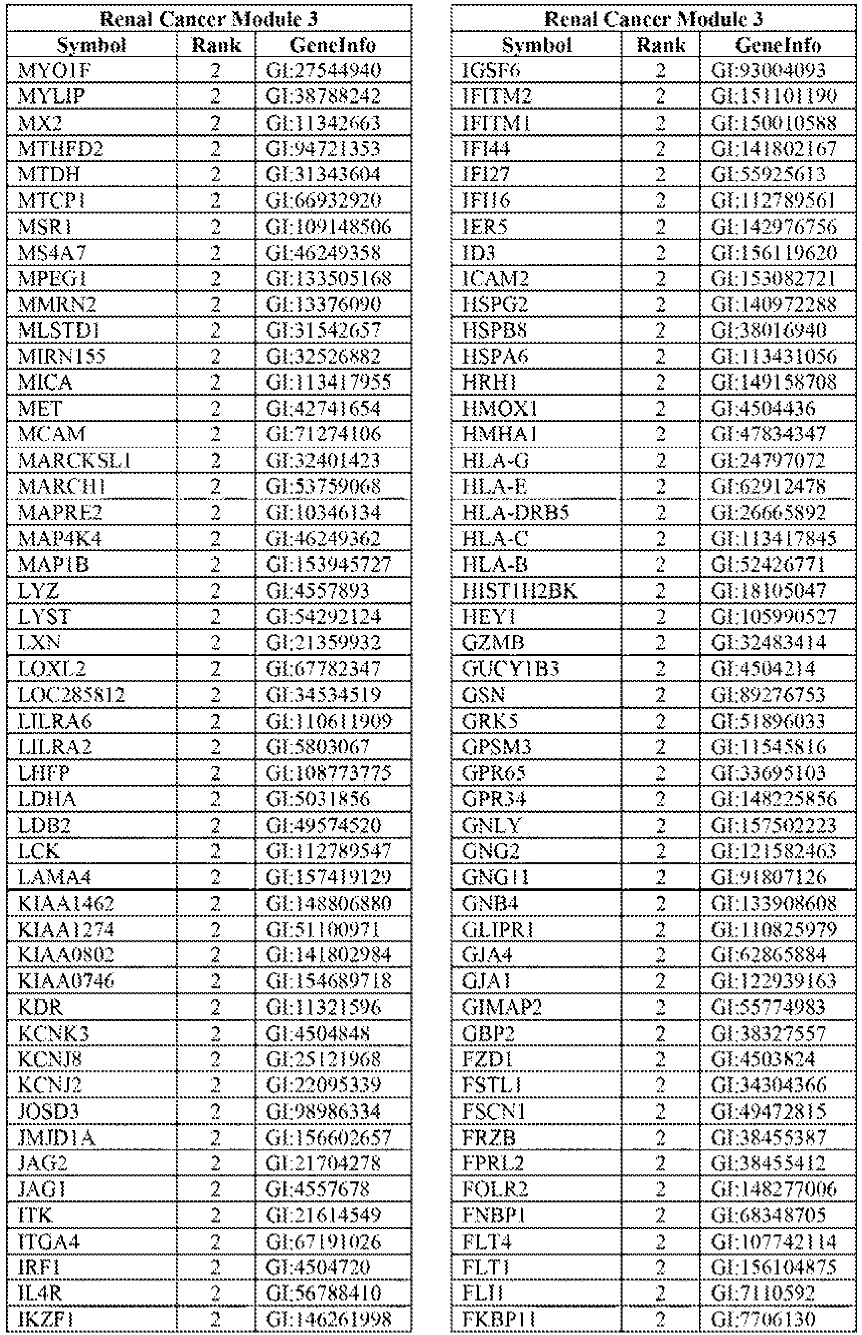

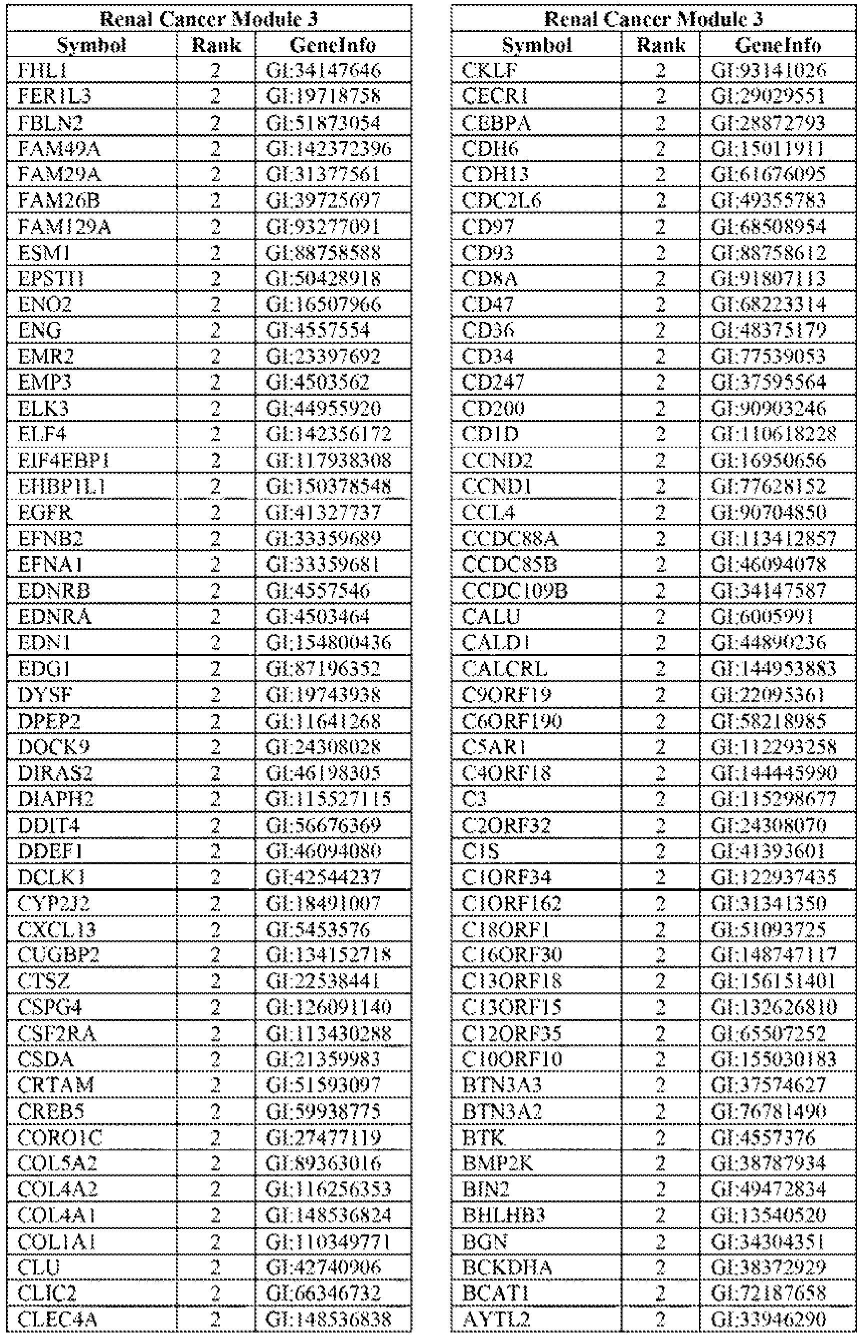

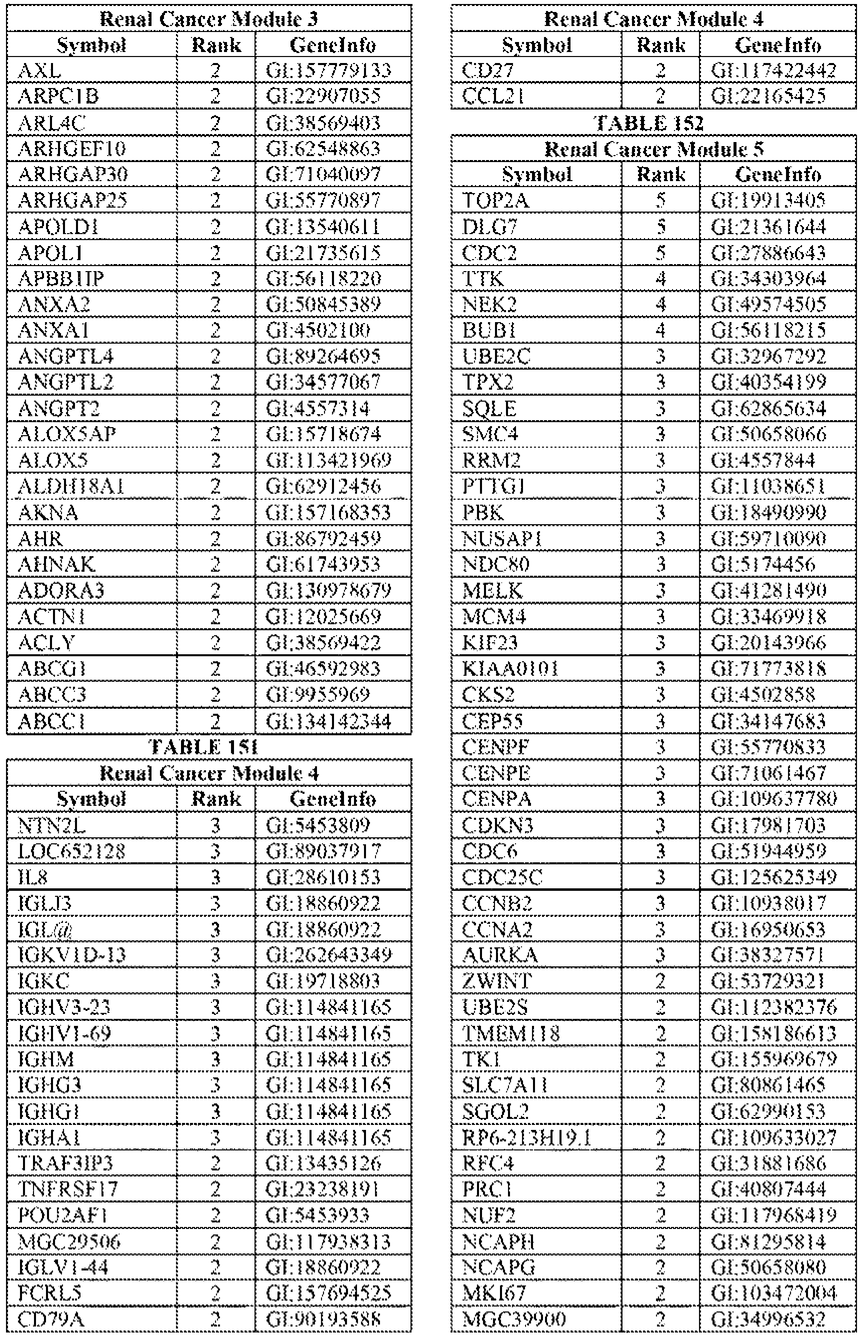

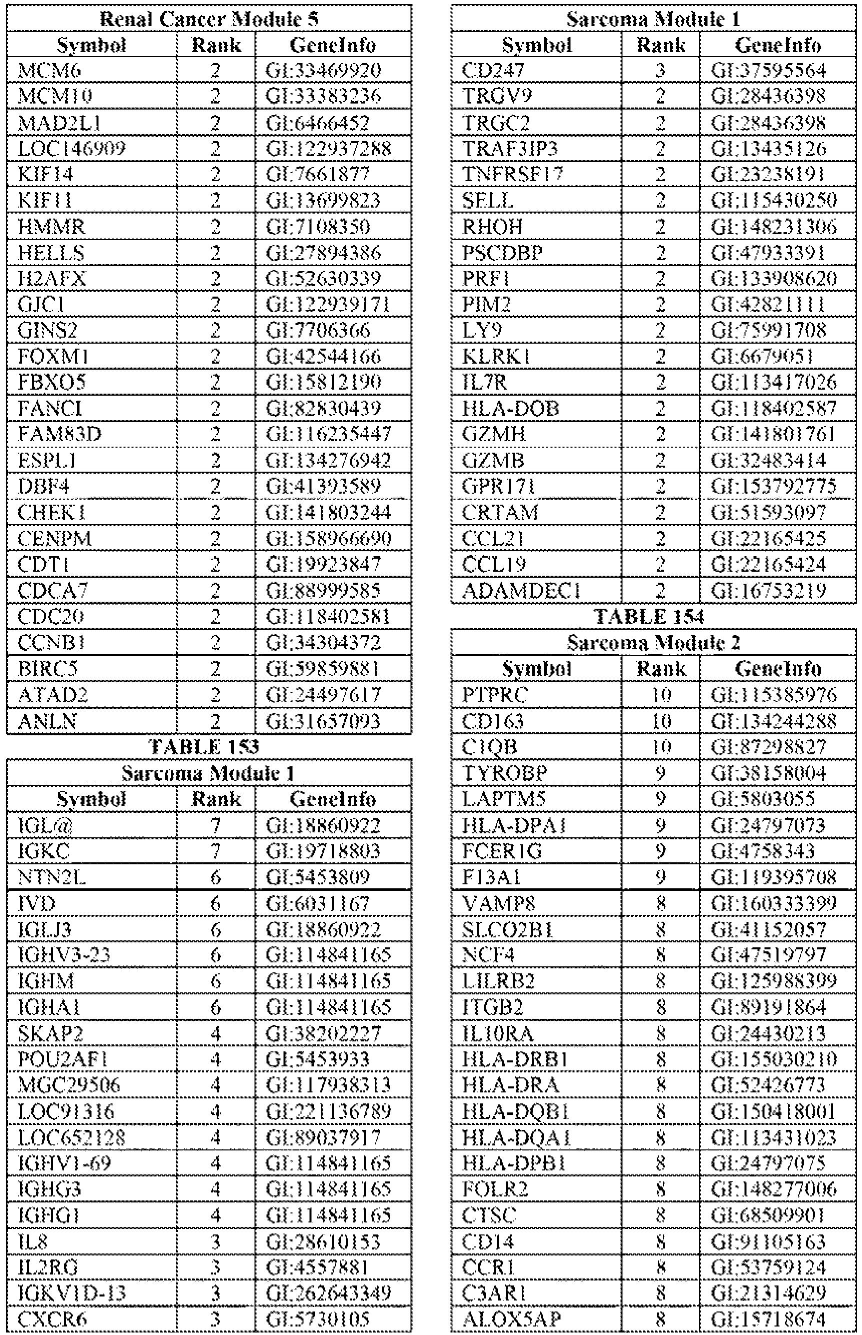

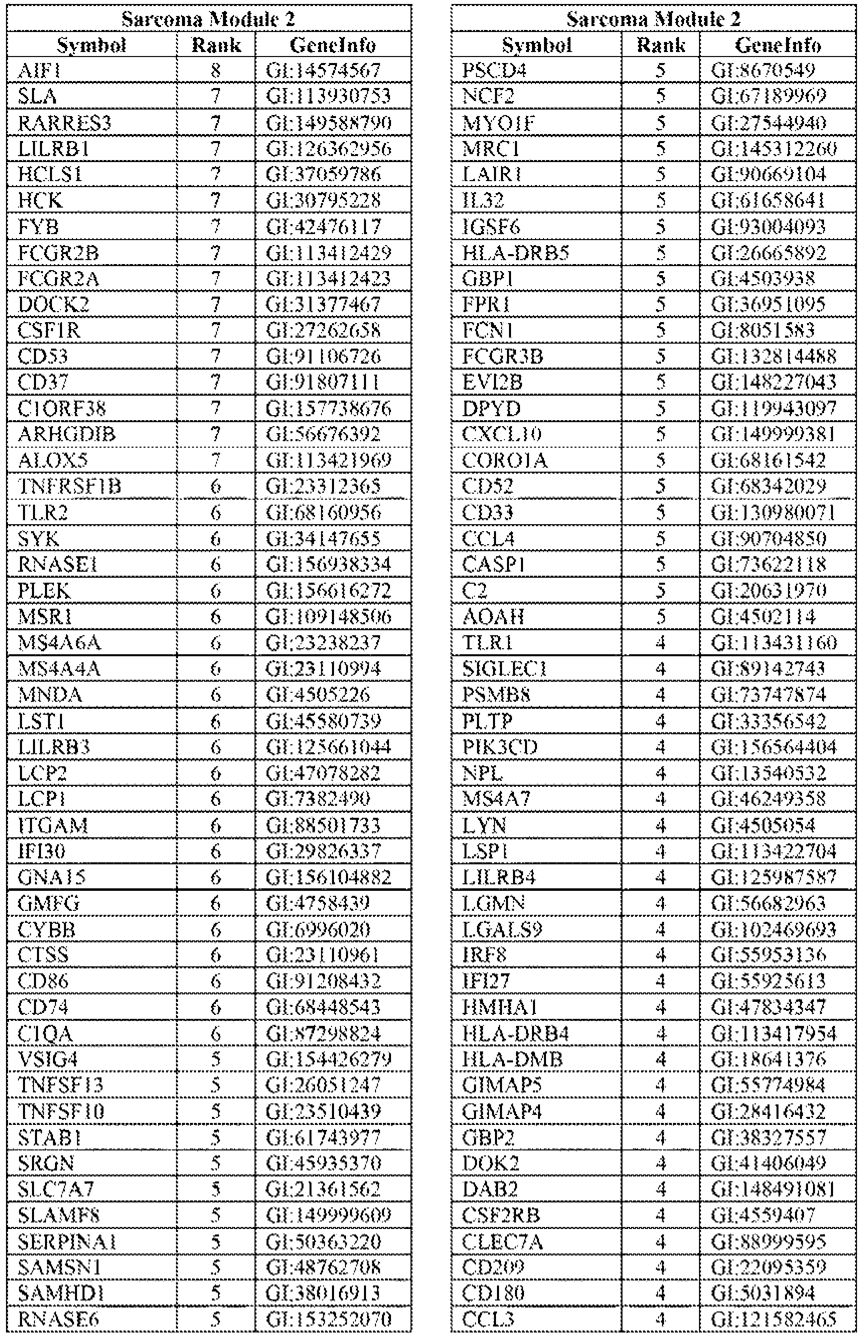

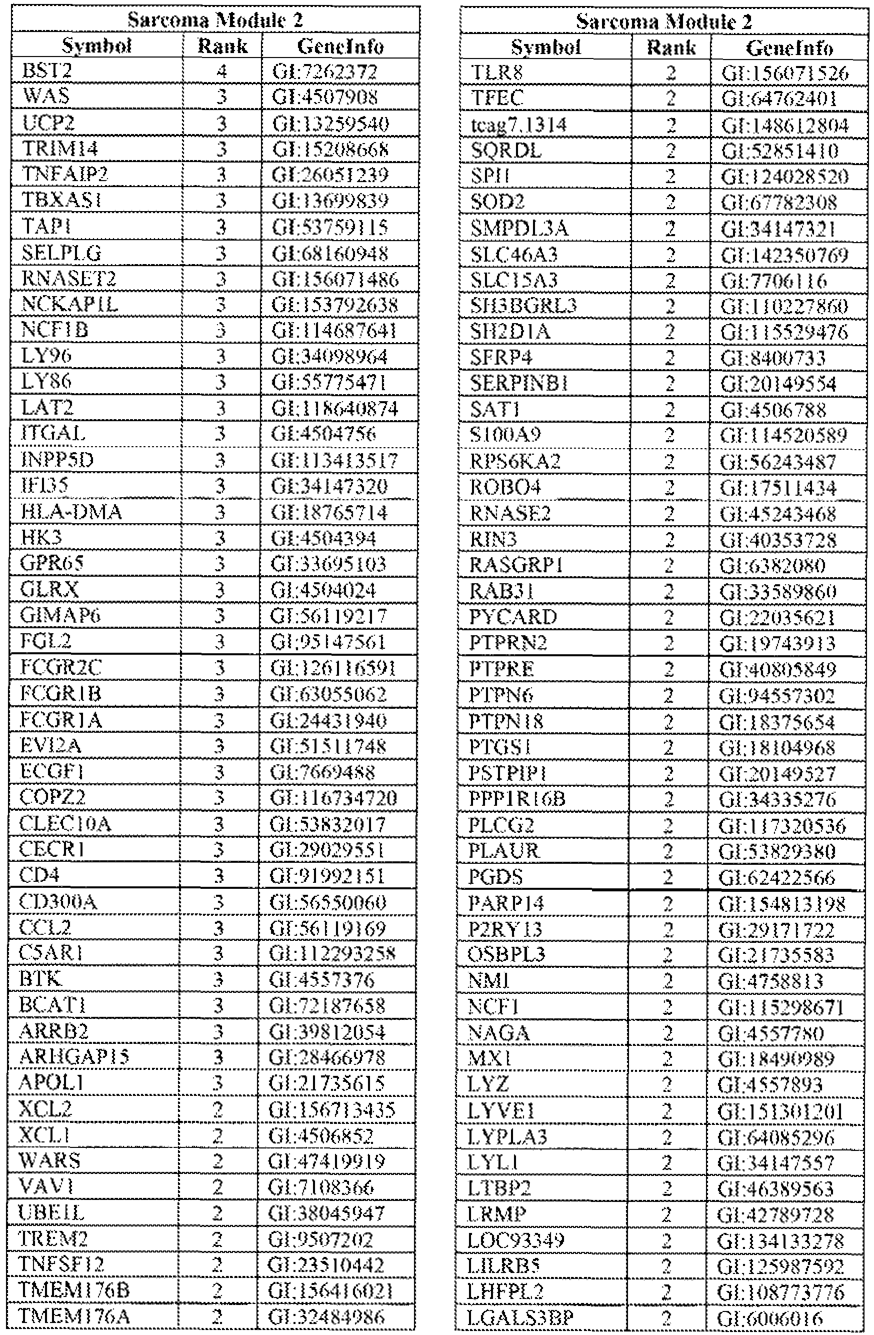

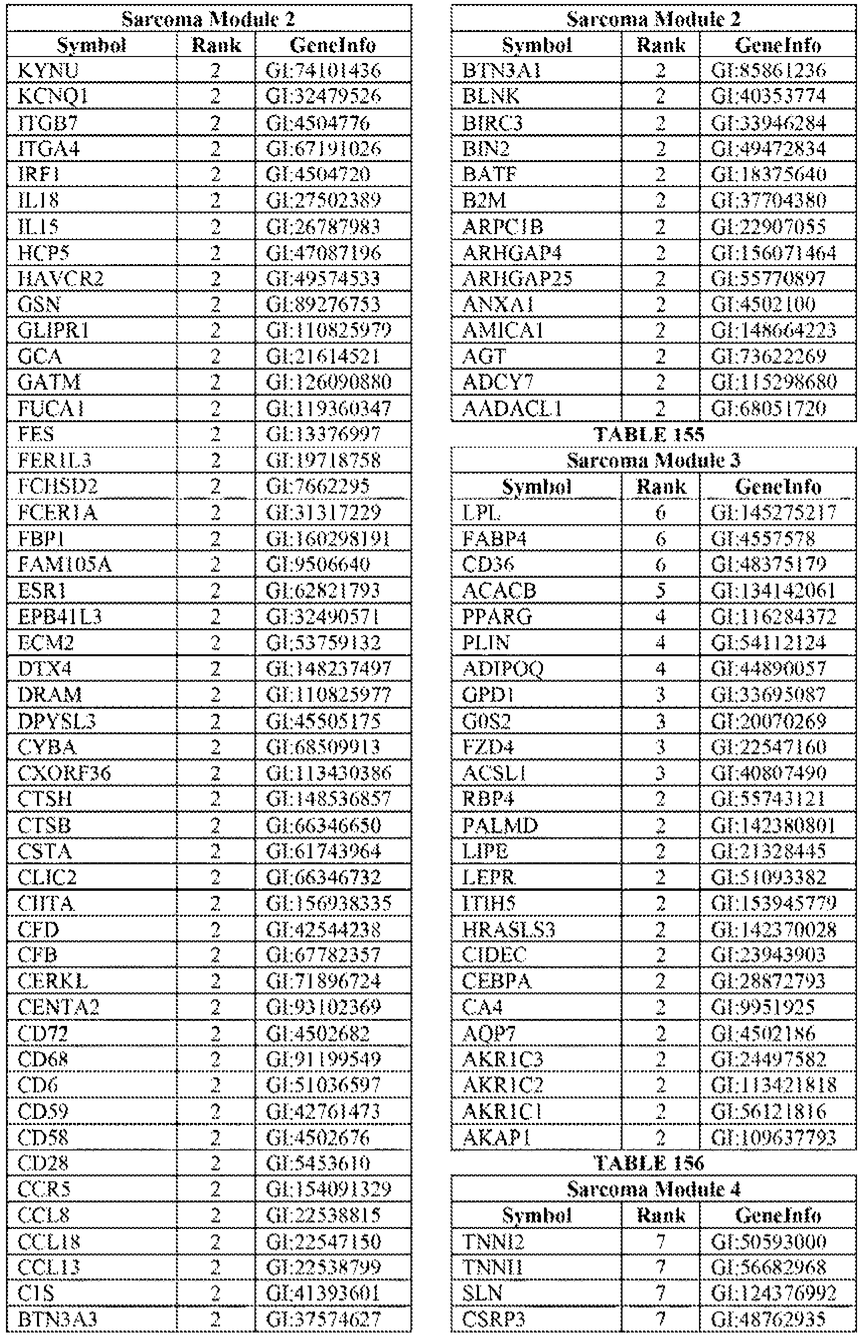

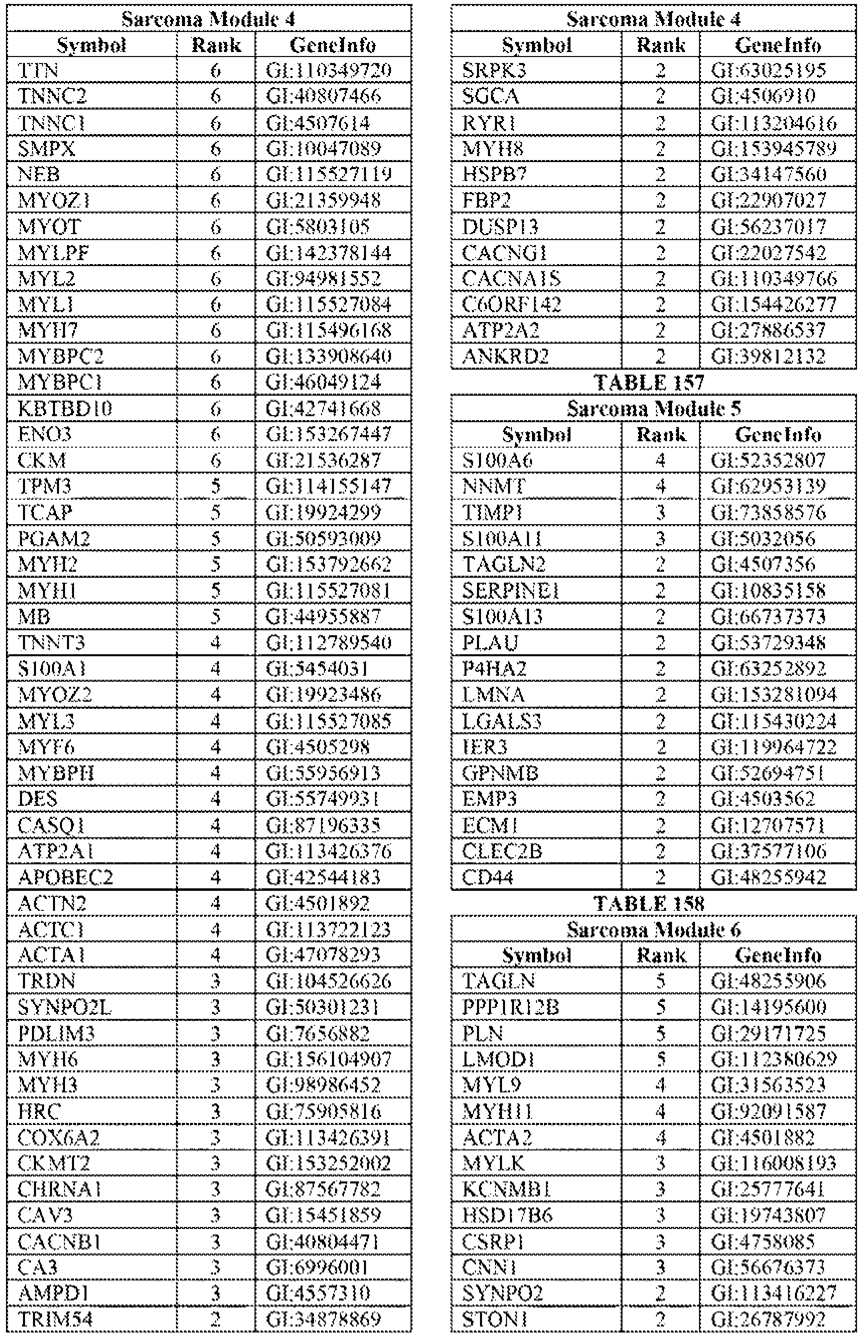

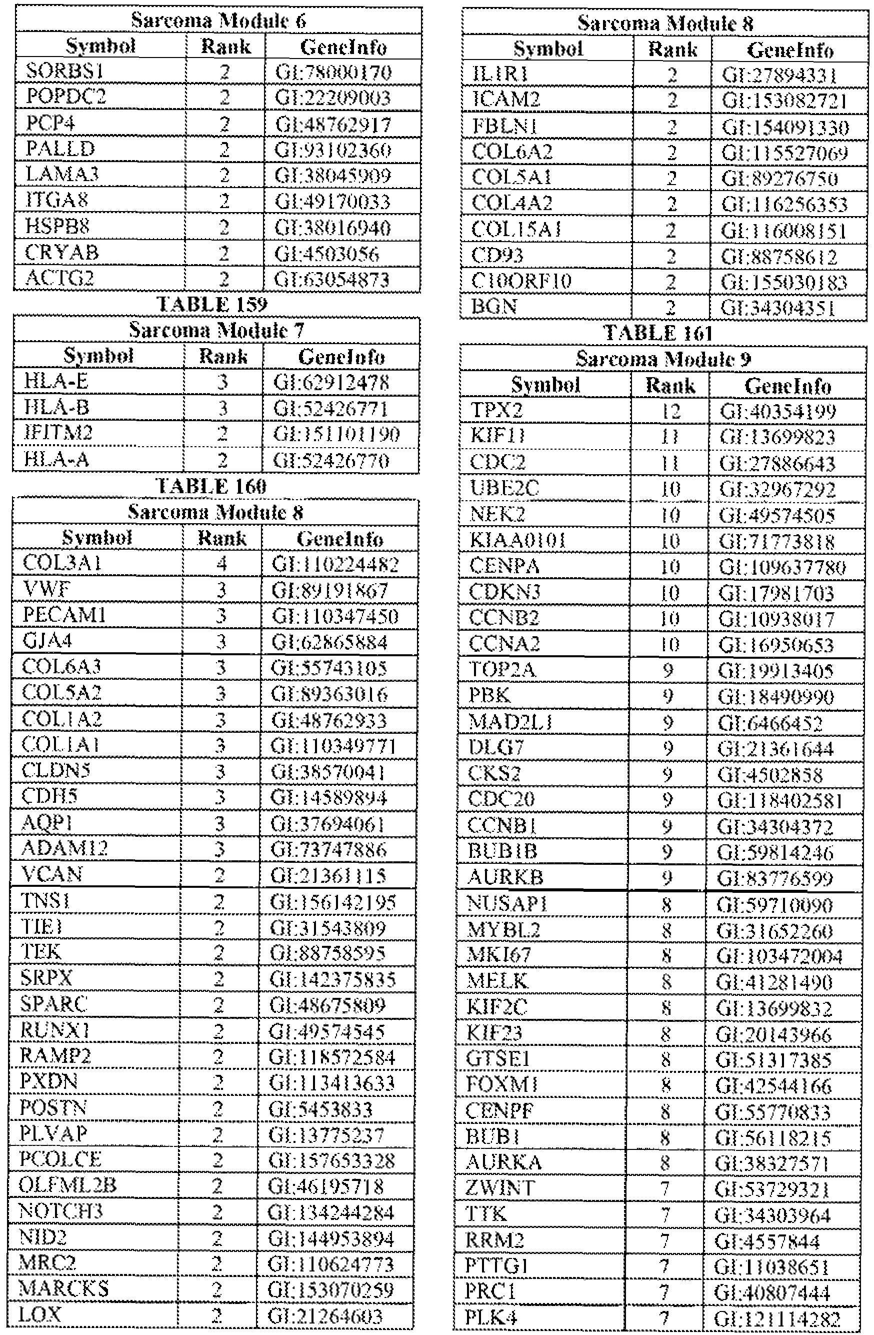

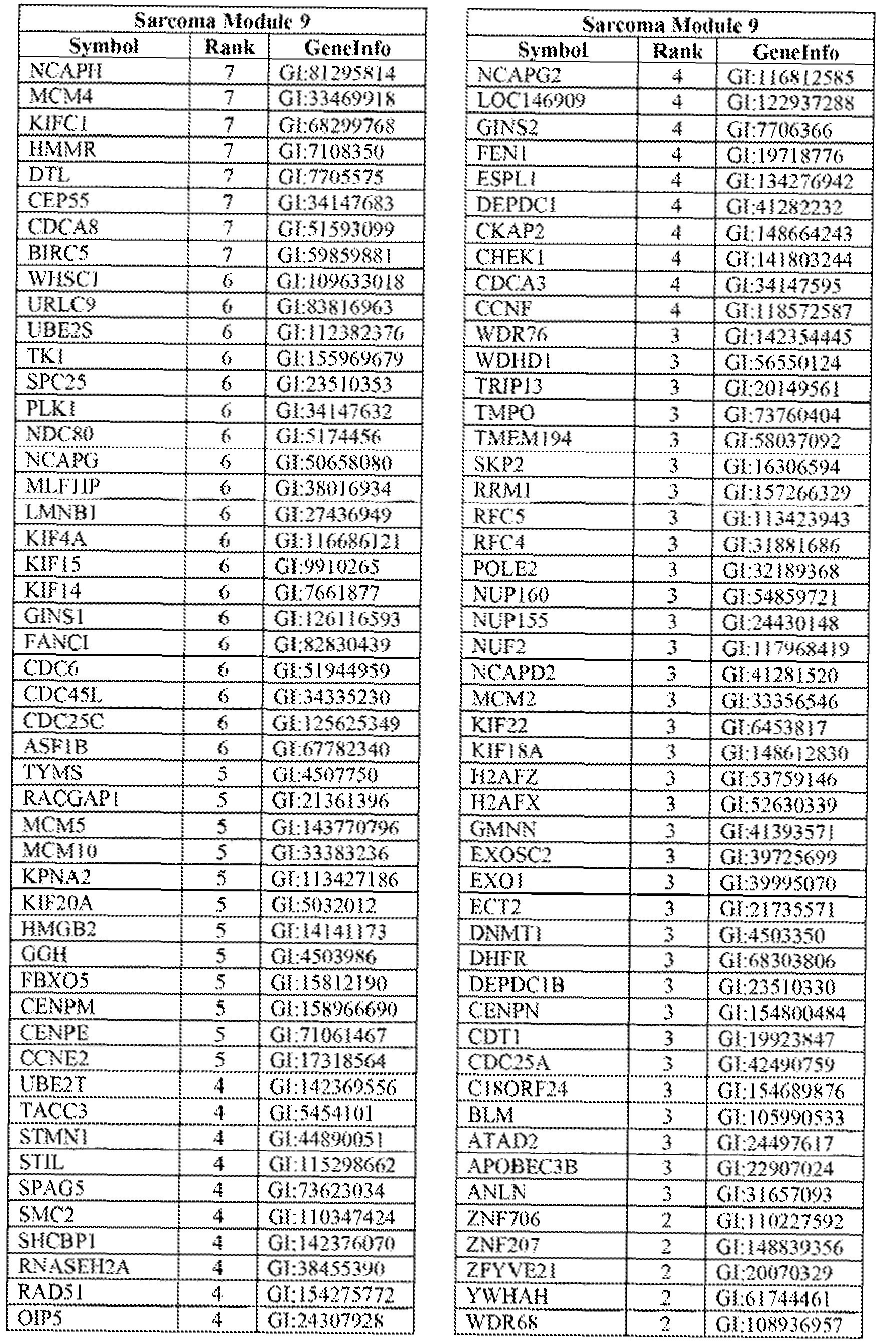

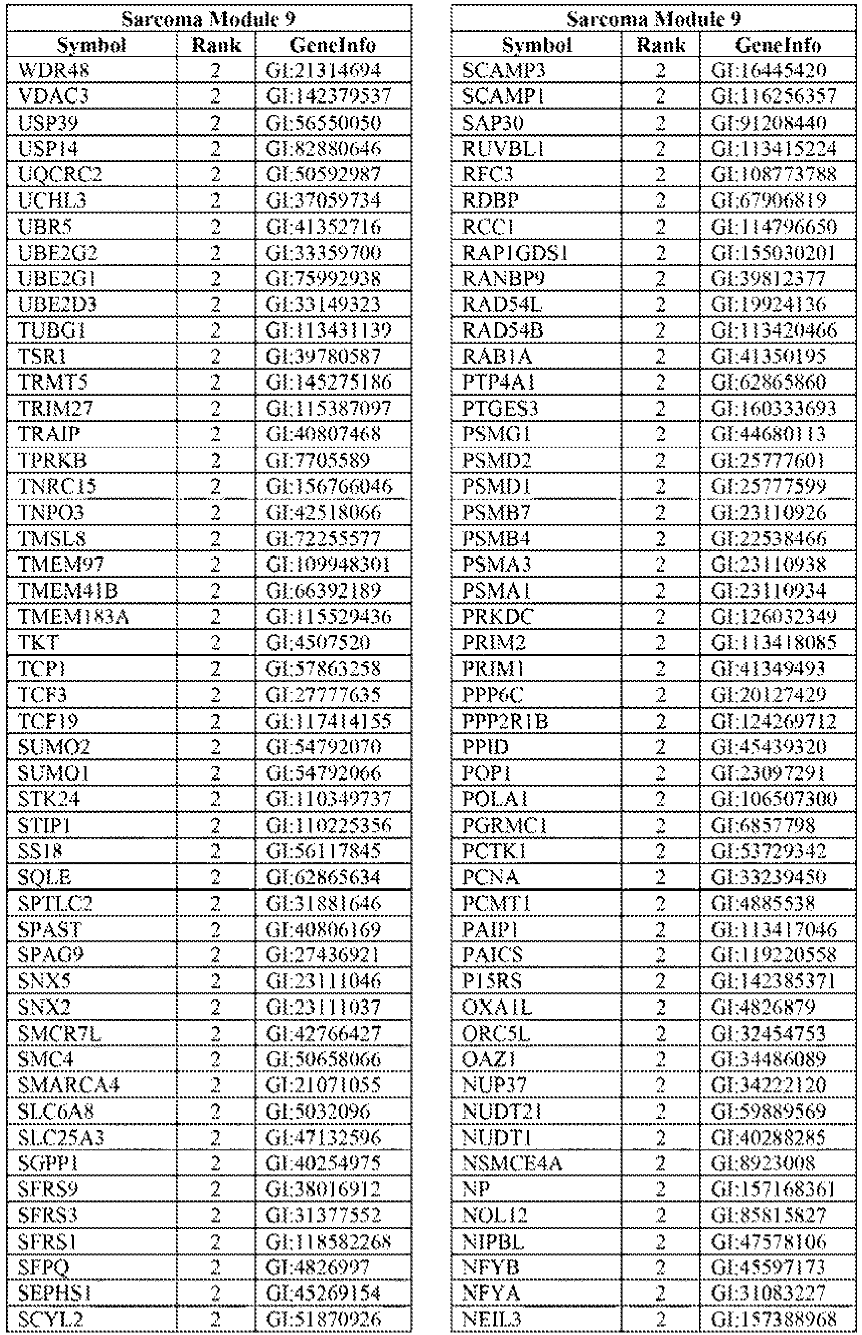

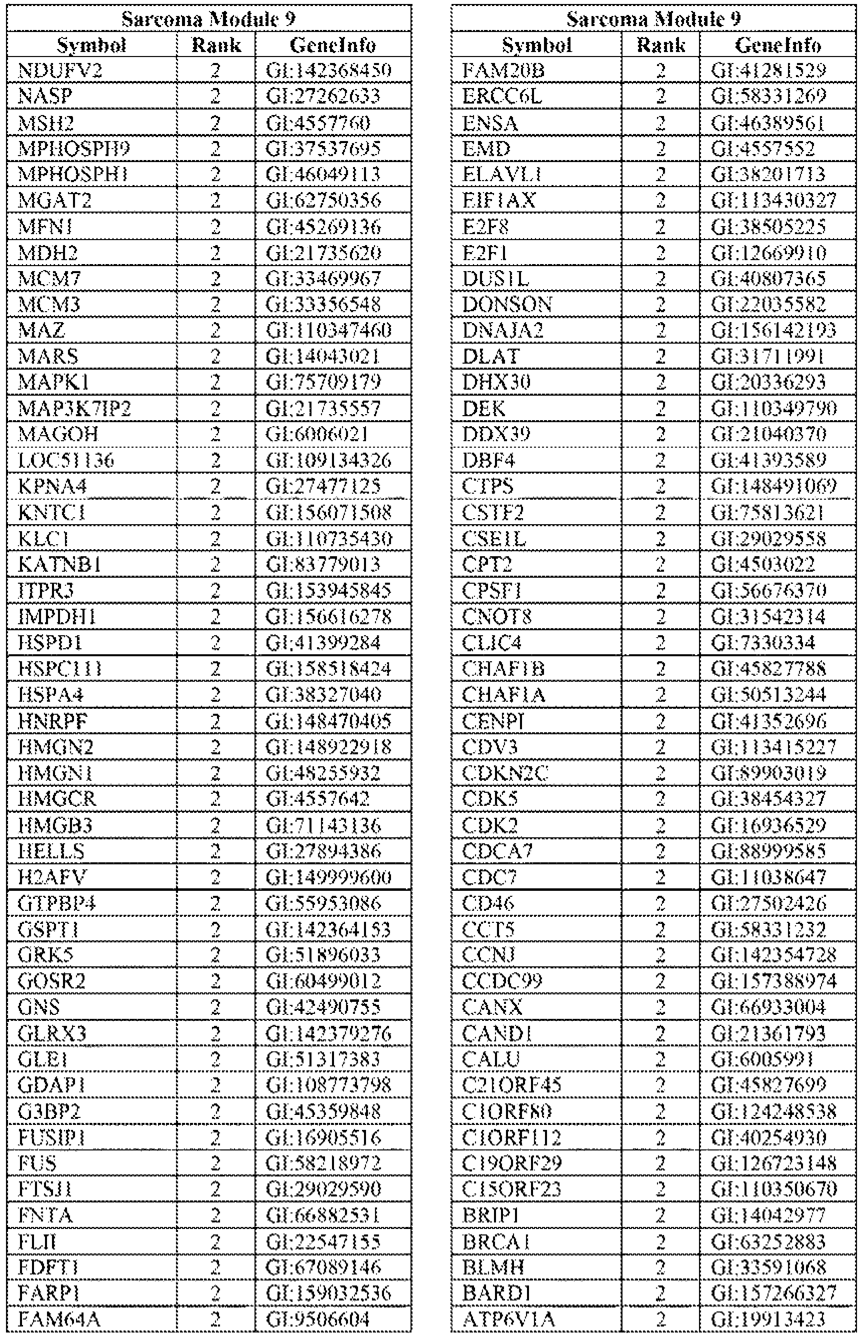

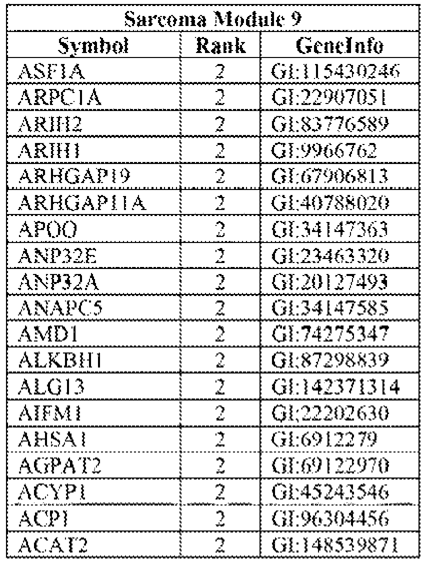

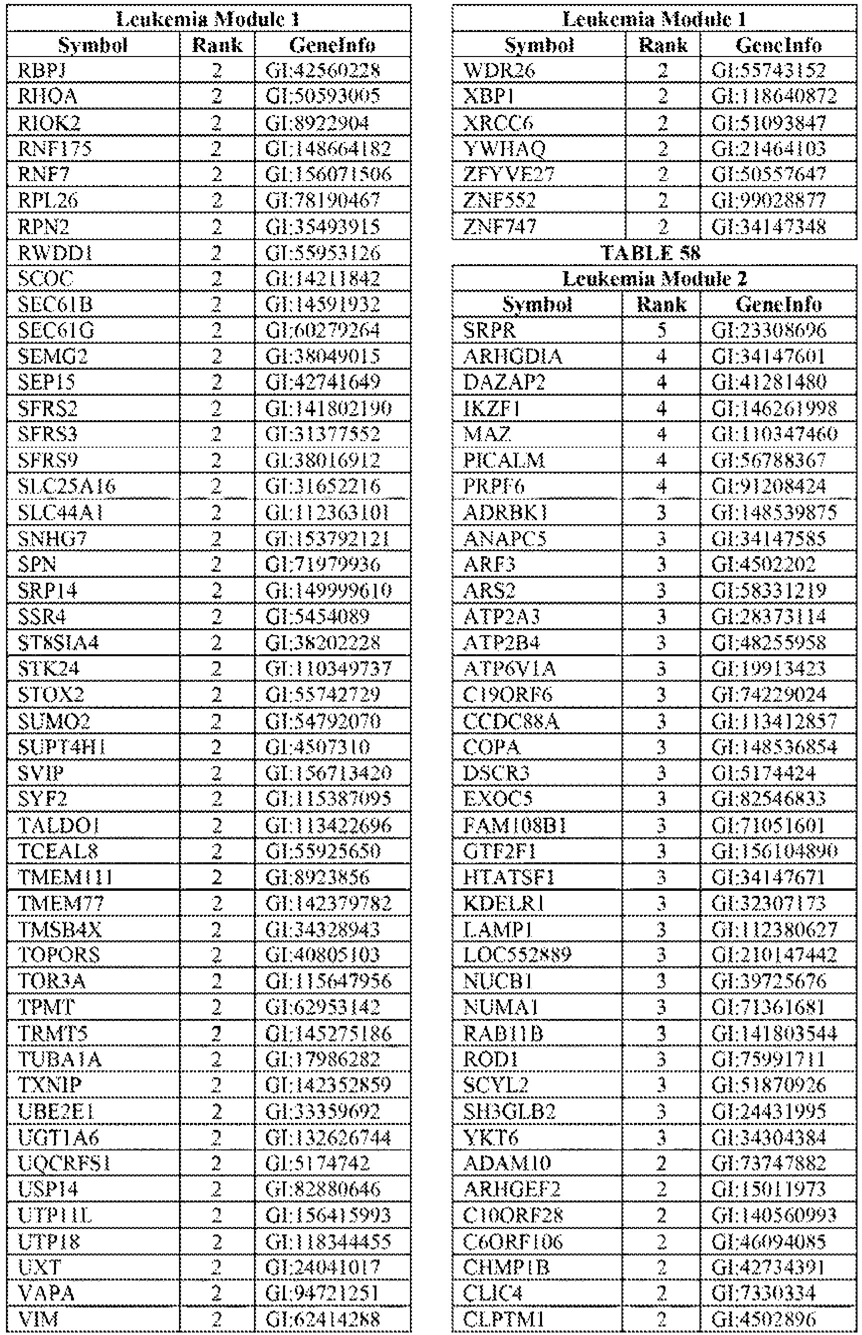

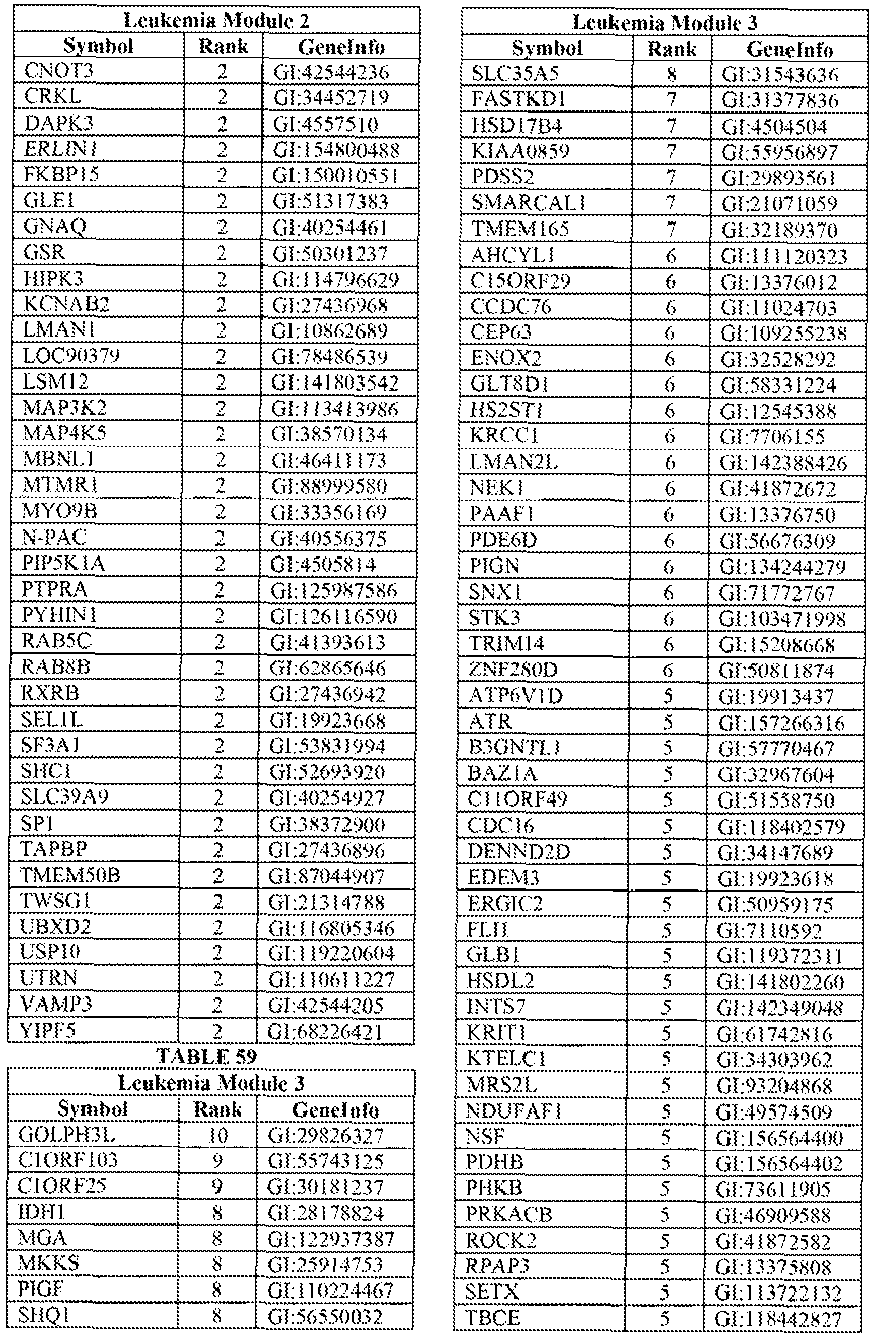

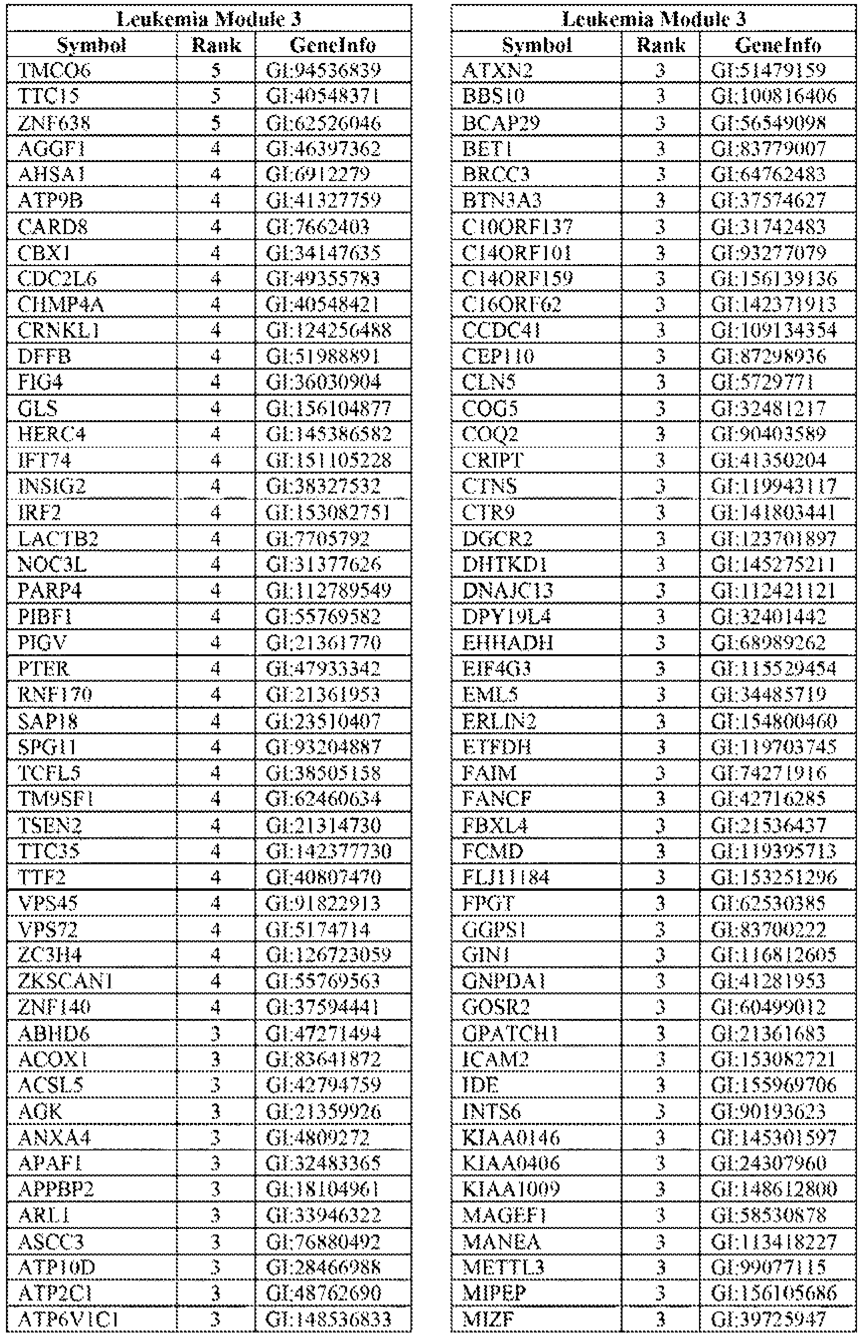

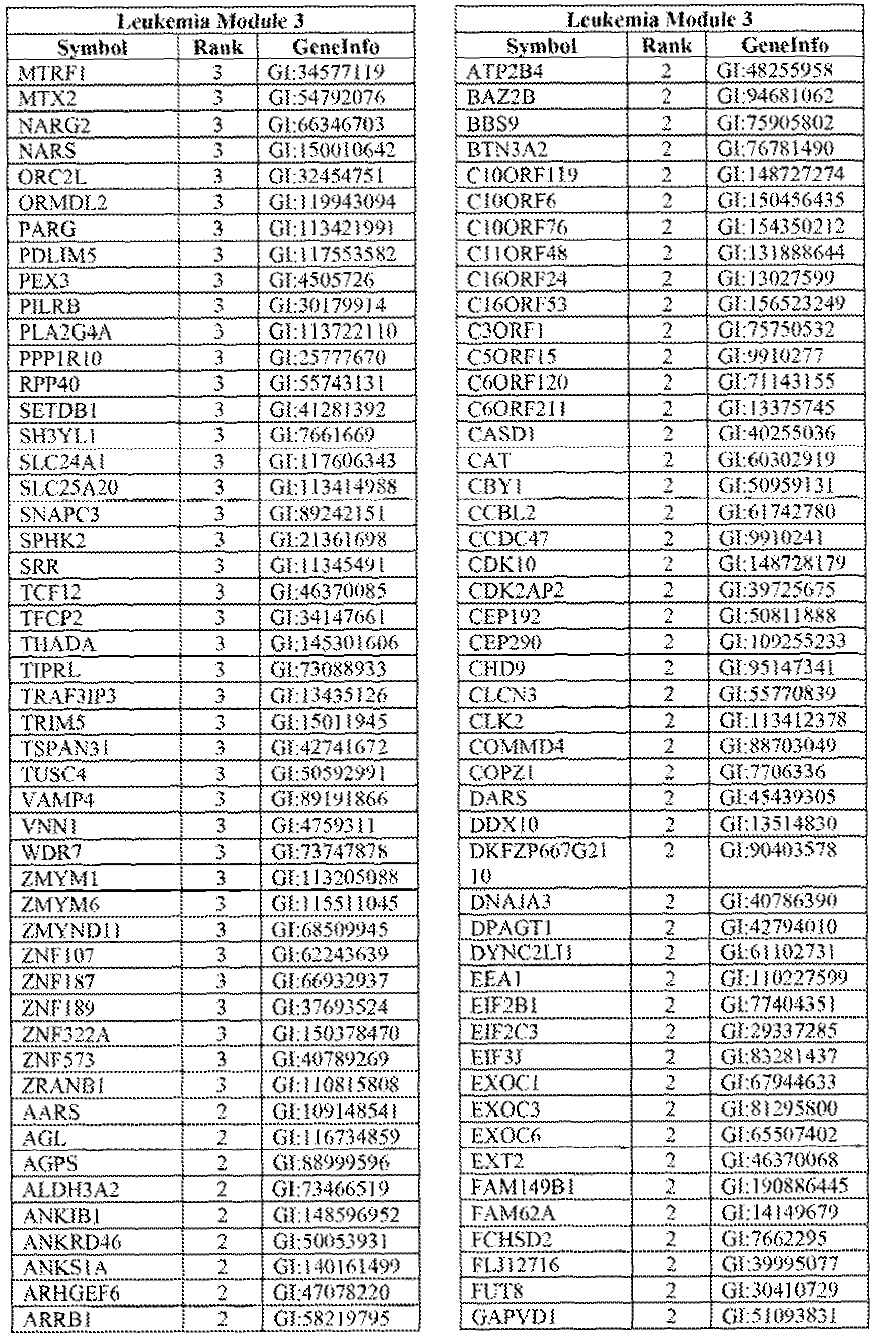

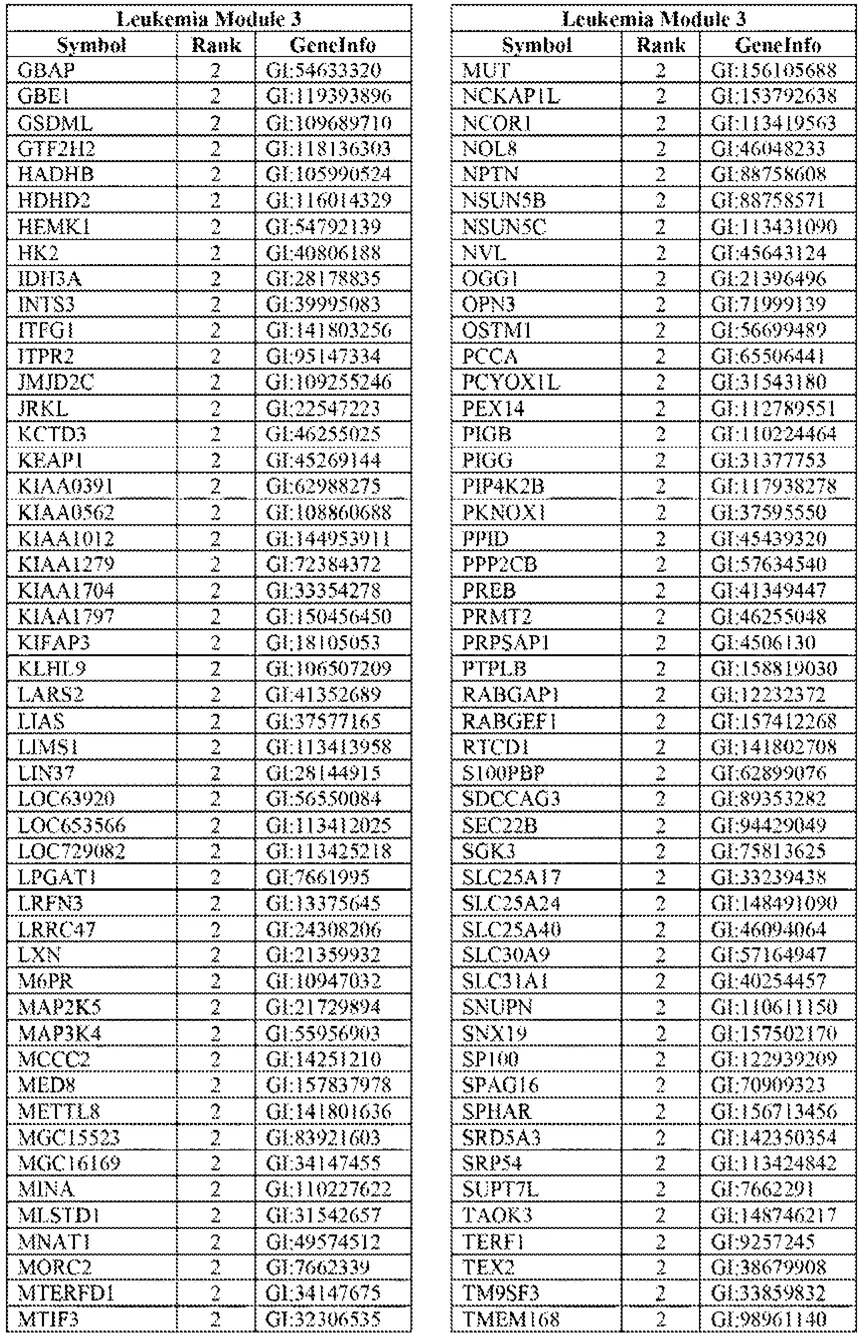

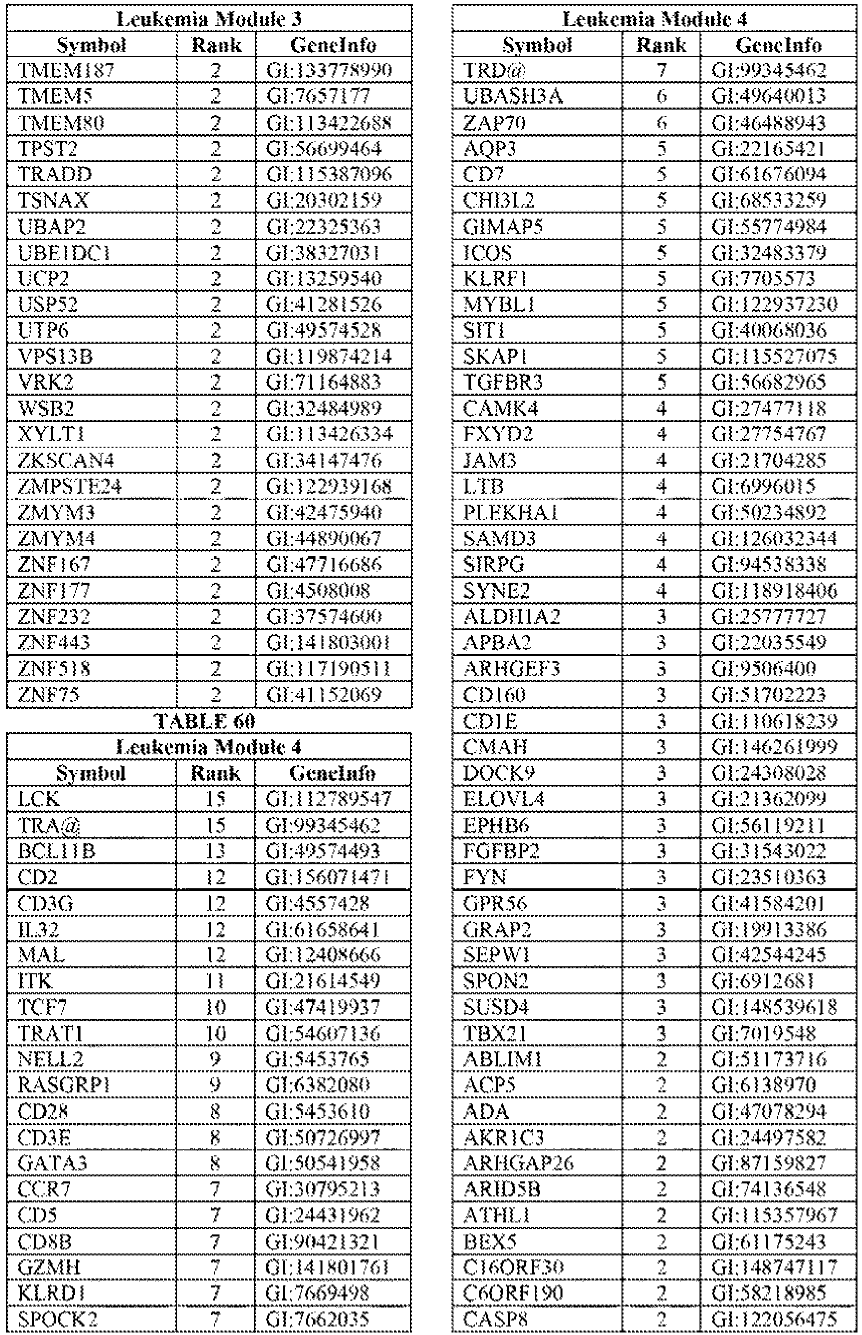

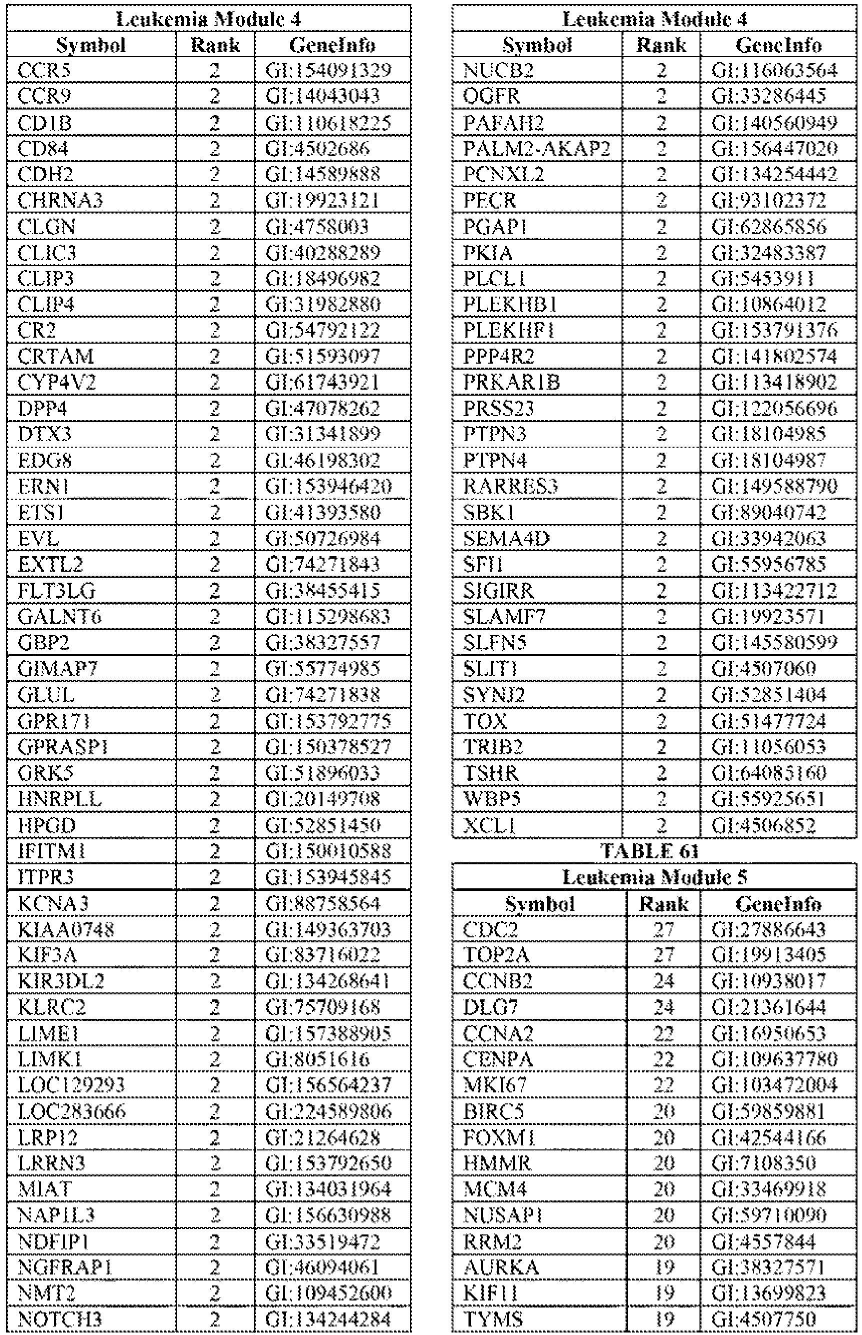

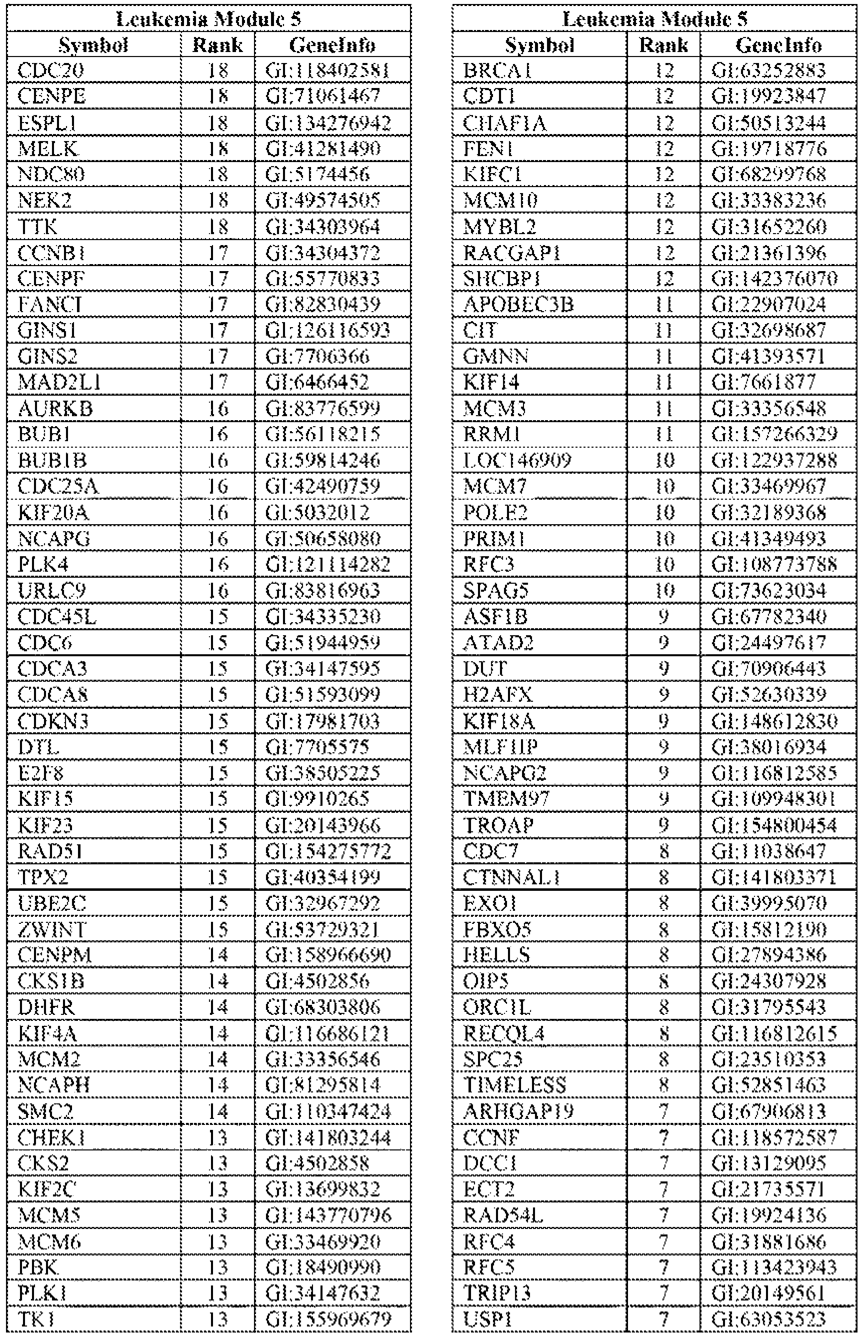

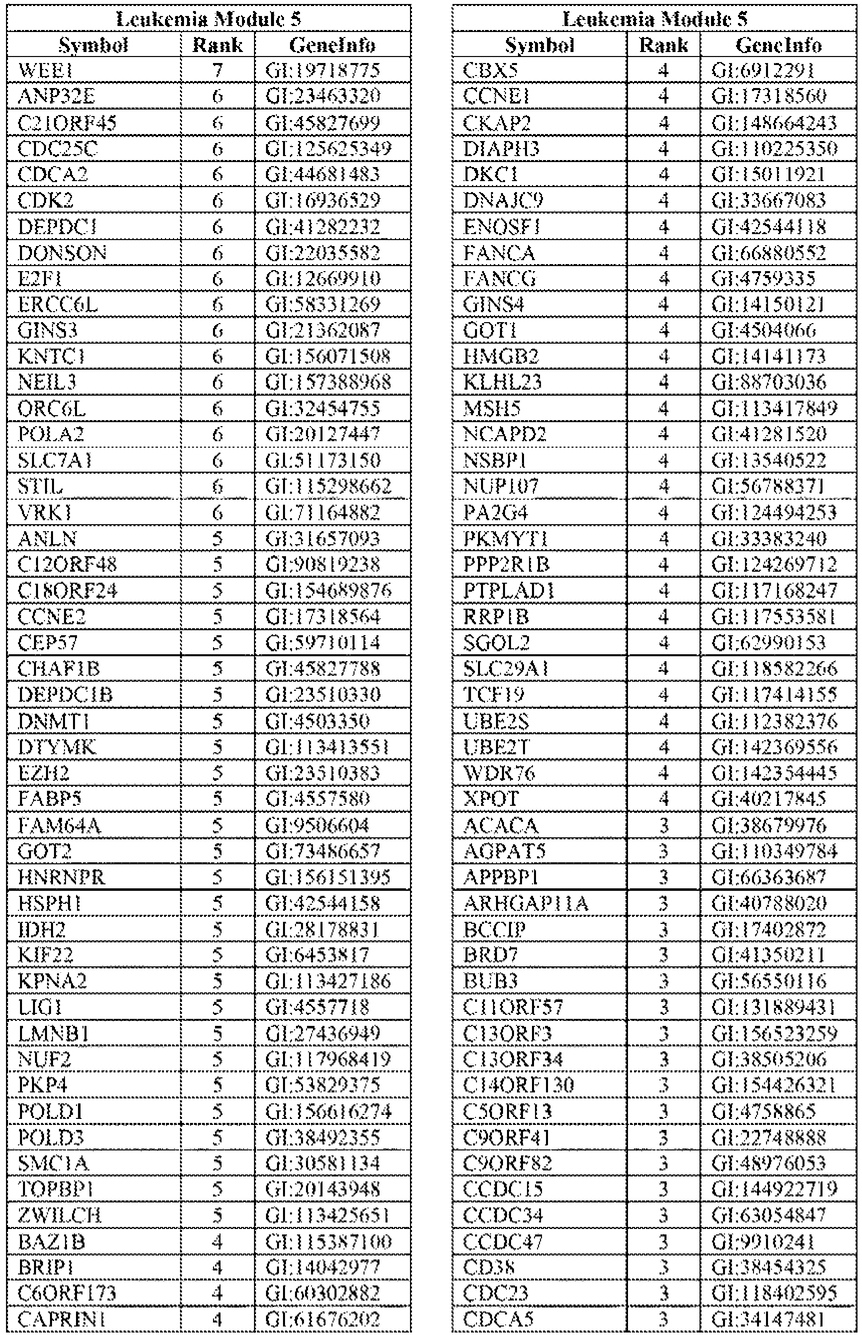

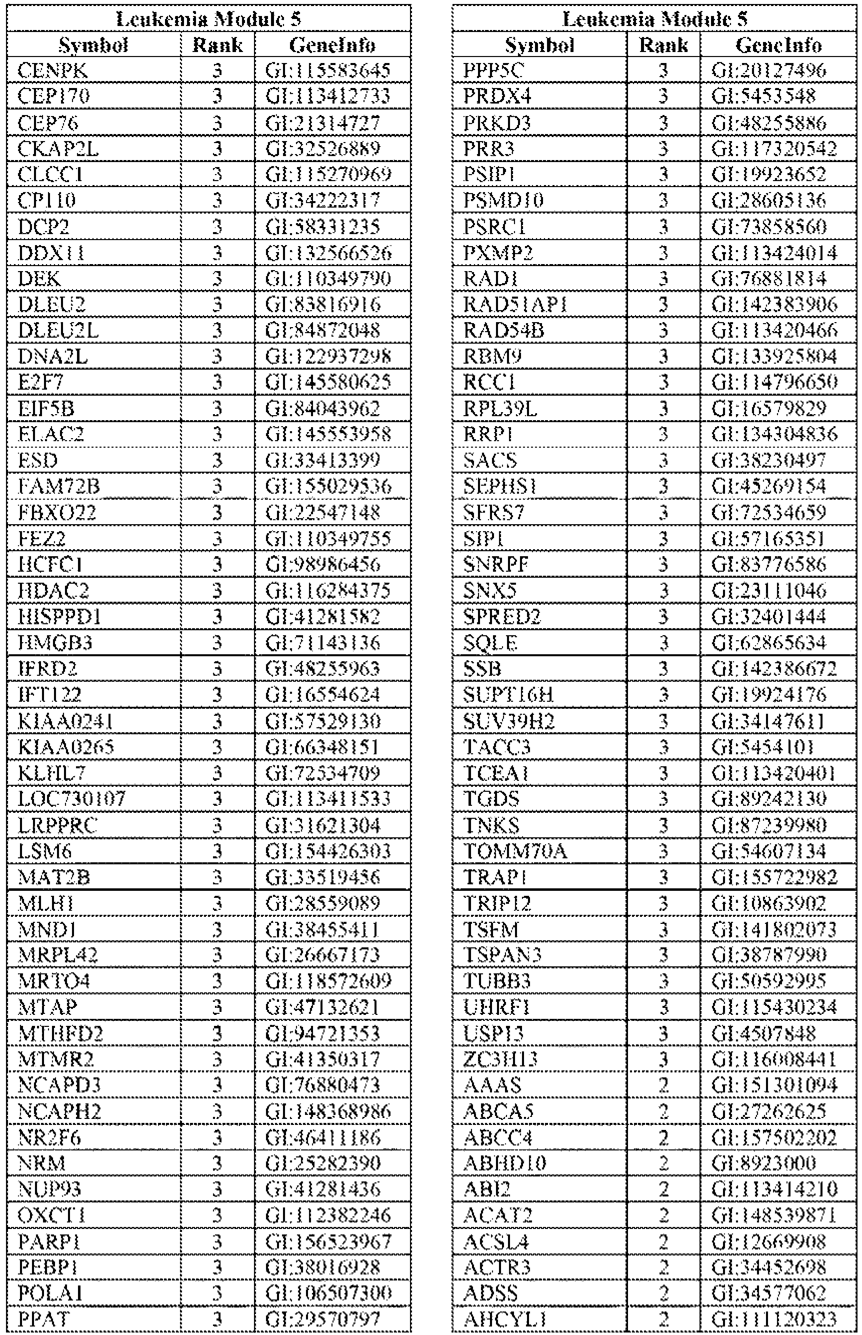

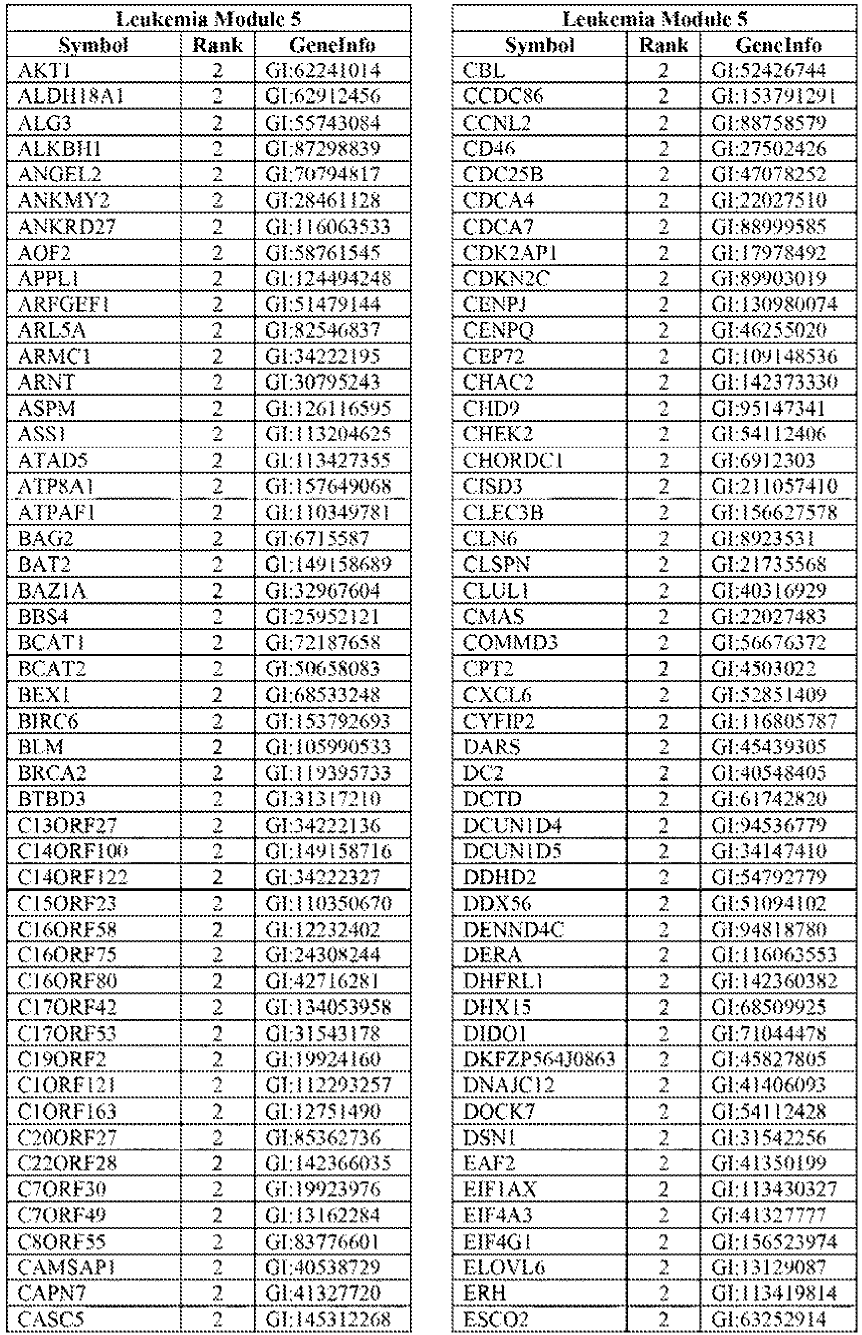

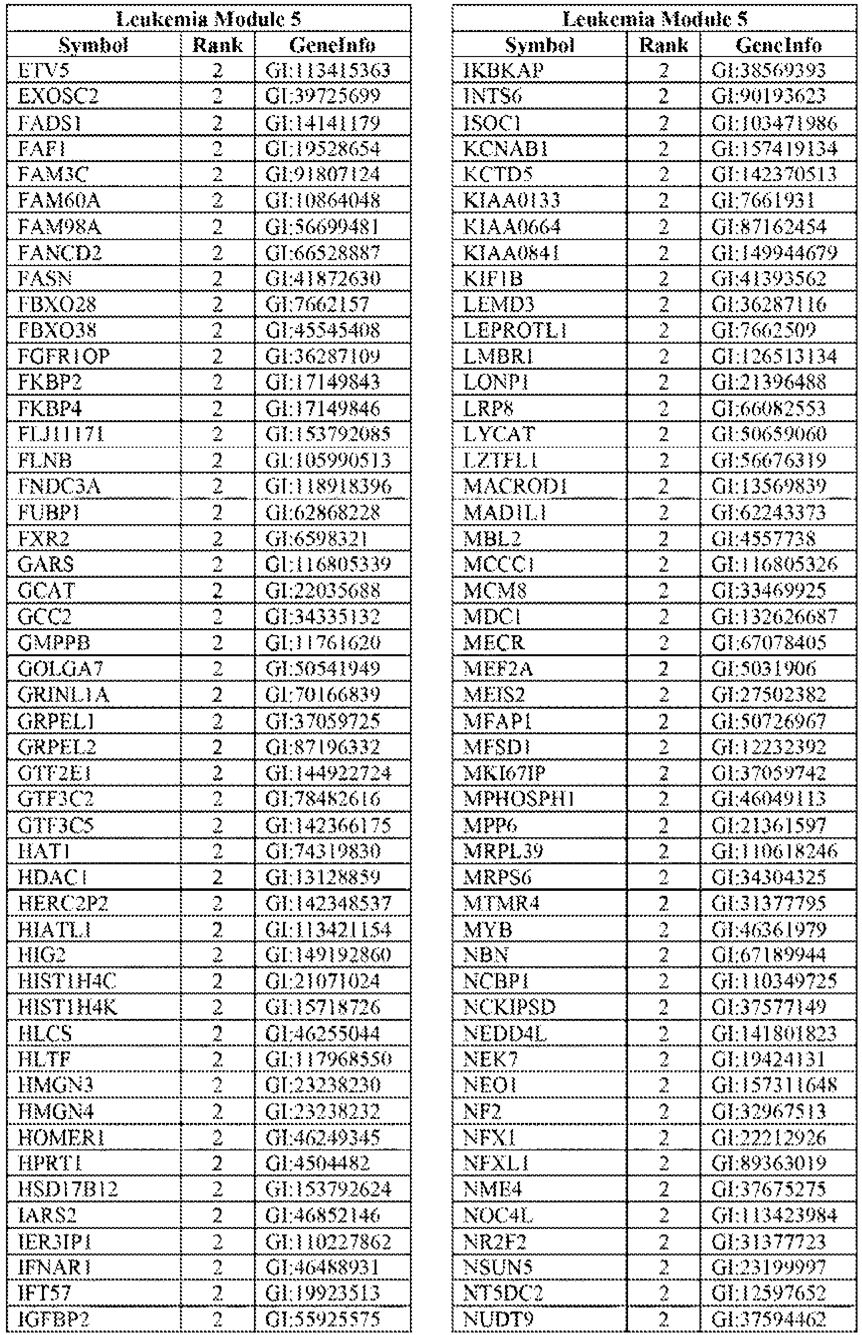

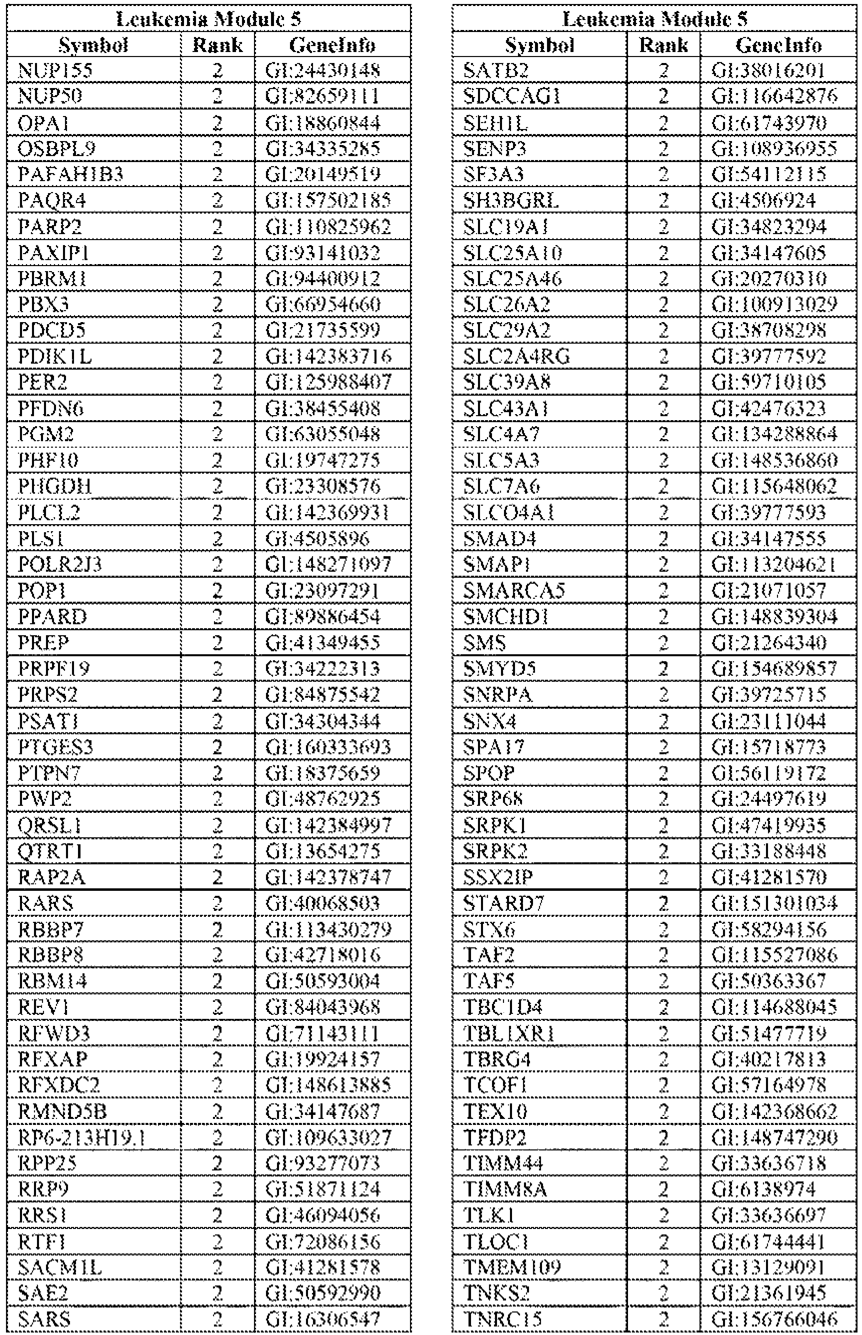

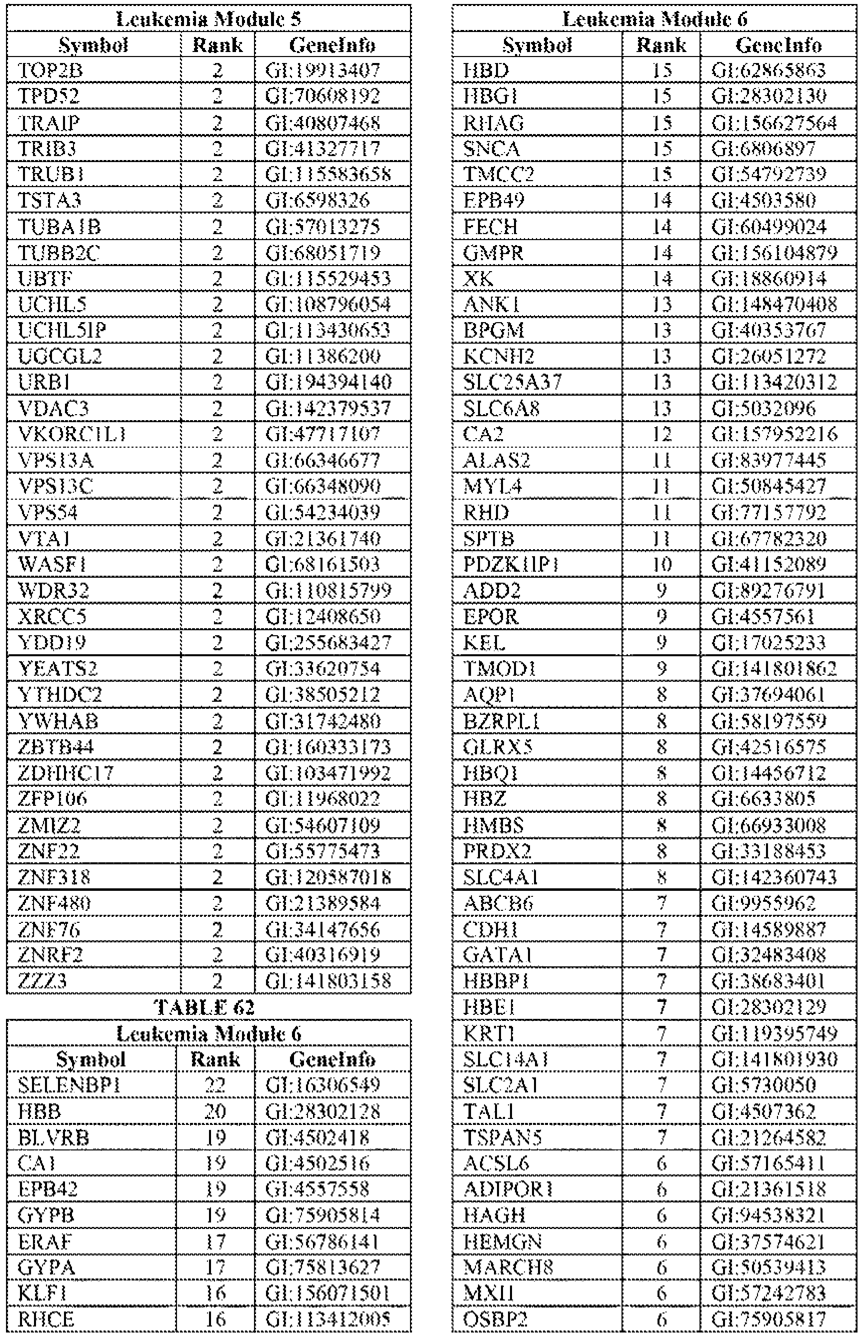

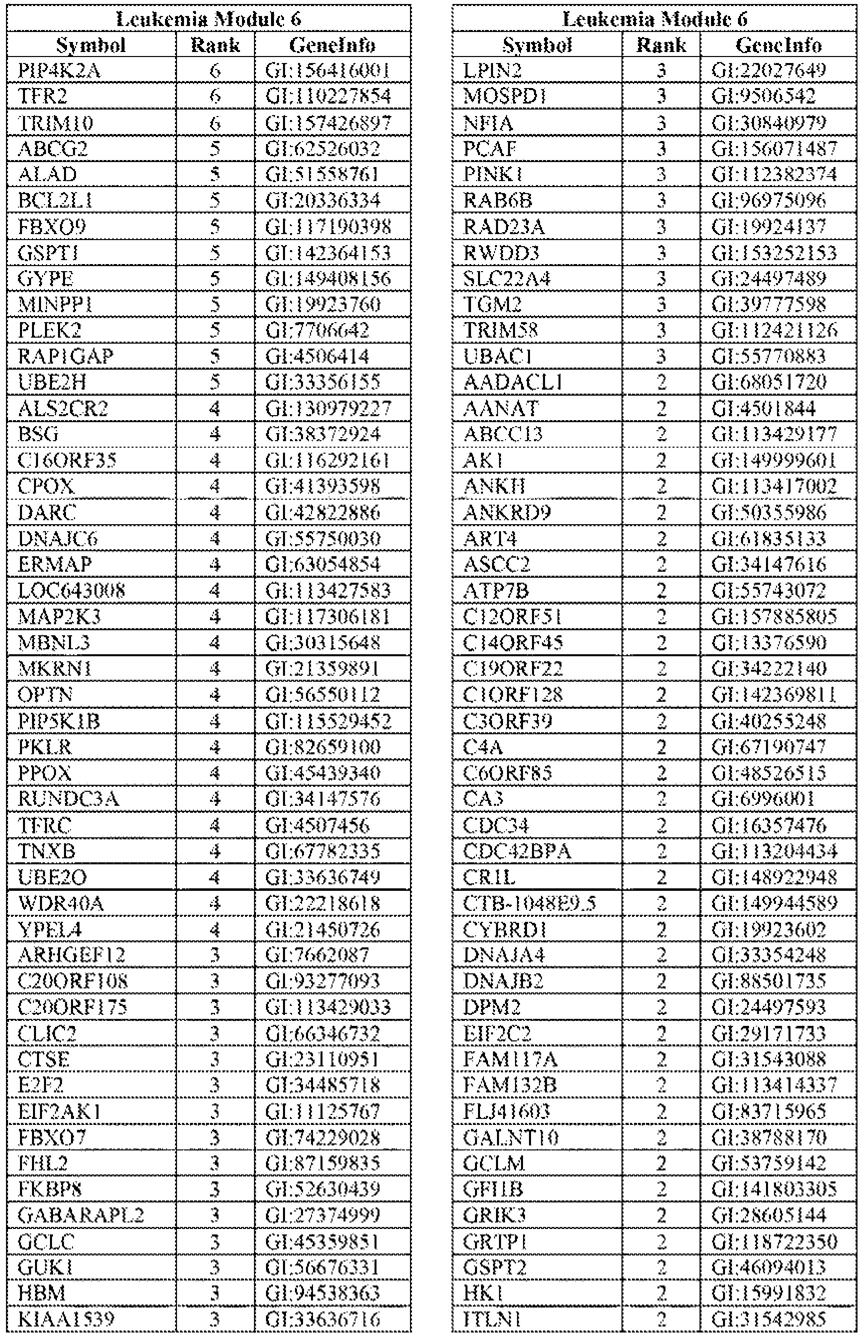

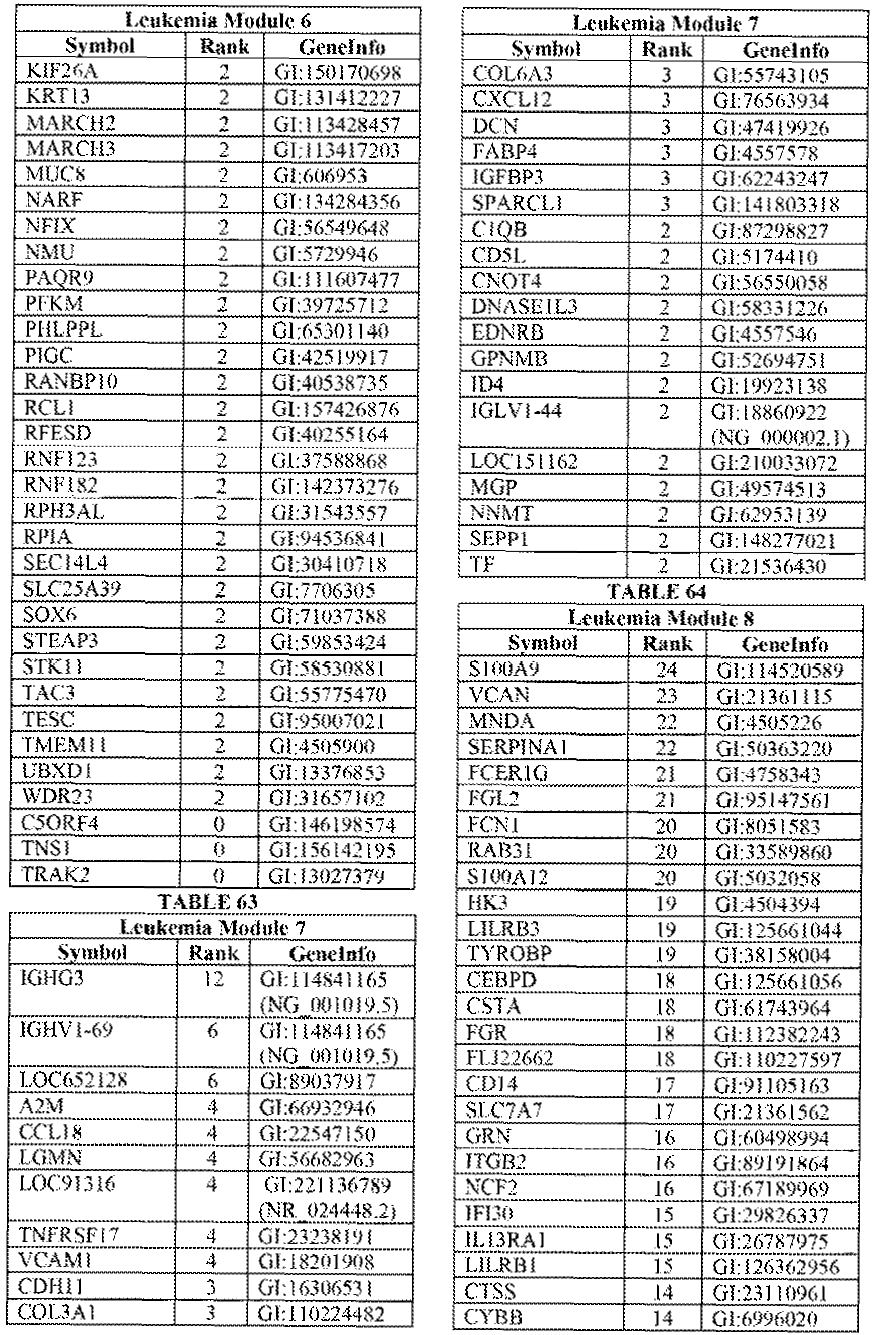

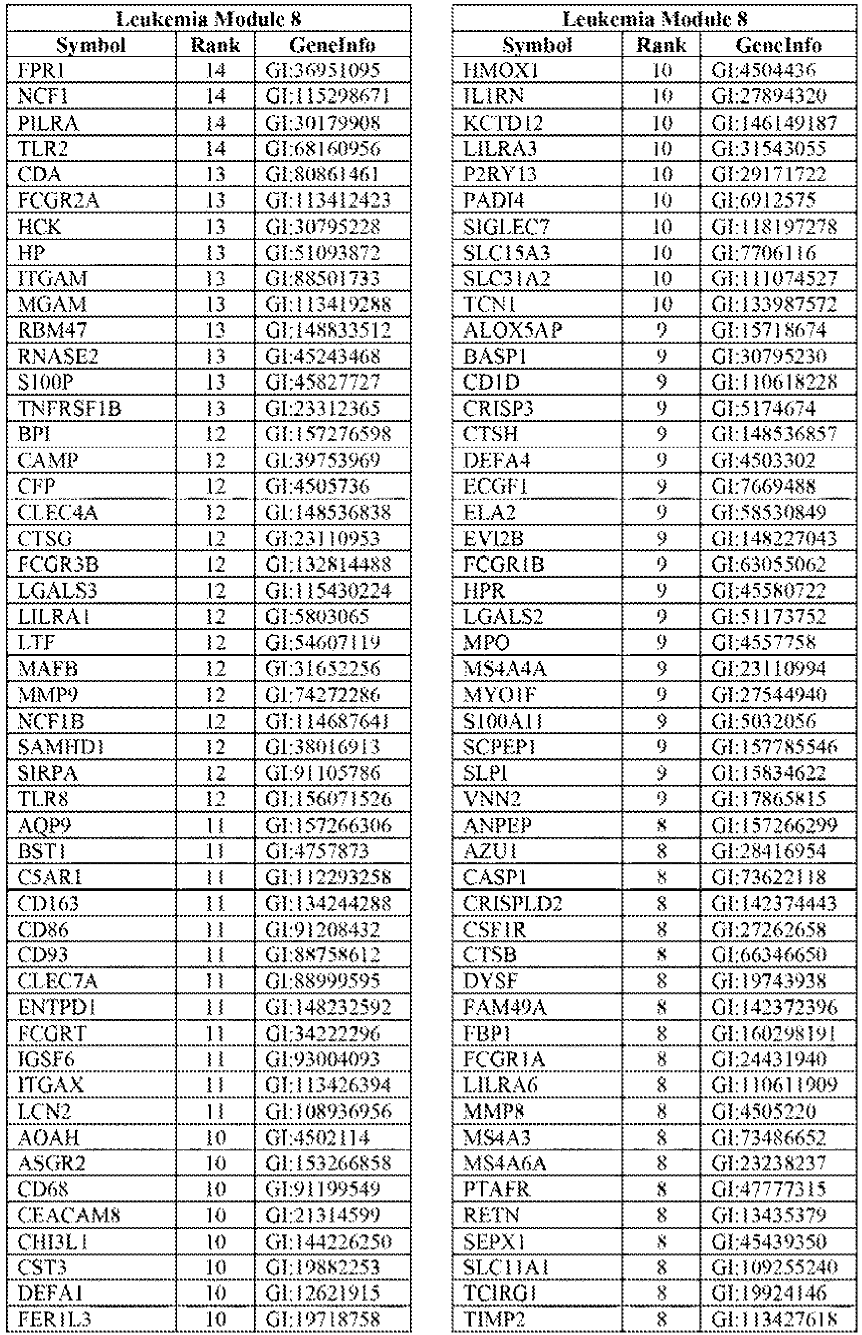

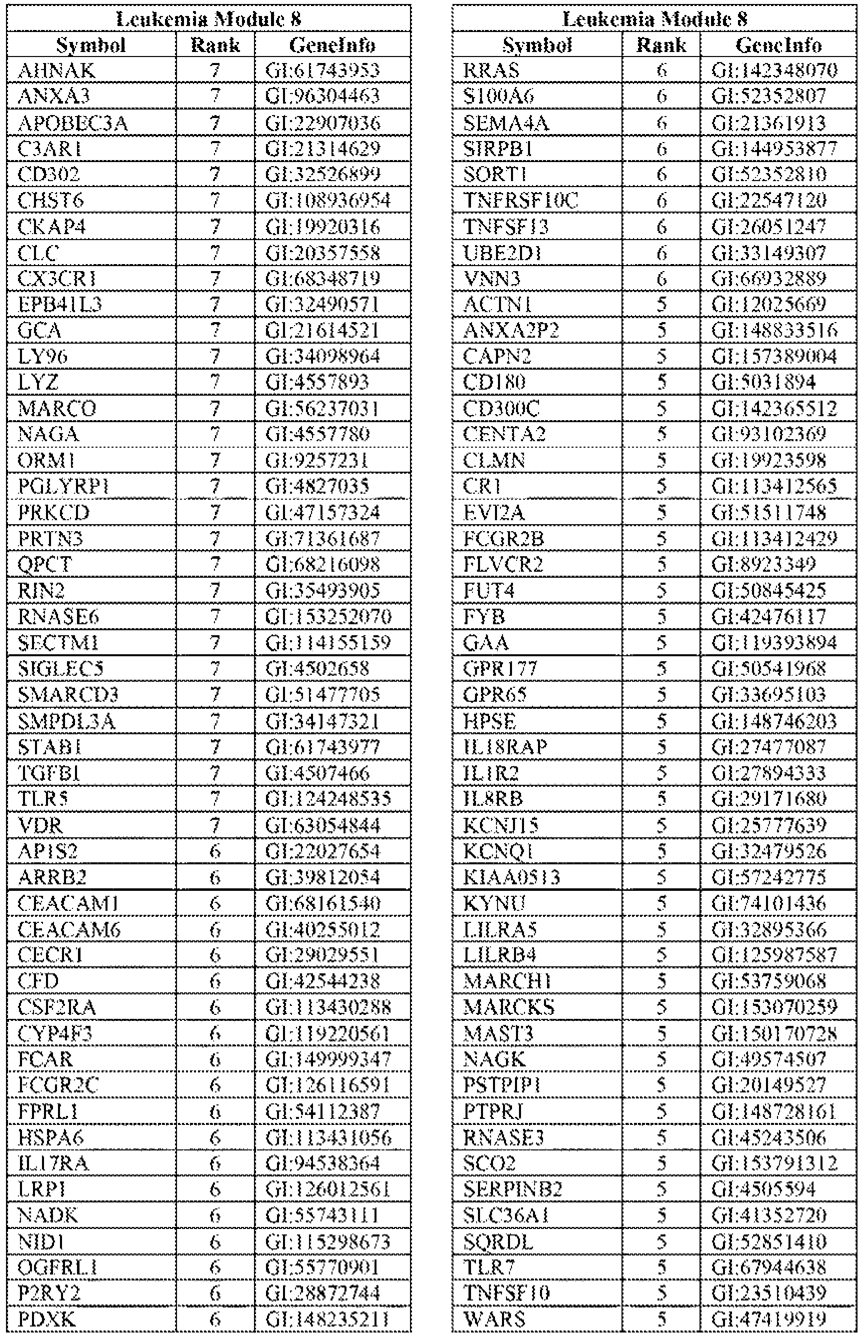

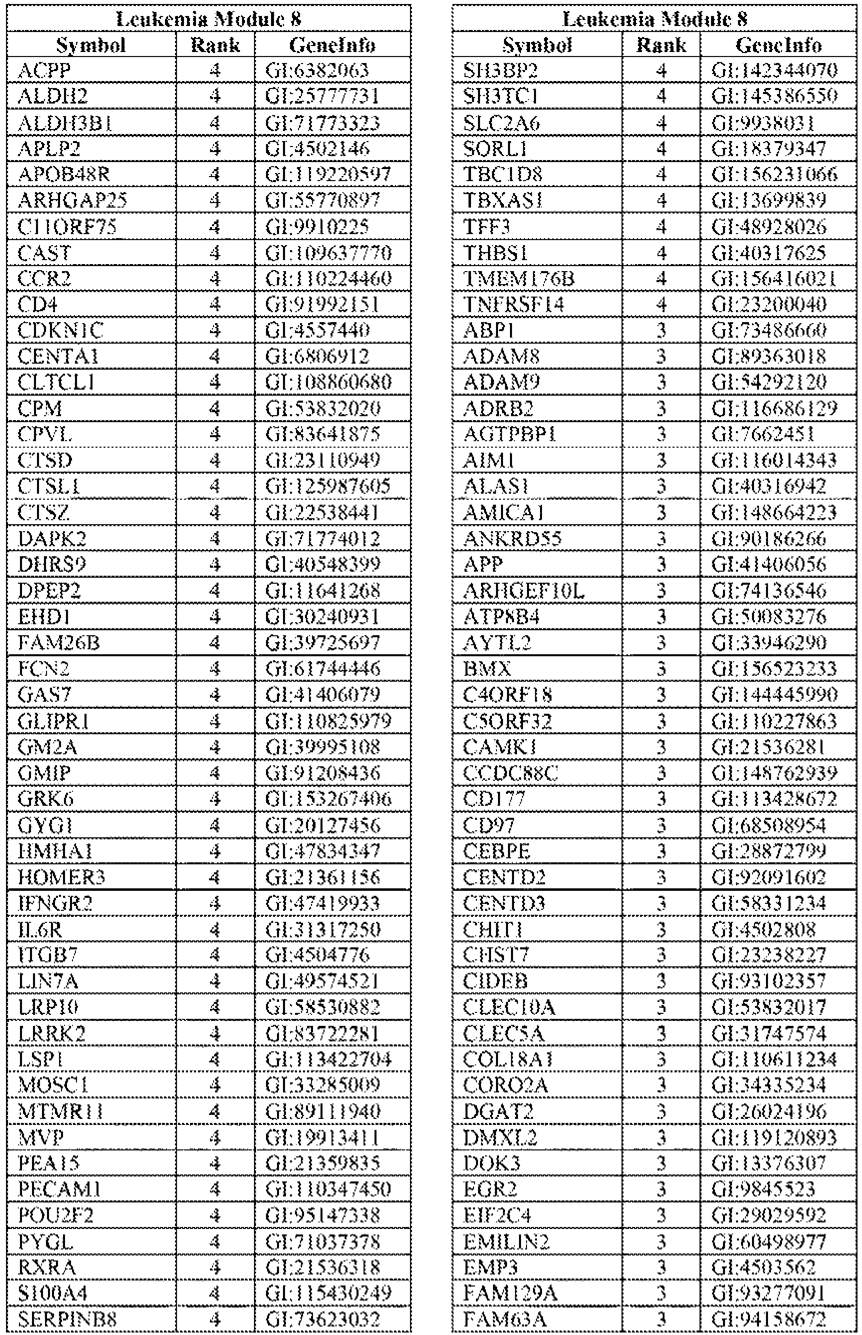

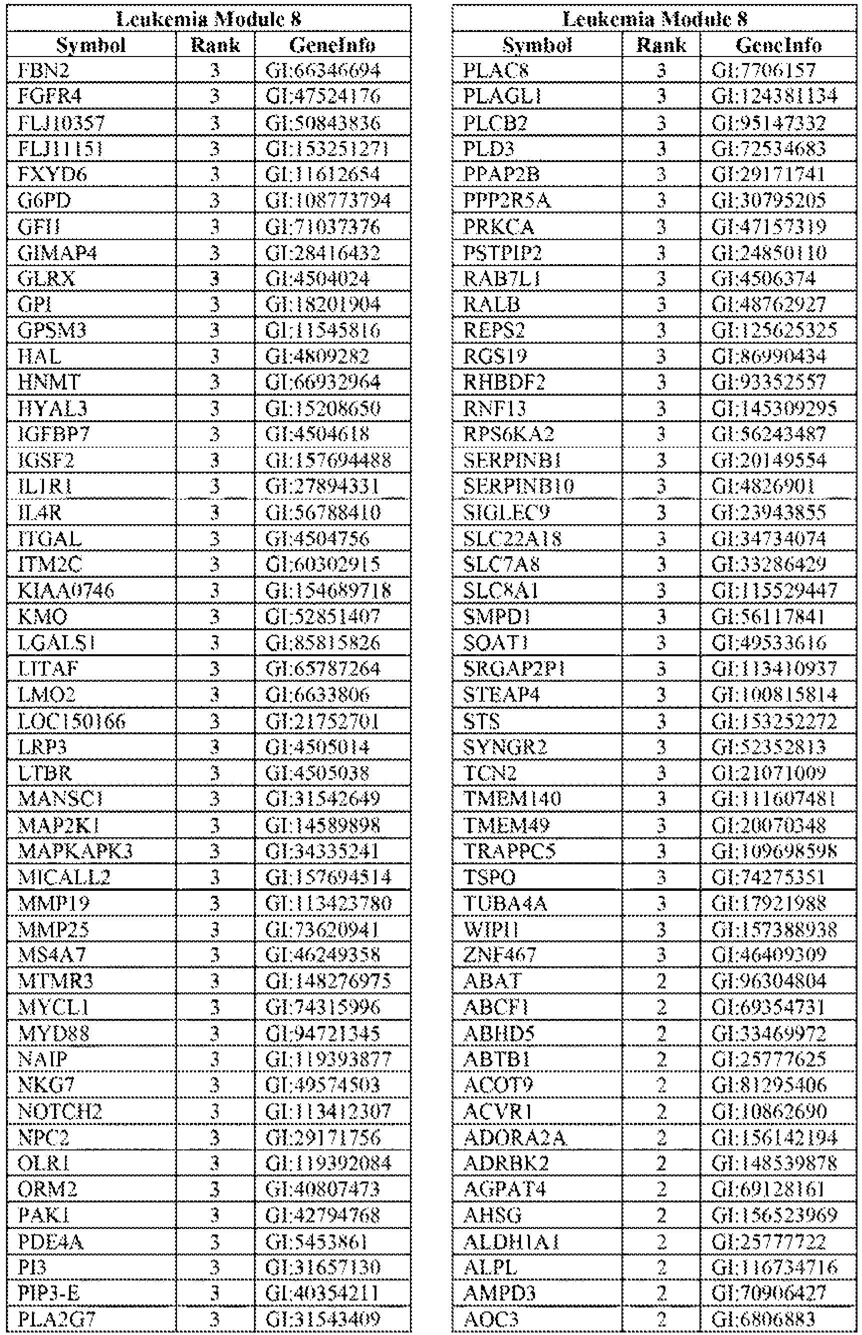

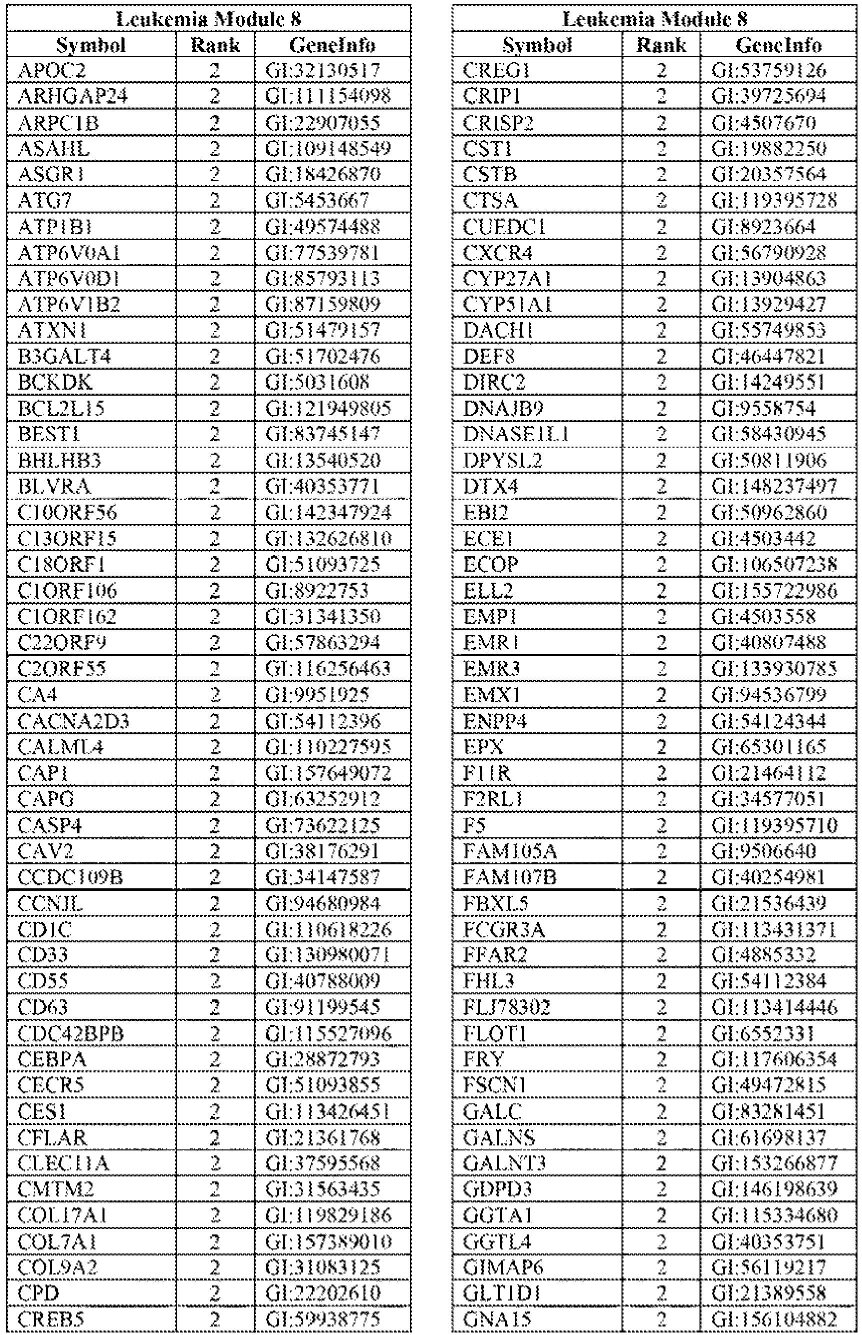

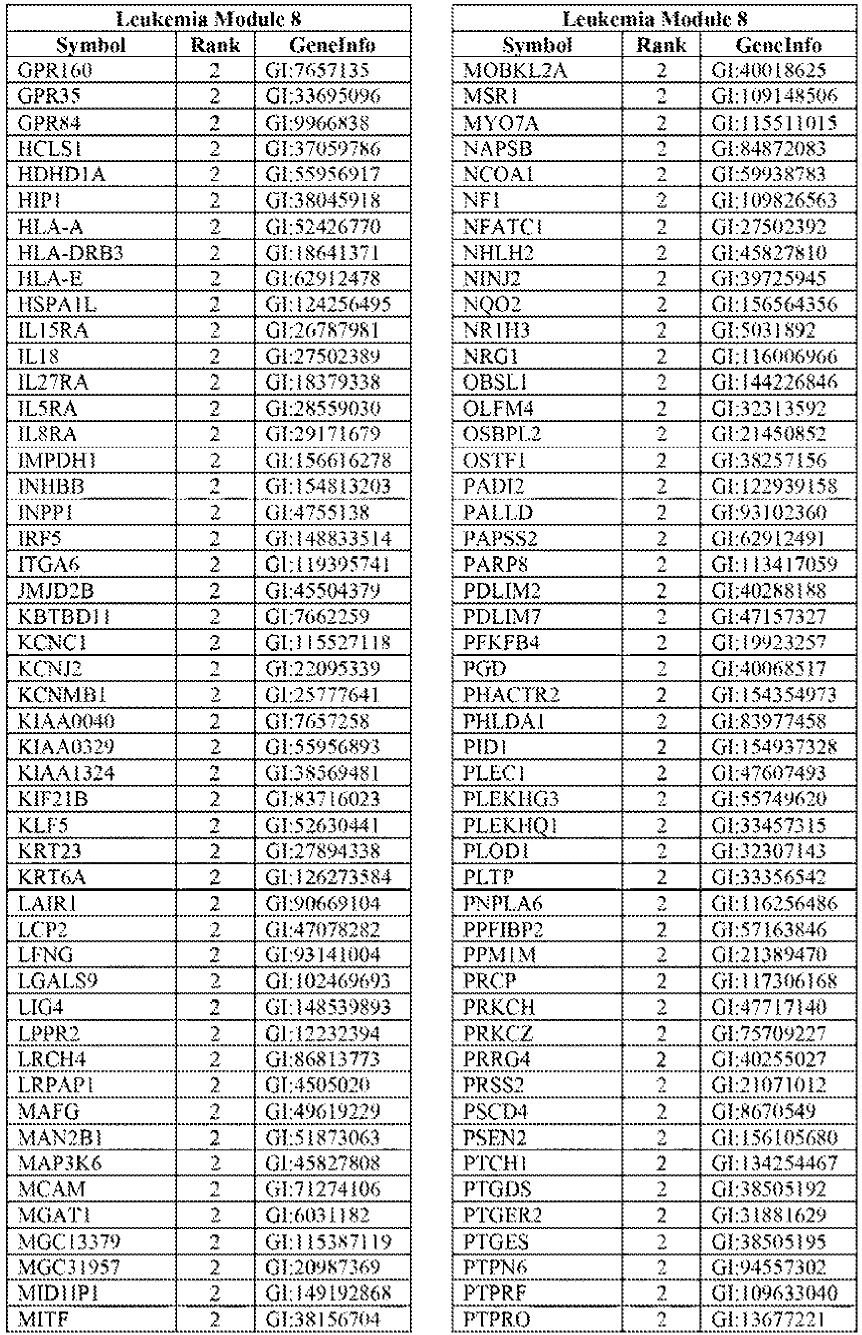

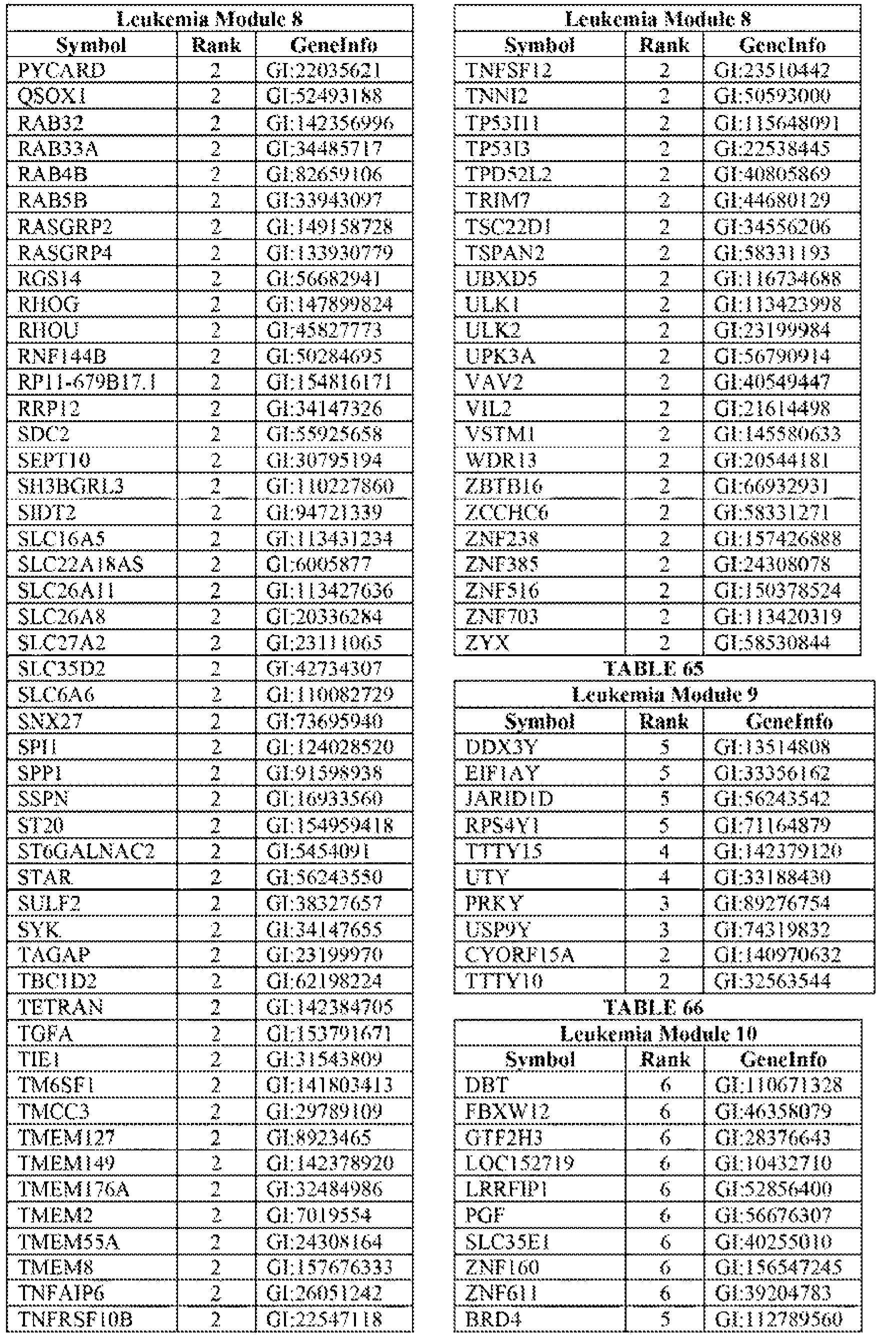

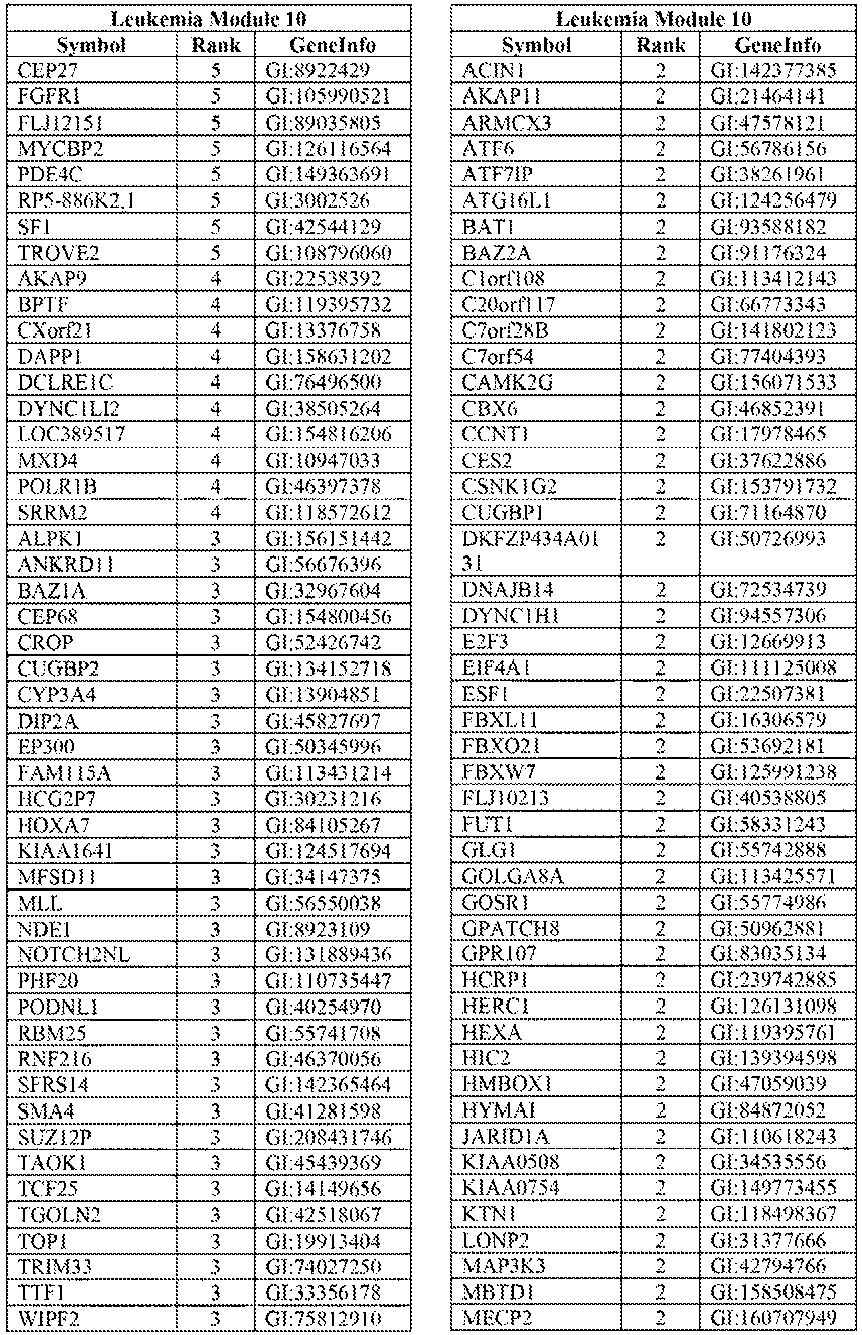

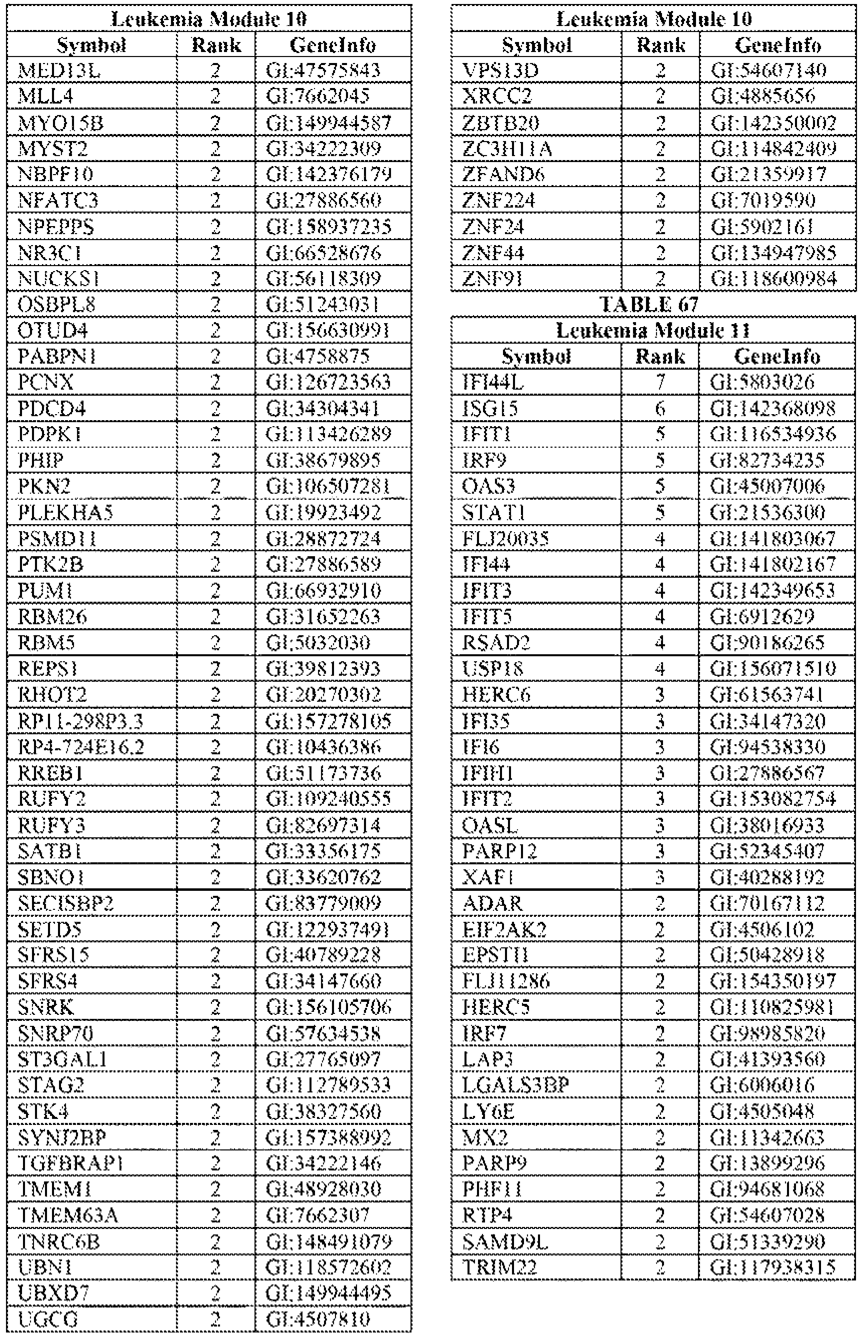

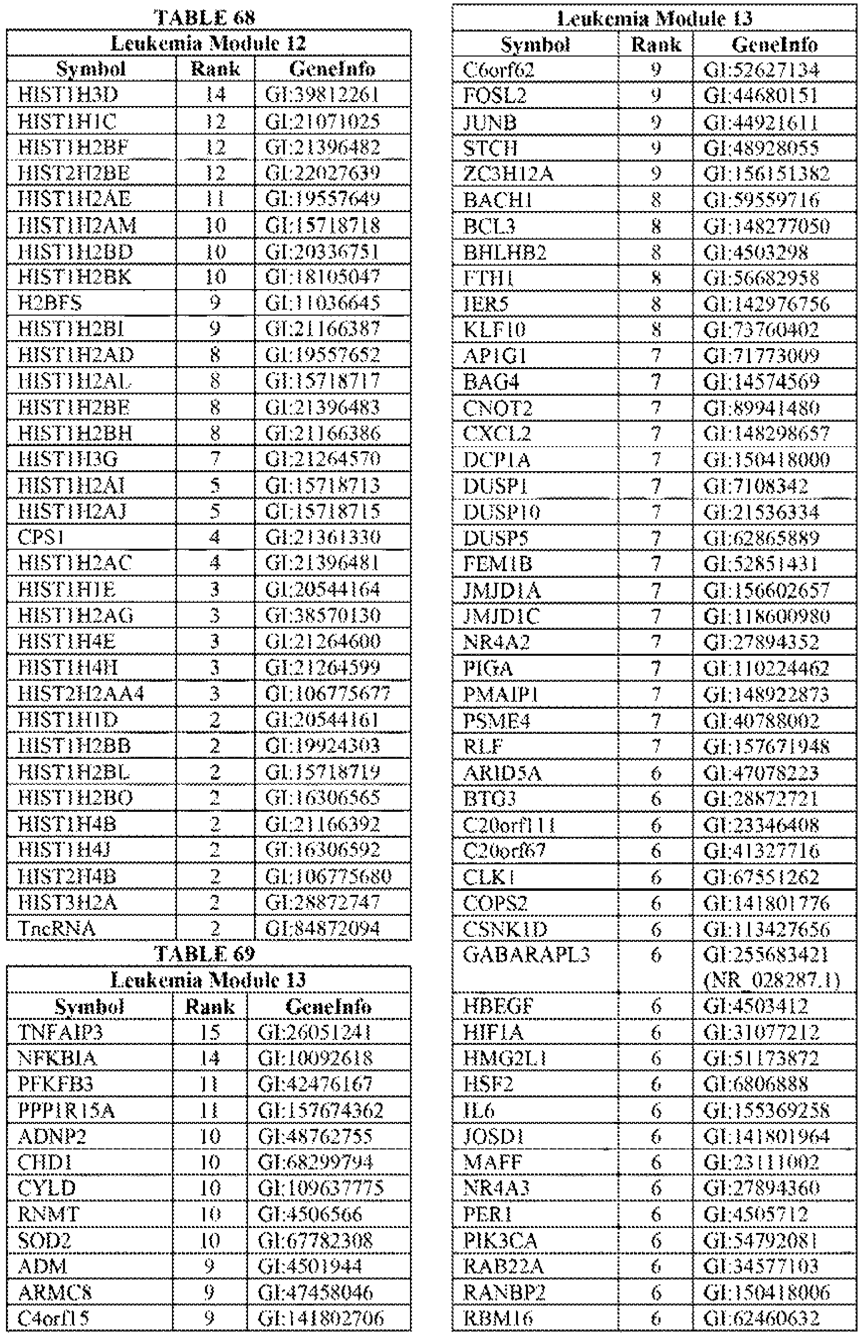

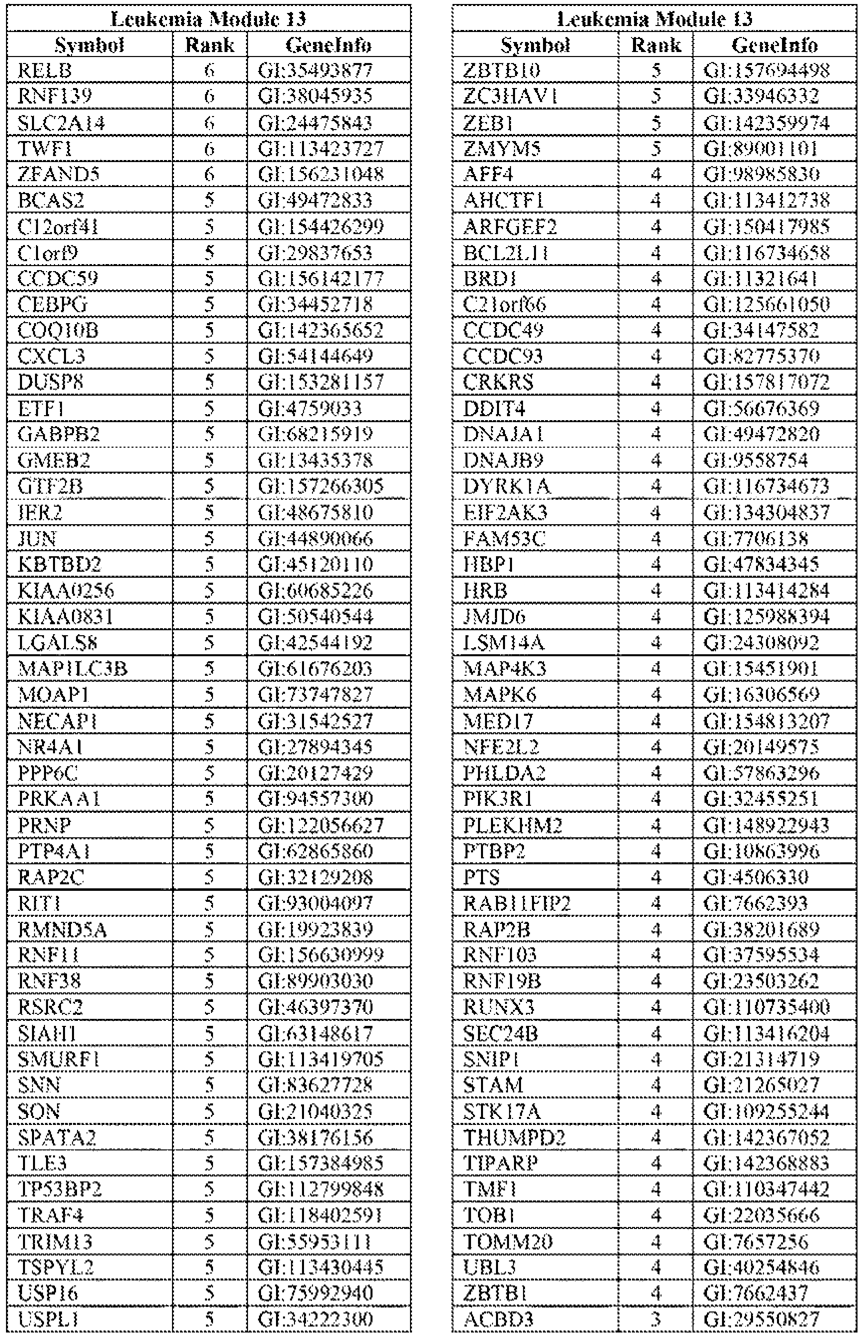

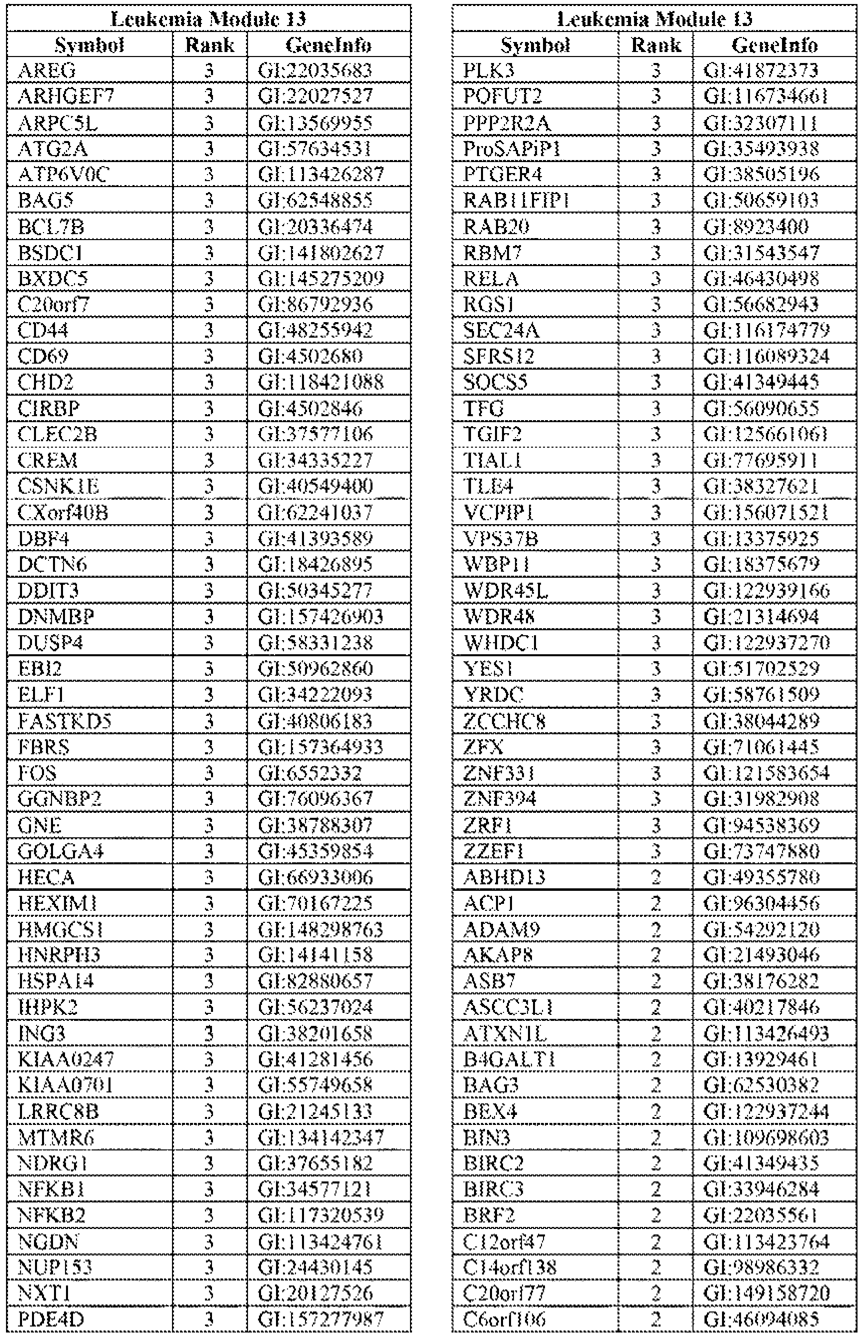

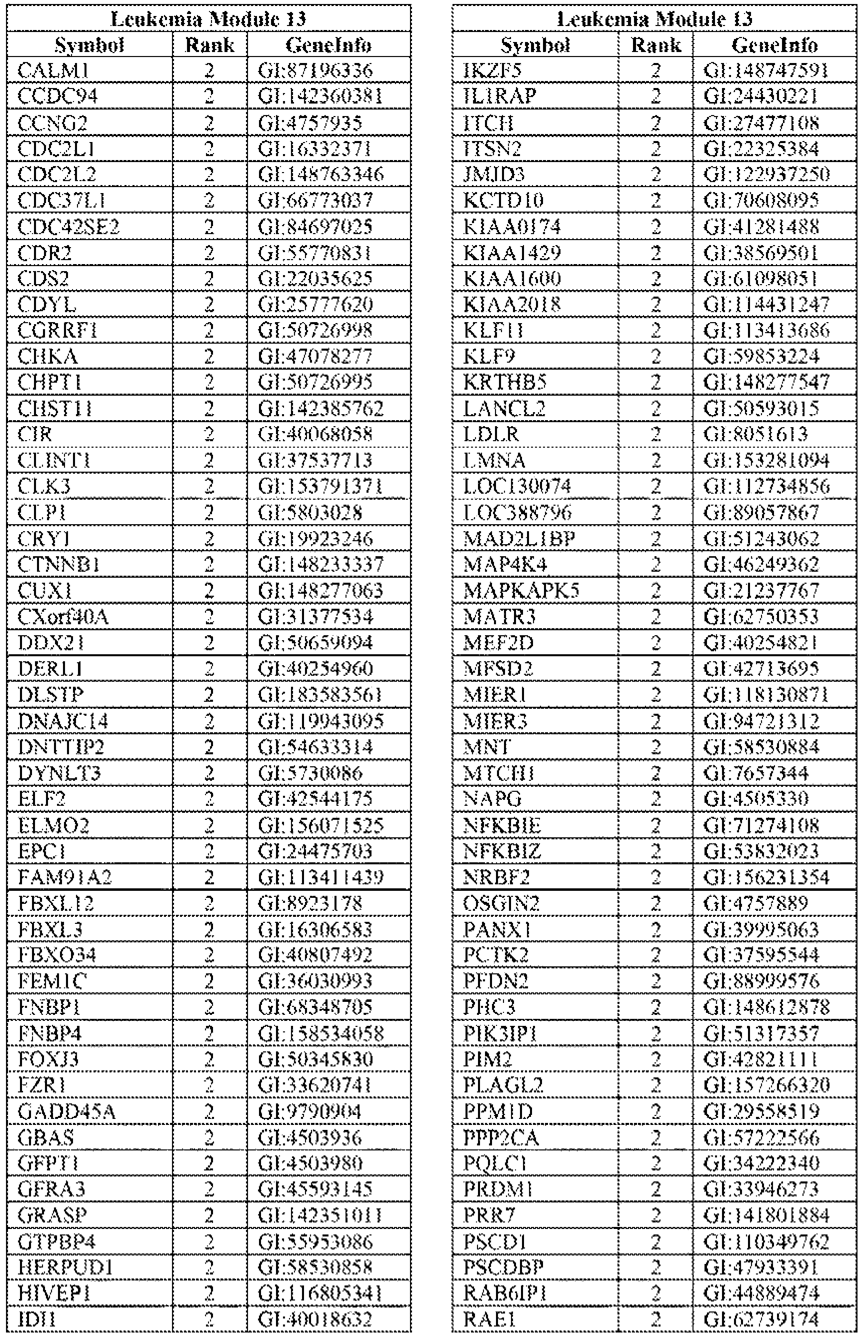

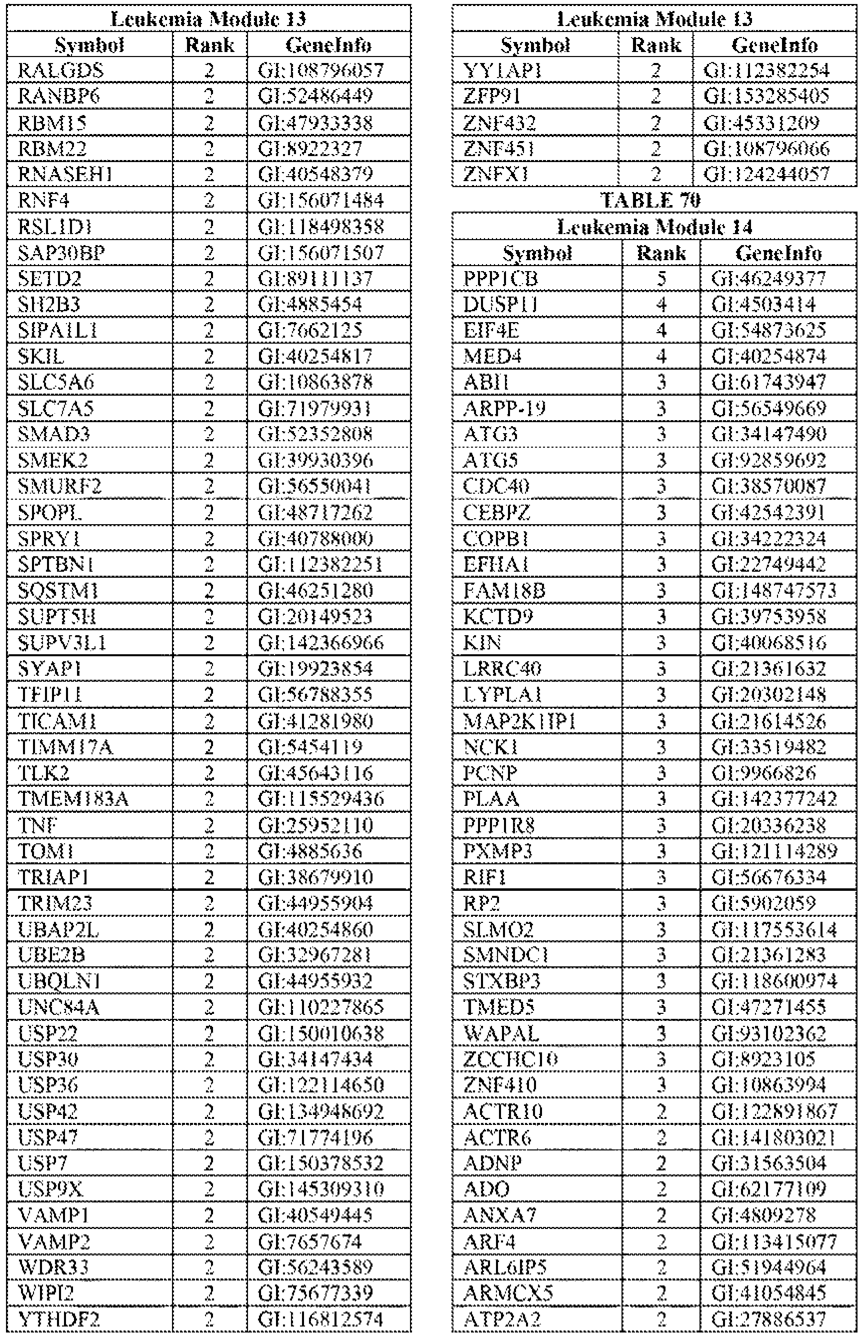

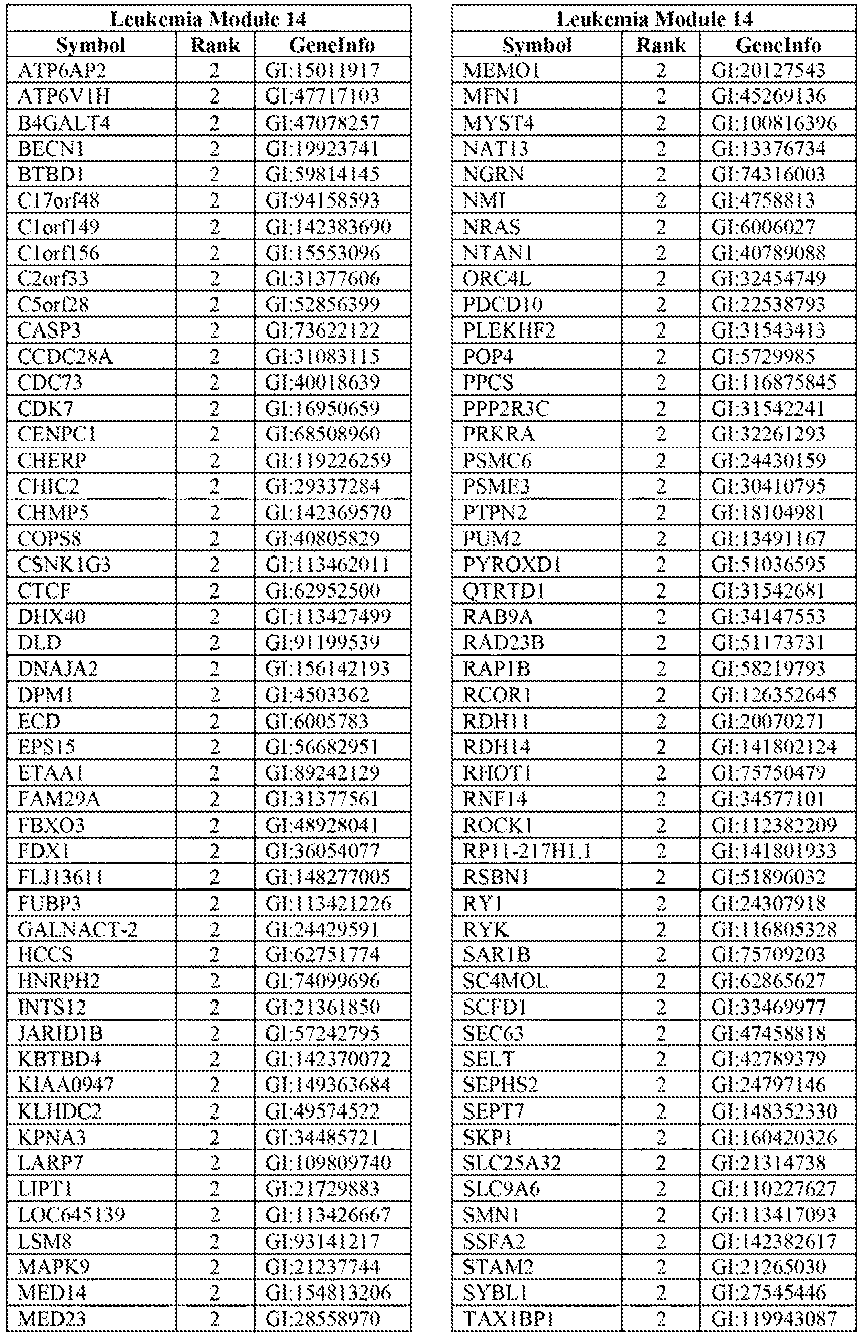

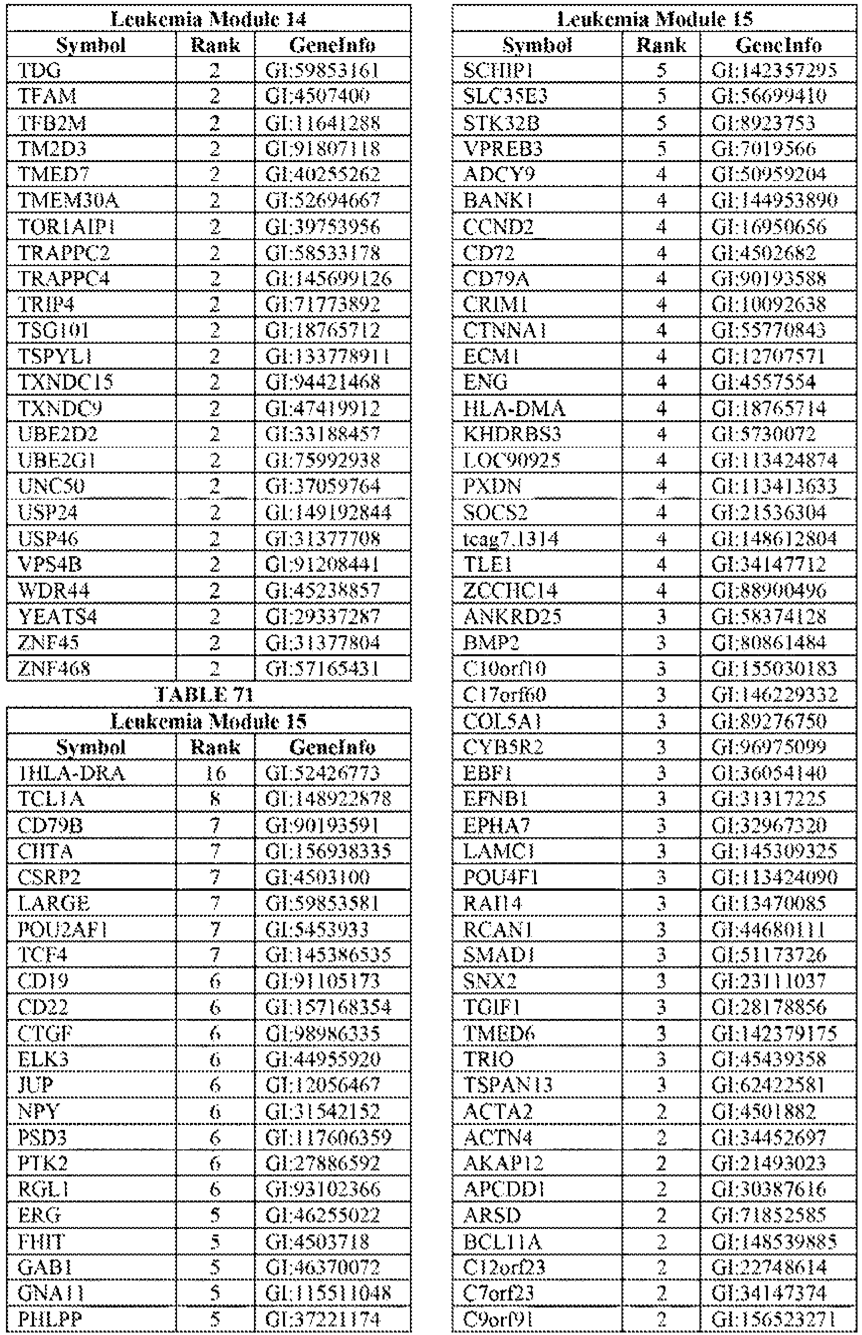

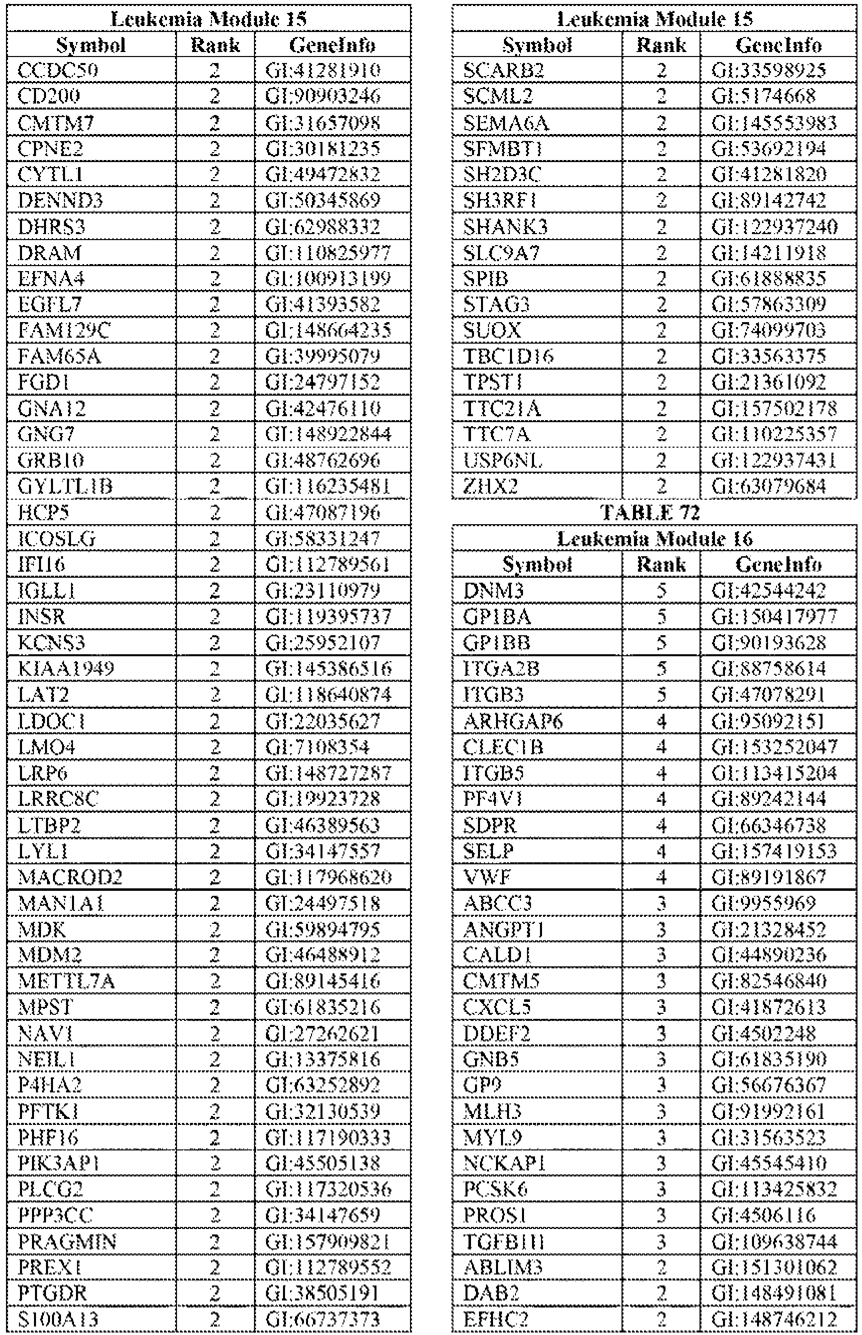

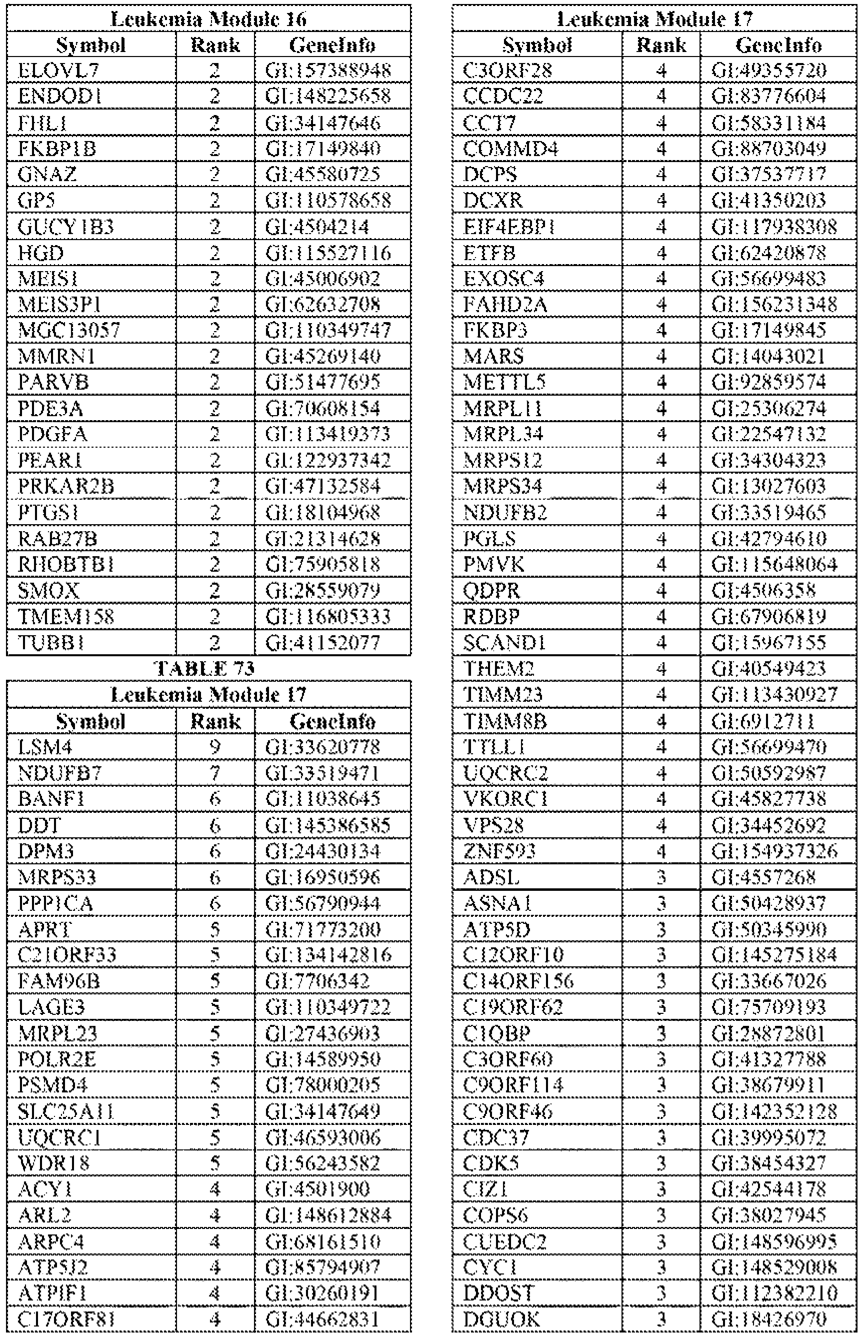

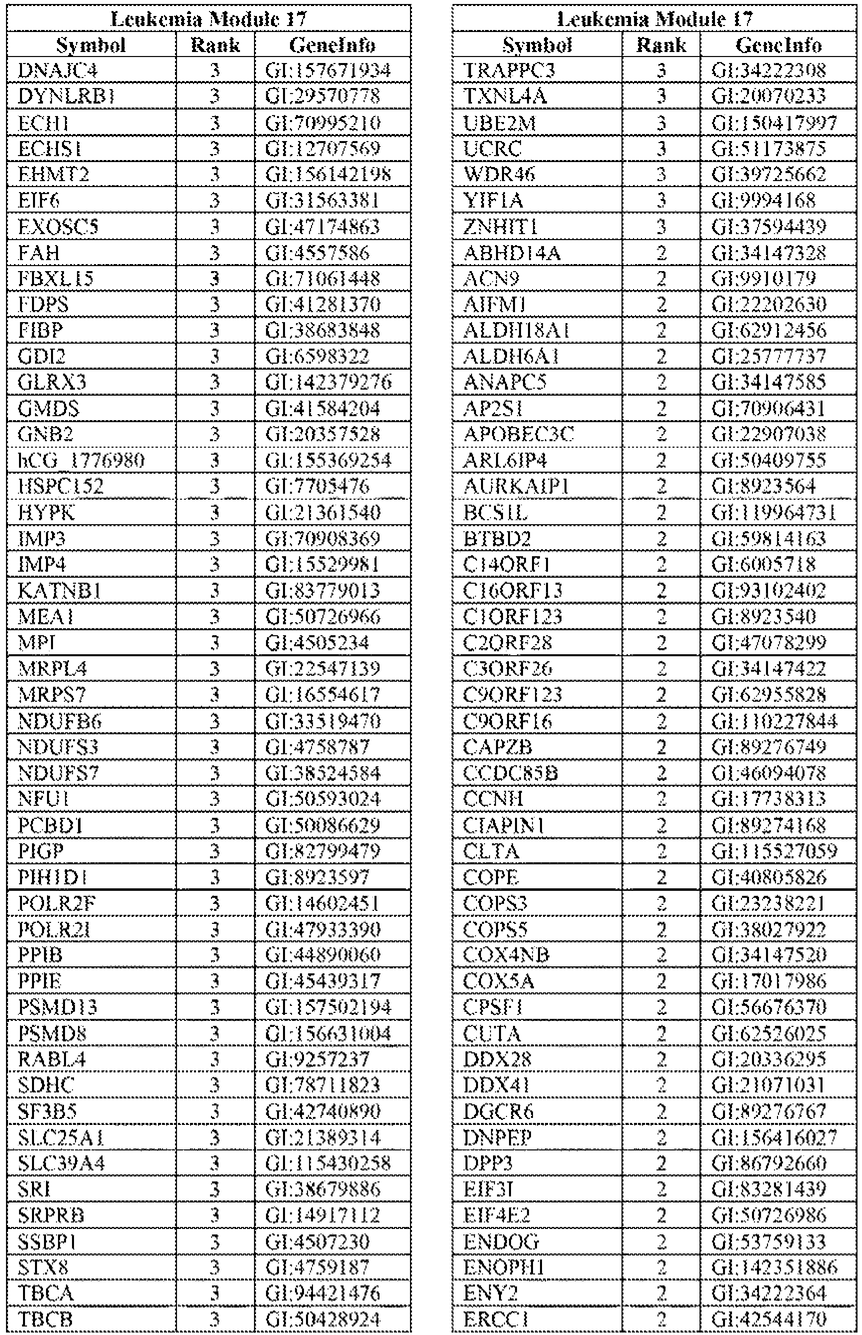

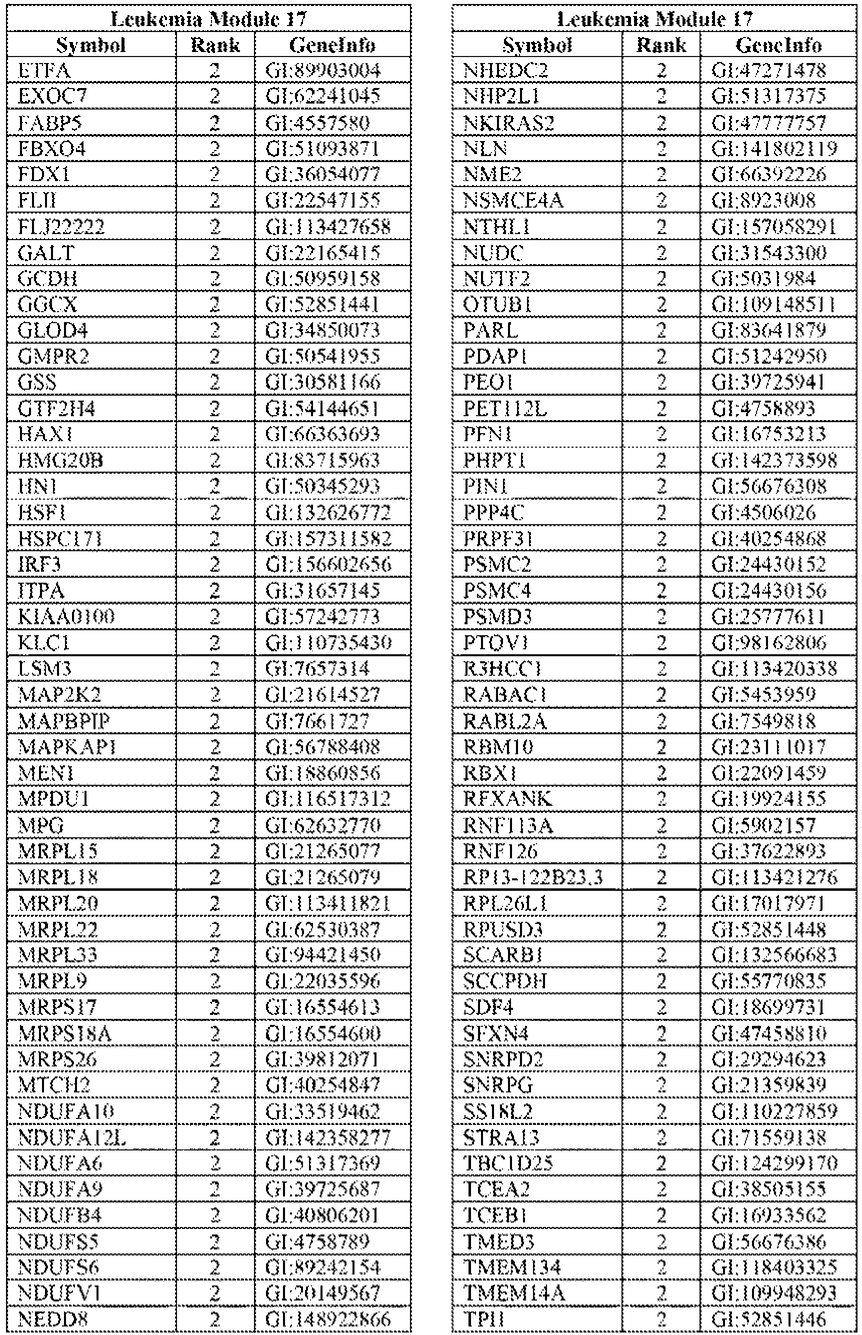

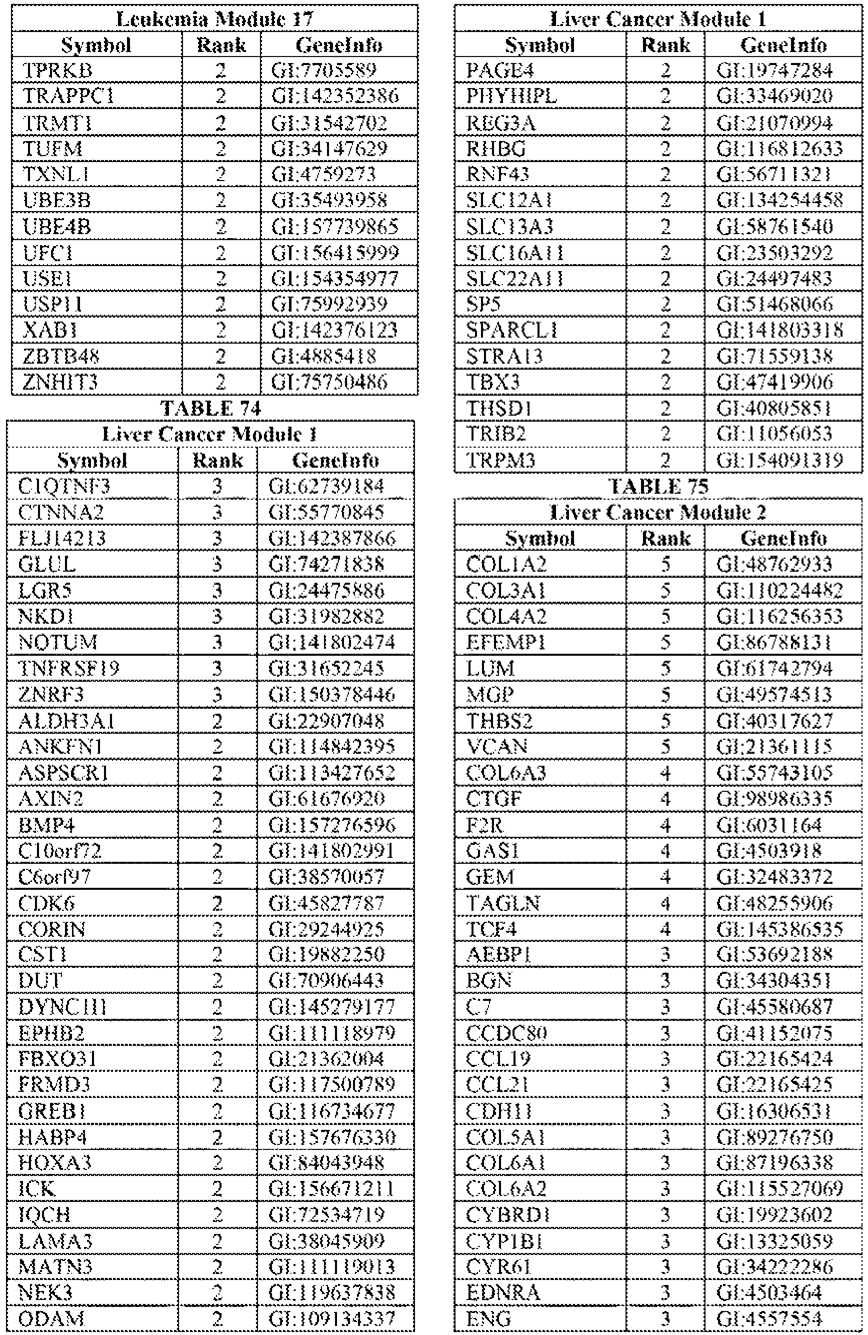

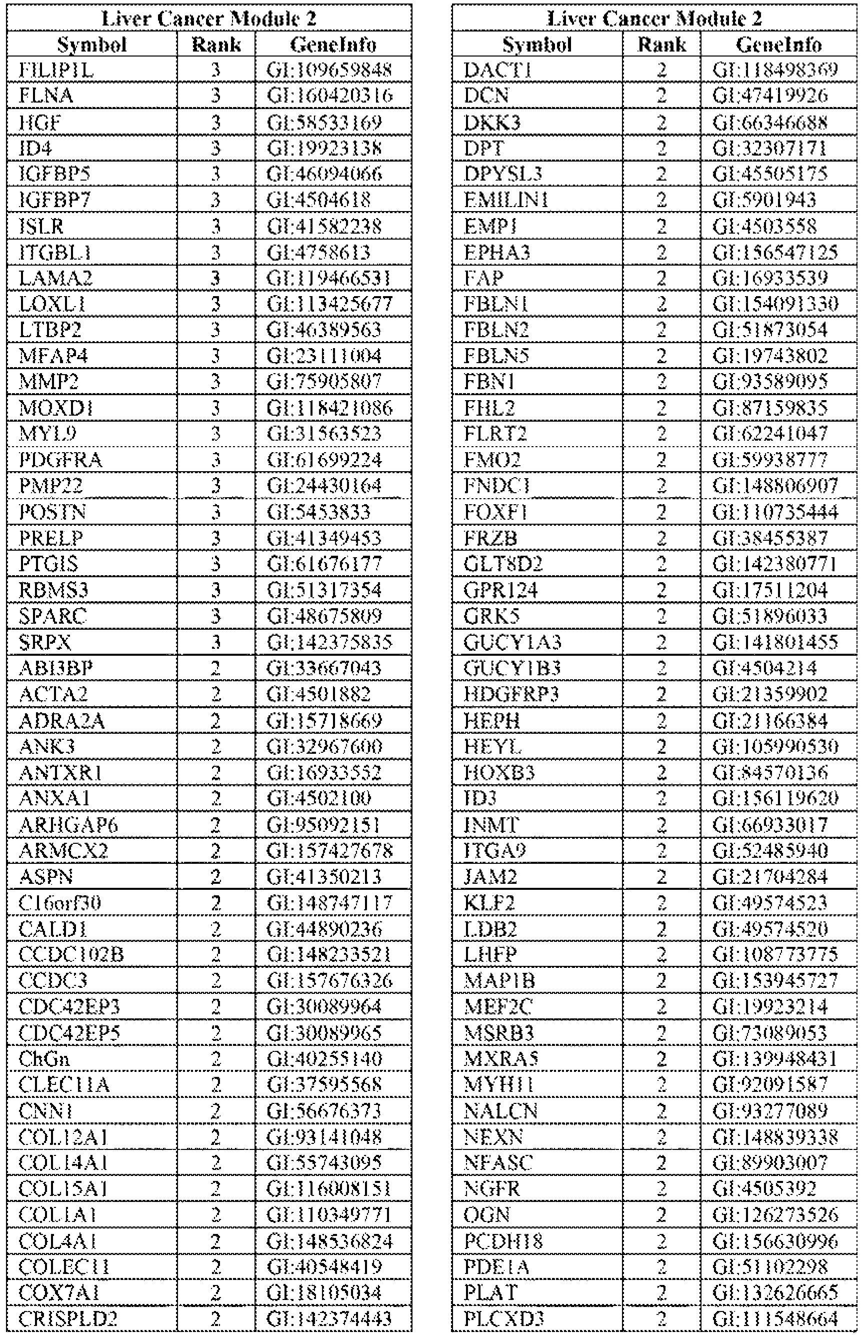

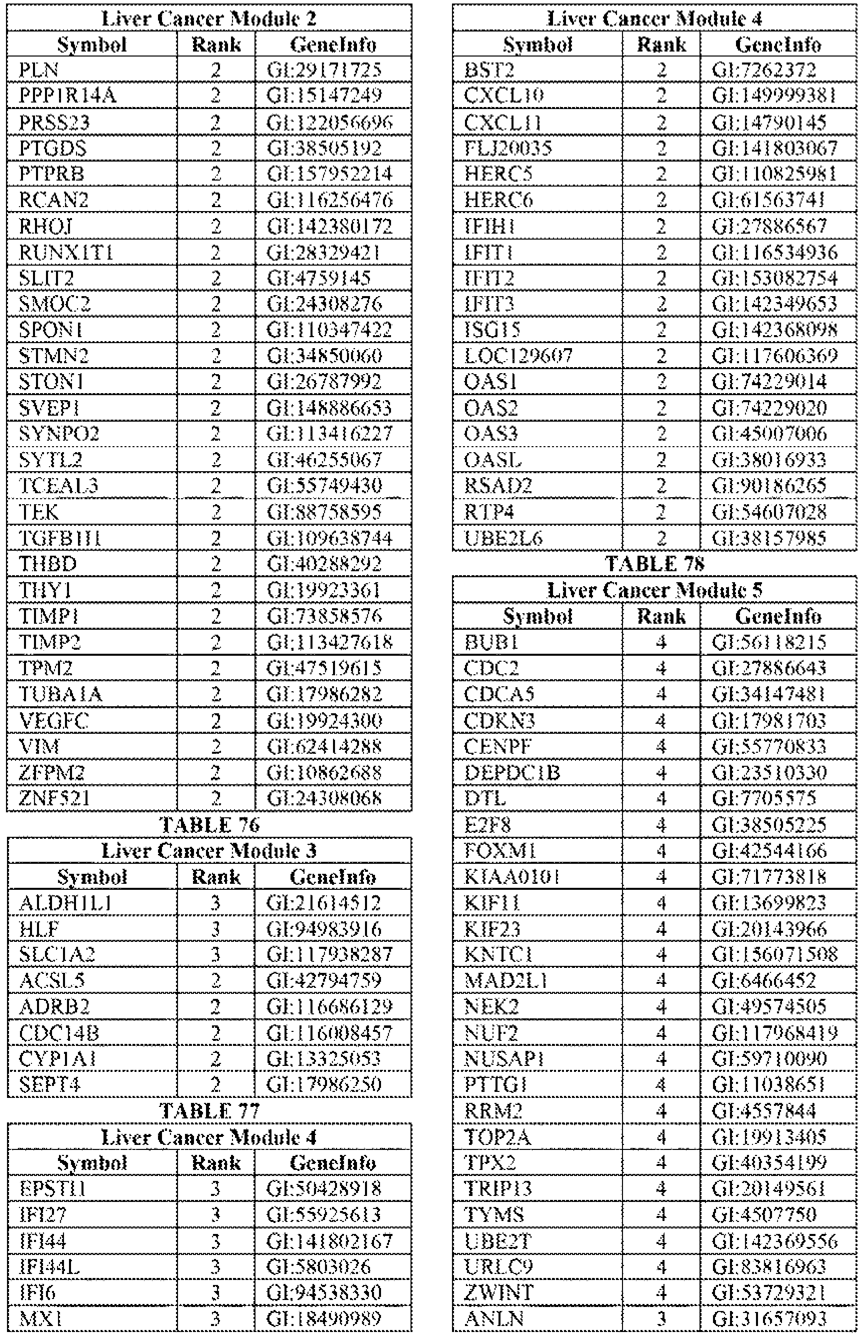

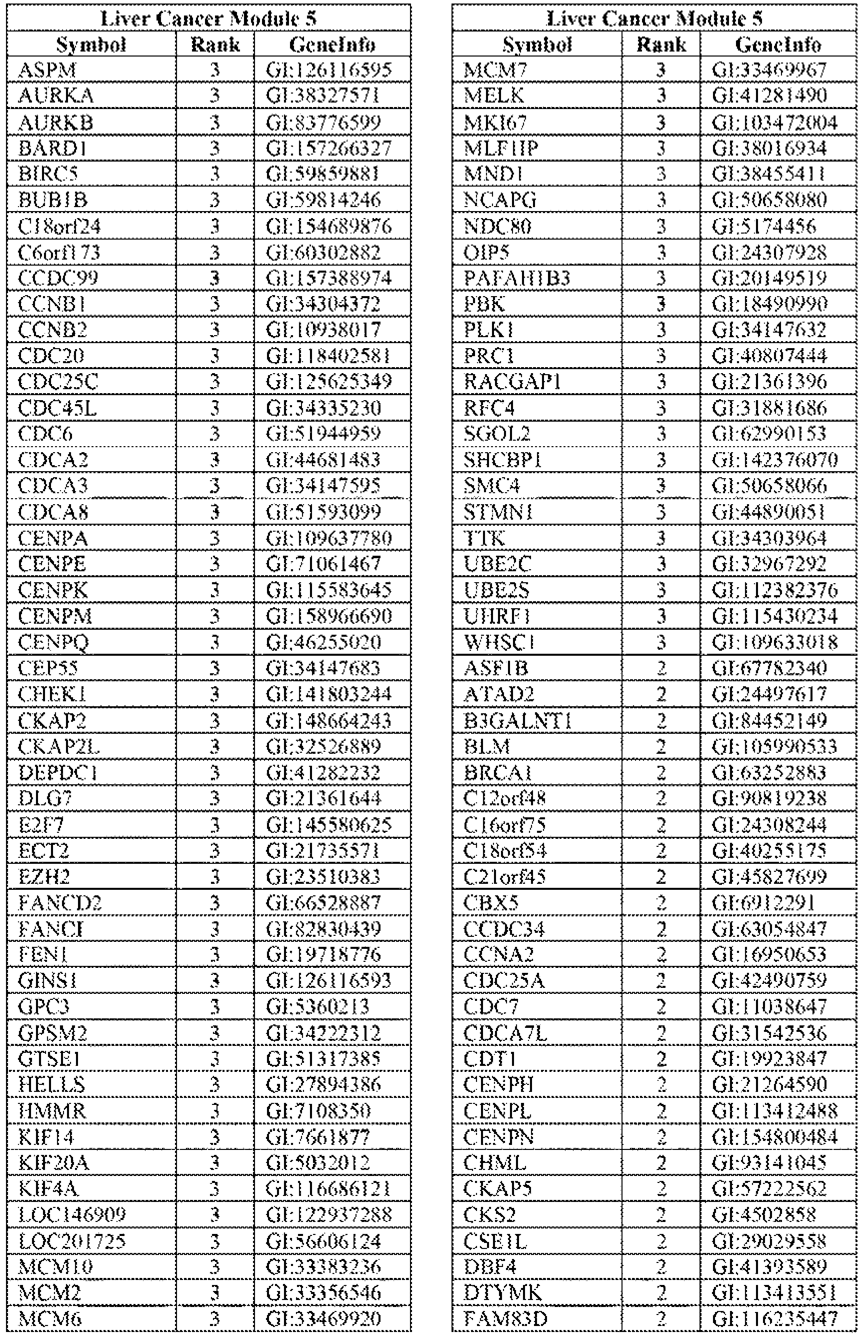

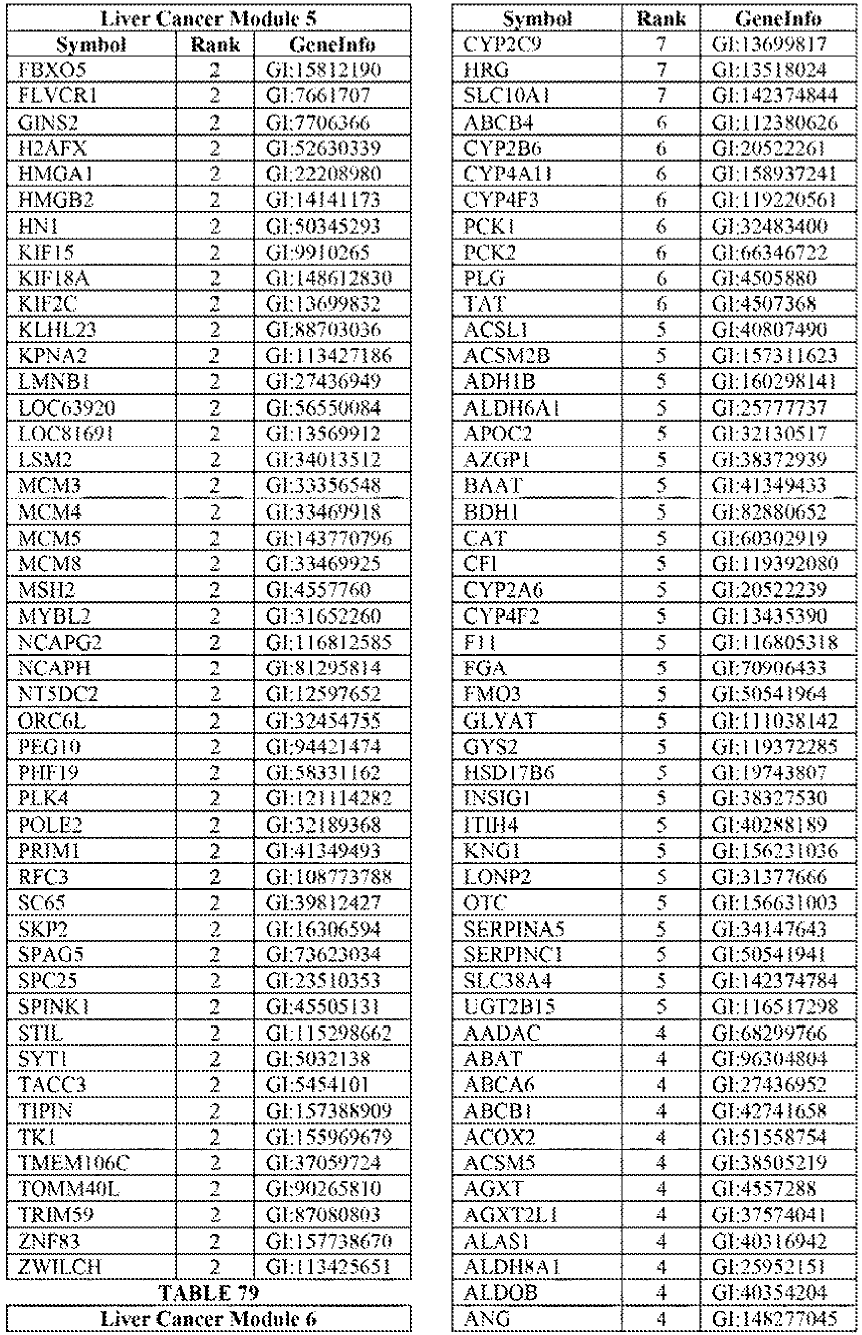

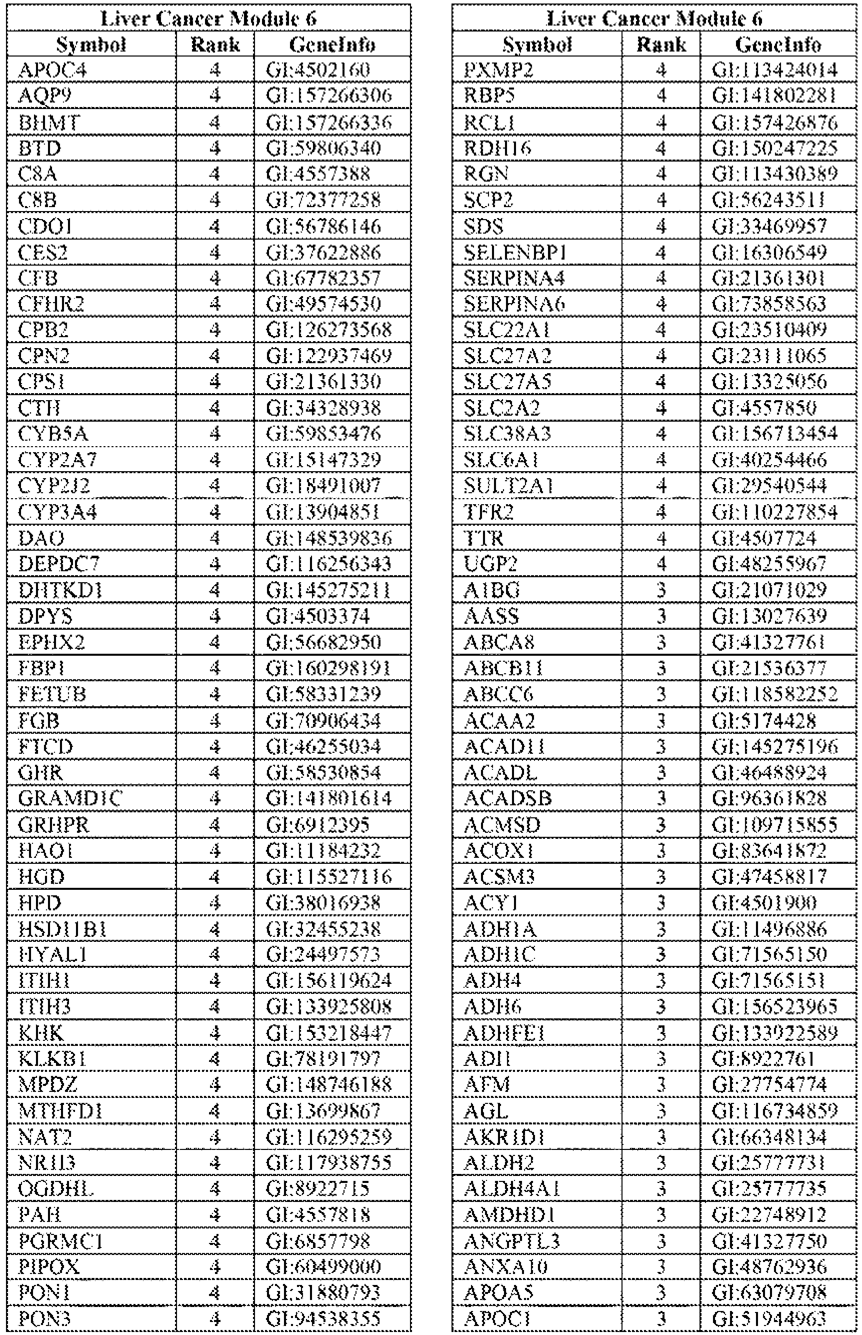

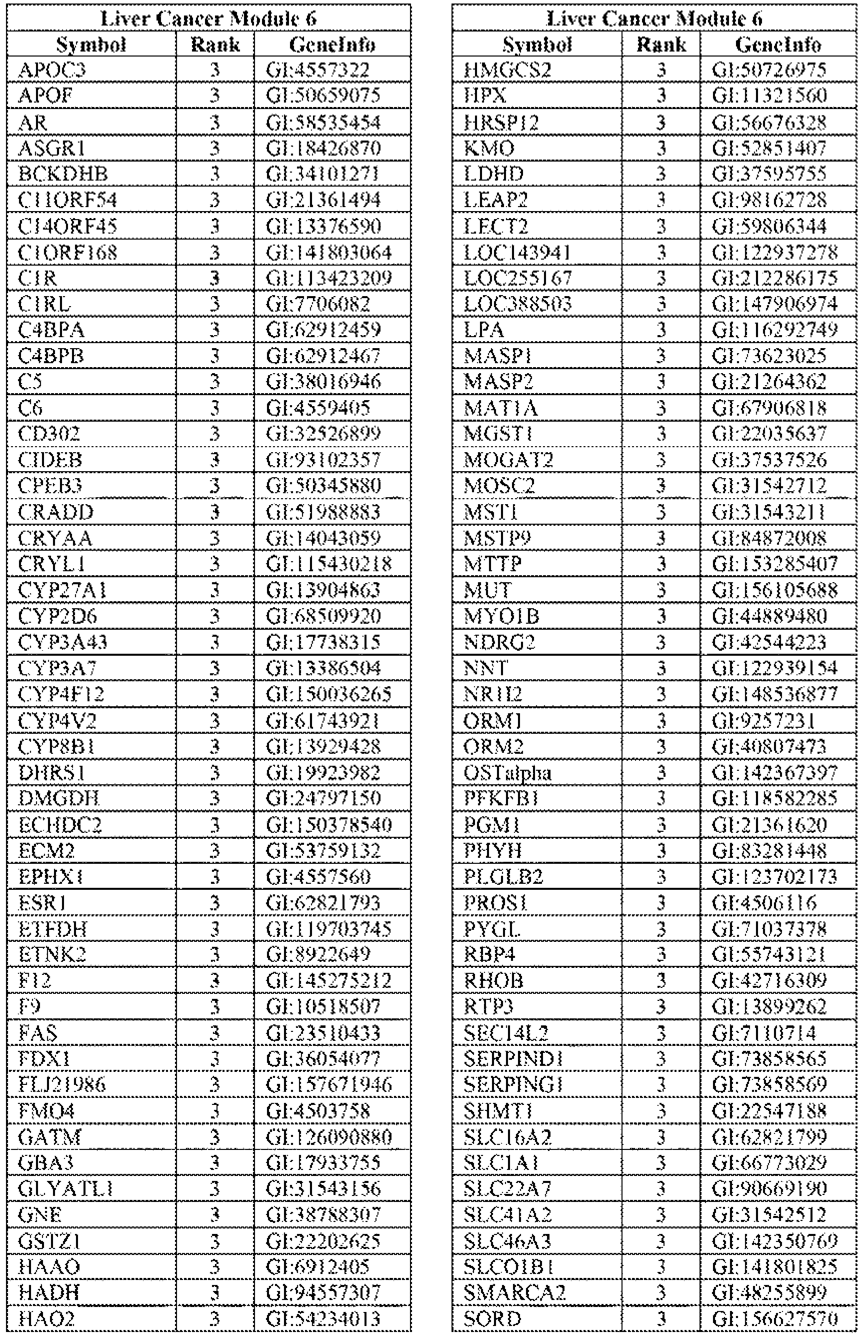

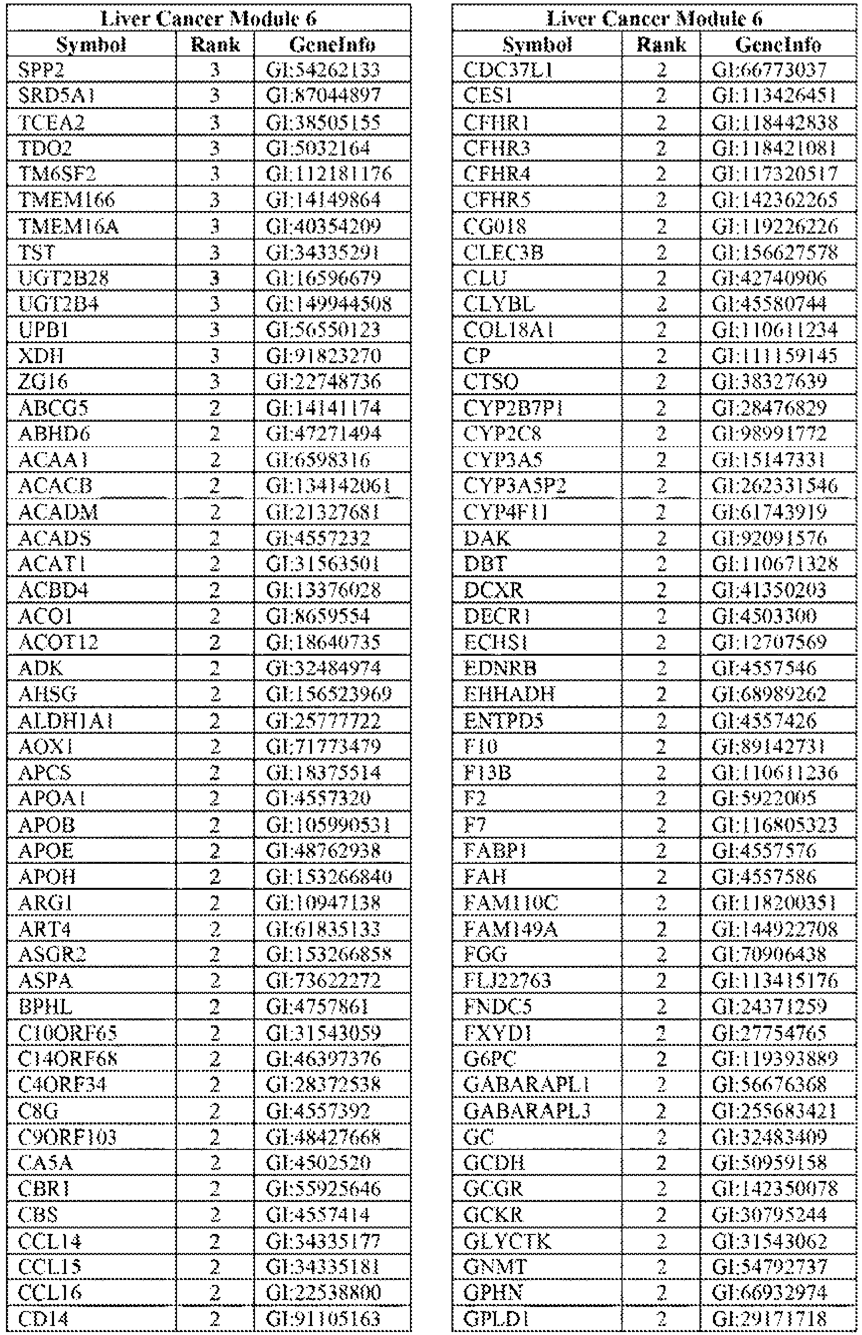

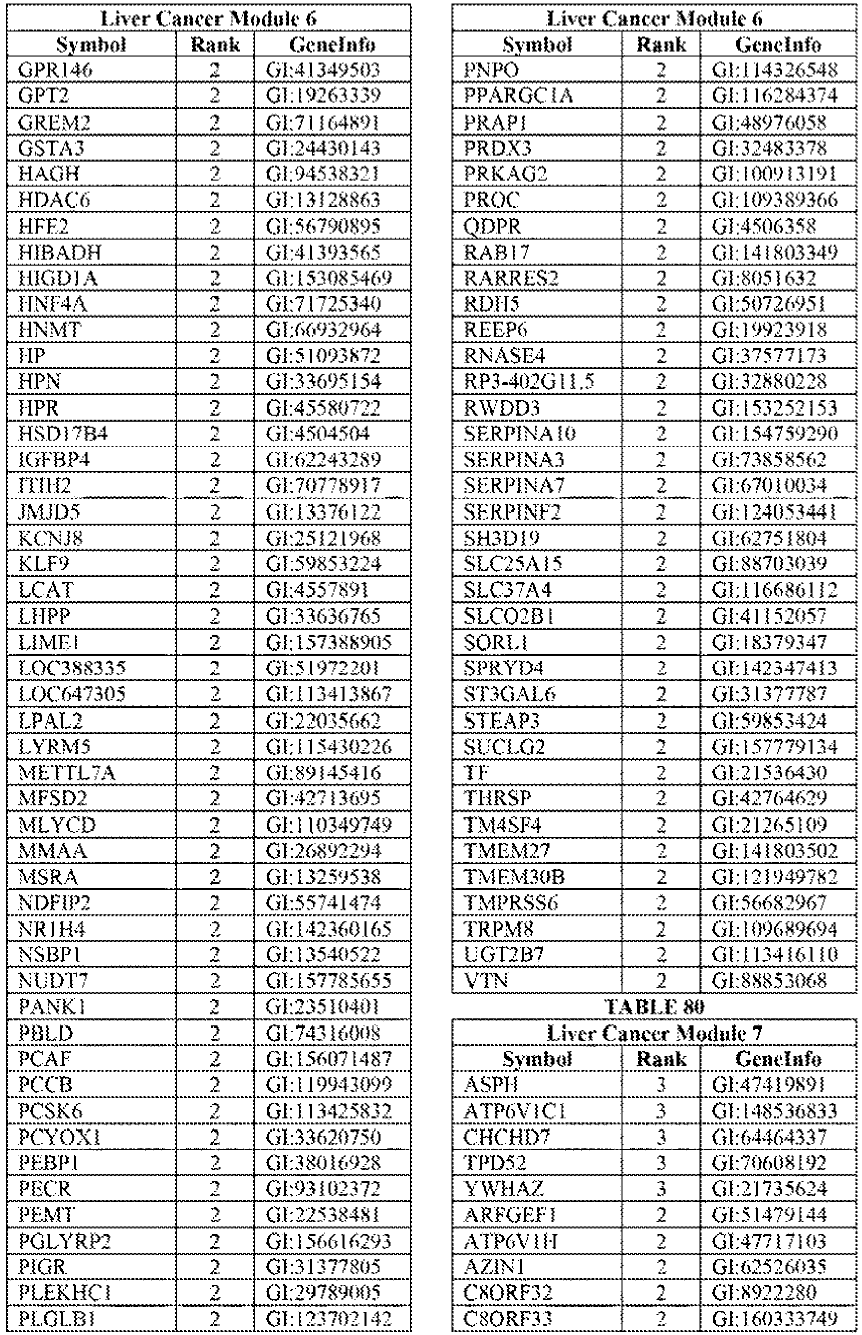

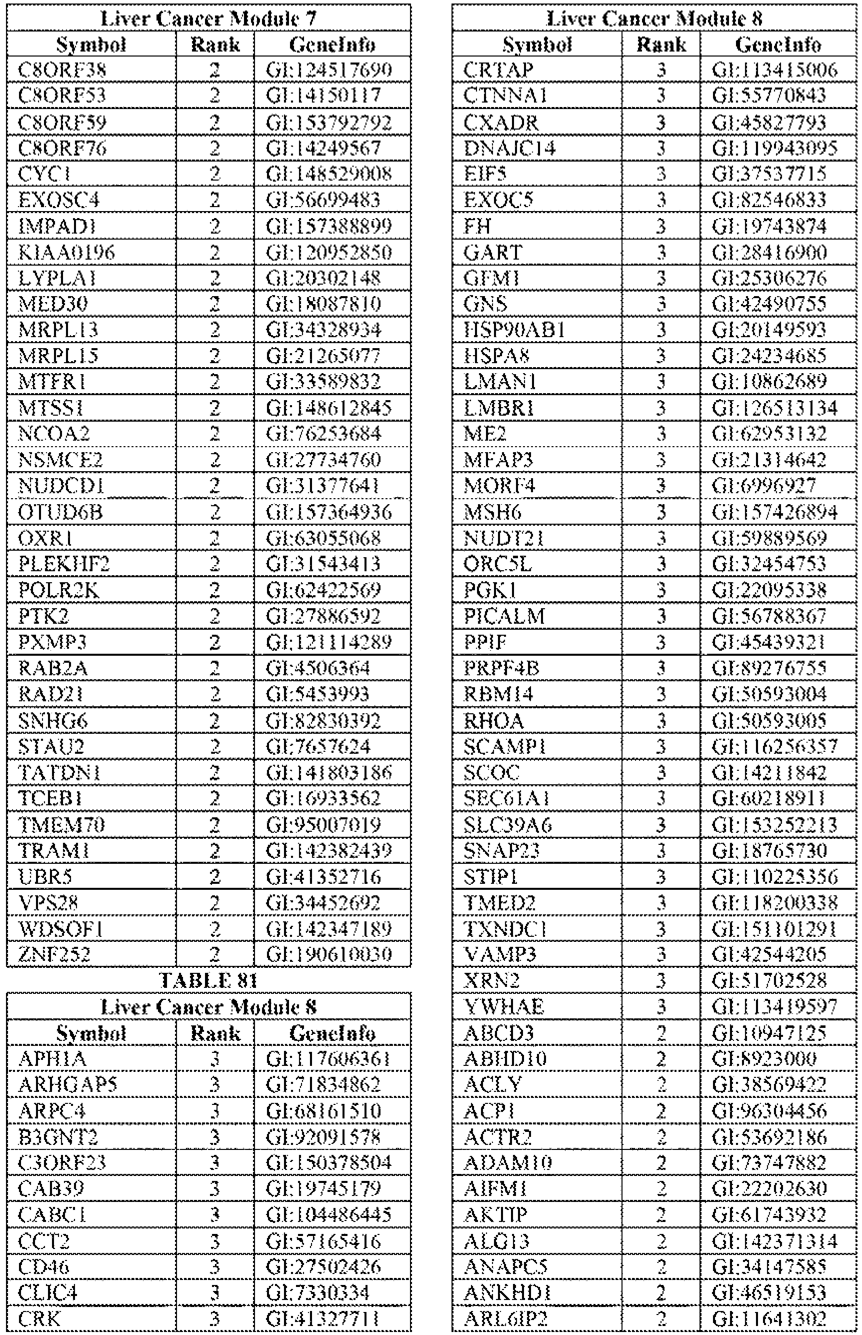

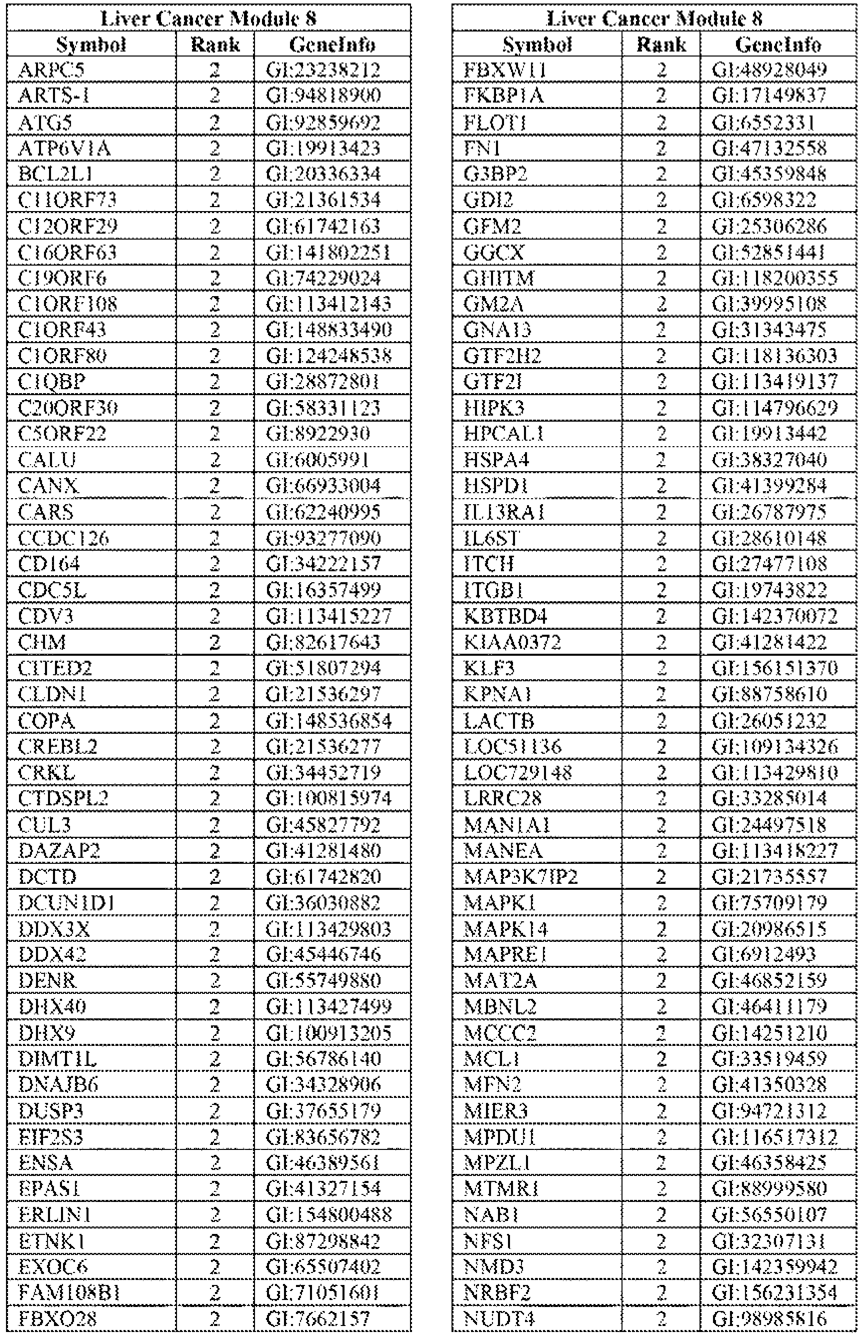

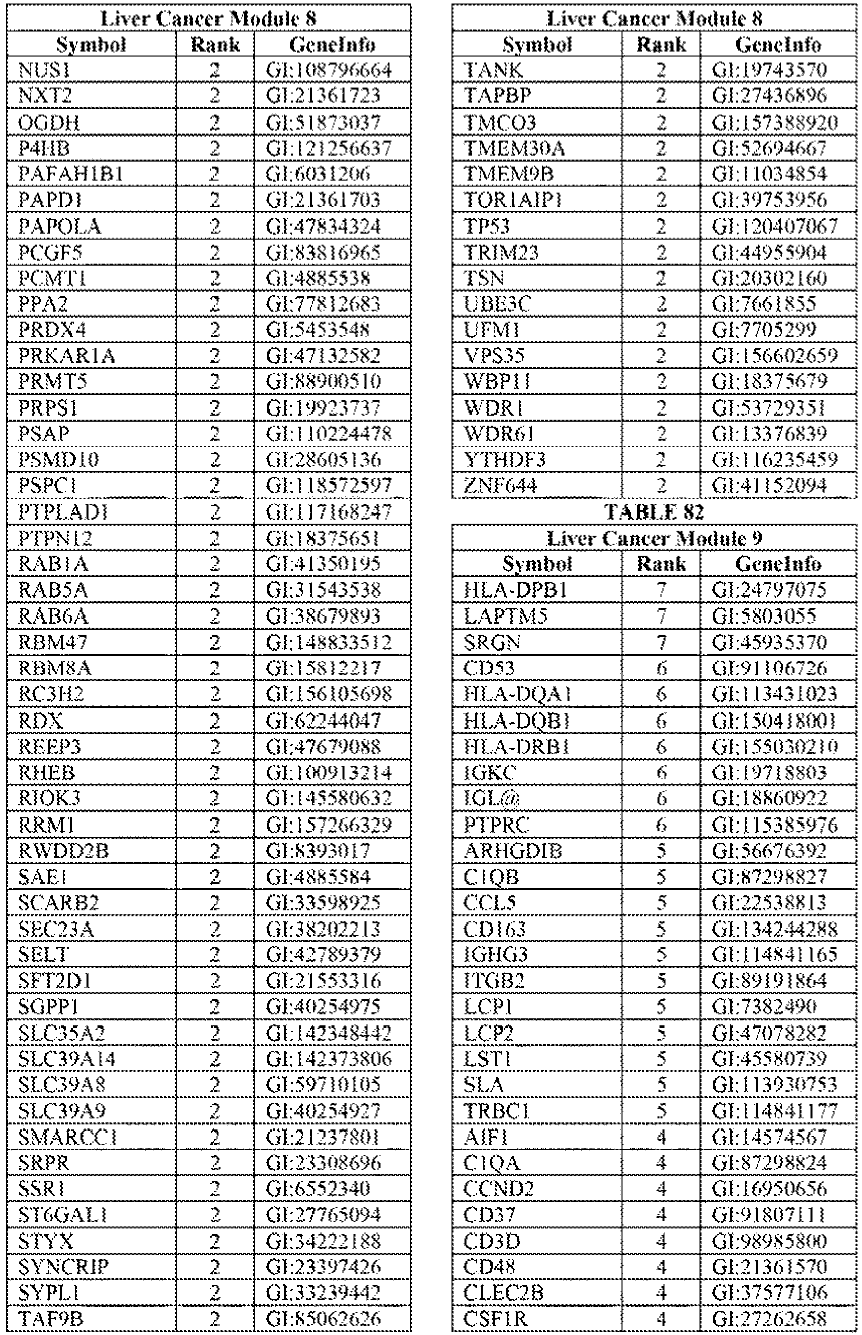

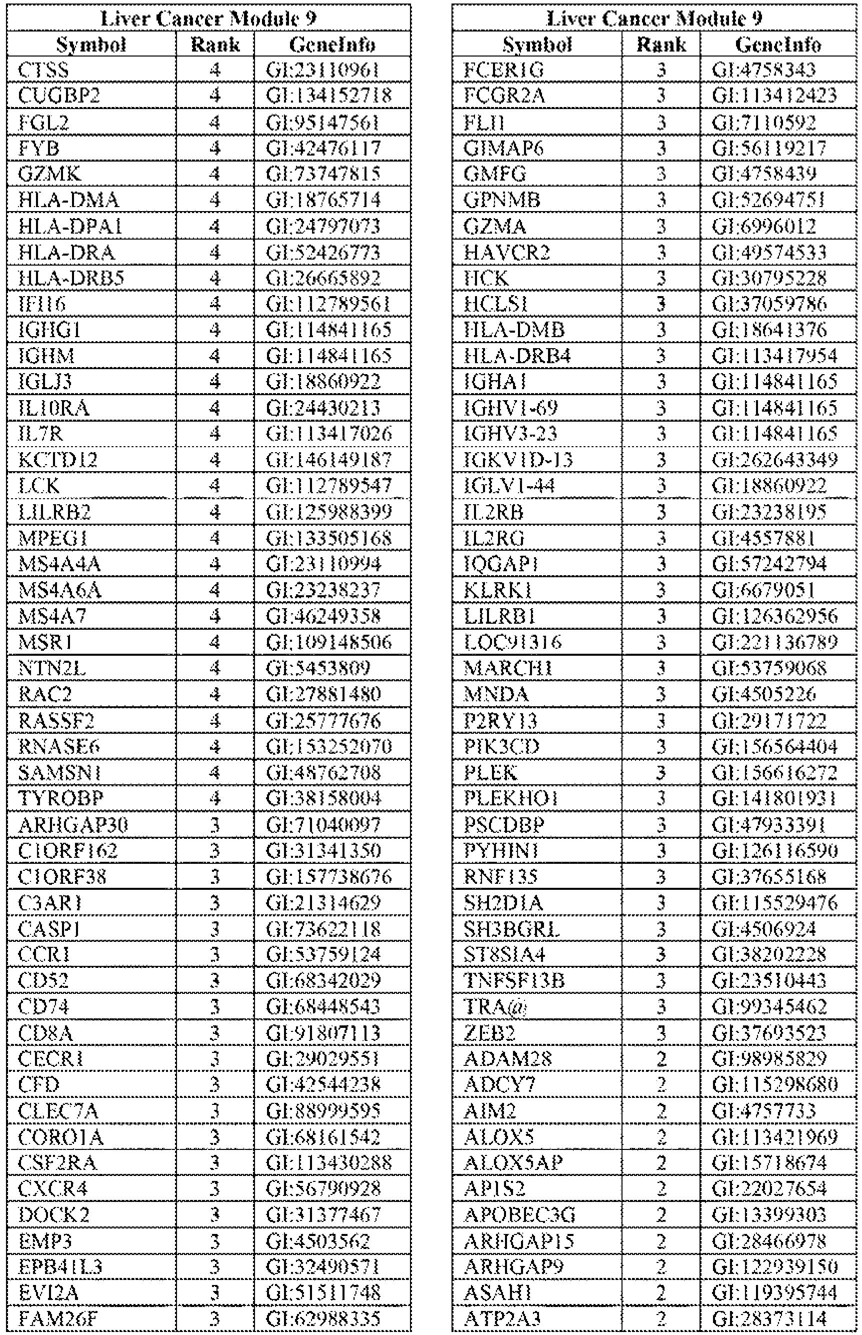

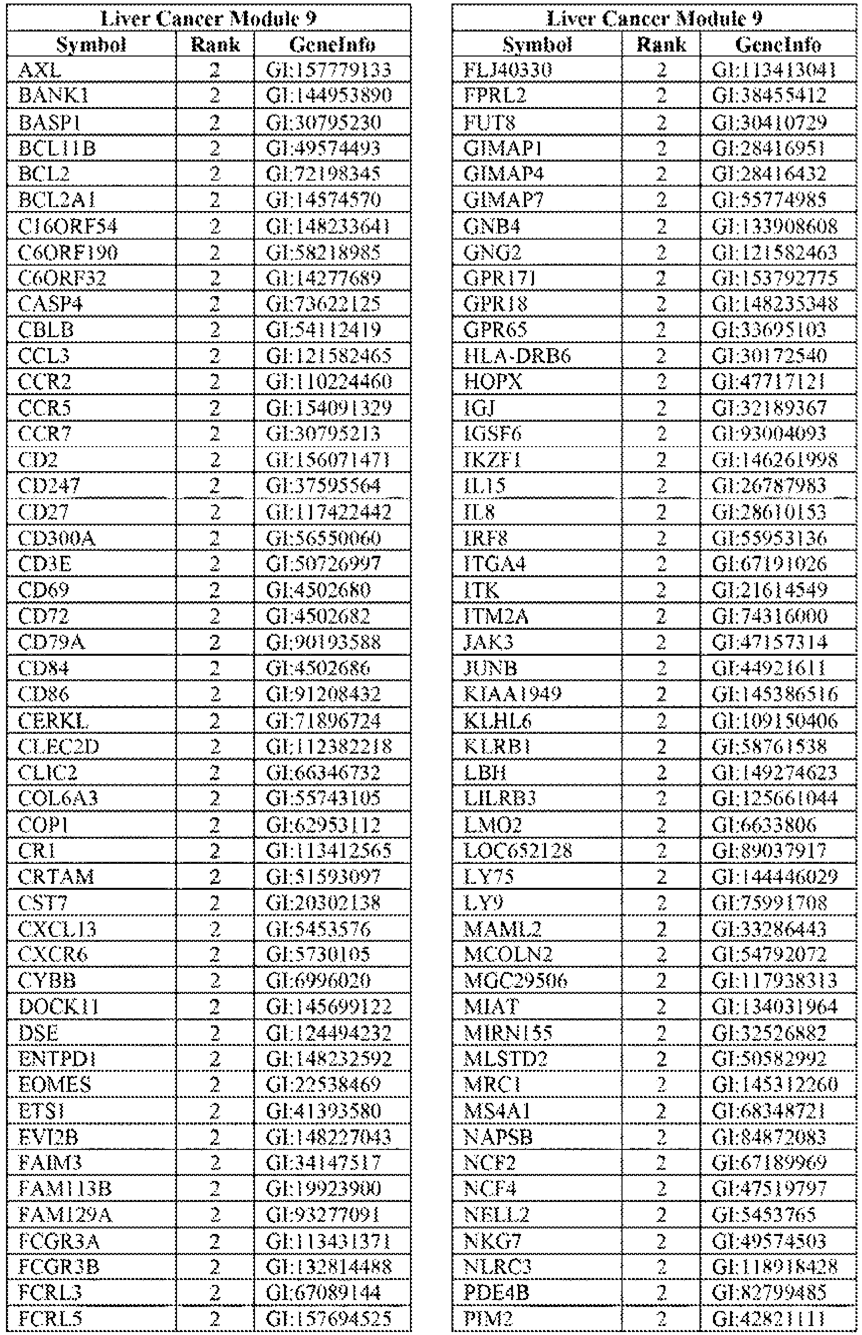

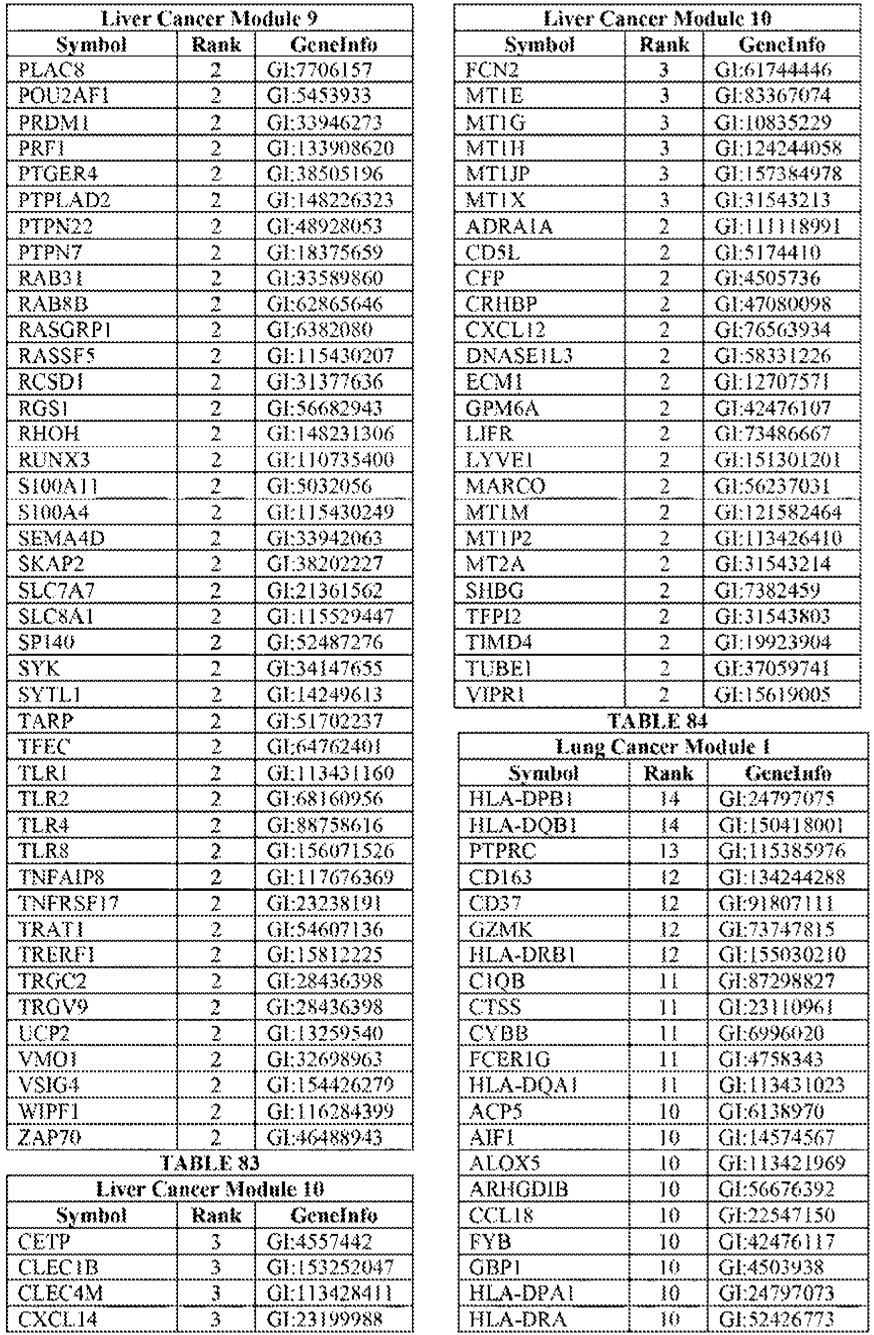

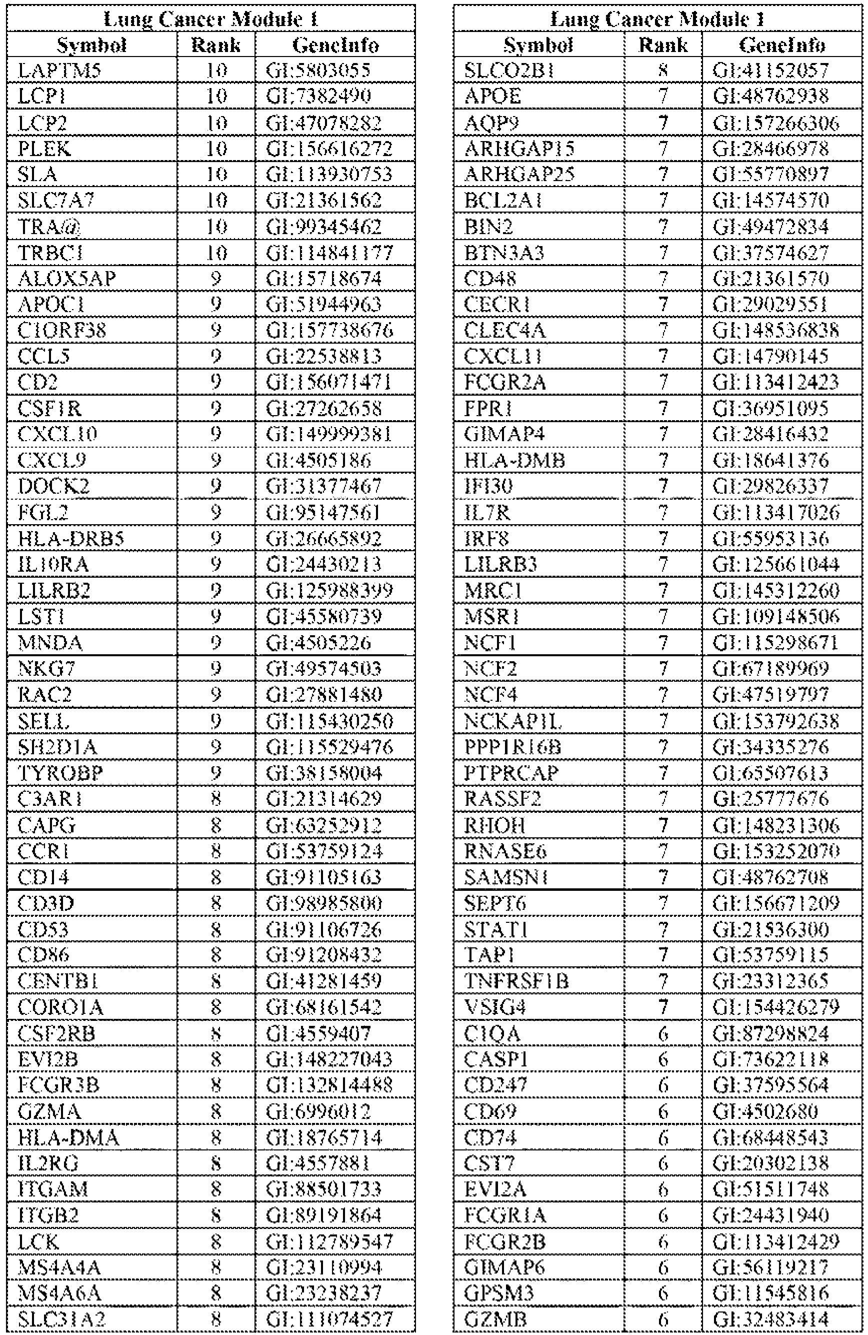

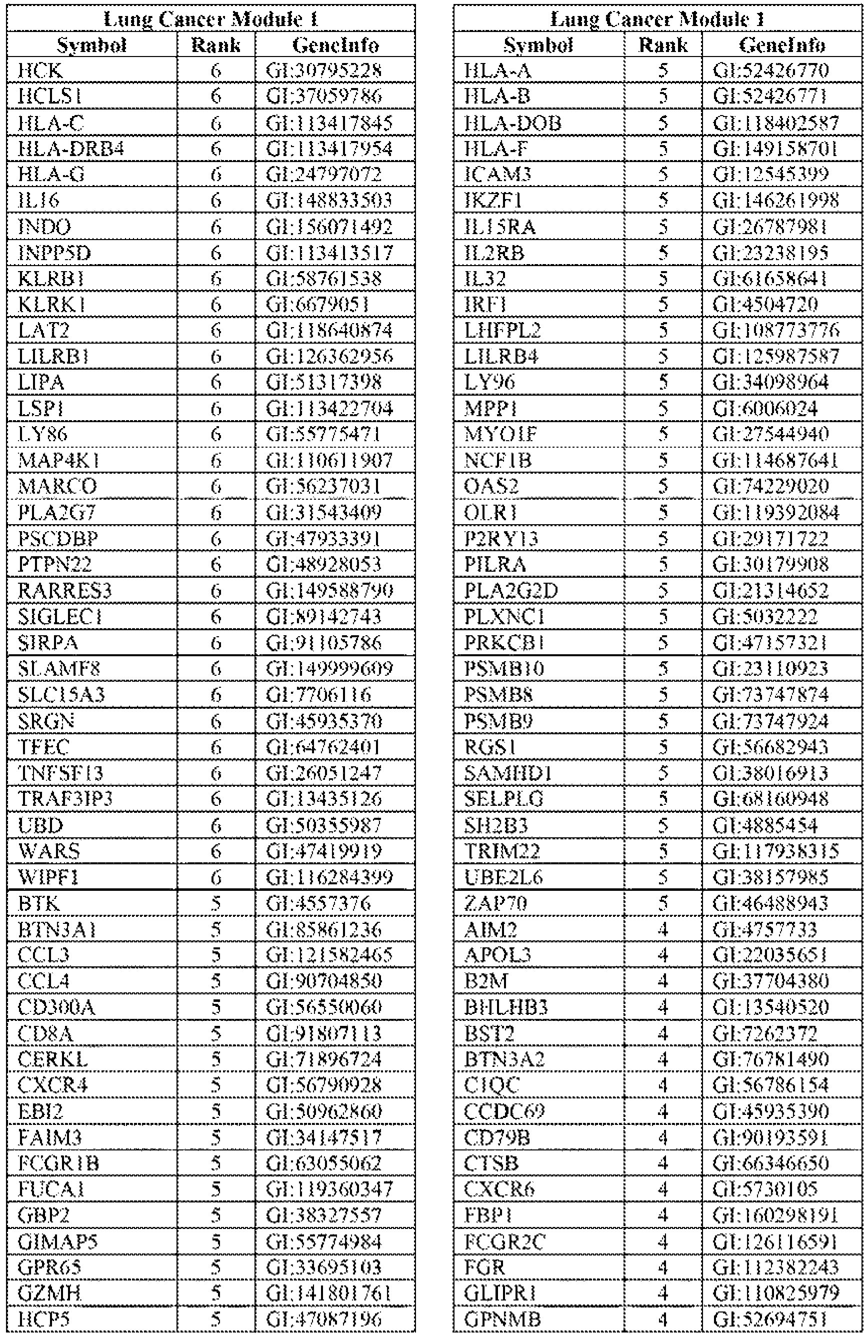

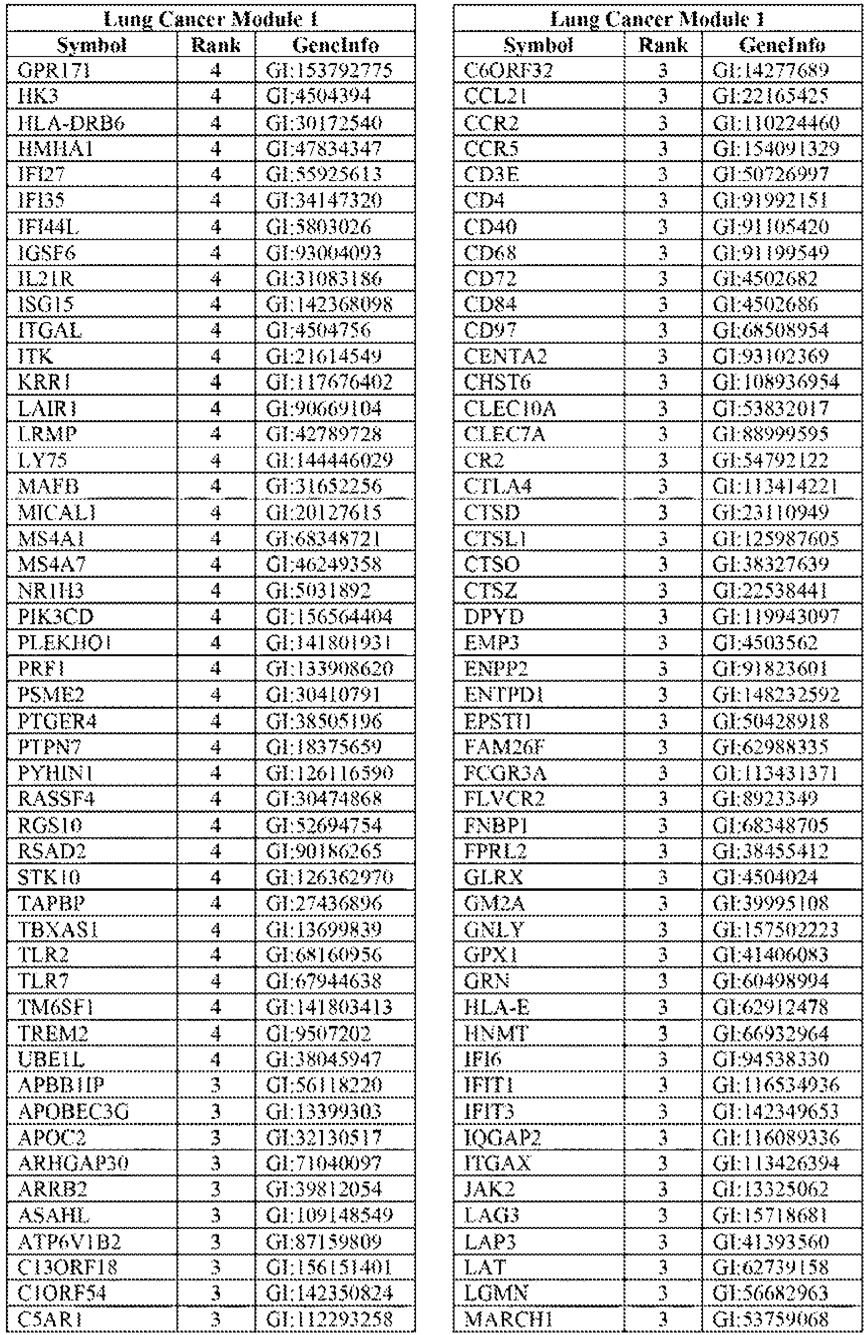

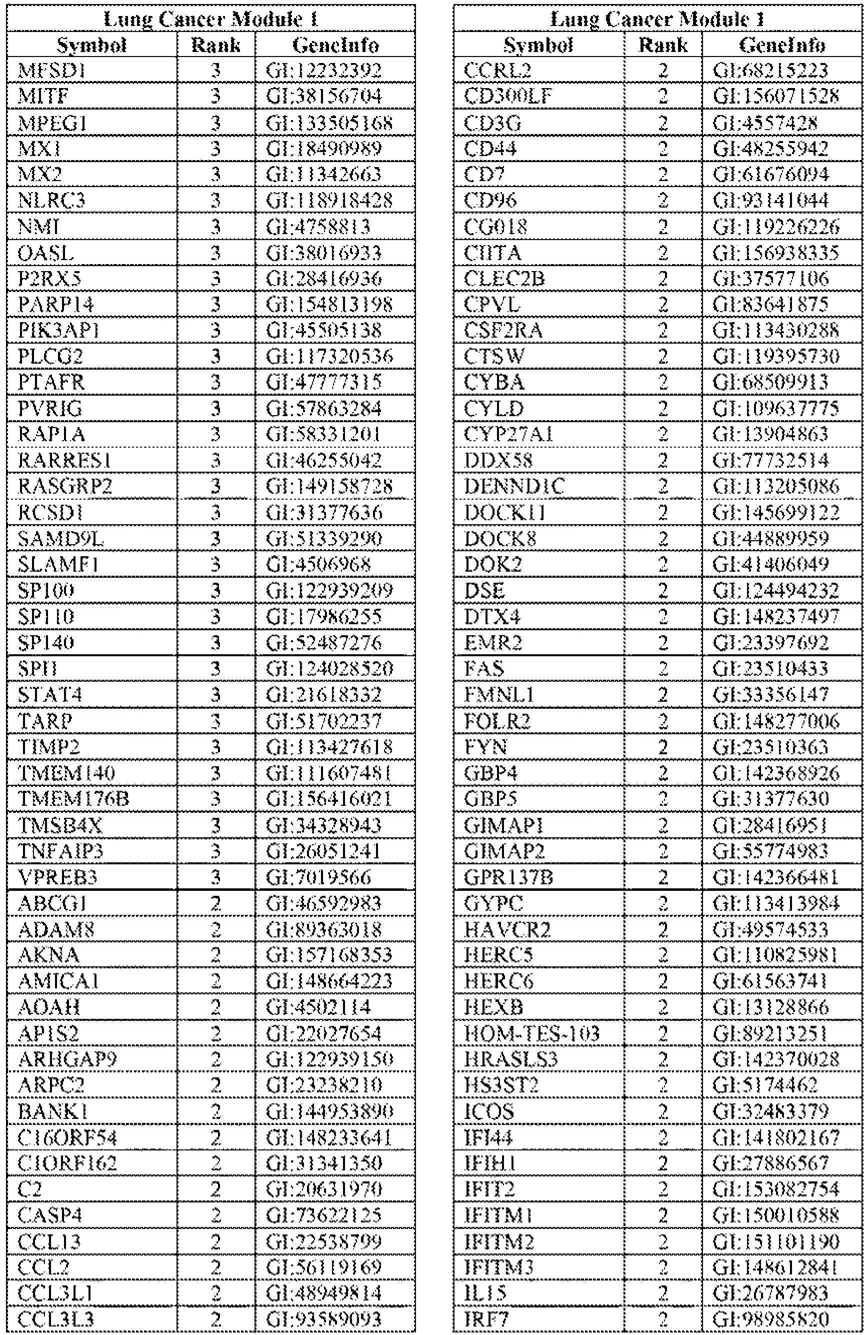

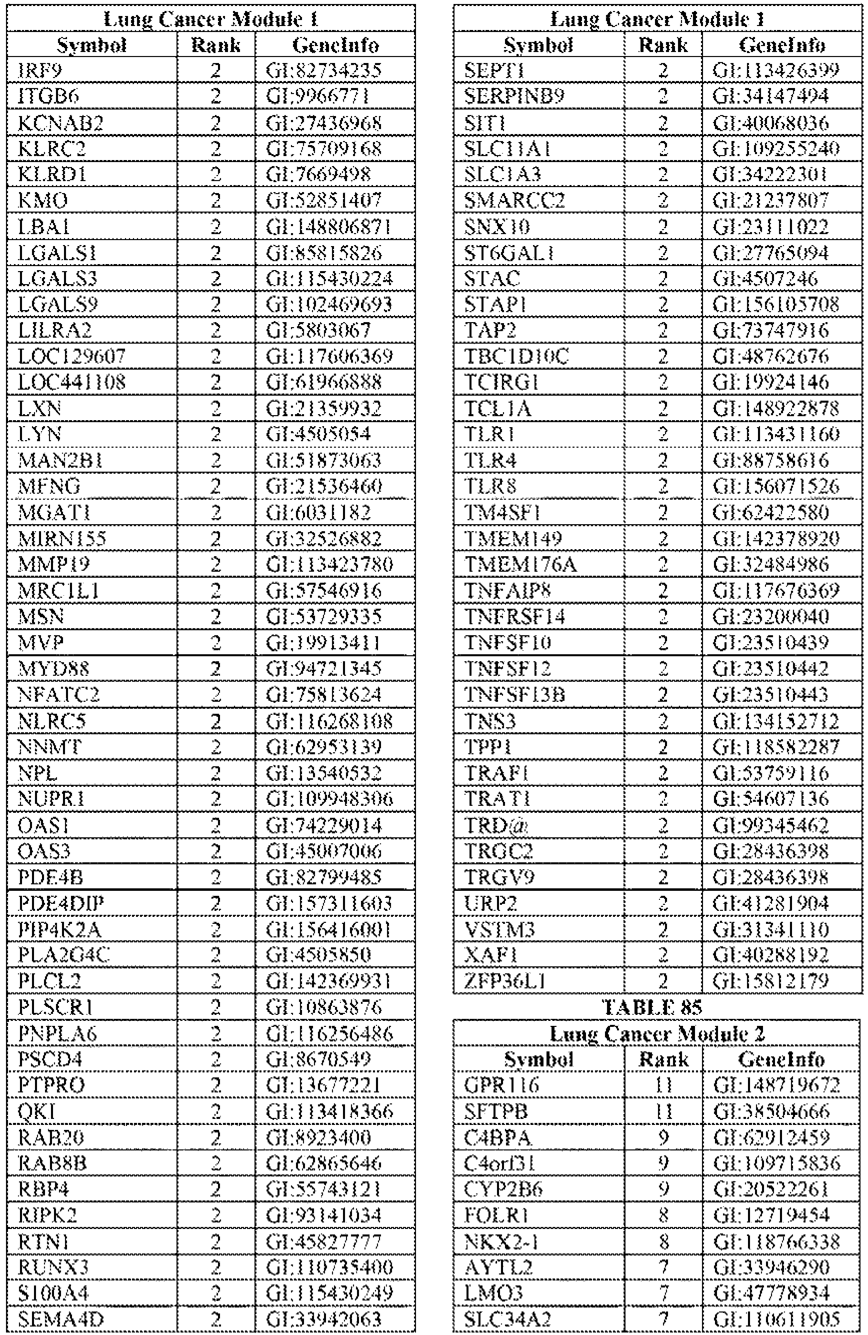

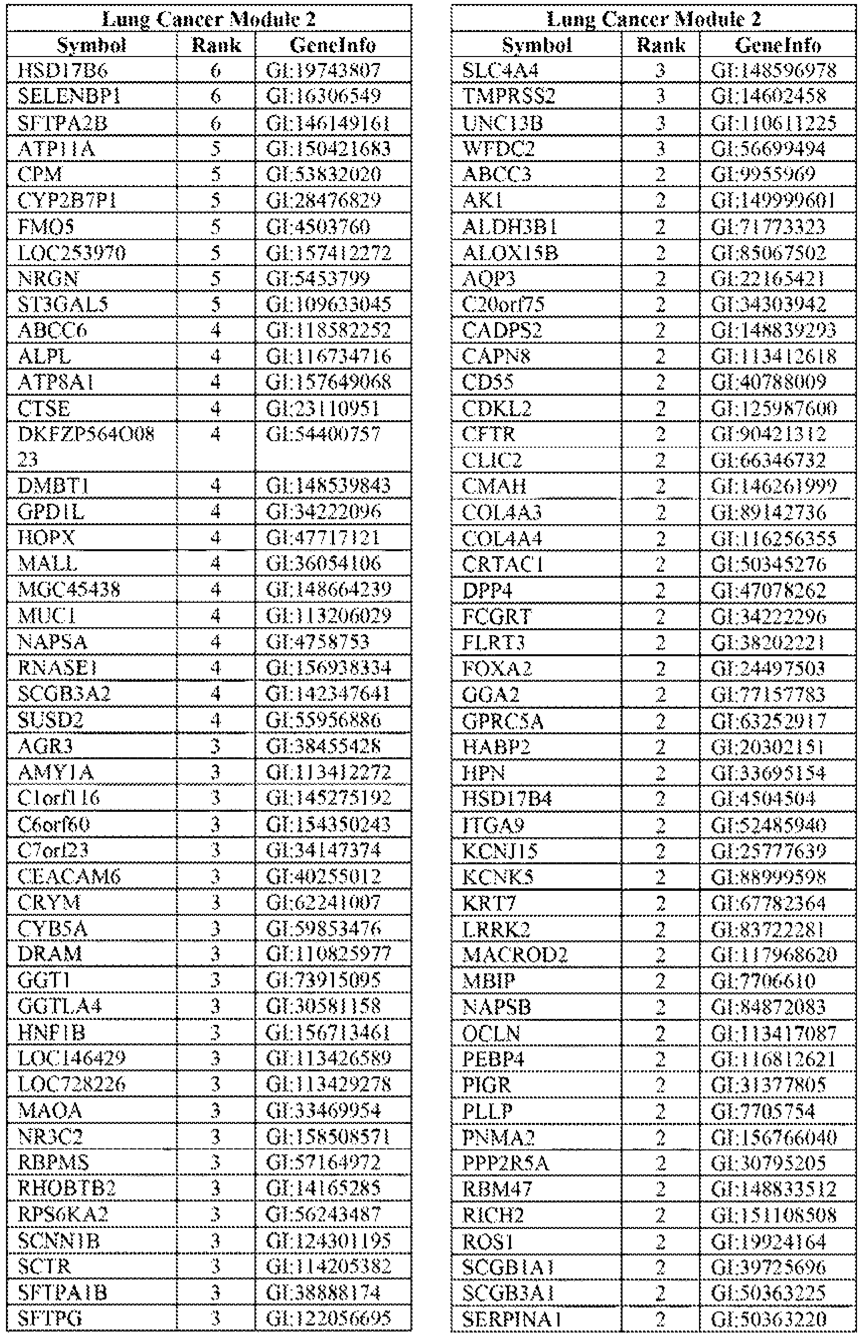

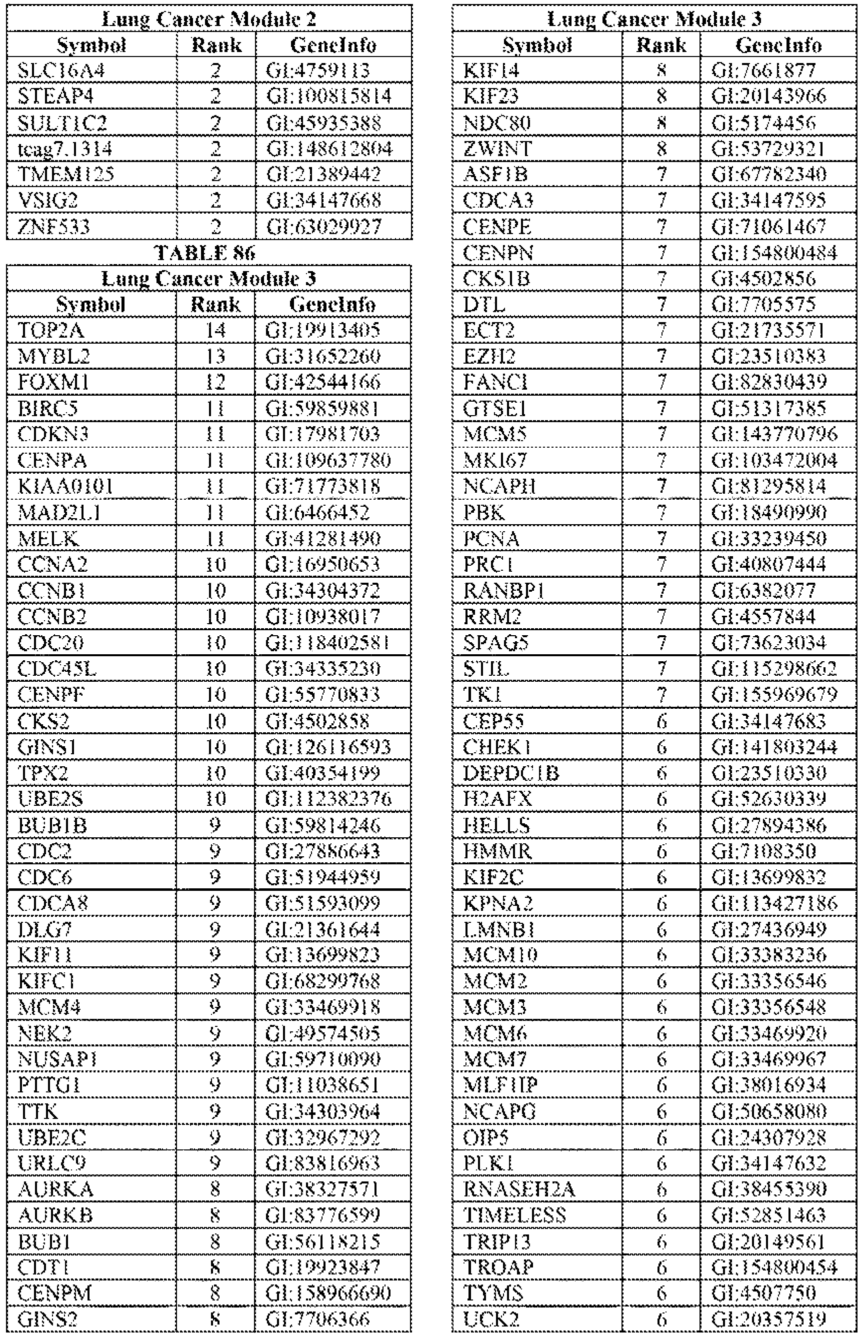

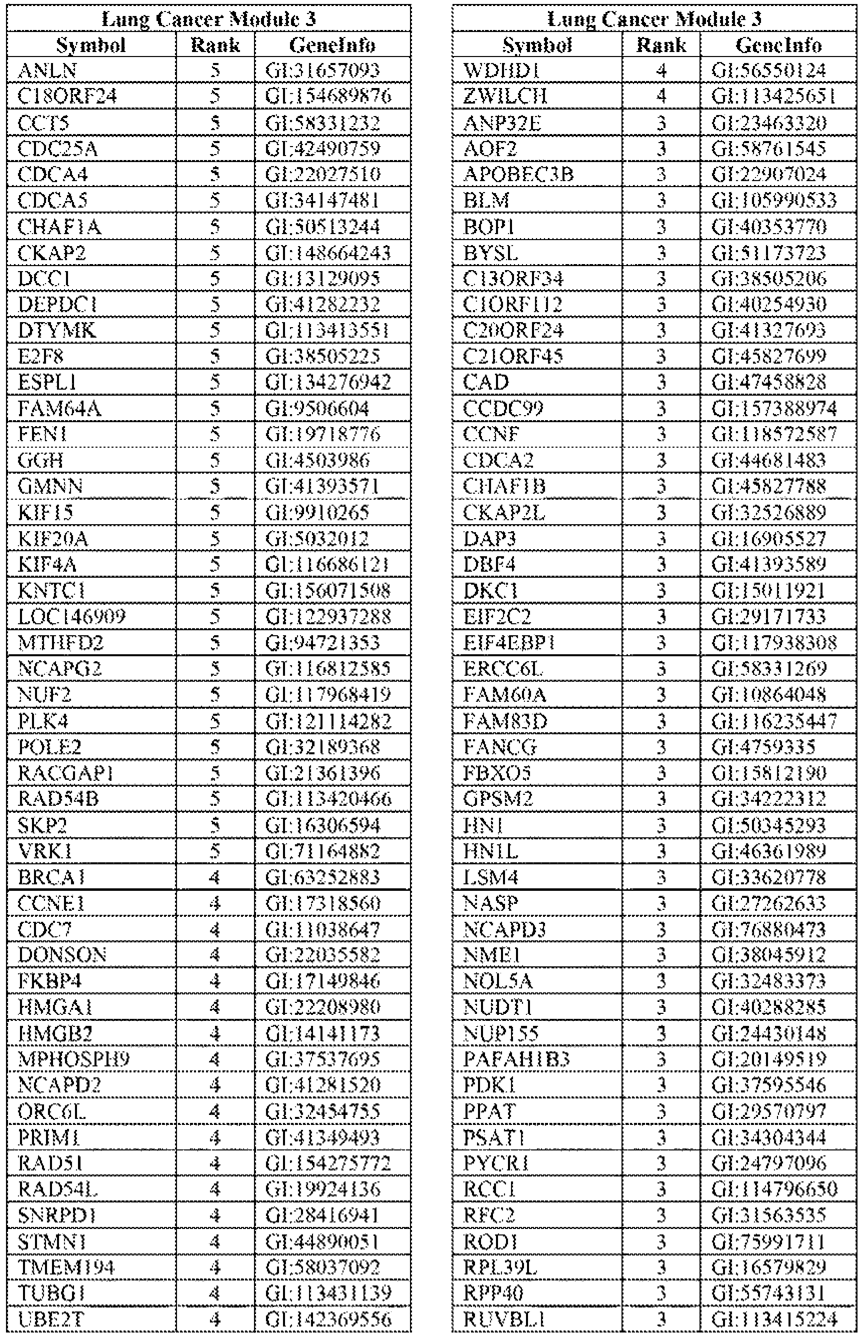

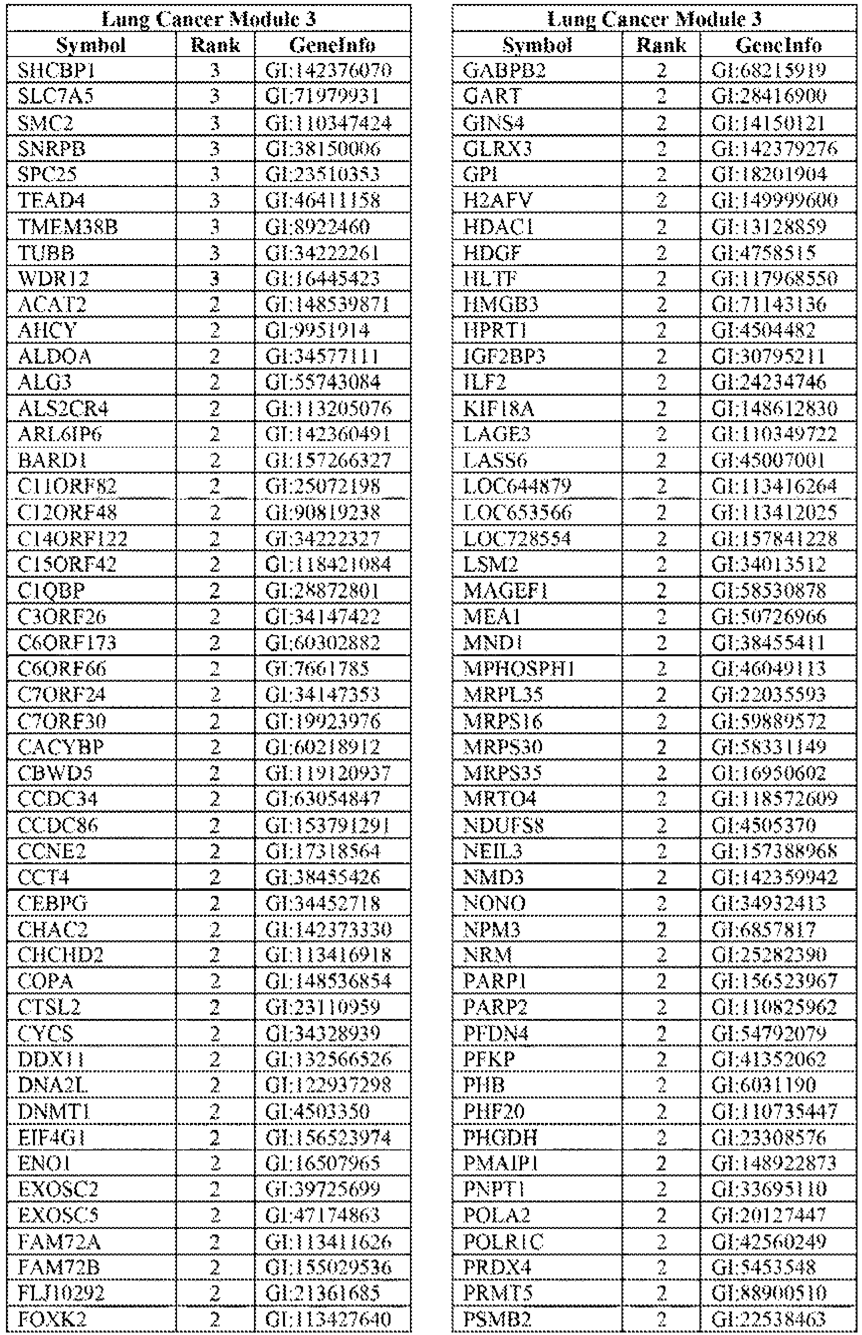

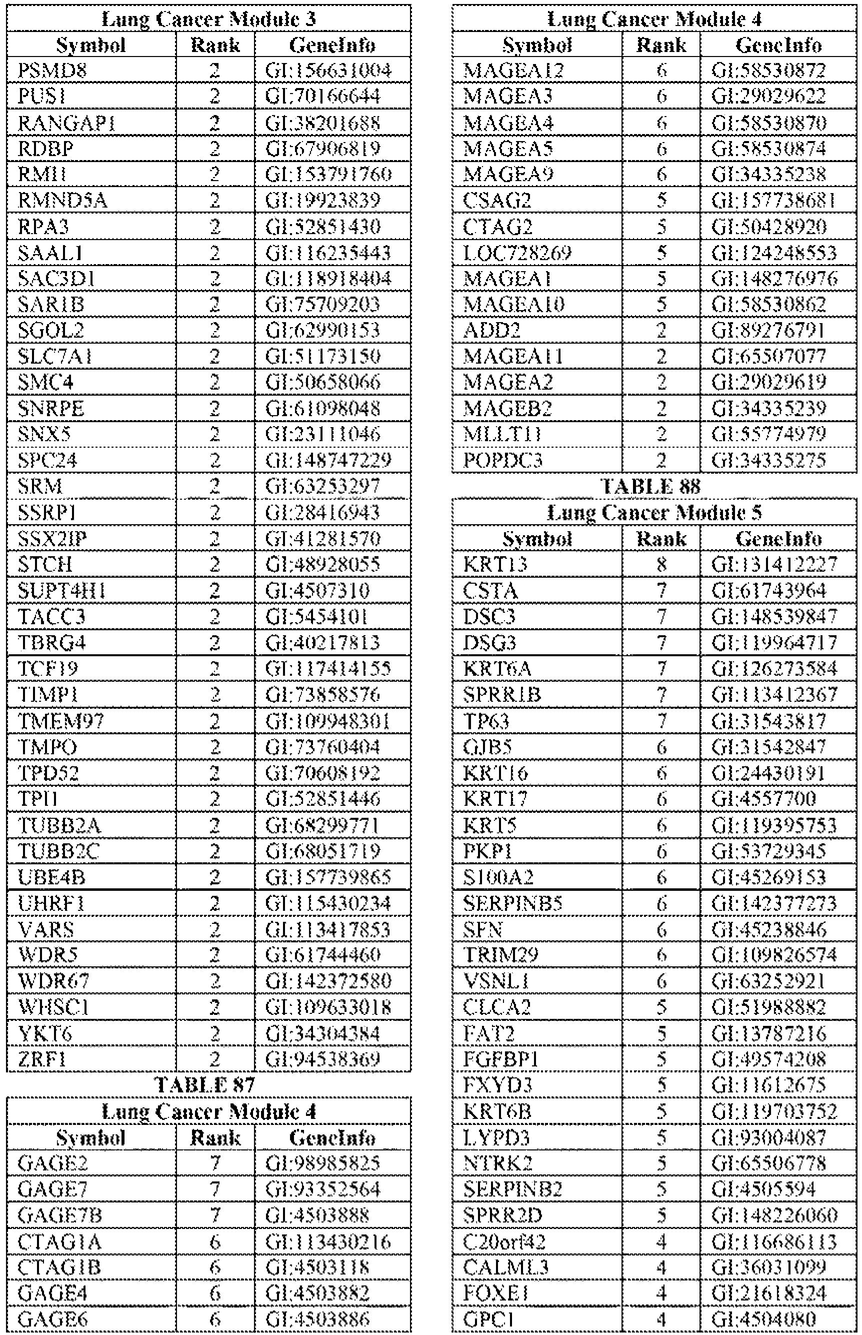

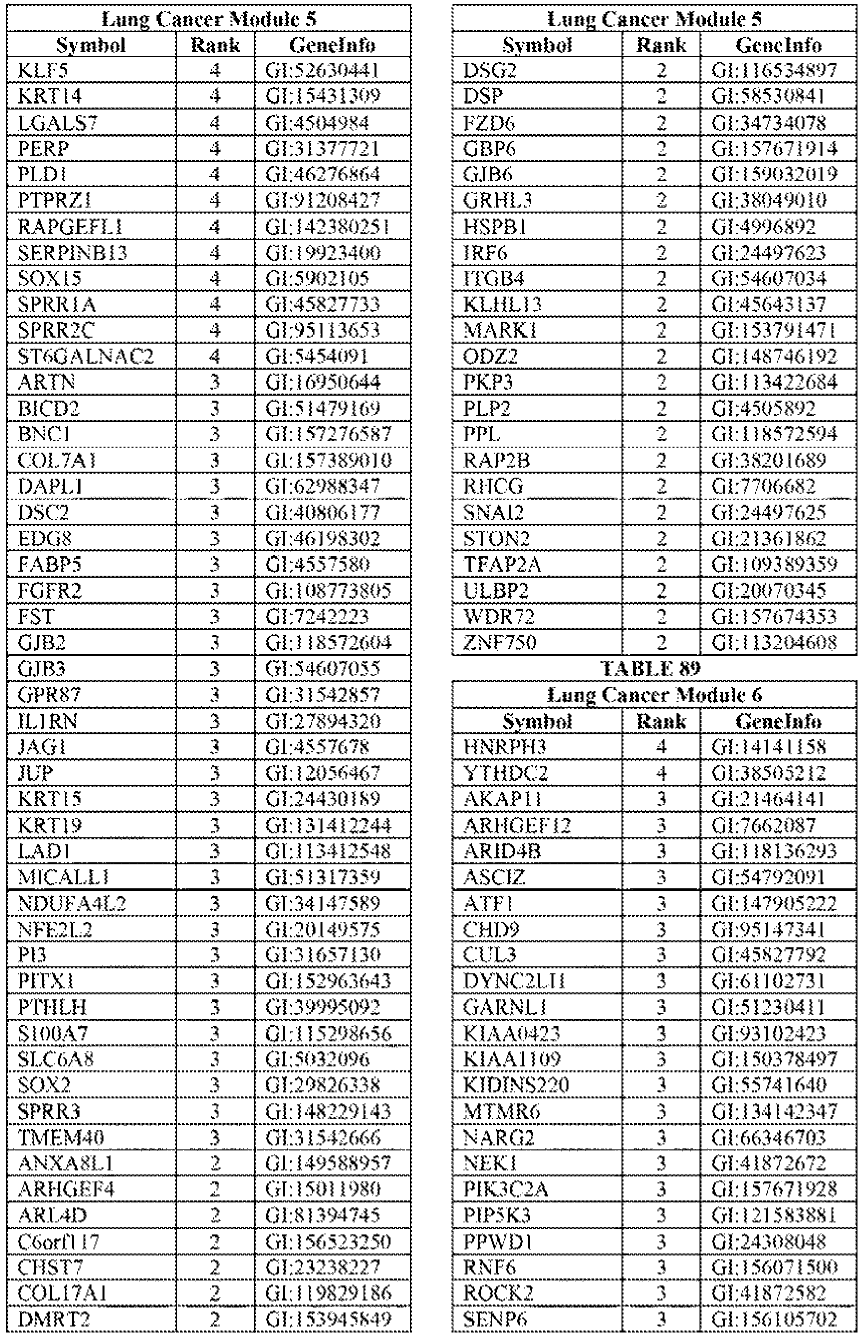

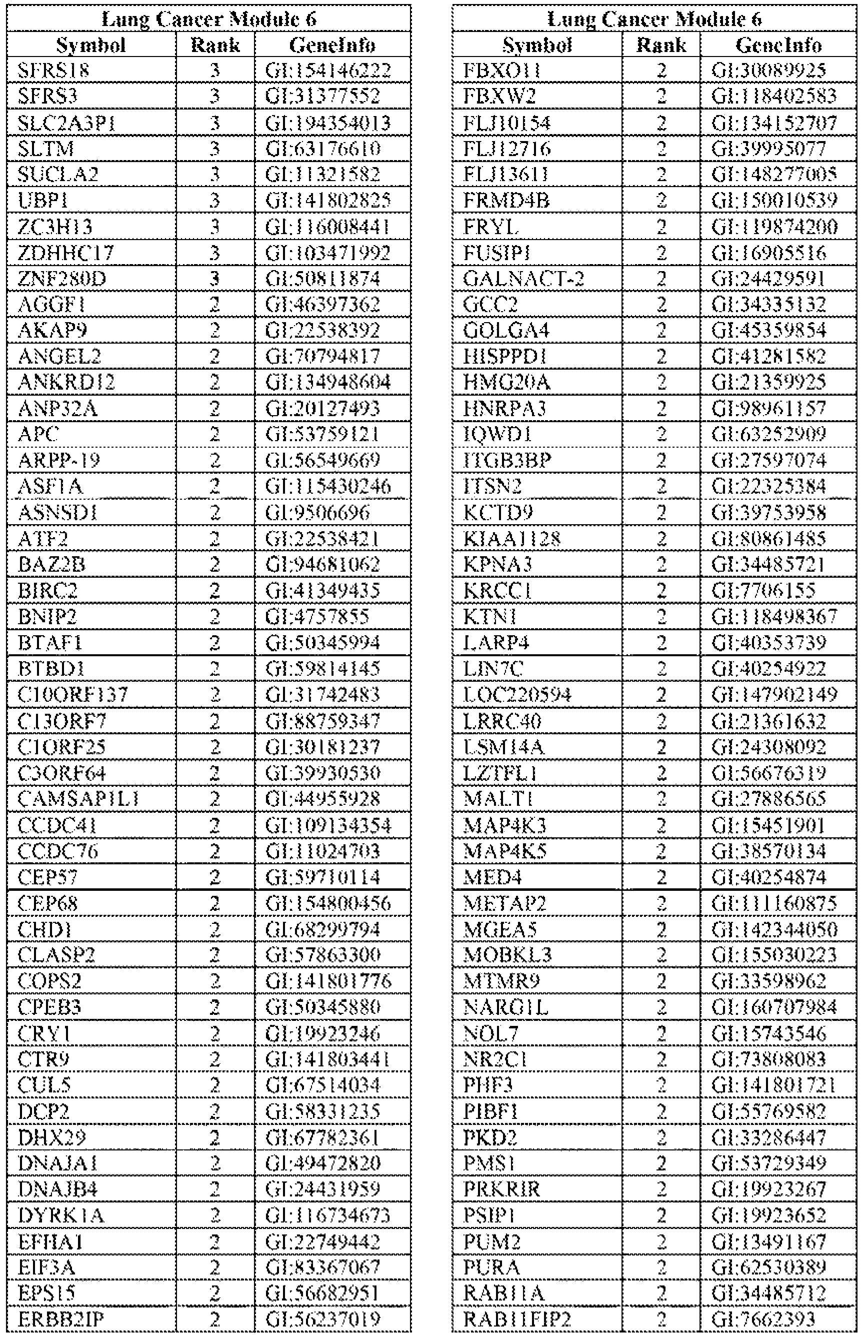

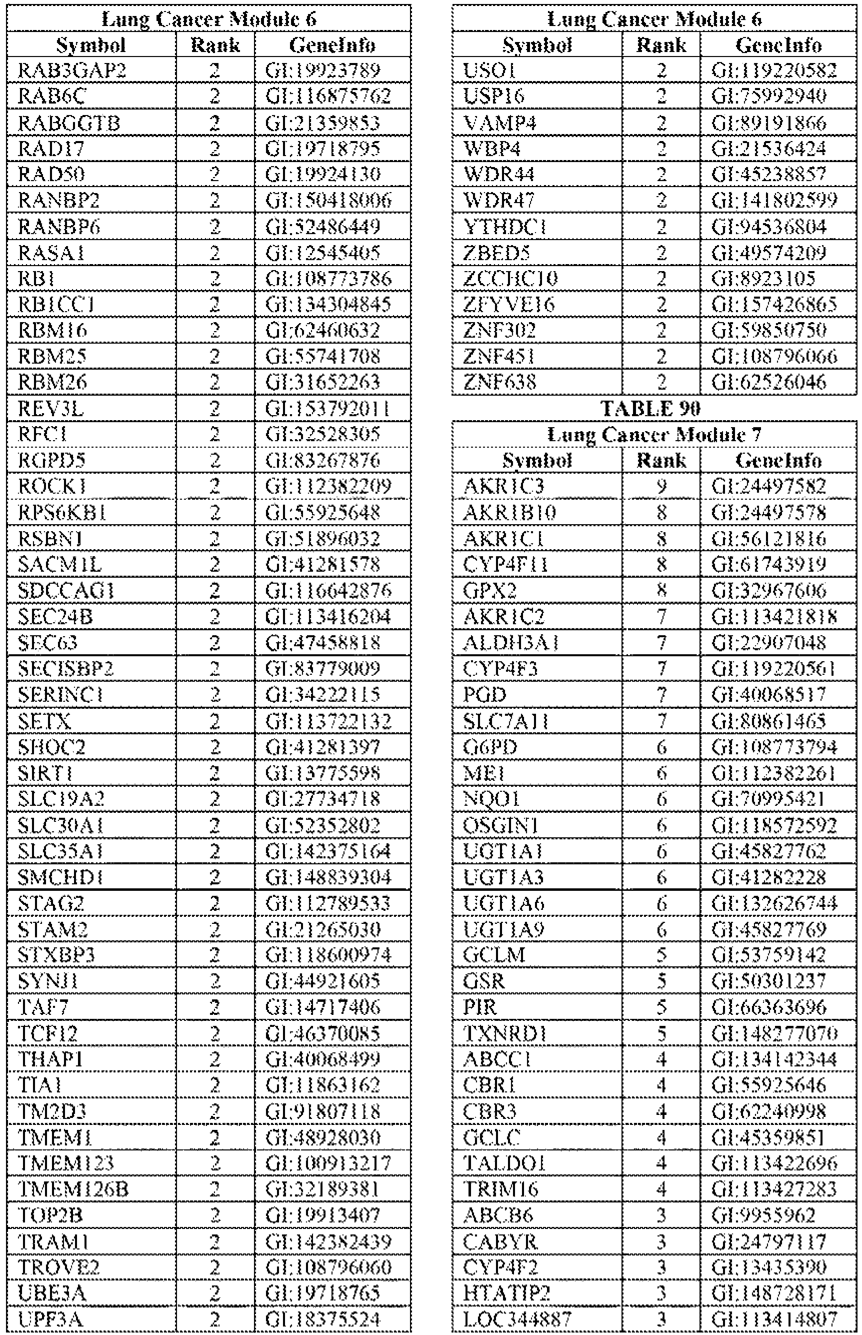

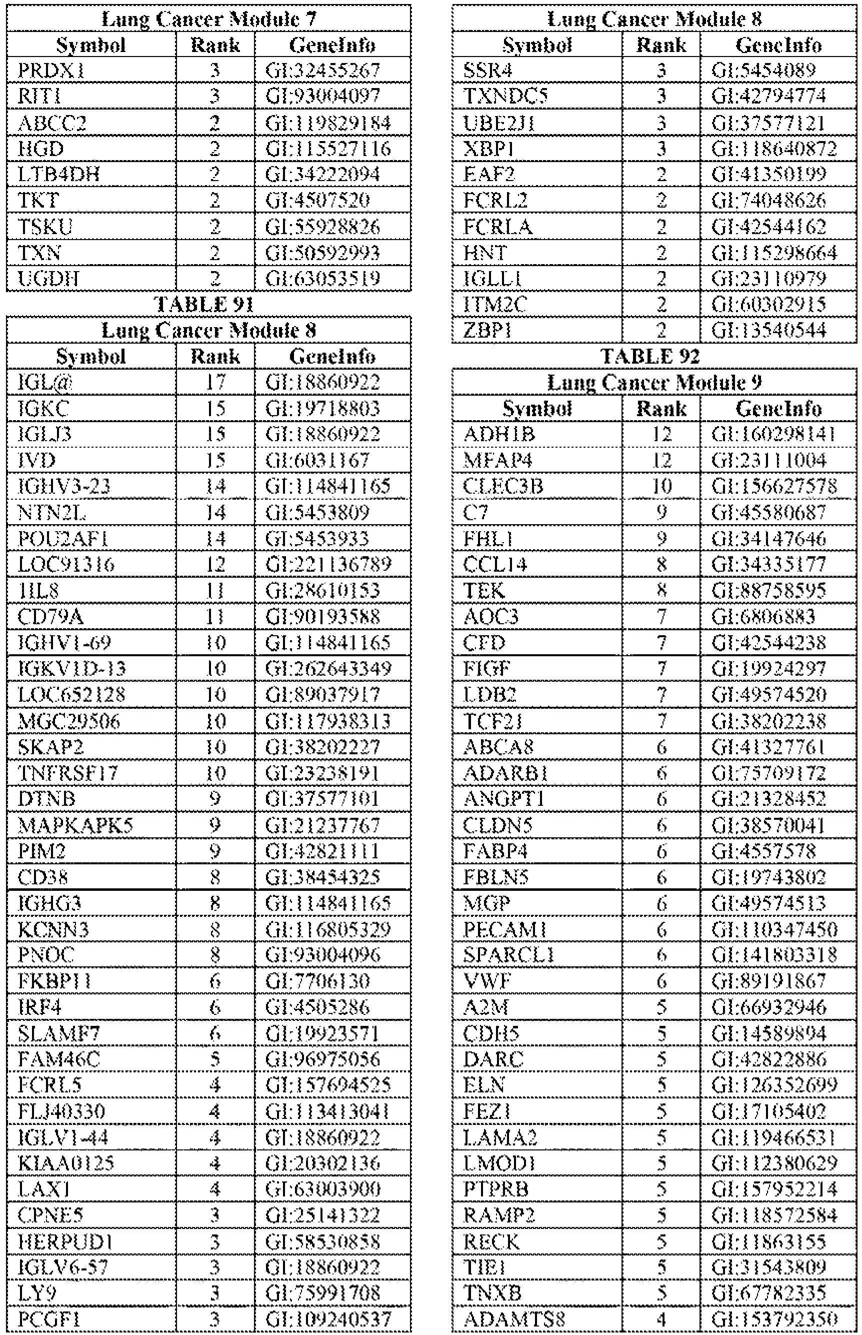

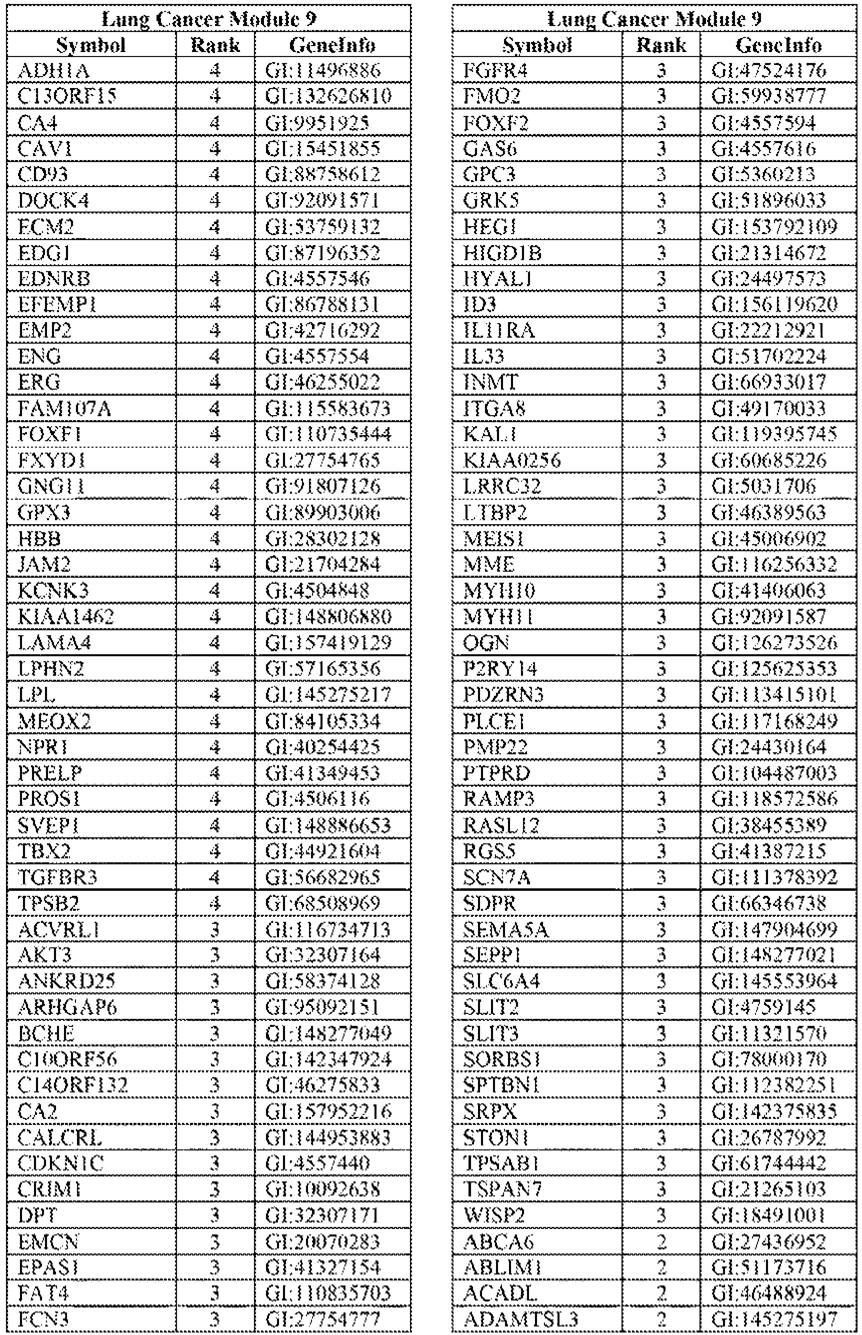

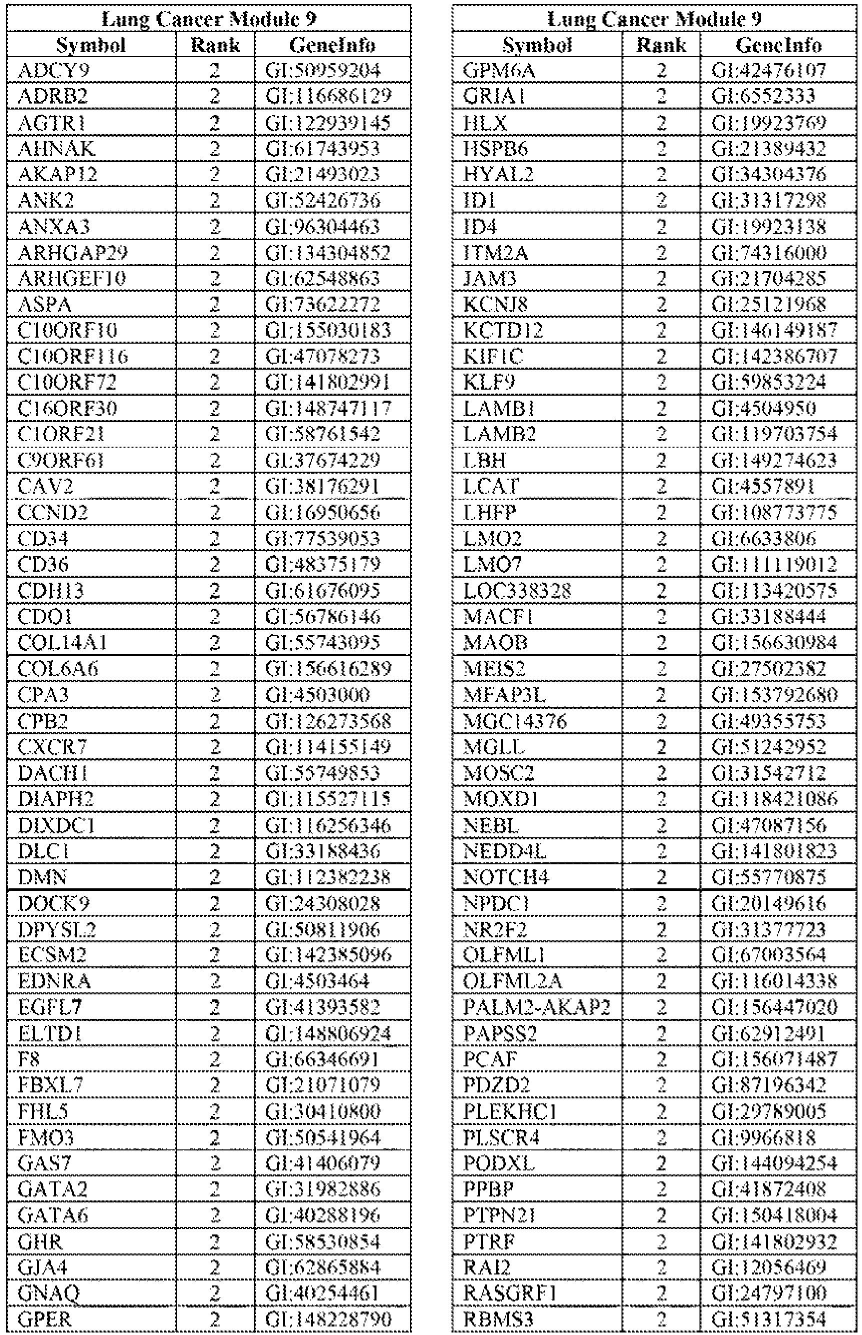

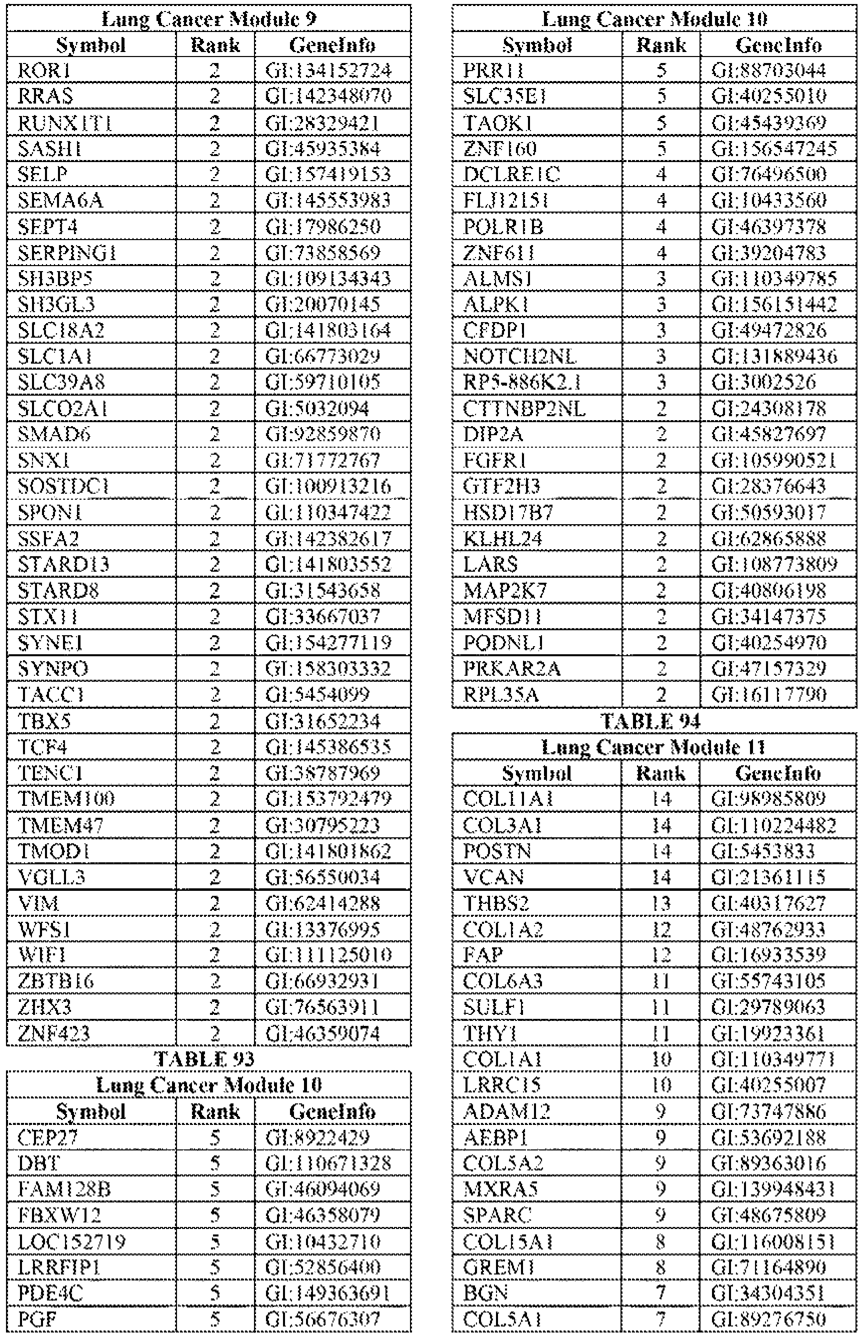

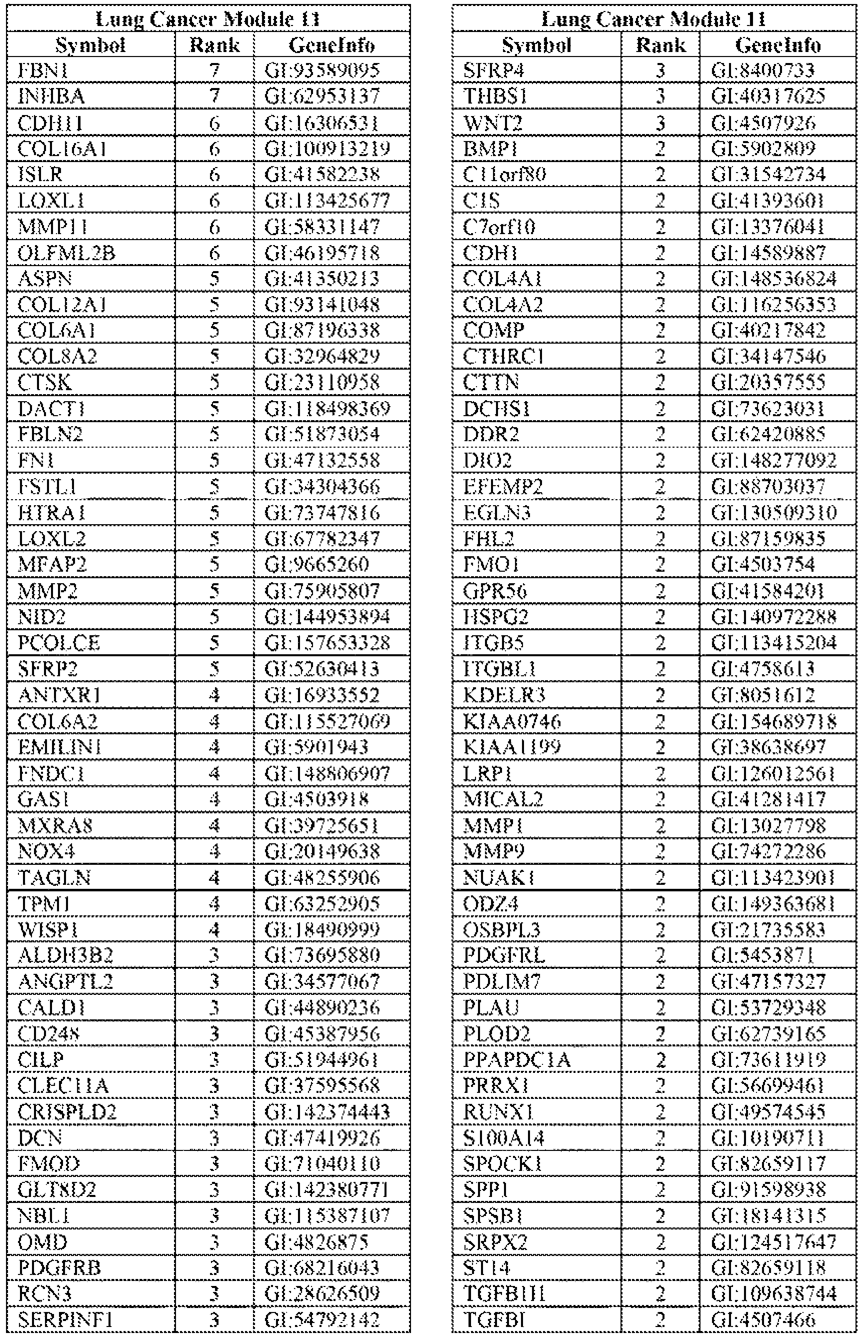

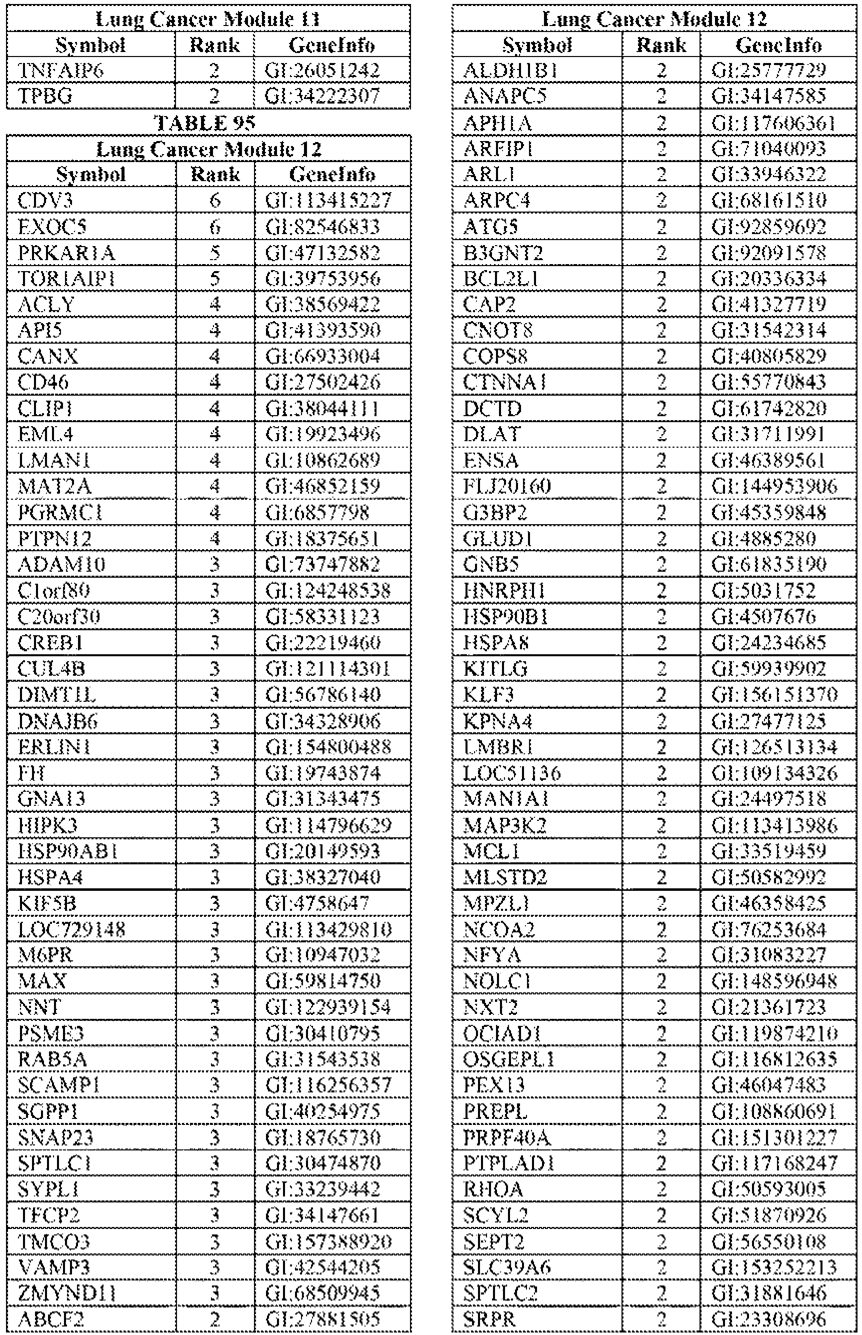

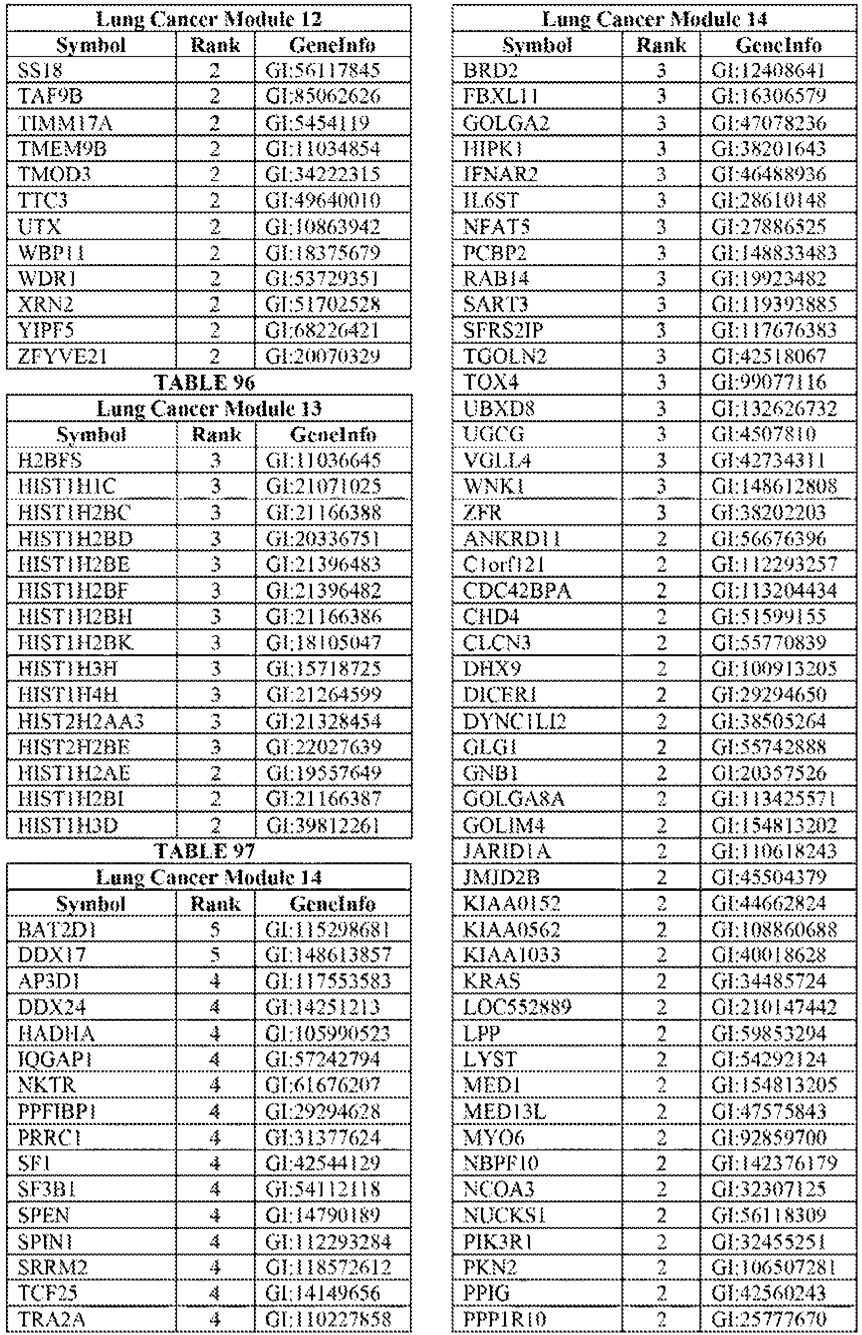

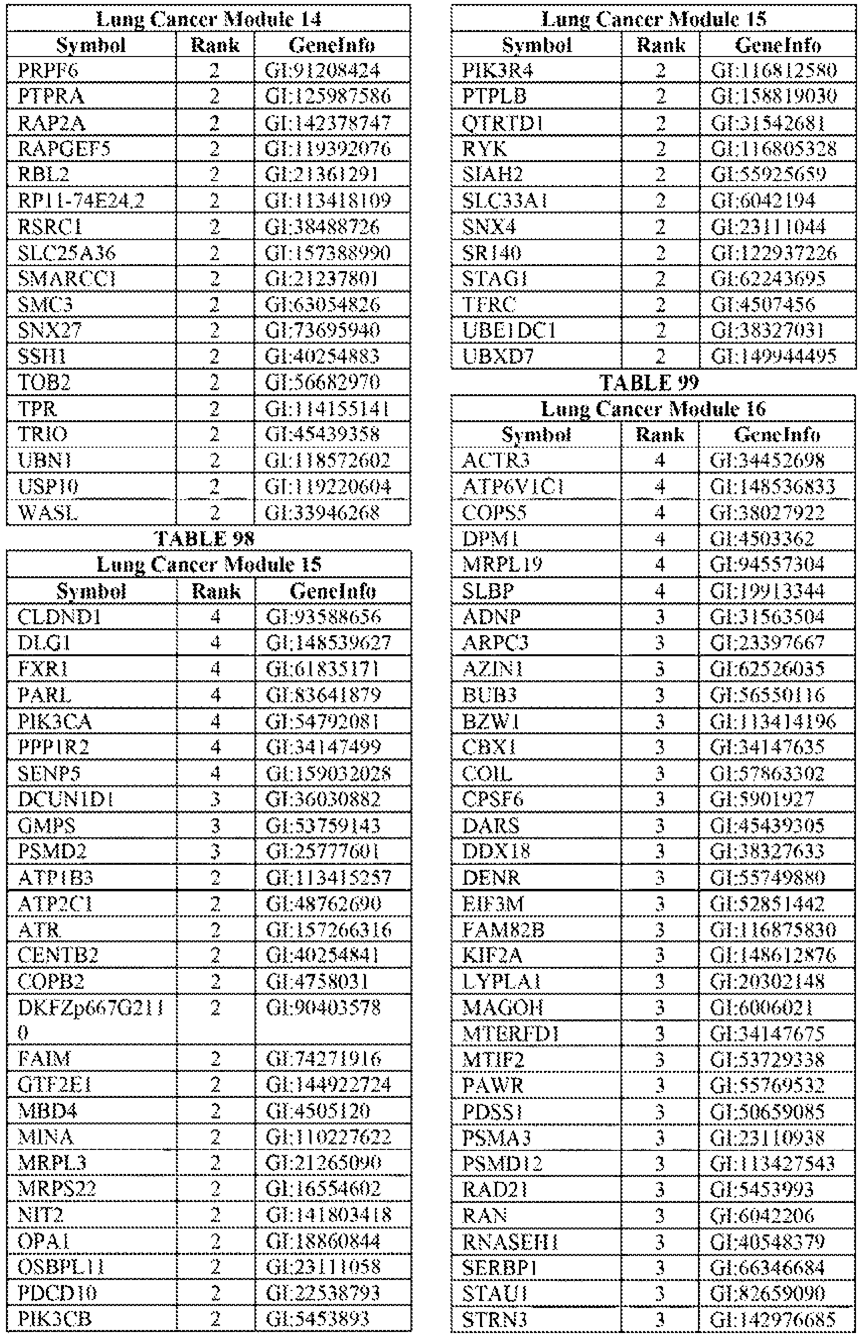

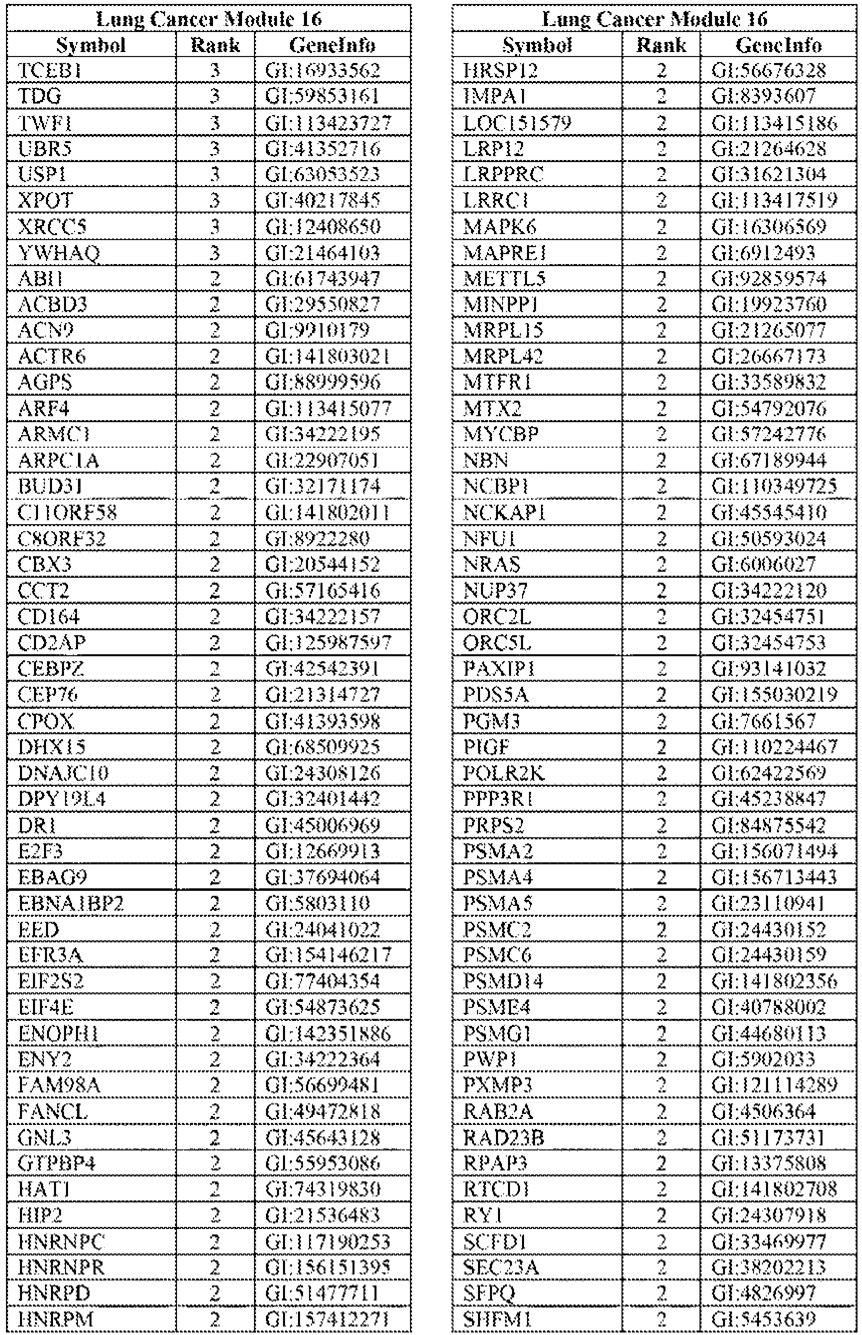

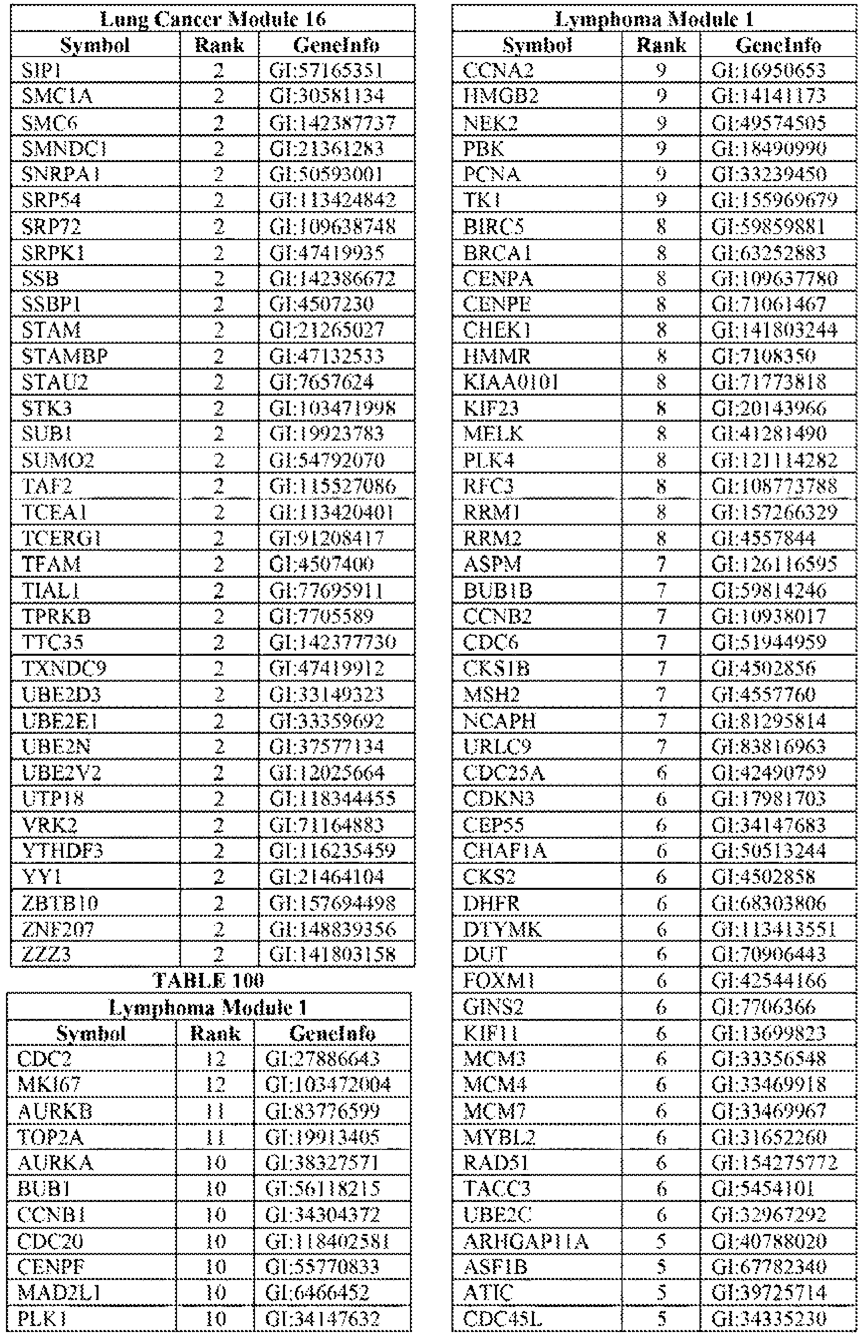

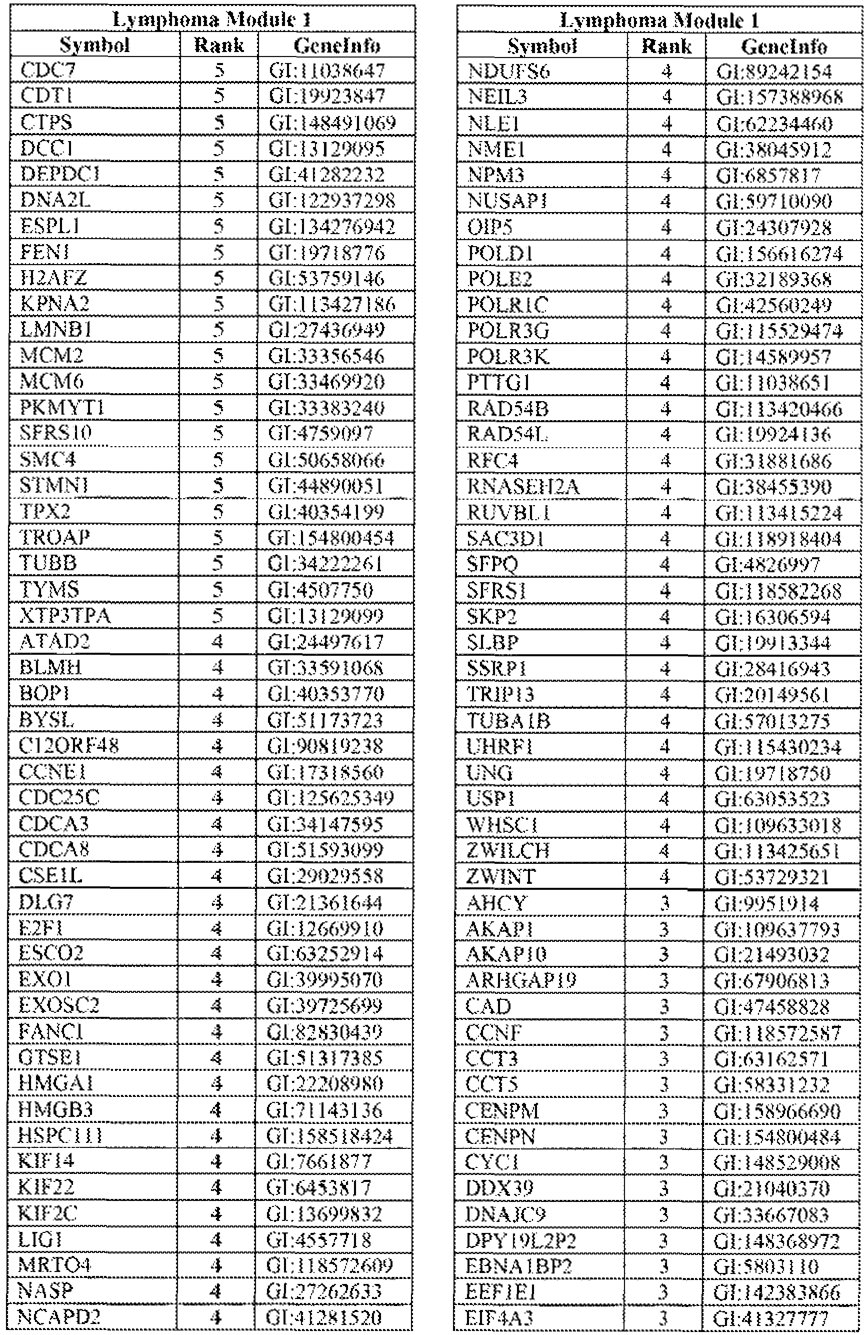

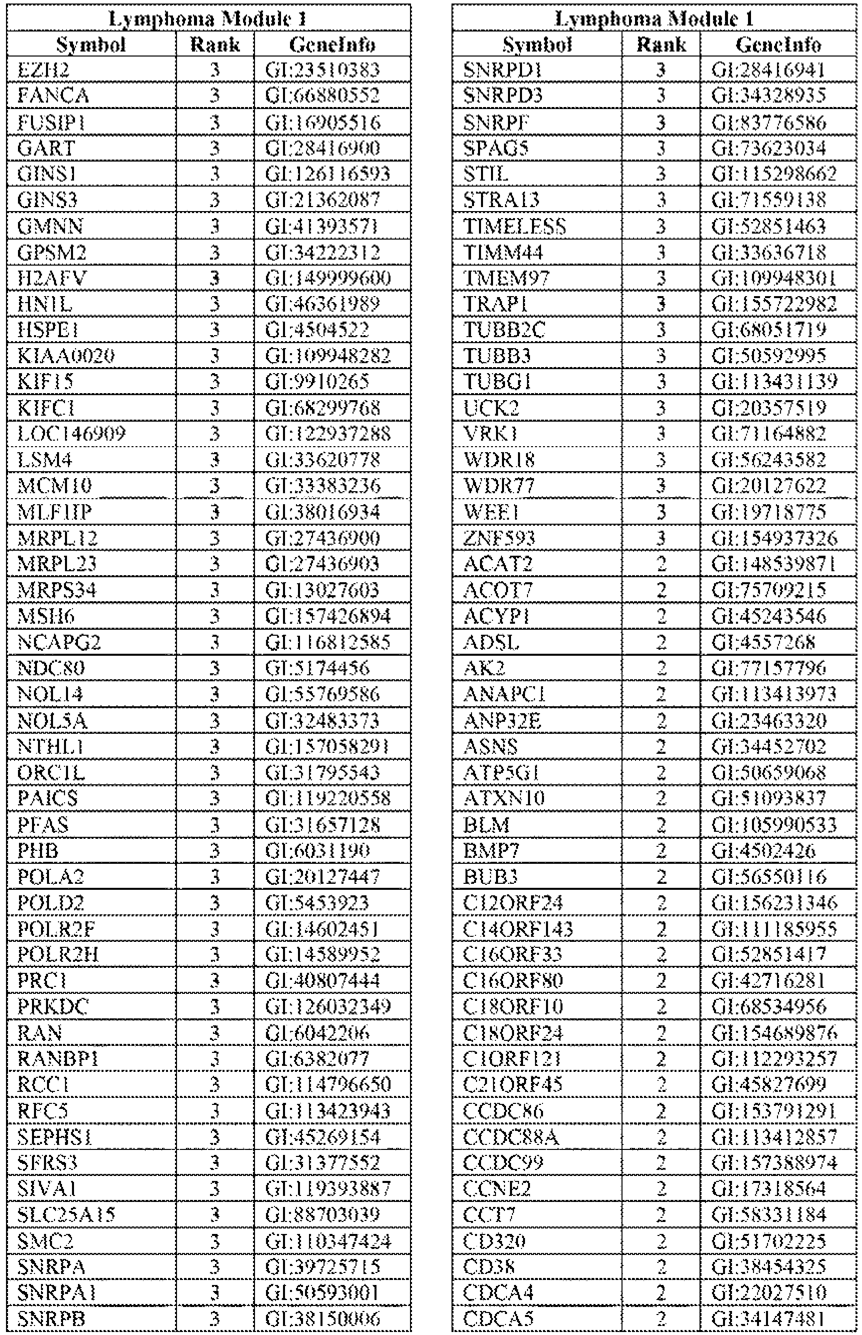

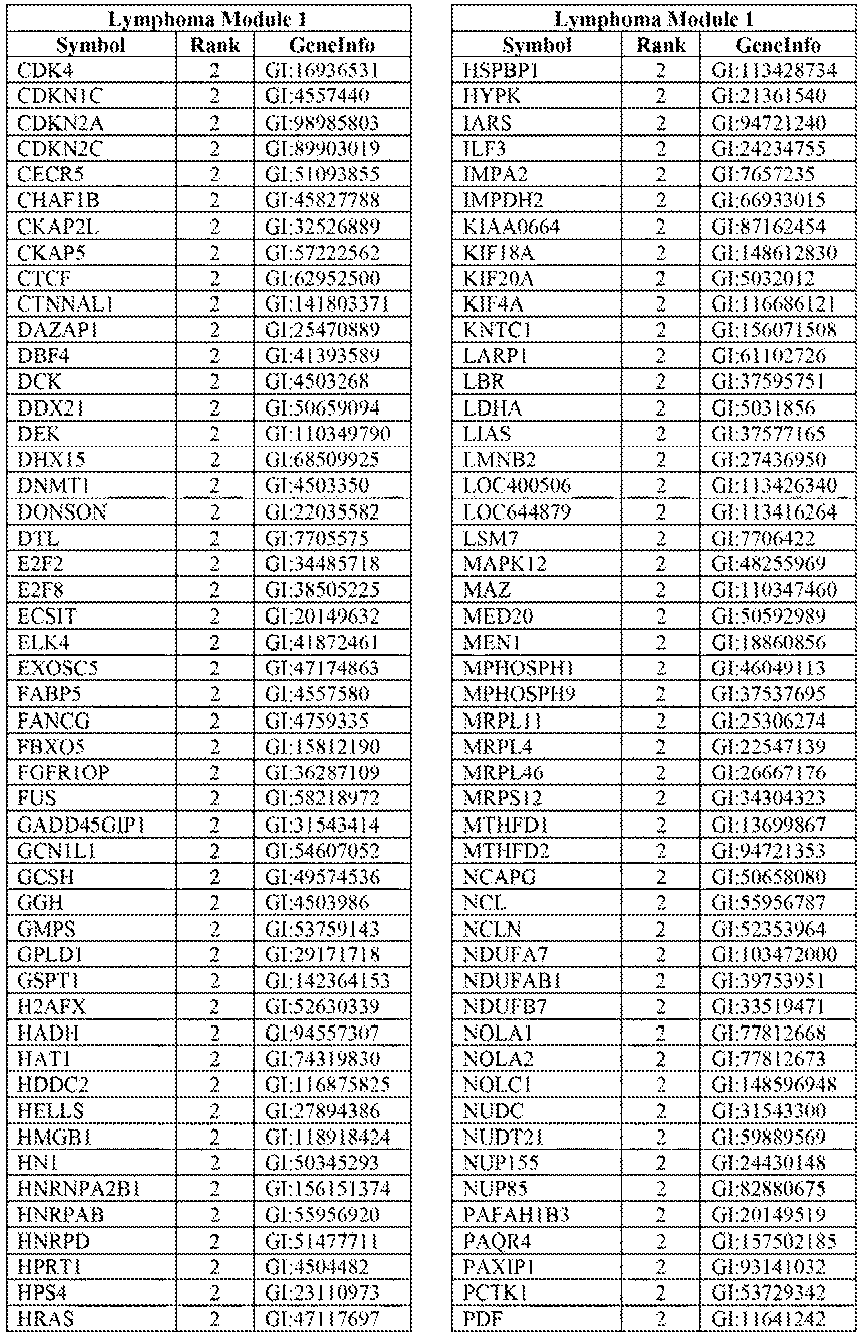

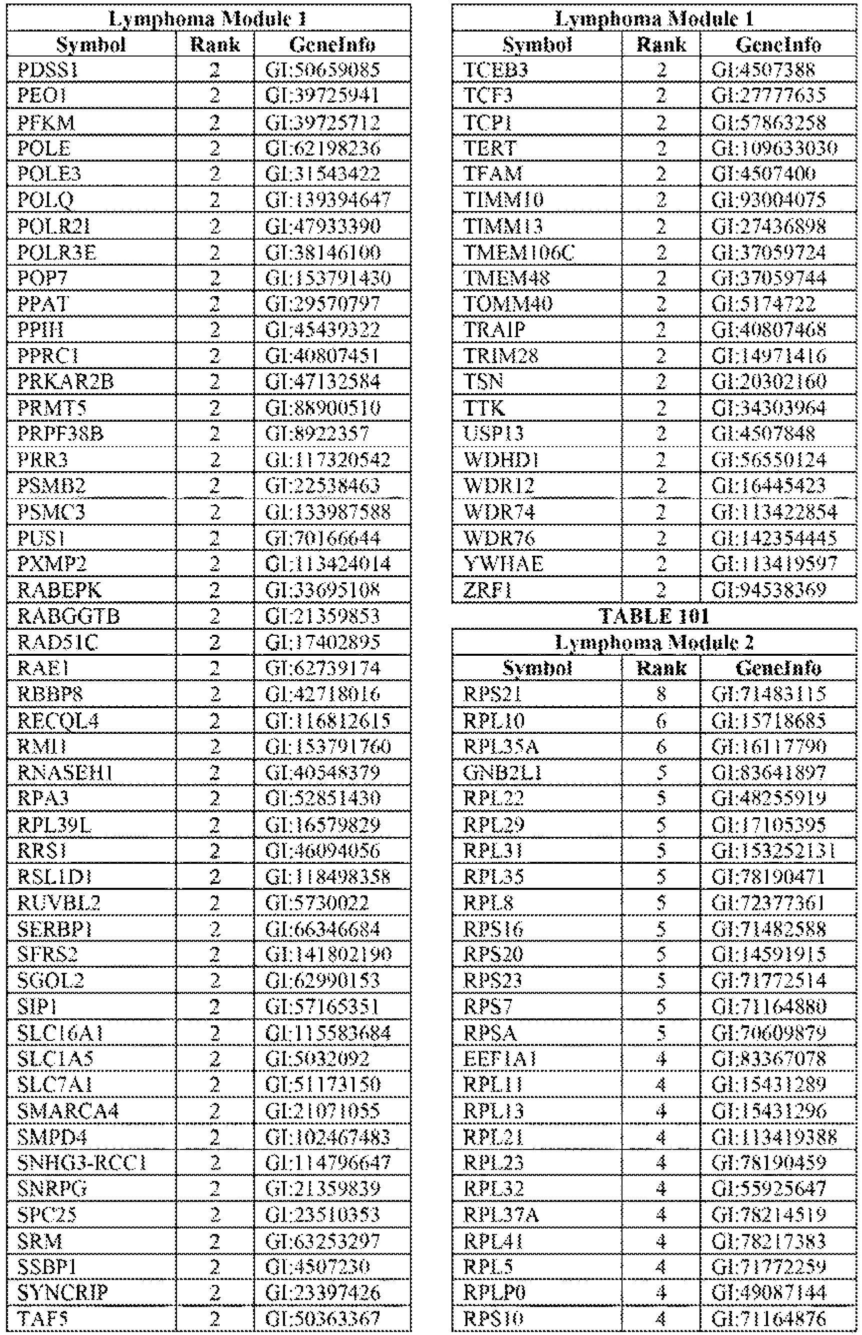

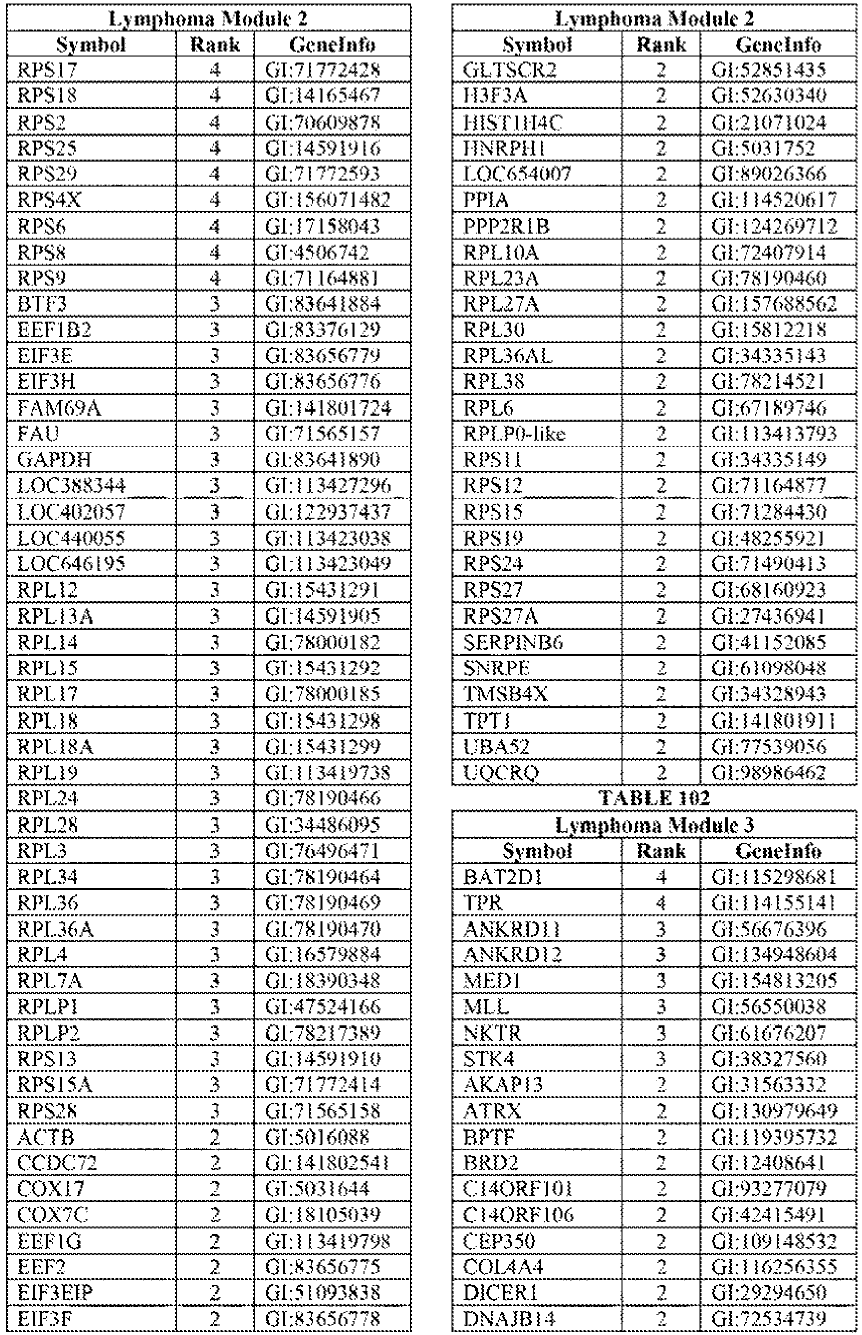

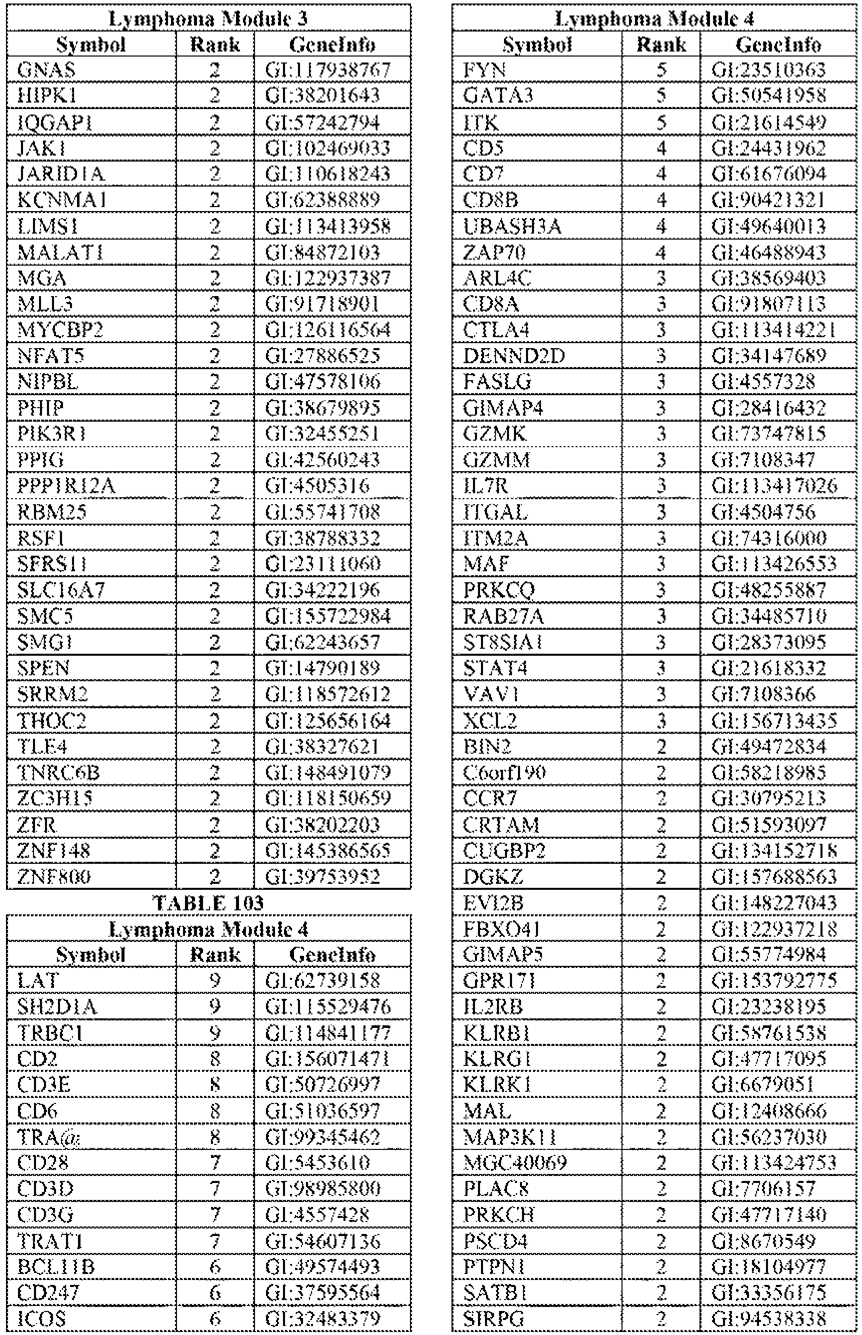

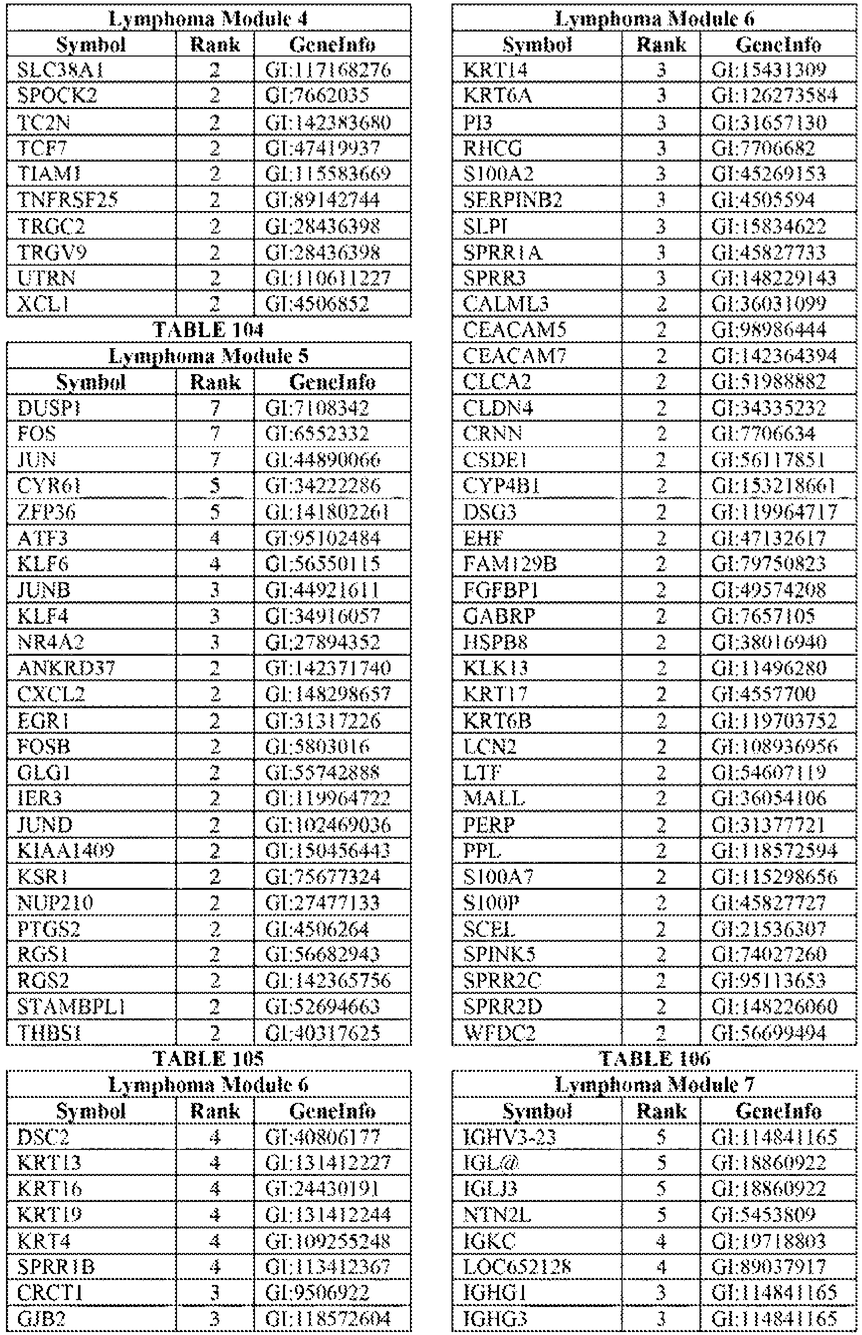

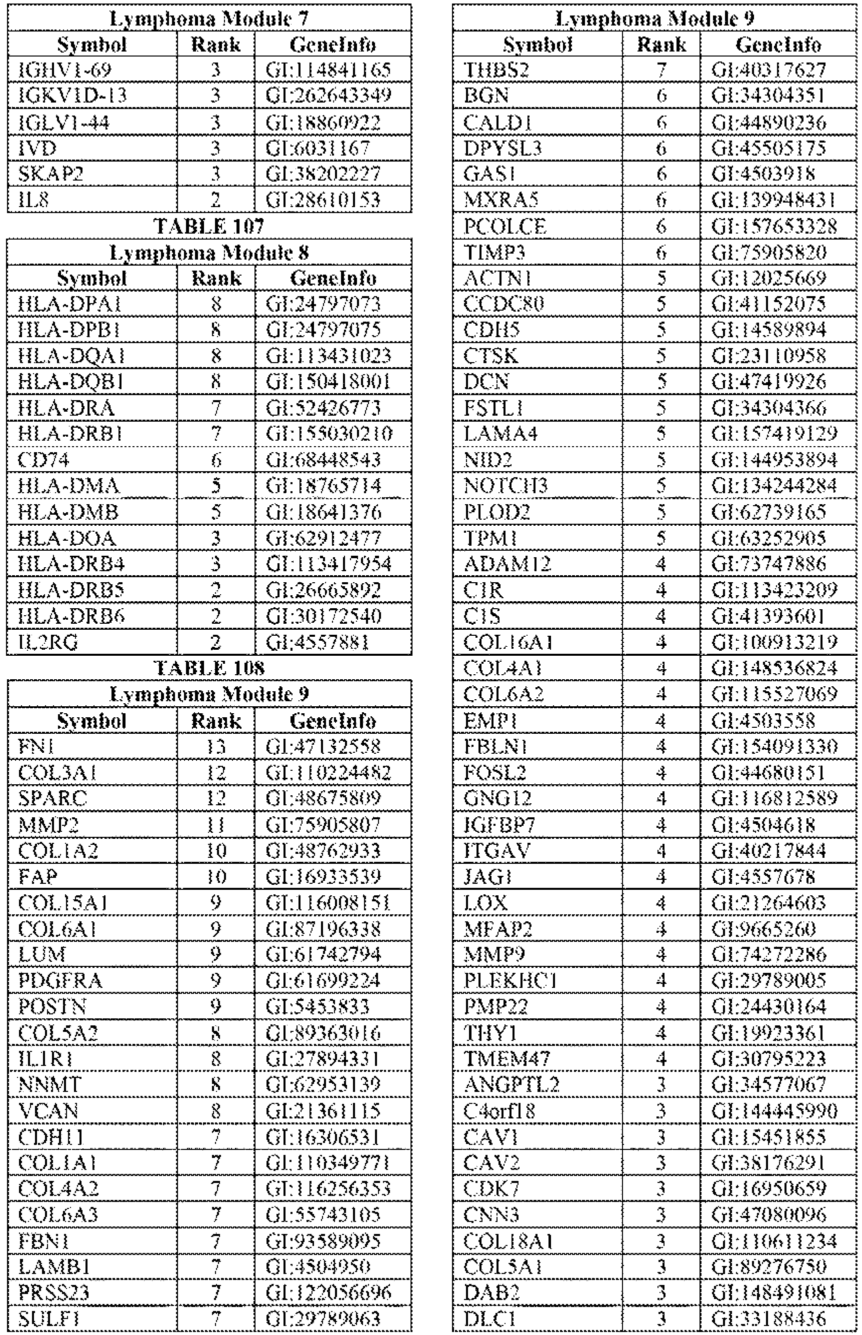

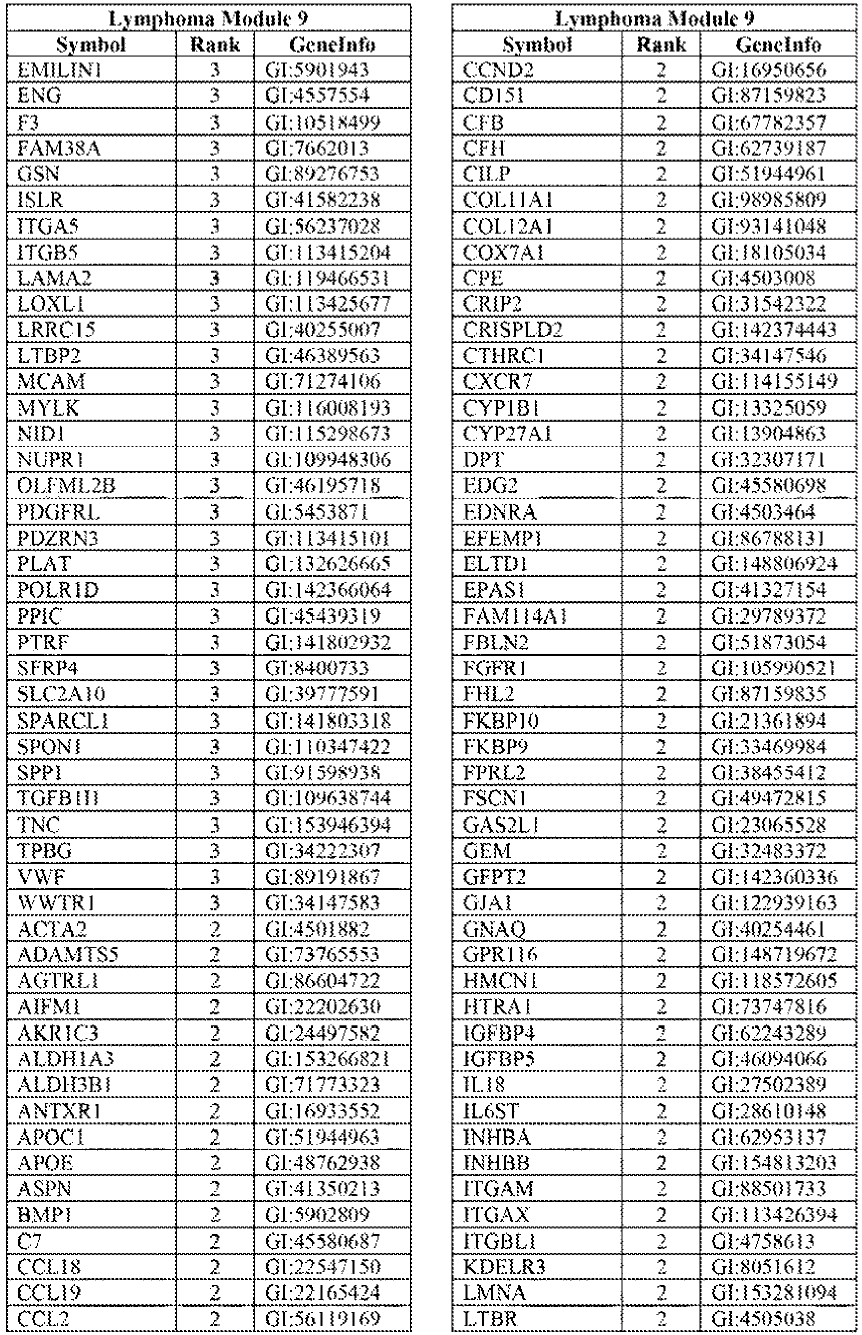

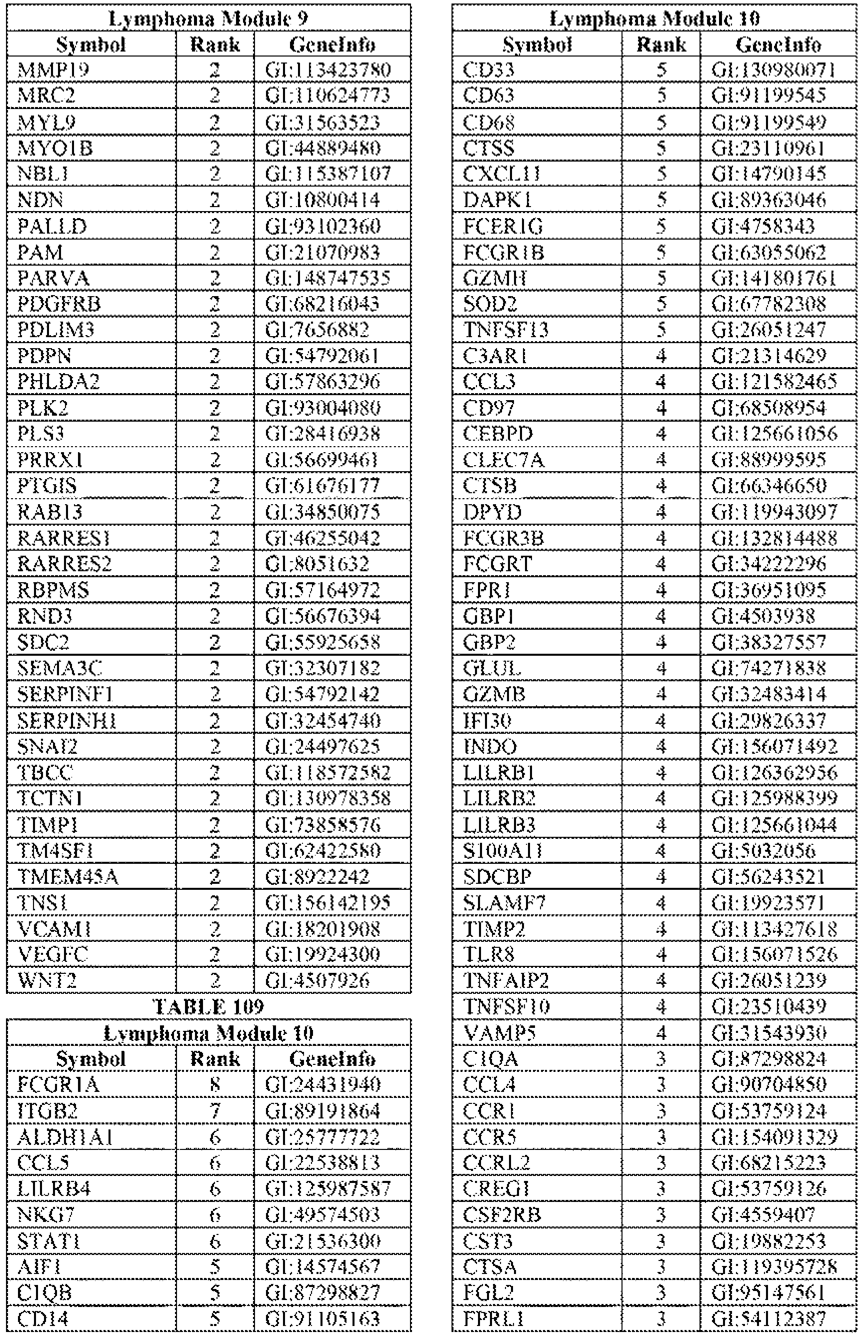

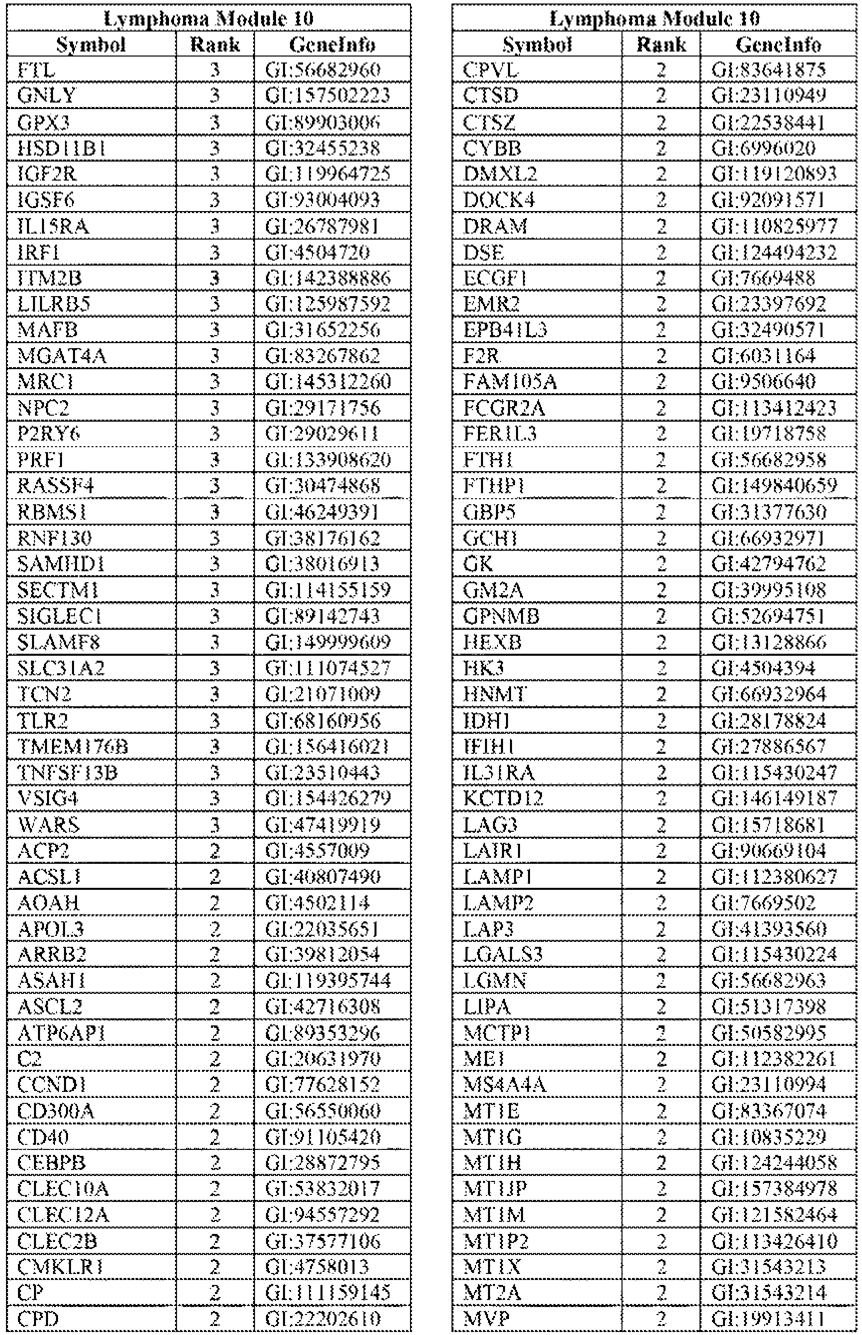

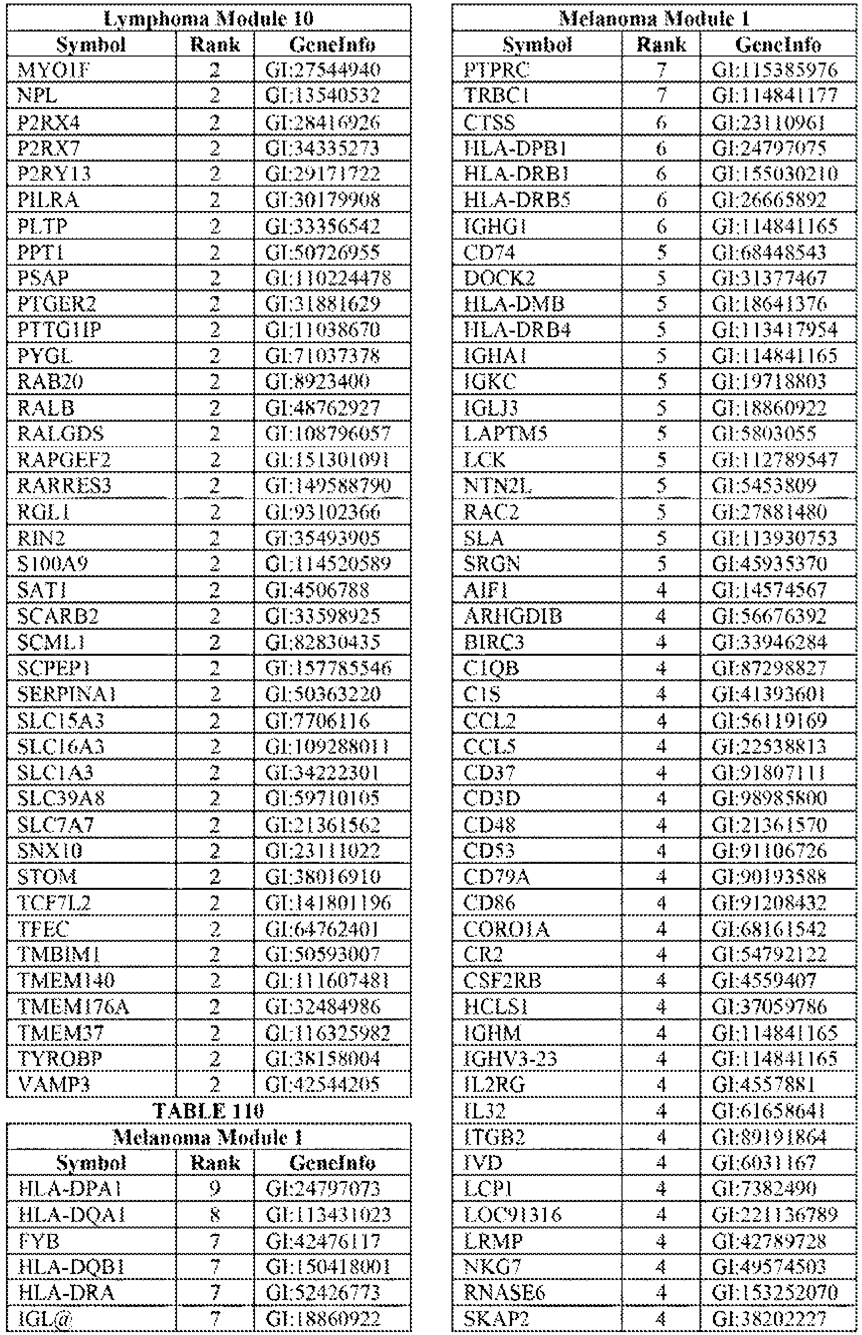

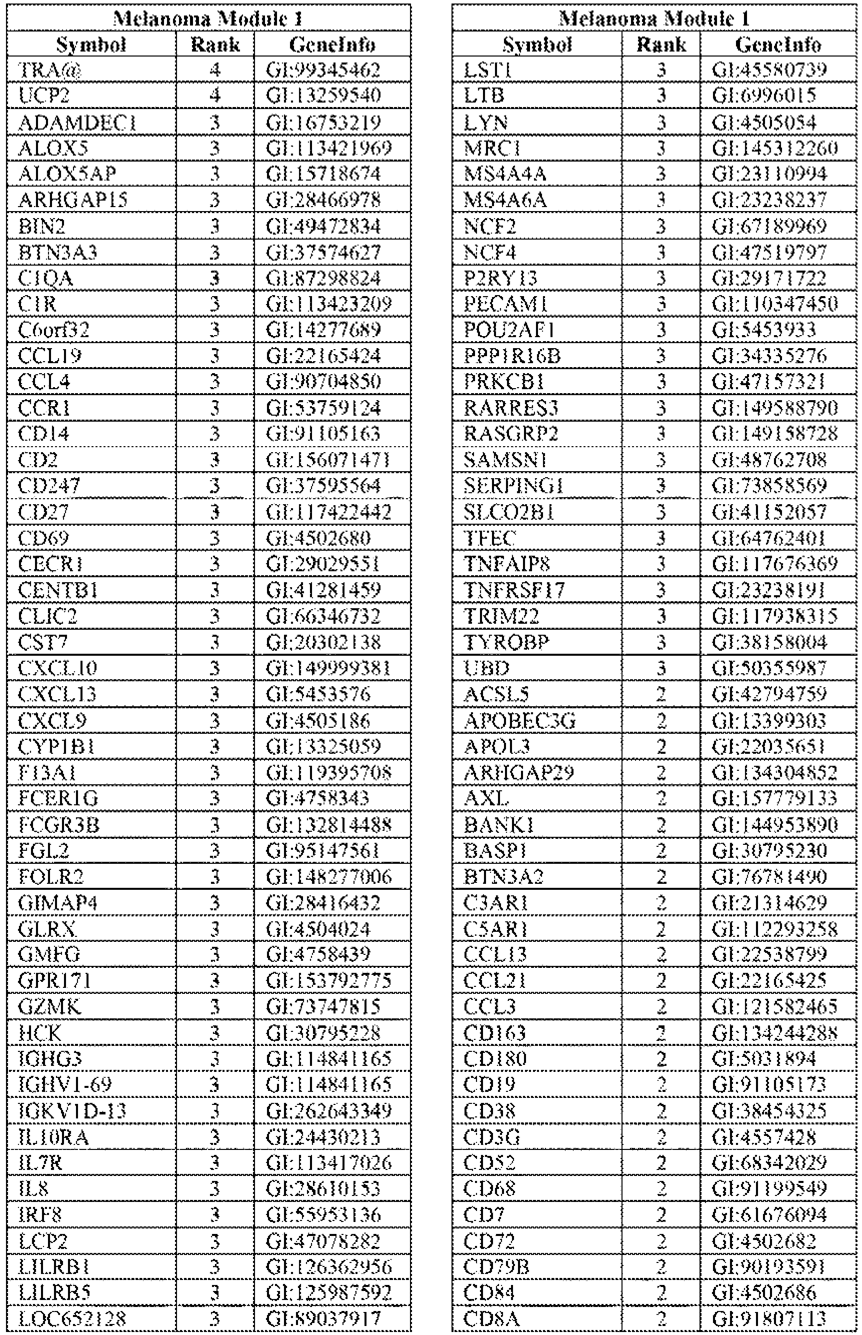

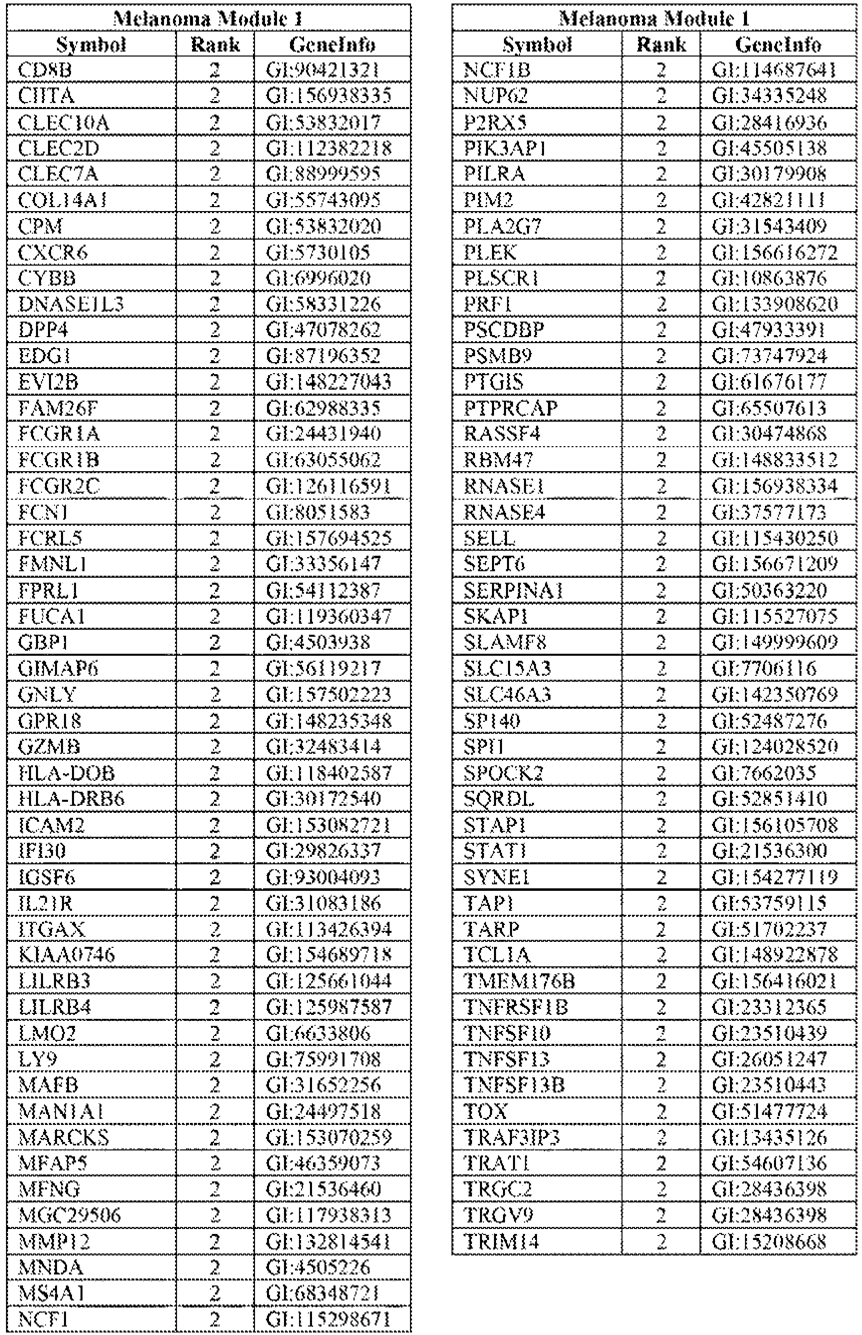

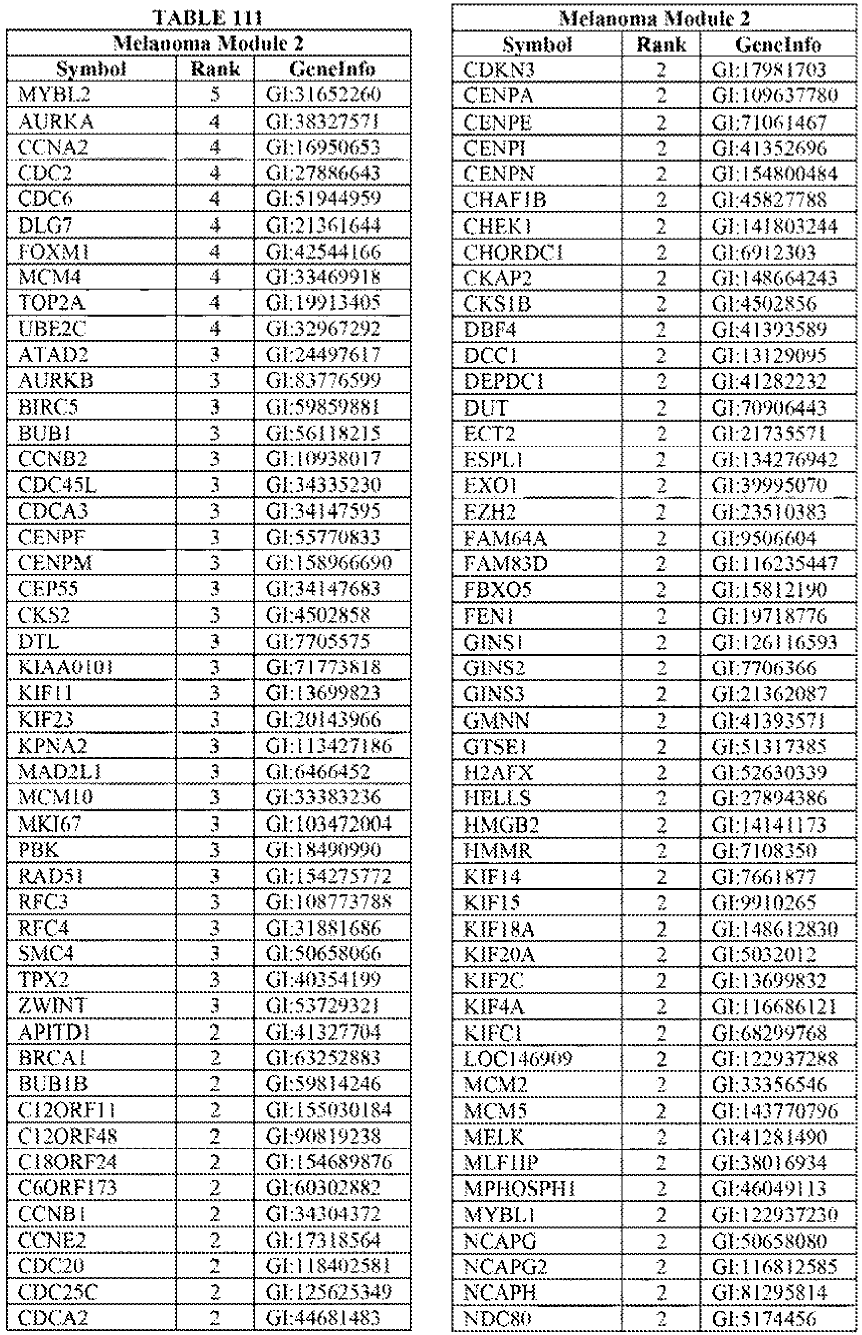

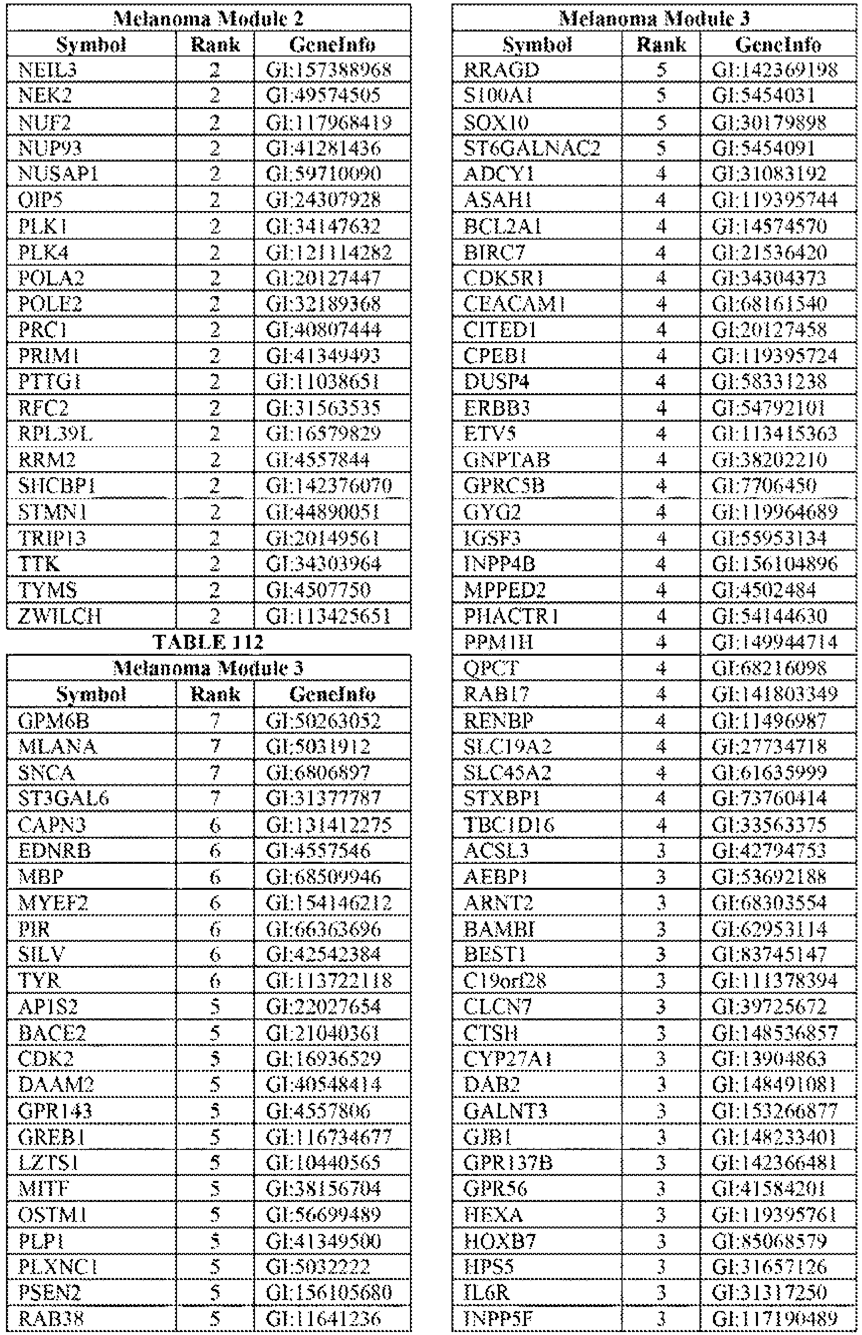

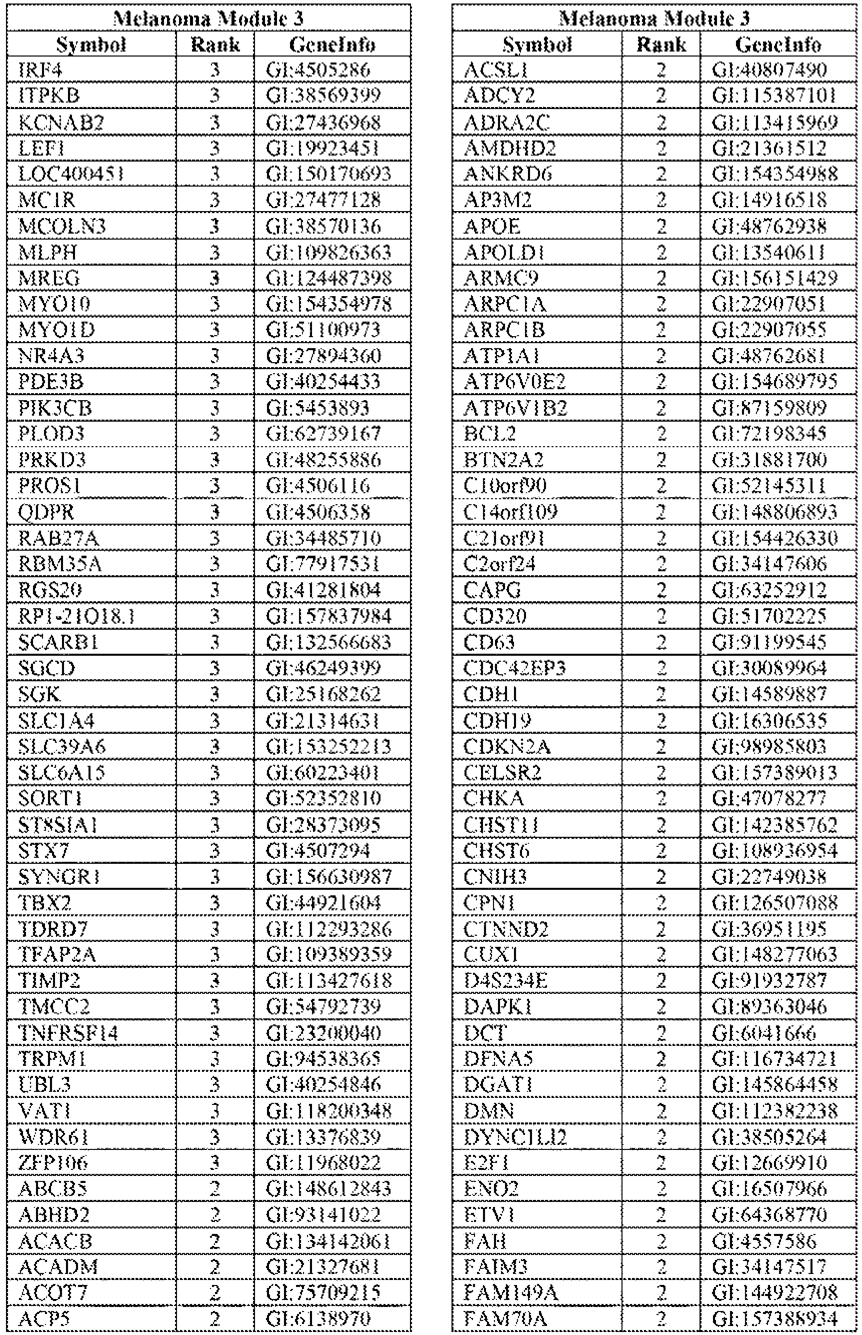

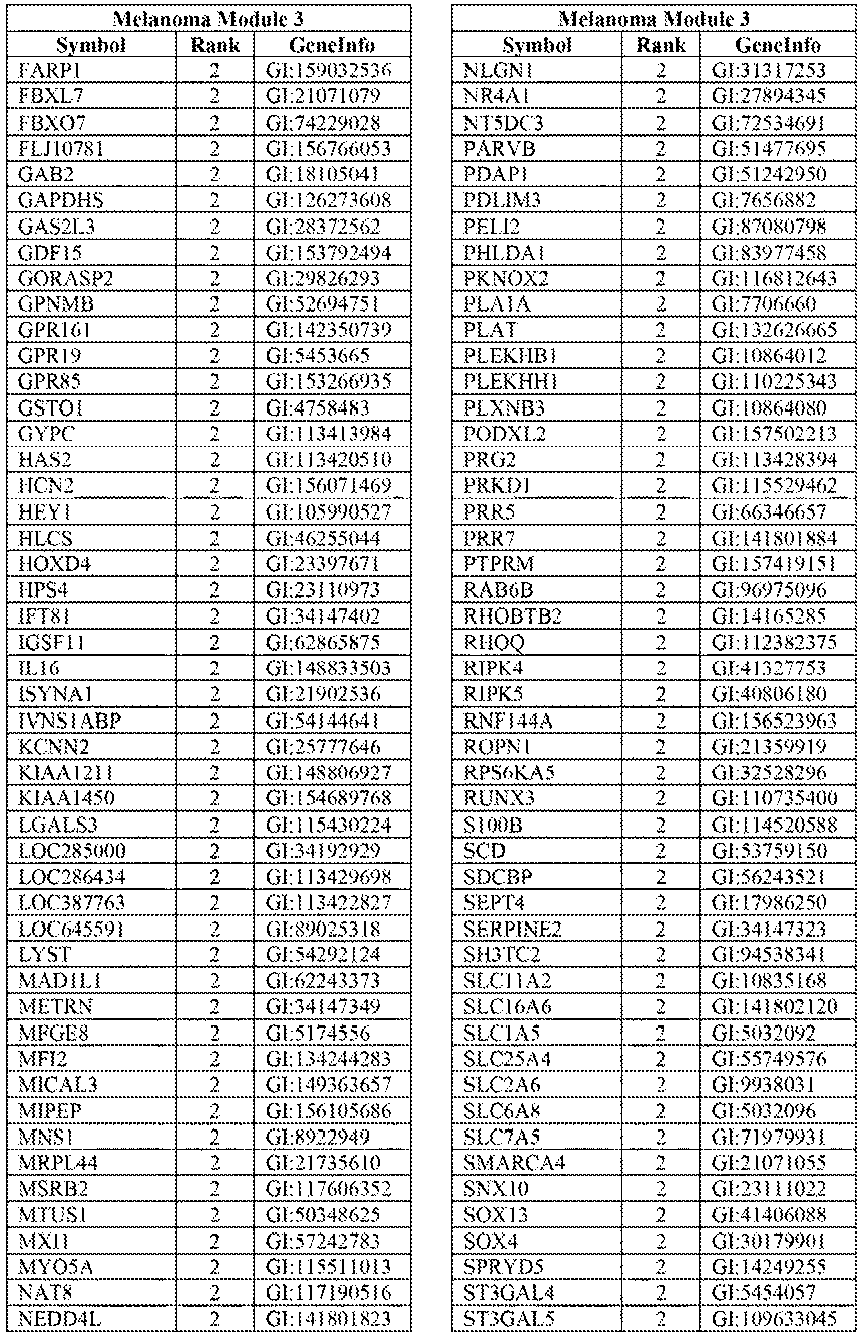

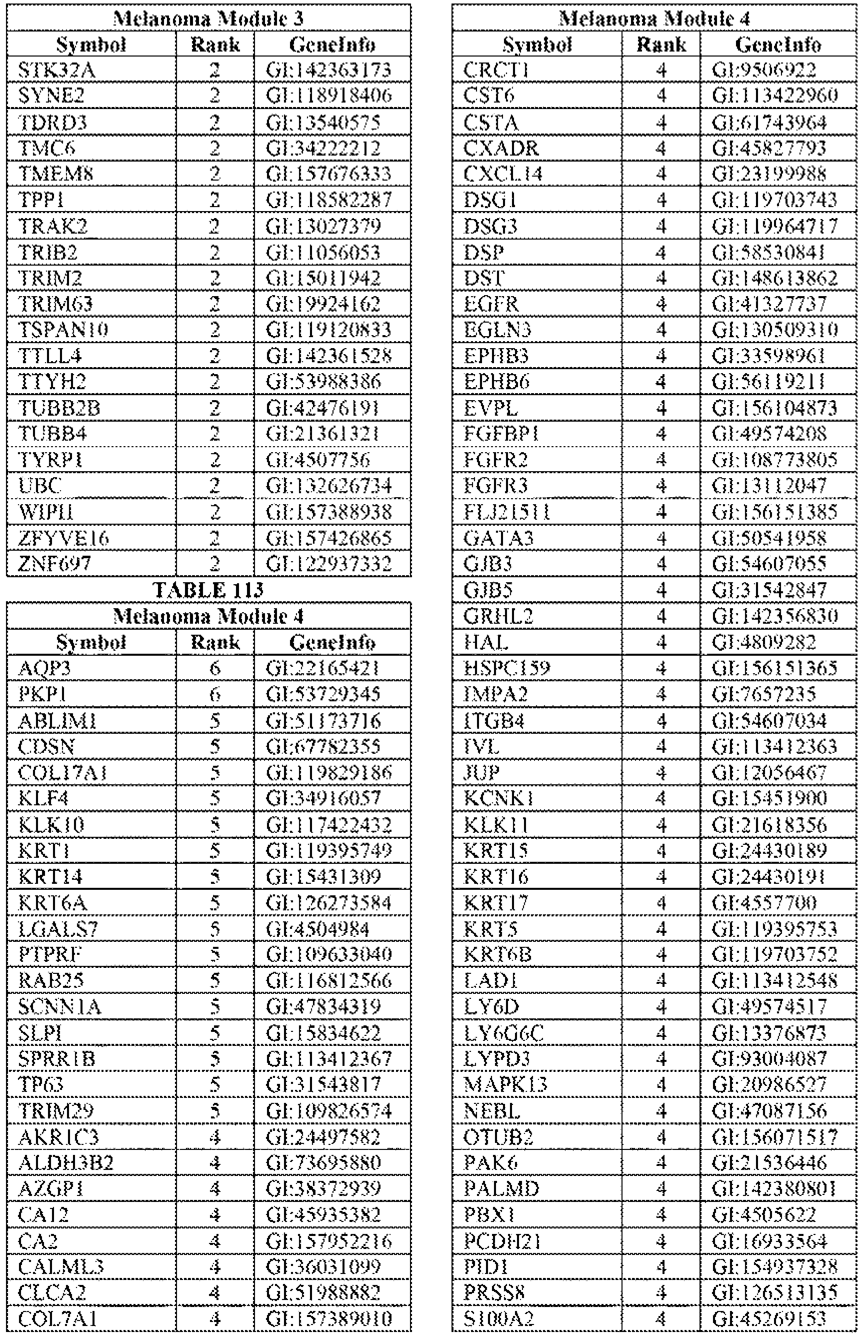

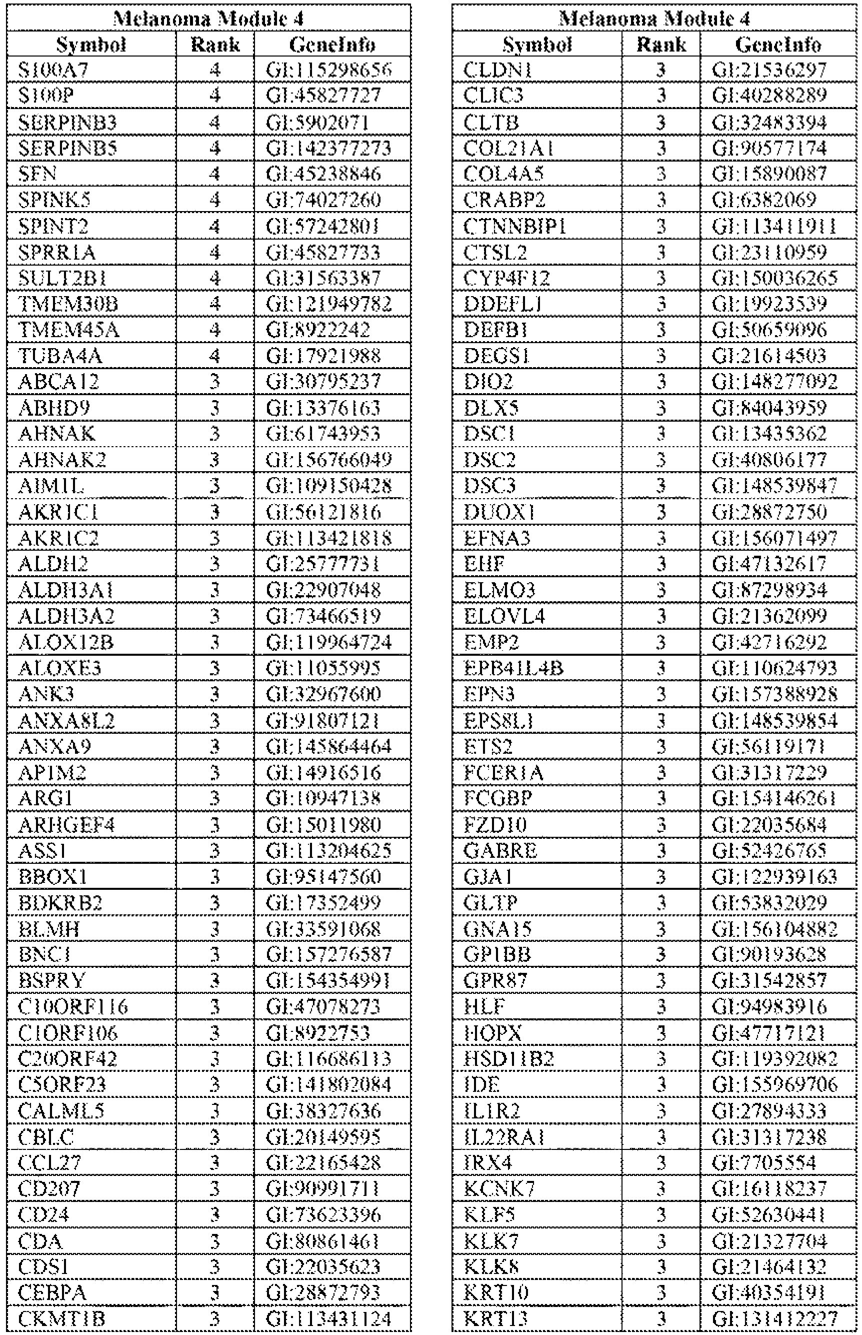

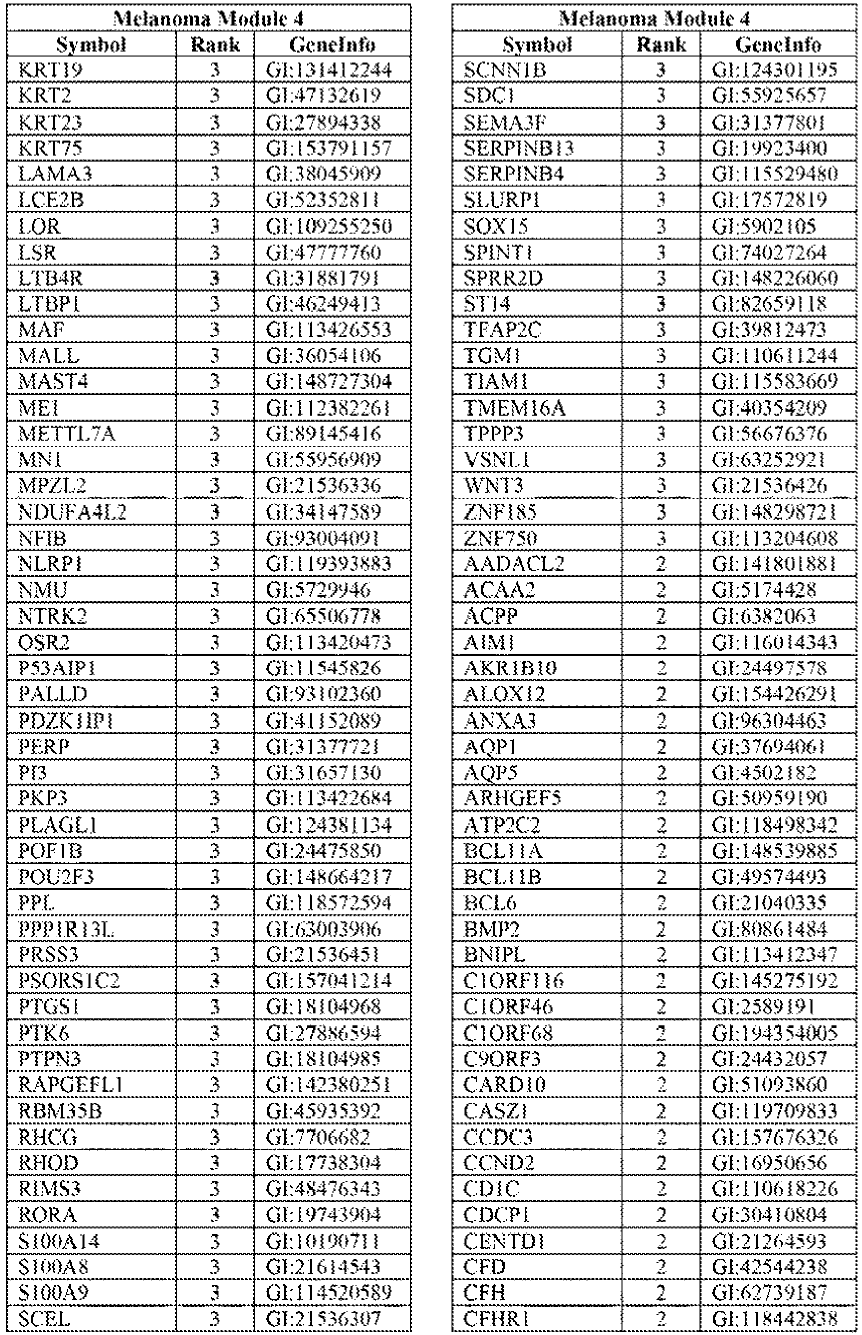

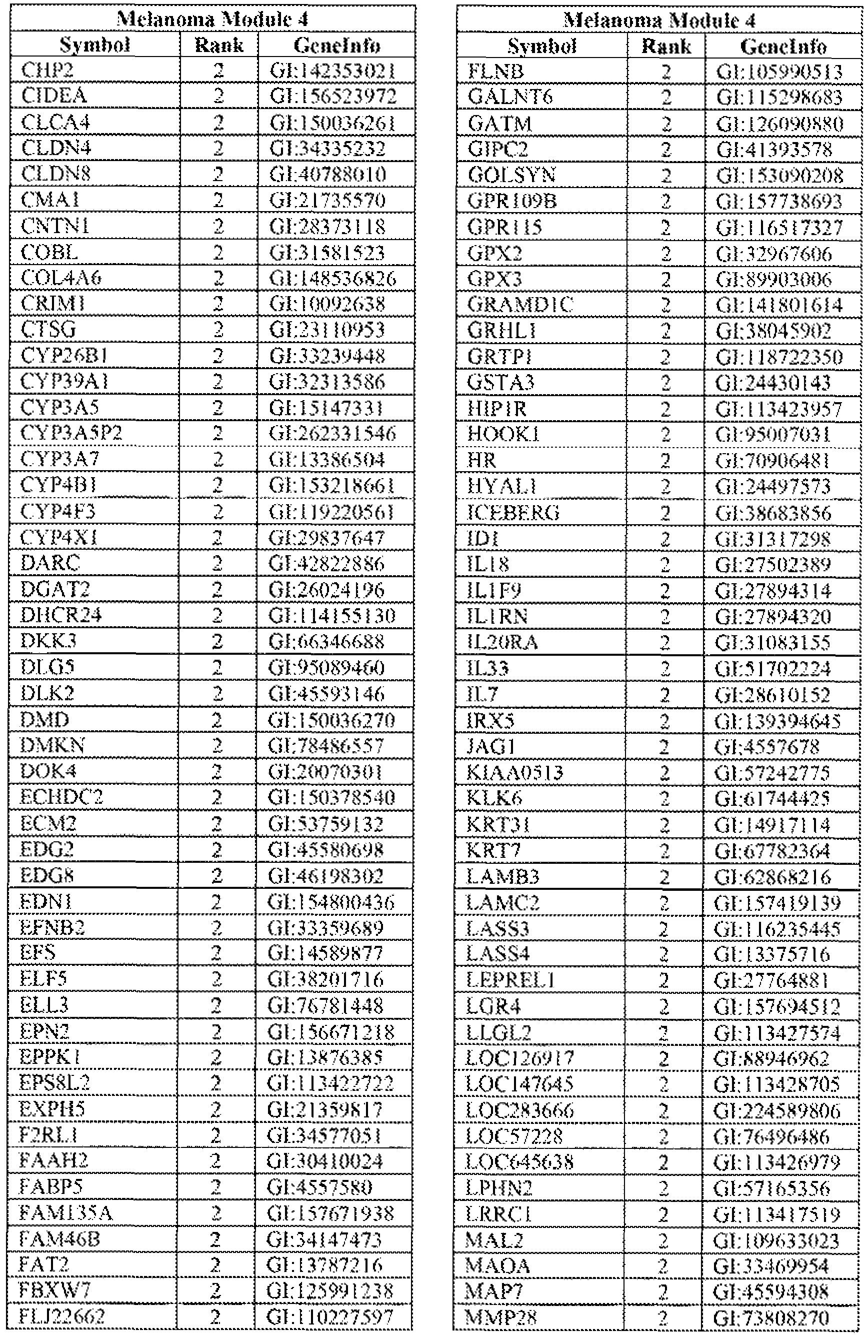

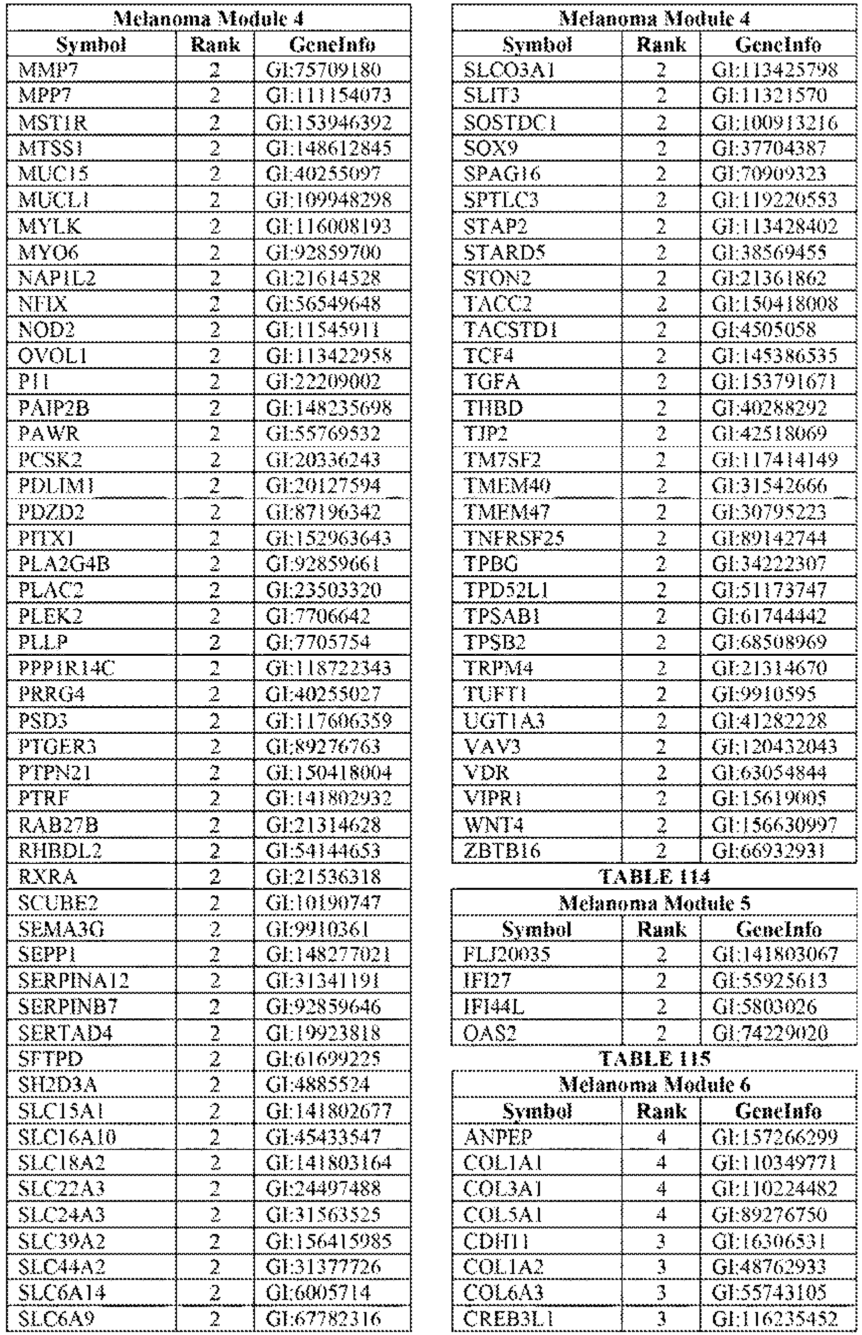

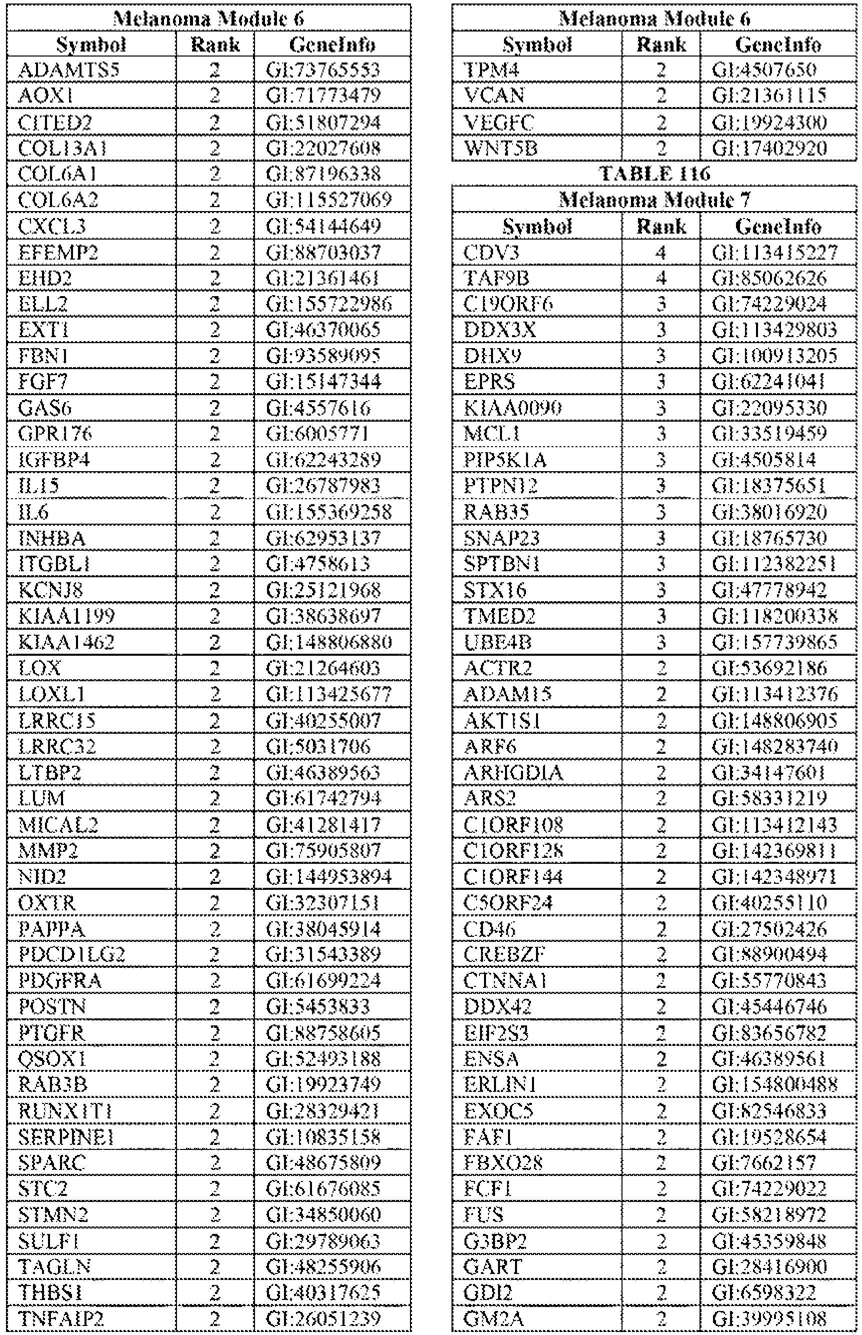

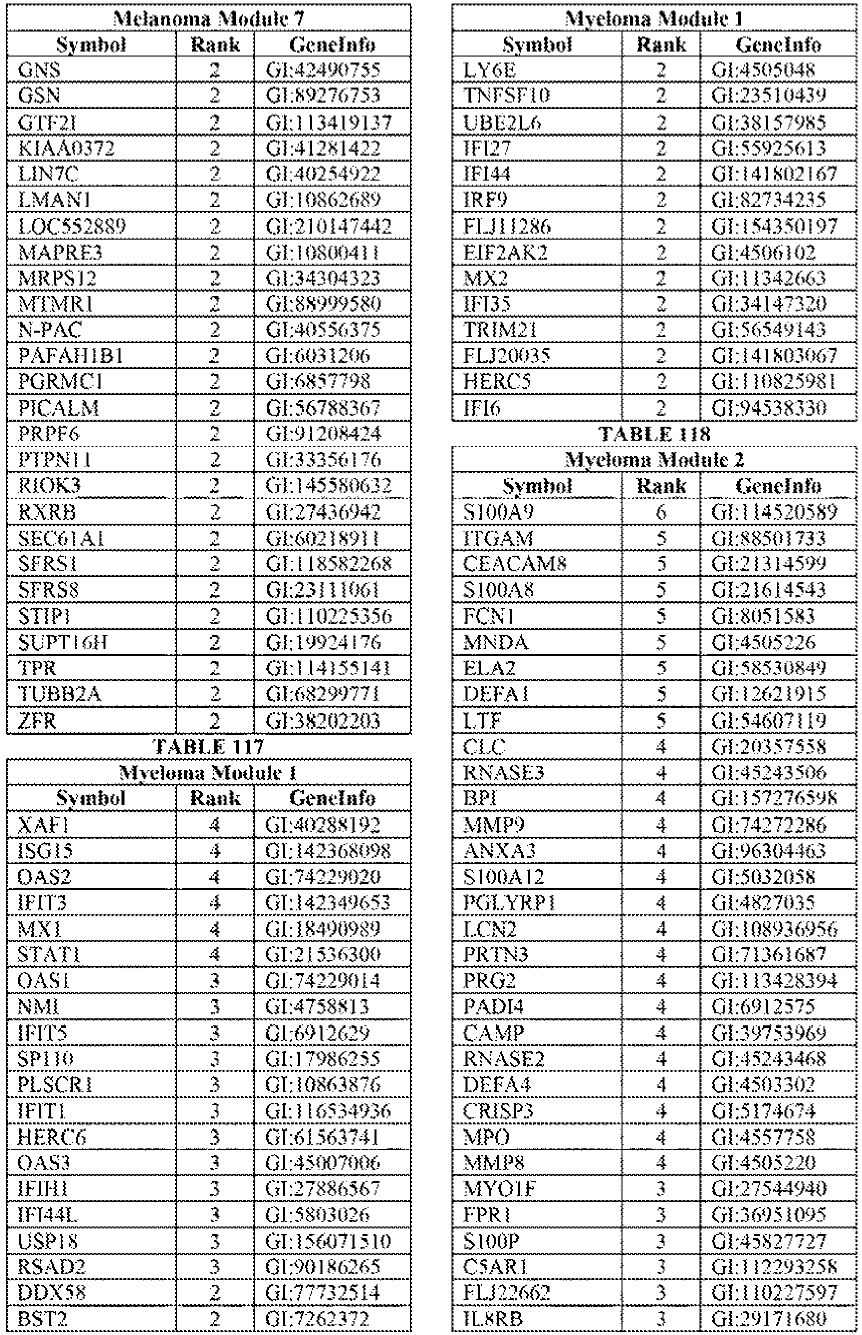

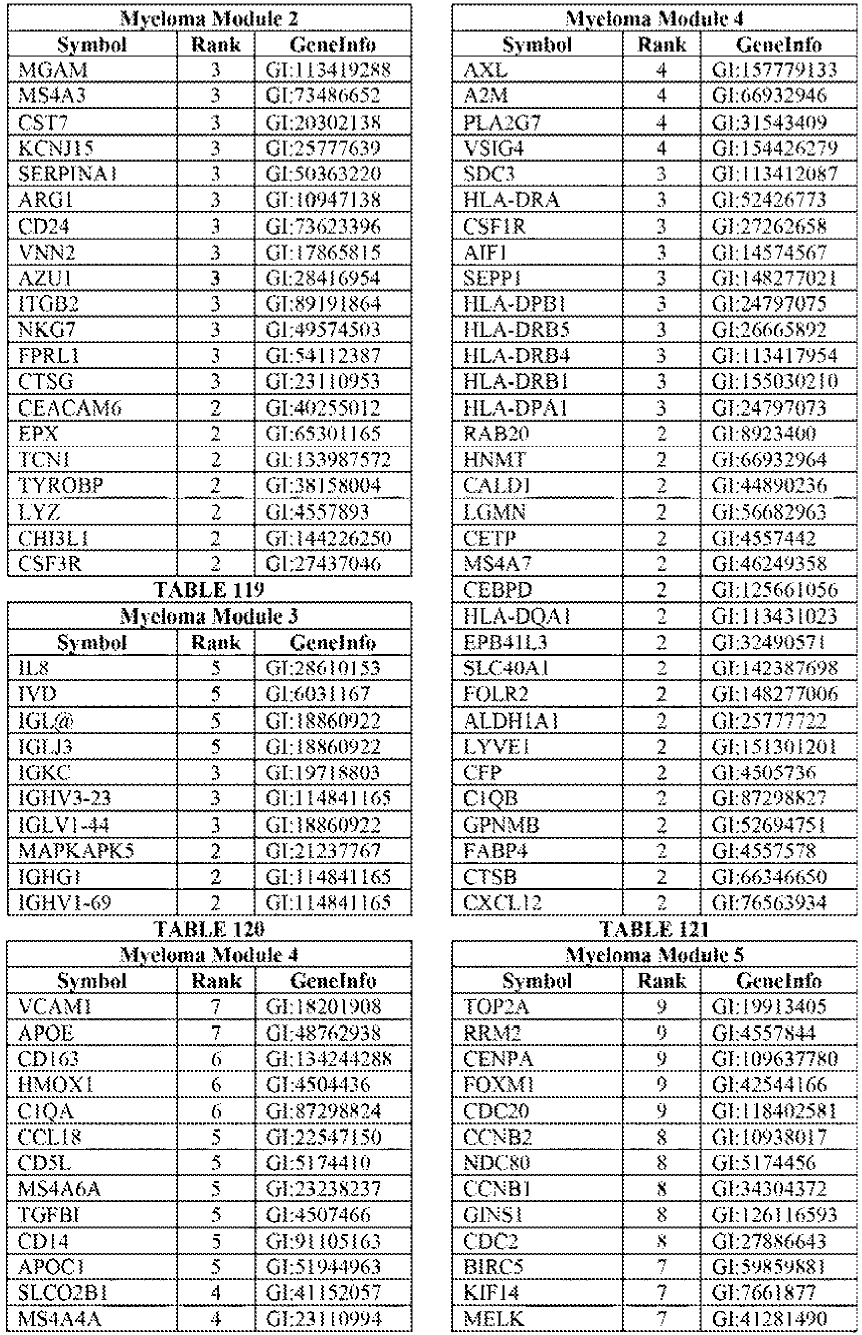

Aspects of the invention are as follows:
1. Aspect 1: A method of identifying a patient's cancer as belonging to a Cancer Module, comprising:
   a. interrogating a biological sample obtained from the patient for expression of one or more gene member of one or more Cancer Module selected from:
      i. one or more of Bladder Cancer Modules 1 - 6 of Tables 1-6;
      ii. one or more of Brain Cancer Modules 1 - 15 of Tables 7-21;
      iii. one or more of Breast Cancer Modules 1 - 25 of Tables 22-46;
      iv. one or more of Colon Cancer Modules 1 - 10 of Tables 47-56;
      v. one or more of Leukemia Modules 1 - 17 of Tables 57-73;
      vi. one or more of Liver Cancer Modules 1 - 10 of Tables 74-83;
      vii. one or more of Lung Cancer Modules 1 - 16 of Tables 84-99;
      viii. one or more of Lymphoma Modules 1 - 10 of Tables 100-109;
      ix. one or more of Melanoma Modules 1 - 7 of Tables 110-116;
      x. one or more of Myeloma Modules 1 - 5 of Tables 117-121;
      xi. one or more of Ovarian Cancer Modules 1 - 10 of Tables 122-131;
      xii. one or more of Pancreatic Cancer Modules 1 - 5 of Tables 132- 136;
      xiii. one or more of Prostate Cancer Modules 1 - 11 of Tables 137-147;
      xiv. one or more of Renal Cancer Modules 1 - 5 of Tables 148-152; and
      xv. one or more of Sarcoma Modules 1 - 9 of Tables 153-161; and
   b. identifying the patient's cancer as belonging to a Cancer Module having a pattern of gene expression consistent with the expression of said one or more gene member interrogated in the biological sample.
2. Aspect 2: A method for predicting drug responsiveness in a cancer patient population, comprising:
   a. interrogating biological samples obtained from patients in a training group for expression of one or more gene member of one or more Cancer Module selected from:
      i. one or more of Bladder Cancer Modules 1 - 6 of Tables 1-6;
      ii. one or more of Brain Cancer Modules 1 - 15 of Tables 7-21;
      iii. one or more of Breast Cancer Modules 1 - 25 of Tables 22-46;
      iv. one or more of Colon Cancer Modules 1 - 10 of Tables 47-56;
      v. one or more of Leukemia Modules 1 - 17 of Tables 57-73;
      vi. one or more of Liver Cancer Modules 1 - 10 of Tables 74-83;
      vii. one or more of Lung Cancer Modules 1 - 16 of Tables 84-99;
      viii. one or more of Lymphoma Modules 1 - 10 of Tables 100-109;
      ix. one or more of Melanoma Modules 1 - 7 of Tables 110-116;
      x. one or more of Myeloma Modules 1 - 5 of Tables 117-121;
      xi. one or more of Ovarian Cancer Modules 1 - 10 of Tables 122-131;
      xii. one or more of Pancreatic Cancer Modules 1 - 5 of Tables 132- 136;
      xiii. one or more of Prostate Cancer Modules 1 - 11 of Tables 137-147;
      xiv. one or more of Renal Cancer Modules 1 - 5 of Tables 148-152; and
      xv. one or more of Sarcoma Modules 1 - 9 of Tables 153-161; and
   b. identifying each training patient's cancer as belonging to a Cancer Module having a pattern of gene expression consistent with the expression of said one or more gene interrogated in the patient's biological sample;
   c. identifying response of each training patient's cancer to a test drug as responsive or non-responsive to the drug;
   d. identifying one or more Cancer Module as consistent with training patient responsiveness to the drug;
   e. identifying one or more Cancer Module as consistent with training patient non-responsiveness to the drug;
   f. predicting as responsive to the drug test patients expressing one or more gene member of a Cancer Module identified as consistent with training patient responsiveness in step d; and/or
   g. predicting as non-responsive to the drug test patients expressing one or more gene member of a Cancer Module identified as consistent with training patient non-responsiveness in step e.
3. Aspect 3: A method for predicting risk of recurrence in a cancer patient population, comprising:
   a. interrogating biological samples obtained from patients in a training group for expression of one or more gene member of one or more Cancer Module selected from:
      i. one or more of Bladder Cancer Modules 1 - 6 of Tables 1-6;
      ii. one or more of Brain Cancer Modules 1 - 15 of Tables 7-21;
      iii. one or more of Breast Cancer Modules 1 - 25 of Tables 22-46;
      iv. one or more of Colon Cancer Modules 1 - 10 of Tables 47-56;
      v. one or more of Leukemia Modules 1 - 17 of Tables 57-73;
      vi. one or more of Liver Cancer Modules 1 - 10 of Tables 74-83;
      vii. one or more of Lung Cancer Modules 1 - 16 of Tables 84-99;
      viii. one or more of Lymphoma Modules 1 - 10 of Tables 100-109;
      ix. one or more of Melanoma Modules 1 - 7 of Tables 110-116;
      x. one or more of Myeloma Modules 1 - 5 of Tables 117-121;
      xi. one or more of Ovarian Cancer Modules 1 - 10 of Tables 122-131;
      xii. one or more of Pancreatic Cancer Modules 1 - 5 of Tables 132- 136;
      xiii. one or more of Prostate Cancer Modules 1 - 11 of Tables 137-147;
      xiv. one or more of Renal Cancer Modules 1 - 5 of Tables 148-152; and
      xv. one or more of Sarcoma Modules 1 - 9 of Tables 153-161; and
   b. identifying each training patient's cancer as belonging to a Cancer Module having a pattern of gene expression consistent with the expression of said one or more gene interrogated in the patient's biological sample;
   c. identifying recurrence of each training patient's cancer as recurrent or non-recurrent;
   d. identifying one or more Cancer Module as consistent with recurrence of training patient cancer;
   e. identifying one or more Cancer Module as consistent with non- recurrence of training patient cancer;
   f. predicting cancer recurrence in test patients expressing one or more gene member of a Cancer Module identified as consistent with training patient recurrence in step d; and/or
   g. predicting non-recurrence of cancer in test patients expressing one or more gene member of a Cancer Module identified as consistent with non-recurrence of training patient cancer in step e.
4. Aspect 4: A method for predicting risk of metastasis in a patient population, comprising:
   a. interrogating biological samples obtained from patients in a training group for expression of one or more gene member of one or more Cancer Module selected from:
      i. one or more of Bladder Cancer Modules 1 - 6 of Tables 1-6;
      ii. one or more of Brain Cancer Modules 1 - 15 of Tables 7-21;
      iii. one or more of Breast Cancer Modules 1 - 25 of Tables 22-46;
      iv. one or more of Colon Cancer Modules 1 - 10 of Tables 47-56;
      v. one or more of Leukemia Modules 1 - 17 of Tables 57-73;
      vi. one or more of Liver Cancer Modules 1 - 10 of Tables 74-83;
      vii. one or more of Lung Cancer Modules 1 - 16 of Tables 84-99;
      viii. one or more of Lymphoma Modules 1 - 10 of Tables 100-109;
      ix. one or more of Melanoma Modules 1 - 7 of Tables 110-116;
      x. one or more of Myeloma Modules 1 - 5 of Tables 117-121;
      xi. one or more of Ovarian Cancer Modules 1 - 10 of Tables 122-131;
      xii. one or more of Pancreatic Cancer Modules 1 - 5 of Tables 132- 136;
      xiii. one or more of Prostate Cancer Modules 1 - 11 of Tables 137-147;
      xiv. one or more of Renal Cancer Modules 1 - 5 of Tables 148-152; and
      xv. one or more of Sarcoma Modules 1 - 9 of Tables 153-161; and
   b. identifying each training patient's cancer as belonging to a Cancer Module having a pattern of gene expression consistent with the expression of said one or more gene interrogated in the patient's biological sample;
   c. identifying each training patient's cancer as demonstrating metastasis or not demonstrating metastasis;
   d. identifying one or more Cancer Module as consistent with demonstrated metastasis in the training patients;
   e. identifying one or more Cancer Module as consistent with non- demonstrated metastasis in the training patients;
   f. predicting risk of metastasis in test patients expressing one or more gene member of a Cancer Module identified as consistent with metastasis in step d; and/or
   g. predicting no risk of metastasis in test patients expressing one or more gene member of a Cancer Module identified as consistent with non-demonstrated metastasis in step e.
5. Aspect 5: A method for treating a patient with a therapeutic drug, comprising:
   a. interrogating a biological sample obtained the patient for expression of one or more gene member of one or more Cancer Module selected from:
      i. one or more of Bladder Cancer Modules 1 - 6 of Tables 1-6;
      ii. one or more of Brain Cancer Modules 1 - 15 of Tables 7-21;
      iii. one or more of Breast Cancer Modules 1 - 25 of Tables 22-46;
      iv. one or more of Colon Cancer Modules 1 - 10 of Tables 47-56;
      v. one or more of Leukemia Modules 1 - 17 of Tables 57-73;
      vi. one or more of Liver Cancer Modules 1 - 10 of Tables 74-83;
      vii. one or more of Lung Cancer Modules 1 - 16 of Tables 84-99;
      viii. one or more of Lymphoma Modules 1 - 10 of Tables 100-109;
      ix. one or more of Melanoma Modules 1 - 7 of Tables 110-116;
      x. one or more of Myeloma Modules 1 - 5 of Tables 117-121;
      xi. one or more of Ovarian Cancer Modules 1 - 10 of Tables 122- 131;
      xii. one or more of Pancreatic Cancer Modules 1 - 5 of Tables 132- 136;
      xiii. one or more of Prostate Cancer Modules 1 - 11 of Tables 137- 147;
      xiv. one or more of Renal Cancer Modules 1-5 of Tables 148-152; and
      xv. one or more of Sarcoma Modules 1-9 of Tables 153-161; and
   b. identifying the patient's cancer as belonging to a Cancer Module having a pattern of gene expression consistent with the expression of said one or more gene interrogated in the patient's biological sample;
   c. administering to the patient a drug having demonstrated activity for cancers belonging to the modules identified in step b.
6. Aspect 6: A method for selecting a therapeutic drug for an individual cancer patient comprising:
   a. interrogating a biological sample obtained the patient for expression of one or more gene member of one or more Cancer Module selected from:
      i. one or more of Bladder Cancer Modules 1-6 of Tables 1-6;
      ii. one or more of Brain Cancer Modules 1-15 of Tables 7-21;
      iii. one or more of Breast Cancer Modules 1-25 of Tables 22-46;
      iv. one or more of Colon Cancer Modules 1-10 of Tables 47-56;
      v. one or more of Leukemia Modules 1-17 of Tables 57-73;
      vi. one or more of Liver Cancer Modules 1-10 of Tables 74-83;
      vii. one or more of Lung Cancer Modules 1-16 of Tables 84-99;
      viii. one or more of Lymphoma Modules 1-10 of Tables 100-109;
      ix. one or more of Melanoma Modules 1-7 of Tables 110-116;
      x. one or more of Myeloma Modules 1-5 of Tables 117-121;
      xi. one or more of Ovarian Cancer Modules 1-10 of Tables 122-131;
      xii. one or more of Pancreatic Cancer Modules 1-5 of Tables 132- 136;
      xiii. one or more of Prostate Cancer Modules 1-11 of Tables 137-147;
      xiv. one or more of Renal Cancer Modules 1-5 of Tables 148-152; and
      xv. one or more of Sarcoma Modules 1-9 of Tables 153-161; and
   b. identifying the patient's cancer as belonging to a Cancer Module having a pattern of gene expression consistent with the expression of said one or more gene interrogated in the patient's biological sample;
   c. selecting for administration to the patient a drug having demonstrated activity against cancers belonging to the modules identified in step b; and/or
   d. not selecting for administration to the patient a drug demonstrated as lacking activity against cancers belonging to the modules identified in step b.
7. Aspect 7: The method of any of aspects 1-6, wherein said interrogating includes analyzing gene expression by direct or indirect methods.
8. Aspect 8: The method of aspect 7, wherein analyzing gene expression comprises DNA analysis, RNA analysis, or protein analysis methods.
9. Aspect 9: The method of any of aspect 1-6, wherein expression of 2, 3, 4, 5, 6, 7, 8, 9, 10-15, or more gene members is analyzed.
10. Aspect 10: The method of any of aspects 1-9, wherein 3 or more gene members from a Cancer Module are selected for analysis.
11. Aspect 11: The method of aspect 10, wherein said 3 or more gene members are selected from gene members having the highest ranking within a Cancer Module.
12. Aspect 12: The method of aspect 10, wherein said 3 or more gene members are selected from gene members having a ranking of 3 or greater.
13. Aspect 13: The method of aspect 10, wherein said 3 or more gene members are selected from gene members having a ranking among the highest 2 rankings within a Cancer Module.
14. Aspect 14: The method of aspect 10, wherein said 3 or more gene members are selected from gene members having a ranking among the highest 3 rankings within a Cancer Module.
15. Aspect 15: An assay system for identifying a patient's cancer as belonging to a Cancer Module as performed according to the method of aspect 1, comprising:
   a. reagents or tools designed for measuring expression of one or more gene member of one or more gene expression module selected from:
      i. one or more of Bladder Cancer Modules 1 - 6 of Tables 1-6;
      ii. one or more of Brain Cancer Modules 1 - 15 of Tables 7-21;
      iii. one or more of Breast Cancer Modules 1 - 25 of Tables 22-46;
      iv. one or more of Colon Cancer Modules 1 - 10 of Tables 47-56;
      v. one or more of Leukemia Modules 1 - 17 of Tables 57-73;
      vi. one or more of Liver Cancer Modules 1 - 10 of Tables 74-83;
      vii. one or more of Lung Cancer Modules 1 - 16 of Tables 84-99;
      viii. one or more of Lymphoma Modules 1 - 10 of Tables 100-109;
      ix. one or more of Melanoma Modules 1 - 7 of Tables 110-116;
      x. one or more of Myeloma Modules 1 - 5 of Tables 117-121;
      xi. one or more of Ovarian Cancer Modules 1 - 10 of Tables 122-131;
      xii. one or more of Pancreatic Cancer Modules 1 - 5 of Tables 132-136;
      xiii. one or more of Prostate Cancer Modules 1 - 11 of Tables 137- 147;
      xiv. one or more of Renal Cancer Modules 1 - 5 of Tables 148-152; and
      xv. one or more of Sarcoma Modules 1 - 9 of Tables 153-161; and
   b. means for identifying the patient's cancer as belonging to a Cancer Module having a pattern of gene expression consistent with the expression of said one or more gene interrogated in the patient's biological sample.
16. Aspect 16: The assay system of aspect 15, wherein said reagents and tools are designed to measure two or more gene members of one or more Cancer Module.
17. Aspect 17: The assay of aspects 15 or 16, wherein 3 or more gene members from a Cancer Module are selected for analysis.
18. Aspect 18: The assay of aspect 17, wherein said 3 or more gene members are selected from gene members having the highest ranking within a Cancer Module.
19. Aspect 19: The assay of aspect 17, wherein said 3 or more gene members are selected from gene members having a ranking of 3 or greater.
20. Aspect 20: The assay of aspect 17, wherein said 3 or more gene members are selected from gene members having a ranking among the highest 2 rankings within a Cancer Module.
21. Aspect 21: The assay of aspect 17, wherein said 3 or more gene members are selected from gene members having a ranking among the highest 3 rankings within a Cancer Module.

## Claims

1. A method of classifying a patient's cancer as belonging to a Lung Cancer Module, comprising:
a. interrogating a biological sample obtained from the patient for expression of one or more gene member of
each of Lung Cancer Modules 1 - 16 of Tables 84-99;
and
b. identifying the patient's cancer as belonging to a Lung Cancer Module having a pattern of gene expression consistent with the expression of said one or more gene member interrogated in the biological sample.

2. A method for predicting drug responsiveness in a cancer patient population, comprising:
a. interrogating biological samples obtained from patients in a training group for expression of one or more gene member of
each of Lung Cancer Modules 1 - 16 of Tables 84-99;
and
b. identifying each training patient's cancer as belonging to a Lung Cancer Module having a pattern of gene expression consistent with the expression of said one or more gene interrogated in the patient's biological sample;
c. identifying response of each training patient's cancer to a test drug as responsive or non-responsive to the drug;
d. identifying one or more Lung Cancer Module as consistent with training patient responsiveness to the drug;
e. identifying one or more Lung Cancer Module as consistent with training patient non-responsiveness to the drug;
f. predicting as responsive to the drug test patients expressing one or more gene member of a Lung Cancer Module identified as consistent with training patient responsiveness in step d; and/or
g. predicting as non-responsive to the drug test patients expressing one or more gene member of a Lung Cancer Module identified as consistent with training patient non-responsiveness in step e.

3. A method for predicting risk of recurrence in a cancer patient population, comprising:
a. interrogating biological samples obtained from patients in a training group for expression of one or more gene member of each of Lung Cancer Modules 1 - 16 of Tables 84-99;
and
b. identifying each training patient's cancer as belonging to a Lung Cancer Module having a pattern of gene expression consistent with the expression of said one or more gene interrogated in the patient's biological sample;
c. identifying recurrence of each training patient's cancer as recurrent or non-recurrent;
d. identifying one or more Lung Cancer Module as consistent with recurrence of training patient cancer;
e. identifying one or more Lung Cancer Module as consistent with non- recurrence of training patient cancer;
f. predicting cancer recurrence in test patients expressing one or more gene member of a Lung Cancer Module identified as consistent with training patient recurrence in step d; and/or
g. predicting non-recurrence of cancer in test patients expressing one or more gene member of a Lung Cancer Module identified as consistent with non-recurrence of training patient cancer in step e.

4. A method for predicting risk of metastasis in a patient population, comprising:
a. interrogating biological samples obtained from patients in a training group for expression of one or more gene member of each of Lung Cancer Modules 1 - 16 of Tables 84-99;
and
b. identifying each training patient's cancer as belonging to a Lung Cancer Module having a pattern of gene expression consistent with the expression of said one or more gene interrogated in the patient's biological sample;
c. identifying each training patient's cancer as demonstrating metastasis or not demonstrating metastasis;
d. identifying one or more Lung Cancer Module as consistent with demonstrated metastasis in the training patients;
e. identifying one or more Cancer Module as consistent with non-demonstrated metastasis in the training patients;
f. predicting risk of metastasis in test patients expressing one or more gene member of a Lung Cancer Module identified as consistent with metastasis in step d; and/or
g. predicting no risk of metastasis in test patients expressing one or more gene member of a Lung Cancer Module identified as consistent with non-demonstrated metastasis in step e.

5. A method for treating a patient with a therapeutic drug, comprising:
a. interrogating a biological sample obtained the patient for expression of one or more gene member of each of Lung Cancer Modules 1 - 16 of Tables 84-99;
and
b. identifying the patient's cancer as belonging to a Lung Cancer Module having a pattern of gene expression consistent with the expression of said one or more gene interrogated in the patient's biological sample;
c. administering to the patient a drug having demonstrated activity for cancers belonging to the modules identified in step b.

6. A method for selecting a therapeutic drug for an individual cancer patient comprising:
a. interrogating a biological sample obtained the patient for expression of one or more gene member of each of Lung Cancer Modules 1-16 of Tables 84-99;
and
b. identifying the patient's cancer as belonging to a Lung Cancer Module having a pattern of gene expression consistent with the expression of said one or more gene interrogated in the patient's biological sample;
c. selecting for administration to the patient a drug having demonstrated activity against cancers belonging to the modules identified in step b; and/or
d. not selecting for administration to the patient a drug demonstrated as lacking activity against cancers belonging to the modules identified in step b.

7. The method of any of claims 1-6, wherein expression of 2, 3, 4, 5, 6, 7, 8, 9, 10-15, or more gene members is analyzed.

8. The method of any of claims 1-7, wherein 3 or more gene members from each of Lung Cancer Modules 1 - 16 are selected for analysis.

9. The method of claim 8, wherein said 3 or more gene members are selected from gene members having a ranking of 3 or greater.

10. The method of claim 8, wherein said 3 or more gene members are selected from gene members having a ranking among the highest 3 rankings within each Cancer Module.

11. An assay system for identifying a patient's cancer as belonging to a Lung Cancer Module as performed according to the method of claim 1, comprising:
a. reagents or tools designed for measuring expression of one or more gene member of each of Lung Cancer Modules 1 - 16 of Tables 84-99;
and
b. means for identifying the patient's cancer as belonging to a Lung Cancer Module having a pattern of gene expression consistent with the expression of said one or more gene interrogated in the patient's biological sample.

12. The assay system of claim 11, wherein said reagents and tools are designed to measure two or more gene members of each Lung Cancer Module.

13. The assay of claims 11 or 12, wherein 3 or more gene members from each Cancer Module are selected for analysis.

14. The assay of claim 13, wherein said 3 or more gene members are selected from gene members having a ranking of 3 or greater.

15. The assay of claim 13, wherein said 3 or more gene members are selected from gene members having a ranking among the highest 3 rankings within each Cancer Module.
